# EUROPEAN PATENT SPECIFICATION

(11) **EP 2 202 297 B1**
(45) Date of publication and mention of the grant of the patent: **14.05.2014**
(21) Application number: 10003287.9
(22) Date of filing: 18.07.2008
(51) Int. Cl.: C12N 7/04, C12N 15/863, C07K 14/07, A61K 35/76, A61K 39/285, A61K 48/00

(54) **Use of a chemotherapeutic agent in the preparation of a medicament for treating or ameliorating an adverse side effect associated with oncolytic viral therapy**
Verwendung eines chemotherapeutischen Wirkstoffs zur Vorbereitung eines Arzneimittels zur Behandlung oder Milderung der mit oncolytischen Virentherapie verbundenen Nebenwirkungen
Utilisation d'un agent chemothérapeutique dans la préparation d'un médicament pour le traitement ou l'amélioration d'effets collatéraux des thérapies avec virus oncolytique

(30) Priority: 18.07.2007 US 950587 P; 22.10.2007 US 981748 P
(43) Date of publication of application: 30.06.2010
(62) Divisional of application: 08794597.8
(73) Proprietor: Genelux Corporation, San Diego, California 92109 (US)
(72) Inventor: Szalay, Aladar A., Highland, CA 92346 (US); Chen, Nanhai, San Diego, CA 92129 (US); Yu, Yong A., San Diego, CA 92130 (US)
(74) Representative: Boult Wade Tennant

(56) References cited:
- US-A1- 2005 031 643
- SIDWELL R W ET AL: "In vivo antiviral properties of biologically active compounds" APPLIED MICROBIOLOGY, AMERICAN SOCIETY FOR MICROBIOLOGY, US, vol. 16, no. 2, 1 February 1968 (1968-02-01), pages 370-392, XP002499728 ISSN: 0003-6919
- TOPALIS (2005); FEBS J 272:6254-5265
- RUIZ (2006); ANTIVIRAL RES. 75(1):87-90
- REEVES (2005); NAT. MED. 11:731-739
- MCFADDEN (2005); NAT. MED. 11:711-712
- YANG (2005); J. VIROL 79:13139-13149
- BLACK (1992); JBC 267(14):9743-9748
- METZGER (1994); J. VIROL. 68(12):8423-8427
- DE CLERCQ (1998); J. MED. MICROBIOL. 47:1-3
- KANERVA (2007); J. GENE MED. 9:3-9
- E. DE CLERCQ: "Historical perspectives in the development of antiviral agents against poxviruses", VIRUSES, vol. 2010, no. 2, 14 June 2010 (2010-06-14), pages 1322-1339,

## Description

### RELATED APPLICATIONS

Benefit of priority is claimed to U.S. Provisional Application Serial No. 60/950,587, to Nanhai Chen and Yong A. Yu, filed on July 18, 2007, entitled "USE OF MODIFIED VACCINIA VIRUS STRAINS IN COMBINATION WITH A CHEMOTHERAPEUTIC AGENT FOR USE IN THERAPEUTIC METHODS" and to U.S. Provisional Application Serial No. 60/981,748, to Nanhai Chen and Yong A. Yu, filed on October 22, 2007, entitled "USE OF MODIFIED VACCINIA VIRUS STRAINS IN COMBINATION WITH A CHEMOTHERAPEUTIC AGENT FOR USE IN THERAPEUTIC METHODS"

This application is related to U.S. Application Serial No. 12/218,953 to Nanhai Chen and Yong A. Yu, filed on July 18,2008, entitled "USE OF MODIFIED VACCINIA VIRUS STRAINS IN COMBINATION WITH A CHEMOTHERAPEUTIC AGENT FOR USE IN THERAPEUTIC METHODS," which also claims priority to U.S. Provisional Application Serial Nos. 60/950,587 and 60/981,748.

This application is related to U.S. Application Serial No. 11/975,088, filed on October 16, 2007, entitled "METHODS FOR ATTENUATING VIRUS STRAINS FOR DIAGNOSTIC AND THERAPEUTIC USES," to U.S. Application Serial No. 11/975,090, filed on October 16, 2007, entitled "MODIFIED VACCINIA VIRUS STRAINS FOR USE IN DIAGNOSTIC AND THERAPEUTIC METHODS," to U.S. Application Serial No. 12/080,766, filed on April 4, 2008, entitled "METHODS FOR ATTENUATING VIRUS STRAINS FOR DIAGNOSTIC AND THERAPEUTIC USES," and to International Application No. PCT/US2007/022172, filed on October 16, 2007, entitled "MODIFIED VACCINIA VIRUS STRAINS FOR USE IN DIAGNOSTIC AND THERAPEUTIC METHODS."

This application also is related to U.S. Application Serial No. 12/157,960 to Nanhai Chen, Yuman Fong, Aladar A. Szalay, Yong A. Yu and Qian Zhang, filed on June 13, 2008, entitled "MICROORGANISMS FOR IMAGING AND/OR TREATMENT OF TUMORS" and to International Application No. PCT/US2008/007377 to Nanhai Chen, Yuman Fong, Aladar A. Szalay, Yong A. Yu and Qian Zhang, filed on June 13, 2008, entitled "MICROORGANISMS FOR IMAGING AND/OR TREATMENT OF TUMORS."

This application is related to U.S. Application Serial No. 10/872,156, to Aladar A. Szalay, Tatyana Timiryasova, Yong A. Yu and Qian Zhang, filed on June 18,2004, entitled "MICROORGANISMS FOR THERAPY," which claims the benefit of priority under 35 U.S.C. §119(a) to each of EP Application No. 03 013 826.7, filed 18 June 2003, entitled "Recombinant vaccinia viruses useful as tumor-specific delivery vehicle for cancer gene therapy and vaccination," EP Application No. 03 018 478.2, filed 14 August 2003, entitled "Method for the production of a polypeptide, RNA or other compound in tumor tissue," and EP Application No. 03 024 283.8, filed 22 October 2003, entitled "Use of a Microorganism or Cell to Induce Autoimmunization of an Organism Against a Tumor." This application also is related to International Application No. PCT/US04/19866, filed on June 18, 2004, entitled "MICROORGANISMS FOR THERAPY."

This application also is related to U.S. Application Serial No. 10/866,606, filed June 10, 2004, entitled "Light emitting microorganisms and cells for diagnosis and therapy of tumors," which is a continuation of U.S. Application Serial No. 10/189,918, filed July 3, 2002, entitled "Light emitting microorganisms and cells for diagnosis and therapy of tumors." This application also is related to International PCT Application PCT/IB02/04767, filed July 31, 2002, entitled "Microorganisms and Cells for Diagnosis and Therapy of Tumors," EP Application No. 01 118 417.3, filed July 31, 2001, entitled "Light-emitting microorganisms and cells for tumor diagnosis/therapy," EP Application No. 01 125 911.6, filed October 30, 2001, entitled "Light emitting microorganisms and cells for diagnosis and therapy of tumors" and EP Application No. 02 0794 632.6, filed July 31, 2002, entitled "Microorganisms and Cells for Diagnosis and Therapy of Tumors."

### FIELD OF THE INVENTION

The invention relates to compositions containing modified and/or attenuated viruses in combination with chemotherapeutic agents, and methods for preparing and using the compositions. The invention also relates to methods for using compositions containing modified and/or attenuated viruses that are administered with chemotherapeutic agents. The invention also relates to Diagnostic and therapeutic methods.

### BACKGROUND

Chemotherapeutic agents are commonly used in the treatment of cancer. Examples of chemotherapeutic agents include, for example, 5-fluorouracil (5-FU), gemcitabine, cisplatin, irinotecan and doxorubicin. Chemotherapeutic agents often are involved in interference with DNA replication and transcription; they act upon cancerous cells and tumors by inhibiting the ability of cells to divide and grow. 5-FU is a pyrimidine analogue that exhibits anti-tumor activity. Its mode of action is principally through inhibition of an enzyme, thymidylate synthase, that is involved in pyrimidine synthesis for DNA replication. Gemcitabine, also known as 4-amino-1-[3,3-difluoro-4-hydroxy-5-(hydroxymethyl) tetrahydrofuran-2-yl]-1H-pyrimidine-2-one, is a nucleoside analogue that also exhibits antitumor activity. The mechanism of therapy is related to the ability of gemcitabine to inhibit DNA synthesis through competition with deoxycytosinetriphosphate (dCTP) for incorporation into DNA. Cisplatin (also known as cisplatinum and CDDP) is a platinum-based chemotherapy that acts by crosslinking DNA, which inhibits DNA replication. Irinotecan is a chemotherapeutic agent that is a topoisomerase 1 inhibitor; thus, it acts to inhibit the relaxation of DNA during winding/unwinding of the double helix during DNA replication, leading to inhibition of both DNA replication and transcription. Doxorubicin is a DNA-interacting drug that can intercalate DNA and interfere with the action of topoisomerase II, thereby leading to inhibition of DNA replication and transcription.

Modified and attenuated viruses are used for treatment and therapy of diseases such as, for example, cancer. Mutation of non-essential genes is a method of attenuation that preserves the ability of the virus to propagate without the need of a packaging cell lines. In viruses such as vaccinia virus, mutations in non-essential genes, such as the thymidine kinase (*TK*) gene or hemagglutinin (*HA*) gene have been employed to attenuate the virus (e.g., Buller et al. (1985) Nature 317, 813-815, Shida et al. (1988) J. Virol. 62(12):4474-80, Taylor et al. (1991) J. Gen. Virol. 72 (Pt 1): 125-30, U.S. Patent Nos. 5,364,773, 6,265,189, 7,045,313). The inactivation of these genes decreases the overall pathogenicity of the virus without affecting the ability of the viruses to replicate in certain cell types. The viruses selectively infect, replicate within, and lyse cancer cells, and can be used in treatment of a wide variety of cancers. The treatment of disease by these agents can be accompanied unpleasant side effects that can result in patient non-compliance or cessation of treatment.

US 2005/031643 A1 relates to microorganisms for therapy.

### SUMMARY

The present invention provides:
(1) Use of an anti-viral agent in the preparation of a medicament for treating an adverse side effect associated with oncolytic pox virus therapy of cancer, wherein the anti-viral agent is selected from among ST-246, cidofovir, alkoxyalkyl esters of cidofovir and imatinib.
(2) An anti-virus agent for use in ameliorating an adverse side effect associated with oncolytic pox virus therapy of cancer, wherein the anti-viral agent is selected from among ST-246, cidofovir, alkoxyalkyl esters of cidofovir and imatinib.
(3) The use of (1) or (2), wherein the adverse side effect comprises one or more of pock formation, weight loss, fever, abdominal pain, aches or pains in muscles, cough, diarrhea and feeling of discomfort or illness.
(4) The use or agent of (1) or (2), wherein the oncolytic pox virus is a vaccinia virus.
(5) The use or agent of (4), wherein the virus is a Lister strain vaccinia virus.
(6) The use or agent of (5), wherein the virus strain is LIVP.
(7) The use or agent of (6), wherein the virus is selected from among GLV-1h68, whose genome comprises SEQ ID NO:1, and viruses derived from GLV-1h68.
(8) The use or agent of any of (1)-(6), wherein the cancer is a metastatic cancer and/or a solid tumor.
(9) The use or agent of any of (1)-(8), wherein the virus encodes a detectable gene product or gene product that induces a detectable signal.
(10) The use or agent of (9), wherein the gene product is a luciferase or a fluorescent protein.
(11) The use or agent of any of (1)-(8), wherein the virus encodes a therapeutic gene product.

Described herein are methods and compositions for treatment of diseases, such as cancer, that minimize or reduce undesired side effects. Among the compositions and methods are those for clearance of a virus administered to a subject for treatment of disease. Also described are methods for the use of chemotherapeutic agents in conjunction with modified vaccinia viruses, or viral vectors, for use in therapeutic methods. The methods permit the control of viral titer or viral load such that the patient from experiences minimal or reduced side effects and/or reduced toxicity associated with the administered virus. In particular examples, the adverse side effects are one or more of pock formation, weight loss, fever, abdominal pain, aches or pains in muscles, cough, diarrhea, and feeling of discomfort or illness.

Described herein are methods for treating one or more adverse side effects associated with viral treatment, where a chemotherapeutic agent is administered to a subject being treated with a therapeutic virus and the amount of chemotherapeutic agent administered is sufficient to control or reduce viral titer in the subject. In such methods, the subject is identified as one who exhibits one or more adverse effects following administration of the virus.

Described herein are methods for controlling viral load in a subject, comprising administering a chemotherapeutic agent to a subject treated with a therapeutic virus, where the subject exhibits a viral titer that is equal to or exceeds an amount that causes one or more adverse side effects in the subject during treatment with the virus. In such methods, the amount of chemotherapeutic agent administered is sufficient to control or reduce viral titer in the subject.

Described herein are methods of for treating one or more adverse side effects associated with viral treatment or controlling viral load in a subject, where the chemotherapeutic agents employed the methods can be administered systemically, intravenously, intraarterially, intratumorally, endoscopically, intralesionally, intramuscularly, intradermally, intraperitoneally, intravesicularly, intraarticularly, intrapleurally, percutaneously, subcutaneously, orally, parenterally, intranasally, intratracheally, by inhalation, intracranially, intraprostaticaly, intravitreally, topically, ocularly, vaginally, or rectally. Typically, the chemotherapeutic agent is administered systemically.

Methods to maintain or control numbers of a modified vaccinia virus delivered to a patient for treatment of cancer are contemplated such that the patient suffers minimal side effects or systemic toxicity associated with the administered virus, the methods comprising co-administration of the virus with a regimen of a chemotherapeutic agent that inhibits the replication of the virus. In some embodiments, the co-administration of virus and the chemotherapeutic agent allows the sustained release of virally-expressed antigens and the chemotherapeutic agent in a patient. In some embodiments, the co-administration of virus and the chemotherapeutic agent allows the sustained delivery of virally-expressed antigens and the chemotherapeutic agent in a patient.

Methods to maintain or control numbers of a modified vaccinia virus delivered to a patient for treatment of cancer such that the patient suffers minimal side effects or systemic toxicity associated with the virus are described, comprising: initial administration of a chemotherapeutic agent that inhibits the replication of the virus to the patient; administration of the virus; and subsequent co-administration of virus and the chemotherapeutic agent.

Methods are also described herein to maintain or control numbers of a modified vaccinia virus delivered to a patient for treatment of cancer such that the patient suffers minimal side effects or systemic toxicity associated with the virus comprising: initial administration of a chemotherapeutic agent that inhibits the replication of the virus to the patient; administration of the virus; and subsequent administration of the chemotherapeutic agent alone or in combination with one or more antiviral agents.

Also described herein are methods to effectively clear a modified vaccinia virus administered to a patient for treatment of a disease, comprising: administration of a chemotherapeutic agent that inhibits the replication of the virus in an amount effective to clear the virus in the patient, such that symptoms associated with the virus are reduced or eliminated in the patient.

Methods to maintain or control numbers of a modified vaccinia virus delivered to a patient for treatment of cancer are contemplated such that the patient suffers minimal side effects or systemic toxicity associated with the administered virus, the methods comprising co-administration of the virus with a regimen of gemcitabine. In some embodiments, the co-administration of virus and gemcitabine allows the sustained release of virally-expressed antigens and gemcitabine in a patient. In some embodiments, the co-administration of virus and gemcitabine allows the sustained delivery of virally-expressed antigens and gemcitabine in a patient.

Methods to maintain or control numbers of a modified vaccinia virus delivered to a patient for treatment of cancer such that the patient suffers minimal side effects or systemic toxicity associated with the virus are described, comprising: initial administration of gemcitabine to the patient; administration of the virus; and subsequent co-administration of virus and gemcitabine.

Methods are also described herein to maintain or control numbers of a modified vaccinia virus delivered to a patient for treatment of cancer such that the patient suffers minimal side effects or systemic toxicity associated with the virus comprising: initial administration of gemcitabine to the patient; administration of the virus; and subsequent administration of gemcitabine alone or in combination with one or more antiviral agents.

Also described herein are methods to effectively clear a modified vaccinia virus administered to a patient for treatment of a disease, comprising: administration of gemcitabine in an amount effective to clear the virus in the patient, such that symptoms associated with the virus are reduced or eliminated in the patient.

Also described herein are methods to maintain or control numbers of a modified vaccinia virus delivered to a patient for treatment of cancer such that the patient suffers minimal side effects or systemic toxicity associated with the virus comprising co-administration of the virus with a regimen of irinotecan. In some embodiments, the co-administration of virus and irinotecan allows the sustained release of virally-expressed antigens and irinotecan in a patient. In some embodiments, the co-administration of virus and irinotecan allows the sustained delivery of virally-expressed antigens and irinotecan in a patient.

Methods to maintain or control numbers of a modified vaccinia virus delivered to a patient for treatment of cancer such that the patient suffers minimal side effects or systemic toxicity associated with the virus are described, comprising: initial administration of irinotecan to the patient; administration of the virus; and subsequent co-administration of virus and irinotecan.

Methods are also described herein to maintain or control numbers of a modified vaccinia virus delivered to a patient for treatment of cancer such that the patient suffers minimal side effects or systemic toxicity associated with the virus comprising: initial administration of irinotecan to the patient; administration of the virus; and subsequent administration of irinotecan alone or in combination with one or more antiviral agents.

Also described herein are methods to effectively clear a modified vaccinia virus administered to a patient for treatment of a disease, comprising: administration of irinotecan in an amount effective to clear the virus in the patient, such that symptoms associated with the virus are reduced or eliminated in the patient.

In addition, methods to maintain or control numbers of a modified vaccinia virus delivered to a patient for treatment of cancer are contemplated such that the patient suffers minimal side effects or systemic toxicity associated with the virus, the methods comprising co-administration of the virus with a regimen of doxorubicin. In some embodiments, the co-administration of virus and doxorubicin allows the sustained release of virally-expressed antigens and doxorubicin in a patient. In some embodiments, the co-administration of virus and doxorubicin allows the sustained delivery of virally-expressed antigens and doxorubicin in a patient.

Methods to maintain or control numbers of a modified vaccinia virus delivered to a patient for treatment of cancer such that the patient suffers minimal side effects or systemic toxicity associated with the virus are described, comprising: initial administration of doxorubicin to the patient; administration of the virus; and subsequent co-administration of virus and doxorubicin.

Methods are also described herein to maintain or control numbers of a modified vaccinia virus delivered to a patient for treatment of cancer such that the patient suffers minimal side effects or systemic toxicity associated with the virus comprising: initial administration of doxorubicin to the patient; administration of the virus; and subsequent administration of doxorubicin alone or in combination with one or more antiviral agents.

Also described herein are methods to effectively clear a modified vaccinia virus administered to a patient for treatment of a disease, comprising: administration of doxorubicin in an amount effective to clear the virus in the patient, such that symptoms associated with the virus are reduced or eliminated in the patient.

Furthermore, described herein are methods to maintain or control numbers of a modified vaccinia virus delivered to a patient for treatment of cancer such that the patient suffers minimal side effects or systemic toxicity associated with the virus comprising co-administration of the virus with a regimen of cisplatin. In some embodiments, the co-administration of virus and cisplatin allows the sustained release of virally-expressed antigens and cisplatin in a patient. In some embodiments, the co-administration of virus and cisplatin allows the sustained delivery of virally-expressed antigens and cisplatin in a patient.

Methods to maintain or control numbers of a modified vaccinia virus delivered to a patient for treatment of cancer such that the patient suffers minimal side effects or systemic toxicity associated with the virus comprising: initial administration of cisplatin to the patient; administration of the virus; and subsequent co-administration of virus and cisplatin.

Methods are described herein to maintain or control numbers of a modified vaccinia virus delivered to a patient for treatment of cancer such that the patient suffers minimal side effects or systemic toxicity associated with the virus comprising: initial administration of cisplatin to the patient; administration of the virus; and subsequent administration of cisplatin alone or in combination with one or more antiviral agents.

Also described herein are methods to effectively clear a modified vaccinia virus administered to a patient for treatment of a disease, comprising: administration of cisplatin in an amount effective to clear the virus in the patient, such that symptoms associated with the virus are reduced or eliminated in the patient.

The viruses for use in the methods described can be any virus that is employed for therapy. In particular methods the virus is one that is administered for the treatment of a tumor or a metastasis. Exemplary viruses include, but are not limited to, is a poxvirus, adenovirus, adeno-associated virus, herpes simplex virus, Newcastle disease virus, vesicular stomatitis virus, mumps virus, influenza virus, measles virus, reovirus, human immunodeficiency virus (HIV), hanta virus, myxoma virus, cytomegalovirus (CMV), lentivirus or Sindbis virus. In particular examples, the virus is a vaccinia virus, such as a Lister strain vaccinia virus (*e*.*g*., an LIVP virus). Exemplary LIVP viruses includes but are not limited to GLV-1h68, GLV-1h70, GLV-1h71, GLV-1h72, GLV-1h73, GLV-1h74, GLV-1h81, GLV-1h82, GLV-1h83, GLV-1h84, GLV-1h85, GLV-1h86, GLV-1h90, GLV-1h91, GLV-1h92, GLV-1h96, GLV-1h97, GLV-1h98, GLV-1h99, GLV-1h100, GLV-1h101, GLV-1h104, GLV-1h105, GLV-1h106, GLV-1h107, GLV-1h108, GLV-1h109, GLV-1h139, GLV-1h146, GLV-1h150 GLV-1h151, GLV-1h152 or GLV-1h153.

Also described herein are combinations, including a composition containing a therapeutic virus, wherein the virus is effective for treatment of cancer and a composition containing a chemotherapeutic agent in an amount effective for clearing the virus from a subject.

Described herein are containing a combination of a therapeutic virus and a chemotherapeutic agent in an amount effective for clearing the virus from a subject, and optionally, instructions for administration of the combination.

Described herein is a chemotherapeutic agent for use in ameliorating an adverse side effect associated with viral treatment of cancer, where the chemotherapeutic agent is selected from among gemcitabine, irinotecan, doxorubicin and cisplatin. Also described herein are uses of a chemotherapeutic agent in the preparation of a medicament for the treating an adverse side effect associated with viral treatment of cancer, wherein the chemotherapeutic agent is selected from among gemcitabine, irinotecan, doxorubicin and cisplatin.

### DETAILED DESCRIPTION

### Outline

**A. Definitions**
**B. Methods for Treatment and Diagnosis**
   **1. Administration**
      **a. Virus Administration and Dosages**
      **b. Chemotherapeutic Agent Administration Dosage**
         **i. Gemcitabine Dosage Regimens**
         **ii. Irinotecan Dosage Regimens**
         **iii. Doxorubicin Dosage Regimens**
         **iv. Cisplatin Dosage Regimens**
   **2. Number of administrations**
   **3. Co-administrations**
   **4. State of the Subject**
**C. Viruses and Chemotherapeutic Agents for Treatment and Diagnosis**
   **1. Viruses, vectors**
   **2. Modifications of viruses**
   **3. Exemplary viruses**
      **a. Poxviruses**
         **i. Vaccinia viruses**
         **ii. Modification of Vaccinia Viruses**
         **iii. Exemplary Modified Vaccinia** Viruses
      **b. Other cytoplasmic viruses**
      **c. Adenovirus, Herpes, Retroviruses**
   **4. Chemotherapeutic agents for virus inhibition**
D. **Monitoring**
   **1. Monitoring viral gene expression**
   **2. Monitoring tumor size**
   **3. Monitoring antibody titer**
   **4. Monitoring general health diagnostics**
   **5. Monitoring coordinated with treatment**
**E. Pharmaceutical compositions, combinations and kits**
**F. Examples**

### A. DEFINITIONS

Unless defined otherwise, all technical and scientific terms used herein have the same meaning as is commonly understood by one of skill in the art to which the invention(s) belong.

In the event that there are pluralities of definitions for terms herein, those in this section prevail. Where reference is made to a URL or other such identifier or address, it is understood that such identifiers can change and particular information on the internet can come and go, but equivalent information is known and can be readily accessed, such as by searching the internet and/or appropriate databases. Reference thereto evidences the availability and public dissemination of such information.

As used herein, "virus" refers to any of a large group of entities referred to as viruses. Viruses typically contain a protein coat surrounding an RNA or DNA core of genetic material, and are capable of growth and multiplication only in living cells. Viruses for use in the methods provided herein include, but are not limited, to a poxvirus, including a vaccinia virus. Other exemplary viruses include, but are not limited to, adenovirus, adeno-associated virus, herpes simplex virus, Newcastle disease virus, vesicular stomatitis virus, mumps virus, influenza virus, measles virus, reovirus, human immunodeficiency virus (HIV), hanta virus, myxoma virus, cytomegalovirus (CMV), lentivirus, Sindbis virus, and any plant or insect virus.

As used herein, the term "viral vector" is used according to its art-recognized meaning. It refers to a nucleic acid vector construct that includes at least one element of viral origin and can be packaged into a viral vector particle. The viral vector particles can be used for the purpose of transferring DNA, RNA or other nucleic acids into cells either *in vitro* or *in vivo*. Viral vectors include, but are not limited to, retroviral vectors, vaccinia vectors, lentiviral vectors, herpes virus vectors (e.g., HSV), baculoviral vectors, cytomegalovirus (CMV) vectors, papillomavirus vectors, simian virus (SV40) vectors, semliki forest virus vectors, phage vectors, adenoviral vectors, and adeno-associated viral (AAV) vectors.

As used herein, the term "modified" with reference to a gene refers to a deleted gene, a gene encoding a gene product having one or more truncations, mutations, insertions or deletions, or a gene that is inserted (into the chromosome or on a plasmid, phagemid, cosmid, and phage) encoding a gene product, typically accompanied by at least a change in function of the modified gene product or virus.

As used herein, the term "modified virus" refers to a virus that is altered with respect to a parental strain of the virus. Typically modified viruses have one or more truncations, mutations, insertions or deletions in the genome of virus. A modified virus can have one or more endogenous viral genes modified and/or one or more intergenic regions modified. Exemplary modified viruses can have one or more heterologous nucleic acid sequences inserted into the genome of the virus. Modified viruses can contain one more heterologous nucleic acid sequences in the form of a gene expression cassette for the expression of a heterologous gene. As used herein, modification of a heterologous nucleic acid molecule with respect to a virus containing a heterologous nucleic acid molecule refers to any alteration of the heterologous nucleic acid molecule including truncations, mutations, insertions, or deletions of the nucleic acid molecule. Modification of a heterologous nucleic acid molecule can also include alteration of the viral genome, which can be, for example, a deletion of all or a potion heterologous nucleic from the viral genome or insertion of an additional heterologous nucleic acid molecule into the viral genome.

As used herein, the term "therapeutic virus" refers to a virus that is administered for the treatment of a disease or disorder. A therapeutic virus is typically a modified virus. Such modifications include one or more insertions, deletions, or mutations in the genome of the virus. Therapeutic viruses typically possess modifications in one or more endogenous viral genes or one or more intergenic regions, which attenuate the toxicity of the virus, and can optionally express a heterologous therapeutic gene product and/or detectable protein. Therapeutic viruses can contain heterologous nucleic acid molecules, including one or more gene expression cassettes for the expression of the therapeutic gene product and/or detectable protein. Therapeutic viruses can be replication competent viruses (e.g., oncolytic viruses) including conditional replicating viruses, or replication-defective viruses. As used herein, the term, "therapeutic gene product" refers to any heterologous protein expressed by the therapeutic virus that ameliorates the symptoms of a disease or disorder or ameliorates the disease or disorder.

As used herein, attenuation of a virus means to a reduction or elimination of deleterious or toxic effects to a host upon administration of the virus compared to an un-attenuated virus. As used herein, a virus with low toxicity means that upon administration a virus does not accumulate in organs and tissues in the host to an extent that results in damage or harm to organs, or that impacts survival of the host to a greater extent than the disease being treated does. For the purposes herein, attenuation of toxicity is used interchangeably with attenuation of virulence and attenuation of pathogenicity.

As used herein, the term "viral load" is the amount of virus present in the blood of a patient. Viral load is also referred to as viral titer or viremia. Viral load can be measured in variety of standard ways, including immunochemistry methods or by plaque assay.

As used herein, the term "toxicity" with reference to a virus refers to the ability of the virus to cause harm to the subject to which the virus has been administered.

As used herein virulence and pathogenicity with reference to a virus refers to the ability of the virus to cause disease or harm in the subject to which the virus has been administered. Hence, for the purposes herein the terms toxicity, virulence, and pathogenicity with reference to a virus are used interchangeably.

As used herein, a delivery vehicle for administration refers to a lipid-based or other polymer-based composition, such as liposome, micelle, or reverse micelle, which associates with an agent, such as a virus provided herein, for delivery into a host animal.

As used herein, a disease or disorder refers to a pathological condition in an organism resulting from, for example, infection or genetic defect, and characterized by identifiable symptoms.

As used herein, treatment means any manner in which the symptoms of a condition, disorder or disease are ameliorated or otherwise beneficially altered. Treatment also encompasses any pharmaceutical use of the viruses described and provided herein.

As used herein, amelioration or alleviation of symptoms associated with a disease refers to any lessening, whether permanent or temporary, lasting or transient of symptoms that can be attributed to or associated with a disease. Similarly, amelioration or alleviation of symptoms associated with administration of a virus refers to any lessening, whether permanent or temporary, lasting or transient of symptoms that can be attributed to or associated with an administration of the virus for treatment of a disease.

As used herein, an effective amount of a virus or compound for treating a particular disease is an amount that is sufficient to ameliorate, or in some manner reduce the symptoms associated with the disease. Such an amount can be administered as a single dosage or can be administered according to a regimen, whereby it is effective. The amount can cure the disease but, typically, is administered in order to ameliorate the symptoms of the disease. Repeated administration can be required to achieve the desired amelioration of symptoms.

As used herein, an effective amount of a therapeutic agent for control of viral unit numbers or viral titer in a patient is an amount that is sufficient to prevent a virus introduced to a patient for treatment of a disease from overwhelming the patient's immune system such that the patient suffers adverse side effects due to virus toxicity or pathogenicity. Such side effects can include, but are not limited to fever, abdominal pain, aches or pains in muscles, cough, diarrhea, or general feeling of discomfort or illness that are associated with virus toxicity and are related to the subject's immune and inflammatory responses to the virus. Side effects or symptoms can also include escalation of symptoms due to a systemic inflammatory response to the virus, such as, but not limited to, jaundice, blood-clotting disorders and multiple-organ system failure. Such an amount can be administered as a single dosage or can be administered according to a regimen, whereby it is effective. The amount can prevent the appearance of side effects but, typically, is administered in order to ameliorate the symptoms of the side effects associated with the virus and virus toxicity. Repeated administration can be required to achieve the desired amelioration of symptoms.

As used herein, an *in vivo* method refers to a method performed within the living body of a subject.

As used herein, a subject includes any animal for whom diagnosis, screening, monitoring or treatment is contemplated. Animals include mammals such as primates and domesticated animals. An exemplary primate is human. A patient refers to a subject such as a mammal, primate, human, or livestock subject afflicted with a disease condition or for which a disease condition is to be determined or risk of a disease condition is to be determined.

As used herein, the term "neoplasm" or "neoplasia" refers to abnormal new cell growth, and thus means the same as tumor, which can be benign or malignant. Unlike hyperplasia, neoplastic proliferation persists even in the absence of the original stimulus.

As used herein, neoplastic disease refers to any disorder involving cancer, including tumor development, growth, metastasis and progression.

As used herein, cancer is a term for diseases caused by or characterized by any type of malignant tumor, including metastatic cancers, lymphatic tumors, and blood cancers. Exemplary cancers include, but are not limited to: leukemia, lymphoma, pancreatic cancer, lung cancer, ovarian cancer, breast cancer, cervical cancer, bladder cancer, prostate cancer, glioma tumors, adenocarcinomas, liver cancer and skin cancer. Exemplary cancers in humans include a bladder tumor, breast tumor, prostate tumor, basal cell carcinoma, biliary tract cancer, bladder cancer, bone cancer, brain and CNS cancer (e.g., glioma tumor), cervical cancer, choriocarcinoma, colon and rectum cancer, connective tissue cancer, cancer of the digestive system; endometrial cancer, esophageal cancer; eye cancer; cancer of the head and neck; gastric cancer; intra-epithelial neoplasm; kidney cancer; larynx cancer; leukemia; liver cancer; lung cancer (e.g. small cell and non-small cell); lymphoma including Hodgkin's and Non-Hodgkin's lymphoma; melanoma; myeloma, neuroblastoma, oral cavity cancer (e.g., lip, tongue, mouth, and pharynx); ovarian cancer; pancreatic cancer, retinoblastoma; rhabdomyosarcoma; rectal cancer, renal cancer, cancer of the respiratory system; sarcoma, skin cancer; stomach cancer, testicular cancer, thyroid cancer; uterine cancer, cancer of the urinary system, as well as other carcinomas and sarcomas. Malignant disorders commonly diagnosed in dogs, cats, and other pets include, but are not limited to, lymphosarcoma, osteosarcoma, mammary tumors, mastocytoma, brain tumor, melanoma, adenosquamous carcinoma, carcinoid lung tumor, bronchial gland tumor, bronchiolar adenocarcinoma, fibroma, myxochondroma, pulmonary sarcoma, neurosarcoma, osteoma, papilloma, retinoblastoma, Ewing's sarcoma, Wilm's tumor, Burkitt's lymphoma, microglioma, neuroblastoma, osteoclastoma, oral neoplasia, fibrosarcoma, osteosarcoma and rhabdomyosarcoma, genital squamous cell carcinoma, transmissible venereal tumor, testicular tumor, seminoma, Sertoli cell tumor, hemangiopericytoma, histiocytoma, chloroma (e.g., granulocytic sarcoma), corneal papilloma, corneal squamous cell carcinoma, hemangiosarcoma, pleural mesothelioma, basal cell tumor, thymoma, stomach tumor, adrenal gland carcinoma, oral papillomatosis, hemangioendothelioma and cystadenoma, follicular lymphoma, intestinal lymphosarcoma, fibrosarcoma and pulmonary squamous cell carcinoma. In rodents, such as a ferret, exemplary cancers include insulinoma, lymphoma, sarcoma, neuroma, pancreatic islet cell tumor, gastric MALT lymphoma and gastric adenocarcinoma. Neoplasias affecting agricultural livestock include leukemia, hemangiopericytoma and bovine ocular neoplasia (in cattle); preputial fibrosarcoma, ulcerative squamous cell carcinoma, preputial carcinoma, connective tissue neoplasia and mastocytoma (in horses); hepatocellular carcinoma (in swine); lymphoma and pulmonary adenomatosis (in sheep); pulmonary sarcoma, lymphoma, Rous sarcoma, reticulo-endotheliosis, fibrosarcoma, nephroblastoma, B-cell lymphoma and lymphoid leukosis (in avian species); retinoblastoma, hepatic neoplasia, lymphosarcoma (lymphoblastic lymphoma), plasmacytoid leukemia and swimbladder sarcoma (in fish), caseous lumphadenitis (CLA): chronic, infectious, contagious disease of sheep and goats caused by the bacterium Corynebacterium pseudotuberculosis, and contagious lung tumor of sheep caused by jaagsiekte.

As used herein, the term "malignant," as it applies to tumors, refers to primary tumors that have the capacity of metastasis with loss of growth control and positional control.

As used herein, metastasis refers to a growth of abnormal or neoplastic cells distant from the site primarily involved by the morbid process.

As used herein, proliferative disorders include any disorders involving abnormal proliferation of cells, such as, but not limited to, neoplastic diseases.

As used herein, a method for treating or preventing neoplastic disease means that any of the symptoms, such as the tumor, metastasis thereof, the vascularization of the tumors or other parameters by which the disease is characterized are reduced, ameliorated, prevented, placed in a state of remission, or maintained in a state of remission. It also means that the indications of neoplastic disease and metastasis can be eliminated, reduced or prevented by the treatment. Non-limiting examples of the indications include uncontrolled degradation of the basement membrane and proximal extracellular matrix, migration, division, and organization of the endothelial cells into new functioning capillaries, and the persistence of such functioning capillaries.

As used herein, a prodrug is a compound that, upon *in vivo* administration, is metabolized or otherwise converted to the biologically, pharmaceutically or therapeutically active form of the compound. To produce a prodrug, the pharmaceutically active compound is modified such that the active compound is regenerated by metabolic processes. The prodrug can be designed to alter the metabolic stability or the transport characteristics of a drug, to mask side effects or toxicity, to improve the flavor of a drug or to alter other characteristics or properties of a drug. By virtue of knowledge of pharmacodynamic processes and drug metabolism *in vivo,* those of skill in this art, once a pharmaceutically active compound is known, can design prodrugs of the compound (see, e.g., Nogrady (1985) Medicinal Chemistry A Biochemical Approach, Oxford University Press, New York, pages 388-392).

As used herein, an anti-cancer agent or compound (used interchangeably with "anti-tumor or anti-neoplastic agent") refers to any agents, or compounds, used in anti-cancer treatment. These include any agents, when used alone or in combination with other compounds, that can alleviate, reduce, ameliorate, prevent, or place or maintain in a state of remission of clinical symptoms or diagnostic markers associated with neoplastic disease, tumors and cancer, and can be used in methods, combinations and compositions provided herein. Exemplary anti-cancer agent agents include, but are not limited to, the viruses provided herein used singly or in combination and/or in combination with other anti-cancer agents, such as cytokines, growth factors, hormones, photosensitizing agents, radionuclides, toxins, anti-metabolites, signaling modulators, anti-cancer antibiotics, anti-cancer antibodies, anti-cancer oligopeptides, angiogenesis inhibitors, radiation therapy, hypothermia therapy, hyperthermia therapy, laser therapy, chemotherapeutic compounds, or a combination thereof.

Chemotherapeutic compounds include, but are not limited to platinum; platinum analogs anthracenediones; vinblastine; alkylating agents; alkyl sulfonates; aziridines; ethylenimines and methylamelamines; nitrosureas; antibiotics; anti-metabolites; folic acid analogues; androgens; anti-adrenals; folic acid replenisher; aminolevulinic acid; amsacrine; bestrabucil; bisantrene; edatraxate; defofamine; demecolcine; diaziquone; elfomithine; elliptinium acetate; etoglucid; gallium nitrate; substituted ureas; hydroxyurea; lentinan; lonidamine; mitoguazone; mitoxantrone; mopidamol; nitracrine; pentostatin; phenamet; pirarubicin; podophyllinic acid; 2-ethylhydrazide; procarbazine; anti-cancer polysaccharides; polysaccharide-K; razoxane; sizofiran; spirogermanium; tenuazonic acid; triaziquone; 2, 2',2"-trichlorotriethylamine; urethan; vindesine; dacarbazine; mannomustine; mitobronitol; mitolactol; pipobroman; gacytosine; cytosine arabinoside; cyclophosphamide; thiotepa; taxoids, such as paclitaxel and doxetaxel; chlorambucil; gemcitabine; 6-thioguanine; mercaptopurine; methotrexate; etoposide (VP-16); ifosfamide; mitomycin C; vincristine; vinorelbine; navelbine; novantrone; teniposide; daunomycin; aminopterin; xeloda; ibandronate; CPT11; topoisomerase inhibitor RFS 2000; difluoromethylomithine (DMFO); retinoic acid; esperamicins; capecitabine; methylhydrazine derivatives; and pharmaceutically acceptable salts, acids or derivatives of any of the above. Chemotherapeutic compounds also include, but are not limited to, adriamycin, non-sugar containing chloroethylnitrosoureas, 5-fluorouracil, bleomycin, doxorubicin, taxol, fragyline, Meglamine GLA, valrubicin, carmustine and polifeprosan, MM 1270, BAY 12-9566, RAS farnesyl transferase inhibitor, famesyl transferase inhibitor, MMP, MTA/LY231514, LY264618/Lometrexol, Glamolec, CI-994, TNP-470, Hycamtin/Topotecan, PKC412, Valspodar/PSC833, Novantrone/Mitroxantrone, Metaret/Suramin, Batimastat, E7070, BCH-4556, CS-682, 9-AC, AG3340, AG3433, Incel/VX-710, VX-853, ZD0101, IS1641, ODN 698, TA 2516/Marmistat, BB2516/Marmistat, CDP 845, D2163, PD183805, DX8951f, Lemonal DP 2202, FK 317, Picibanil/OK-432, AD 32/Valrubicin, Metastron/strontium derivative, Temodal/Temozolomide, Evacet/liposomal doxorubicin, Yewtaxan/Placlitaxel, Taxol®/Paclitaxel, Xeload/Capecitabine, Furtulon/Doxifluridine, Cyclopax/oral paclitaxel, Oral Taxoid, SPU-077/Cisplatin, HMR 1275/Flavopiridol, CP-358 (774)EGFR, CP-609 (754)/RAS oncogene inhibitor, BMS-182751/oral platinum, UFT(Tegafur/Uracil), Ergamisol/Levamisole, Eniluracil/776C85/5FU enhancer, Campto/Levamisole, Camptosar/Irinotecan, Tumodex/Ralitrexed, Leustatin/Cladribine, Paxex/Paclitaxel, Doxil/liposomal doxorubicin, Caelyx/liposomal doxorubicin, Fludara/Fludarabine, Pharmarubicin/Epirubicin, DepoCyt, ZD1839, LU 79553/Bis-Naphtalimide, LU 103793/Dolastain, Gemzar/Gemcitabine, ZD 0473/Anormed, YM 116, Iodine seeds, CDK4 and CDK2 inhibitors, PARP inhibitors, D4809/Dexifosamide, Ifes/Mesnex/Ifosfamide, Vumon®/Teniposide, Paraplatin/Carboplatin, Plantinol/cisplatin, Vepeside/Etoposide, ZD 9331, Taxotere/Docetaxel, prodrug of guanine arabinoside, Taxane Analog, nitrosoureas, alkylating agents such as melphelan and cyclophosphamide, Aminoglutethimide, Anastrozole, Asparaginase, Busulfan, Carboplatin, Chlorombucil, Cladribine, Cytarabine HCl, Dactinomycin, Daunorubicin HCl, Denileukin diftitox, Estramustine phosphate sodium, Etoposide (VP 16-213), Exemestane, Floxuridine, Fluorouracil (5-FU®), Flutamide, Hydroxyurea (hydroxycarbamide), Interferon Alfa-2a, Interferon Alfa-2b, Interferon Gamma-1b, Letrozole, Leuprolide acetate (LHRH-releasing factor analogue), Lomustine (CCNU), Mechlorethamine HCl (nitrogen mustard), Megestrol, Mercaptopurine, Mesna, Mitotane (o.p'-DDD), Mitoxantrone HCl, Octreotide, Pegaspargase, Plicamycin, Procarbazine HCl, Streptozocin, Tamoxifen citrate, Thioguanine, Thiotepa, Tretinoin, Vinblastine sulfate, Amsacrine (m-AMSA), Azacitidine, Erythropoietin, Hexamethylmelamine (HMM), Interleukin 2, Mitoguazone (methyl-GAG; methyl glyoxal bis-guanylhydrazone; MGBG), Pentostatin (2'deoxycoformycin), Semustine (methyl-CCNU), Teniposide (VM-26®), Vindesine sulfate, Altretamine, Carmustine, Estramustine, Gemtuzumab ozogamicin, Idarubicin, Isotretinoin, Leuprolide, Melphalan, Testolactone, Uracil mustard, and the like. Also included in this definition are anti-hormonal agents that act to regulate or inhibit hormone action on tumors such as anti-estrogens, adrenocortical suppressants, antiandrogens and pharmaceutically acceptable salts, acids or derivatives of any of the above. Such chemotherapeutic compounds that can be used herein include compounds whose toxicities preclude use of the compound in general systemic chemotherapeutic methods.

As used herein the term assessing or determining is intended to include quantitative and qualitative determination in the sense of obtaining an absolute value for the activity of a product, and also of obtaining an index, ratio, percentage, visual or other value indicative of the level of the activity. Assessment.can be direct or indirect. As used herein, activity refers to the *in vivo* activities of a compound or viruses on physiological responses that result following *in vivo* administration thereof (or of a composition or other mixture). Activity, thus, encompasses resulting therapeutic effects and pharmaceutical activity of such compounds, compositions and mixtures. Activities can be observed in *in vitro* and/or *in vivo* systems designed to test or use such activities.

As used herein, a vaccine refers to a composition which, upon administration to a subject, elicits an immune response in a subject to which it is administered and which protects the immunized subject against subsequent challenge by the immunizing agent or an immunologically cross-reactive agent. A vaccine can be used to enhance the immune response against a pathogen, such as a virus, that expresses the immunological agent and/or has already infected the subject. Protection can be complete or partial (i.e., a reduction in symptoms or infection as compared with an unvaccinated subject). Typically a vaccine is administered to a subject that is a mammal. An immunologically cross-reactive agent can be, for example, the whole protein (e.g., tumor antigen) from which a subunit peptide used as the immunogen is derived. Alternatively, an immunologically cross-reactive agent can be a different protein which is recognized in whole or in part by the antibodies elicited by the immunizing agent. Exemplary vaccines can be modified vaccinia viruses that express an immunologically cross-reactive agent.

An oncolytic virus is a virus that preferentially replicates in, and kills, neoplastic or cancer cells. The virus can be a naturally-occurring virus or an engineered virus. Preferably, the virus is a modified vaccinia virus.

As used herein, the phrase "immunoprivileged cells and tissues" refers to cells and tissues, such as solid tumors and wounded tissues, which are sequestered from the immune system.

As used herein, nanoparticle refers to a microscopic particle whose size is measured in nanometers. Often such particles in nanoscale are used in biomedical applications acting as drug carriers or imaging agents. Nanoparticles can be conjugated to other agents, including, but not limited to detectable/diagnostic agents or therapeutic agents.

As used herein, "a combination" refers to any association between two or among more items. Such combinations can be packaged as kits.

As used herein, a composition refers to any mixture. It can be a solution, a suspension, an emulsion, liquid, powder, a paste, aqueous, non-aqueous or any combination of such ingredients.

As used herein, fluid refers to any composition that can flow. Fluids thus encompass compositions that are in the form of semi-solids, pastes, solutions, aqueous mixtures, gels, lotions, creams and other such compositions.

As used herein, a kit is a packaged combination, optionally, including instructions for use of the combination and/or other reactions and components for such use.

### B. METHODS FOR TREATMENT AND DIAGNOSIS

Described herein are uses of and methods of administering chemotherapeutic agents in combination with modified vaccinia viruses, and/or other viruses or therapeutic viral vectors, to a subject for treatment of diseases, such as cancer. In particular, the methods involve administration of chemotherapeutic agents in combination with viruses.

In particular, methods for ameliorating or reducing toxicity and/or side-effects associated with viral therapy of diseases such as cancer are discussed. The methods employ intermediate doses of chemotherapeutic agents in combination with the viruses or viral vectors. Regimens that exploit this benefit are discussed. Administration of intermediate dosages of chemotherapeutic agents at appropriate times or intervals during viral therapy can promote the arrest and shrinkage of neoplastic cell masses and solid or nonsolid tumors in patients, while reducing or preventing the patient from experiencing undesirable side effects or systemic toxicity that can result treatment with viruses or viral vectors. In certain embodiments, higher doses of chemotherapeutic agents can be administered to a patient who has received an injection of virus or viral vector for disease treatment.

For example, as described herein, low doses of chemotherapeutic agents (for example, approximately 10-50 mg/kg of gemcitabine), delivered to a subject treated with modified vaccinia viruses or other viral vectors, have been effective in arresting and/or inhibiting tumor cell growth. Higher doses of the chemotherapeutic agents (for example, approximately 100-200 mg/kg of gemcitabine) delivered in combination with administration of the virus or viral vector do not provide a more potent effect. The higher dose instead have a negative effect on viral titer. As described herein, the chemotherapeutic agents can interfere with the ability of the virus or viral vector to replicate. Without wishing to be bound by theory, it is believed that the mechanisms by which the agents control or shrink cancerous cell growths (*e*.*g*. inhibition of DNA transcription and replication) also contribute to interference of viral replication. Consequently, chemotherapeutic agents can be co-administered with the virus to control viral replication, thereby maintaining viral titers at a level that prevents a patient from experiencing side effects or symptoms associated with viral toxicity. Chemotherapeutic agents also can be administered to a patient who has received a dose of modified vaccinia virus for treatment of a disease. For example, a chemotherapeutic agent can be administered to a patient who experiences adverse effects or systemic toxicity following administration of the virus. In such case, the chemotherapeutic agent is delivered to the patient in order to elicit rapid clearance of the virus from the subject's body.

Exemplary methods described herein include methods for treatment of a disease where a modified vaccinia virus is administered to a subject in combination with at least one dose of a chemotherapeutic agent, whereby the administration of agent with virus maintains or controls viral unit numbers, or viral titers, within the subject as compared to administrate of the virus without the agent. In such methods, the subject is exposed to minimal physiological side effects associated with administration of the virus. In some embodiments, the virus is first administered to a patient, followed by regularly scheduled doses with a chemotherapeutic agent over a period of time. In further embodiments, the virus is first administered to a patient, followed by sustained release of a chemotherapeutic agent from a release vehicle placed within the patient. In some embodiments, the virus is administered concurrently with a chemotherapeutic agent in regularly scheduled doses to the patient over a period of time. In further embodiments, the virus is released concurrently with a chemotherapeutic agent from a sustained release vehicle placed within the patient.

Exemplary methods described herein include methods to prevent the appearance of symptoms or side effects associated with administration of a modified vaccinia virus for treatment of a disease, by delivering a dose of a chemotherapeutic agent prior to administration of the virus to a subject. In some embodiments, a chemotherapeutic agent is first administered to a patient as a single dose before the virus is introduced. In other embodiments, a chemotherapeutic agent is administered to a patient as regularly scheduled doses before the virus is introduced. Subsequent administration of the virus can be as a single dose to the patient. The virus also can be introduced to the patient concurrently with the chemotherapeutic agent in one or more further regularly scheduled doses over a period of time.

Exemplary methods described herein include methods to improve symptoms or side effects associated with delivery of a modified vaccinia virus to a subject for treatment of disease, by administration of a dose of chemotherapeutic agent to the subject for clearing the virus from the subject's body. In methods described, the virus is administered in combination with a chemotherapeutic agent for controlling viral titer. The chemotherapeutic agent acts to control viral replication in the subject, which can minimize any immune response raised against the virus and prevent the subject from experiencing toxic side effects. For example, subjects who are immune-compromised can suffer side effects from uncontrolled replication of viruses, including modified vaccinia viruses delivered for treatment of a disease. Exemplary side effects or symptoms include, but are not limited to fever, abdominal pain, aches or pains in muscles, cough, diarrhea, or general feeling of discomfort or illness that are associated with virus toxicity and are related to the subject's immune and inflammatory responses to the virus. Exemplary side effects or symptoms can also include escalation of symptoms due to a systemic inflammatory response to the virus, such as, but not limited to, jaundice, blood-clotting disorders, generalized vaccinia, eczema vaccinatum, uncontrolled progressive vaccinia, encephalitis, multiple-organ system failure, and the like. Methods are also contemplated wherein the subject, when suffering from adverse side effects associated with modified vaccinia virus administered for treatment of a disease, is provided a chemotherapeutic agent to clear the virus from the subject's body, thereby alleviating symptoms or side effects associated with viral toxicity.

In addition, methods are herein described for the optimization of dosage administration of a chemotherapeutic agent to a patient, wherein the agent is administered in combination with a modified vaccinia virus for treatment of a disease. The optimization of dosage administration is contemplated for targeting DNA replication in the modified vaccinia virus, such that viral replication is carefully controlled and side effects associated with the virus are reduced or minimized. The optimal dose of the chemotherapeutic agent is sufficiently high to lower the virus-associated side effects, such as for example, pock formation, weight loss, fever, abdominal pain, aches or pains in muscles, cough, diarrhea, and feeling of discomfort or illness. In addition, the optimal dose is within a range such that it does not adversely interfere the oncolytic therapeutic effects of the virus.

Optimized doses of a chemotherapeutic agent are contemplated wherein the virus is first administered to a patient, followed by regularly scheduled doses with the agent over a period of time. Methods are also described wherein an optimized dose of chemotherapeutic agent wherein the virus is administered concurrently with the agent in regularly scheduled doses over a period of time. Methods are also described wherein an optimized dose of chemotherapeutic agent wherein the agent is first administered to a patient as regularly scheduled doses before the virus is introduced and the virus is subsequently introduced as a single dose or administered concurrently with agent in further regularly scheduled doses to the patient over a period of time.

Further, methods are herein described wherein an optimized chemotherapeutic agent dosage is administered to a patient, wherein the agent is administered subsequent to delivery of a modified vaccinia virus to the patient for treatment of a disease, and wherein the agent is provided to clear the virus from the patient's body to reduce physiological toxicity effects associated with the virus. The optimized chemotherapeutic agent dosage is provided such that the subject, when suffering from adverse side effects due to administration of a modified vaccinia virus for treatment of a disease, is administered a chemotherapeutic agent to clear the virus from the subject's body, alleviating symptoms or side effects associated with viral toxicity.

In some embodiments, the methods described can be used for treatment of a neoplastic disease. In some embodiments, the methods described herein can be used for the treatment of cancer. In some embodiments, the methods described can be used to inhibit or slow tumor cell growth. In some embodiments, the methods described herein can be used to decrease or shrink tumor cell volume. In some examples, the tumor cells are lung, ovarian, breast or pancreatic tumor cells.

Exemplary modified vaccinia viruses that can be used in the methods or uses described include, but are not limited to, the recombinant vaccinia LIVP strain GLV-1h68 (described in U.S. Pat. Pub. No. 2005-0031643, incorporated herein by reference in its entirety) and related strains GLV-1h22, GLV-1i69, GLV-1h70, GLV-1h71, GLV-1h72, GLV-1h73, GLV-1h74, GLV-1h81, GLV-1h82, GLV-1h83, GLV-1h84, GLV-1h85, GLV-1h86, GLV-1h90, GLV-1h91, GLV-1h92, GLV-1h96, GLV-1h97, GLV-1h98, GLV-1h99, GLV-1h100, GLV-1h101, GLV-1h104, GLV-1h105, GLV-1h106, GLV-1h107, GLV-1h108 and GLV-1h109, as described elsewhere herein.

Exemplary chemotherapeutic agents that can be used in the methods or uses described include, but are not limited to cisplatin, gemcitabine, doxorubicin, irinotecan and 5'-fluorouracil.

As shown previously, solid tumors can be treated with therapeutic viruses, such as vaccinia viruses, resulting in an enormous tumor-specific virus replication, which can be used to produce large amounts of tumor protein antigen and viral protein in the tumors (U.S. Patent Publication No. 2005/0031643). Vaccinia virus administration to mice resulted in lysis of the infected tumor cells and a resultant release of tumor-cell-specific antigens. Continuous leakage of these antigens into the body led to a very high level of antibody titer (in approximately 7-14 days) against tumor proteins, viral proteins, and the virus encoded engineered proteins in the mice. The newly synthesized anti-tumor antibodies and the enhanced macrophage, neutrophils count were continuously delivered via the vasculature to the tumor and thereby provided for the recruitment of an activated immune system against the tumor. The activated immune system then eliminated the foreign compounds of the tumor including the viral particles. This interconnected release of foreign antigens boosted antibody production and continuous response of the antibodies against the tumor proteins to function like an autoimmunizing vaccination system initiated by vaccinia viral infection and replication, followed by cell lysis, protein leakage and enhanced antibody production. Thus, the therapeutic viruses described herein can be administered, alone or in combination with a chemotherapeutic agent, in a complete process that can be applied to all tumor systems with immunoprivileged tumor sites as site of privileged viral growth, which can lead to tumor elimination by the host's own immune system.

In some embodiments, the therapeutic virus can act as an oncolytic virus. For example, the therapeutic virus can utilize multiple mechanisms of action to directly kill tumor and cancer cells, such as, for example, by cell lysis, cell apoptosis, anti-angiogenesis and cell necrosis. The virus can infect the tumor cell and begin to replicate. The virus continues to replicate until the membrane of the host cell lyses, or bursts, due to the inability of the tumor cell to contain the virus. The tumor cell is destroyed, and the newly created viruses can spread to neighboring cancer cells to continue the cycle. Preferably, the virus replicates only in cancer cells and leaves normal tissue unharmed.

Described herein are therapeutic methods, including methods of treating and/or preventing disease or disorders associated with immunoprivileged cells or tissue, including cancerous cells, tumors and metastases. Such sites, diseases and disorders include sites of cell proliferation, proliferative conditions, neoplasms, tumors, neoplastic disease, wounds and inflammation. The therapeutic methods described herein include, but are not limited to, administering a therapeutic virus described herein to a subject containing a tumor and/or metastases, wherein the virus is administered alone or in combination with a chemotherapeutic agent. Viruses described herein include viruses that have been modified as described herein and elsewhere. The administered viruses can posses one or more characteristics including, but not limited to, attenuated pathogenicity, low toxicity, preferential accumulation in tumor, ability to activate an immune response against tumor cells, immunogenicity, replication competence, ability to express exogenous genes, and ability to elicit antibody production against an expressed gene product.

In the methods described, the chemotherapeutic agent is administered to control viral replication or viral titer of the administered therapeutic virus in a subject such that the subject to which the chemotherapeutic agent and therapeutic virus is administered experiences minimal side effects associated with the therapeutic virus. In some examples, the chemotherapeutic agent is administered to a subject to which a therapeutic virus has been administered whereby the chemotherapeutic agent decreases the viral titer in the subject. In other examples, the chemotherapeutic agent is administered to a subject to which a therapeutic virus has been administered whereby the chemotherapeutic agent eliminates or clears the virus from the subject. In some embodiments, the therapeutic viruses provided herein can be administered in combination with a chemotherapeutic agent to a subject such that the combination prevents the therapeutic virus from causing adverse side effects, systemic toxicity or disease in the subject.

The chemotherapeutic agent can be administered simultaneously in combination with the therapeutic virus or can be administered sequentially, prior to or following treatment with the therapeutic virus.

The chemotherapeutic agent can be administered in combination with or sequentially with the therapeutic virus to control viral replication or viral titer of the therapeutic virus in a subject in addition to treating a disease or disorder in the subject. In such methods, the chemotherapeutic agent contributes to the treatment of the disease or disorder as well as the control of viral replication or viral titer. The chemotherapeutic agent can also be administered separately to a patient for the treatment of a disease or disorder in the subject.

In some embodiments, the therapeutic viruses can accumulate in tumors or metastases. In some embodiments, the therapeutic methods provided herein inhibit tumor growth in a subject, where the methods include administering to a subject a therapeutic virus, alone or in combination with a chemotherapeutic agent, where the therapeutic virus can accumulate in a tumor and/or metastasis and can cause or enhance an anti-tumor response. In some embodiments, the administration of a therapeutic virus as described herein, alone or in combination with a chemotherapeutic agent, results in a slowing or inhibition of tumor growth. In other embodiments, the administration of a therapeutic virus in combination with a chemotherapeutic agent as described herein results in a decrease in tumor volume or elimination of the tumor from the subject. The therapeutic methods described herein, however, do not require the administered combination of virus and chemotherapeutic agent to directly kill tumor cells or decrease the tumor size. Instead, the therapeutic methods described herein include administering to a subject a therapeutic virus and chemotherapeutic agent where the therapeutic virus and/or the combination of the therapeutic virus with chemotherapeutic agent can cause or enhance an anti-tumor response in the subject. The anti-tumor response induced as a result of tumor or metastases-accumulated viruses can result in inhibition of tumor growth, a decrease in tumor volume and/or elimination of the tumor.

In some embodiments, the therapeutic viruses, alone or combination with a chemotherapeutic agent, can elicit an anti-tumor response in the subject, where typically the viral-mediated anti-tumor response can develop, for example, over several days, a week or more, 10 days or more, two weeks or more, or a month or more. In some exemplary methods, the therapeutic virus can be present in the tumor, and can cause an anti-tumor immune response without the virus itself causing enough tumor cell death to prevent tumor growth. In some embodiments, the tumor is a monotherapeutic tumor or monotherapeutic cancer, where the tumor or cancer does not decrease, or does not decrease significantly or satisfactorily in volume when treated with the therapeutic virus or a therapeutic agent alone.

In some embodiments, the anti-tumor response is a result of oncolysis of tumor cells by the administered virus and/or leakage of antigens from the lysed tumor cell, whereby the subject develops an immune response against the antigen. In other embodiments, the anti-tumor response is a result of leakage of antigens from the viral-infected tumor cell, whereby the subject develops an immune response against the antigen.

The methods described herein include eliciting or enhancing antibody production against a selected antigen or a selected antigen type in a subject. For example, such methods include administering to a subject a therapeutic virus, alone or in combination with a chemotherapeutic agent, that can accumulate in a tumor and/or metastasis, and can cause release of a selected antigen or selected antigen type from the tumor, resulting in antibody production against the selected antigen or selected antigen type. Any of a variety of antigens can be targeted in the methods described herein, including a selected antigen such as an exogenous gene product expressed by the virus, or a selected antigen type such as one or more tumor antigens released from the tumor as a result of viral infection of the tumor (*e*.*g*., by lysis, apoptosis, secretion or other mechanism of causing antigen release from the tumor).

In some embodiments, it can be desirable to maintain release of the selected antigen or selected antigen type over a series of days, for example, at least a week, at least ten days, at least two weeks or at least a month. Described herein are methods for providing a sustained antigen release within a subject, where the methods include administering to a subject a therapeutic virus, alone or in combination with a chemotherapeutic agent, whereby the virus accumulates in a tumor and/or metastasis, and can cause sustained release of an antigen, resulting in antibody production against the antigen. The sustained release of antigen can cause an immune response by the viral-infected host, in which the host can develop antibodies against the antigen and/or the host generates an immune response against cells expressing the antigen, including tumor cells expressing the antigen. In such methods, the viral-infect host can develop an immune response against tumor cells. Thus, the sustained release of antigen can result in immunization against tumor cells. In some embodiments, the anti-tumor immune response induced by the viral-mediated sustained release of antigen can result in complete removal or killing of all tumor cells. In some embodiments, the therapeutic virus is administered in combination with a chemotherapeutic agent, whereby an anti-tumor immune response is induced by the viral-mediated sustained release of antigen and where the chemotherapeutic agent is administered over a period of time (*e*.*g*., repeated administration or sustained release) to control viral replication or viral titer, such that the virus does not cause adverse side effects, systemic toxicity or disease in the subject.

Such methods of antigen production or tumor and/or metastasis treatment can include administration of a therapeutic virus provided herein, alone or in combination with a chemotherapeutic agent, for therapy, such as for gene therapy, for cancer gene therapy, for oncolytic virotherapy, or for vaccine therapy. Such a virus can be used to stimulate humoral and/or cellular immune response, induce strong cytotoxic T lymphocytes responses in subjects who can benefit from such responses. For example, the virus can provide prophylactic and therapeutic effects against a tumor infected by the virus or other infectious diseases, by rejection of cells from tumors or lesions using viruses that express immunoreactive antigens (Earl et al., Science 234: 728-831 (1986); Lathe et al., Nature (London) 32: 878-880 (1987)), cellular tumor-associated antigens (Bernards et al., Proc. Natl. Acad. Sci. USA 84: 6854-6858 (1987); Estin et al., Proc. Natl. Acad Sci. USA 85: 1052-1056 (1988); Kantor et al., J. Natl. Cancer Inst. 84: 1084-1091 (1992); Roth et al., Proc. Natl. Acad. Sci. USA 93: 4781-4786 (1996)) and/or cytokines (e.g., IL-2, IL-12), costimulatory molecules (B7-1, B7-2) (Rao et al., J. Immunol. 156: 3357-3365 (1996); Chamberlain et al., Cancer Res. 56: 2832-2836 (1996); Oertli et al., J. Gen. Virol. 77: 3121-3125 (1996); Qin and Chatterjee, Human Gene Ther. 7: 1853-1860 (1996); McAneny et al., Ann. Surg. Oncol.3: 495-500 (1996)), or other therapeutic proteins.

In some embodiments, the therapeutic methods described herein inhibit growth or formation of a metastasis in a subject, where the methods include administering to a subject a therapeutic virus provided herein, alone or in combination with a chemotherapeutic agent, where the virus can accumulate in a tumor and/or metastasis and can cause or enhance an anti-tumor immune response. The anti-tumor immune response induced as a result of tumor or metastasis-accumulated viruses can result in inhibition of metastasis growth or formation. In some embodiments, the anti-tumor response is a result of oncolysis of tumor cells by the administered virus.

In other embodiments, the therapeutic methods provided herein decrease the size of a tumor and/or metastasis in a subject, where the methods include administering to a subject a therapeutic virus provided herein, alone or in combination with a chemotherapeutic agent, where the virus can accumulate in a tumor and/or metastasis and can cause or enhance an anti-tumor immune response. The anti-tumor immune response induced as a result of tumor or metastasis-accumulated viruses can result in a decrease in the size of the tumor and/or metastasis.

In some embodiments, the therapeutic methods described herein eliminate a tumor and/or metastasis from a subject, where the methods include administering to a subject a therapeutic virus described herein, alone or in combination with a chemotherapeutic agent, where the virus can accumulate in a tumor and/or metastasis and can cause or enhance an anti-tumor immune response. The anti-tumor immune response induced as a result of tumor or metastasis-accumulated viruses can result in elimination of the tumor and/or metastasis from the subject.

Furthermore, described herein are methods of reducing or inhibiting tumor growth, inhibiting metastasis growth and/or formation, decreasing the size of a tumor or metastasis, eliminating a tumor or metastasis, or other tumor therapeutic methods, where the method includes administering to a subject a therapeutic virus described herein, alone or in combination with a chemotherapeutic agent, where the virus accumulates in at least one tumor or metastasis and causes or enhances an anti-tumor immune response in the subject, and the immune response also is mounted against a tumor and/or metastasis in which the virus cell did not accumulate.

In another embodiment, methods are described for inhibiting or preventing recurrence of a neoplastic disease or inhibiting or preventing new tumor growth, where the methods include administering to a subject a therapeutic virus described herein, alone or in combination with a chemotherapeutic agent, where the virus can accumulate in a tumor and/or metastasis and can cause or enhance an anti-tumor immune response, which can inhibit or prevent recurrence of a neoplastic disease or inhibit or prevent new tumor growth.

The therapeutic methods described herein for the treatment of tumor or neoplastic disease, such as, for example, methods of reducing or inhibiting tumor growth, inhibiting metastasis growth and/or formation, decreasing the size of a tumor or metastasis, eliminating a tumor or metastasis, or other tumor therapeutic methods, also can include administering to a subject a therapeutic virus described herein, alone or in combination with a chemotherapeutic agent, where the virus can cause tumor cell lysis or tumor cell death. In such methods, the virus can cause tumor cell lysis or tumor cell death and also can cause or enhance an anti-tumor immune response in the subject. Viruses, such as the therapeutic viruses provided herein, can cause cell lysis or tumor cell death as a result of expression of an endogenous gene or as a result of an exogenous gene. Endogenous or exogenous genes can cause tumor cell lysis or inhibit cell growth as a result of direct or indirect actions, as is known in the art, including lytic channel formation or activation of an apoptotic pathway. Gene products, such as exogenous gene products can function to activate a prodrug to an active, cytotoxic form, resulting in cell death where such genes are expressed.

In one embodiment, the tumor treated is a cancer such as pancreatic cancer, non-small cell lung cancer, ovarian cancer, breast cancer, prostate cancer, multiple myeloma, or leukemia, although the cancer is not limited in this respect, and other metastatic diseases can be treated by the combinations described herein. For example, the tumor treated can be a solid tumor, such as of the lung and bronchus, breast, colon and rectum, kidney, stomach, esophagus, liver and intrahepatic bile duct, urinary bladder, brain and other nervous system, head and neck, oral cavity and pharynx, cervix, uterine corpus, thyroid, ovary, testes, prostate, malignant melanoma, cholangiocarcinoma, thymoma, non-melanoma skin cancers, as well as hematologic tumors and/or malignancies, such as childhood leukemia and lymphomas, multiple myeloma, Hodgkin's disease, lymphomas of lymphocytic and cutaneous origin, acute and chronic leukemia such as acute lymphoblastic, acute myelocytic or chronic myelocytic leukemia, plasma cell neoplasm, lymphoid neoplasm and cancers associated with AIDS. Exemplary tumors include, for example, pancreatic tumors, ovarian tumors, lung tumors, colon tumors, prostate tumors, cervical tumors and breast tumors. In one embodiment, the tumor is a carcinoma such as, for example, an ovarian tumor or a pancreatic tumor.

### 1. Administration

In performing the therapeutic methods described herein, a therapeutic virus can be administered, alone or in combination with a chemotherapeutic agent, to a subject. Exemplary subjects to whom a therapeutic virus can be administered include a subject having a tumor, metastases, neoplastic cells, wounded or inflamed tissue or a subject to be immunized. An administered therapeutic virus can be a virus described herein or any other virus generated using the methods described herein. In some embodiments, the therapeutic virus administered is a virus that exhibits a characteristic, such as attenuated pathogenicity, low toxicity, preferential accumulation in tumor, ability to activate an immune response against tumor cells, high immunogenicity, replication competence, and ability to express exogenous proteins and combinations thereof.

In some embodiments, one or more steps can be performed prior to administration of the therapeutic virus to the subject. Any of a variety of preceding steps can be performed, including, but not limited to, diagnosing the subject with a condition appropriate for virus administration, determining the immunocompetence of the subject, immunizing the subject, treating the subject with a chemotherapeutic agent, treating the subject with radiation, or surgically treating the subject. Therapeutic methods or steps performed prior to administration of the therapeutic virus to the subject are well known in the art and are described elsewhere, for example, in U.S. Patent Publication No. 2005-0031643 and U.S. Application Serial Nos. 11/975,088 and 11/975,090.

Any mode of administration of a virus to a subject can be used, provided the mode of administration permits the virus to enter a tumor or metastasis. Modes of administration can include, but are not limited to, intravenous, intraperitoneal, subcutaneous, intramuscular, transdermal, intradermal, intra-arterial (*e*.*g*., hepatic artery infusion), intravesicular perfusion, intrapleural, intraarticular, tapical, intratumoral, intralesional, multipuncture (*e*.*g*., as used with smallpox vaccines), inhalation, percutaneous, subcutaneous, intranasal, intratracheal, oral, intracavity (*e*.*g*., administering to the bladder via a catheter, administering to the gut by suppository or enema), vaginal, rectal, intracranial, intraprostatic, intravitreal, aural, or ocular administration.

Also described are methods in which an additional therapeutic agent, such as an additional therapeutic virus or a therapeutic compound is administered. These can be administered simultaneously, sequentially or intermittently with the first therapeutic virus. The additional therapeutic agent can interact with the virus or a gene product thereof, or the additional therapeutic agent can act independently of the virus.

One skilled in the art can select any mode of administration compatible with the subject and the virus, which is administered alone or in combination with a chemotherapeutic agent, and that also is likely to result in the virus reaching tumors and/or metastases. The route of administration can be selected by one skilled in the art according to any of a variety of factors, including the nature of the disease, the kind of tumor, and the particular virus to be administered. Administration to the target site can be performed, for example, by direct injection into a tumor or by systemic administration at a site distal to the tumor, such as by injection into the bloodstream (*e*.*g*., artery or vein) or other parenteral route or by ballistic delivery, such as a colloidal dispersion system. Typically, the therapeutic viruses provided herein are administered systemically.

### a. Virus Administration and Dosages

The dosage regimen can be any of a variety of methods of administration and amounts of each administered agent, and can be determined by one skilled in the art according to known clinical factors. As is known in the medical arts, dosages for any one patient can depend on many factors, including the subject's species, size, body surface area, age, sex, immunocompetence, and general health, the particular therapeutic virus to be administered, duration and route of administration, the kind and stage of the disease, for example, tumor size, and other treatments or compounds, such as chemotherapeutic agents that are administered concurrently. In addition to the above factors, such levels can be affected by the infectivity of the virus, and the nature of the virus, as can be determined by one skilled in the art. In the present methods, appropriate minimum dosage levels of viruses can be levels sufficient for the virus to survive, grow and replicate in a tumor or metastasis. Exemplary minimum levels for administering a virus to a 65 kg human can include at least about 1 x 10⁵ plaque forming units (PFU), at least about 5 x 10⁵ PFU, at least about 1 x 10⁶ PFU, at least about 5 x 10⁶ PFU, at least about 1 x 10⁷ PFU, at least about 1 x 10⁸ PFU, at least about 1 x 10⁹ PFU, or at least about 1 x 10¹⁰ PFU. In the present methods, appropriate maximum dosage levels of viruses can be levels that are not toxic to the host, levels that do not cause splenomegaly of 3 times or more, levels that do not result in colonies or plaques in normal tissues or organs after about 1 day or after about 3 days or after about 7 days. Exemplary maximum levels for administering a virus to a 65 kg human can include no more than about 1 x 10¹¹ PFU, no more than about 5x10¹⁰ PFU, no more than about 1 x 10¹⁰ PFU, no more than about 5 x 10⁹ PFU, no more than about 1 x 10⁹ PFU, or no more than about 1 x 10⁸ PFU.

### b. Chemotherapeutic Agent Administration Dosage

For combination therapies with chemotherapeutic compounds, dosages for the administration of such compounds are known in the art or can be determined by one skilled in the art according to known clinical factors (*e*.*g*., subject's species, size, body surface area, age, sex, immunocompetence, and general health, duration and route of administration, the kind and stage of the disease, for example, tumor size, and other viruses, treatments, or compounds, such as other chemotherapeutic drugs, being administered concurrently). In addition to the above factors, such levels can be affected by the infectivity of the virus, and the nature of the virus, as can be determined by one skilled in the art.

One skilled in the art can select any mode of administration compatible with the chemotherapeutic agent to be administered including, but not limited to systemic or local injection. Exemplary methods of administration include, but are not limited to, systemically, intravenously, intraarterially, intratumorally, endoscopically, intralesionally, intramuscularly, intradermally, intraperitoneally, intravesicularly, intraarticularly, intrapleurally, percutaneously, subcutaneously, orally, parenterally, intranasally, intratracheally, by inhalation, intracranially, intraprostaticaly, intravitreally, topically, ocularly, vaginally, or rectally. When administered simultaneously with the therapeutic virus, the chemotherapeutic agents typically are administered by the same route, but can be administered by a different route.

### i. Gemcitabine Dosage and Administration

Gemcitabine (GEMZAR^{®}) is a nucleoside analogue that exhibits antitumor activity. Gemcitabine is employed in the therapy of breast cancer, non-small cell lung cancer and pancreatic cancer. Methods of employing gemcitabine clinically are well known in the art. For example, for the treatment of pancreatic cancer, gemcitabine has been administered by intravenous infusion at a dose of 1000 mg/m² over 30 minutes once weekly for up to 7 weeks (or until toxicity necessitates reducing or holding a dose), followed by a week of no treatment. Subsequent cycles can consist of infusions once weekly for 3 consecutive weeks out of every 4 weeks. Gemcitabine has also been employed in combination with cisplatin in cancer therapy.

In one exemplary embodiment, a therapeutic virus, such as a modified vaccinia virus, is administered to a subject for the treatment of a tumor or metastasis about once, twice, three times or four times with approximately 0-60 days between each administration, followed by approximately 1-30 days where no anti-cancer treatment is administered, then gemcitabine is administered about once, twice, three times, four times, five times, six times or seven times with approximately 0-30 days between each administration, followed by approximately 1-30 days where no anti-cancer treatment is administered. Such treatment scheme can be repeated.

In another exemplary embodiment, gemcitabine is administered 1-7 times with approximately 0-30 days between each administration, followed by approximately 1-10 days where no anti-cancer treatment is administered, then the mutant vaccinia virus is administered once or 2-4 times with approximately 0-60 days between each administration. This is followed by approximately 5-60 days where no anti-cancer treatment is administered. Such treatment scheme can be repeated.

In another exemplary embodiment, gemcitabine is administered 1-7 times with approximately 0-30 days between each administration, followed by approximately 1-10 days where no anti-cancer treatment is administered, then the mutant vaccinia virus is administered once or 2-4 times with approximately 0-60 days between each administration. This is followed by approximately 5-60 days where no anti-cancer treatment is administered, then gemcitabine is administered again for 1-7 times with approximately 0-30 days between each administration. Such treatment scheme can be repeated.

In another exemplary embodiment, gemcitabine is first administered to a patient 1-7 times with approximately 0-30 days between each administration, followed by approximately 1-30 days where no anti-cancer treatment is administered. The mutant vaccinia virus is then administered once or 2-4 times with approximately 0-60 days between each administration, followed by approximately 1-30 days where no anti-cancer treatment is administered, then gemcitabine is administered 1-7 times with approximately 0-30 days between each administration, followed by approximately 1-30 days where no anti-cancer treatment is administered. Such treatment scheme can be repeated.

In another exemplary embodiment, gemcitabine is first administered to a patient 1-7 times with approximately 0-30 days between each administration, followed by approximately 1-10 days where no anti-cancer treatment is administered, then the mutant vaccinia virus is administered once or 2-4 times with approximately 0-60 days between each administration. This is followed by approximately 5-60 days where no anti-cancer treatment is administered. Such treatment scheme can be repeated.

In another exemplary embodiment, gemcitabine is first administered to a patient 1-7 times with approximately 0-30 days between each administration, followed by approximately 1-10 days where no anti-cancer treatment is administered, then the mutant vaccinia virus is administered once or 2-4 times with approximately 0-60 days between each administration. This is followed by approximately 5-60 days where no anti-cancer treatment is administered, then gemcitabine is administered again for 1-7 times with approximately 0-30 days between each administration. Such treatment scheme can be repeated.

In another exemplary embodiment, the mutant vaccinia virus is administered once or 2-4 times with approximately 0-60 days between each administration, followed by approximately 1-30 days where no anti-cancer treatment is administered. Symptoms associated with virus toxicity or with a virus-induced disease state appear in the patient, wherein the appearance of symptoms results from virus administration. Gemcitabine is then administered 1-7 times with approximately 0-30 days between each administration, followed by approximately 1-30 days where no anti-cancer treatment is administered. Such treatment scheme can be repeated.

### ii. Irinotecan Dosage and Administration

Irinotecan (Camptosar^{®}) is a compound primarily employed in the therapy of colon cancer, particularly in combination with other chemotherapy agents such as 5-FU and leucovorin. Irinotecan is a topoisomerase 1 inhibitor, which leads to inhibition of both DNA replication and transcription.

Methods of employing irinotecan clinically are well known in the art and are recommended over a dose range of from about 50 to about 350 mg/m². For example, irinotecan has been administered by intravenous infusion at about 350 mg/m² over a 30 minute period once every three weeks for up to 4 months (or until toxicity necessitates reducing or holding a dose) for colorectal cancer. Irinotecan has also been administered by intravenous infusion at doses ranging from about 100 to 150 mg/m² over a 90 minute period once weekly for 4 weeks, followed by a 2-week rest period; subsequent 6-week cycles were then repeated over a period of between 3 to 5 months. Irinotecan has also been employed in combination with 5-FU and leucovirin in colorectal cancer therapy.

In one exemplary embodiment, the mutant vaccinia virus is administered once or 2-4 times with 0-60 days between each administration, followed by 1-30 days where no anti-cancer treatment is administered, then irinotecan is administered 1-7 times with 0-30 days between each administration, followed by 1-30 days where no anti-cancer treatment is administered. Such treatment scheme can be repeated. In another exemplary embodiment, irinotecan is administered 1-7 times with 0-30 days between each administration, followed by 1-10 days where no anti-cancer treatment is administered, then the mutant vaccinia virus is administered once or 2-4 times with 0-60 days between each administration. This is followed by 5-60 days where no anti-cancer treatment is administered. Such treatment scheme can be repeated. In another exemplary embodiment, irinotecan is administered 1-7 times with 0-30 days between each administration, followed by 1-10 days where no anti-cancer treatment is administered, then the mutant vaccinia virus is administered once or 2-4 times with 0-60 days between each administration. This is followed by 5-60 days where no anti-cancer treatment is administered, then irinotecan is administered again for 1-7 times with 0-30 days between each administration. Such treatment scheme can be repeated.

In another exemplary embodiment, irinotecan is first administered to a patient 1-7 times with 0-30 days between each administration, followed by 1-30 days where no anti-cancer treatment is administered. The mutant vaccinia virus is then administered once or 2-4 times with 0-60 days between each administration, followed by 1-30 days where no anti-cancer treatment is administered, then irinotecan is administered 1-7 times with 0-30 days between each administration, followed by 1-30 days where no anti-cancer treatment is administered. Such treatment scheme can be repeated. In another exemplary embodiment, irinotecan is first administered to a patient 1-7 times with 0-30 days between each administration, followed by 1-10 days where no anti-cancer treatment is administered, then the mutant vaccinia virus is administered once or 2-4 times with 0-60 days between each administration. This is followed by 5-60 days where no anti-cancer treatment is administered. Such treatment scheme can be repeated. In another exemplary embodiment, irinotecan is first administered to a patient 1-7 times with 0-30 days between each administration, followed by 1-10 days where no anti-cancer treatment is administered, then the mutant vaccinia virus is administered once or 2-4 times with 0-60 days between each administration. This is followed by 5-60 days where no anti-cancer treatment is administered, then irinotecan is administered again for 1-7 times with 0-30 days between each administration. Such treatment scheme can be repeated.

In another exemplary embodiment, the mutant vaccinia virus is administered once or 2-4 times with 0-60 days between each administration, followed by 1-30 days where no anti-cancer treatment is administered. Symptoms associated with virus toxicity or with a virus-induced disease state appear in the patient, wherein the appearance of symptoms results from virus administration. Irinotecan is then administered 1-7 times with 0-30 days between each administration, followed by 1-30 days where no anti-cancer treatment is administered. Such treatment scheme can be repeated.

### iii. Doxorubicin Dosage Regimens

Doxorubicin is a DNA-interacting drug that is widely used in chemotherapy. It is an anthracycline antibiotic that is structurally related to daunomycin, which also intercalates DNA. The interactions between doxorubicin and DNA appear to interfere with the action of topoisomeriase II, thus leading to inhibition of DNA replication and transcription.

Methods of employing doxorubicin clinically are well known in the art and are recommended over a dose range of from about 30 to about 75 mg/m². For example, doxorubicin has been administered by intravenous infusion at about 60 to about 75 mg/m² over a 30 minute period once every three weeks to four weeks until toxicity necessitates reducing or holding a dose.

In one exemplary embodiment, the mutant vaccinia virus is administered once or 2-4 times with 0-60 days between each administration, followed by 1-30 days where no anti-cancer treatment is administered, then doxorubicin is administered 1-7 times with 0-30 days between each administration, followed by 1-30 days where no anti-cancer treatment is administered. Such treatment scheme can be repeated. In another exemplary embodiment, doxorubicin is administered 1-7 times with 0-30 days between each administration, followed by 1-10 days where no anti-cancer treatment is administered, then the mutant vaccinia virus is administered once or 2-4 times with 0-60 days between each administration. This is followed by 5-60 days where no anti-cancer treatment is administered. Such treatment scheme can be repeated. In another exemplary embodiment, doxorubicin is administered 1-7 times with 0-30 days between each administration, followed by 1-10 days where no anti-cancer treatment is administered, then the mutant vaccinia virus is administered once or 2-4 times with 0-60 days between each administration. This is followed by 5-60 days where no anti-cancer treatment is administered, then doxorubicin is administered again for 1-7 times with 0-30 days between each administration. Such treatment scheme can be repeated.

In another exemplary embodiment, doxorubicin is first administered to a patient 1-7 times with 0-30 days between each administration, followed by 1-30 days where no anti-cancer treatment is administered. The mutant vaccinia virus is then administered once or 2-4 times with 0-60 days between each administration, followed by 1-30 days where no anti-cancer treatment is administered, then doxorubicin is administered 1-7 times with 0-30 days between each administration, followed by 1-30 days where no anti-cancer treatment is administered. Such treatment scheme can be repeated. In another exemplary embodiment, doxorubicin is first administered to a patient 1-7 times with 0-30 days between each administration, followed by 1-10 days where no anti-cancer treatment is administered, then the mutant vaccinia virus is administered once or 2-4 times with 0-60 days between each administration. This is followed by 5-60 days where no anti-cancer treatment is administered. Such treatment scheme can be repeated. In another exemplary embodiment, doxorubicin is first administered to a patient 1-7 times with 0-30 days between each administration, followed by 1-10 days where no anti-cancer treatment is administered, then the mutant vaccinia virus is administered once or 2-4 times with 0-60 days between each administration. This is followed by 5-60 days where no anti-cancer treatment is administered, then doxorubicin is administered again for 1-7 times with 0-30 days between each administration. Such treatment scheme can be repeated.

In another exemplary embodiment, the mutant vaccinia virus is administered once or 2-4 times with 0-60 days between each administration, followed by 1-30 days where no anti-cancer treatment is administered. Symptoms associated with virus toxicity or with a virus-induced disease state appear in the patient, wherein the appearance of symptoms results from virus administration. Doxorubicin is then administered 1-7 times with 0-30 days between each administration, followed by 1-30 days where no anti-cancer treatment is administered. Such treatment scheme can be repeated.

### iv. Cisplatin Dosage Regimens

Cisplatin (also known as cisplatinum and CDDP) is a platinum-based chemotherapy that acts by crosslinking DNA, making it difficult for dividing cells to duplicate their DNA during mitosis.

Methods of employing cisplatin clinically are well known in the art and are recommended over a dose range of from about 20 mg/m² to about 140 mg/m². For example, cisplatin has been administered by intravenous infusion at about 20 mg/m² over at least a one hour period, typically over a 6- to 8 hour period, daily for 5 days per cycle for testicular cancer. Cisplatin has also been administered by intravenous infusion at about 75 mg/m² to about 100 mg/m² over at least a one hour period, typically over a 6- to 8-hour period, once every four weeks (until toxicity necessitates reducing or holding a dose) for ovarian cancer. The cisplatin can be administered in combination with another chemotherapeutic agent or as a single agent. In addition, cisplatin has been administered as a single agent by intravenous infusion at about 50 mg/m² to about 70 mg/m² over at least a one hour period, typically over a 6- to 8-hour period, once every three to four weeks (until toxicity necessitates reducing or holding a dose) for bladder cancer.

In one exemplary embodiment, the mutant vaccinia virus is administered once or 2-4 times with 0-60 days between each administration, followed by 1-30 days where no anti-cancer treatment is administered, then cisplatin is administered 1-7 times with 0-30 days between each administration, followed by 1-30 days where no anti-cancer treatment is administered. Such treatment scheme can be repeated. In another exemplary embodiment, cisplatin is administered 1-7 times with 0-30 days between each administration, followed by 1-10 days where no anti-cancer treatment is administered, then the mutant vaccinia virus is administered once or 2-4 times with 0-60 days between each administration. This is followed by 5-60 days where no anti-cancer treatment is administered. Such treatment scheme can be repeated. In another exemplary embodiment, cisplatin is administered 1-7 times with 0-30 days between each administration, followed by 1-10 days where no anti-cancer treatment is administered, then the mutant vaccinia virus is administered once or 2-4 times with 0-60 days between each administration. This is followed by 5-60 days where no anti-cancer treatment is administered, then cisplatin is administered again for 1-7 times with 0-30 days between each administration. Such treatment scheme can be repeated.

In another exemplary embodiment, cisplatin is first administered to a patient 1-7 times with 0-30 days between each administration, followed by 1-30 days where no anti-cancer treatment is administered. The mutant vaccinia virus is then administered once or 2-4 times with 0-60 days between each administration, followed by 1-30 days where no anti-cancer treatment is administered, then cisplatin is administered 1-7 times with 0-30 days between each administration, followed by 1-30 days where no anti-cancer treatment is administered. Such treatment scheme can be repeated. In another exemplary embodiment, cisplatin is first administered to a patient 1-7 times with 0-30 days between each administration, followed by 1-10 days where no anti-cancer treatment is administered, then the mutant vaccinia virus is administered once or 2-4 times with 0-60 days between each administration. This is followed by 5-60 days where no anti-cancer treatment is administered. Such treatment scheme can be repeated. In another exemplary embodiment, cisplatin is first administered to a patient 1-7 times with 0-30 days between each administration, followed by 1-10 days where no anti-cancer treatment is administered, then the mutant vaccinia virus is administered once or 2-4 times with 0-60 days between each administration. This is followed by 5-60 days where no anti-cancer treatment is administered, then cisplatin is administered again for 1-7 times with 0-30 days between each administration. Such treatment scheme can be repeated.

In another exemplary embodiment, the mutant vaccinia virus is administered once or 2-4 times with 0-60 days between each administration, followed by 1-30 days where no anti-cancer treatment is administered. Symptoms associated with virus toxicity or with a virus-induced disease state appear in the patient, wherein the appearance of symptoms results from virus administration. Cisplatin is then administered 1-7 times with 0-30 days between each administration, followed by 1-30 days where no anti-cancer treatment is administered. Such treatment scheme can be repeated.

As will be understood by one of skill in the art, the optimal treatment regimen will vary and it is within the scope of the treatment methods to evaluate the status of the disease under treatment and the general health of the patient prior to, and following one or more cycles of combination therapy in order to determine the optimal therapeutic combination.

### 2. Number of administrations

The methods described herein can include a single administration of a virus, alone or in combination with a chemotherapeutic agent, to a subject or multiple administrations of a virus to a subject. In some embodiments, a single administration is sufficient to establish a virus in a tumor, where the virus can proliferate and can cause or enhance an anti-tumor response in the subject; such methods do not require additional administrations of a virus in order to cause or enhance an anti-tumor response in a subject, which can result, for example in inhibition of tumor growth, inhibition of metastasis growth or formation, reduction in tumor or size, elimination of a tumor or metastasis, inhibition or prevention of recurrence of a neoplastic disease or new tumor formation, or other cancer therapeutic effects. In other embodiments, a virus can be administered on different occasions, alone or in combination with a chemotherapeutic agent, separated in time typically by at least one day. Separate administrations can increase the likelihood of delivering a virus to a tumor or metastasis, where a previous administration has been ineffective in delivering a virus to a tumor or metastasis. Separate administrations can increase the locations on a tumor or metastasis where virus proliferation can occur or can otherwise increase the titer of virus accumulated in the tumor, which can increase the scale of release of antigens or other compounds from the tumor in eliciting or enhancing a host's anti-tumor immune response, and also can, optionally, increase the level of virus-based tumor lysis or tumor cell death. Separate administrations of a virus can further extend a subject's immune response against viral antigens, which can extend the host's immune response to tumors or metastases in which viruses have accumulated, and can increase the likelihood of a host mounting an anti-tumor immune response. In other embodiments, a virus can be administered on different occasions, wherein the virus is sometimes administered alone and at other times administered with a chemotherapeutic agent, separated in time typically by at least one day.

When separate administrations are performed, each administration can be a dosage amount that is the same or different relative to other administration dosage amounts. Dosage amount can refer to dosage of virus, dosage of chemotherapeutic agent, or dosage of both. In one embodiment, all administration dosage amounts are the same. In other embodiments, a first dosage amount, with respect to the dosage of virus, dosage of chemotherapeutic agent, or dosage of both, can be a larger dosage amount than one or more subsequent dosage amounts, for example, at least 10x larger, at least 100x larger, or at least 1000x larger than subsequent dosage amounts. Any combination of dosage amount for the virus, either administered alone or in combination with a chemotherapeutic agent, is contemplated. In one example of a method of separate administrations in which a virus is administered alone or in combination with a chemotherapeutic agent, the first virus dosage amount is greater than one or more subsequent virus dosage amounts, and all subsequent dosage amounts can be the same, smaller amount relative to the first administration. In another example of a method of separate administrations in which a virus is in combination with a chemotherapeutic agent, the first chemotherapeutic agent dosage amount is greater than one or more subsequent agent dosage amounts, and all subsequent dosage amounts can be the same, smaller amount relative to the first administration. In another example of a method of separate administrations in which a virus is in combination with a chemotherapeutic agent, the first virus dosage amount and first chemotherapeutic agent dosage amount are both greater than one or more subsequent virus and agent dosage amounts, and all subsequent virus and agent dosage amounts can be the same, smaller amount relative to the first administration.

Separate administrations can include any number of two or more administrations, including two, three, four, five, six, seven, eight, nine, ten or more administrations. One skilled in the art can readily determine the number of administrations to perform or the desirability of performing one or more additional administrations according to methods known in the art for monitoring therapeutic methods and other monitoring methods provided herein. Accordingly, the methods provided herein include methods of providing to the subject one or more administrations of a virus, alone or in combination with a chemotherapeutic agent, where the number of administrations can be determined by monitoring the subject, and, based on the results of the monitoring, determining whether or not to provide one or more additional administrations. Deciding on whether or not to provide one or more additional administrations can be based on a variety of monitoring results, including, but not limited to, indication of tumor growth or inhibition of tumor growth, appearance of new metastases or inhibition of metastasis, the subject's anti-virus antibody titer, the subject's anti-tumor antibody titer, the overall health of the subject, the weight of the subject, the presence of virus solely in tumor and/or metastases, the presence of virus in normal tissues or organs.

The time period between administrations can be any of a variety of time periods. The time period between administrations can be a function of any of a variety of factors, including monitoring steps, as described in relation to the number of administrations, the time period for a subject to mount an immune response, the time period for a subject to clear the virus from normal tissue, or the time period for virus proliferation in the tumor or metastasis. As described in U.S. Patent Publication No. 2005-0031643 and U.S. Provisional Application No. 60/852,390, one of skill in the art can determine the time period between administrations.

### 3. Co-administrations

Any therapeutic or anti-cancer agent can be used as the second, therapeutic or anti-cancer agent in the combined cancer treatment methods provided herein. The methods can include administering one or more therapeutic compounds to the subject in addition to administering a virus or plurality thereof to a subject. Therapeutic compounds can act independently, or in conjunction with the virus, for tumor therapeutic effects.

Therapeutic compounds that act in conjunction with the viruses include, for example, compounds that alter the expression of the viruses or compounds that can interact with a virally-expressed gene, or compounds that can inhibit virus proliferation, including compounds toxic to the virus. Therapeutic compounds that can act in conjunction with the virus include, for example, therapeutic compounds that increase the proliferation, toxicity, tumor cell killing, or immune response eliciting properties of a virus, and also can include, for example, therapeutic compounds that decrease the proliferation, toxicity, or cell killing properties of a virus. Optionally, the therapeutic agent can exhibit or manifest additional properties, such as, properties that permit its use as an imaging agent, as described elsewhere herein.

Therapeutic compounds also include, but are not limited to, chemotherapeutic agents, nanoparticles, radiation therapy, siRNA molecules, enzyme/pro-drug pairs, photosensitizing agents, toxins, microwaves, a radionuclide, an angiogenesis inhibitor, a mitosis inhibitor protein (e.g., cdc6), an antitumor oligopeptide (e.g., antimitotic oligopeptides, high affinity tumor-selective binding peptides), a signaling modulator, anti-cancer antibiotics, or a combination thereof.

Toxins include, but are not limited to, chemotherapeutic compounds such as, but not limited to, 5-fluorouridine, calicheamicin and maytansine. Signaling modulators include, but are not limited to, for example, inhibitors of macrophage inhibitory factor, toll-like receptor agonists and stat 3 inhibitors. In one embodiment, a vaccinia virus, such as a vaccinia virus provided herein, is administered to a subject having a tumor, cancer or metastasis in combination with a toxin or a signaling modulator.

Chemotherapeutic compounds include, but are not limited to, alkylating agents such as thiotepa and cyclosphosphamide; alkyl sulfonates such as busulfan, improsulfan and piposulfan; aziridines such as benzodopa, carboquone, meturedopa and uredopa; ethylenimines and methylamelamines including altretamine, triethylenemelamine, trietylenephosphoramide, triethylenethiophosphaoramide and trimethylolmelamine nitrogen mustards such as chlorambucil, chlomaphazine, cholophosphamide, estramustine, ifosfamide, mechlorethamine, mechlorethamine oxide hydrochloride, melphalan, novembichin, phenesterine, prednimustine, trofosfamide, uracil mustard; nitrosureas such as carmustine, chlorozotocin, fotemustine, lomustine, nimustine, ranimustine; antibiotics such as aclacinomysins, actinomycin, authramycin, azaserine, bleomycins, cactinomycin, calicheamicin, carabicin, carminomycin, carzinophilin, chromomycins, dactinomycin, daunorubicin, detorubicin, 6-diazo-5-oxo-L-norleucine, doxorubicin, epirubicin, esorubicin, idarubicin, marcellomycin, mitomycins, mycophenolic acid, nogalamycin, olivomycins, peplomycin, potfiromycin, puromycin, quelamycin, rodorubicin, streptonigrin, streptozocin tubercidin, ubenimex, zinostatin, zorubicin; anti-metabolites such as methotrexate and 5-fluorouracil (5-FU®); folic acid analogues such as denopterin, methotrexate, pteropterin, trimetrexate; purine analogs such as fludarabine, 6-mercaptopurine, thiamiprine, thioguanine; pyrimidine analogs such as ancitabine, azacitidine, 6-azauridine, carmofur, cytarabine, dideoxyuridine, doxifluridine, enocitabine, floxuridine; androgens such as calusterone, dromostanolone propionate, epitiostanol, mepitiostane, testolactone; anti-adrenals such as aminoglutethimide, mitotane, trilostane; folic acid replenishers such as frolinic acid; aceglatone; aldophosphamide glycoside; aminolevulinic acid; amsacrine; bestrabucil; bisantrene; edatraxate; defofamine; demecolcine; diaziquone; elfornithine; elliptinium acetate; etoglucid; gallium nitrate; hydroxyurea; lentinan; lonidamine; mitoguazone; mitoxantrone; mopidamol; nitracrine; pentostatin; phenamet; pirarubicin; podophyllinic acid; 2-ethylhydrazide; procarbazine; polysaccharide-K; razoxane; sizofiran; spirogermanium; tenuazonic acid; triaziquone; 2,2',2"-trichlorotriethylamine; urethan; vindesine; dacarbazine; mannomustine; mitobronitol; mitolactol; pipobroman; gacytosine; cytosine arabinoside; cyclophosphamide; thiotepa; taxoids, e.g., paclitaxel and doxetaxel; chlorambucil; gemcitabine; 6-thioguanine; mercaptopurine; methotrexate; platinum analogs such as cisplatin, carboplatin and oxaliplatin; vinblastine; platinum; etoposide (VP-16); ifosfamide; mitomycin C; mitoxantrone;vincristine; vinorelbine; navelbine; novantrone; teniposide; daunomycin; aminopterin; xeloda; ibandronate; CPT11; topoisomerase inhibitor RFS 200; difluoromethylomithine (DMFO); retinoic acid; esperamicins; capecitabine; interleukin 2; cladribine; denileukin diftitox; docetaxel; exemestane; gemtuzumab ozogamicin; interferon alfa-2a; interferon alfa-2b; Interferon gamma-1b; isotretinoin; letrozole; mechlorethamine HCL; megestrol; mercaptopurine; pegaspargase; plicamycin; temozolomide, tretinoin, valrubicin, and pharmaceutically acceptable salts, acids or derivatives of any of the above. Also included are anti-hormonal agents that act to regulate or inhibit hormone action on tumors such as anti-estrogens including for example tamoxifen, raloxifene, aromatase inhibiting 4(5)-imidazoles, 4-hydroxytamoxifen, trioxifene, keoxifene, LY117018, onapristone and toremifene (Fareston); and antiandrogens such as flutamide, nilutamide, bicalutamide, leuprolide and goserelin; and pharmaceutically acceptable salts, acids or derivatives of any of the above. Such chemotherapeutic compounds that can be used herein include compounds whose toxicities preclude use of the compound in general systemic chemotherapeutic methods. Chemotherapeutic agents also include new classes of targeted chemotherapeutic agents such as, for example, imatinib (sold by Novartis under the trade name Gleevec in the United States), gefitinib (developed by Astra Zeneca under the trade name Iressa) and erlotinib. Particular chemotherapeutic agents include, but are not limited to, cisplatin, carboplatin, oxaliplatin, DWA2114R, NK121, IS 3 295, and 254-S vincristine, prednisone, doxorubicin and L-asparaginase; mechloroethamine, vincristine, procarbazine and prednisone (MOPP), cyclophosphamide, irinotecan, doxorubicin, bleomycin, vinblastine, gemcitabine and 5-flurouracil. Exemplary chemotherapeutic agents are, for example, cisplatin, carboplatin, oxaliplatin, DWA2114R, NK121, IS 3 295, and 254-S. In a non-limiting embodiment, a vaccinia virus, such as a vaccinia virus described herein, is administered to a subject having a tumor, cancer or metastasis in combination with a platinum coordination complex, such as cisplatin, carboplatin, oxaliplatin, DWA2114R, NK121, IS 3 295, and 254-S. Tumors, cancers and metastasis can be any of those provided herein, and in particular, can be a pancreatic tumor, an ovarian tumor, a lung tumor, a colon tumor, a prostate tumor, a cervical tumor or a breast tumor; exemplary tumors are pancreatic and ovarian tumors. Tumors, cancers and metastasis can be a monotherapy-resistant tumor such as, for example, one that does not respond to or poorly responds to therapy with virus alone or anti-cancer agent alone, but that does respond to therapy with a combination of virus and anti-cancer agent. Typically, a therapeutically effective amount of virus is systemically administered to the subject and the virus localizes and accumulates in the tumor. Subsequent to administering the virus, the subject is administered a therapeutically effective amount of an anti-cancer agent, such as cisplatin. In one example, cisplatin is administered once-daily for five consecutive days. One of skill in the art could determine when to administer the anti-cancer agent subsequent to the virus using, for example, *in vivo* animal models. Using the methods described herein, administration of a virus and anti-cancer agent, such as cisplatin can cause a reduction in tumor volume, can cause tumor growth to stop or be delayed or can cause the tumor to be eliminated from the subject. The status of tumors, cancers and metastasis following treatment can be monitored using any of the methods provided herein and known in the art.

In one embodiment, nanoparticles can be designed such that they carry one or more therapeutic agents provided herein. Additionally, nanoparticles can be designed to carry a molecule that targets the nanoparticle to the tumor cells. In one non-limiting example, nanoparticles can be coated with a radionuclide and, optionally, an antibody immunoreactive with a tumor-associated antigen. In one embodiment, a vaccinia virus, such as a vaccinia virus provided herein, is administered to a subject having a tumor, cancer or metastasis in combination with a nanoparticle carrying any of the therapeutic agents described herein.

Thus, described herein are methods of administering to a subject one or more therapeutic compounds that can act in conjunction with the virus to increase the proliferation, toxicity, tumor cell killing, or immune response eliciting properties of a virus. Also described herein are methods of administering to a subject one or more therapeutic compounds that can act in conjunction with the virus to decrease the proliferation, toxicity, or cell killing properties of a virus. Therapeutic compounds to be administered can be any of those provided herein or in the art.

In some embodiments, therapeutic compounds that can act in conjunction with the virus to decrease the proliferation, toxicity, or cell killing properties of a virus are compounds that can inhibit viral replication, inhibit viral toxins, or cause viral death. A therapeutic compound that can inhibit viral replication, inhibit viral toxins, or cause viral death can generally include a compound that can block one or more steps in the viral life cycle, including, but not limited to, compounds that can inhibit viral DNA replication, viral RNA transcription, viral coat protein assembly, outer membrane or polysaccharide assembly. Any of a variety of compounds that can block one or more steps in a viral life cycle are known in the art, including any known antiviral compound (e.g., cidofovir), viral DNA polymerase inhibitors, viral RNA polymerase inhibitors, inhibitors of proteins that regulate viral DNA replication or RNA transcription. In another example, a virus can contain a gene encoding a viral life cycle protein, such as DNA polymerase or RNA polymerase that can be inhibited by a compound that is, optionally, non-toxic to the host organism.

Effective delivery of each components of the combination therapy is an important aspect of the methods provided herein. In accordance with one aspect, the modes of administration discussed below exploit one of more of the key features: (i) delivery of a virus provided herein to the tumors by a mode of administration effect to achieve highest titer of virus and highest therapeutic effect; (ii) delivery of any other mentioned therapeutic modalities to the tumor by a mode of administration to achieve the optimal therapeutic effect. The dose scheme of the combination therapy administered is such that the combination of the two or more therapeutic modalities is therapeutically effective. Dosages will vary in accordance with such factors as the age, health, sex, size and weight of the patient, the route of administration, the toxicity of the drugs, frequency of treatment, and the relative susceptibilities of the cancer to each of the therapeutic modalities.

Disclosure of second agents and co-administration and modes of delivery of such that are not described here can be found in U.S. Pat. Pub. No. 2005-0031643 and U.S. Provisional Application No. 60/852,390.

### 4. State of the Subject

In some embodiments, the methods described herein for administering a virus to a subject can be performed on a subject in any of a variety of states, including an anesthetized subject, an alert subject, a subject with elevated body temperature, a subject with reduced body temperature, or other state of the subject that is known to affect the accumulation of a virus in the tumor.

### C. VIRUSES AND CHEMOTHERAPEUTIC AGENTS FOR

### TREATMENT AND DIAGNOSIS

### 1. Viruses, vectors

Described herein are viruses and compositions containing the viruses, alone or in combination with a chemotherapeutic agent, for therapeutic use. The viruses described herein are typically attenuated. Attenuated viruses have a decreased capacity to cause disease in a host. The decreased capacity can result from any of a variety of different modifications to the ability of a virus to be pathogenic. For example, a virus can have reduced toxicity, reduced ability to accumulate in non-tumorous organs or tissue, reduced ability to cause cell lysis or cell death, or reduced ability to replicate compared to the non-attenuated form thereof. Attenuation of virus can be achieved by inserting copies of exogenous expression cassettes into the viral genome to compete; this causes competition for the transcriptional and translational machineries and as well as for nutritional supplies with the virus' associated with endogenous transcription and translation activities of the virus. The attenuated viruses described herein, however, retain at least some capacity to replicate and to cause immunoprivileged cells and tissues, such as tumor cells to leak or lyse, undergo cell death, or otherwise cause or enhance an immune response to immunoprivileged cells and tissues, such as tumor cells.

The viruses described herein can accumulate in immunoprivileged cells or immunoprivileged tissues, including tumors and/or metastases, and also including wounded tissues and cells. While the viruses described herein can typically be cleared from the subject to whom the viruses are administered by activity of the subject's immune system, viruses can nevertheless accumulate, survive and proliferate in immunoprivileged cells and tissues such as tumors because such immunoprivileged areas are sequestered from the host's immune system. Accordingly, the methods described herein, as applied to tumors and/or metastases, and therapeutic methods relating thereto, can readily be applied to other immunoprivileged cells and tissues, including wounded cells and tissues.

Though the viruses described herein can typically be cleared from the subject to whom the viruses are administered by activity of the subject's immune system, circumstances can arise wherein the virus provokes a response in the subject's immune system. This can lead to the experiencing of physiological side effects associated with the administered virus. Accordingly, methods are described herein where the virus can be rapidly cleared from a patient by administration of a chemotherapeutic agent to the patient for alleviation of side effects associated with the virus.

The viruses described herein are modified from their wild type form. Modifications can include any of a variety of changes, and include changes to the genome of the virus. Exemplary nucleic acid modifications include truncations, insertions, deletions and mutations. In an exemplary modification, a viral gene can be modified by truncation, insertion, deletion or mutation. Modifications of the viruses described herein can result in a modification of virus characteristics, including those described herein such as pathogenicity, toxicity, ability to preferentially accumulate in tumor, ability to lyse cells or cause cell death, ability to elicit an immune response against tumor cells, immunogenicity, and replication competence.

The viruses can be RNA or DNA viruses. The viruses can be cytoplasmic viruses, such as poxviruses, or can be nuclear viruses such as adenoviruses. The viruses described herein can have as part of their life cycle lysis of the host cell's plasma membrane. Alternatively, the viruses described herein can have as part of their life cycle exit of the host cell by non-lytic pathways such as budding or exocytosis.

One skilled in the art can select from any of a variety of viruses, according to a variety of factors, including, but not limited to, the intended use of the virus, such as a diagnostic and/or therapeutic use (*e*.*g*., tumor therapy or diagnosis, vaccination, antibody production, or heterologous protein production), the host organism, and the type of tumor.

### 2. Modifications of viruses

The viruses employed in the methods and use described typically are modified viruses. Modified viruses, including modified vaccinia viruses, are contemplated for use herein, where the viruses have been modified by insertions, mutations or deletions, as described more generally elsewhere herein. The vaccinia viruses are modified or selected to have low toxicity and to accumulate in the target tissue. Exemplary of such viruses are those derived from the LIVP strain.

Exemplary insertions, mutations or deletions are those that result in an attenuated virus relative to the wild type strain. For example, virus insertions, mutations or deletions can decrease pathogenicity of the virus, for example, by reducing the toxicity, reducing the infectivity, reducing the ability to replicate, or reducing the number of non-tumor organs or tissues to which the virus can accumulate. Other exemplary insertions, mutations or deletions include, but are not limited to, those that increase antigenicity of the virus, those that permit detection or imaging, those that increase toxicity of the virus (optionally, controlled by an inducible promoter).

Also described herein are modifications of the viruses provided above to enhance one or more characteristics relative to the wild type virus. Such characteristics can include, but are not limited to, attenuated pathogenicity, reduced toxicity, preferential accumulation in tumor, increased ability to activate an immune response against tumor cells, increased immunogenicity, increased or decreased replication competence, and are able to express exogenous proteins, and combinations thereof. In some embodiments, the modified viruses have an ability to activate an immune response against tumor cells without aggressively killing the tumor cells. In other embodiments, the viruses can be modified to express one or more detectable genes, including genes that can be used for imaging. In other embodiments, the viruses can be modified to express one or more genes for harvesting the gene products and/or for harvesting antibodies against the gene products.

### 3. Exemplary viruses

Among the viruses described herein are cytoplasmic viruses, which do not require entry of viral nucleic acid molecules in to the nucleus of the host cell during the viral life cycle. A variety of cytoplasmic viruses are known, including, but not limited to, pox viruses, African swine flu family viruses, and various RNA viruses such as picornaviruses, caliciviruses, togaviruses, coronaviruses and rhabdoviruses. Exemplary cytoplasmic viruses provided herein are viruses of the poxvirus family, including orthopoxviruses. Exemplary of poxviruses provided herein are vaccinia viruses

### a. Poxviruses

In one embodiment, the virus described herein is selected from the poxvirus family. Poxviruses include Chordopoxviridae such as orthopoxvirus, parapoxvirus, avipoxvirus, capripoxvirus, leporipoxvirus, suipoxvirus, molluscipoxvirus and yatapoxvirus, as well as Entomopoxvirinae such as entomopoxvirus A, entomopoxvirus B, and entomopoxvirus C. One skilled in the art can select a particular genus or individual chordopoxviridae according to the known properties of the genus or individual virus, and according to the selected characteristics of the virus (e.g., pathogenicity, ability to elicit an immune response, preferential tumor localization), the intended use of the virus, the tumor type and the host organism. Exemplary chordopoxviridae genera are orthopoxvirus and avipoxvirus.

Avipoxviruses are known to infect a variety of different birds and have been administered to humans. Exemplary avipoxviruses include canarypox, fowlpox, juncopox, mynahpox, pigeonpox, psittacinepox, quailpox, peacockpox, penguinpox, sparrowpox, starlingpox, and turkeypox viruses.

Orthopoxviruses are known to infect a variety of different mammals including rodents, domesticated animals, primates and humans. Several orthopoxviruses have a broad host range, while others have narrower host range. Exemplary orthopoxviruses include buffalopox, camelpox, cowpox, ectromelia, monkeypox, raccoon pox, skunk pox, tatera pox, uasin gishu, vaccinia, variola, and volepox viruses. In some embodiments, the orthopoxvirus selected can be an orthopoxvirus known to infect humans, such as cowpox, monkeypox, vaccinia, or variola virus. Optionally, the orthopoxvirus known to infect humans can be selected from the group of orthopoxviruses with a broad host range, such as cowpox, monkeypox, or vaccinia virus.

### i. Vaccinia viruses

One exemplary orthopoxvirus presented in the methods described herein is vaccinia virus. Vaccinia is a cytoplasmic virus, thus, it does not insert its genome into the host genome during its life cycle. The linear dsDNA viral genome of vaccinia virus is approximately 200 kb in size, encoding a total of approximately 200 potential genes. A variety of vaccinia virus strains are available for the uses and methods provided, including Western Reserve (WR), Copenhagen, Tashkent, Tian Tan, Lister, Wyeth, IHD-J, and IHD-W, Brighton, Ankara, MVA, Dairen I, LIPV, LC16M8, LC16MO, LIVP, WR 65-16, Connaught, New York City Board of Health. Exemplary vaccinia viruses are Lister or LIVP vaccinia viruses. In one embodiment, the Lister strain can be an attenuated Lister strain, such as the LIVP (Lister virus from the Institute of Viral Preparations, Moscow, Russia) strain, which was produced by further attenuation of the Lister strain. The LIVP strain was used for vaccination throughout the world, particularly in India and Russia, and is widely available. In another embodiment, the viruses and methods provided herein can be based on modifications to the Lister strain of vaccinia virus. Lister (also referred to as Elstree) vaccinia virus is available from any of a variety of sources. For example, the Elstree vaccinia virus is available at the ATCC under Accession Number VR-1549. The Lister vaccinia strain has high transduction efficiency in tumor cells with high levels of gene expression.

Vaccinia virus possesses a variety of features for use in cancer gene therapy and vaccination including broad host and cell type range, a large carrying capacity for foreign genes (up to 25 kb of exogenous DNA fragments (approximately 12% of the vaccinia genome size) can be inserted into the vaccinia genome), high sequence homology among different strains for designing and generating modified viruses in other strains, and techniques for production of modified vaccinia strains by genetic engineering are well established (Moss (1993) Curr. Opin. Genet. Dev. 3: 86-90; Broder and Earl (1999) Mol. Biotechnol. 13: 223-245; Timiryasova et al. (2001) Biotechniques 31: 534-540). A variety of vaccinia virus strains are available, including Western Reserve (WR), Copenhagen, Tashkent, Tian Tan, Lister, Wyeth, IHD-J, and IHD-W, Brighton, Ankara, MVA, Dairen I, LIPV, LC16M8, LC16MO, LIVP, WR 65-16, Connaught, New York City Board of Health. Exemplary of vaccinia viruses described herein include, but are not limited to, Lister strain or LIVP strain of vaccinia viruses.

The modifications of the Lister strain described herein also can be adapted to other vaccinia viruses (*e.g.,* Western Reserve (WR), Copenhagen, Tashkent, Tian Tan, Lister, Wyeth, IHD-J, and IHD-W, Brighton, Ankara, MVA, Dairen I, LIPV, LC16M8, LC16MO, LIVP, WR 65-16, Connaught, New York City Board of Health). The modifications of the Lister strain described herein also can be adapted to other viruses, including, but not limited to, viruses of the poxvirus family, adenoviruses, herpes viruses and retroviruses.

### ii. Modification of Vaccinia Viruses

Described herein are vaccinia viruses with insertions, mutations or deletions, as described elsewhere herein. Exemplary insertions, mutations or deletions are those that result in an attenuated vaccinia virus relative to the wild type strain. For example, vaccinia virus insertions, mutations or deletions can decrease pathogenicity of the vaccinia virus, for example, by reducing the toxicity, reducing the infectivity, reducing the ability to replicate, or reducing the number of non-tumor organs or tissues to which the vaccinia virus can accumulate. Other exemplary insertions, mutations or deletions include, but are not limited to, those that increase antigenicity of the virus, those that permit detection or imaging, those that alter attenuation of the virus, and those that alter infectivity. For example, the ability of vaccinia viruses described herein to infect and replicate within tumors can be enhanced by mutations that increase the extracellular enveloped form of the virus (EEV) that is released from the host cell, as described elsewhere herein. Modifications can be made, for example, in genes that are involved in nucleotide metabolism, host interactions and virus formation or at other nonessential gene loci. Any of a variety of insertions, mutations or deletions of the vaccinia virus known in the art can be used herein, including insertions, mutations or deletions of: the thymidine kinase (*TK*) gene, the hemagglutinin (*HA*) gene, and *F14.5L* gene, among others (*e.g.*, E2L/E3L, K1L/K2L, superoxide dismutase locus, 7. 5K, C7-K1L, J2R, B13R+B14R, A56R, A26L or 14L gene loci). The vaccinia viruses provided herein also can contain two or more insertions, mutations or deletions. Thus, included are vaccinia viruses containing two or more insertions, mutations or deletions of the loci provided herein or other loci known in the art. The viruses described herein can be based on modifications to the Lister strain and/or LIVP strain of vaccinia virus. Any known vaccinia virus, or modifications thereof that correspond to those provided herein or known to those of skill in the art to reduce toxicity of a vaccinia virus. Generally, however, the mutation will be a multiple mutant and the virus will be further selected to reduce toxicity.

The modified viruses described herein can contain one more heterologous nucleic acid sequences for the expression of a heterologous gene. The heterologous nucleic acid is typically operably linked to a promoter for expression of the heterologous gene in the infected cells. Suitable promoter include viral promoters, such as a vaccinia virus natural and synthetic promoters. Exemplary vaccinia viral promoters include, but are not limited to, P11k, P7.5k early/late, P7.5k early, P28 late, synthetic early P_{SE}, synthetic early/late P_{SEL} and synthetic late P_{SL} promoters.

The viruses described herein can express one or more genes whose products are useful for tumor therapy. For example, a virus can express a proteins cause cell death or whose products cause an anti-tumor immune response. Such genes can be considered therapeutic genes. A variety of therapeutic gene products, such as toxic or apoptotic proteins, or siRNA, are known in the art, and can be used with the viruses provided herein. The therapeutic genes can act by directly killing the host cell, for example, as a channel-forming or other lytic protein, or by triggering apoptosis, or by inhibiting essential cellular processes, or by triggering an immune response against the cell, or by interacting with a compound that has a similar effect, for example, by converting a less active compound to a cytotoxic compound. Exemplary proteins useful for tumor therapy include, but are not limited to, tumor suppressors, toxins, cytostatic proteins, antiangiogenic proteins, antitumor antibodies, and costimulatory molecules, such as cytokines and chemokines among others provided elsewhere herein and known in the art. The viruses provided herein can also be effective against tumors without the introduction of additional exogenous therapeutic genes.

The viruses described herein can express one or more genes whose products are useful for tumor detection and/or imaging. Exemplary gene products for imaging or detection include detectable proteins or proteins that induce detectable signals. Exemplary of detectable proteins or proteins that induce detectable signals are proteins, such as luciferases, fluorescent proteins, receptors that can bind imaging agents or proteins linked to imaging or diagnostic moieties. Imaging or diagnostic moieties include those that can emit a signal that is detectable by optical or non-optical imaging methods. Detection of the signal by imaging modalities such as, for example, by positron emission tomography (PET) and, thereby allows visualization of the infected tissues, such a tumor or an inflammation.

The viruses described herein can also encode proteins, such as transporter proteins (*e.g.*, the human norepinephrine transporter (hNET) or the human sodium iodide symporter (hNIS)), which can provide increase uptake diagnostic and therapeutic moieties across the cell membrane of infected cells for therapy, imaging or detection.

One skilled in the art can select from any of a variety of viruses, according to a variety of factors, including, but not limited to, the intended use of the virus, such as a diagnostic and/or therapeutic use (e.g., tumor therapy or diagnosis, vaccination, antibody production, or heterologous protein production), the host organism, and the type of tumor. A therapeutic virus for the methods described herein can exhibit one or more desired characteristics for use as a therapeutic agent, such as, for example attenuated pathogenicity, reduced toxicity, preferential accumulation in immunoprivileged cells and tissues, such as tumor, ability to activate an immune response against tumor cells, immunogenic, replication competent, and are able to express exogenous proteins, and combinations thereof.

### iii. Exemplary Modifies Vaccinia Viruses

Exemplary vaccinia viruses contemplated for use in the methods and uses described include those derived from vaccinia virus strain GLV-1h68 (also named RVGL21, SEQ ID NO: 1), which has been described in U.S. Pat. Pub. No. 2005-0031643 GLV-1h68 contains DNA insertions gene loci of the vaccinia virus LIVP strain (SEQ ID NO: 2, a vaccinia virus strain, originally derived by adapting the Lister strain (ATCC Catalog No. VR-1549) to calf skin (Institute of Viral Preparations, Moscow, Russia, Al'tshtein et al., (1983) Dokl. Akad. Nauk USSR 285:696-699)). GLV-1h68 contains expression cassettes encoding detectable marker proteins in the *F14.5L* (also designated in LIVP as *F3*), thymidine kinase (*TK*) and hemagglutinin (*HA*) gene loci. An expression cassette containing a *Ruc-GFP* cDNA molecule (a fusion of DNA encoding *Renilla* luciferase and DNA encoding GFP) under the control of a vaccinia synthetic early/late promoter P_{SEL} ((P_{SEL})*Ruc*-*GFP*) is inserted into the *F14.5L* gene locus; an expression cassette containing a DNA molecule encoding beta-galactosidase under the control of the vaccinia early/late promoter P_{7.5k} ((P_{7.5k})*LacZ*) and DNA encoding a rat transferrin receptor positioned in the reverse orientation for transcription relative to the vaccinia synthetic early/late promoter P_{SEL} ((P_{SEL})*rTrfR*) is inserted into the *TK* gene locus (the resulting virus does not express transferrin receptor protein since the DNA molecule encoding the protein is positioned in the reverse orientation for transcription relative to the promoter in the cassette); and an expression cassette containing a DNA molecule encoding β-glucuronidase under the control of the vaccinia late promoter P₁₁ₖ ((P₁₁ₖ)*gusA*) is inserted into the *HA* gene locus. The GLV-1h68 virus exhibits a strong preference for accumulation in tumor tissues as compared to non-tumorous tissues following systemic administration of the virus to tumor bearing subjects. This preference is significantly higher than the tumor selective accumulation of other vaccinia viral strains, such as WR (see, *e.g.* U.S. Pat. Pub. No. 2005-0031643 and Zhang et al. (2007) Cancer Res. 67(20):10038-46). Modified viruses described herein for the uses and methods provided can be derived from GLV-1h68. Exemplary viruses are generated by replacement of one or more expression cassettes of the GLV-1h68 strain with heterologous DNA encoding gene products for therapy and/or imaging.

Non-limiting examples viruses that are derived from attentuated LIVP viruses, such as GLV-1h68, and can be employed in the methods and uses described include, but are not limited to, LIVP viruses described in U.S. Patent Publication Nos. 2005/0031643, 2004/0234455 and 2004/0213741 and U.S. Patent Application Serial Nos. 11/975,088. For example, the vaccinia virus can be selected from among GLV-1h22, GLV-1h68, GLV-1i69, GLV-1h70, GLV-1h71, GLV-1h72, GLV-1h73, GLV-1h74, GLV-1h81, GLV-1h82, GLV-1h83, GLV-1h84, GLV-1h85, or GLV-1h86, which are described in U.S. Application Serial Nos. 11/975,088 and 11/975,090. Other exemplary vaccinia viruses include, but are not limited to, GLV-1h99, GLV-1h100, GLV-1h101, GLV-1h139, GLV-1h146, GLV-1h151, GLV-1h152 and GLV-1h153, which are described in U.S. Application Serial No. 12/157,960, entitled "MICROORGANISMS FOR IMAGING AND/OR TREATMENT OF TUMORS" and filed June 13, 2008, as well as additional related strains GLV-1h104, GLV-1h105, GLV-1h106, GLV-1h107, GLV-1h108 and GLV-1h109.

Exemplary of viruses which have one or more expression cassettes removed from GLV-1h68 and replaced with a heterologous non-coding DNA molecule include GLV-1h70, GLV-1h71, GLV-1h72, GLV-1h73, GLV-1h74, GLV-1h85, and GLV-1h86. GLV-1h70 contains (P_{SEL})*Ruc*-*GFP* inserted into the *F14.5L* gene locus, (P_{SEL})*rTrfR* and (P_{7.5k})*LacZ* inserted into the *TK* gene locus, and a non-coding DNA molecule inserted into the *HA* gene locus in place of (P₁₁ₖ)*gusA.* GLV-1h71 contains a non-coding DNA molecule inserted into the *F14.5L* gene locus in place of (P_{SEL})*Ruc*-*GFP,* (P_{SEL})*rTrfR* and (P_{7.5k})*LacZ* inserted into the *TK* gene locus, and (P₁₁ₖ)*gusA* inserted into the *HA* gene locus. GLV-1h72 contains (P_{SEL})*Ruc-GFP* inserted into the *F14.5L* gene locus, a non-coding DNA molecule inserted into the *TK* gene locus in place of (P_{SEL})*rTrfR* and (P_{7.5k})*LacZ,* and P₁₁ₖ*gusA* inserted into the *HA* gene locus. GLV-1h73 contains a non-coding DNA molecule inserted into the *F14.5L* gene locus in place of (P_{SEL})*Ruc-GFP,* (P_{SEL})*rTrfR* and (P_{7.5k})*LacZ* inserted into the *TK* gene locus, and a non-coding DNA molecule inserted into the *HA* gene locus in place of (P₁₁ₖ)*gusA.* GLV-1h74 contains a non-coding DNA molecule inserted into the *F14.5L* gene locus in place of (P_{SEL})*Ruc-GFP,* a non-coding DNA molecule inserted into the *TK* gene locus in place of (P_{SEL})*rTrfR* and (P_{7.5k})*LacZ,* and a non-coding DNA molecule inserted into the *HA* gene locus in place of (P₁₁ₖ)*gusA.* GLV-1h85 contains a non-coding DNA molecule inserted into the *F14.5L* gene locus in place of *(P_{SEL})Ruc-GFP,* a non-coding DNA molecule inserted into the *TK* gene locus in place of (P_{SEL})*rTrfR* and (P_{7.5k})*LacZ,* and (P₁₁ₖ)*gusA* inserted into the *HA* gene locus. GLV-1h86 contains (P_{SEL})*Ruc-GFP* inserted into the *F14.5L* gene locus, a non-coding DNA molecule inserted into the *TK* gene locus in place of *(P_{SEL})rTrfR* and (P_{73.5k})*LacZ*, and a non-coding DNA molecule inserted into the HA gene locus in place of (P_{11*k*})*gusA.*

Other exemplary viruses include, but are not limited to, LIVP viruses that express one or more therapeutic gene products, such as angiogenesis inhibitors (*e.g.,* GLV-1h81, which contains DNA encoding the plasminogen K5 domain (SEQ ID NO: 11) under the control of the vaccinia synthetic early-late promoter in place of the *gusA* expression cassette at the *HA* locus in GLV-1h68; GLV-1h104, GLV-1h105 and GLV-1h106, which contain DNA encoding a truncated human tissue factor fused to the αᵥβ₃-integrin RGD binding motif (tTF-RGD) (SEQ ID NO: 7) under the control of a vaccinia synthetic early promoter, vaccinia synthetic early/late promoter or vaccinia synthetic late promoter, respectively, in place of the *LacZ*/*rTFr* expression cassette at the *TK* locus of GLV-1h68; GLV-1h107, GLV-1h108 and GLV-1h109, which contain DNA encoding an anti-VEGF single chain antibody G6 (SEQ ID NO: 8) under the control of a vaccinia synthetic early promoter, vaccinia synthetic early/late promoter or vaccinia synthetic late promoter, respectively, in place of the *LacZ*/*rTFr* expression cassette at the *TK* locus of GLV-1h68) and proteins for tumor growth suppression (*e.g.,* GLV-1h90, GLV-1h91 and GLV-1h92, which express a fusion protein containing an IL-6 fused to an IL-6 receptor (sIL-6R/IL-6) (SEQ ID NO: 10) under the control of a vaccinia synthetic early promoter, vaccinia synthetic early/late promoter or vaccinia synthetic late promoter, respectively, in place of the *gusA* expression cassette at the *HA* locus in GLV-1h68; and GLV-1h96, GLV-1h97 and GLV-1h98, which express IL-24 (melanoma differentiation gene, mda-7; SEQ ID NO: 9) under the control of a vaccinia synthetic early promoter, vaccinia synthetic early/late promoter or vaccinia synthetic late promoter, respectively, in place of the *Ruc-GFP* fusion gene expression cassette at the *F14.5L* locus of GLV-1h68). Additional therapeutic gene products that can be engineered in the viruses provided herein also are described elsewhere herein.

Exemplary transporter proteins that can be encoded by the viruses provided herein include, for example, the human norepinephrine transporter (hNET; SEQ ID NO: 3 (cDNA), 4 (protein)) and the human sodium iodide symporter (hNIS; SEQ ID NO: 6 (cDNA), 5 (protein)). Exemplary viruses that can be employed in the methods and use provided herein that encode the human norepinephrine transporter (hNET) include, but are not limited to, GLV-1h99, GLV-1h100, GLV-1h101, GLV-1h139, GLV-1h146, GLV-1h150, GLV-1h151 and GLV-1h152. GLV-1h99 encodes hNET under the control of a vaccinia synthetic early promoter in place of the *Ruc-GFP* fusion gene expression cassette at the *F14.5L* locus of GLV-1h68. GLV-1h100, GLV-1h101 encode hNET under the control of a vaccinia synthetic early promoter or vaccinia synthetic late promoter, respectively, in place of the *LacZ*/*rTFr* expression cassette at the *TK* locus of GLV-1h68. GLV-1h39 encodes hNET under the control of a vaccinia synthetic early promoter in place of the *gusA* expression cassette at the *HA* locus in GLV-1h68. GLV-1h146 and GLV-1h150, encode hNET under the control of a vaccinia synthetic early promoter or vaccinia synthetic late promoter, respectively, in place of the *LacZ*/*rTFr* expression cassette at the *TK* locus of GLV-1h100 and GLV-101, respectively. Thus, GLV-1h146 and GLV-1h150 encode both hNET and IL-24. Exemplary viruses that can be employed in the methods and use described herein that encode the human sodium iodide transporter (hNIS) include, but are not limited to, GLV-1h151, GLV-1h151 and GLV-1h153. GLV-1h151, GLV-1h151 and GLV-1h153 encode hNIS under the control of a vaccinia synthetic early promoter, vaccinia synthetic early/late promoter or vaccinia synthetic late promoter, respectively, in place of the *gusA* expression cassette at the *HA* locus in GLV-1h68.

Other exemplary viruses include, but are not limited to, LIVP viruses that encode additional imaging agents such as ferritin and/or a transferrin receptor (*e.g.*, GLV-1h82 and GLV-1h83 which encode E.coli ferritin at the *HA* locus; GLV-1h82 addition encodes the human transferrin receptor at the *TK* locus) or a click beetle luciferase-red fluorescent protein fusion protein (e.g., GLV-1h84, which encodes CBG99 and mRFP1 at the TK locus). During translation, the two proteins are cleaved into two individual proteins at picornavirus 2A element (Osbom et al., Mol. Ther. 12: 569-74, 2005). CBG99 produces a more stable luminescent signal than does *Renilla* luciferase with a half-life of greater than 30 minutes, which makes both *in vitro* and *in vivo* assays more convenient. mRFP1 provides improvements in *in vivo* imaging relative to GFP since mRFP 1 can penetrate tissue deeper than GFP.

### b. Other cytoplasmic viruses

Also described herein are cytoplasmic viruses that are not poxviruses. Cytoplasmic viruses can replicate without introducing viral nucleic acid molecules into the nucleus of the host cell. A variety of such cytoplasmic viruses are known in the art, and include African swine flu family viruses and various RNA viruses such as arenaviruses, picornaviruses, caliciviruses, togaviruses, coronaviruses, paramyxoviruses, flaviviruses, reoviruses, and rhaboviruses. Exemplary togaviruses include Sindbis viruses. Exemplary arenaviruses include lymphocytic choriomeningitis virus. Exemplary rhaboviruses include vesicular stomatitis viruses. Exemplary paramyxoviruses include Newcastle Disease viruses and measles viruses. Exemplary picornaviruses include polio viruses, bovine enteroviruses and rhinoviruses. Exemplary flaviviruses include Yellow fever virus; attenuated Yellow fever viruses are known in the art, as exemplified in Barrett et al. (Biologicals 25: 17-25 (1997)), and McAllister et al. (J. Virol. 74: 9197-9205 (2000)).

Also described herein are modifications of the viruses described above to enhance one or more characteristics relative to the wild type virus. Such characteristics can include, but are not limited to, attenuated pathogenicity, reduced toxicity, preferential accumulation in tumor, increased ability to activate an immune response against tumor cells, increased immunogenicity, increased or decreased replication competence, and are able to express exogenous proteins, and combinations thereof. In some embodiments, the modified viruses have an ability to activate an immune response against tumor cells without aggressively killing the tumor cells. In other embodiments, the viruses can be modified to express one or more detectable genes, including genes that can be used for imaging. In other embodiments, the viruses can be modified to express one or more genes for harvesting the gene products and/or for harvesting antibodies against the gene products.

### c. Adenovirus, Herpes, Retroviruses

Further described herein are viruses that include in their life cycle entry of a nucleic acid molecule into the nucleus of the host cell. A variety of such viruses is known in the art, and includes herpesviruses, papovaviruses, retroviruses, adenoviruses, parvoviruses and orthomyxoviruses. Exemplary herpesviruses include herpes simplex type 1 viruses, cytomegaloviruses, and Epstein-Barr viruses. Exemplary papovaviruses include human papillomavirus and SV40 viruses. Exemplary retroviruses include lentiviruses. Exemplary orthomyxoviruses include influenza viruses. Exemplary parvoviruses include adeno associated viruses.

Also described herein are modifications of the viruses described above to enhance one or more characteristics relative to the wild type virus. Such characteristics can include, but are not limited to, attenuated pathogenicity, reduced toxicity, preferential accumulation in tumor, increased ability to activate an immune response against tumor cells, increased immunogenicity, increased or decreased replication competence, and are able to express exogenous proteins, and combinations thereof. In some embodiments, the modified viruses have an ability to activate an immune response against tumor cells without aggressively killing the tumor cells. In other embodiments, the viruses can be modified to express one or more detectable genes, including genes that can be used for imaging. In other embodiments, the viruses can be modified to express one or more genes for harvesting the gene products and/or for harvesting antibodies against the gene products.

### 4. Chemotherapeutic agents for virus inhibition

The chemotherapeutics agents that can be administered with the viruses described herein to control or maintain viral titer include any chemotherapeutic agent the adversely affects any step in the viral life cycle. For example, the chemotherapeutic agent can inhibit viral replication, inhibit viral toxins, or cause viral death. The chemotherapeutic agent can block one or more steps in the viral life cycle, including, but not limited to, compounds that can inhibit viral DNA replication, viral RNA transcription, viral coat protein assembly, outer membrane or polysaccharide assembly. Typically, the chemotherapeutic agents employed in the methods and uses described herein decrease or inhibit viral replication. Exemplary chemotherapeutic agents that can be used in the methods or uses described include, but are not limited to, cisplatin, gemcitabine, doxorubicin, irinotecan and 5'-fluorouracil.

### D. Monitoring

The methods described herein can further include one or more steps of monitoring the subject, monitoring the tumor, and/or monitoring the virus administered to the subject. Any of a variety of monitoring steps can be included in the methods provided herein, including, but not limited to, monitoring tumor size, monitoring anti-(tumor antigen) antibody titer, monitoring the presence and/or size of metastases, monitoring the subject's lymph nodes, monitoring the subject's weight or other health indicators including blood or urine markers, monitoring anti-(viral antigen) antibody titer, monitoring viral expression of a detectable gene product, and directly monitoring viral titer in a tumor, tissue or organ of a subject.

The purpose of the monitoring can be simply for assessing the health state of the subject or the progress of therapeutic treatment of the subject, or can be for determining whether or not further administration of the same or a different virus is warranted, or for determining when or whether or not to administer a compound to the subject where the compound can act to increase the efficacy of the therapeutic method, or the compound can act to decrease the pathogenicity of the virus administered to the subject.

### 1. Monitoring viral gene expression

In some embodiments, the methods described herein can include monitoring one or more virally expressed genes. Viruses, such as those provided herein or otherwise known in the art, can express one or more detectable gene products, including but not limited to, detectable proteins.

As described herein, measurement of a detectable gene product expressed by a virus can provide an accurate determination of the level of virus present in the subject. As further described herein, measurement of the location of the detectable gene product, for example, by imaging methods including, but not limited to, magnetic resonance, fluorescence, and tomographic methods, can determine the localization of the virus in the subject. Accordingly, the methods described herein that include monitoring a detectable viral gene product can be used to determine the presence or absence of the virus in one or more organs or tissues of a subject, and/or the presence or absence of the virus in a tumor or metastases of a subject. Further, the methods described herein that include monitoring a detectable viral gene product can be used to determine the titer of virus present in one or more organs, tissues, tumors or metastases. Methods that include monitoring the localization and/or titer of viruses in a subject can be used for determining the pathogenicity of a virus; since viral infection, and particularly the level of infection, of normal tissues and organs can indicate the pathogenicity of the probe, methods of monitoring the localization and/or amount of viruses in a subject can be used to determine the pathogenicity of a virus. Since methods described herein can be used to monitor the amount of viruses at any particular location in a subject, the methods that include monitoring the localization and/or titer of viruses in a subject can be performed at multiple time points, and, accordingly can determine the rate of viral replication in a subject, including the rate of viral replication in one or more organs or tissues of a subject; accordingly, the methods of monitoring a viral gene product can be used for determining the replication competence of a virus. The methods described herein also can be used to quantitate the amount of virus present in a variety of organs or tissues, and tumors or metastases, and can thereby indicate the degree of preferential accumulation of the virus in a subject; accordingly, the viral gene product monitoring methods described herein can be used in methods of determining the ability of a virus to accumulate in tumor or metastases in preference to normal tissues or organs. Since the viruses used in the methods described herein can accumulate in an entire tumor or can accumulate at multiple sites in a tumor, and can also accumulate in metastases, the methods described herein for monitoring a viral gene product can be used to determine the size of a tumor or the number of metastases that are present in a subject. Monitoring such presence of viral gene product in tumor or metastasis over a range of time can be used to assess changes in the tumor or metastasis, including growth or shrinking of a tumor, or development of new metastases or disappearance of metastases, and also can be used to determine the rate of growth or shrinking of a tumor, or development of new metastases or disappearance of metastases, or the change in the rate of growth or shrinking of a tumor, or development of new metastases or disappearance of metastases. Accordingly, the methods of monitoring a viral gene product can be used for monitoring a neoplastic disease in a subject, or for determining the efficacy of treatment of a neoplastic disease, by determining rate of growth or shrinking of a tumor, or development of new metastases or disappearance of metastases, or the change in the rate of growth or shrinking of a tumor, or development of new metastases or disappearance of metastases.

Any of a variety of detectable proteins can be detected in the monitoring methods described herein; an exemplary, non-limiting list of such detectable proteins includes any of a variety of fluorescent proteins (*e.g.*, green or red fluorescent proteins), any of a variety of luciferases, transferrin or other iron binding proteins; or receptors, binding proteins, and antibodies, where a compound that specifically binds the receptor, binding protein or antibody can be a detectable agent or can be labeled with a detectable substance (*e.g.*, a radionuclide or imaging agent). Viruses expressing a detectable protein can be detected by a combination of the method provided herein and know in the art. Viruses expressing more than one detectable protein or two or more viruses expressing various detectable protein can be detected and distinguished by dual imaging methods. For example, a virus expressing a fluorescent protein and an iron binding protein can be detected *in vitro* or *in vivo* by low light fluorescence imaging and magnetic resonance, respectively. In another example, a virus expressing two or more fluorescent proteins can be detected by fluorescence imaging at different wavelength. *In vivo* dual imaging can be performed on a subject that has been administered a virus expressing two or more detectable gene products or two or more viruses each expressing one or more detectable gene products.

### 2. Monitoring tumor size

Also described herein are methods of monitoring tumor and/or metastasis size and location. Tumor and or metastasis size can be monitored by any of a variety of methods known in the art, including external assessment methods or tomographic or magnetic imaging methods. In addition to the methods known in the art, methods described herein, for example, monitoring viral gene expression, can be used for monitoring tumor and/or metastasis size.

Monitoring size over several time points can provide information regarding the increase or decrease in size of a tumor or metastasis, and can also provide information regarding the presence of additional tumors and/or metastases in the subject. Monitoring tumor size over several time points can provide information regarding the development of a neoplastic disease in a subject, including the efficacy of treatment of a neoplastic disease in a subject.

### 3. Monitoring antibody titer

The methods described herein also can include monitoring the antibody titer in a subject, including antibodies produced in response to administration of a virus to a subject. The viruses administered in the methods provided herein can elicit an immune response to endogenous viral antigens. The viruses administered in the methods described herein also can elicit an immune response to exogenous genes expressed by a virus. The viruses administered in the methods described herein also can elicit an immune response to tumor antigens. Monitoring antibody titer against viral antigens, viral expressed exogenous gene products, or tumor antigens can be used in methods of monitoring the toxicity of a virus, monitoring the efficacy of treatment methods, or monitoring the level of gene product or antibodies for production and/or harvesting.

In one embodiment, monitoring antibody titer can be used to monitor the toxicity of a virus. Antibody titer against a virus can vary over the time period after administration of the virus to the subject, where at some particular time points, a low anti-(viral antigen) antibody titer can indicate a higher toxicity, while at other time points a high anti-(viral antigen) antibody titer can indicate a higher toxicity. The viruses used in the methods described herein can be immunogenic, and can, therefore, elicit an immune response soon after administering the virus to the subject. Generally, a virus against which a subject's immune system can quickly mount a strong immune response can be a virus that has low toxicity when the subject's immune system can remove the virus from all normal organs or tissues. Thus, in some embodiments, a high antibody titer against viral antigens soon after administering the virus to a subject can indicate low toxicity of a virus. In contrast, a virus that is not highly immunogenic can infect a host organism without eliciting a strong immune response, which can result in a higher toxicity of the virus to the host. Accordingly, in some embodiments, a high antibody titer against viral antigens soon after administering the virus to a subject can indicate low toxicity of a virus.

In other embodiments, monitoring antibody titer can be used to monitor the efficacy of treatment methods. In the methods described herein, antibody titer, such as anti-(tumor antigen) antibody titer, can indicate the efficacy of a therapeutic method such as a therapeutic method to treat neoplastic disease. Therapeutic methods described herein can include causing or enhancing an immune response against a tumor and/or metastasis. Thus, by monitoring the anti-(tumor antigen) antibody titer, it is possible to monitor the efficacy of a therapeutic method in causing or enhancing an immune response against a tumor and/or metastasis. The therapeutic methods described herein also can include administering to a subject a virus that can accumulate in a tumor and can cause or enhance an anti-tumor immune response. Accordingly, it is possible to monitor the ability of a host to mount an immune response against viruses accumulated in a tumor or metastasis, which can indicate that a subject has also mounted an anti-tumor immune response, or can indicate that a subject is likely to mount an anti-tumor immune response, or can indicate that a subject is capable of mounting an anti-tumor immune response.

In other embodiments, monitoring antibody titer can be used for monitoring the level of gene product or antibodies for production and/or harvesting. As described herein, methods can be used for producing proteins, RNA molecules or other compounds by expressing an exogenous gene in a virus that has accumulated in a tumor. Further described herein are methods for producing antibodies against a protein, RNA molecule or other compound produced by exogenous gene expression of a virus that has accumulated in a tumor. Monitoring antibody titer against the protein, RNA molecule or other compound can indicate the level of production of the protein, RNA molecule or other compound by the tumor-accumulated virus, and also can directly indicate the level of antibodies specific for such a protein, RNA molecule or other compound.

### 4. Monitoring general health diagnostics

The methods described herein also can include methods of monitoring the health of a subject. Some of the methods described herein are therapeutic methods, including neoplastic disease therapeutic methods. Monitoring the health of a subject can be used to determine the efficacy of the therapeutic method, as is known in the art. The methods described herein also can include a step of administering to a subject a virus. Monitoring the health of a subject can be used to determine the pathogenicity of a virus administered to a subject. Any of a variety of health diagnostic methods for monitoring disease such as neoplastic disease, infectious disease, or immune-related disease can be monitored, as is known in the art. For example, the weight, blood pressure, pulse, breathing, color, temperature or other observable state of a subject can indicate the health of a subject. In addition, the presence or absence or level of one or more components in a sample from a subject can indicate the health of a subject. Typical samples can include blood and urine samples, where the presence or absence or level of one or more components can be determined by performing, for example, a blood panel or a urine panel diagnostic test. Exemplary components indicative of a subject's health include, but are not limited to, white blood cell count, hematocrit, or reactive protein concentration.

### 5. Monitoring coordinated with treatment

Also described herein are methods of monitoring a therapy, where therapeutic decisions can be based on the results of the monitoring. Therapeutic methods described herein can include administering to a subject a virus, where the virus can preferentially accumulate in a tumor and/or metastasis, and where the virus can cause or enhance an anti-tumor immune response. Such therapeutic methods can include a variety of steps including multiple administrations of a particular virus, administration of a second virus, or administration of a therapeutic compound. Determination of the amount, timing or type of virus or compound to administer to the subject can be based on one or more results from monitoring the subject. For example, the antibody titer in a subject can be used to determine whether or not it is desirable to administer a virus or compound, the quantity of virus or compound to administer, and the type of virus or compound to administer, where, for example, a low antibody titer can indicate the desirability of administering additional virus, a different virus, or a therapeutic compound such as a compound that induces viral gene expression. In another example, the overall health state of a subject can be used to determine whether or not it is desirable to administer a virus or compound, the quantity of virus or compound to administer, and the type of virus or compound to administer, where, for example, determining that the subject is healthy can indicate the desirability of administering additional virus, a different virus, or a therapeutic compound such as a compound that induces viral gene expression. In another example, monitoring a detectable virally expressed gene product can be used to determine whether or not it is desirable to administer a virus or compound, the quantity of virus or compound to administer, and the type of virus or compound to administer. Such monitoring methods can be used to determine whether or not the therapeutic method is effective, whether or not the therapeutic method is pathogenic to the subject, whether or not the virus has accumulated in a tumor or metastasis, and whether or not the virus has accumulated in normal tissues or organs. Based on such determinations, the desirability and form of further therapeutic methods can be derived.

In one embodiment, determination of whether or not a therapeutic method is effective can be used to derive further therapeutic methods. Any of a variety of methods of monitoring can be used to determine whether or not a therapeutic method is effective, as provided herein or otherwise known in the art. If monitoring methods indicate that the therapeutic method is effective, a decision can be made to maintain the current course of therapy, which can include further administrations of a virus or compound, or a decision can be made that no further administrations are required. If monitoring methods indicate that the therapeutic method is ineffective, the monitoring results can indicate whether or not a course of treatment should be discontinued (*e.g.*, when a virus is pathogenic to the subject), or changed (*e.g.*, when a virus accumulates in a tumor without harming the host organism, but without eliciting an anti-tumor immune response), or increased in frequency or amount (*e.g.*, when little or no virus accumulates in tumor).

In one example, monitoring can indicate that a virus is pathogenic to a subject. In such instances, a decision can be made to terminate administration of the virus to the subject, to administer lower levels of the virus to the subject, to administer a different virus to a subject, or to administer to a subject a compound that reduces the pathogenicity of the virus. In one example, administration of a virus that is determined to be pathogenic can be terminated. In another example, the dosage amount of a virus that is determined to be pathogenic can be decreased for subsequent administration; in one version of such an example, the subject can be pre-treated with another virus that can increase the ability of the pathogenic virus to accumulate in tumor, prior to re-administering the pathogenic virus to the subject. In another example, a subject can have administered thereto a virus that is pathogenic to the subject; administration of such a pathogenic virus can be accompanied by administration of, for example, an antiviral compound (*e.g.*, cidofovir), pathogenicity attenuating compound (*e.g.*, a compound that down-regulates the expression of a lytic or apoptotic gene product), or other compound that can decrease the proliferation, toxicity, or cell killing properties of a virus, as described herein elsewhere. In one variation of such an example, the localization of the virus can be monitored, and, upon determination that the virus is accumulated in tumor and/or metastases but not in normal tissues or organs, administration of the antiviral compound or pathogenicity attenuating compound can be terminated, and the pathogenic activity of the virus can be activated or increased, but limited to the tumor and/or metastasis. In another variation of such an example, after terminating administration of the antiviral compound or pathogenicity attenuating compound, the presence of the virus and/or pathogenicity of the virus can be further monitored, and administration of such a compound can be reinitiated if the virus is determined to pose a threat to the host by, for example, spreading to normal organs or tissues, releasing a toxin into the vasculature, or otherwise having pathogenic effects reaching beyond the tumor or metastasis.

In another example, monitoring can determine whether or not a virus has accumulated in a tumor or metastasis of a subject. Upon such a determination, a decision can be made to further administer additional virus, a different virus or a compound to the subject. In another example, monitoring the presence of a virus in a tumor can be used in deciding to administer to the subject a compound, where the compound can increase the pathogenicity, proliferation, or immunogenicity of a virus or the compound can otherwise act in conjunction with the virus to increase the proliferation, toxicity, tumor cell killing, or immune response eliciting properties of a virus; in one variation of such an example, the virus can, for example, have little or no lytic or cell killing capability in the absence of such a compound; in a further variation of such an example, monitoring of the presence of the virus in a tumor or metastasis can be coupled with monitoring the absence of the virus in normal tissues or organs, where the compound is administered if the virus is present in tumor or metastasis and not at all present or substantially not present in normal organs or tissues; in a further variation of such an example, the amount of virus in a tumor or metastasis can be monitored, where the compound is administered if the virus is present in tumor or metastasis at sufficient levels.

### E. PHARMACEUTICAL COMPOSITIONS, COMBINATIONS AND KITS

Described herein are pharmaceutical compositions, combinations and kits containing a virus described herein and one or more components. Pharmaceutical compositions can include a virus described herein and a pharmaceutical carrier. Combinations can include two or more viruses, a virus and a detectable compound, a virus and a viral expression modulating compound, a virus and a therapeutic compound, or any combination thereof. Kits can include the pharmaceutical compositions and/or combinations described herein, and one or more components, such as instructions for use, a device for detecting a virus in a subject, a device for administering a compound to a subject, and a device for administering a compound to a subject.

### 1. Pharmaceutical Compositions

Described herein are pharmaceutical compositions containing a virus described herein and a chemotherapeutic agent and a suitable pharmaceutical carrier. Pharmaceutical compositions described herein can be in various forms, e.g., in solid, liquid, powder, aqueous, or lyophilized form. Suitable pharmaceutical carriers are known in the art. Examples of suitable pharmaceutical carriers are known in the art and include but are not limited to water, buffers, saline solutions, phosphate buffered saline solutions, various types of wetting agents, sterile solutions, alcohols, gum arabic, vegetable oils, benzyl alcohols, gelatin, glycerin, carbohydrates such as lactose, sucrose, amylose or starch, magnesium stearate, talc, silicic acid, viscous paraffin, perfume oil, fatty acid monoglycerides and diglycerides, pentaerythritol fatty acid esters, hydroxy methylcellulose, powders, among others. Pharmaceutical compositions described herein can contain other additives including, for example, antioxidants and preservatives, analgesic agents, binders, disintegrants, coloring, diluents, exipients, extenders, glidants, solubilizers, stabilizers, tonicity agents, vehicles, viscosity agents, flavoring agents, emulsions, such as oil/water emulsions, emulsifying and suspending agents, such as acacia, agar, alginic acid, sodium alginate, bentonite, carbomer, carrageenan, carboxymethylcellulose, cellulose, cholesterol, gelatin, hydroxyethyl cellulose, hydroxypropyl cellulose, hydroxypropyl methylcellulose, methylcellulose, octoxynol 9, oleyl alcohol, povidone, propylene glycol monostearate, sodium lauryl sulfate, sorbitan esters, stearyl alcohol, tragacanth, xanthan gum, and derivatives thereof, solvents, and miscellaneous ingredients such as crystalline cellulose, microcrystalline cellulose, citric acid, dextrin, dextrose, liquid glucose, lactic acid, lactose, magnesium chloride, potassium metaphosphate, starch, among others. Such carriers and/or additives can be formulated by conventional methods and can be administered to the subject at a suitable dose. Stabilizing agents such as lipids, nuclease inhibitors, polymers, and chelating agents can preserve the compositions from degradation within the body.

Colloidal dispersion systems that can be used for delivery of viruses include macromolecule complexes, nanocapsules, microspheres, beads and lipid-based systems including oil-in-water emulsions (mixed), micelles, liposomes and lipoplexes. Organ-specific or cell-specific liposomes can be used in order to achieve delivery only to the desired tissue. The targeting of liposomes can be carried out by the person skilled in the art by applying commonly known methods. In compositions and methods described, monoclonal antibodies can be used to target liposomes to specific tissues, for example, tumor tissue, via specific cell-surface ligands.

### 2. Combinations

Described are combinations of the viruses provided herein and a second agent, such as a second virus or other therapeutic or diagnostic agent. For example the combination can include a composition containing a therapeutic virus, wherein the virus is effective for treatment of cancer and a composition comprising a chemotherapeutic agent in an amount effective for clearing the virus from a subject.

A combination can include any virus or reagent for effecting attenuation thereof in accord with the methods described herein. Combinations can include a virus described herein with one or more additional viruses. Combinations of the viruses described can also contain pharmaceutical compositions containing the viruses or host cells containing the viruses as described herein.

In one embodiment, the virus in a combination is an attenuated virus, such as for example, an attenuated vaccinia virus. Exemplary attenuated viruses include vaccinia viruses provided herein, such as, but not limited to, for example, vaccinia viruses such as: GLV-1h22, GLV-1h68, GLV-1i69, GLV-1h70, GLV-1h71, GLV-1h72, GLV-1h73, GLV-1h74, GLV-1h81, GLV-1h82, GLV-1h83, GLV-1h84, GLV-1h85, GLV-1h86, GLV-1h90, GLV-1h91, GLV-1h92, GLV-1h96, GLV-1h97, GLV-1h98, GLV-1h99, GLV-1h100, GLV-1h101, GLV-1h104, GLV-1h105, GLV-1h106, GLV-1h107, GLV-1h108, GLV-1h109, GLV-1h139, GLV-1h146, GLV-1h150 GLV-1h151, GLV-1h152 or GLV-1h153.

Combinations described herein can contain a virus and a therapeutic compound. Therapeutic compounds for the compositions described herein can be, for example, an anti-cancer or chemotherapeutic compound. Exemplary therapeutic compounds include, for example, cytokines, growth factors, photosensitizing agents, radionuclides, toxins, siRNA molecules, enzyme/pro-drug pairs, anti-metabolites, signaling modulators, anti-cancer antibiotics, anti-cancer antibodies, angiogenesis inhibitors, chemotherapeutic compounds, or a combination thereof. Viruses described herein can be combined with a chemotherapeutic agent that can kill or inhibit viral growth or toxicity in addition to enhancing the therapeutic effect of the combination itself. Exemplary chemotherapeutic agents include, but are not limited to, methotrexate, vincristine, adriamycin, non-sugar containing chloroethylnitrosoureas, 5-fluorouracil, mitomycin C, bleomycin, doxorubicin, dacarbazine, taxol, fragyline, Meglamine GLA, valrubicin, carmustine and polifeprosan, MM1270, BAY 12-9566, RAS farnesyl transferase inhibitor, farnesyl transferase inhibitor, MMP, MTA/LY231514, LY264618/Lometrexol, Glamolec, CI-994, TNP-470, Hycamtin/Topotecan, PKC412, Valspodar/PSC833, Novantrone/Mitroxantrone, Metaret/Suramin, Batimastat, E7070, BCH-4556, CS-682, 9-AC, AG3340, AG3433, Incel/VX-710, VX-853, ZD0101, IS1641, ODN 698, TA 2516/Marmistat, BB2516/Marmistat, CDP 845, D2163, PD183805, DX8951f, Lemonal DP 2202, FK 317, Picibanil/OK-432, AD 32/Valrubicin, Metastron/strontium derivative, Temodal/Temozolomide, Evacet/liposomal doxorubicin, Yewtaxan/Paclitaxel, Taxol®/Paclitaxel, Xeloda/Capecitabine, Furtulon/Doxifluridine, Cyclopax/oral paclitaxel, Oral Taxoid, SPU-077/Cisplatin, HMR 1275/Flavopiridol, CP-358 (774)/EGFR, CP-609 (754)/RAS oncogene inhibitor, BMS-182751/oral platinum, UFT(Tegafur/Uracil), Ergamisol/Levamisole, Eniluracil/776C85/5FU enhancer, Campto/Levamisole, Camptosar/Irinotecan, Tumodex/Ralitrexed, Leustatin/Cladribine, Paxex/Paclitaxel, Doxil/liposomal doxorubicin, Caelyx/liposomal doxorubicin, Fludara®/Fludarabine, Pharmarubicin/Epirubicin, DepoCyt, ZD1839, LU 79553/Bis-Naphtalimide, LU 103793/Dolastain, Gemzar/Gemcitabine, ZD 0473/Anormed, YM 116, Iodine seeds, CDK4 and CDK2 inhibitors, PARP inhibitors, D4809/Dexifosamide, Ifes/Mesnex/Ifosfamide, Vumon®/Teniposide, Paraplatin/Carboplatin, Plantinol/cisplatin, Vepeside/Etoposide, ZD 9331, Taxotere/Docetaxel, prodrug of guanine arabinoside, Taxane Analog, nitrosoureas, alkylating agents such as melphelan and cyclophosphamide, Aminoglutethimide, Anastrozole, Asparaginase, Busulfan, Carboplatin, Chlorombucil, Cladribine, Cytaribine HCl, Dactinomycin, Daunorubicin HCl, Denileukin diftitox, Estramustine phosphate sodium, Etoposide (VP16-213), Exemestane, Floxuridine, Fluorouracil (5-FU®), Flutamide, Hydroxyurea (hydroxycarbamide), Interferon Alfa-2a, Interferon Alfa-2b, Interferon Gamma-1b, Letrozole, Leuprolide acetate (LHRH-releasing factor analogue), Lomustine (CCNU), Mechlorethamine HCl (nitrogen mustard), Megestrol, Mercaptopurine, Mesna, Mitotane (o.p'-DDD), Mitoxantrone HCl, Octreotide, Pegaspargase, Plicamycin, Procarbazine HCl, Streptozocin, Tamoxifen citrate, Thioguanine, Thiotepa, Tretinoin, Vinblastine sulfate, Amsacrine (m-AMSA), Azacitidine, Erythropoietin, Hexamethylmelamine (HMM), Interleukin 2, Mitoguazone (methyl-GAG; methyl glyoxal bis-guanylhydrazone; MGBG), Pentostatin (2'deoxycoformycin), Semustine (methyl-CCNU), Teniposide (VM-26®), Vindesine sulfate, Altretamine, Carmustine, Chlorambucil, Estramustine, Gemtuzumab ozogamicin, Idarubicin, Isotretinoin, Leuprolide, Melphalan, Mitoxantrone, Pipobroman, Procarbazine, Testolactone, Uracil mustard, Vinblastine and Vinorelbine.

In a further embodiment, the combination can include additional therapeutic compounds such as, for example, compounds that are substrates for enzymes encoded and expressed by the virus, or other therapeutic compounds provided herein or known in the art to act in concert with a virus. For example, the virus can express an enzyme that converts a prodrug into an active chemotherapy drug for killing the cancer cell. Hence, combinations provided herein can contain therapeutic compounds, such as prodrugs. Any of a variety of known combinations known in the art can be included in the combinations provided herein.

In a further embodiment, combinations can include other compounds other than chemotherapeutic agents that can kill or inhibit viral growth or toxicity. Combinations provided herein can contain antibiotic, antifungal, anti-parasitic or antiviral compounds for treatment of infections. Exemplary antibiotics which can be included in a combination with a virus provided herein include, but are not limited to, ceftazidime, cefepime, imipenem, aminoglycoside, vancomycin, and antipseudomonal β-lactam. Exemplary antifungal agents which can be included in a combination with a virus provided herein include, but are not limited to, amphotericin B, dapsone, fluconazole, flucytosine, griseofluvin, intraconazole, ketoconazole, miconazole, clotrimazole, nystatin, and combinations thereof. Exemplary antiviral agents can be included in a combination with a virus provided herein include, but are not limited to, cidofovir, alkoxyalkyl esters of cidofovir (CDV), gancyclovir, cyclic CDV, and (S)-9-(3-hydroxy-2 phosphonylmethoxypropyl)adenine, 5-(Dimethoxymethyl)-2'-deoxyuridine, isatin-beta-thiosemicarbazone, N-methanocarbathymidine, brivudin, 7-deazaneplanocin A, ST-246, Gleevec, 2'-beta-fluoro-2',3'-dideoxyadenosine, indinavir, nelfinavir, ritonavir, nevirapine, AZT, ddI, ddC, vaccinia immunoglobulin, interferon beta-1a, interferon beta 1-b and combinations thereof. Typically, combinations with an antiviral agent contain an antiviral agent known to be effective against the virus of the combination. For example, combinations can contain a vaccinia virus with an antiviral compound, such as cidofovir, alkoxyalkyl esters of cidofovir, gancyclovir, acyclovir, ST-246, and Gleevec.

In another embodiment, the combination can further include a virus gene expression modulating compound. Compounds that modulate gene expression are known in the art, and include, but are not limited to, transcriptional activators, inducers, transcriptional suppressors, RNA polymerase inhibitors, and RNA binding compounds such as siRNA or ribozymes. Any of a variety of gene expression modulating compounds known in the art can be included in the combinations provided herein. Typically, the gene expression modulating compound included with a virus in the combinations provided herein will be a compound that can bind, inhibit, or react with one or more compounds, active in gene expression such as a transcription factor or RNA of the virus of the combination. A variety of other virus/expression modulator combinations known in the art can be included in the combinations described herein.

In a further embodiment, combination can further contain nanoparticles. Nanoparticles can be designed such that they carry one or more therapeutic agents provided herein. Additionally, nanoparticles can be designed to carry a molecule that targets the nanoparticle to the tumor cells.

### 3. Kits

The viruses, cells, pharmaceutical compositions, or combinations described herein can be packaged as kits. Kits can optionally include one or more components such as instructions for use, devices, and additional reagents, and components, such as tubes, containers and syringes for practice of the methods. Exemplary kits can include the viruses described herein, and can optionally include instructions for use, a device for detecting a virus in a subject, a device for administering the virus to a subject, and a device for administering a compound to a subject.

In one example, a kit can contain instructions. Instructions typically include a tangible expression describing the virus and, optionally, other components included in the kit, and methods for administration, including methods for determining the proper state of the subject, the proper dosage amount, and the proper administration method, for administering the virus. Instructions can also include guidance for monitoring the subject over the duration of the treatment time.

In another example, a kit can contain a device for detecting a virus in a subject. Devices for detecting a virus in a subject can include a low light imaging device for detecting light, for example, emitted from luciferase, or fluoresced from fluorescent protein, such as a green or red fluorescent protein, a magnetic resonance measuring device such as an MRI or NMR device, a tomographic scanner, such as a PET, CT, CAT, SPECT or other related scanner, an ultrasound device, or other device that can be used to detect a protein expressed by the virus within the subject. Typically, the device of the kit will be able to detect one or more proteins expressed by the virus of the kit. Any of a variety of kits containing viruses and detection devices can be included in the kits described herein, for example, a virus expressing luciferase and a low light imager, or a virus expressing fluorescent protein, such as a green or red fluorescent protein, and a low light imager.

Kits described herein also can include a device for administering a virus to a subject. Any of a variety of devices known in the art for administering medications or vaccines can be included in the kits provided herein. Exemplary devices include, but are not limited to, a hypodermic needle, an intravenous needle, a catheter, a needle-less injection device, an inhaler, and a liquid dispenser, such as an eyedropper. Typically, the device for administering a virus of the kit will be compatible with the virus of the kit; for example, a needle-less injection device such as a high pressure injection device can be included in kits with viruses not damaged by high pressure injection, but is typically not included in kits with viruses damaged by high pressure injection.

Kits described herein also can include a device for administering a compound to a subject. Any of a variety of devices known in the art for administering medications to a subject can be included in the kits provided herein. Exemplary devices include a hypodermic needle, an intravenous needle, a catheter, a needle-less injection, but are not limited to, a hypodermic needle, an intravenous needle, a catheter, a needle-less injection device, an inhaler, and a liquid dispenser such as an eyedropper. Typically the device for administering the compound of the kit will be compatible with the desired method of administration of the compound. For example, a compound to be delivered subcutaneously can be included in a kit with a hypodermic needle and syringe.

### G. EXAMPLES

The following examples are offered for illustrative purposes only, and are not intended to limit the scope of the present invention in any way. Various modifications of the invention in addition to those shown and described herein will be apparent to those skilled in the art from the foregoing description.

### EXAMPLE 1

### EFFECT OF GEMCITABINE ON ABILITY OF MODIFIED VACCINIA VIRUSES TO ARREST OR REVERSE in vivo LUNG TUMOR GROWTH

The *in vivo* effect of gemcitabine on tumor growth inhibition by modified vaccinia virus strain GLV-1h68 was evaluated using a mouse model of lung cancer. Tumors were established in nude mice by subcutaneously (s.c.) injecting A549 lung carcinoma cells into male nude mice [s.c. on right lateral thigh; 5 × 10⁶ cells; ATCC# CCL-185CRL]. Eight mice [Hsd:Athymic Nude-*Foxn1*^{nu}; Harlan, Indianapolis, IN] were tested for each treatment. On day 23 after A549 cell implantation, viruses at a dose of 5 x 10⁶ PFU/mouse were injected intravenously (i.v.) into the femoral vein. At 7, 10, 13, 16, and 19 days after virus injection, Gemcitabine [Gemzar®, Eli Lilly and Company, at 50 mg/kg or 100 mg/kg] was injected intraperitoneally (i.p.). Median tumor volume (mm³) was measured at 22, 30, 37, 44, 51, 58, and 65 days post-tumor cell implantation. Results are provided in Table 1.

Gemcitabine alone at both 50 mg/kg and 100 mg/kg doses did not cause any inhibition of tumor growth in the mice. At both doses, the tumors actually exhibited increased size relative to the no treatment control group. In contrast, gemcitabine, when administered in combination with GLV-1h69, was able to enhance the slowing of tumor growth and tumor shrinkage effect of GLV-1 h68 at earlier time points following administration of the virus (compare *e.g.*, day 37 post-tumor cell implantation onward). The combination of 100 mg/kg gemcitabine with GLV-1h68 was not as potent in enhancing slowing of tumor growth and tumor shrinkage compared to the combination of 50 mg/kg gemcitabine with GLV-1h68 (see, *e.g.*, data for day 37 to day 58 post-tumor cell implantation, demonstrating that the lower dose of gemcitabine exhibited a more potent effect in the combination therapy). This indicates that higher doses of gemcitabine can have an adverse effect on virus itself.

**Table 1. Effect of gemcitabine and/or GLV-1h68 virus on A459 tumor cell growth**

| **Days Post-implantation** | **Median tumor volume (mm³)** | | | | | |
|---|---|---|---|---|---|---|
| | **No treatment** | **GLV-1h68** | **Gemcitabine 50 mg/kg** | **GLV-1h68 + Gemcitabine 50 mg/kg** | **Gemcitabine 100 mg/kg** | **GLV-1h68** + **Gemcitabine 100 mg/kg** |
| **22** | 226.5 | 303.8 | 245.6 | 234.0 | 228.1 | 251.9 |
| **30** | 477.4 | 677.2 | 579.8 | 565.7 | 487.4 | 599.1 |
| **37** | 557.1 | 1031.0 | 745.3 | 725.5 | 693.6 | 906.5 |
| **44** | 870.0 | 885.7 | 1023.9 | 544.4 | 1046.0 | 796.8 |
| **51** | 1442.7 | 902.0 | 1229.7 | 485.6 | 1580.2 | 616.3 |
| **58** | 1520.4 | 456.8 | 1619.0 | 336.9 | 2216.6 | 444.9 |
| **65** | 2168.7 | 262.1 | 2852.0 | 393.5 | 3413.4 | 424.1 |

### EXAMPLE 2

### EFFECT OF GEMCITABINE ON ABILITY OF MODIFIED VACCINIA VIRUSES TO ARREST OR REVERSE in vivo PANCREATIC TUMOR GROWTH

The *in vivo* effect of gemcitabine on tumor growth inhibition by modified vaccinia virus strain GLV-1h68 was evaluated using a mouse model of pancreatic cancer. Tumors were established in nude mice by subcutaneously (s.c.) injecting PANC-1 human pancreatic carcinoma cells into the right lateral thigh of male nude mice [5 × 10⁶ cells; ATCC# CRL-1469; n=3-8 mice/group, Hsd:Athymic Nude-*Foxn1*^{nu}*;* Harlan, Indianapolis, IN]. On day 28 after PANC-1 cell implantation, viruses at a dose of 5 x 10⁶ PFU/mouse were injected intravenously (i.v.) into the tail vein. At 7, 10, 13, 16, and 19 days after virus injection, Gemcitabine [Gemzar®, Eli Lilly and Company, at 50 mg/kg or 100 mg/kg] was injected intraperitoneally (i.p.). Median tumor volume (mm³) was measured at 28, 35, 42, 50, 56, 63, 71 and 79 days post-tumor cell implantation. Results are provided in Table 2.

Gemcitabine alone was observed to decrease tumor growth in the mice at both 50 mg/kg and 100 mg/kg doses to some extent relative to the no treatment control. By contrast, gemcitabine, when administered in combination with GLV-1h68, was able to enhance the slowing of tumor growth and tumor shrinkage of GLV-1h68 at earlier time points following administration of the virus (compare *e.g.*, day 37 post-tumor cell implantation onward). The combination of 100 mg/kg gemcitabine with GLV-1h68 was not as potent in enhancing slowing of tumor growth and tumor shrinkage compared to the combination of 50 mg/kg gemcitabine with GLV-1h68 (see, *e.g.*, data for day 42 to day 71 post-tumor cell implantation, demonstrating that the lower dose of gemcitabine exhibited a more potent effect in the combination therapy). This indicates that higher doses of gemcitabine can have an adverse effect on virus itself.

**Table 2. Effect of gemcitabine and/or GLV-1h68 virus on PANC-1 tumor cell growth**

| **Days Post-implantation** | **Median tumor volume (mm³)** | | | | | |
|---|---|---|---|---|---|---|
| | **No treatment** | **GLV-1h68** | **Gemcitabine 50 mg/kg** | **GLV-1h68 + Gemcitabine 50 mg/kg** | **Gemcitabine 100 mg/kg** | **GLV-1h68 + Gemcitabine 100 mg/kg** |
| **28** | 281.6 | 231.7 | 268.1 | 226.2 | 209.7 | 227.8 |
| **35** | 395.4 | 425.9 | 359.2 | 408.6 | 417.8 | 401.3 |
| **42** | 616.7 | 724.5 | 543.6 | 592.3 | 652.5 | 662.5 |
| **50** | 1114.6 | 845.1 | 584.8 | 513.2 | 810.3 | 569.7 |
| **56** | 1146.5 | 776.3 | 682.9 | 510.1 | 655.6 | 554.1 |
| **63** | 1446.1 | 547.9 | 654.7 | 372.5 | 407.3 | 508.8 |
| **71** | 2074.6 | 376.3 | 1006.2 | 257.9 | 789.1 | 281.6 |
| **79** | 2907.7 | 268.2 | 1534.1 | 257.7 | 1437.0 | 242.4 |

### EXAMPLE 3

### EFFECT OF GEMCITABINE ON VACCINIA VIRUS REPLICATION IN BREAST TUMOR CELLS

A chemotherapeutic agent administered to a subject to whom a virus is administered for tumor treatment can have an effect on the virus itself. Therefore, the effect of the chemotherapeutic agent gemcitabine on virus yield of the recombinant vaccinia virus GLV-1h68 was assessed *in vitro* by infection of human breast tumor GI-101A cells (derived from GI-101, a human ductal adenocarcinoma cell line (Rathinavelu et al. (1999) Cancer Biochem. Biophys 17:133-146, which is incorporated herein by reference in its entirety). GI-101A cells were cultured in RPMI 1640 with 20% fetal bovine serum (FBS, Mediatech, Inc., Herndon, VA), 1% antibiotic-antimycotic solution, 10 mM HEPES, 1% sodium pyruvate, 5 ng/ml of β-estradiol (Sigma, St. Louis, MO), and 5 ng/ml of progesterone (Sigma, St. Louis, MO). African green monkey kidney fibroblast cells, CV-1 (CCL-70; ATCC, Manassas, VA), were used to titrate virus and were grown in DMEM (Mediatech, Inc., Herndon, VA) with 10% FBS (Mediatech, Inc., Herndon, VA). All cell lines were maintained at 37°C with 5% CO₂ in a humidified incubator.

GI-101A cells were plated in 6-well plates. The cells were then infected with GLV-1h68 at a multiplicity of infection (m.o.i.) of 0.01 for 1 hour at 37°C in the presence of gemcitabine [Gemzar®, Eli Lilly and Company] at the following concentrations: 0, 0.01, 0.1, 1, 10, 100 and 1000 µM. The inoculum was removed by aspiration, and the cell monolayers were washed twice with 2 ml of DPBS (Mediatech, Inc., Herndon, VA). Two (2) ml of cell culture medium containing 2% FBS with corresponding amount of gemcitabine were added into each well. Three wells of each concentration were harvested at 48 hours post infection. The harvested cells were subjected to three cycles of freeze-thaw and sonicated three times for 1 minute at full power (Bronson sonicator) prior to titration. The virus was titrated in CV-1 cells in duplicate.

The number of plaque forming units (PFU) per 10⁶ cells decreased with increasing gemcitabine concentration. At a concentration of 0.01 µM, gemcitabine did not significantly affect GLV-1h68 growth in GI-101A cells. However, at a concentration of 0.1 µM, gemcitabine significantly inhibited the virus growth. The growth of GLV-1 h68 was completely inhibited at concentration of 1.0 µM and above. Gemcitabine thus appears to inhibit vaccinia viral replication in breast tumor cells.

**Table 3. Effect of gemcitabine on viral replication in GI-101A breast cancer tumor cells**

| **Gemcitabine Concentration (µM)** | **Virus Titer** |
|---|---|
| 0 | 8.02 x 10⁶ ± 1.34 x 10⁶ |
| 0.01 | 7.38 x 10⁶ ± 2.00 x 10⁶ |
| 0.1 | 7.12 x 10⁶ ± 2.53 x 10⁵ |
| 1 | 0 |
| 10 | 0 |
| 100 | 0 |
| 1000 | 0 |

### EXAMPLE 4

### EFFECT OF IRINOTECAN ON THE ABILITY OF MODIFIED VACCINIA VIRUSES TO ARREST OR REVERSE in vivo BREAST TUMOR GROWTH

The *in vivo* effect of irinotecan on tumor growth inhibition by modified vaccinia virus strain GLV-1h68 was evaluated using a mouse model of breast cancer. Tumors were established in nude mice by subcutaneously (s.c.) injecting GI-101A. human breast carcinoma cells into four groups of female nude mice (Hsd:Athymic Nude-*Foxn1*^{nu}; Harlan, Indianapolis, IN) (n = 6) [s.c. on the right lateral thigh; 5 × 10⁶ cells; GI-101A cells: Rumbaugh-Goodwin Institute for Cancer Research Inc. Plantation, FL; U.S. Pat. No. 5,693,533]. Twenty two days following tumor cell implantation, two groups of mice (n = 6) were injected intravenously [in 100 µl of PBS, through femoral vein under anesthesia] with 5 × 10⁶ PFU of GLV-1h68. Four doses of irinotecan at 80mg/kg were administered by intraperitoneal route beginning 2 weeks after virus injection. The irinotecan doses were administered once weekly. Tumor volume (mm³) was measured at different time points post-cancer cell injection. Results of median tumor volume are provided in Table 4.

Irinotecan alone actually increased tumor growth in the mice. In contrast, irinotecan was able to enhance the slowing of tumor growth and tumor shrinkage effect of GLV-1h68 when administered in the combination with GLV-1h68 relative to administration of GLV-1h68 alone (compare *e.g.,* day 42 post-tumor cell implantation onward).

**Table 4. Effect of irinotecan on viral replication in GI-101A breast cancer tumor cells**

| **Days post-implantation of tumor cells** | **Median tumor volume (mm³)** | | | |
|---|---|---|---|---|
| | **No Treatment** | **GLV-1h68** | **Irinotecan** | **GLV-1h68 + Irinotecan** |
| **22** | 244.5 | 166 | 184 | 219.45 |
| **32** | 594.9 | 604.3 | 458.45 | 546 |
| **36** | 695.85 | 649.3 | 546.2 | 734.55 |
| **42** | 951.55 | 1002 | 668.75 | 921.25 |
| **49** | 1261.05 | 1200.8 | 712.9 | 973.35 |
| **59** | 1844.7 | 1694 | 831.8 | 901.55 |
| **66** | nd | 2049.2 | 1011.8 | 1003.25 |
| **80** | nd | 2608.4 | 1502.15 | 817.45 |
| **86** | nd | 2296.8 | 1678.4 | 684.8 |
| **99** | nd | 1647.5 | 2712.55 | 526.15 |
| **108** | nd | nd | nd | 429.25 |
| **116** | nd | nd | nd | 337.65 |

### EXAMPLE 5

### EFFECT OF IRINOTECAN ON IMPROVING GENERAL HEALTH IN SUBJECTS TREATED BY MODIFIED VACCINIA VIRUS

Body weight can be used as an indicator of patient health. In mouse models of cancer, decreasing body weight is typically associated with stress and decline in health. The percent of body weight change following intravenous administration of modified vaccinia virus strain GLV-1h68 with and without irinotecan was examined to determine what effect co-administration of irinotecan with virus would have on the mouse models of breast cancer (Example 4). Net body weight was calculated by subtracting the estimated tumor weight from gross body weight for each mouse. Results of net body weight change are provided in Table 5.

It was observed that mice treated with the combination of virus with irinotecan experienced a greater change in net body weight relative to those treated with virus alone. These results suggest that the combination of modified vaccinia virus with irinotecan imposes less physiological stress on the overall health of the mice compared to that imposed by the administration of virus alone. As a result, in addition to producing a more potent therapeutic effect, the combination of virus and irinotecan can help minimize toxic side effects that can result from viral administration.

**Table 5. Net body weight of mice injected with in GI-101A breast cancer tumor cells during administration of cancer treatment in the absence and presence of GLV-1h68 virus and irinotecan.**

| **Days post-implantation of tumor cells** | **Net Median Body Weight Change (%)** | | | |
|---|---|---|---|---|
| | **No Treatment** | **GLV-1h68** | **Irinotecan** | **GLV-1h68 + Irinotecan** |
| **22** | 0 | 0 | 0 | 0 |
| **32** | 4.87 | 0.92 | 2.37 | 5.02 |
| **36** | 6.43 | 9.22 | 4.09 | 8.7 |
| **42** | 10.64 | 9.22 | 2.58 | 6.62 |
| **49** | 13.08 | 12.9 | 6.67 | 8.7 |
| **59** | 19.07 | 11.98 | 13.98 | 20.78 |
| **66** | nd | 12.9 | 12.69 | 17.35 |
| **80** | nd | 11.98 | 15.27 | 16.21 |
| **86** | nd | 14.75 | 16.77 | 15.07 |
| **99** | nd | 8.76 | 17.85 | 14.38 |
| **108** | nd | nd | nd | 16.44 |
| **116** | nd | nd | nd | 12.56 |

### EXAMPLE 6

### EFFECT OF IRINOTECAN ON VACCINIA VIRUS REPLICATION IN BREAST TUMOR CELLS

The effect of the chemotherapeutic agent irinotecan on virus yield of the recombinant vaccinia virus GLV-1h68 was assessed *in vitro* by infection of human breast tumor GI-101A cells. GI-101A cells were cultured as described in Example 3. African green monkey kidney fibroblast cells, CV-1 (CCL-70; ATCC, Manassas, VA), were used to titrate virus and were also grown as described in Example 3. All cell lines were maintained at 37°C with 5% CO₂ in a humidified incubator.

GI-101A cells in 6-well plates were infected with GLV-1h68 at a multiplicity of infection (m.o.i) of 0.01 for 1h at 37°C in presence of irinotecan at the following concentrations: 0, 0.01, 0.1, 1.0, 10, 100, and 1000 µM. The inoculum was aspirated and the cell monolayers were washed twice with 2 ml of DPBS (Mediatech, Inc., Herndon, VA). Two ml of cell culture medium containing 2% FBS with corresponding amount of cisplatin were added into each well. Three wells of each concentration were harvested at 48 hours post infection. The harvested cells were subject to three cycles of freeze-thaw and were sonicated thrice for 1. minute at full power prior to titration. The virus was titrated in CV-1 cells in duplicate.

At the concentration of up to 10 µM, irinotecan did not show any inhibitory effect on the growth of GLV-1h68 virus in GI-101A cells. However, the growth of GLV-1h68 was significantly inhibited at the concentration of 100 µM, and completely inhibited at 1000 uM. (See Table 6) The cytotoxic effect of irinotecan to GI-101A cells can contribute to the viral replication inhibition observed at higher concentrations. Nevertheless, the results indicate that irinotecan can inhibit vaccinia viral replication in breast tumor cells.

**Table 6. Effect of irinotecan on viral replication in GI-101A breast cancer tumor cells**

| **Irinotecan Concentration (µM)** | **Virus Titer** |
|---|---|
| 0 | 2.03 x 10⁵ ± 2.73 x 10⁴ |
| 0.01 | 3.95 x 10⁵ ± 2.79 x 10⁵ |
| 0.1 | 5.50 x 10⁵ ± 3.83 x 10⁵ |
| 1 | 3.18 x 10⁵ ± 1.29 x 10⁵ |
| 10 | 4.00 x 10⁵ ± 3.64 x 10⁵ |
| 100 | 5.31 x 10⁴ ± 1.11 x 10⁴ |
| 1000 | 1.23 x 10² ± 43.7 |

### EXAMPLE 7

### EFFECT OF IRINOTECAN ON PREVENTING SYSTEMIC TOXICITY INDUCED BY ADMINISTRATION OF MODIFIED VACCINIA VIRUSES FOR CANCER TREATMENT

Subjects treated with vaccinia viral therapy often suffer from adverse effects of viral therapy. Exemplary side effects include the formation of pocks in the tails, paws, face, ear tag wounds and other areas of the body surface in immunocompromised hosts, such as in nude mice. Pock formation may start any time from between one week to months after virus injection, and the timing of pock formation is dependent upon the dose of virus delivered, the delivery route, subject body temperature at the time of virus injection, and the immune status of the recipient.

It was observed that when 5x10⁶ PFU of GLV-1h68 was delivered through femoral vein in mice injected subcutanously with GI-101A tumor cells (n=8), 7 out of 8 animals developed pocks in tails, paws, face and/or other areas 80 days after virus injection. Variations in the timing of pock formation were observed in the group. In contrast, four doses of irinotecan (80mg/kg, by intraperitoneal route, beginning 2 weeks after virus injection, one week apart between each dose administered) were delivered to GLV-1h68 virus-treated animals (n=8), and no pocks were observed in 7 out of 8 animals on any part of the body 80 days after virus injection. The exception was in one animal with an ear tag wound.

Thus, irinotecan appears to prevent systemic toxicity (evaluated by pock formation of virus-infected subjects) of the intravenously injected vaccinia virus. In addition to the synergistic antitumor effect observed in Example 4, irinotecan also appears to provide an overall benefit in limiting symptoms associated with systemic toxicity of the virus.

### EXAMPLE 8

### EFFECT OF DOXORUBICIN ON VACCINIA VIRUS REPLICATION IN BREAST TUMOR CELLS

The effect of the chemotherapeutic agent doxorubicin on virus yield of the recombinant vaccinia virus GLV-1h68 was assessed *in vitro* by infection of human breast tumor GI-101A cells. GI-101A cells were cultured as described in Example 3. African green monkey kidney fibroblast cells, CV-1 (CCL-70; ATCC, Manassas, VA), were used to titrate virus and were also grown as described in Example 3. All cell lines were maintained at 37°C with 5% CO₂ in a humidified incubator.

GI-101A cells in 6-well plates were infected with GLV-1h68 at a multiplicity of infection (m.o.i) of 0.01 for 1h at 37°C in presence of doxorubicin at the following concentrations: 0, 0.01, 0.1, 1.0, 10, 100, and 1000 µM. The inoculum was aspirated and the cell monolayers were washed twice with 2 ml of DPBS (Mediatech, Inc., Herndon, VA). Two ml of cell culture medium containing 2% FBS with corresponding amount of doxorubicin were added into each well. Three wells of each concentration were harvested at 48 hours post infection. The harvested cells were subject to three cycles of freeze-thaw and were sonicated thrice for 1 minute at full power prior to titration. The virus was titrated in CV-1 cells in duplicate.

At the concentration of 0.1 µM, doxorubicin started to inhibit GLV-1h68 virus growth in GI-101A cells. The growth of GLV-1h68 was completely inhibited at the concentration of 1 µM and above. (See Table 7.) The results indicate that doxorubicin can inhibit vaccinia viral replication in breast tumor cells.

**Table 7. Effect of doxorubicin on viral replication in GI-101A breast cancer tumor cells**

| **Doxorubicin Concentration (µM)** | **Virus Titer** |
|---|---|
| 0 | 2.4 x 10⁶ ± 6.9 x 10⁵ |
| 0.01 | 3.1 x 10⁶ ± 1.1 x 10⁶ |
| 0.1 | 1.4 x 10⁶ ± 9.4 x 10⁵ |
| 1 | 0 |
| 10 | 0 |
| 100 | 0 |
| 1000 | 0 |

### EXAMPLE 9

### EFFECT OF CISPLATIN ON VACCINIA VIRUS REPLICATION IN BREAST TUMOR CELLS

The effect of the chemotherapeutic agent cisplatin on virus yield of the recombinant vaccinia virus GLV-1h68 was assessed *in vitro* by infection of human breast tumor GI-101A cells. GI-101A cells were cultured as described'in Example 3. African green monkey kidney fibroblast cells, CV-1 (CCL-70; ATCC, Manassas, VA), were used to titrate virus and were also grown as described in Example 3. All cell lines were maintained at 37°C with 5% CO₂ in a humidified incubator

GI-101A cells in 6-well plates were infected with GLV-1h68 at a multiplicity of infection (m.o.i) of 0.01 for 1h at 37°C in presence of cisplatin at the following concentrations: 0, 0.01, 0.1, 1.0, 10, 100, and 1000 µM. The inoculum was aspirated and the cell monolayers were washed twice with 2 ml of DPBS (Mediatech, Inc., Herndon, VA). Two ml of cell culture medium containing 2% FBS with corresponding amount of cisplatin were added into each well. Three wells of each concentration were harvested at 48 hours post infection. The harvested cells were subject to three cycles of freeze-thaw and were sonicated thrice for 1 minute at full power prior to titration. The virus was titrated in CV-1 cells in duplicate.

At a concentration of 1 µM, cisplatin started to inhibit GLV-1h68 growth in GI-101A cells. At a concentration of 10 µM, cisplatin significantly inhibited the virus growth. The growth of GLV-1h68 was completely inhibited at the concentration of 100 µM and above. (See Table 8.) The cytotoxic effect of cisplatin to GI-101A cells can contribute to the viral replication inhibition at higher concentrations. Nevertheless, the results indicate that cisplatin can inhibit vaccinia viral replication in breast tumor cells.

**Table 8. Effect of cisplatin on viral replication in GI-101A breast cancer tumor cells**

| **Cisplatin Concentration (µM)** | **Virus Titer** |
|---|---|
| 0 | 9.1 x 10⁶ ± 5.5 x 10⁶ |
| 0.01 | 7.6 x 10⁶ ± 3.6 x 10⁶ |
| 0.1 | 1.1 x 10⁷ ± 6.1 x 10⁶ |
| 1 | 3.4 x 10⁶ ± 2.2 x 10⁶ |
| 10 | 1.6 x 10⁵ ± 1.6 x 10⁵ |
| 100 | 0 |
| 1000 | 0 |

### EXAMPLE 10

### EFFECT OF GEMCITABINE ON VIRAL LOAD IN A PATIENT

The chemotherapeutic agent gemcitabine is administered to a subject to whom a modified vaccinia virus, such as, for example, GLV-1h68, is administered for tumor treatment. The patient is injected with a virus at a level effective for arrest and/or shrinkage of tumor growth. Gemcitabine is subsequently administered by intravenous infusion once a week for up to seven weeks to the patient at a dose of 8.1 mg/kg (conversion calculated according to PDA's Guidance for Industry and Reviewers: "Estimating the Maximum Safe Starting Dose in Initial Clinical Trials for Therapeutics in Adult Healthy Volunteers", U.S. Department of Health and Human Services, Food and Drug Administration(FDA), Center for Drug Evaluation and Research (CDER), July 6, 2005). Viral load in the bloodstream is measured intermittently over the period of time that gemcitabine is administered and for 7 weeks afterwards. It is expected that the viral load in the bloodstream increases to a lesser extent relative to that of a subject which does not receive gemcitabine. In addition, viral load decreases more quickly during over the 14 week period relative to that of a control subject which does not receive gemcitabine.

### EXAMPLE 11

### EFFECT OF IRINOTECAN ON VIRAL LOAD IN A PATIENT

The chemotherapeutic agent irinotecan is administered to a subject to whom a modified vaccinia virus, such as, for example, GLV-1h68 is administered for tumor treatment. The patient is injected with a virus at a level effective for arrest and/or shrinkage of tumor growth. Irinotecan is subsequently administered by intravenous infusion once a week for up to four weeks to the patient at a dose of 125 mg/m², followed by a two-week rest period. The administration of irinotecan is optionally repeated over a 6-week cycle as described. Viral load in the bloodstream is measured intermittently over the period of time that irinotecan is administered and for 7 weeks afterwards. It is expected that the viral load in the bloodstream increases to a lesser extent relative to that of a subject which does not receive irinotecan. In addition, viral load decreases more quickly during the entire observation period relative to that of a control subject which does not receive irinotecan.

### EXAMPLE 12

### EFFECT OF DOXORUBICIN ON VIRAL LOAD IN A PATIENT

The chemotherapeutic agent doxorubicin is administered to a subject to whom a modified vaccinia virus, such as, for example, GLV-1h68, is administered for tumor treatment. The patient is injected with a virus at a level effective for arrest and/or shrinkage of tumor growth. Doxorubicin is subsequently administered by intravenous infusion once every three weeks per cycle at a dose of 60 mg/m². The administration of doxorubicin is optionally repeated for additional cycles as described. Viral load in the bloodstream is measured intermittently over the period of time that doxorubicin is administered and for 7 weeks afterwards. It is expected that the viral load in the bloodstream increases to a lesser extent relative to that of a subject which does not receive doxorubicin. In addition, viral load decreases more quickly during the entire observation period relative to that of a control subject which does not receive doxorubicin.

### EXAMPLE 13

### EFFECT OF CISPLATIN ON VIRAL LOAD IN A PATIENT

The chemotherapeutic agent cisplatin is administered to a subject to whom a modified vaccinia virus, such as, for example, GLV-1h68, is administered for tumor treatment. The patient is injected with a virus at a level effective for arrest and/or shrinkage of tumor growth. Cisplatin is subsequently administered by intravenous infusion once every four weeks per cycle at a dose of 75 mg/m². The administration of cisplatin is optionally repeated for additional cycles as described. Viral load in the bloodstream is measured intermittently over the period of time that cisplatin is administered and for 7 weeks afterwards. It is expected that the viral load in the bloodstream increases to a lesser extent relative to that of a subject which does not receive cisplatin. In addition, viral load decreases more quickly during the entire observation period relative to that of a control subject which does not receive cisplatin.

### EXAMPLE 14

### USE OF GEMCITABINE TO REDUCE SIDE EFFECTS OF VACCINIA VIRUS POST-VACCINATION

The chemotherapeutic agent gemcitabine is administered to a subject to whom a modified vaccinia virus, such as, for example, GLV-1h68, is administered for tumor treatment. The patient is initially injected with a dose of virus effective for arrest and/or shrinkage of tumor growth. The patient begins to experience side effects related to use of the vaccinia virus, such as pock formation, weight loss, fever, abdominal pain, aches or pains in muscles, cough, diarrhea, or general feeling of discomfort or illness. Gemcitabine is subsequently administered by intravenous injection once a week for up to seven weeks to the patient at a dose of 15 mg/kg, 20 mg/kg or 30 mg/kg. Viral load in the bloodstream is measured intermittently over the period of time that gemcitabine is administered and for 7 weeks afterwards. It is expected that body weight of the patient is improved and any pock formation dissipates relative to that of a control subject which does not receive gemcitabine. Furthermore, it is expected that the viral load in the bloodstream decreases over the period of time after gemcitabine is administered, and the patient's symptoms subside after gemcitabine treatment is initiated.

### EXAMPLE 15

### USE OF IRINOTECAN TO REDUCE SIDE EFFECTS OF VIRUS POST-VACCINATION

The chemotherapeutic agent irinotecan is administered to a subject to whom a modified vaccinia virus, such as, for example, GLV-1h68, is administered for tumor treatment. The patient is initially injected with a dose of virus effective for arrest and/or shrinkage of tumor growth. The patient begins to experience side effects related to use of the vaccinia virus, such as pock formation, weight loss, fever, abdominal pain, aches or pains in muscles, cough, diarrhea, or general feeling of discomfort or illness. Irinotecan is subsequently administered by intravenous injection once a week for up to four weeks to the patient at a dose of between 100 to 150 mg/m², followed by a two-week rest period. The administration of irinotecan is optionally repeated over a 6-week cycle as described. Viral load in the bloodstream is measured intermittently over the period of time that irinotecan is administered and for 7 weeks afterwards. It is expected that body weight of the patient is improved and any pock formation dissipates relative to that of a control subject which does not receive irinotecan. Furthermore, it is expected that the viral load in the bloodstream of the patient treated with irinotecan decreases over the period of time after irinotecan is administered, and the patient's symptoms subside after irinotecan treatment is initiated.

### EXAMPLE 16

### USE OF DOXORUBICIN TO REDUCE SIDE EFFECTS OF VIRUS POST-VACCINATION

The chemotherapeutic agent doxorubicin is administered to a subject to whom a modified vaccinia virus, such as, for example, GLV-1h68, is administered for tumor treatment. The patient is initially injected with a dose of virus effective for arrest and/or shrinkage of tumor growth. The patient begins to experience side effects related to use of the viral vaccine, such as pock formation, weight loss, fever, abdominal pain, aches or pains in muscles, cough, diarrhea, or general feeling of discomfort or illness. Doxorubicin is subsequently administered by intravenous injection once every four weeks per cycle to the patient at a dose of between 60 to 75 mg/m². The administration of doxorubicin is optionally repeated for additional cycles as described. Viral load in the bloodstream is measured intermittently over the period of time that doxorubicin is administered and for 7 weeks afterwards. It is expected that body weight of the patient is improved and any pock formation dissipates relative to that of a control subject which does not receive doxorubicin. Furthermore, it is expected that the viral load in the bloodstream of the patient treated with doxorubicin decreases over the period of time after doxorubicin is administered, and the patient's symptoms subside after doxorubicin treatment is initiated.

### EXAMPLE 17

### USE OF CISPLATIN TO REDUCE SIDE EFFECTS OF VIRUS POST-VACCINATION

The chemotherapeutic agent cisplatin is administered to a subject to whom a modified vaccinia virus, such as, for example, GLV-1h68, is administered for tumor treatment. The patient is initially injected with a dose of virus effective for arrest and/or shrinkage of tumor growth. The patient begins to experience side effects related to use of the viral vaccine, such as pock formation, weight loss, fever, abdominal pain, aches or pains in muscles, cough, diarrhea, or general feeling of discomfort or illness. Cisplatin is subsequently administered by intravenous injection once every three weeks per cycle to the patient at a dose of between 50 to 75 mg/m². The administration of cisplatin is optionally repeated for additional cycles as described. Viral load in the bloodstream is measured intermittently over the period of time that cisplatin is administered and for 7 weeks afterwards. It is expected that body weight of the patient is improved and any pock formation dissipates relative to that of a control subject which does not receive cisplatin. Furthermore, it is expected that the viral load in the bloodstream of the patient treated with cisplatin decreases over the period of time after cisplatin is administered, and the patient's symptoms subside after cisplatin treatment is initiated.

### EXAMPLE 18

### SIMULTANEOUS ADMINISTRATION OF GEMCITABINE WITH ADMINISTRATION OF VIRUS TO REDUCE SIDE EFFECTS OF VIRUS

The chemotherapeutic agent gemcitabine is administered to a subject to whom a modified vaccinia virus, such as, for example, GLV-1h68, is administered for tumor treatment. The patient is injected with a dose of virus effective for arrest and/or shrinkage of tumor growth. The patient simultaneously is administered gemcitabine by intravenous infusion at a dose of 1000 mg/m² or 1500 mg/m² once a week for up to seven weeks. It is expected that tumor growth is arrested or decreases post-injection of virus. It is also expected that the patient suffers fewer side effects from the virus compared to a control patient which does not receive simultaneous administration of gemcitabine with the virus.

### EXAMPLE 19

### SIMULTANEOUS ADMINISTRATION OF IRINOTECAN WITH ADMINISTRATION OF VIRUS TO REDUCE SIDE EFFECTS OF VIRUS

The chemotherapeutic agent irinotecan is administered to a subject to whom a modified vaccinia virus, such as, for example, GLV-1h68, is administered for tumor treatment. The patient is injected with a dose of virus effective for arrest and/or shrinkage of tumor growth. The patient simultaneously is administered irinotecan by intravenous infusion at a dose of 100 to 150 mg/m² once a week for up to four weeks, followed by a two-week rest period. The administration of irinotecan is optionally repeated over a 6-week cycle as described. It is expected that tumor growth is arrested or decreases post-injection of virus. It also is expected that the patient suffers fewer side effects from the virus (e.g., pock formation, fever, abdominal pain, aches or pains in muscles, cough, diarrhea, or general feeling of discomfort or illness) and exhibits better general health (e.g., maintenance of body weight) compared to a control patient which does not receive simultaneous administration of irinotecan with the virus.

### EXAMPLE 20

### SIMULTANEOUS ADMINISTRATION OF DOXORUBICIN WITH ADMINISTRATION OF VIRUS TO REDUCE SIDE EFFECTS OF VIRUS

The chemotherapeutic agent doxorubicin is administered to a subject to whom a modified vaccinia virus, such as, for example, GLV-1h68, is administered for tumor treatment. The patient is injected with a dose of virus effective for arrest and/or shrinkage of tumor growth. The patient simultaneously is administered doxorubicin by intravenous infusion at a dose of 60 to 75 mg/m² once every four weeks per cycle. The administration of doxorubicin is optionally repeated for additional cycles as described. It also is expected that the patient suffers fewer side effects from the virus (e.g., pock formation, fever, abdominal pain, aches or pains in muscles, cough, diarrhea, or general feeling of discomfort or illness) and exhibits better general health (e.g., maintenance of body weight) compared to a control patient which does not receive simultaneous administration of doxorubicin with the virus.

### EXAMPLE 21

### SIMULTANEOUS ADMINISTRATION OF CISPLATIN WITH ADMINISTRATION OF VIRUS TO REDUCE SIDE EFFECTS OF VIRUS

The chemotherapeutic agent cisplatin is administered to a subject to whom a modified vaccinia virus, such as, for example, GLV-1h68, is administered for tumor treatment. The patient is injected with a dose of virus effective for arrest and/or shrinkage of tumor growth. The patient simultaneously is administered cisplatin by intravenous infusion at a dose of 50 to 75 mg/m² once every three weeks per cycle. The administration of cisplatin is optionally repeated for additional cycles as described. It also is expected that the patient suffers fewer side effects from the virus (e.g., pock formation, fever, abdominal pain, aches or pains in muscles, cough, diarrhea, or general feeling of discomfort or illness) and exhibits better general health (e.g., maintenance of body weight) compared to a control patient which does not receive simultaneous administration of cisplatin with the virus.

### EXAMPLE 22

### ADMINISTRATION OF GEMCITABINE PRIOR TO ADMINISTRATION OF VIRUS TO REDUCE SIDE EFFECTS OF VIRUS

The chemotherapeutic agent gemcitabine is administered to a subject to whom a modified vaccinia virus, such as, for example, GLV-1h68, is administered for tumor treatment. The patient begins chemotherapy treatment with gemcitabine prior to being injected with virus. Gemcitabine is administered to the patient by intravenous infusion at a dose of 8 mg/kg once a week for up to four weeks. The patient is then injected a dose of virus effective for arrest and/or shrinkage of tumor growth. Gemcitabine is subsequently administered post-viral injection by intravenous infusion at a dose of 8 mg/kg once a week for up to seven weeks. It is expected that tumor growth is arrested or decreases post-injection of virus. It also is expected that the patient suffers fewer side effects from the virus (e.g., pock formation, fever, abdominal pain, aches or pains in muscles, cough, diarrhea, or general feeling of discomfort or illness) and exhibits better general health (e.g., maintenance of body weight) compared to a patient who does not receive administration of gemcitabine both prior to and subsequent to administration of the virus.

### EXAMPLE 23

### ADMINISTRATION OF IRINOTECAN PRIOR TO ADMINISTRATION OF VIRUS TO REDUCE SIDE EFFECTS OF VIRUS

The chemotherapeutic agent irinotecan is administered to a subject to whom a modified vaccinia virus, such as, for example, GLV-1h68, is administered for tumor treatment. The patient begins chemotherapy treatment with irinotecan prior to being injected with virus. Irinotecan is administered to the patient by intravenous infusion at a dose of between 100 to 150 mg/m² once a week for up to four weeks, followed by a two-week rest period. The administration of irinotecan is optionally repeated over a 6-week cycle as described. The patient is then injected a dose of virus effective for arrest and/or shrinkage of tumor growth. Irinotecan is subsequently administered post-viral injection by intravenous infusion at a dose of between 100 to 150 mg/m² once a week for up to four weeks, again followed by a two-week rest period and optionally including a repeated administration of irinotecan over a 6-week cycle. It is expected that tumor growth is arrested or decreases post-injection of virus. It also is expected that the patient suffers fewer side effects from the virus (e.g., pock formation, fever, abdominal pain, aches or pains in muscles, cough, diarrhea, or general feeling of discomfort or illness) and exhibits better general health (e.g., maintenance of body weight) compared to a control patient which does not receive administration of irinotecan both prior to and after administration of the virus.

### EXAMPLE 24

### ADMINISTRATION OF DOXORUBICIN PRIOR TO ADMINISTRATION OF VIRUS TO REDUCE SIDE EFFECTS OF VIRUS

The chemotherapeutic agent doxorubicin is administered to a subject to whom a modified vaccinia virus, such as, for example, GLV-1h68, is administered for tumor treatment. The patient begins chemotherapy treatment with irinotecan prior to being injected with virus. Doxorubicin is administered to the patient by intravenous infusion at a dose of between 60 to 75 mg/m² once every four weeks per cycle. The administration of doxorubicin is optionally repeated for additional cycles as described. The patient is then injected a dose of virus effective for arrest and/or shrinkage of tumor growth. Doxorubicin is subsequently administered post-viral injection by intravenous infusion at a dose of between 60 to 75 mg/m² per four-week cycle, optionally including additional cycles of doxorubicin administration as needed. It is expected that tumor growth is arrested or decreases post-injection of virus. It also is expected that the patient suffers fewer side effects from the virus (e.g., pock formation, fever, abdominal pain, aches or pains in muscles, cough, diarrhea, or general feeling of discomfort or illness) and exhibits better general health (e.g., maintenance of body weight) compared to a control patient which does not receive administration of doxorubicin both prior to and after administration of the virus.

### EXAMPLE 25

### ADMINISTRATION OF CISPLATIN PRIOR TO ADMINISTRATION OF VIRUS TO REDUCE SIDE EFFECTS OF VIRUS

The chemotherapeutic agent cisplatin is administered to a subject to whom a modified vaccinia virus, such as, for example, GLV-1h68, is administered for tumor treatment. The patient begins chemotherapy treatment with cisplatin prior to being injected with virus. Cisplatin is administered to the patient by intravenous infusion at a dose of between 50 to 75 mg/m² once every three weeks per cycle. The administration of cisplatin is optionally repeated for additional cycles as described. The patient is then injected a dose of virus effective for arrest and/or shrinkage of tumor growth. Cisplatin is subsequently administered post-viral injection by intravenous infusion at a dose of between 50 to 75 mg/m² per three-week cycle, optionally including additional cycles of cisplatin administration as needed. It is expected that tumor growth is arrested or decreases post-injection of virus. It also is expected that the patient suffers fewer side effects from the virus (e.g., pock formation, fever, abdominal pain, aches or pains in muscles, cough, diarrhea, or general feeling of discomfort or illness) and exhibits better general health (e.g., maintenance of body weight) compared to a control patient which does not receive administration of cisplatin both prior to and after administration of the virus.

### EXAMPLE 26

### EFFECT OF GEMCITABINE ON VIRAL LOAD IN A PATIENT

The chemotherapeutic agent gemcitabine is administered to a subject to whom a modified vaccinia virus, such as, for example, GLV-1h68, is administered for tumor treatment. The patient is injected with a virus at a level effective for arrest and/or shrinkage of tumor growth. Gemcitabine is subsequently administered by intravenous infusion once a week for up to seven weeks to the patient at a dose of 9 mg/kg (conversion calculated according to FDA's Guidance for Industry and Reviewers: "Estimating the Maximum Safe Starting Dose in Initial Clinical Trials for Therapeutics in Adult Healthy Volunteers", U.S. Department of Health and Human Services, Food and Drug Administration (FDA), Center for Drug Evaluation and Research (CDER), July 6, 2005). Viral load in the bloodstream is measured intermittently over the period of time that gemcitabine is administered and for 7 weeks afterwards. It is expected that the viral load in the bloodstream is maintained or increases before subsequently decreasing over the 14 week period.

### EXAMPLE 27

### EFFECT OF IRINOTECAN ON VIRAL LOAD IN A PATIENT

The chemotherapeutic agent irinotecan is administered to a subject to whom a modified vaccinia virus, such as, for example, GLV-1h68, is administered for tumor treatment. The patient is injected with a virus at a level effective for arrest and/or shrinkage of tumor growth. Irinotecan is subsequently administered by intravenous infusion once a week for up to four weeks to the patient at a dose of between 100 to 150 mg/m², followed by a two-week rest period. The administration of irinotecan is optionally repeated over a 6-week cycle as described. Viral load in the bloodstream is measured intermittently over the period of time that gemcitabine is administered and for 7 weeks afterwards. It is expected that the viral load in the bloodstream is maintained or increases before subsequently decreasing over the observation period.

### EXAMPLE 28

### EFFECT OF DOXORUBICIN ON VIRAL LOAD IN A PATIENT

The chemotherapeutic agent doxorubicin is administered to a subject to whom a modified vaccinia virus, such as, for example, GLV-1h68, is administered for tumor treatment. The patient is injected with a virus at a level effective for arrest and/or shrinkage of tumor growth. Doxorubicin is subsequently administered by intravenous infusion once every four weeks to the patient at a dose of between 60 to 75 mg/m², and administration of doxorubicin is optionally repeated for additional cycles as described. Viral load in the bloodstream is measured intermittently over the period of time that doxorubicin is administered and for 7 weeks afterwards. It is expected that the viral load in the bloodstream is maintained or increases before subsequently decreasing over the observation period.

### EXAMPLE 29

### EFFECT OF CISPLATIN ON VIRAL LOAD IN A PATIENT

The chemotherapeutic agent cisplatin is administered to a subject to whom a modified vaccinia virus, such as, for example, GLV-1h68, is administered for tumor treatment. The patient is injected with a virus at a level effective for arrest and/or shrinkage of tumor growth. Cisplatin is subsequently administered by intravenous infusion once every three weeks to the patient at a dose of between 50 to 75 mg/m², and administration of cisplatin is optionally repeated for additional cycles as described. Viral load in the bloodstream is measured intermittently over the period of time that cisplatin is administered and for 7 weeks afterwards. It is expected that the viral load in the bloodstream is maintained or increases before subsequently decreasing over the observation period.

### EXAMPLE 30

### EFFECT OF SUSTAINED RELEASE OF A COMBINATION OF VIRUS AND GEMCITABINE ON VIRAL LOAD IN A PATIENT

A composition comprising a modified vaccinia virus and the chemotherapeutic agent gemcitabine is implanted near the site of a tumor in a subject. The composition provides sustained release of an antigen expressed by the virus and of gemcitabine, wherein the antigen is provided to induce the subject's immune system to target the tumor. Sustained release of the antigen and gemcitabine takes place over a period of up to 8 weeks or longer. Viral load in the bloodstream is measured intermittently after implantation of the composition. It is expected that the viral load in the bloodstream is increases to a lesser extent than in a subject to which a composition comprising vaccinia virus without gemcitabine is administered. In addition, viral load in the subject decreases more rapidly over the observation period than does that of the subject in which gemcitabine is not sustainably released.

### EXAMPLE 31

### EFFECT OF SUSTAINED RELEASE OF A COMBINATION OF VIRUS AND IRINOTECAN ON VIRAL LOAD IN A PATIENT

A composition comprising a modified vaccinia virus and the chemotherapeutic agent irinotecan is implanted near the site of a tumor in a subject. The composition provides sustained release of an antigen expressed by the virus and of irinotecan, wherein the antigen is provided to induce the subject's immune system to target the tumor. Sustained release of the antigen and irinotecan takes place over a period of up to 8 weeks or longer. Viral load in the bloodstream is measured intermittently after implantation of the composition. It is expected that the viral load in the bloodstream is increased to a lesser extent than in a control subject to which a composition comprising vaccinia virus without irinotecan is administered. In addition, viral load in the subject decreases more rapidly over the observation period than does that of the subject in which irinotecan is not sustainably released.

### EXAMPLE 32

### EFFECT OF SUSTAINED RELEASE OF A COMBINATION OF VIRUS AND DOXORUBICIN ON VIRAL LOAD IN A PATIENT

A composition comprising a modified vaccinia virus and the chemotherapeutic agent doxorubicin is implanted near the site of a tumor in a subject. The composition provides sustained release of an antigen expressed by the virus and of doxorubicin, wherein the antigen is provided to induce the subject's immune system to target the tumor. Sustained release of the antigen and doxorubicin takes place over a period of up to 8 weeks or longer. Viral load in the bloodstream is measured intermittently after implantation of the composition. It is expected that the viral load in the bloodstream is increased to a lesser extent than in a control subject to which a composition comprising vaccinia virus without doxorubicin is administered. In addition, viral load in the subject decreases more rapidly over the observation period than does that of the subject in which doxorubicin is not sustainably released.

### EXAMPLE 33

### EFFECT OF SUSTAINED RELEASE OF A COMBINATION OF VIRUS AND CISPLATIN ON VIRAL LOAD IN A PATIENT

A composition comprising a modified vaccinia virus and the chemotherapeutic agent cisplatin is implanted near the site of a tumor in a subject. The composition provides sustained release of an antigen expressed by the virus and of cisplatin, wherein the antigen is provided to induce the subject's immune system to target the tumor. Sustained release of the antigen and cisplatin takes place over a period of up to 8 weeks or longer. Viral load in the bloodstream is measured intermittently after implantation of the composition. It is expected that the viral load in the bloodstream is increased to a lesser extent than in a control subject to which a composition comprising vaccinia virus without cisplatin is administered. In addition, viral load in the subject decreases more rapidly over the observation period than does that of the subject in which cisplatin is not sustainably released.

### EXAMPLE 34

### EFFECT OF SUSTAINED RELEASE OF A COMBINATION OF VIRUS AND GEMCITABINE ON VIRUS-INDUCED SYMPTOMS IN A PATIENT

A composition comprising a modified vaccinia virus and the chemotherapeutic agent gemcitabine is implanted near the site of a tumor in a subject. The composition provides sustained release of an antigen expressed by the virus and of gemcitabine, wherein the antigen is provided to induce the subject's immune system to target the tumor. Sustained release of the antigen and gemcitabine takes place over a period of up to 8 weeks. Fewer virus-associated side effects (*e.g*., pock formation, weight loss, fever, abdominal pain, aches or pains in muscles, cough, diarrhea, or general feeling of discomfort or illness) are expected in the subject than in a subject in which a composition comprising virus without gemcitabine is provided.

### EXAMPLE 35

### EFFECT OF SUSTAINED RELEASE OF A COMBINATION OF VIRUS AND IRINOTECAN ON VIRUS-INDUCED SYMPTOMS IN A PATIENT

A composition comprising a modified vaccinia virus and the chemotherapeutic agent irinotecan is implanted near the site of a tumor in a subject. The composition provides sustained release of an antigen expressed by the virus and of irinotecan, wherein the antigen is provided to induce the subject's immune system to target the tumor. Sustained release of the antigen and irinotecan takes place over a period of up to 8 weeks. Fewer virus-associated side effects (*e.g*., pock formation, weight loss, fever, abdominal pain, aches or pains in muscles, cough, diarrhea, or general feeling of discomfort or illness) are expected in the subject than in a control subject in which a composition comprising virus without irinotecan is provided.

### EXAMPLE 36

### EFFECT OF SUSTAINED RELEASE OF A COMBINATION OF VIRUS AND DOXORUBICIN ON VIRUS-INDUCED SYMPTOMS IN A PATIENT

A composition comprising a modified vaccinia virus and the chemotherapeutic agent doxorubicin is implanted near the site of a tumor in a subject. The composition provides sustained release of an antigen expressed by the virus and of doxorubicin, wherein the antigen is provided to induce the subject's immune system to target the tumor. Sustained release of the antigen and doxorubicin takes place over a period of up to 8 weeks. Fewer virus-associated side effects (*e.g*., pock formation, weight loss, fever, abdominal pain, aches or pains in muscles, cough, diarrhea, or general feeling of discomfort or illness) are expected in the subject than in a control subject in which a composition comprising virus without doxorubicin is provided.

### EXAMPLE 37

### EFFECT OF SUSTAINED RELEASE OF A COMBINATION OF VIRUS AND CISPLATIN ON VIRUS-INDUCED SYMPTOMS IN A PATIENT

A composition comprising a modified vaccinia virus and the chemotherapeutic agent cisplatin is implanted near the site of a tumor in a subject. The composition provides sustained release of an antigen expressed by the virus and of cisplatin, wherein the antigen is provided to induce the subject's immune system to target the tumor. Sustained release of the antigen and cisplatin takes place over a period of up to 8 weeks. Fewer virus-associated side effects (*e.g*., pock formation, weight loss, fever, abdominal pain, aches or pains in muscles, cough, diarrhea, or general feeling of discomfort or illness) are expected in the subject than in a control subject in which a composition comprising virus without cisplatin is provided.

### EQUIVALENTS

The foregoing written specification is considered to be sufficient to enable one skilled in the art to practice the invention. The foregoing description and Examples detail certain preferred embodiments of the invention and describes the best mode contemplated by the inventors. It will be appreciated, however, that no matter how detailed the foregoing may appear in text, the invention may be practiced in many ways and the invention should be construed in accordance with the appended claims and any equivalents thereof.

Also described are:
1. Use of a chemotherapeutic agent in the preparation of a medicament for treating an adverse side effect associated with viral treatment of cancer, wherein the chemotherapeutic agent is selected from among gemcitabine, irinotecan, doxorubicin and cisplatin.
2. A chemotherapeutic agent for use in ameliorating an adverse side effect associated with viral treatment of cancer, wherein the chemotherapeutic agent is selected from among gemcitabine, irinotecan, doxorubicin and cisplatin.
3. The use of (1) or (2), wherein the adverse side effect comprises one or more of pock formation, weight loss, fever, abdominal pain, aches or pains in muscles, cough, diarrhea, and feeling of discomfort or illness.
4. Use of a chemotherapeutic agent in the preparation of a medicament for controlling viral load associated with viral treatment of cancer, wherein the amount of chemotherapeutic agent administered is sufficient to control or reduce viral titer in the subject to a level that does not cause adverse side effects.
5. The use or agent of any of (1)-(4), wherein the side effect is associated with treatment with a vaccinia virus.
6. The use or agent of any of (1)-(5), wherein the virus is a Lister strain vaccinia virus.
7. The use or agent of (6), wherein the virus strain is LIVP.
8. The use or agent of (7), wherein the virus is selected from among GLV-1h68, GLV-1h70, GLV-1h71, GLV-1h72, GLV-1h73, GLV-1h74, GLV-1h81, GLV-1h82, GLV-1h83, GLV-1h84, GLV-1h85, GLV-1h86, GLV-1h90, GLV-1h91, GLV-1h92, GLV-1h96, GLV-1h97, GLV-1h98, GLV-1h99, GLV-1h100, GLV-1h101, GLV-1h104, GLV-1h105, GLV-1h106, GLV-1h107, GLV-1h108, GLV-1h109, GLV-1h139, GLV-1h146, GLV-1h150, GLV-1h151, GLV-1h152 and GLV-1h153.
9. The use or agent of any of (1)-(8), wherein the cancer is a metastatic cancer, metastasis, or a solid tumor.
10. A combination, comprising:
   a composition containing a therapeutic virus, wherein the virus is effective for treatment of cancer; and
   a composition containing a chemotherapeutic agent in an amount effective for clearing the virus from a subject, when the composition containing a chemotherapeutic agent is administered as a single dose.
11. The combination of (10), wherein: the chemotherapeutic agent is selected from among gemcitabine, irinotecan, doxorubicin and cisplatin.
12. The combination of (10) or (11), wherein the therapeutic virus is a vaccinia virus.
13. The combination of (12), wherein the virus is a Lister strain vaccinia virus.
14. The combination of (13), wherein the virus strain is LIVP.
15. The combination of (14), wherein the virus is selected from among GLV-1h68, GLV-1h70, GLV-1h71, GLV-1h72, GLV-1h73, GLV-1h74, GLV-1h81, GLV-1h82, GLV-1h83, GLV-1h84, GLV-1h85, GLV-1h86, GLV-1h90, GLV-1h91, GLV-1h92, GLV-1h96, GLV-1h97, GLV-1h98, GLV-1h99, GLV-1h100, GLV-1h101, GLV-1h104, GLV-1h105, GLV-1h106, GLV-1h107, GLV-1h108, GLV-1h109, GLV-1h139, GLV-1h146, GLV-1h150, GLV-1h151, GLV-1h152 and GLV-1h153.
16. The combination of any of (10)-(15), wherein the chemotherapeutic agent and virus are formulated as a single composition or separately in two compositions.
17. The use or agent of any of (1)-(9), or combination of any of (10)-(16), wherein the virus encodes a detectable gene product or gene product that induces a detectable signal.
18. The use or agent or combination of (17), wherein the gene product is a luciferase or a fluorescent protein.
19. The use or agent of any of (1)-(9), or combination of any of (10)-(16), wherein the virus encodes a therapeutic gene product.
20. The use or agent of any of (1)-(9), or combination of any of (10)-(16), wherein the virus encodes an antigen or antibody.

## Claims

1. Use of an anti-viral agent in the preparation of a medicament for treating an adverse side effect associated with oncolytic pox virus therapy of cancer, wherein the anti-viral agent is selected from among ST-246, cidofovir, alkoxyalkyl esters of cidofovir and imatinib.

2. An anti-virus agent for use in ameliorating an adverse side effect associated with oncolytic pox virus therapy of cancer, wherein the anti-viral agent is selected from among ST-246, cidofovir, alkoxyalkyl esters of cidofovir and imatinib.

3. The use of claim 1 or claim 2, wherein the adverse side effect comprises one or more of pock formation, weight loss, fever, abdominal pain, aches or pains in muscles, cough, diarrhea and feeling of discomfort or illness.

4. The use or agent of claim 1 or claim 2, wherein the oncolytic pox virus is a vaccinia virus.

5. The use or agent of claim 4, wherein the virus is a Lister strain vaccinia virus.

6. The use or agent of claim 5, wherein the virus strain is LIVP.

7. The use or agent of claim 6, wherein the virus is selected from among GLV-1h68, whose genome comprises SEQ ID NO:1, and viruses derived from GLV-1h68.

8. The use or agent of any of claims 1-6, wherein the cancer is a metastatic cancer and/or a solid tumor.

9. The use or agent of any of claims 1-8, wherein the virus encodes a detectable gene product or gene product that induces a detectable signal.

10. The use or agent of claim 9, wherein the gene product is a luciferase or a fluorescent protein.

11. The use or agent of any of claims 1-8, wherein the virus encodes a therapeutic gene product.

## Patentansprüche

1. Verwendung einer antiviralen Substanz zur Herstellung eines Medikaments zur Behandlung einer Nebenwirkung, die mit onkolytischer Pockenvirus Therapie von Krebs in Verbindung steht, wobei die antivirale Substanz aus ST-246, Cidofovir, Alkoxyalkyl-Estern von Cidofovir und Imatinib ausgewählt ist.

2. Antivirale Substanz zur Verwendung in der Verbesserung einer Nebenwirkung, die mit onkolytischer Pockenvirus Therapie von Krebs in Verbindung steht, wobei die antivirale Substanz aus ST-246, Cidofovir, Alkoxyalkyl-Estern von Cidofovir und Imatinib ausgewählt ist.

3. Verwendung nach Anspruch 1 oder Anspruch 2, wobei die Nebenwirkung eines oder mehrere von Pockenbildung, Gewichtsverlust, Fieber, Bauchschmerz, Schmerzen oder Muskelschmerzen, Husten, Durchfall und das Gefühl von Unwohlsein oder Kranksein umfasst.

4. Verwendung oder Substanz nach Anspruch 1 oder Anspruch 2, wobei das onkolytische Pockenvirus ein Vaccinia Virus ist.

5. Verwendung oder Substanz nach Anspruch 4, wobei das Virus ein Lister-Stamm Vaccinia Virus ist.

6. Verwendung oder Substanz nach Anspruch 5, wobei der Virusstamm LIVP ist.

7. Verwendung oder Substanz nach Anspruch 6, wobei das Virus aus GLV-1 h68, dessen Genom SEQ ID NO:1 umfasst und von GLV-1h68 abstammenden Viren ausgewählt wird.

8. Verwendung oder Substanz nach einem der Ansprüche 1-6, wobei der Krebs ein metastasierender Krebs und/oder ein fester Tumor ist.

9. Verwendung oder Substanz nach einem der Ansprüche 1-8, wobei das Virus für ein nachweisbares Genprodukt oder ein Genprodukt kodiert, welches ein nachweisbares Signal auslöst.

10. Verwendung oder Substanz nach Anspruch 9, wobei das Genprodukt eine Luciferase oder ein Fluoreszenz-Protein ist.

11. Verwendung oder Substanz nach einem der Ansprüche 1-8, wobei das Virus für ein therapeutisches Genprodukt kodiert.

## Revendications

1. Utilisation d'un agent anti-viral dans la préparation d'un médicament pour le traitement d'un effet secondaire néfaste associé à une thérapie du cancer avec un poxvirus oncolytique, dans laquelle l'agent anti-viral est choisi entre le ST-246, le cidofovir, des esters alkoxyalkyliques de cidofovir et l'imatinib.

2. Agent anti-viral pour une utilisation dans l'amélioration d'un effet secondaire néfaste associé à une thérapie du cancer avec un poxvirus oncolytique, ledit agent anti-viral étant choisi entre le ST-246, le cidofovir, des esters alkoxyalkyliques de cidofovir et l'imatinib.

3. Utilisation suivant la revendication 1 ou la revendication 2, dans laquelle l'effet secondaire néfaste comprend un ou plusieurs des effets consistant en la formation de pustules, une perte de poids, de la fièvre, une douleur abdominale, un endolorissement ou des douleurs dans les muscles, de la toux, de la diarrhée et une sensation d'inconfort ou de maladie.

4. Utilisation ou agent suivant la revendication 1 ou la revendication 2, le poxvirus oncolytique étant un virus vaccinal.

5. Utilisation ou agent suivant la revendication 4, le virus étant un virus vaccinal de la souche Lister.

6. Utilisation ou agent suivant la revendication 5, la souche de virus étant la souche LIVP.

7. Utilisation ou agent suivant la revendication 6, le virus étant choisi entre le GLV-1h68, dont le génome comprend la SEQ ID NO:1, et des virus dérivés du GLV-1h68.

8. Utilisation ou agent suivant l'une quelconque des revendications 1 à 6, le cancer étant un cancer métastatique et/ou une tumeur solide.

9. Utilisation ou agent suivant l'une quelconque des revendications 1 à 8, le virus codant pour un produit de gène détectable ou un produit de gène qui induit un signal détectable.

10. Utilisation ou agent suivant la revendication 9, dans laquelle le produit de gène est une luciférase ou une protéine fluorescente.

11. Utilisation ou agent suivant l'une quelconque des revendications 1 à 8, le virus codant pour un produit de gène thérapeutique.


### SEQUENCE LISTING

<110> Genelux corporation
   Chen, Nanhai
   Yu, Yong A.
<120> USE OF A CHEMOTHERAPEUTIC AGENT IN THE PREPARATION OF A MEDICAMENT FOR TREATING OR AMELIORATING AN ADVERSE SIDE EFFECT ASSOCIATED WITH ONCOLYTIC VIRAL THERAPY
<130> SCB/P102399EP01
<140> Unknown
   <141> Filed herewith
<160> 14
<170> FastSEQ for Windows version 4.0
<210> 1
   <211> 203057
   <212> DNA
   <213> vaccinia virus
<220>
   <223> GLV-1h68
<400> 1
   ttatgagacc atcaaagaga gaaagagaat aaaaatattt ttgtaaaact ttttttatga 60
   gaccatcaaa gagagaaaga gaataaaaat atttttgtaa aactttttta tgagaccatc 120
   aaagagagaa agagaataaa aatatttttg taaaactttt ttatgagacc atcaaagaga 180
   gaaagagaat aaaaatattt ttgtaaaact ttttttatga gaccatcaaa gagagaaaga 240
   gaataaaaat atttttgtaa aacttttttt atgagaccat caaagagaga aagagaataa 300
   aaatattttt gtaaaacttt ttttatgaga ccatcaaaga gagaaagaga ataaaaatat 360
   ttttgtaaaa ctttttttat gagaccatca aagagagaaa gagaataaaa atatttttgt 420
   aaaacttttt ttatgagacc atcaaagaga gaaagagaat aaaaatattt ttgtaaaact 480
   ttttttatga gaccatcaaa gagagaaaga gaataaaaat atttttgtaa aacttttttt 540
   atgagaccat caaagagaga aagagaataa aaatattttt gtaaaacttt ttttatgaga 600
   ccatcaaaga gagaaagaga ataaaaatat ttttgtaaaa ctttttttat gagaccatca 660
   aagagagaaa gagaataaaa atatttttgt aaaacttttt ttatgagacc atcaaagaga 720
   gaaagagaat aaaaatattt ttgtaaaact ttttttatga gaccatcaaa gagagaaaga 780
   gaataaaaat atttttgtaa aacttttttt atgagaccat cagaaagagg tttaatattt 840
   ttgtgatact ctgaaaggaa ataggaatag gaataggaat agtgtcataa tcgtatcaca 900
   ctattgagac agaaaaagaa gaagtcgcga gaggtaactt tttgtgaatg tagttaagaa 960
   catttttgtt ttgcaaaccg gaatatagtg tccggtacac ttttttaatt cgtggtgtgc 1020
   ctgaatcgtt cgattaaccc tactcatcca atttcagatg aatagagtta tcgattcaga 1080
   cacacgcttt gagttttgtt gaatcgatga gtgaagtatc atcggttgca ccttcagatg 1140
   ccgatccgtc gacatacttg aatccatcct tgacctcaag ttcagatgat tccttgcaca 1200
   tgtctccgat acgaacgcta aactctagat tcttgacgca ttttgtatcg acgatcgttg 1260
   aaccgatgat atcttcgtaa ctcactttct tatgagagat gttagacccg agtactggat 1320
   gggtcttgat gtcgctgtct ttctcttctt cgctacatct gatgtcgata gacacttcac 1380
   agtctttgat catagcaaga gcttcttcat gagtgatcgc gggagagtcc ttaccttgtc 1440
   ctggggacac gctggacaat ctagcattca ctgtgtttcc atcagcggat tctgagatgg 1500
   atttaatctg aggacatttg gtgaatccaa agttcattct cagacctcca ccgatgatgg 1560
   agtaataagt ggtaggagga tctacatcct cgactgatgt ggaatcatct tctgattcca 1620
   cctcgggatc tggatctgac tcggactctg taatttccgt tacggattgg caaatcttat 1680
   cattggtcgg tgtttggtct tgctttgtga ctttgataat aacatcgatt cccatatgat 1740
   gtttgttttc ttcttccgta cacgaggagg aggatgagga tgattgctga agactggcag 1800
   gcatagcagc tgccgccagg cacatgcatg ccaggacgat atattgtttc ataattgcta 1860
   ttgattgagt actgttcttt atgattctac ttccttaccg tgcaataaat tagaatatat 1920
   tttctacttt tacgagaaat taattattgt atttattatt tatgggtgaa aaacttacta 1980
   taaaaagtgg gtgggtttgg aattagtgat cagtttatgt atatcgcaac taccgggcat 2040
   atggctatcg acatcgagaa cattacccac atgataagag attgtatcag tttcgtagtc 2100
   ttgagtattg gtattactat atagtatata gatgtcgccc actagagtta ctgtctccga 2160
   atgcggcatg atagtatcat tctttgcttt cgttaactgt ttggaggaag aatctttgtt 2220
   attgcattta atctcgaaat tcagagtgca cacctttctc ctgtaaagaa acctgaagtc 2280
   gctaccttat taagaacgga gaagtatcca tcacgaaata cgggattaca gtctttatga 2340
   ttcatagtaa tagttagttc cgacgttgag atggattcgc tgagaccggt agtggtcgtc 2400
   cgagtacacg acgtgtcgtt aactggatac aggttaattt ccacatcgat atagttaaac 2460
   gtatttctgg gtacgggttc gcatttatct gcggaagaga cggtgtgaga atatgttccg 2520
   agaccacacg gagaacagat gacgtctccg gatactccgt atcctattcc acattttgtt 2580
   tgggaaacac atgccttgca tccggatgat cctttgagaa gacaataata tccgggagag 2640
   cattcacaga ttctattgtg agtcgtgtta cacgatcgcg tcttccgtta caacttagac 2700
   aagcgggtaa atgattattg cgagatgtga aggtacccga accacacggc gtacatcgtg 2760
   tgttagtctt gctatcgcat aatctggaag cgtatgttcc cggacacaaa ttatggcgtt 2820
   tgtattcgtt gtctttacac tttccatcgg atggtgcatg cggtgctata tctcttccgt 2880
   ttattattat acatgagaga aacaatatat acgagtataa tacggacttc atgatttaat 2940
   aatgtagtaa tcgttgtctt gttcctgttt cctacttctc caatcatata gatattttct 3000
   ttctatcatg gataatattt gtaatggttc tttccgtaca acatactgtt tagatgatat 3060
   tgcgcataat ttccggaggc aaatacgata gtctagattg accgatggta gactctaatt 3120
   tattgagtgc tttgtcgacg agtttacttt tacgctccat cgatagatgg cactgttcta 3180
   tgagatcgtc gtacatggga aatgaaatgt gactgtctga atgtatggct ttaagatagc 3240
   tgtgataccg tatacaggtc ggtgtcggag attcgaatct ctttgaggcg acttatgtca 3300
   cgatgatgga atctatctta tcgaatgata tatttttcat aaatacactt ttatagtcct 3360
   cgtttaaaca gaatttacta tgtagttccg cgaatgactc gtcccttaat aggcagtagg 3420
   ctagtatctt ttttacgtag taatcgtcgt agggagagac atcttgtaga acaacgattt 3480
   cgttttgttt tctcttctgt aactgctttt ctggatggcc gtattgatta tcgagcgtga 3540
   tactcgctcc atattccaat aaccgctttg caaattgtat attattgaca tcgaccgcgt 3600
   aatatagtag agttatcgat catatctata tcatccatgt acttgcttag tatatcaaat 3660
   acatctatca gtatggtttc ataacagtga tacccgcaat tattaaatct cgataatatc 3720
   agaccgtaca tacatagacg gccattgttc gatacgtgat ttacagccgc gtgtccatat 3780
   tttccacgat aaaccttacg acgtttacat cgacgagatt attattaaca aagtagtcgt 3840
   gtagaggata gttgttgtcc gttatctaac atgcatcgaa cgaccatatc gccgtaatgt 3900
   aagtagttta tcagtatggc ttgtacgatg gattcatcct gttgtctaaa tctctttaga 3960
   atgttatcga tgatgtagtg gttatattct ctggaatcgt acgaagtaat actacgcatt 4020
   acgtcgacaa gagtatgacg tctctcaata agaagattaa cgatttccat gtctacatta 4080
   tatggggtta ctctaaatcg cttgtttaga taatacgcct ctaatatagg gctgacgtcg 4140
   tatactctac acgtgtccac atcctttatt aataataatt taacaatctc tatatctatg 4200
   gttgagaaag accagtagta ttggatgggt aaagatcctc cttcgtctct gccatggatg 4260
   gaaacattgt tatcgatcaa acatttaatt acatccttgg atagagattg agattctcta 4320
   tgagacgata tatagtaatg aagagagttc ttacacatat cactgtcgta catacaggta 4380
   cgaaatacgt aaccggtgct gtaacattct gatttaagaa gccatagcaa tacttctggt 4440
   ctcggattag gcgtcgttac gtatatatcc accaatccga gaccattgat tgcataattc 4500
   gtattcttgg acggacgtat ccgtttatcc acaattaggt attttagcag acgtaagtcg 4560
   atattatccg aatacagatc gaaatcattt atattcgact tgagttcgtt agaggaattt 4620
   gaatagctgg atatcagtag atgcacaatc tgagatttta cgtatctatg cttactgtat 4680
   actcctagcg gagttaatcc ttcgttgttt ctacaaagtc tctcgactcc gcgagagaac 4740
   aatcttaatg tctgtatcgc atttattgga gacgtaacaa tgtagcgcat tgtttcctcg 4800
   tctatctata tgttttgata agttgtgaca cgtttcaatt tctagtttta tttttttgta 4860
   cgtcacatct tcatccagta gacgacatag aatagtgcac tctctaccac aataatccat 4920
   agctattctg gtgctaatta ttcctatttc acgaaggcaa tcattcctca taagatgata 4980
   aaaagtgtag tggagattta gtatttagca gtgcggatat gatccaagag ggtgagatag 5040
   tcgttctcgt tcagaatctt tcgcagcata agtagtatgt cgatatactt atcgttgaag 5100
   actctttata aaagtctatt tgtttatatt tattgcggat agcagtattt ccctataaaa 5160
   agtatacgtc ctgtggtgtc tttaatcatg tacatgaatg gatggtttat gtagaccttc 5220
   gtacgatata ccatcgaaaa gttaatcgta aatactcctg taacggccga tgcttctgta 5280
   tactcctcat taacatctat aaacgtcgta tgtagaaatt tttctacagt gatagtttca 5340
   ttacacatct tgctaaaatc tgcataatat ccgaatatat tagtaagtcc taaattttct 5400
   aaaatcggta ccagattata cggttctgtc atttccactt taaactttgg catatacaag 5460
   tctatacttt tagtagataa cataccacac cattttttaa atttttcatc tgttatattt 5520
   ttttctatgt tatatatacc ttctatgtcg tccggtagta taattaccat actagagttt 5580
   ccctcgtatg gaatatcgat aatagagaat cctccgaata attcattaat atgtacatat 5640
   tgcaagttat tctcggtacc caccatcata tcaacgctgg taactatatt cttagaaata 5700
   taaaacttgt ctgtatatgt aagatgttta gaaaatggat atttccacat tgctttaaaa 5760
   tggacggcgc taacaactgt catacgagta ttaatggata gcggactagt caataaggaa 5820
   ttaattttac catttgtcat tgtcttaacc cattcgttga ttagttcctt tgtttggtta 5880
   gcattattaa agtttacagt ttgaaaatcg tcttttattt tttgtaggaa ggaggcgtgg 5940
   aactcgatac tatcgctacc gtatatttta tttgcggtag ctagtgtcgc acaatacgga 6000
   atatctacct ccatgtcatc attattgtca tcgggtgtat tctcattcat attctctata 6060
   tattttgata gttgttcagc tgtagaacca gctgctccat gatttagaat agataaagta 6120
   gataaaatgg aaactggaga aatcaaaaca ttttcatcag ggtgttttaa gattagttct 6180
   ttaaagatat ccatggtata gacctaacga taacgatata tatcataaat aaataatgtt 6240
   aaatttcaat ttatgtttgt accccgtatt catacttaac aaattggtat tgcgtacaca 6300
   atcaatcata ttacatacca ttaataatgc aagcataaaa aattgttagt agatgtttct 6360
   aaatataggt tccgtaagca aagaatataa gaatgaagcg gtaatgataa aatcaatcgt 6420
   tatctaaaat gatcatactc atttatttta ttctattata ttaacacata catttttaac 6480
   agcaacacat tcaatattgt attgttattt ttatattatt tacacaatta acaatatatt 6540
   attagtttat attactgaat taataatata aaattcccaa tcttgtcata aacacacact 6600
   gagaaacagc ataaacacag aatccatcaa aaatgttgat aaattatctg atgttgttgt 6660
   tcgctgctat gataatcaga tcattcgccg atagtggtaa cgctatcgaa acgacattgc 6720
   cagaaattac aaacgctaca acagatattc cagctatcag attatgcggt ccagagggag 6780
   atggatattg tttacacggt gactgtatcc acgctagaga tattgacggt atgtattgta 6840
   gatgctctca tggttataca ggcattagat gtcagcatgt agtattagta gactatcaac 6900
   gttcagaaaa cccaaacact acaacgtcat atatcccatc tcccggtatt atgcttgtat 6960
   tagtaggcat tattatgtgt tgtctattat ctgtttatag gttcactcga cgaactaaac 7020
   tacctataca agatatggtt gtgccataat ttttataaat ttttttatga gtatttttac 7080
   aaaaatgtat aaagtgtatg tcttatgtat atttataaaa atgctaagta tgcgatgtat 7140
   ctatgttatt tgtatttatc taaacaatac ctctacctct agatattata caaaaatttt 7200
   ttatttcggc atattaaagt aaaatctagt taccttgaaa atgaatacag tgggtggttc 7260
   cgtatcacca gtaagaacat aatagtcaaa tacagtatcc gattgagatt ttgcatacaa 7320
   tactagtcta gaaagaaatt tgtaatcatc ttctgtgacg ggagtccata tatctgtatc 7380
   atcgtctagt ttatcagtgt cccatgctat attcctgtta tcatcattag ttaatgaaaa 7440
   taactctcgt gcttcagaaa agtcaaatat tgtatccata catacatctc caaaactatc 7500
   gcttatacgt ttatctttaa cgatacctat acctagatgg ttatttacta acagacattt 7560
   tccagatcta ttgactataa ctcctatagt ttccacatca accaagtaat gatcatctat 7620
   tgttatataa caataacata actcttttcc atttttatca gtatgtatat ctatatcaac 7680
   gtcgtcgttg tagtgaatag tagtcattga tctattatat gaaacggata tgtctagaac 7740
   ggcaattgtt ttacgtccag ttaacacttt cgttgattta aagtctagag tctttgcaaa 7800
   cataatatcc ttatccgact ttatatttcc tgtagggtgg tataatttta ttttgcctcc 7860
   acatatcggt gtttccaaat atattactag acaatattcc atatagttat tagttaaggg 7920
   tacccaatta gaacacgtac gcttattatc atcatttgga tcgtatttca taaaagttat 7980
   tgtgttatcg atgtcaacac attctacatt ttttaatcgt ctatatagta tttttctgat 8040
   attttctata atatcagaat tgtcttccat cggaagttgt atactatcgg aatcagttac 8100
   atgtttaaat aattctctga tgtcattcct tatacaatca aattcattat taaacagttt 8160
   aatagtctgt agacctttat cgtcgtaaat atccattgtc ttattagtta cgcttatttt 8220
   tatgtgtttt tacgttgctt tattatattt tataagaatg attgtttgac gaatcacgag 8280
   aactattaag acacattatt aggtatatat tataaaaaag tttttgatta cgatgttata 8340
   agaggaaaga ggacacatta acatcataca tcaattaact acattcttat aacatcgtaa 8400
   tcaaaagaat tgcaattttg atgtataaca actgtcaatg ggttatggaa ttgtatatta 8460
   catattatac ggtatgttgg taacgacaaa taccggtcgg taattgtctg ccggtgtaat 8520
   agaattatat atatctatct attacaccgg ccttgtatac ataataataa gttgtggtag 8580
   tatgatctcc atatttataa tttaggactt tgtattcagt atttttggaa tcataaaaaa 8640
   taaaaaaaag ttttactaat ttaaaattta aaaagtattt acattttttt cactgtttag 8700
   tcgcggatat ggaattcgat cctgccaaaa tcaatacatc atctatagat catgtaacaa 8760
   tattacaata catagatgaa ccaaatgata taagactaac agtatgcatt atccgaaata 8820
   ttatatcaat atcacaaaaa taaatacaca tttggctaat caatttcggg cttggaaaaa 8880
   acgtatcgcc ggaagggact atatgactaa cttatctaga gatacaggaa tacaacaatc 8940
   aaaacttact gaaactatac gtaactgtca aaaaaataga aacatatatg gtctatatat 9000
   acactacaat ttagttatta attggataac cgatgtgatt atcaatcaat attaagaggg 9060
   ttggtaaatt ggtacatagc taataatacc tatactccaa atacacccaa taatacaaca 9120
   accatttctg agttggatat catcaaaata ctaaataaat acgaggacgt gtatagagta 9180
   agtaaagaaa aagaatgtgg aatttgctat gaagttgttt actcaaaacg atagatactt 9240
   tggtttattg gattcgtgta ctcatatatt ttgcataaca tgcatcaata tatggcatag 9300
   aacacgaaga gaaaccggtg cgtcggataa ttgtcctata tgccgtaccc gttttagaaa 9360
   cataacaatg agcaagttct ataagctagt taactaataa ataaaaagtt taatttgttg 9420
   acgacgtatg tcgttatttt ttctcgtata aaagattaat ttgattctaa tataatcttt 9480
   agtattggat aaatatcaat tcaaattaat tccattagat tatatcataa ataaaaatag 9540
   tagcacacaa ctacttcagc aaacattctt tttataaatg ccatctagcg tagcgaggac 9600
   acaagtgaac ctataattat caaatttatt agtatcagtc acatgaagga ctttacgtag 9660
   agtgacgatt ccactatctg tggtacgaac ggtttcatct tctttgatgc catcacccag 9720
   atgttctata aacttggtat cctcgtccga tttcatatcc tttgccaacc aatacatata 9780
   gctaaactca ggcatatgtt ccacacatcc tgaacaatga aattctccag aagatgttac 9840
   aatgtctaga tttggacatt tggtttcaac cgcgttaaca tatgagtgaa cacacccata 9900
   catgaaagcg atgagaaata ggattctcat cttgccaaaa tatcactaga aaaaatttat 9960
   ttatcagttt taaaggtata aaaaatactt attgttgctc gaatattttg tatttgatgg 10020
   tatacggaag attagaaatg taggtattat catcaactga ttctatggtt ttatgtattc 10080
   tatcatgttt cactattgcg ttggaaataa tatcatatgc ttccacatat attttatttt 10140
   gttttaactc ataatactca cgtaattctg gattattggc atatctatga ataattttag 10200
   ctccatgatc agtaaatatt aatgagaaca tagtattacc acctaccatt atttttttca 10260
   tctcattcaa ttcttaattg caaagatcta tataatcatt atagcgttga cttatggact 10320
   ctggaatctt agacgatgta cagtcatcta taatcatggc atatttaata cattgtttta 10380
   tagcatagtc gttatctacg atgttagata tttctctcaa tgaatcaatc acacaatcta 10440
   atgtaggttt atgacataat agcattttca gcagttcaat gtttttagat tcgttgatgg 10500
   caatggctat acatgtatat ccgttatttg atctaatgtt gacatctgaa ccggattcta 10560
   gcagtaaaga tactagagat tgtttattat atctaacagc cttgtgaaga agtgtttctc 10620
   ctcgtttgtc aatcatgtta atgtctttaa gataaggtag gcaaatgttt atagtactaa 10680
   gaattgggca agcataagac atgtcacaaa gacccttttt tgtatgtata agtgtaaaaa 10740
   ttataacatc catagttgga tttacatagg tgtccaatcg ggatctctcc atcatcgaga 10800
   taattgatgg catctccctt ccttttttag tagatatttc atcgtgtaag aatcaatatt 10860
   aatatttcta aagtatccgt gtatagcctc tttatttacc acagctccat attccactag 10920
   agggatatcg ccgaatgtca tatactcaat tagtatatgt tggaggacat ccgagttcat 10980
   tgttttcaat atcaaagaga tggtttcctt atcatttctc catagtggta caatactaca 11040
   cattattccg tgcggctttc cattttccaa aaacaatttg accaaatcta aatctacatc 11100
   tttattgtat ctataatcac tatttagata atcagccata attcctcgag tgcaacatgt 11160
   tagatcgtct atatatgaat aagcagtgtt atctattcct ttcattaaca atttaacgat 11220
   gtctatatct atatgagatg acttaatata atattgaaga gctgtacaat agtttttatc 11280
   tataaaagac ggcttgattc cgtgattaat tagacattta acaacttccg gacgcacata 11340
   tgctctcgta tccgactctg aatacagatg agagatgata tacagatgca atacggtacc 11400
   gcaatttcgt agttgataat catcatacgc gtatcagtac tcgtcctcat aaagaacact 11460
   gcagccattt tctatgaaca aatcaataat tttagaaaca ggatcattgt cattacataa 11520
   ttttctataa ctgaacgatg gttttcacat ttaacactca agtcaaatcc atgttctacc 11580
   aacaccttta tcaagtcaac gtctacattt ttggatttca tatagctgaa tatattaaag 11640
   ttatttatgt tgctaaatcc agtggcttct agtagagcca tcgctatatc cttattaact 11700
   ttaacatgtc tactatttgt gtattcttct aatggggtaa gctgtctcca atttttgcgt 11760
   aatggattag tgccactgtc tagtagtagt ttgacgacct cgacattatt acaatgctca 11820
   ttaaaaaggt atgcgtgtaa agcattattc ttgaattggt tcctggtatc attaggatct 11880
   ctgtctttca acatctgttt aagttcatca agagccacct cctcattttc caaatagtca 11940
   aacattttga ctgaatgagc tactgtgaac tctatacacc cacacaacta atgtcattaa 12000
   atatcatgtc aaaaacttgt acaattatta ataaaaataa tttagtgttt aaattttacc 12060
   agttccagat tttacacctc cgttaatacc tccattaacc ccactggacg atcctcctcc 12120
   ccacattcca ccgccaccag atgtataagt tttagatcct ttattactac catcatgtcc 12180
   atggataaag acactccaca tgccgccact acccccttta gaagacatat taataagact 12240
   taaggacaag tttaacaata aaattaatca cgagtaccct actaccaacc tacactatta 12300
   tatgattata gtttctattt ttacagtacc ttgactaaag tttctagtca caagagcaat 12360
   actaccaacc tacactatta tatgattata gtttctattt ttataggaac gcgtacgaga 12420
   aaatcaaatg tctaatttct aacggtagtg ttgataaacg attgttatcc gcggatacct 12480
   cctctatcat gtcgtctatt ttcttacttt gttctattaa cttattagca ttatatatta 12540
   tttgattata aaacttatat tgcttattag cccaatctgt aaatatcgga ttattaacat 12600
   atcgtttctt tgtaggttta tttaacatgt acatcactgt aagcatgtcc ttaccattta 12660
   ttttaatttg acgcatatcc gcaatttctt tttcgcagtc ggttataaat tctatatatg 12720
   atggatacat gctacatgtg tacttataat cgactaatat gaagtacttg atacatattt 12780
   tcagtaacga tttattatta ccacctatga ataagtacct gtgatcgtct aggtaatcaa 12840
   ctgttttctt aatacattcg atggttggta atttactcag aataatttcc aatatcttaa 12900
   tatataattc tgctatttct gggatatatt tatctgccag tataacacaa atagtaatac 12960
   atgtaaaccc atattttgtt attatattaa tgtctgcgcc attatctatt aaccattcta 13020
   ctaggctgac actatgcgac tcaatacaat gataaagtat actacatcca tgtttatcta 13080
   ttttgtttat atcatcaata tacggcttac aaagttttag tatcgataac acatccaact 13140
   cacgcataga gaaggtaggg aataatggca taatatttat taggttatca tcattgtcat 13200
   tatctacaac taagtttcca ttttttaaaa tatactcgac aactttagga tctctattgc 13260
   caaatttttg aaaatattta tttatatgct taaatctata taatgtagct ccttcatcaa 13320
   tcatacattt aataacattg atgtatactg tatgataaga tacatattct aacaatagat 13380
   cttgtataga atctgtatat cttttaagaa ttgtggatat taggatatta ttacataaac 13440
   tattacacaa ttctaaaata taaaacgtat cacggtcgaa taatagttga tcaactatat 13500
   aattatcgat tttgtgattt ttcttcctaa actgtttacg taaatagtta gatagaatat 13560
   tcattagttc atgaccacta tagttactat cgaataacgc gtcaaatatt tcccgtttaa 13620
   tatcgcattt gtcaagataa taatagagtg tggtatgttc acgataagta taataacgca 13680
   tctctttttc gtgtgaaatt aaatagttta ttacgtccaa agatgtagca taaccatctt 13740
   gtgacctagt aataatataa taatagagaa ctgttttacc cattctatca tcataatcag 13800
   tggtgtagtc gtaatcgtaa tcgtctaatt catcatccca attataatat tcaccagcac 13860
   gtctaatctg ttctattttg atcttgtatc catactgtat gttgctacat gtaggtattc 13920
   ctttatccaa taatagttta aacacatcta cattgggatt tgatgttgta gcgtattttt 13980
   ctacaatatt aataccattt ttgatactat ttatttctat acctttcgaa attagtaatt 14040
   tcaataagtc tatattgatg ttatcagaac atagatattc gaatatatca aaatcattga 14100
   tatttttata gtcgactgac gacaataaca aaatcacaac atcgtttttg atattattat 14160
   ttttcttggt aacgtatgcc tttaatggag tttcaccatc atactcatat aatggatttg 14220
   caccactttc tatcaatgat tgtgcactgc tggcatcgat gttaaatgtt ttacaactat 14280
   catagagtat cttatcgtta accatgattg gttgttgatg ctatcgcatt ttttggtttc 14340
   tttcatttca gttatgtatg gatttagcac gtttgggaag catgagctca tatgatttca 14400
   gtactgtagt gtcagtacta ttagtttcaa taagatcaat ctctagatct atagaatcaa 14460
   aacacgatag gtcagaagat aatgaatatc tgtaggcttc ttgttgtact gtaacttctg 14520
   gttttgttag atggttgcat cgtgctttaa cgtcaatggt acaaatttta tcctcgcttt 14580
   gtgtatcata ttcgtcccta ctataaaatt gtatattcag attatcatgc gatgtgtata 14640
   cgctaacggt atcaataaac ggagcacacc atttagtcat aaccgtaatc caaaaatttt 14700
   taaagtatat cttaacgaaa gaagttgtgt cattgtctac ggtgtatggt actagatcct 14760
   cataagtgta tatatctaga gtaatgttta atttattaaa tggttgataa tatggatcct 14820
   cgtgacaatt tccgaagatg gaaataagac ataaacacgc aataaatcta attgcggaca 14880
   tggttactcc ttaaaaaaat acgaataatc accttggcta tttagtaagt gtcatttaac 14940
   actatactca tattaatcca tggactcata atctctatac gggattaacg gatgttctat 15000
   atacggggat gagtagttct cttctttaac tttatacttt ttactaatca tatttagact 15060
   gatgtatggg taatagtgtt tgaagagctc gttctcatca tcagaataaa tcaatatctc 15120
   tgtttttttg ttatacagat gtattacagc ctcatatatt acgtaataga acgtgtcatc 15180
   taccttatta actttcaccg catagttgtt tgcaaatacg gttaatcctt tgacctcgtc 15240
   gatttccgac caatctgggc gtataatgaa tctaaacttt aattgcttgt aatcattcga 15300
   aataattttt agtttgcatc cgtagttatc ccctttatgt aactgtaaat ttctcaacgc 15360
   gatatctcca ttaataatga tgtcgaattc gtgctgtata cccatactga atggatgaac 15420
   gaataccgac ggcgttaata gtaatttact ttttcatctt tacatattgg gtactagttt 15480
   tactatcata agtttataaa ttccacaagc tactatggaa taagccaacc atcttagtat 15540
   accacacatg tcttaaagtt tattaattaa ttacatgttg ttttatatat atcgctacga 15600
   atttaaagag aaattagttt aggaagaaaa attatctatc tacatcatca cgtctctgta 15660
   ttctacgata gagtgctact ttaagatgcg acagatccgt gtcatcaaat atatactcca 15720
   ttaaaatgat tattccggca gcgaacttga tattggatat atcacaacct ttgttaatat 15780
   ctacgacaat agacagcagt cccatggttc cataaacagt gagtttatct ttctttgaag 15840
   agatattttg tagagatctt ataaaactgt cgaatgacat cgcatttata tctttagcta 15900
   aatcgtatat gttaccatcg taatatctaa ccgcgtctat cttaaacgtt tccatcgctt 15960
   taaagacgtt tccgatagat ggtctcattt catcagtcat actgagccaa caaatataat 16020
   cgtgtataac atctttgata gaatcagact ctaaagaaaa cgaatcggct ttattatacg 16080
   cattcatgat aaacttaatg aaaaatgttt ttcgttgttt aagttggatg aatagtatgt 16140
   cttaataatt gttattattt cattaattaa tatttagtaa cgagtacact ctataaaaac 16200
   gagaatgaca taactaatca taactagtta tcaaagtgtc taggacgcgt aattttcata 16260
   tggtatagat cctgtaagca ttgtctgtat tctggagcta ttttctctat cgcattagtg 16320
   agttcagaat atgttataaa tttaaatcga ataacgaaca taactttagt aaagtcgtct 16380
   atattaactc ttttattttc tagccatcgt aataccatgt ttaagatagt atattctcta 16440
   gttactacga tctcatcgtt gtctagaata tcacatactg aatctacatc caattttaga 16500
   aattggtctg tgttacatat ctcttctata ttattgttga tgtattgtcg tagaaaacta 16560
   ttacgtagac cattttcttt ataaaacgaa tatatagtac tccaattatc tttaccgata 16620
   tatttgcaca cataatccat tctctcaatc actacatctt taagattttc gttgttaaga 16680
   tatttggcta aactatataa ttctattaga tcatcaacag aatcagtata tatttttcta 16740
   gatccaaaga cgaactcttt ggcgtcctct ataatattcc cagaaaagat attttcgtgt 16800
   tttagtttat cgagatctga tctgttcata tacgccatga ttgtacggta cgttatgata 16860
   accgcataaa ataaaaatcc attttcattt ttaaccaata ctattcataa ttgagattga 16920
   tgtaatactt tgttactttg aacgtaaaga cagtacacgg atccgtatct ccaacaagca 16980
   cgtagtaatc aaatttggtg ttgttaaact tcgcaatatt catcaattta gatagaaact 17040
   tatactcatc atctgtttta ggaatccatg tattattacc actttccaac ttatcattat 17100
   cccaggctat gtttcgtcca tcatcgttgc gcagagtgaa taattctttt gtattcggta 17160
   gttcaaatat atgatccatg catagatcgg caaagctatt gtagatgtga tttttcctaa 17220
   atctaatata aaactcgttt actagcaaac actttcctga tttatcgacc aagacacata 17280
   tggtttctaa atctatcaag tggtggggat ccatagttat gacgcagtaa catatattat 17340
   tacattcttg actgtcgcta atatctaaat atttattgtt atcgtattgg attctgcata 17400
   tagatggctt gtatgtcaaa gatatagaac acataaccaa tttatagtcg cgctttacat 17460
   tctcgaatct aaagttaaga gatttagaaa acattatatc ctcggatgat gttatcactg 17520
   tttctggagt aggatatatt aaagtcttta cagatttcgt ccgattcaaa taaatcacta 17580
   aataatatcc cacattatca tctgttagag tagtatcatt aaatctatta tattttatga 17640
   aagatatatc actgctcacc tctatatttc gtacattttt aaactgtttg tataatatct 17700
   ctctgataca atcagatata tctattgtgt cggtagacga taccgttaca tttgaattaa 17760
   tggtgttcca ttttacaact tttaacaagt tgaccaattc atttctaata gtatcaaact 17820
   ctccatgatt aaatatttta atagtatcca ttttatatca ctacggacac aaagtagctg 17880
   acataaacca ttgtataatt tttatgtttt atgtttatta gcgtacacat tttggaagtt 17940
   ccggcttcca tgtatttcct ggagagcaag tagatgatga ggaaccagat agtttatatc 18000
   cgtacttgca cttaaagtct acattgtcgt tgtatgagta tgatctttta aacccgctag 18060
   acaagtatcc gtttgatatt gtaggatgtg gacatttaac aatctgacac gtgggtggat 18120
   cggaccattc tcctcctgaa cacaggacac tagagttacc aatcaacgaa tatccactat 18180
   tgcaactata agttacaacg ctcccatcgg tataaaaatc ctcgtatccg ttatgtcttc 18240
   cgttggatat agatggaggg gattggcatt taacagattc acaaataggt gcctcgggat 18300
   tccataccat agatccagta gatcctaatt cacaatacga tttagattca ccgatcaact 18360
   gatatccgct attacaagag tacgttatac tagagccaaa gtctactcca ccaatatcaa 18420
   gttggccatt atcgatatct cgaggcgatg ggcatctccg tttaatacat tgattaaaga 18480
   gtgtccatcc agtacctgta catttagcat atataggtcc cattttttgc tttctgtatc 18540
   caggtagaca tagatattct atagtgtctc ctatgttgta attagcatta gcatcagtct 18600
   ccacactatt cttaaatttc atattaatgg gtcgtgacgg aatagtacag catgatagaa 18660
   cgcatcctat tcccaacaat gtcaggaacg tcacgctctc caccttcata tttatttatc 18720
   cgtaaaaatg ttatcctgga catcgtacaa ataataaaaa gcccatatat gttcgctatt 18780
   gtagaaattg tttttcacag ttgctcaaaa acgatggcag tgacttatga gttacgttac 18840
   actttggagt ctcatcttta gtaaacatat cataatattc gatattacga gttgacatat 18900
   cgaacaaatt ccaagtattt gattttggat aatattcgta ttttgcatct gctataatta 18960
   agatataatc accgcaagaa cacacgaaca tctttcctac atggttaaag tacatgtaca 19020
   attctatcca tttgtcttcc ttaactatat atttgtatag ataattacga gtctcgtgag 19080
   taattccagt aattacatag atgtcgccgt cgtactctac agcataaact atactatgat 19140
   gtctaggcat gggagacttt tttatccaac gatttttagt gaaacattcc acatcgttta 19200
   atactacata tttctcatac gtggtataaa ctccacccat tacatatata tcatcgttta 19260
   cgaataccga cgcgcctgaa tatctaggag taattaagtt tggaagtctt atccatttcg 19320
   aagtgccgtg tttcaaatat tctgccacac ccgttgaaat agaaaattct aatcctccta 19380
   ttacatataa ctttccatcg ttaacacaag tactaacttc tgattttaac gacgacatat 19440
   tagtaaccgt tttccatttt ttcgttttaa gatctacccg cgatacggaa taaacatgtc 19500
   tattgttaat catgccgcca ataatgtata gacaattatg taaaacattt gcattataga 19560
   attgtctatc tgtattaccg actatcgtcc aatattctgt tctaggagag taatgggtta 19620
   ttgtggatat ataatcagag tttttaatga ctactatatt atgttttata ccatttcgtg 19680
   tcactggctt tgtagatttg gatatagtta atcccaacaa tgatatagca ttgcgcatag 19740
   tattagtcat aaacttggga tgtaaaatgt tgatgatatc tacatcgttt ggatttttat 19800
   gtatccactt taataatatc atagctgtaa catcctcatg atttacgtta acgtcttcgt 19860
   gggataagat agttgtcagt tcatcctttg ataattttcc aaattctgga tcggatgtca 19920
   ccgcagtaat attgttgatt atttctgaca tcgacgcatt atatagtttt ttaattccat 19980
   atcttttaga aaagttaaac atccttatac aatttgtgaa attaatatta tgaatcatag 20040
   tttttacaca tagatctact acaggcggaa catcaattat tatggcagca actagtatca 20100
   tttctacatt gtttatggtg atgtttatct tcttccagcg catatagtct aatagcgatt 20160
   caaacgcgtg atagtttata ccattcaata taatcgcttc atcctttaga tggtgatcct 20220
   gaatgcgttt aaaaaaatta tacggagacg ccgtaataat ttccttattc acttgtataa 20280
   tttccccatt gatagaaaat attacgcttt ccattcttaa agtactataa gtaattatag 20340
   tataatgtaa acgtttatat attcaatatt tttataaaaa tcattttgac attaattcct 20400
   ttttaaattt ccgtctatca tctatagaaa cgtattctat gaatttataa aatgctttta 20460
   cgtgtcctat cgtaggcgat agaaccgcta aaaagcctat cgaatttcta caaaagaatc 20520
   tgttatatgg tatagggaga gtataaaaca ttaaatgtcc gtacttatta aagtattcag 20580
   tagccaatcc taactctttc gaatacttat taatggctct tgttctgtac gaatctattt 20640
   ttttgaacaa cggacctagt ggtatatctt gttctatgta tctaaaataa tgtctgacta 20700
   gatccgttag tttaatatcc gcagtcatct tgtctagaat ggcaaatcta actgcgggtt 20760
   taggctttag tttagtttct atatctacat ctatgtcttt atctaacacc aaaaatataa 20820
   tagctaatat tttattacaa tcatccggat attcttctac gatctcacta actaatgttt 20880
   ctttggttat actagtatag tcactatcgg acaaataaag aaaatcagat gatcgatgaa 20940
   taatacattt aaattcatca tctgtaagat ttttgagatg tctcattaga atattattag 21000
   ggttagtact cattatcatt cggcagctat tacttatttt attatttttc accatataga 21060
   tcaatcatta gatcatcaaa atatgtttca atcatcctaa agagtatggt gaatgactct 21120
   tcccatctaa tttctgaacg ttcaccaatg tctctagcca ctttggcact aatagcgatc 21180
   attcgcttag cgtcttctat attattaact ggttgattta atctatctag caatggaccg 21240
   tcggacagcg tcattctcat gttcttaatc aatgtacata catcgccgtc atctaccaat 21300
   tcatccaaca acataagctt tttaaaatca tcattataat aggtttgatc gttgtcattt 21360
   ctccaaagaa tatatctaat aagtagagtc ctcatgctta gttaacaact attttttatg 21420
   ttaaatcaat tagtacaccg ctatgtttaa tacttattca tattttagtt tttaggattg 21480
   agaatcaata caaaaattaa tgcatcatta attttagaaa tacttagttt ccacgtagtc 21540
   aatgaaacat ttgaactcat cgtacaggac gttctcgtac aggacgtaac tataaaccgg 21600
   tttatatttg ttcaagatag atacaaatcc gataactttt tttacgaatt ctacgggatc 21660
   cactttaaaa gtgtcatacc gggttctttt tattttttta aacagatcaa tggtgtgatg 21720
   ttgattaggt cttttacgaa tttgatatag aatagcgttt acatattctc cataatggtc 21780
   aatcgccatt tgttcgtatg tcataaattc tttaattata tgacactgtg tattatttag 21840
   ttcatccttg ttcattgtta ggaatctatc caaaatggca attatactag aactataggt 21900
   gcgttgtata cacatattga tgtgtctgtt tatacaatcc atgatatttg gatccatgct 21960
   actaccttcg ggtaaaattg tagcatcata taccatttct agtactttag gttcattatt 22020
   atccattgca gaggacgtca tgatcgaatc ataaaaaaat atattatttt tatgttattt 22080
   tgttaaaaat aatcatcgaa tacttcgtaa gatactcctt catgaacata atcagttaca 22140
   aaacgtttat atgaagtaaa gtatctacga tttttacaaa agtccggatg cataagtaca 22200
   aagtacgcga taaacggaat aataatagat ttatctagtc tatctttttc tatagctttc 22260
   atagttagat acatggtctc agaagtagga ttatgtaaca tcagcttcga taaaatgact 22320
   gggttattta gtcttacaca ttcgctcata catgtatgac cgttaactac agagtctaca 22380
   ctaaaatgat tgaacaatag atagtctacc attgtttcgt attcagatag tacagcgtag 22440
   tacatggcat cttcacaaat tatatcattg tctaatagat atttgacgca tcttatggat 22500
   cccacttcaa cagccatctt aaaatcatat tgctttcctt tatcattaat aatttctaga 22560
   acatcatctc tatcataaaa gatacaaata ttaactgttt gatccgtaat aacattgcta 22620
   gtcaatagca atttgttaat aagatgcgct gggctcaatg tcttaataag aagtgtaaga 22680
   ggactatctc cgaatttgtt ttgtttatta acatccgttg atggaagtaa aagatctata 22740
   atgtctacat tcttgactgt tttagagcat acaatatgga gaggtgtatt tccatcatga 22800
   tctggttttg agggactaat tcctagtttc atcatccatg agattgtaga agcttttgga 22860
   ttgtctgaca taagatgtct atgaatatga tttttgccaa atttatccac tatcctggct 22920
   tcgaatccga tggacattat ttttttaaac actctttctg aaggatctgt acacgccaac 22980
   aacggaccac atccttcttc atcaaccgag ttgttaatct tggctccata ctgtaccaat 23040
   aaatttattc tctctatgac ttcatcatct gttcccgaga gataatatag aggtgtttta 23100
   tgctgtttat cacacgcgtt tggatctgcg ccgtgcgtca gcagcatcgc gactattcta 23160
   ttattattaa ttttagaagc tatatgcaat ggataatttc catcatcatc cgtctcattt 23220
   ggagagtatc ctctatgaag aagttcttcg acaaatcgtt catctagtcc tttaattcca 23280
   caatacgcat gtagaatgtg ataattattt ccagaaggtt cgatagcttg tagcatattc 23340
   ctaaatacat ctaaattttt actattatat ttggcataaa gagatagata atactcggcc 23400
   gacataatgt tgtccattgt agtataaaaa ttaatatttc tatttctatt tctgtatatt 23460
   tgcaacaatt tactctctat aacaaatatc ataacttagt ttttttatgt caagaaggca 23520
   ctggtttagt tcatctataa atgtcacgcc ataactacca cgcatgccat actcagaatt 23580
   atgataaaga tatttatcct tggggtgtag gtaatgggga ttaatctttg ttggatcagt 23640
   ctctaagtta acacatgtca cacatgatcc atttatagtt atatcacacg atgatgattt 23700
   atgaattgat tccggaagat cgctatcgta ttttgtggtt ccacaattca tttccataca 23760
   tgttattgtc acactaatat tatgatgaac tttatctagc cgctgagtgg taaacaacag 23820
   aacagatagt ttattatctt taccaacacc ctcagccgct gccacaaatc tctgatccgt 23880
   atccatgatg gtcatgttta tttctagtcc gtatccagtc aacactatgt tagcatttct 23940
   gtcgatatag ctttcactca tatgacactc accaataata gtagaattaa tgtcgtaatt 24000
   tacaccaata gtgagttcgg cggcaaagta ccaataccgg taatcttgtc gaggaggaca 24060
   tatagtattc ttgtattcta ccgaataccc gagagatgcg atacaaaaga gcaagactaa 24120
   tttgtaaacc atcttactca aaatatgtaa caatagtacg atgcaatgag taagacaata 24180
   ggaaatctat cttatataca cataattatt ctatcaattt taccaattag ttagtgtaat 24240
   gttaacaaaa atgtgggaga atctaattag tttttcttta cacaattgac gtacatgagt 24300
   ctgagttcct tgtttttgct aattatttca tccaatttat tattcttgac gatatcgaga 24360
   tcttttgtat aggagtcaaa cttgtattca acatgctttt ctataatcat tttagctatt 24420
   tcggcatcat ccaatagtac attttccaga ttagcagaat agatattaat gtcgtatttg 24480
   aacagagcct gtaacatctc aatgtcttta ttatctatag ccaatttaat gtccggaatg 24540
   aagagaaggg aattattggt gtttgtcgac gtcatatagt cgagcaagag aatcatcata 24600
   tccacgtgtc cattttttat agtgatgtga atacaactaa ggagaatagc cagatcaaaa 24660
   gtagatggta tctctgaaag aaagtaggaa acaatactta catcattaag catgacggca 24720
   tgataaaatg aagttttcca tccagttttc ccatagaaca tcagtctcca atttttctta 24780
   acaaacagtt ttaccgtttg catgttacca ctatcaaccg cataatacaa tgcggtgttt 24840
   cccttgtcat caaattgtga atcatccagt ccactgaata gcaaaatctt tactattttg 24900
   gtatcttcca atgtggctgc ctgatgtaat ggaaattcat tctctagaag atttttcaat 24960
   gctccagcgt tcaacaacgt acatactaga cgcacgttat tatcagctat tgcataatac 25020
   aaggcactat gtccatggac atccgcctta aatgtatctt tactagagag aaagcttttc 25080
   agctgcttag acttccaagt attaattcgt gacagatcca tgtctgaaac gagacgctaa 25140
   ttagtgtata ttttttcatt ttttataatt ttgtcatatt gcaccagaat taataatatc 25200
   tttaatagat ctgattagta gatacatggc tatcgcaaaa caacatatac acatttaata 25260
   aaaataatat ttattaagaa aattcagatt tcacgtaccc atcaatataa ataaaataat 25320
   gattccttac accgtaccca tattaaggag attccacctt acccataaac aatataaatc 25380
   cagtaatatc atgtctgatg atgaacacaa atggtgtatt aaattccagt ttttcaggag 25440
   atgatctcgc cgtagctacc ataatagtag atgcctctgc tacagttcct tgttcgtcga 25500
   catctatctt tgcattctga aacattttat aaatatataa tgggtcccta gtcatatgtt 25560
   taaacgacgc attatctgga ttaaacatac taggagccat catttcggct atcgacttaa 25620
   tatccctctt attttcgata gaaaatttag ggagtttaag attgtacact ttattcccta 25680
   attgagacga ccaatagtct aattttgcag ccgtgataga atctgtgaaa tgggtcatat 25740
   tatcacctat tgccaggtac atactaatat tagcatcctt atacggaagg cgtaccatgt 25800
   catattcttt gtcatcgatt gtgattgtat ttccttgcaa tttagtaact acgttcatca 25860
   tgggaaccgt tttcgtaccg tacttattag taaaactagc attgcgtgtt ttagtgatat 25920
   caaacggata ttgccatata cctttaaaat atatagtatt aatgattgcc catagagtat 25980
   tattgtcgag catattagaa tctactacat tagacatacc ggatctacgt tctactatag 26040
   aattaatttt attaaccgca tctcgtctaa agtttaatct atataggccg aatctatgat 26100
   attgttgata atacgacggt ttaatgcaca cagtattatc tacgaaactt tgataagtta 26160
   gatcagtgta cgtatattta gatgttttca gcttagctaa tcctgatatt aattctgtaa 26220
   atgctggacc cagatctctt tttctcaaat ccatagtctt caataattct attctagtat 26280
   tacctgatgc aggcaatagc gacataaaca tagaaaacga ataaccaaac ggtgagaaga 26340
   caatattatc atcttgaata tttttatacg ctactatacc ggcattggta aatccttgta 26400
   gacgataggc ggacgctgaa cacgctaacg atagtatcaa taacgcaatc atgattttat 26460
   ggtattaata attaacctta tttttatgtt cggtataaaa aaattattga tgtctacaca 26520
   tccttttgta attgacatct atatatcctt ttgtataatc aactctaatc actttaactt 26580
   ttacagtttt ccctaccagt ttatccctat attcaacata tctatccata tgcatcttaa 26640
   cactctctgc caagatagct tcagagtgag gatagtcaaa aagataaata tatagagcat 26700
   aatcattctc gtatactctg ccctttatta catcacccgc attgggcaac gaataacaaa 26760
   atgcaagcat cttgttaacg ggctcgtaaa ttgggataaa aattatgttt ttattgtctt 26820
   atatctattt tattcaagag aatattcagg aatttctttt tccggttgta tctcgtcgca 26880
   gtatatatca tttgtacatt gtttcatatt ttttaatagt ttacaccttt tagtaggact 26940
   agtatcgtac aattcatagc tgtattttga attccaatca cgcataaaaa tatcttccaa 27000
   ttgttgacga agacctaatc catcatccgg tgtaatatta atagatgctc cacatgtatc 27060
   cgtaaagtaa tttcctgtcc aatttgaggt acctatatag gccgttttat cggttaccat 27120
   atatttggca tggtttaccc tagaatacgg aatgggagga tcagcatctg gtacaataaa 27180
   tagctttact tctatattta tgtttttaga ttttagcata gcgatagatc ttaaaaagtt 27240
   tctcatgata aacgaagatc gttgccagca actaatcaat agcttaacgg atacttgtct 27300
   gtctatagcg gatcttctta attcatcttc tatataaggc caaaacaaaa ttttacccgc 27360
   cttcgaataa ataataggga taaagttcat aacagataca taaacgaatt tactcgcatt 27420
   tctaatacat gacaataaag cggttaaatc attggttctt tccatagtac atagttgttg 27480
   cggtgcagaa gcaataaata cagagtgtgg aacgccgctt acgttaatac taagaggatg 27540
   atctgtatta taatacgacg gataaaagtt tttccaatta tatggtagat tgttaactcc 27600
   aagataccag tatacctcaa aaatttgagt gagatccgct gccaagttcc tattattgaa 27660
   gatcgcaata cccaattcct tgacctgagt tagtgatctc caatccatgt tagcgcttcc 27720
   taaataaata tgtgtattat cagatatcca aaattttgta tgaagaactc ctcctaggat 27780
   atttgtaata tctatgtatc gtacttcaac tccggccatt tgtagtcttt caacatcctt 27840
   taatggtttg ttagatttat taacggctac tctaactcgt actcctcttt tgggtaattg 27900
   tacaatctcg tttaatatta tcgtgccgaa attcgtaccc acttcatccg ataaactcca 27960
   ataaaaagat gatatatcta gtgtttttgt ggtattggat agaatttccc tccacatgtt 28020
   aaatgtagac aaatatactt tatcaaattg catacctata ggaatagttt ctgtaatcac 28080
   tgcgattgta ttatccggat tcattttatt tgttaaaaga ataatcctat atcacttcac 28140
   tctattaaaa atccaagttt ctatttcttt catgactgat tttttaactt catccgtttc 28200
   cttatgaaga tgatgtttgg caccttcata aatttttatt tctctattac aatttgcatg 28260
   ttgcatgaaa taatatgcac ctaaaacatc gctaatctta ttgtttgttc cctggagtat 28320
   gagagtcggg gggtgttaat cttggaaatt atttttctaa ccttgttggt agccttcaag 28380
   acctgactag caaatccagc cttaattttt tcatgattga ctaatgggtc gtattggtat 28440
   ttataaactt tatccatatc tctagatact gattctggac atagctttcc gactggcgca 28500
   tttggtgtga tggttcccat aagtttggca gctagcagat tcagtcttga aacagcatct 28560
   gcattaacta gaggagacat tagaatcatt gctgtaaaca agtttggatt atcgtaagag 28620
   gctagctccc atggaatgac ccaataagta gatttaatag ttaccacgtg ctgtaccaaa 28680
   gtcatcaatc atcatttttt caccattact tcttccatgt ccaatatgat catgtgagaa 28740
   tactaaaatt cctaacgatg atatgttttc agctagttcg tcataacgtc cagaatgttt 28800
   accagctcca tgacttatga atactaatgc cttaggatat gtaataggtt tccaatattt 28860
   acaatatatg taatcattgt ccagattgaa catacagttt gcactcatga ttcacgttat 28920
   ataactatca atattaacag ttcgtttgat gatcatatta tttttatgtt ttattgataa 28980
   ttgtaaaaac atacaattaa atcaatatag aggaaggaga cggctactgt cttttgtaag 29040
   atagtcatgg cgactaaatt agattatgag gatgctgttt tttactttgt ggatgatgat 29100
   aaaatatgta gtcgcgactc catcatcgat ctaatagatg aatatattac gtggagaaat 29160
   catgttatag tgtttaacaa agatattacc agttgtggaa gactgtacaa ggaattgatg 29220
   aagttcgatg atgtcgctat acggtactat ggtattgata aaattaatga gattgtcgaa 29280
   gctatgagcg aaggagacca ctacatcaat tttacaaaag tccatgatca ggaaagttta 29340
   ttcgctacca taggaatatg tgctaaaatc actgaacatt ggggatacaa aaagatttca 29400
   gaatctagat tccaatcatt gggaaacatt acagatttga tgaccgacga taatataaac 29460
   atcttgatac tttttctaga aaaaaaattg aattgatgat ataggggtct tcataacgca 29520
   taattattac gttagcattc tatatccgtg ttaaaaaaaa ttatcctatc atgtatttga 29580
   gagttttata tgtagcaaac atgatagctg tgatgccaat aagctttaga tattcacgcg 29640
   tgctagtgtt agggatggta ttatctggtg gtgaaatgtc cgttatataa tctacaaaac 29700
   aatcatcgca tatagtatgc gatagtagag taaacatttt tatagttttt actggattca 29760
   tacatcgtct acccaattcg gttataaatg aaattgtcgc caatcttaca cccaacccct 29820
   tgttatccat tagcatagta ttaacttcgt tatttatgtc ataaactgta aatgattttg 29880
   tagatgccat atcatacatg atattcatgt ccctattata atcattacta actttatcac 29940
   aatatatgtt gataatatct atatatgatc tagtctttgt gggcaactgt ctatacaagt 30000
   cgtctaaacg ttgtttactc atatagtatc gaacagccat cattacatgg tcccgttccg 30060
   ttgatagata atcgagtatg ttagtggact tgtcaaatct atataccata ttttctggaa 30120
   gtggatatac atagtcgtga tcaacattat tgctagcctc atcttctata tcctgtacta 30180
   taccattatc tatatcatct acataatcta tgatattatt acacataaac atcgacaaca 30240
   tactattgtt tattatctaa gtcctgttga tccaaaccct tgatctcctc tatttgtact 30300
   atctagagat tgtacttctt ccagttctgg ataatatata cgttgataga ttagctgagc 30360
   tattctatct ccagtattta cattaaacgt acattttcca ttattaataa gaatgactcc 30420
   tatgtttccc ctataatctt cgtctattac accacctcct atatcaatgc cttttagtga 30480
   cagaccagac ctaggagcta ttctaccata gcagaactta ggcatggaca tactaatatc 30540
   tgtcttaatt aactgtcttt ctcctggagg gatagtataa tcgtaagcgc tatacaaatc 30600
   atatccggca gcacccggcg attgcctagt aggagattta gctctgttag tttccttaac 30660
   aaatctaact ggtgagttaa tattcatgtt gaacataaaa ctaatatttt atttcaaaat 30720
   tatttaccat cccatatatt ccatgaataa gtgtgatgat tgtacacttc tatagtatct 30780
   atatacgatc cacgataaaa tcctcctatc aatagcagtt tattatccac tatgatcaat 30840
   tctggattat ccctcggata aataggatca tctatcagag tccatgtatt gctggattca 30900
   caataaaatt ccgcatttct accaaccaag aataaccttc taccgaacac taacgcgcat 30960
   gatttataat gaggataata agtggatggt ccaaactgcc actgatcatg attgggtagc 31020
   aaatattctg tagttgtatc agtttcagaa tgtcctccca ttacgtatat aacattgttt 31080
   atggatgcca ctgctggatt acatctaggt ttcagaagac tcggcatatt aacccaagca 31140
   gcatccccgt ggaaccaacg ctcaacagat gtgggatttg gtagacctcc tactacgtat 31200
   aatttattgt tagcgggtat cccgctagca tacagtctgg ggctattcat cggaggaatt 31260
   ggaatccaat tgtttgatat ataatttaca gctatagcat tgttatgtat ttcattgttc 31320
   atccatccac cgatgagata tactacttct ccaacatgag tacttgtaca catatggaat 31380
   atatctataa tttgatccat gttcatagga tactctatga atggatactt gtatgatttg 31440
   cgtggttgtt tatcacaatg aaatattttg gtacagtcta gtatccattt tacattattt 31500
   atacctctgg gagaaagata atttgacctg attacatttt tgataaggag tagcagattt 31560
   cctaatttat ttcttcgcct catataccac ttaatgacaa aatcaactac ataatcctca 31620
   tctggaacat ttagttcatc gctttctaga ataagtttca tagatagata atcaaaattg 31680
   tctatgatgt catcttccag ttccaaaaag tgtttggcaa taaagttttt agtatgacat 31740
   aagagattgg atagtccgta ttctataccc atcatgtaac actcgacaca atattccttt 31800
   ctaaaatctc gtaagataaa gtttatacaa gtgtagatga taaattctac agaggttaat 31860
   atagaagcac gtaataaatt gacgacgtta tgactatcta tatatacctt tccagtatat 31920
   gagtaaataa ctatagaagt taaactgtga atgtcaaggt ctagacaaac ccttgtaact 31980
   ggatctttat ttttcgtgta tttttgacgt aaatgtgtgc gaaagtaagg agataacttt 32040
   ttcaatatcg tagaattgac tattatattg cctcctatgg catcaataat tgttttgaat 32100
   ttcttagtca tagacaatgc taatatattc ttacagtaca cagtattgac aaatatcggc 32160
   atttatgttt ctttaaaagt caacatctag agaaaaatga ttatcttttt gagacataac 32220
   tcccattttt tggtattcac ccacacgttt ttcgaaaaaa ttagtttttc cttccaatga 32280
   tatattttcc atgaaatcaa acggattggt aacattataa atttttttaa atcccaattc 32340
   agaaatcaat ctatccgcga cgaattctat atatgttttc atcatttcac aattcattcc 32400
   tataagttta actggaagag ccgcagtaag aaattcttgt tcaatggata ctgcatctgt 32460
   tataatagat ctaacggttt cttcactcgg tggatgcaat aaatgtttaa acatcaaaca 32520
   tgcgaaatcg cagtgcagac cctcgtctct actaattagt tcgttggaaa acgtgagtcc 32580
   gggcattagg ccacgctttt taagccaaaa tatggaagcg aatgatccag aaaagaaaat 32640
   tccttctact gcagcaaagg caataagtct ctctccataa ccggcgctgt catgtatcca 32700
   cttttgagcc caatcggcct tcttttttac acaaggcatc gtttctatgg cattaaagag 32760
   atagtttttt tcattactat ctttaacata agtatcgatc aaaagactat acatttccga 32820
   atgaatgttt tcaatggcca tctgaaatcc gtagaaacat ctagcctcgg taatctgtac 32880
   ttctgtacaa aatcgttccg ccaaattttc attcactatt ccgtcactgg ctgcaaaaaa 32940
   cgccaataca tgttttataa aatatttttc gtctggtgtt agtttattcc aatcattgat 33000
   atctttagat atatctactt cttccactgt ccaaaatgat gcctctgcct ttttatacat 33060
   gttccagatg tcatgatatt ggattgggaa aataacaaat ctatttggat ttggtgcaag 33120
   gatgggttcc ataactaaat taacaataac aataaatttt ttttcagtta tctatatgcc 33180
   tgtacttgga ttttttgtac atcgatatcg ccgcaatcac tacaataatt acaagtatta 33240
   ttgatagcat tgttattagt actatcataa ttaaattatc tacattcatg ggtgctgaat 33300
   aatcgttatt atcatcatta tcattttgta attgtgacat catactagat aaatcgtttg 33360
   cgagattgtt gtgggaagcg ggcatggagg atgcattatc attattattt aacgccttcc 33420
   atttggattc acaaatgtta cgcacattca acattttatg gaaactataa ttttgtgaaa 33480
   acaaataaca agaaaactcg tcatcgttca aatttttaac gatagtaaac cgattaaacg 33540
   tcgagctaat ttctaacgct agcgactctg ttggatatgg gtttccagat atatatcttt 33600
   tcagttcccc tacgtatcta taatcatctg taggaaatgg aagatatttc catttatcta 33660
   ctgttcctaa tatcatatgt ggtggtgtag tagaaccatt aagcgcgaaa gatgttattt 33720
   cgcatcgtat tttaacttcg caataatttc tggttagata acgcactcta ccagtcaagt 33780
   caatgatatt agcctttaca gatatattca tagtagtcgt aacgatgact ccatctttta 33840
   gatgcgatac tcctttgtat gtaccagaat cttcgtacct caaactcgat atatttaaac 33900
   aagttaatga gatattaacg cgttttatga atgatgatat ataaccagaa gttttatcct 33960
   cggtggctag cgctataacc ttatcattat aataccaact agtgtgatta atatgtgaca 34020
   cgtcagtgtg ggtacaaata tgtacattat cgtctacgtc gtattcgata catccgcata 34080
   cagccaacaa atataaaatg acaaatactc taacgccgtt cgtacccatc ttgatgcggt 34140
   ttaataaatg ttttgatttc aatttattgt aaaaaaagat tcggttttat actgttcgat 34200
   attctcattg cttatatttt catctatcat ctccacacag tcaaatccgt ggttagcatg 34260
   cacctcatca accggtaaaa gactatcgga ctcttctatc attataactc tagaatattt 34320
   aatttggtca ttattaatca agtcaattat cttattttta acaaacgtga gtattttact 34380
   cattttttat aaaaactttt agaaatatac agactctatc gtgtgtctat atcttctttt 34440
   tatatccaat gtatttatgt ctgatttttc ttcatttatc atatataatg gtccaaattc 34500
   tacacgtgct tcggattcat ccagatcatt aaggttctta taattgtaac atccttctct 34560
   tccctcttct acatcttcct tcttattctt attcttagcg tcacagaatc taccacagca 34620
   ggatcccatg acgagcgtca tattaaacta atccattttc aattataata tatgattagt 34680
   aatgaccatt aaaataaaaa atattcttca taaccggcaa gaaagtgaaa agttcacatt 34740
   gaaactatgt cagtagtata catcatgaaa tgatgatata tatatactct attttggtgg 34800
   aggattatat gatataattc gtggataatc atttttaaga cacatttctt tattcgtaaa 34860
   tcttttcacg ttaaatgagt gtccatattt tgcaatttct tcatatgatg gcggtgtacg 34920
   tggacgaggc tgctcctgtt cttgttgtgg tcgccgactg tcgtgtctgc gtttagatcc 34980
   ctccattatc gcgattgcgt agatggagta ctattttata ccttgtaatt aaattttttt 35040
   attaattaaa cgtataaaaa cgttccgtat ctgtatttaa gagccagatt tcgtctaata 35100
   gaacaaatag ctacagtaaa aataactaga ataattgcta cacccactag aaaccacgga 35160
   tcgtaatacg gcaatcggtt ttcgataata ggtggaacgt atattttatt taaggactta 35220
   acaattgtct gtaaaccaca atttgcttcc gcggatcctg tattaactat ctgtaaaagc 35280
   atatgttgac cgggcggagc cgaacattct ccgatatcta atttctgtat atctataata 35340
   ttattaacct ccgcatacgc attacagttc ttttctagct tggataccgc actaggtaca 35400
   tcgtctagat ctattcctat ttcctcagcg atagctcttc tatccttttc cggaagcaat 35460
   gaaatcactt caataaatga ttcaaccatg agtgtgaaac taagtcgaga attactcatg 35520
   catttgttag ttattcggag cgcgcaattt ttaaactgtc ctataacctc tcctatatga 35580
   atagcacaag tgacattagt agggatagaa tgttgagcta atttttgtaa ataactatct 35640
   ataaaaagat tatacaaagt tttaaactct ttagtttccg ccatttatcc agtctgagaa 35700
   aatgtctctc ataataaatt tttccaagaa actaattggg tgaagaatgg aaacctttaa 35760
   tctatattta tcacagtctg ttttggtaca catgatgaat tcttccaatg ccgtactaaa 35820
   ttcgatatct ttttcgattt ctggatatgt ttttaataaa gtatgaacaa agaaatggaa 35880
   atcgtaatac cagttatgtt taactttgaa attgtttttt attttcttgt taatgattcc 35940
   agccacttgg gaaaagtcaa agtcgtttaa tgccgattta atacgttcat taaaaacaaa 36000
   ctttttatcc tttagatgaa ttattattgg ttcattggaa tcaaaaagta agatattatc 36060
   gggtttaaga tctgcgtgta aaaagttgtc gcaacagggt agttcgtaga ttttaatgta 36120
   taacagagcc atctgtaaaa agataaactt tatgtattgt accaaagatt taaatcctaa 36180
   tttgatagct aactcggtat ctactttatc tgccgaatac agtgctaggg gaaaaattat 36240
   aatgtttcct ctttcatatt cgtagttagt tctcttttca tgttcgaaaa agtgaaacat 36300
   gcggttaaaa tagtttataa cattaatatt actgttaata actgccggat aaaagtggga 36360
   tagtaatttc acgaatttga tactgtcctt tctctcgtta aacgccttta aaaaaacttt 36420
   agaagaatat ctcaatgaga gttcctgacc atccatagtt tgtatcaata atagcaacat 36480
   atgaagaaca cgtttataca gagtatgtaa aaatgttaat ttatagttta atcccatggc 36540
   ccacgcacac acgattaatt ttttttcatc tccctttaga ttgttgtata gaaatttggg 36600
   tactgtgaac tccgccgtag tttccatggg actatataat tttgtggcct cgaatacaaa 36660
   ttttactaca tagttatcta tcttaaagac tataccatat cctcctgtag atatgtgata 36720
   aaaatcgtcg tttataggat aaaatcgttt atccttttgt tggaaaaagg atgaattaat 36780
   gtaatcattc tcttctatct ttagtagtgt ttccttatta aaattcttaa aataatttaa 36840
   caatctaact gacggagccc aattttggtg taaatctaat tgggacatta tattgttaaa 36900
   atacaaacag tctcctaata taacagtatc tgataatcta tggggagaca tccattgata 36960
   ttcaggggat gaatcattgg caacacccat ttattgtaca aaaagcccca atttacaaac 37020
   gaaagtccag gtttgataga gacaaacaat taactatttt gtctctgttt ttaacacctc 37080
   cacagttttt aatttcttta gtaatgaaat tattcacaat atcagtatct tctttatcta 37140
   ccagagattt tactaacttg ataaccttgg ctgtctcatt caatagggta gtaatatttg 37200
   tatgtgtgat attgatatct ttttgaattg tttcttttag aagtgattct ttgatggtgc 37260
   cagcatacga attacaataa tgcagaaact cggttaacat gcaggaatta tagtaagcca 37320
   attccaattg ttgcctgtgt tgtattagag tgtcaatatg agcaatggtg tccttgcgtt 37380
   tctctgatag aatgcgagca gcgattttgg cgttatcatt tgacgatatt tctggaatga 37440
   cgaatcctgt ttctactaac tttttggtag gacaaagtga aacaatcaag aagatagctt 37500
   ctcctcctat ttgtggaaga aattgaactc ctctagatga tctactgacg atagtatctc 37560
   cttgacagat attggaccga attacagaag tacctggaat gtaaagccct gaaaccccct 37620
   cattttttaa gcagattgtt gccgtaaatc ctgcactatg cccaagatag agagctcctt 37680
   tggtgaatcc atctctatgt ttcagtttaa ccaagaaaca gtcagctggt ctaaaatttc 37740
   catctctatc taatacagca tctaacttga tgtcaggaac tatgaccggt ttaatgttat 37800
   atgtaacatt gagtaaatcc ttaagttcat aatcatcact gtcatcagtt atgtacgatc 37860
   caaacaatgt ttctaccggc atagtggata cgaagatgct atccatcaga atgtttccct 37920
   gattagtatt ttctatatag ctattcttct ttaaacgatt ttccaaatca gtaactatgt 37980
   tcattttttt aggagtagga cgcctagcca gtatggaaga ggattttcta gatcctctct 38040
   tcaacatctt tgatctcgat ggaatgcaaa accccatagt gaaacaacca acgataaaaa 38100
   taatattgtt tttcactttt tataatttta ccatctgact catggattca ttaatatctt 38160
   tataagagct actaacgtat aattctttat aactgaactg agatatatac accggatcta 38220
   tggtttccat aattgagtaa atgaatgctc ggcaataact aatggcaaat gtatagaaca 38280
   acgaaattat actagagttg ttaaagttaa tattttctat gagctgttcc aataaattat 38340
   ttgttgtgac tgcgttcaag tcataaatca tcttgatact atccagtaaa ccgtttttaa 38400
   gttctggaat attatcatcc cattgtaaag cccctaattc gactatcgaa tatcctgctc 38460
   tgatagcagt ttcaatatcg acggacgtca atactgtaat aaaggtggta gtattgtcat 38520
   catcgtgata aactacggga atatggtcgt tagtaggtac ggtaacttta cacaacgcga 38580
   tatataactt tccttttgta ccatttttaa cgtagttggg acgtcctgca gggtattgtt 38640
   ttgaagaaat gatatcgaga acagatttga tacgatattt gttggattcc tgattattca 38700
   ctataatata atctagacag atagatgatt cgataaatag agaaggtata tcgttggtag 38760
   gataatacat ccccattcca gtattctcgg atactctatt gatgacacta gttaagaaca 38820
   tgtcttctat tctagaaaac gaaaacatcc tacatggact cattaaaact tctaacgctc 38880
   ctgattgtgt ctcgaatgcc tcgtacaagg atttcaagga tgccatagat tctttgacca 38940
   acgatttaga attgcgttta gcatctgatt tttttattaa atcgaatggt cggctctctg 39000
   gtttgctacc ccaatgataa caatagtctt gtaaagataa accgcaagaa aatttatacg 39060
   catccatcca aataacccta gcaccatcgg atgatattaa tgtattatta tagattttcc 39120
   atccacaatt attgggccag tatactgtta gcaacggtat atcgaataga ttactcatgt 39180
   aacctactag aatgatagtt cgtgtactag tcataatatc tttaatccaa tctaaaaaat 39240
   ttaaaattag attttttaca ctgttaaagt taacaaaagt attacccggg tacgtggata 39300
   tcatatatgg cattggtcca ttatcagtaa tagctccata aactgatacg gcgatggttt 39360
   ttatatgtgt ttgatctaac gaggaagaaa ttcgcgccca caattcatct ctagatatgt 39420
   atttaatatc aaacggtaac acatcaattt cgggacgcgt atatgtttct aaatttttaa 39480
   tccaaatata atgatgacct atatgcccta ttatcatact gtcaactata gtacacctag 39540
   ggaacttacg atacatctgt ttcctataat cgttaaattt tacaaatcta taacatgcta 39600
   aaccttttga cgacagccat tcattaattt ctgatatgga atctgtattc tcgataccgt 39660
   atcgttctaa agccagtgct atatctccct gttcgtggga acgctttcgt ataatatcga 39720
   tcaacggata atctgaagtt tttggagaat aatatgactc atgatctatt tcgtccataa 39780
   acaatctaga cataggaatt ggaggcgatg atcttaattt tgtgcaatga gtcgtcaatc 39840
   ctataacttc taatcttgta atattcatca tcgacataat actatctatg ttatcatcgt 39900
   atattagtat accatgacct tcttcatttc gtgccaaaat gatatacagt cttaaatagt 39960
   tacgcaatat ctcaatagtt tcataattgt tagctgtttt catcaaggtt tgtatcctgt 40020
   ttaacatgat ggcgttctat aacgtctcta ttttctattt ttaatttttt aaatttttaa 40080
   cgatttactg tggctagata cccaatctct ctcaaatatt tttttagcct cgcttacaag 40140
   ctgtttatct atactattaa aactgacgaa tccgtgattt tggtaatggg ttccgtcgaa 40200
   atttgccgaa gtgatatgaa catattcgtc gtcgactatc aacaattttg tattattctg 40260
   aatagtgaaa accttcacag atagatcatt ttgaacacac aacgcatcta gacttttggc 40320
   ggttgccata gaatatacgt cgttcttatc ccaattacca actagaagtc tgatcttaac 40380
   tcctctatta atggctgctt ctataatgga gttgtaaatg tcgggccaat agtagctatt 40440
   accgtcgaca cgtgtagtgg gaactatggc caaatgttca atatctatac tagtcttagc 40500
   tgacctgagt ttatcaataa ctacatcggt atctagatct ctagaatatc ccaataggtg 40560
   ttccggagaa tcagtaaaga acactccacc tataggattc ttaatatgat acgcagtgct 40620
   aactggcaaa caacaagccg cagagcataa attcaaccat gaattttttg cgctattaaa 40680
   ggctttaaaa gtatcaaatc ttctacgaag atctgtggcc agcgggggat aatcagaata 40740
   tacacctaac gttttaatcg tatgtataga tcctccagta aatgacgcgt ttcctacata 40800
   acatctttca tcatctgaca cccaaaaaca accgagtagt agtcccacat tatttttttt 40860
   atctatatta acggttataa aatttatatc cgggcagtga ctttgtagct ctcccagatt 40920
   tcttttccct cgttcatcta gcaaaactat tattttaatc cctttttcag atgcctcttt 40980
   tagtttatca aaaataagcg ctcccctagt cgtactcaga ggattacaac aaaaagatgc 41040
   tatgtatata tatttcttag ctagagtgat aatttcgtta aaacattcaa atgttgttaa 41100
   atgatcggat ctaaaatcca tattttctgg tagtgtttct accagcctac attttgctcc 41160
   cgcaggtacc gatgcaaatg gccacattta gttaacataa aaacttatac atcctgttct 41220
   atcaacgatt ctagaatatc atcggctata tcgctaaaat tttcatcaaa gtcgacatca 41280
   caacctaact cagtcaatat attaagaagt tccatgatgt catcttcgtc tatttctata 41340
   tccgtatcca ttgtagattg ttgaccgatt atcgagttta aatcattact aatactcaat 41400
   ccttcagaat acaatctgtg tttcattgta aatttatagg cggtgtattt aagttggtag 41460
   attttcaatt atgtattaat atagcaacag tagttcttgc tcctccttga ttctagcatc 41520
   ctcttcatta ttttcttcta cgtacataaa catgtccaat acgttagaca acacaccgac 41580
   gatggcggcc gcagatctcg agttatgatc tacttcctta ccgtgcaata aattagaata 41640
   tattttctac ttttacgaga aattaattat tgtatttatt atttatgggt gaaaaactta 41700
   ctataaaaag cgggtgggtt tggaattagt gaaagcttaa aaattgaaat tttatttttt 41760
   ttttttggaa tataaataag ctcgaagtcg acagatctag gcctggtacc aggaggagga 41820
   attcagctta aagatgactt cgaaagttta tgatccagaa caaaggaaac ggatgataac 41880
   tggtccgcag tggtgggcca gatgtaaaca aatgaatgtt cttgattcat ttattaatta 41940
   ttatgattca gaaaaacatg cagaaaatgc tgttattttt ttacatggta acgcggcctc 42000
   ttcttattta tggcgacatg ttgtgccaca tattgagcca gtagcgcggt gtattatacc 42060
   agatcttatt ggtatgggca aatcaggcaa atctggtaat ggttcttata ggttacttga 42120
   tcattacaaa tatcttactg catggtttga acttcttaat ttaccaaaga agatcatttt 42180
   tgtcggccat gattggggtg cttgtttggc atttcattat agctatgagc atcaagataa 42240
   gatcaaagca atagttcacg ctgaaagtgt agtagatgtg attgaatcat gggatgaatg 42300
   gcctgatatt gaagaagata ttgcgttgat caaatctgaa gaaggagaaa aaatggtttt 42360
   ggagaataac ttcttcgtgg aaaccatgtt gccatcaaaa atcatgagaa agttagaacc 42420
   agaagaattt gcagcatatc ttgaaccatt caaagagaaa ggtgaagttc gtcgtccaac 42480
   attatcatgg cctcgtgaaa tcccgttagt aaaaggtggt aaacctgacg ttgtacaaat 42540
   tgttaggaat tataatgctt atctacgtgc aagtgatgat ttaccaaaaa tgtttattga 42600
   atcggatcca ggattctttt ccaatgctat tgttgaaggc gccaagaagt ttcctaatac 42660
   tgaatttgtc aaagtaaaag gtcttcattt ttcgcaagaa gatgcacctg atgaaatggg 42720
   aaaatatatc aaatcgttcg ttgagcgagt tctcaaaaat gaacaagcgg ccgccgccac 42780
   catgagcaag ggcgaggaac tgttcactgg cgtggtccca attctcgtgg aactggatgg 42840
   cgatgtgaat gggcacaaat tttctgtcag cggagagggt gaaggtgatg ccacatacgg 42900
   aaagctcacc ctgaaattca tctgcaccac tggaaagctc cctgtgccat ggccaacact 42960
   ggtcactacc ttcacctatg gcgtgcagtg cttttccaga tacccagacc atatgaagca 43020
   gcatgacttt ttcaagagcg ccatgcccga gggctatgtg caggagagaa ccatcttttt 43080
   caaagatgac gggaactaca agacccgcgc tgaagtcaag ttcgaaggtg acaccctggt 43140
   gaatagaatc gagctgaagg gcattgactt taaggaggat ggaaacattc tcggccacaa 43200
   gctggaatac aactataact cccacaatgt gtacatcatg gccgacaagc aaaagaatgg 43260
   catcaaggtc aacttcaaga tcagacacaa cattgaggat ggatccgtgc agctggccga 43320
   ccattatcaa cagaacactc caatcggcga cggccctgtg ctcctcccag acaaccatta 43380
   cctgtccacc cagtctgccc tgtctaaaga tcccaacgaa aagagagacc acatggtcct 43440
   gctggagttt gtgaccgctg ctgggatcac acatggcatg gacgagctgt acaagtgagc 43500
   ggccgccctg ctagcaagct tgctagcggc cgctcgagat ctgcggccgc tacagacacg 43560
   aatatgacta aaccgatgac catttaaaaa cccctctcta gctttcactt aaactgtatc 43620
   gatcattctt ttagcacatg tataatataa aaacattatt ctatttcgaa tttaggcttc 43680
   caaaaatttt tcatccgtaa accgataata atatatatag acttgttaat agtcggaata 43740
   aatagattaa tgcttaaact atcatcatct ccacgattag agatacaata tttacattct 43800
   ttttgctgtt tcgaaacttt atcaatacac gttaatacaa acccaggaag gagatattga 43860
   aactgaggct gttgaaaatg aaacggtgaa tacaataatt cagataatgt aaaatcatga 43920
   ttccgtattc tgatgatatt agaactgcta atggatgtcg atggtatgta tctaggagta 43980
   tctattttaa caaagcatcg atttgctaat atacaattat ccttttgatt aattgttatt 44040
   ttattcatat tcttaaaagg tttcatattt atcaattctt ctacattaaa aatttccatt 44100
   tttaatttat gtagccccgc aatactcctc attacgtttc attttttgtc tataatatcc 44160
   attttgttca tctcggtaca tagattatcc aattgagaag cgcatttagt agttttgtac 44220
   attttaagtt tattgacgaa tcgtcgaaaa ctagttatag ttaacatttt attatttgat 44280
   accctgatat taatacccct gccgttacta ttatttataa ctgatgtaat ccacgtaaca 44340
   ttggaattaa ctatcgatag taatgcatcg acgcttccaa aattgtctat tataaactca 44400
   ccgataattt ttttattgca tgttttcata ttcattagga ttatcaaatc tttaatctta 44460
   ttacgattgt atgcgttgat attacaagac gtcattctaa aagacggagg atctccatca 44520
   aatgccagac aatcacgtac aaagtacatg gaaataggtt ttgttctatt gcgcatcata 44580
   gatttatata gaacacccgt agaaatacta atttgtttta ctctataaaa tactaatgca 44640
   tctatttcat cgttttgtat aacgtctttc caagtgtcaa attccaaatt tttttcattg 44700
   atagtaccaa attcttctat ctctttaact acttgcatag ataggtaatt acagtgatgc 44760
   ctacatgccg ttttttgaaa ctgaatagat gcgtctagaa gcgatgctac gctagtcaca 44820
   atcaccactt tcatatttag aatatatata tgtaaaaata tagtagaatt tcattttgtt 44880
   tttttctatg ctataaatga attctcattt tgcatctgct catactccgt tttatattaa 44940
   taccaaagaa ggaagatatc tggttctaaa agccgttaaa gtatgcgatg ttagaactgt 45000
   agaatgcgaa ggaagtaaag cttcctgcgt actcaaagta gataaaccct catcgcccgc 45060
   gtgtgagaga agaccttcgt ccccgtccag atgcgagaga atgaataacc ctggaaaaca 45120
   agttccgttt atgaggacgg acatgctaca aaatatgttc gcggctaatc gcgataatgt 45180
   agcttctaga cttttgtcct aaaatactat tatatccttt tcgatattaa taaatccgtg 45240
   tcgtccaggt tttttatctc tttcagtatg tgaatagata ggtattttat ctctattcat 45300
   catcgaattt aagagatccg ataaacattg tttgtattct ccagatgtca gcatctgata 45360
   caacaatata tgtgcacata aacctctggc acttatttca tgtaccttcc ccttatcact 45420
   aaggagaata gtatttgaga aatatgtata catgatatta tcatgaatta gatatacaga 45480
   atttgtaaca ctctcgaaat cacacgatgt gtcggcgtta agatctaata tatcactcga 45540
   taacacattt tcatctagat acactagaca ttttttaaag ctaaaatagt ctttagtagt 45600
   aacagtaact atgcgattat tttcatcgat gatacatttc atcggcatat tattacgctt 45660
   accatcaaag actataccat gtgtatatct aacgtattct agcatggttg ccatacgcgc 45720
   attaaacttt tcaggatctt tggatagatc ttccaatcta tctatttgag aaaacatttt 45780
   tatcatgttc aatagttgaa acgtcggatc cactatatag atattatcta taaagatttt 45840
   aggaactacg ttcatggtat cctggcgaat attaaaacta tcaatgatat gattatcgtt 45900
   ttcatctttt atcaccatat agtttctaag atatgggatt ttacttaata taatattatt 45960
   tcccgtgata aattttatta gaaaggccaa atctataaga aaagtcctag aattagtctg 46020
   aagaatatct atatcgccgt atagtatatt tggattaatt agatatagag aatatgatcc 46080
   gtaacatata caacttttat tatggcgtct aagatattct tccatcaact tattaacatt 46140
   tttgactagg gaagatacat tatgacgtcc cattactttt gccttgtcta ttactgcgac 46200
   gttcatagaa tttagcatat ctcttgccaa ttcttccatt gatgttacat tataagaaat 46260
   tttagatgaa attacatttg gagctttaat agtaagaact cctaatatgt ccgtgtatgt 46320
   ggtcactaat acagattgta gttctataat cgtaaataat ttacctatat tatatgtttg 46380
   agtctgttta gaaaagtagc taagtatacg atcttttatt tctgatgcag atgtattaac 46440
   atcggaaaaa aatctttttt tattcttttt tactaaagat acaaatatgt ctttgttaaa 46500
   aacagttatt ttctgaatat ttctagcttg taattttaac atatgatatt cgttcacact 46560
   aggtactctg cctaaatagg tttctataat ctttaatgta atattaggaa aagtattctg 46620
   atcaggattc ctattcattt tgaggattta aaactctgat tattgtctaa tatggtctct 46680
   acgcaaactt tttcacagag cgatagagtt tttgataact cgtttttctt aagaaatata 46740
   aaactactgt ctccagagct cgctctatct tttattttat ctaattcgat acaaactcct 46800
   gatactggtt cagaaagtaa ttcattaatt ttcagtcctt tatagaagat atttaatata 46860
   gataatacaa aatcttcagt ttttgatatc gatctgattg atcctagaac tagatatatt 46920
   aataacgtgc tcattaggca gtttatggca gcttgataat tagatatagt atattccagt 46980
   tcatatttat tagataccgc attgcccaga ttttgatatt ctatgaattc ctctgaaaat 47040
   aaatccaaaa taactagaca ttctattttt tgtggattag tgtactctct tccctctatc 47100
   atgttcacta ctggtgtcca cgatgataaa tatctagagg gaatataata tagtccatag 47160
   gatgccaatc tagcaatgtc gaataactgt aattttattc ttcgctcttc attatgaatt 47220
   gattcttgag gtataaacct aacacaaatt atattattag acttttcgta tgtaatgtct 47280
   ttcatgttat aagtttttaa tcctggaata gaatctattt taatgaggct tttaaacgca 47340
   gagttctcca acgagtcaaa gcataatact ctgttgtttt tcttatatac gatgttacga 47400
   ttttcttctt tgaatggaat aggtttttga attagtttat aattacaaca taatagataa 47460
   ggaagtgtgc aaatagtacg cggaaaaaac ataatagctc ccctgttttc atccatggtt 47520
   ttaagtaaat gatcactggc ttctttagtc aatggatatt cgaacattaa ccgtttcatc 47580
   atcattggac agaatccata tttcttaatg taaagagtga tcaaatcatt gtgtttattg 47640
   taccatcttg ttgtaaatgt gtattcggtt atcggatctg ctcctttttc tattaaagta 47700
   tcgatgtcaa tctcgtctaa gaattcaact atatcgacat atttcatttg tatacacata 47760
   accattacta acgtagaatg tataggaaga gatgtaacgg gaacagggtt tgttgattcg 47820
   caaactattc taatacataa ttcttctgtt aatacgtctt gcacgtaatc tattatagat 47880
   gccaagatat ctatataatt attttgtaag atgatgttaa ctatgtgatc tatataagta 47940
   gtgtaataat tcatgtattt tgatatatgt tccaactctg tctttgtgat gtctagtttc 48000
   gtaatatcta tagcatcctc aaaaaatata ttcgcatata ttcccaagtc ttcagttcta 48060
   tcttctaaaa aatcttcaac gtatggaata taataatcta ttttacctct tctgatatca 48120
   ttaatgatat agtttttgac actatcttct gtcaattgat tcttattcac tatatctaag 48180
   aaacggatag cgtccctagg acgaactact gccattaata tctctattat agcttctgga 48240
   cataattcat ctattatacc agaattaatg ggaactattc cgtatctatc taacatagtt 48300
   ttaagaaagt cagaatctaa gacttgatgt tcatatattg gttcatacat gaaatgatct 48360
   ctattgatga tagtgactat ttcattctct gaaaattggt aactcattct atatatgctt 48420
   tccttgttga tgaaggatag aatatactca atagaatttg taccaacaaa ctgttctctt 48480
   atgaatcgta tatcatcatc tgaaataatc atgtaaggca tacatttaac aattagagac 48540
   ttgtctcctg ttatcaatat actattcttg tgataattta tgtgtgaggc aaatttgtcc 48600
   acgttcttta attttgttat agtagatatc aaatccaatg gagctacagt tcttggctta 48660
   aacagatata gtttttctgg aacaaattct acaacattat tataaaggac tttgggtaga 48720
   taagtgggat gaaatcctat tttaattaat gcgatagcct tgtcctcgtg cagatatcca 48780
   aacgcttttg tgatagtatg gcattcattg tctagaaacg ctctacgaat atctgtgaca 48840
   gatatcatct ttagagaata tactagtcgc gttaatagta ctacaatttg tattttttaa 48900
   tctatctcaa taaaaaaatt aatatgtatg attcaatgta taactaaact actaactgtt 48960
   attgataact agaatcagaa tctaatgatg acgtaaccaa gaagtttatc tactgccaat 49020
   ttagctgcat tatttttagc atctcgttta gattttccat cggccttatc gaatactctt 49080
   ccgtcgatgt ctacacaggc ataaaatgta ggagagttac taggcccaac tgattcaata 49140
   cgaaaagacc aatctctcct agtaatttgg cagtactcat taataacggt gacagggtta 49200
   gcatctttcc aatcaataat ttttttagcc ggaataacat catcaaaaga cttatgatcc 49260
   tctctcattg atttttcgcg ggatacatca tctattatga cgtcagccat agcatcagca 49320
   tccggcttat ccgcctccgt tgtcataaac caacgaggag gaatatcgtc ggagctgtac 49380
   accatagcac tacgttgaag atcgtacaga gctttattaa cttctcgctt ctccatatta 49440
   agttgtctag ttagttgtgc agcagtagct ccttcgattc caatgttttt aatagccgca 49500
   cacacaatct ctgcgtcaga acgctcgtca atatagatct tagacatttt tagagagaac 49560
   taacgcaacc agcaataaaa ctgaacctac tttatcattt ttttattcat catcctctgg 49620
   tggttcgtcg ttcctatcaa atgtagctct gattaacccg tcatctatag gtgatgctgg 49680
   ttctggagat tctggaggag atggattatt atctggaaga atctctgtta tttccttgtt 49740
   ttcatgtatc gattgcgttg taacattaag attgcgaaat gctctaaatt tgggaggctt 49800
   aaagtgttgt ttgcaatctc tacacgcgtg tctaactagt ggaggttcgt cagctgctct 49860
   agtttgaatc atcatcggcg tagtattcct acttttacag ttaggacacg gtgtattgta 49920
   tttctcgtcg agaacgttaa aataatcgtt gtaactcaca tcctttattt tatctatatt 49980
   gtattctact cctttcttaa tgcattttat accgaataag agatagcgaa ggaattcttt 50040
   ttcggtgccg ctagtaccct taatcatatc acatagtgtt ttatattcca aatttgtggc 50100
   aatagacggt ttatttctat acgatagttt gtttctggaa tcctttgagt attctatacc 50160
   aatattattc tttgattcga atttagtttc ttcgatatta gattttgtat tacctatatt 50220
   cttgatgtag tactttgatg atttttccat ggcccattct attaagtctt ccaagttggc 50280
   atcatccaca tattgtgata gtaattctcg gatatcagta gcggctaccg ccattgatgt 50340
   ttgttcattg gatgagtaac tactaatgta tacattttcc atttataaca cttatgtatt 50400
   aactttgttc atttatattt tttcattatt atgttgatat taacaaaagt gaatatatat 50460
   atgttaataa ttgtattgtg gttatacggc tacaatttta taatgagtga aagtcagtgt 50520
   ccgatgatca atgacgatag ctttactctg aaaagaaagt atcaaatcga tagtgcggag 50580
   tcaacaataa aaatggataa gaagaggata aagtttcaga atagagccaa aatggtaaaa 50640
   gaaataaatc agacaataag agcagcacaa actcattacg agacattgaa actaggatac 50700
   ataaaattta agagaatgat tatgactact actctagaag atatagcacc atctattcca 50760
   aataatcaga aaacttataa actattctcg gacatttcag ccatcggcaa agcatcacag 50820
   aatccgagta agatggtata tgctctgctg ctttacatgt ttcccaattt gtttggagat 50880
   gatcatagat tcattcgtta tagaatgcat ccaatgagta aaatcaaaca caagatcttc 50940
   tctcctttca aacttaatct tattagaata ttagtggaag aaagattcta taataatgaa 51000
   tgcagatcta ataaatggag aataattgga acacaagttg ataaaatgtt gatagctgaa 51060
   tctgataaat atacaataga tgcaaggtat aacctaaaac ccatgtatag aatcaaggga 51120
   gaatctgaag aagataccct ctttatcaaa cagatggtag aacaatgtgt gacatcccag 51180
   gaattggtgg aaaaagtgtt gaagatactg tttagagatt tgttcaagag tggagaatac 51240
   aaagcgtaca gatacgatga tgatgtagaa aatggattta ttggattgga tacactaaaa 51300
   ttaaacattg ttcatgatat agttgaacca tgtatgcctg ttcgtaggcc agtggctaag 51360
   atactgtgta aagaaatggt aaataaatac tttgagaatc cgctacatat tattggtaaa 51420
   aatcttcaag agtgcattga ctttgttagt gaataggcat ttcatctttc tccaatacta 51480
   attcaaattg ttaaattaat aatggatagt ataaatagtt attagttata agatagtaaa 51540
   aataattatt agaataagag tgtagtatca tagataactc tcttctataa aaatggattt 51600
   tattcgtaga aagtatctta tatacacagt agaaaataat atagattttt taaaggatga 51660
   tacattaagt aaagtaaaca attttaccct caatcatgta ctagctctca agtatctagt 51720
   tagcaatttt cctcaacacg ttattactaa ggatgtatta gctaatacca atttttttgt 51780
   tttcatacat atggtacgat gttgtaaagt gtacgaagcg gttttacgac acgcatttga 51840
   tgcacccacg ttgtacgtta aagcattgac taagaattat ttatcgttta gtaacgcaat 51900
   acaatcgtac aaggaaaccg tgcataaact aacacaagat gaaaaatttt tagaggttgc 51960
   cgaatacatg gacgaattag gagaacttat aggcgtaaat tatgacttag ttcttaatcc 52020
   attatttcac ggaggggaac ccatcaaaga tatggaaatc atttttttaa aactgtttaa 52080
   gaaaacagac ttcaaagttg ttaaaaaatt aagtgttata agattactta tttgggcata 52140
   cctaagcaag aaagatacag gcatagagtt tgcggataat gatagacaaa atatatacac 52200
   tctatttcaa caaactggta gaatcgtcca tagcaatcta acagaaacgt ttagagatta 52260
   tatctttccc ggagataaga ctagctattg ggtgtggtta aacgaaagta tagctaatga 52320
   tgcggatatc gttcttaata gacacgccat taccatgtat gataaaattc ttagttatat 52380
   atactctgag ataaaacaag gacgcgttaa taaaaacatg cttaagttag tttatatctt 52440
   tgagcctgaa aaagatatca gagaacttct gctagaaatc atatatgata ttcctggaga 52500
   tatcctatct attattgatg caaaaaacga cgattggaaa aaatatttta ttagttttta 52560
   taaagctaat tttattaacg gtaatacatt tattagtgat agaacgttta acgaggactt 52620
   attcagagtt gttgttcaaa tagatcccga atatttcgat aatgaacgaa ttatgtcttt 52680
   attctctacg agtgctgcgg acattaaacg atttgatgag ttagatatta ataacagtta 52740
   tatatctaat ataatttatg aggtgaacga tatcacatta gatacaatgg atgatatgaa 52800
   gaagtgtcaa atctttaacg aggatacgtc gtattatgtt aaggaataca atacatacct 52860
   gtttttgcac gagtcggatc ccatggtcat agagaacgga atactaaaga aactgtcatc 52920
   tataaaatcc aagagtagac ggctgaactt gtttagcaaa aacattttaa aatattattt 52980
   agacggacaa ttggctcgtc taggtcttgt gttagatgat tataaaggag acttgttagt 53040
   taaaatgata aaccatctta agtctgtgga ggatgtatcc gcattcgttc gattttctac 53100
   agataaaaac cctagtattc ttccatcgct aatcaaaact attttagcta gttataatat 53160
   ttccatcatc gtcttatttc aaaggttttt gagagataat ctatatcatg tagaagaatt 53220
   cttggataaa agcatccatc taaccaagac ggataagaaa tatatacttc aattgataag 53280
   acacggtaga tcatagaaca gaccaaatat attattaata atttgtatat acatagatat 53340
   aattatcaca catttttgat aaatgggaac tgctgcaaca attcagactc ccaccaaatt 53400
   aatgaataaa gaaaatgcag aaatgatttt ggaaaaaatt gttgatcata tagttatgta 53460
   tattagtgac gaatcaagtg attcagaaaa taatcctgaa tatattgatt ttcgtaacag 53520
   atacgaagac tatagatctc tcattataaa aagtgatcac gagtttgtaa agctatgtaa 53580
   aaatcatgcg gagaaaagtt ctccagaaac gcaacaaatg attatcaaac acatatacga 53640
   acaatatctt attccagtat ctgaagtact attaaaacct ataatgtcca tgggtgacat 53700
   aattacatat aacggatgta aagacaatga atggatgcta gaacaactct ctaccctaaa 53760
   ctttaacaat ctccgcacat ggaactcatg tagcataggc aatgtaacgc gtctgtttta 53820
   tacatttttt agttatctga tgaaagataa actaaatata taagtataat cccattctaa 53880
   tactttaacc tgatgtatta gcatcttatt agaatattaa cctaactaaa agacataaca 53940
   taaaaactca ttacatagtt gataaaaagc ggtaggatat aaatattatg gctgccaccg 54000
   ttccgcgttt tgacgacgtg tacaaaaatg cacaaagaag aattctagat caagaaacat 54060
   tttttagtag aggtctaagt agaccgttaa tgaaaaacac atatctattt gataattacg 54120
   cgtatggatg gataccagaa actgcaattt ggagtagtag atacgcaaac ctagatgcta 54180
   gtgactatta tcccatttcg ttgggattac ttaaaaagtt cgagtttctc atgtctctat 54240
   ataaaggtcc tattcccgta tatgaagaaa aagtaaatac tgaattcata gccaatggat 54300
   cgttctctgg tagatacgta tcatatcttc gaaagttttc tgcccttcca acaaacgagt 54360
   ttattagttt tttgttactg acttccattc caatctataa tatcttgttc tggtttaaaa 54420
   acacacagtt tgatattact aaacacacat tattcagata cgtctataca gataatgcca 54480
   aacacctggc gttggctagg tatatgcatc aaacaggaga ctataagcct ttgtttagtc 54540
   gtctcaaaga gaattatata tttaccggtc ccgttccaat aagtatcaaa gatatagatc 54600
   accctaatct tagtagagca agaagtccat ccgattatga gacattagct aatattagta 54660
   ctatattgta ctttaccaag tatgatccgg tattaatgtt tttattgttt tacgtacctg 54720
   ggtattcaat tactacaaaa attactccag ccgtagaata tctaatggat aaactgaatc 54780
   taacaaagag cgacgtacaa ctgttgtaaa ttattttatg cttcgtaaaa tgtaggtttt 54840
   gaaccaaaca ttctttcaaa gaatgagatg cataaaactt tattatccaa tagattgact 54900
   atttcggacg tcaatcgttt aaagtaaact tcgtaaaata ttctttgatc actgccgagt 54960
   ttaaaacttc tatcgataat tgtctcatat gttttaatat ttacaagttt tttggtccat 55020
   ggtacattag ccggacaaat atatgcaaaa taatatcgtt ctccaagttc tatagtttct 55080
   ggattatttt tattatattc agtaactaaa tacatattag ggttatctgc ggatttataa 55140
   tttgagtgat gcattctact caacataaat aattctagag gagacgatct actatcaaat 55200
   tcggatcgta aatctgtttc taaagaacgg agaatatcta tacatacctg attagaattc 55260
   atccgtcctt cagacaacat ctcagacagt ctggttttgt acatcttaat catattctta 55320
   tgaaacttgg aaacatctct tctagtttca ctagtacctt tattaattct ctcaggtaca 55380
   gattttgaat tcgacgatgc tgagtatttc atcgttgtat atttcttctt cgattgcata 55440
   atcagattct tatataccgc ctcaaactct attttaaaat tattaaacaa tactctatta 55500
   ttaatcagtc gttctaactc tttcgctatt tctatagact tatctacatc ttgactgtct 55560
   atctctgtaa acacggagtc ggtatctcca tacacgctac gaaaacgaaa tctgtaatct 55620
   ataggcaacg atgttttcac aatcggatta atatctctat cgtccatata aaatggatta 55680
   cttaatggat tggcaaaccg taacataccg ttagataact ctgctccatt tagtaccgat 55740
   tctagataca agatcattct acgtcctatg gatgtgcaac tcttagccga agcgtatgag 55800
   tatagagcac tatttctaaa tcccatcaga ccatatactg agttggctac tatcttgtac 55860
   gtatattgca tggaatcata gatggccttt tcagttgaac tggtagcctg ttttagcatc 55920
   tttttatatc tggctctctc tgccaaaaat gttcttaata gtctaggaat ggttccttct 55980
   atcgatctat cgaaaattgc tatttcagag atgaggttcg gtagtctagg ttcacaatga 56040
   accgtaatat atctaggagg tggatatttc tgaagcaata gctgattatt tatttcttct 56100
   tccaatctat tggtactaac aacgacaccg actaatgttt ccggagatag atttccaaag 56160
   atacacacat taggatacag actgttataa tcaaagatta atacattatt actaaacatt 56220
   ttttgttttg gagcaaatac cttaccgcct tcataaggaa acttttgttt tgtttctgat 56280
   ctaactaaga tagttttagt ttccaacaat agctttaaca gtggaccctt gatgactgta 56340
   ctcgctctat attcgaatac catggattga ggaagcacat atgttgacgc acccgcgtct 56400
   gtttttgttt ctactccata atactcccac aaatactgac acaaacaagc atcatgaata 56460
   cagtatctag ccatatctaa agctatgttt agattataat ccttatacat ctgagctaaa 56520
   tcaacgtcat cctttccgaa agataattta tatgtatcat taggtaaagt aggacataat 56580
   agtacgactt taaatccatt ttcccaaata tctttacgaa ttactttaca tataatatcc 56640
   tcatcaacag tcacataatt acctgtggtt aaaacctttg caaatgcagc ggctttgcct 56700
   ttcgcgtctg tagtatcgtc accgatgaac gtcatttctc taactcctct atttaatact 56760
   ttacccatgc aactgaacgc gttcttggat atagaatcca atttgtacga atccaatttt 56820
   tcaaattttt gaatgaatga atatagatcg aaaaatatag ttccattatt gttattaacg 56880
   tgaaacgtag tattggccat gccgcctact cccttatgac tagactgatt tctctcataa 56940
   atacagagat gtacagcttc ctttttgtcc ggagatctaa agataatttt ctctcctgtt 57000
   aataactcta gacgattagt aatatatctc agatcaaagt tatgtccgtt aaaggtaacg 57060
   acgtagtcga acgttagttc caacaattgt ttagctattc gtaacaaaac tatttcagaa 57120
   catagaacta gttctcgttc gtaatccatt tccattagtg actgtatcct caaacatcct 57180
   ctatcgacgg cttcttgtat ttcctgttcc gttaacatct cttcattaat gagcgtaaac 57240
   aataatcgtt taccacttaa atcgatataa cagtaacttg tatgcgagat tgggttaata 57300
   aatacagaag gaaacttctt atcgaagtga cactctatat ctagaaataa gtacgatctt 57360
   gggatatcga atctaggtat ttttttagcg aaacagttac gtggatcgtc acaatgataa 57420
   catccattgt taatctttgt caaatattgc tcgtccaacg agtaacatcc gtctggagat 57480
   atcccgttag aaatataaaa ccaactaata ttgagaaatt catccatggt ggcattttgt 57540
   atgctgcgtt tctttggctc ttctatcaac cacatatctg cgacggagca ttttctatct 57600
   ttaatatcta gattataact tattgtctcg tcaatgtcta tagttctcat ctttcccaac 57660
   ggcctcgcat taaatggagg aggagacaat gactgatata tttcgtccgt cactacgtaa 57720
   taaaagtaat gaggaaatcg tataaatacg gtctcgccat ttcgacatct ggatttcaga 57780
   tataaaaatc tgttttcacc gtgactttca aaccaattaa tgcaccgaac atccatttat 57840
   agaatttaga aatatatttt catttaaatg aatcccaaac attggggaag agccgtatgg 57900
   accattattt ttatagtact ttcgcaagcg ggtttagacg gcaacataga agcgtgtaaa 57960
   cgaaaactat atactatagt tagcactctt ccatgtcctg catgtagacg gcacgcgact 58020
   atcgctatag aggacaataa tgtcatgtct agcgatgatc tgaattatat ttattatttt 58080
   ttcatcagat tatttaacaa tttggcatct gatcccaaat acgcgatcga tgtgacaaag 58140
   gttaaccctt tataaactta acccattata aaacttatga ttagtcacaa ctgaaataac 58200
   cgcgtgatta ttttttggta taattctaca cggcatggtt tctgtgacta tgaattcaac 58260
   ccccgttaca ttagtgaaat ctttaacaaa cagcaagggt tcgtcaaaga cataaaactc 58320
   attgtttaca atcgaaatag accccctatc acacttaaaa taaaaaatat ccttatcctt 58380
   taccaccaaa taaaattctg attggtcaat gtgaatgtat tcacttaaca gttccacaaa 58440
   tttatttatt aactccgagg cacatacatc gtcggtattt tttatggcaa actttactct 58500
   tccagcatcc gtttctaaaa aaatattaac gagttccatt tatatcatcc aatattattg 58560
   aaatgacgtt gatggacaga tgatacaaat aagaaggtac ggtacctttg tccaccatct 58620
   cctccaattc atgctctatt ttgtcattaa ctttaatgta tgaaaacagt acgccacatg 58680
   cttccatgac agtgtgtaac actttggata caaaatgttt gacattagta taattgtcca 58740
   agactgtcaa tctataatag atagtagcta taatatattc tatgatggta ttgaagaaga 58800
   tgacaacctt ggcatattga tcatttaaca cagacatggt atcaacagat agcttgaatg 58860
   aaagagaatc agtaattgga ataagcgtct tctcgataga gtgtccgtat accaacatgt 58920
   ctgatatttt gatgtattcc attaaattat ttagtttttt ctttttattc tcgttaaaca 58980
   gcatttctgt caacggaccc caacatcgtt gaccgattaa gttttgattg atttttccgt 59040
   gtaaggcgta tctagtcaga tcgtatagcc tatccaataa tccatcgtct gtgtgtagat 59100
   cacatcgtac actttttaat tctctataga agagcgacag acatctggag caattacaga 59160
   cagcaatttc tttattctct acagatgtaa gatacttgaa gacattccta tgatgatgca 59220
   gaattttgga taacacggta ttgatggtat ctgttaccat aattcctttg atggctgata 59280
   gtgtcagagc acaagatttc caatctttga caatttttag caccattatc tttgttttga 59340
   tatctatatc agacagcatg gtgcgtctga caacacaggg attaagacgg aaagatgaaa 59400
   tgattctctc aacatcttca atagatacct tgctattttt tctggcatta tctatatgtg 59460
   cgagaatatc ctctagagaa tcagtatcct ttttgatgat agtggatctc aatgacatgg 59520
   gacgtttaaa ccttcttatt ctatcaccag attgcatggt gatttgtctt ctttctttta 59580
   tcataatgta atctctaaat tcatcggcaa attgtctata tctaaaatca taatatgaga 59640
   tgtttacctc tacaaatatc tgttcgtcca atgttagagt atttacatca gttttgtatt 59700
   ccaaattaaa catggcaacg gatttaattt tatattcctc tattaagtcc tcgtcgataa 59760
   taacagaatg tagataatca tttaatccgt cgtacatggt tggaagatgc ttgttgacaa 59820
   aatctttaat tgtcttgatg aaggtgggac tatatctaac atcttgatta ataaaattta 59880
   taacattgtc cataggatac tttgtaacta gttttataca catctcttca tcggtaagtt 59940
   tagacagaat atcgtgaaca ggtggtatat tatattcatc agatatacga agaacaatgt 60000
   ccaaatctat attgtttaat atattatata gatgtagcgt agctcctaca ggaatatctt 60060
   taactaagtc aatgatttca tcaaccgtta gatctatttt aaagttaatc atataggcat 60120
   tgatttttaa aaggtatgta gccttgacta cattctcatt aattaaccat tccaagtcat 60180
   tgtgtgtaag aagattatat tctatcataa gcttgactac atttggtccc gataccatta 60240
   aagaattctt atgatataag gaaacagatt ttaggtactc atctactcta caagaatttt 60300
   ggagagcctt aacgatatca gtgacgttta ttatttcagg aggaaagaat ctaacattga 60360
   gaatatcgga attaatagct tccagataca gtgattttgg caatagtccg tgtaatccat 60420
   aatccagtaa cacgagctgg tgcttgctag acaccttttc aatgtttaat ttttttgaaa 60480
   taagctttga taaagccttc ctcgcaaatt ccggatacat gaacatgtcg gcgacatgat 60540
   taagtattgt tttttcatta tttttatatt ttctcaacaa gttctcaata ccccaataga 60600
   tgatagaata tcacccaatg cgtccatgtt gtctatttcc aacaggtcgc tatatccacc 60660
   aatagaagtt tttccaaaaa agattctagg aacagttcta ccaccagtaa tttgttcaaa 60720
   ataatcccgc aattcatttt cgggtttaaa ttctttaata tcgacaattt catacgctcc 60780
   tcttttgaaa ctaaacttat ttagaatatc cagtgcattt ctacaaaaag gacatgtata 60840
   cttgacaaaa attgtcactt tgttattggc caacctttgt tgtacaaatt cctcggccat 60900
   tttaatattt aagtgatata aaactatctc gacttattta actctttagt cgagatatat 60960
   ggacgcagat agctatatga tagccaacta cagaaggcaa acgctataaa aaacataatt 61020
   acgacgagca tatttataaa tatttttatt cagcattact tgatatagta atattaggca 61080
   cagtcaaaca ttcaaccact ctcgatacat taactctctc attttcttta acaaattctg 61140
   caatatcttc gtaaaaagat tcttgaaact ttttagaata tctatcgact ctagatgaaa 61200
   tagcgttcgt caacatacta tgttttgtat acataaaggc gcccatttta acagtttcta 61260
   gtgacaaaat gctagcgatc ctaggatcct ttagaatcac atagattgac gattcgtctc 61320
   tcttagtaac tctagtaaaa taatcataca atctagtacg cgaaataata ttatccttga 61380
   cttgaggaga tctaaacaat ctagttttga gaacatcgat aagttcatcg ggaatgacat 61440
   acatactatc tttaatagaa ctcttttcat ccagttgaat ggattcgtcc ttaaccaact 61500
   gattaatgag atcttctatt ttatcatttt ccagatgata tgtatgtcca ttaaagttaa 61560
   attgtgtagc gcttcttttt agtctagcag ccaatacttt aacatcacta atatcgatat 61620
   acaaaggaga tgatttatct atggcattaa gaattcgttt ttcgacatcc gtcaaaacca 61680
   attccttttt gcctgtatca tccagttttc catcctttgt aaagaaatta ttttctacta 61740
   gactattaat aagactgata aggattcctc cataattgca caatccaaac tttttaacaa 61800
   aactagactt tacgagatct acaggaatgc gtacttcagg ttttttagct tgtgattttt 61860
   tcttttgcgg acattttcta gtaaccaact catctaccat ttcattgatt ttagcagtga 61920
   aataagcttt caatgcacgg gcactgatac tattgaaaac gagttgatct tcaaattccg 61980
   ccatttaagt tcaccaaaca acttttaaat acaaatatat caatagtagt agaataagaa 62040
   ctataaaaaa aataataatt aaccaacccc aacaaccggt attattagtt gatgtggtag 62100
   ttttctcatc acttagaaca gatttaacaa tttctataaa gtctgtcaaa tcatcttccg 62160
   gagaccccat aaatacacca aatatagcgg cgtacaactt atccatttat acattgaata 62220
   ttggcttttc tttatcgcta tcttcatcat attcatcatc aatatcaaca agtcccagat 62280
   tacgagccag atcttcttct acattttcag tcattgatac acgttcacta tctccagaga 62340
   gtccgataac gttagccacc acttctctat caatgattag tttcttgagc gcgaaagtaa 62400
   tttttgtttc cgttccggat ctatagaaga cgataggtgt gataattgcc ttggccaatt 62460
   gtctttctct tttactgagt gattctagtt caccttctat agatctgaga atggatgatt 62520
   ctccagtcga aacatattct accatggatc cgtttaattt gttgatgaag atggattcat 62580
   ccttaaatgt tttctctgta atagtttcca ccgaaagact atgcaaagaa tttggaatgc 62640
   gttccttgtg cttaatgttt ccatagacgg cttctagaag ttgatacaac ataggactag 62700
   ccgcggtaac ttttattttt agaaagtatc catcgcttct atcttgttta gatttatttt 62760
   tataaagttt agtctctcct tccaacataa taaaagtgga agtcatttga ctagataaac 62820
   tatcagtaag ttttatagag atagacgaac aattagcgta ttgagaagca tttagtgtaa 62880
   cgtattcgat acattttgca ttagatttac taatcgattt tgcatactct ataacacccg 62940
   cacaagtctg tagagaatcg ctagatgcag taggtcttgg tgaagtttca actctcttct 63000
   tgattacctt actcatgatt aaacctaaat aattgtactt tgtaatataa tgatatatat 63060
   tttcacttta tctcatttga gaataaaaat gtttttgttt aaccactgca tgatgtacag 63120
   atttcggaat cgcaaaccac cagtggtttt attttatcct tgtccaatgt gaattgaatg 63180
   ggagcggatg cgggtttcgt acgtagatag tacattcccg tttttagacc gagactccat 63240
   ccgtaaaaat gcatactcgt tagtttggaa taactcggat ctgctatatg gatattcata 63300
   gattgacttt gatcgatgaa ggctcccctg tctgcagcca tttttatgat cgtcttttgt 63360
   ggaatttccc aaatagtttt ataaactcgc ttaatatctt ctggaaggtt tgtattctga 63420
   atggatccac catctgccat aatcctattc ttgatctcat cattccataa ttttctctcg 63480
   gttaaaactc taaggagatg cggattaact acttgaaatt ctccagacaa tactctccga 63540
   gtgtaaatat tactggtata cggttccacc gactcattat ttcccaaaat ttgagcagtt 63600
   gatgcagtcg gcataggtgc caccaataaa ctatttctaa gaccgtatgt tctgatttta 63660
   tcttttagag gttcccaatt ccaaagatcc gacggtacaa cattccaaag atcatattgt 63720
   agaataccgt tactggcgta cgatcctaca tatgtatcgt atggtccttc cttctcagct 63780
   agttcacaac tcgcctctaa tgcaccgtaa taaatggttt cgaagatctt cttatttaga 63840
   tcttgtgctt ccaggctatc aaatggataa tttaagagaa taaacgcgtc cgctaatcct 63900
   tgaacaccaa taccgatagg tctatgtctc ttattagaga tttcagcttc tggaatagga 63960
   taataattaa tatctataat tttattgaga tttctgacaa ttactttgac cacatccttc 64020
   agtttgagaa aatcaaatcg cccatctatt acaaacatgt tcaaggcaac agatgccaga 64080
   ttacaaacgg ctacctcatt agcatccgca tattgtatta tctcagtgca aagattacta 64140
   cacttgatag ttcctaaatt ttgttgatta ctctttttgt tacacgcatc cttataaaga 64200
   atgaatggag taccagtttc aatctgagat tctataatcg ctttccagac gactcgagcc 64260
   tttattatag atttgtatct cctttctctt tcgtatagtg tatacaatcg ttcgaactcg 64320
   tctccccaaa cattgtccaa tccaggacat tcatccggac acatcaacga ccactctccg 64380
   tcatccttca ctcgtttcat aaagagatca ggaatccaaa gagctataaa tagatctctg 64440
   gttctatgtt cctcgtttcc tgtattcttt ttaagatcga ggaacgccat aatatcagaa 64500
   tgccacggtt ccaagtatat ggccataact ccaggccgtt tgtttcctcc ctgatctatg 64560
   tatctagcgg tgttattata aactctcaac attggaataa taccgtttga tataccattg 64620
   gtaccggaga tatagcttcc actggcacga atattactaa ttgatagacc tattccccct 64680
   gccattttag agattaatgc gcatcgtttt aacgtgtcat agataccctc tatgctatca 64740
   tcgatcatgt taagtagaaa acagctagac atttggtgac gactagttcc cgcattaaat 64800
   aaggtaggag aagcgtgcgt aaaccatttt tcagaaagta gattgtacgt ctcaatagct 64860
   gagtctatat cccattgatg aattcctact gcgacacgca ttaacatgtg ctgaggtctt 64920
   tcaacgatct tgttgtttat tttcaacaag taggattttt ccaaagtttt aaaaccaaaa 64980
   tagttgtatg aaaagtctcg ttcgtaaata ataaccgagt tgagtttatc cttatatttg 65040
   ttaactatat ccatggtgat acttgaaata atcggagaat gtttcccatt tttaggatta 65100
   acatagttga ataaatcctc catcacttca ctaaatagtt tttttgtttc cttgtgtaga 65160
   tttgatacgg ctattctggc ggctagaatg gcataatccg gatgttgtgt agtacaagtg 65220
   gctgctattt cggctgccag agtgtccaat tctaccgttg ttactccatt atatattcct 65280
   tgaataacct tcatagctat tttaatagga tctatatgat ccgtgtttaa gccataacat 65340
   aattttctaa tacgagacgt gattttatca aacatgacat tttccttgta tccatttcgt 65400
   ttaatgacaa acatttttgt tggtgtaata aaaaaattat ttaacttttc attaataggg 65460
   atttgacgta tgtagcgtac aaaatgattg ttcctggtat atagataaag agtcctatat 65520
   atttgaaaat cgttacggct cgattaaact ttaatgattg catagtgaat atatcattag 65580
   gatttaactc cttgactatc atggcggcgc cagaaattac catcaaaagc attaatacag 65640
   ttataccgat cgcagttaga acggttatag catccaccat ttatatctaa aaattagatc 65700
   aaagaatatg tgacaaagtc ctagttgtat actgagaatt gacgaaacaa tgtttcttac 65760
   atattttttt cttattagta accgacttaa tagtaggaac tggaaaacta gacttgatta 65820
   ttctataagt atagataccc ttccaaataa tattctcttt gataaaagtt ccagaaaatg 65880
   tagaattttt taaaaagtta tcttttgcta ttaccaagat tgtgtttaga cgcttattat 65940
   taatatgagt gatgaaatcc acaccgcctc tagatatcgc ctttatttcc acattagatg 66000
   gtaaatccaa tagtgaaact atctttttag gaatgtatgg actcgcgttt agaggagtaa 66060
   acgtcttagg cgtcggaaag gatgattcat caaacgaata aacaatttca caaatggatg 66120
   ttaatgtatt agtaggaaat tttttgacgc tattggaatt gaagattcta atggatgatg 66180
   ttctacctat ttcatccgat aacatgttaa tttccgacac caacggtttt aatatttcga 66240
   tgatatacgg tagtctctct ttcggactta tatagcttat tccacaatac gagtcattat 66300
   atactccaaa aaacaaaata actagtataa aatctgtatc gaatgggaaa aacgaaatta 66360
   tcgacatagg tatagaatcc ggaacattga acgtattaat acttaattct ttttctgtgg 66420
   taagtaccga taggttattg acattgtatg gttttaaata ttctataact tgagacttga 66480
   tagatattag tgatgaattg aaaattattt ttatcaccac gtgtgtttca ggatcatcgt 66540
   cgacgcccgt caaccaaccg aacggagtaa aataaatatc attaatatat gctctagata 66600
   ttagtatttt tatcaatcct ttgattatca tcttctcgta ggcgaatgat tccatgatca 66660
   agagtgattt aagaacatcc tccggagtat taatgggctt agtaaacagt ccatcgttgc 66720
   aataataaaa gttatccaag ttaaaggata ttatgcattc gtttaaagat atcacctcat 66780
   ctgacggaga caattttttg gtaggtttta gagactttga agctacttgt ttaacaaagt 66840
   tattcatcgt cgtctactat tctatttaat tttgtagtta atttatcaca tatcacatta 66900
   attgactttt tggtccactt ttccatacgt ttatattctt ttaatcctgc gttatccgtt 66960
   tccgttatat ccagggatag atcttgcaag ttaaatagaa tgctcttaaa taatgtcatt 67020
   ttcttatccg ctaaaaattt aaagaatgta taaacctttt tcagagattt gaaactctta 67080
   ggtggtgtcc tagtacacaa tatcataaac aaactaataa acattccaca ttcagattcc 67140
   aacagctgat taacttccac attaatacag cctattttcg ctccaaatgt acattcgaaa 67200
   aatctgaata aaacatcgat gtcacaattt gtattatcca atacagaatg tttgtgattc 67260
   gtgttaaaac catcggagaa ggaatagaaa taaaaattat tatagtggtg gaattcagtt 67320
   ggaatattgc ctccggagtc ataaaaggat actaaacatt gttttttatc ataaattaca 67380
   catttccaat gagacaaata acaaaatcca aacattacaa atctagaggt agaactttta 67440
   attttgtctt taagtatata cgataagata tgtttattca taaacgcgtc aaatttttca 67500
   tgaatcgcta aggagtttaa gaatctcatg tcaaattgtc ctatataatc cacttcggat 67560
   ccataagcaa actgagagac taagttctta atacttcgat tgctcatcca ggctcctctc 67620
   tcaggctcta ttttcatctt gacgaccttt ggattttcac cagtatgtat tcctttacgt 67680
   gataaatcat cgattttcaa atccatttgt gagaagtcta tcgccttaga tactttttcc 67740
   cgtagtcgag gtttaaaaaa atacgctaac ggtatactag taggtaactc aaagacatca 67800
   tatatagaat ggtaacgcgt ctttaactcg tcggttaact ctttcttttg atcgagttcg 67860
   tcgctactat tgggtctgct caggtgcccc gactctacta gttccaacat cataccgata 67920
   ggaatacaag acactttgcc ggcggttgta gatttatcat atttttccac tacatatccg 67980
   ttacaatttg ttaaaaattt agatacatct atattgctac ataatccagc tagtgaatat 68040
   atatgacata ataaattggt aaatcctagt tctggtattt tactaattac taaatctgta 68100
   tatctttcca tttatcatgg aaaagaattt accagatatc ttcttttttc caaactgcgt 68160
   taatgtattc tcttacaaat attcacaaga tgaattcagt aatatgagta aaacggaacg 68220
   tgatagtttc tcattggccg tgtttccagt tataaaacat agatggcata acgcacacgt 68280
   tgtaaaacat aaaggaatat acaaagttag tacagaagca cgtggaaaaa aagtatctcc 68340
   tccatcacta ggaaaacccg cacacataaa cctaaccgcg aagcaatata tatacagtga 68400
   acacacaata agctttgaat gttatagttt tctaaaatgt ataacaaata cagaaatcaa 68460
   ttcgttcgat gagtatatat taagaggact attagaagct ggtaatagtt tacagatatt 68520
   ttccaattcc gtaggtaaac gaacagatac tataggtgta ctagggaata agtatccatt 68580
   tagcaaaatt ccattggcct cattaactcc taaagcacaa cgagagatat tttcagcgtg 68640
   gatttctcat agacctgtag ttttaactgg aggaactgga gtgggtaaga cgtcacaggt 68700
   acccaagtta ttgctttggt ttaattattt atttggtgga ttctctactc tagataaaat 68760
   cactgacttt cacgaaagac cagtcattct atctcttcct aggatagctt tagttagatt 68820
   gcatagcaat accattttaa aatcattggg atttaaggta ctagatggat ctcctatttc 68880
   tttacggtac ggatctatac cggaagaatt aataaacaaa caaccaaaaa aatatggaat 68940
   tgtattttct acccataagt tatctctaac aaaactattt agttatggca ctcttattat 69000
   agacgaagtt catgagcatg atcaaatagg agatattatt atagcagtag cgagaaagca 69060
   tcatacgaaa atagattcta tgtttttaat gactgccacg ttagaggatg acagggaacg 69120
   gctaaaagta tttttaccta atcccgcatt tatacatatt cctggagata cactgtttaa 69180
   aattagcgag gtatttattc ataataagat aaatccatct tccagaatgg catacataga 69240
   agaagaaaag agaaatttag ttactgctat acagatgtat actcctcctg atggatcatc 69300
   cggtatagtc tttgtggcat ccgttgcaca gtgtcacgaa tataaatcat atttagaaaa 69360
   aagattaccg tatgatatgt atattattca tggtaaggtc ttagatatag acgaaatatt 69420
   agaaaaagtg tattcatcac ctaatgtatc gataattatt tctactcctt atttggaatc 69480
   cagcgttact atacgcaatg ttacacacat ttatgatatg ggtagagttt ttgtccccgc 69540
   tccttttgga ggatcgcaac aatttatttc taaatctatg agagatcaac gaaaaggaag 69600
   agtaggaaga gttaatcctg gtacatacgt ctatttctat gatctgtctt atatgaagtc 69660
   tatacagcga atagattcag aatttctaca taattatata ttgtacgcta ataagtttaa 69720
   tctaacactc cccgaagatt tgtttataat ccctacaaat ttggatattc tatggcgtac 69780
   aaaggaatat atagactcgt tcgatattag tacagaaaca tggaataaat tattatccaa 69840
   ttattatatg aagatgatag agtatgctaa actttatgta ctaagtccta ttctcgctga 69900
   ggagttggat aactttgaga ggacgggaga attaactagt attgtacgag aagccatttt 69960
   atctctaaat ttacaaatta agattttaaa ttttaaacat aaagatgatg atacgtatat 70020
   acacttttgt aaaatattat tcggtgtcta taacggaaca aacgctacta tatattatca 70080
   tagacctcta acgggatata tgaatatgat ttcagatact atatttgttc ctgtagataa 70140
   taactaaaaa tcaaactcta atgaccacat ctttttttag agatgaaaaa ttttccacat 70200
   ctccttttgt agacacgact aaacattttg cagaaaaaag tttattagtg tttagataat 70260
   cgtatacttc atcagtgtag atagtaaatg tgaacagata aaaggtattc ttgctcaata 70320
   gattggtaaa ttccatagaa tatattaatc ctttcttctt gagatcccac atcatttcaa 70380
   ccagagacgt tttatccaat gatttacctc gtactatacc acatacaaaa ctagattttg 70440
   cagtgacgtc gtacctggta ttcctaccaa acaaaatttt acttttagtt cttttagaaa 70500
   attctaaggt agaatctcta tttgccaata tgtcatctat ggaattacca ctagcaaaaa 70560
   atgatagaaa tatatattga tacatcgcag ctggttttga tctactatac tttaaaaacg 70620
   aatcagattc cataattgcc tgtatatcat cagctgaaaa actatgtttt acacgtattc 70680
   cttcggcatt tctttttaat gatatatctt gtttagacaa tgataaagtt atcatgtcca 70740
   tgagagacgc gtctccgtat cgtataaata tttcattaga tgttagacgc ttcattaggg 70800
   gtatacttct ataaggtttc ttaatcagtc catcattggt tgcgtcaaga actactatcg 70860
   gatgttgttg ggtatctcta gtgttacaca tggccttact aaagtttggg taaataacta 70920
   tgatatctct attaattata gatgcatata tttcattcgt caaggatatt agtatcgact 70980
   tgctatcgtc attaatacgt gtaatgtaat catataaatc atgcgatagc caaggaaaat 71040
   ttaaatagat gttcatcata taatcgtcgc tataattcat attaatacgt tgacattgac 71100
   taatttgtaa tatagcctcg ccacgaagaa agctctcgta ttcagtttca tcgataaagg 71160
   ataccgttaa atataactgg ttgccgatag tctcatagtc tattaagtgg taagtttcgt 71220
   acaaatacag aatccctaaa atattatcta atgttggatt aatctttacc ataactgtat 71280
   aaaatggaga cggagtcata actattttac cgtttgtact tactggaata gatgaaggaa 71340
   taatctccgg acatgctggt aaagacccaa atgtctgttt gaagaaatcc aatgttccag 71400
   gtcctaatct cttaacaaaa attacgatat tcgatcccga tatcctttgc attctattta 71460
   ccagcatatc acgaactata ttaagattat ctatcatgtc tattctccca ccgttatata 71520
   aatcgcctcc gctaagaaac gttagtatat ccatacaatg gaatacttca tttctaaaat 71580
   agtattcgtt ttctaattct ttaatgtgaa atcgtatact agaaagggaa aaattatctt 71640
   tgagttttcc gttagaaaag aaccacgaaa ctaatgttct gattgcgtcc gattccgttg 71700
   ctgaattaat ggatttacac caaaaactca tataacttct agatgtagaa gcattcgcta 71760
   aaaaattagt agaatcaaag gatataagta gatgttccaa caagtgagca attcccaaga 71820
   tttcatctat atcattctcg aatccgaaat tagaaattcc caagtagata tcctttttca 71880
   tccgatcgtt gatgaaaata cgaactttat tcggtaagac aatcatttac taaggagtaa 71940
   aataggaagt aatgttcgta tgtcgttatc atcgtataaa ttaaaggtgt gttttttacc 72000
   attaagtgac attataattt taccaatatt ggaattataa tataggtgta tttgcgcact 72060
   cgcgacggtt gatgcatcgg taaatatagc tgtatctaat gttctagtcg gtatttcatc 72120
   atttcgctgt ctaataatag cgttttctct atctgtttcc attacagctg cctgaagttt 72180
   attggtcgga taatatgtaa aataataaga aatacatacg aataacaaaa ataaaataag 72240
   atataataaa gatgccattt agagatctaa ttttgtttaa cttgtccaaa ttcctactta 72300
   cagaagatga ggaatcgttg gagatagtgt cttccttatg tagaggattt gaaatatctt 72360
   ataatgactt gataacttac tttccagata ggaaatacca taaatatatt tataaagtat 72420
   ttgaacatgt agatttatcg gaggaattaa gtatggaatt ccatgataca actctgagag 72480
   atttagtcta tcttagattg tacaagtatt ccaagtgtat acggccgtgt tataaattag 72540
   gagataatct aaaaggcata gttgttataa aggacaggaa tatttatatt agggaagcaa 72600
   atgatgactt gatagaatat ctcctcaagg aatacactcc tcagatttat acatattcta 72660
   atgagcgcgt ccccataact ggttcaaaat taattctttg tggattttct caagttacat 72720
   ttatggcgta tacaacgtcg catataacaa caaataaaaa ggtagatgtt ctcgtttcca 72780
   aaaaatgtat agatgaacta gtcgatccaa taaattatca aatacttcaa aatttatttg 72840
   ataaaggaag cggaacaata aacaaaatac tcaggaagat attttattcg gtaacaggtg 72900
   gccaaactcc ataggtagct ttttctattt cggattttag aatttccaaa ttcaccagcg 72960
   atttatcggt tttggtgaaa tccaaggatt tattaatgtc cacaaatgcc atttgttttg 73020
   tctgtggatt gtatttgaaa atggaaacga tgtagttaga tagatgcgct gcgaagtttc 73080
   ctattagggt tccgcgcttc acgtcaccca gcatacttga atcaccatcc tttaaaaaaa 73140
   atgataagat atcaacatgg agtatatcat actcgaattt taattcttct actgactcac 73200
   tgacattttc acaaatacta caatacggtt taccgaaaat aatcagtacg ttcttcattt 73260
   atgggtatca aaaacttaaa atcgttactg ctggaaaata aatcactgac gatattagat 73320
   gataatttat acaaagtata caatggaata tttgtggata caatgagtat ttatatagcc 73380
   gtcgccaatt gtgtcagaaa cttagaagag ttaactacgg tattcataaa atacgtaaac 73440
   ggatgggtaa aaaagggagg gcatgtaacc ctttttatcg atagaggaag tataaaaatt 73500
   aaacaagacg ttagagacaa gagacgtaaa tattctaaat taaccaagga cagaaaaatg 73560
   ttagaattag aaaagtgtac atccgaaata caaaatgtta ccggatttat ggaagaagaa 73620
   ataaaggcag aaatgcaatt aaaaatcgat aaactcacat ttcaaatata tttatctgat 73680
   tctgataaca taaaaatatc attgaatgag atactaacac atttcaacaa taatgagaat 73740
   gttacattat tttattgtga tgaacgagac gcagaattcg ttatgtgtct cgaggctaaa 73800
   acacatttct ctaccacagg agaatggccg ttgataataa gtaccgatca ggatactatg 73860
   ctatttgcat ctgctgataa tcatcctaag atgataaaaa acttaactca actgtttaaa 73920
   tttgttccct cggcagagga taactattta gcaaaattaa cggcgttagt gaatggatgt 73980
   gatttctttc ctggactcta tggggcatct ataacaccca ccaacttaaa caaaatacaa 74040
   ttgtttagtg attttacaat cgataatata gtcactagtt tggcaattaa aaattattat 74100
   agaaagacta actctaccgt agacgtgcgt aatattgtta cgtttataaa cgattacgct 74160
   aatttagacg atgtctactc gtatattcct ccttgtcaat gcactgttca agaatttata 74220
   ttttccgcat tagatgaaaa atggaatgaa tttaaatcat cttatttaga aagcgtgccg 74280
   ttaccctgcc aattaatgta cgcgttagaa ccacgcaagg agattgatgt ttcagaagtt 74340
   aaaactttat catcttatat agatttcgaa aatactaaat cagatatcga tgttataaaa 74400
   tctatatcct cgatcttcgg atattctaac gaaaactgta acacgatagt attcggcatc 74460
   tataaggata atttactact gagtataaat aattcatttt actttaacga tagtctgtta 74520
   ataaccaata ctaaaagtga taatataata aatataggtt actagattaa aaatggtgtt 74580
   ccaactcgtg tgctctacat gcggtaaaga tatttctcac gaacgatata aattgattat 74640
   acgaaaaaaa tcattaaagg atgtactcgt cagtgtaaag aacgaatgtt gtaggttaaa 74700
   attatctaca caaatagaac ctcaacgtaa cttaacagtg caacctctat tggatataaa 74760
   ctaatatgga tccggttaat tttatcaaga catatgcgcc tagaggttct attattttta 74820
   ttaattatac catgtcatta acaagtcatt tgaatccatc gatagaaaaa catgtgggta 74880
   tttattatgg tacgttatta tcggaacact tggtagttga atctacctat agaaaaggag 74940
   ttcgaatagt cccattggat agtttttttg aaggatatct tagtgcaaaa gtatacatgt 75000
   tagagaatat tcaagttatg aaaatagcag ctgatacgtc attaacttta ttgggtattc 75060
   cgtatggatt tggtcatgat agaatgtatt gttttaaatt ggtagctgac tgttataaaa 75120
   atgccggtat tgatacatcg tctaaacgaa tattgggcaa agatattttt ctgagccaaa 75180
   acttcacaga cgataataga tggataaaga tatatgattc taataattta acattttggc 75240
   aaattgatta ccttaaaggg tgagttaata tgcataacta ctcctccgtt gttttttccc 75300
   tcgttctttt tcttaacgtt gtttgccatc actctcataa tgtaaagata ttctaaaatg 75360
   gtaaactttt gcatatcgga cgcagaaatt ggtataaatg ttgtaattgt attatttccc 75420
   gtcaatggac tagtcacagc tccatcagtt ttatatcctt tagagtattt ctcactcgtg 75480
   tctaacattc tagagcattc catgatctgt ttatcgttga tattggccgg aaagatagat 75540
   tttttatttt ttattatatt actattggca attgtagata taacttctgg taaatatttt 75600
   tctacctttt caatctcttc tattttcaag ccggctatat attctgctat attgttgcta 75660
   gtatcaatac cttttctggc taagaagtca tatgtggtat tcactatatc agttttaact 75720
   ggtagttcca ttagcctttc cacttctgca gaataatcag aaattggttc tttaccagaa 75780
   aatccagcta ctataatagg ctcaccgatg atcattggca aaatcctata ttgtaccaga 75840
   ttaatgagag catatttcat ttccaataat tctgctagtt cttgagacat tgatttattt 75900
   gatgaatcta gttggttctc tagatactct accatttctg ccgcatacaa taacttgtta 75960
   gataaaatca gggttatcaa agtgtttagc gtggctagaa tagtgggctt gcatgtatta 76020
   aagaatgcgg tagtatgagt aaaccgtttt aacgaattat atagtctcca gaaatctgtg 76080
   gcgttacata catgagccga atgacatcga agattgtcca atatttttaa tagctgctct 76140
   ttgtccatta tttctatatt tgactcgcaa caattgtaga taccattaat caccgattcc 76200
   tttttcgatg ccggacaata gcacaattgt ttagctttgg actctatgta ttcagaatta 76260
   atagatatat ctctcaatac agattgcact atacattttg aaactatgtc aaaaattgta 76320
   gaacgacgct gttctgcagc catttaactt taaataattt acaaaaattt aaaatgagca 76380
   tccgtataaa aatcgataaa ctgcgccaaa ttgtggcata tttttcagag ttcagtgaag 76440
   aagtgtctat aaatgtagac tcgacggatg agttaatgta tatttttgcc gccttgggcg 76500
   gatctgtaaa catttgggca attatacctc taagtgcatc agtgttttac cgaggagccg 76560
   aaaatattgt gtttaatctt cctgtgtcca aggtaaaatc gtgtttgtgt agttttcaca 76620
   atgatgccat catagatata gaacctgatc tggaaaataa tctagtaaaa ctttctagtt 76680
   atcatgtagt aagtgtcgat tgtaacaagg aactgatgcc tattaggaca gatactacta 76740
   tttgtctaag tatagatcaa aagaaatctt acgtgtttaa ttttcacaag tatgaagaaa 76800
   aatgttgtgg tagaaccgtc attcatctag aatggttgtt gggctttatc aagtgtatta 76860
   gtcagcatca gcatctggct attatgttta aagatgacaa tattattatg aagactcctg 76920
   gtaatactga tgcgttttcc agggaatatt ctatgactga atgttctcaa gaactacaaa 76980
   agttttcttt caaaatagct atctcgtctc tcaacaaact acgaggattc aaaaagagag 77040
   tcaatgtttt tgaaactaga atcgtaatgg ataatgacga taacatttta ggaatgttgt 77100
   tttcggatag agttcaatcc tttaagatca acatctttat ggcgttttta gattaatact 77160
   ttcaatgaga taaatatggg tggcggagta agtgttgagc tccctaaacg ggatccgcct 77220
   ccgggagtac ccactgatga gatgttatta aacgtggata aaatgcatga cgtgatagct 77280
   cccgctaagc ttttagaata tgtgcatata ggaccactag caaaagataa agaggataaa 77340
   gtaaagaaaa gatatccaga gtttagatta gtcaacacag gacccggtgg tctttcggca 77400
   ttgttaagac aatcgtataa tggaaccgca cccaattgct gtcgcacttt taatcgtact 77460
   cattattgga agaaggatgg aaagatatca gataagtatg aagagggtgc agtattagaa 77520
   tcgtgttggc cagacgttca cgacactgga aaatgcgatg ttgatttatt cgactggtgt 77580
   cagggggata cgttcgatag aaacatatgc catcagtgga tcggttcagc ctttaatagg 77640
   agtaatagaa ctgtagaggg tcaacaatcg ttaataaatc tgtataataa gatgcaaaca 77700
   ttatgtagta aagatgctag tgtaccaata tgcgaatcat ttttgcatta tttacgcgca 77760
   cacaatacag aagatagcaa agagatgatc gattatattc taagacaaca gtctgcggac 77820
   tttaaacaga aatatatgag atgtagttat cccactagag ataagttaga agagtcatta 77880
   aaatatgcgg aacctcgaga atgttgggat ccagagtgtt cgaatgccaa tgttaatttc 77940
   ttactaacac gtaattataa taatttagga ctttgcaata ttgtacgatg taataccagc 78000
   gtgaacaact tacagatgga taaaacttcc tcattaagat tgtcatgtgg attaagcaat 78060
   agtgatagat tttctactgt tcccgtcaat agagcaaaag tagttcaaca taatattaaa 78120
   cactcgttcg acctaaaatt gcatttgatc agtttattat ctctcttggt aatatggata 78180
   ctaattgtag ctatttaaat gggtgccgcg gcaagcatac agacgacggt gaatacactc 78240
   agcgaacgta tctcgtctaa attagaacaa gaagcgaacg ctagtgctca aacaaaatgt 78300
   gatatagaaa tcggaaattt ttatatccga caaaaccatg gatgtaacct cactgttaaa 78360
   aatatgtgct ctgcggacgc ggatgctcag ttggatgctg tgttatcagc cgctacagaa 78420
   acatatagtg gattaacacc ggaacaaaaa gcatacgtgc cagctatgtt tactgctgcg 78480
   ttaaacattc agacgagtgt aaacactgtt gttagagatt ttgaaaatta tgtgaaacag 78540
   acttgtaatt ctagcgcggt cgtcgataac aaattaaaga tacaaaacgt aatcatagat 78600
   gaatgttacg gagccccagg atctccaaca aatttggaat ttattaatac aggatctagc 78660
   aaaggaaatt gtgccattaa agcgttgatg caattgacga ctaaggccac tactcaaata 78720
   gcacctagac aagttgctgg tacaggagtt cagttttata tgattgttat cggtgttata 78780
   atattggcag cgttgtttat gtactatgcc aagcgtatgt tgttcacatc caccaatgat 78840
   aaaatcaaac ttattttagc caataaggaa aacgtccatt ggactactta catggacaca 78900
   ttctttagaa cttctccgat ggttattgct accacggata tgcaaaactg aaaatatatt 78960
   gataatattt taatagatta acatggaagt tatcgctgat cgtctagacg atatagtgaa 79020
   acaaaatata gcggatgaaa aatttgtaga ttttgttata cacggtctag agcatcaatg 79080
   tcctgctata cttcgaccat taattaggtt gtttattgat atactattat ttgttatagt 79140
   aatttatatt tttacggtac gtctagtaag tagaaattat caaatgttgt tggtggtgct 79200
   agtcatcaca ttaactattt tttattactt tatactataa tagtactaga ctgacttcta 79260
   acaaacatct cacctgccat aaataaatgc ttgatattaa agtcttctat ttctaacact 79320
   attccatctg tggaaaataa tactctgaca ttatcgctaa ttgacacatc ggtgagtgat 79380
   atgcctataa agtaataatc ttctttgggc acatatacca gtgtaccagg ttctaacaac 79440
   ctatttactg gtgctcctgt agcatacttt ttctttacct tgagaatatc catcgtttgc 79500
   ttggtcaata gcgatatgtg attttttatc aaccactcaa aaaagtaatt ggagtgttca 79560
   tatcctctac gggctattgt ctcatggccg tgtatgaaat ttaagtaaca cgactgtggt 79620
   agatttgttc tatagagccg attgccgcaa atagatagaa ctaccaatat gtctgtacaa 79680
   atgttaaaca ttaattgatt aacagaaaaa acaatgttcg ttctgggaat agaaaccaga 79740
   tcaaaacaaa attcgttaga atatatgcca cgtttataca tggaatataa aataactaca 79800
   gtttgaaaaa taacagtatc atttaaacat ttaacttgcg gggttaatct cacaacttta 79860
   ctgtttttga actgttcaaa atatagcatc gatccgtgag aaatacgttt agccgccttt 79920
   aatagaggaa atcccaccgc ctttctggat ctcaccaacg acgatagttc tgaccagcaa 79980
   ctcatttctt catcatccac ctgttttaac atataatagg caggagatag atatccgtca 80040
   ttgcaatatt ccttctcgta ggcacacaat ctaatattga taaaatctcc attctcttct 80100
   ctgcatttat tatcttgtct cggtggctga ttaggctgtg gtcttggttt aggccttggt 80160
   ctatcgttgt tgaatctatt ttggtcatta aatctttcat ttcttcctgg tatatttcta 80220
   tcacctcgtt tggttggatt tttgtctata ttatcgtttg taacatcggt acgggtattc 80280
   atttatcaca aaaaaaactt ctctaaatga gtctactgct agaaaacctc atcgaagaag 80340
   ataccatatt ttttgcagga agtatatctg agtatgatga tttacaaatg gttattgccg 80400
   gcgcaaaatc caaatttcca agatctatgc tttctatttt taatatagta cctagaacga 80460
   tgtcaaaata tgagttggag ttgattcata acgaaaatat cacaggagca atgtttacca 80520
   caatgtataa tataagaaac aatttgggtc taggagatga taaactaact attgaagcca 80580
   ttgaaaacta tttcttggat cctaacaatg aagttatgcc tcttattatt aataatacgg 80640
   atatgactgc cgtcattcct aaaaaaagtg gtaggagaaa gaataagaac atggttatct 80700
   tccgtcaagg atcatcacct atcttgtgca ttttcgaaac tcgtaaaaag attaatattt 80760
   ataaagaaaa tatggaatcc gcgtcgactg agtatacacc tatcggagac aacaaggctt 80820
   tgatatctaa atatgcggga attaatgtcc tgaatgtgta ttctccttcc acatccataa 80880
   gattgaatgc catttacgga ttcaccaata aaaataaact agagaaactt agtactaata 80940
   aggaactaga atcgtatagt tctagccctc ttcaagaacc cattaggtta aatgattttc 81000
   tgggactatt ggaatgtgtt aaaaagaata ttcctctaac agatattccg acaaaggatt 81060
   gattactata aatggagaat gttcctaatg tatactttaa tcctgtgttt atagagccca 81120
   cgtttaaaca ttctttatta agtgtttata aacacagatt aatagtttta tttgaagtat 81180
   tcgttgtatt cattctaata tatgtatttt ttagatctga attaaatatg ttcttcatgc 81240
   ctaaacgaaa aatacccgat cctattgata gattacgacg tgctaatcta gcgtgtgaag 81300
   acgataaatt aatgatctat ggattaccat ggatgacaac tcaaacatct gcgttatcaa 81360
   taaatagtaa accgatagtg tataaagatt gtgcaaagct tttgcgatca ataaatggat 81420
   cacaaccagt atctcttaac gatgttcttc gcagatgatg attcattttt taagtatttg 81480
   gctagtcaag atgatgaatc ttcattatct gatatattgc aaatcactca atatctagac 81540
   tttctgttat tattattgat ccaatcaaaa aataaattag aagctgtggg tcattgttat 81600
   gaatctcttt cagaggaata cagacaattg acaaaattca cagactttca agattttaaa 81660
   aaactgttta acaaggtccc tattgttaca gatggaaggg tcaaacttaa taaaggatat 81720
   ttgttcgact ttgtgattag tttgatgcga ttcaaaaaag aatcctctct agctaccacc 81780
   gcaatagatc ctgttagata catagatcct cgtcgcaata tcgcattttc taacgtgatg 81840
   gatatattaa agtcgaataa agtgaacaat aattaattct ttattgtcat catgaacggc 81900
   ggacatattc agttgataat cggccccatg ttttcaggta aaagtacaga attaattaga 81960
   cgagttagac gttatcaaat agctcaatat aaatgcgtga ctataaaata ttctaacgat 82020
   aatagatacg gaacgggact atggacgcat gataagaata attttgaagc attggaagca 82080
   actaaactat gtgatgtctt ggaatcaatt acagatttct ccgtgatagg tatcgatgaa 82140
   ggacagttct ttccagacat tgttgaatta gatcgataaa aattaattaa ttacccgggt 82200
   accaggccta gatctgtcga gatccggtgg tggtgcaaat caaagaactg ctcctcagtg 82260
   gatgttgcct ttacttctag gcctgtacgg aagtgttact tctgctctaa aagctgctgc 82320
   aggggggggg gggggtgggg ggggccggtt agggccgcca tcccctcaga gcgtcgggat 82380
   atcgggtggc ggctcgggac ggaggacgcg ctagtgttct tctgtgtggc agttcagaat 82440
   gatggatcaa gctagatcag cattctctaa cttgtttggt ggagaaccat tgtcatatac 82500
   ccggttcagc ctggctcggc aagtagatgg cgataacagt catgtggaga tgaaacttgc 82560
   tgtagatgaa gaagaaaatg ctgacaataa cacaaaggcc aatgtcacaa aaccaaaaag 82620
   gtgtagtgga agtatctgct atgggactat tgctgtgatc gtctttttct tgattggatt 82680
   tatgattggc tacttgggct attgtaaagg ggtagaacca aaaactgagt gtgagagact 82740
   ggcaggaacc gagtctccag tgagggagga gccaggagag gacttccctg cagcacgtcg 82800
   cttatattgg gatgacctga agagaaagtt gtcggagaaa ctggacagca cagacttcac 82860
   cagcaccatc aagctgctga atgaaaattc atatgtccct cgtgaggctg gatctcaaaa 82920
   agatgaaaat cttgcgttgt atgttgaaaa tcaatttcgt gaatttaaac tcagcaaagt 82980
   ctggcgtgat caacattttg ttaagattca ggtcaaagac agcgctcaaa actcggtgat 83040
   catagttgat aagaacggta gacttgttta cctggtggag aatcctgggg gttatgtggc 83100
   gtatagtaag gctgcaacag ttactggtaa actggtccat gctaattttg gtactaaaaa 83160
   agattttgag gatttataca ctcctgtgaa tggatctata gtgattgtca gagcagggaa 83220
   aatcaccttt gcagaaaagg ttgcaaatgc tgaaagctta aatgcaattg gtgtgttgat 83280
   atacatggac cagactaaat ttcccattgt taacgcagaa ctttcattct ttggacatgc 83340
   tcatctgggg acaggtgacc cttacacacc tggattccct tccttcaatc acactcagtt 83400
   tccaccatct cggtcatcag gattgcctaa tatacctgtc cagacaatct ccagagctgc 83460
   tgcagaaaag ctgtttggga atatggaagg agactgtccc tctgactgga aaacagactc 83520
   tacatgtagg atggtaacct cagaaagcaa gaatgtgaag ctcactgtga gcaatgtgct 83580
   gaaagagata aaaattctta acatctttgg agttattaaa ggctttgtag aaccagatca 83640
   ctatgttgta gttggggccc agagagatgc atggggccct ggagctgcaa aatccggtgt 83700
   aggcacagct ctcctattga aacttgccca gatgttctca gatatggtct taaaagatgg 83760
   gtttcagccc agcagaagca ttatctttgc cagttggagt gctggagact ttggatcggt 83820
   tggtgccact gaatggctag agggatacct ttcgtccctg catttaaagg ctttcactta 83880
   tattaatctg gataaagcgg ttcttggtac cagcaacttc aaggtttctg ccagcccact 83940
   gttgtatacg cttattgaga aaacaatgca aaatgtgaag catccggtta ctgggcaatt 84000
   tctatatcag gacagcaact gggccagcaa agttgagaaa ctcactttag acaatgctgc 84060
   tttccctttc cttgcatatt ctggaatccc agcagtttct ttctgttttt gcgaggacac 84120
   agattatcct tatttgggta ccaccatgga cacctataag gaactgattg agaggattcc 84180
   tgagttgaac aaagtggcac gagcagctgc agaggtcgct ggtcagttcg tgattaaact 84240
   aacccatgat gttgaattga acctggacta tgagaggtac aacagccaac tgctttcatt 84300
   tgtgagggat ctgaaccaat acagagcaga cataaaggaa atgggcctga gtttacagtg 84360
   gctgtattct gctcgtggag acttcttccg tgctacttcc agactaacaa cagatttcgg 84420
   gaatgctgag aaaacagaca gatttgtcat gaagaaactc aatgatcgtg tcatgagagt 84480
   ggagtatcac ttcctctctc cctacgtatc tccaaaagag tctcctttcc gacatgtctt 84540
   ctggggctcc ggctctcaca cgctgccagc tttactggag aacttgaaac tgcgtaaaca 84600
   aaataacggt gcttttaatg aaacgctgtt cagaaaccag ttggctctag ctacttggac 84660
   tattcaggga gctgcaaatg ccctctctgg tgacgtttgg gacattgaca atgagtttta 84720
   aatgtgatac ccatagcttc catgagaaca gcagggtagt ctggtttcta gacttgtgct 84780
   gatcgtgcta aattttcagt agggctacaa aacctgatgt taaaattcca tcccatcatc 84840
   ttggtactac tagatgtctt taggcagcag cttttaatac agggtagata acctgtactt 84900
   caagttaaag tgaataacca cttaaaaaat gtccatgatg gaatattccc ctatctctag 84960
   aattttaagt gctttgtaat gggaactgcc tctttcctgt tgttgttaat gaaaatgtca 85020
   gaaaccagtt atgtgaatga tctctctgaa tcctaagggc tggtctctgc tgaaggttgt 85080
   aagtggtcgc ttactttgag tgatcctcca acttcatttg atgctaaata ggagatacca 85140
   ggttgaaaga ccttctccaa atgagatcta agcctttcca taaggaatgt agctggtttc 85200
   ctcattcctg aaagaaacag ttaactttca gaagagatgg gcttgttttc ttgccaatga 85260
   ggtctgaaat ggaggtcctt ctgctggata aaatgaggtt caactgttga ttgcaggaat 85320
   aaggccttaa tatgttaacc tcagtgtcat ttatgaaaag aggggaccag aagccaaaga 85380
   cttagtatat tttcttttcc tctgtccctt cccccataag cctccattta gttctttgtt 85440
   atttttgttt cttccaaagc acattgaaag agaaccagtt tcaggtgttt agttgcagac 85500
   tcagtttgtc agactttaaa gaataatatg ctgccaaatt ttggccaaag tgttaatctt 85560
   aggggagagc tttctgtcct tttggcactg agatatttat tgtttattta tcagtgacag 85620
   agttcactat aaatggtgtt tttttaatag aatataatta tcggaagcag tgccttccat 85680
   aattatgaca gttatactgt cggttttttt taaataaaag cagcatctgc taataaaacc 85740
   caacagatac tggaagtttt gcatttatgg tcaacactta agggttttag aaaacagccg 85800
   tcagccaaat gtaattgaat aaagttgaag ctaagattta gagatgaatt aaatttaatt 85860
   aggggttgct aagaagcgag cactgaccag ataagaatgc tggttttcct aaatgcagtg 85920
   aattgtgacc aagttataaa tcaatgtcac ttaaaggctg tggtagtact cctgcaaaat 85980
   tttatagctc agtttatcca aggtgtaact ctaattccca ttttgcaaaa tttccagtac 86040
   ctttgtcaca atcctaacac attatcggga gcagtatctt ccataatgta taaagaacaa 86100
   ggtagttttt acctaccaca gtgtctgtat cggagacagt gatctccata tgttacacta 86160
   agggtgtaag taattatcgg gaacagtgtt tcccataatt ttcttcatgc aatgacatct 86220
   tcaaagcttg aagatcgtta gtatctaaca tgtatcccaa ctcctataat tccctatctt 86280
   ttagttttag ttgcagaaac attttgtggt cattaagcat tgggtgggta aattcaacca 86340
   ctgtaaaatg aaattactac aaaatttgaa atttagcttg ggtttttgtt acctttatgg 86400
   tttctccagg tcctctactt aatgagatag tagcatacat ttataatgtt tgctattgac 86460
   aagtcatttt aactttatca cattatttgc atgttacctc ctataaactt agtgcggaca 86520
   agttttaatc cagaattgac cttttgactt aaagcagagg gactttgtat agaaggtttg 86580
   ggggctgtgg ggaaggagag tcccctgaag gtctgacacg tctgcctacc cattcgtggt 86640
   gatcaattaa atgtaggtat gaataagttc gaagctccgt gagtgaacca tcattataaa 86700
   cgtgatgatc agctgtttgt catagggcag ttggaaacgg cctcctaggg aaaagttcat 86760
   agggtctctt caggttctta gtgtcactta cctagattta cagcctcact tgaatgtgtc 86820
   actactcaca gtctctttaa tcttcagttt tatctttaat ctcctctttt atcttggact 86880
   gacatttagc gtagctaagt gaaaaggtca tagctgagat tcctggttcg ggtgttacgc 86940
   acacgtactt aaatgaaagc atgtggcatg ttcatcgtat aacacaatat gaatacaggg 87000
   catgcatttt gcagcagtga gtctcttcag aaaacccttt tctacagtta gggttgagtt 87060
   acttcctatc aagccagtac gtgctaacag gctcaatatt cctgaatgaa atatcagact 87120
   agtgacaagc tcctggtctt gagatgtctt ctcgttaagg agatgggcct tttggaggta 87180
   aaggataaaa tgaatgagtt ctgtcatgat tcactattct agaacttgca tgacctttac 87240
   tgtgttagct ctttgaatgt tcttgaaatt ttagactttc tttgtaaaca aatgatatgt 87300
   ccttatcatt gtataaaagc tgttatgtgc aacagtgtgg agattccttg tctgatttaa 87360
   taaaatactt aaacactgaa aaaaaaaaaa aaaaaaaaaa aaaaaaaaaa aaaaaaaaaa 87420
   aaaaaaaaaa aaaaaaaaaa aaaaaaaaaa aaaaaaaaag taccttctga ggcggaaaga 87480
   accagccgga tctcgacttc gagcttattt atattccaaa aaaaaaaaat aaaatttcaa 87540
   tttttaagct ttcactaatt ccaaacccac ccgcttttta tagtaagttt ttcacccata 87600
   aataataaat acaataatta atttctcgta aaagtagaaa atatattcta atttattgca 87660
   cggtaaggaa gtagatcata actcgagcat gggagatccc gtcgttttac aacgtcgtga 87720
   ctgggaaaac cctggcgtta cccaacttaa tcgccttgca gcacatcccc ctttcgccag 87780
   ctggcgtaat agcgaagagg cccgcaccga tcgcccttcc caacagttgc gcagcctgaa 87840
   tggcgaatgg cgctttgcct ggtttccggc accagaagcg gtgccggaaa gctggctgga 87900
   gtgcgatctt cctgaggccg atactgtcgt cgtcccctca aactggcaga tgcacggtta 87960
   cgatgcgccc atctacacca acgtgaccta tcccattacg gtcaatccgc cgtttgttcc 88020
   cacggagaat ccgacgggtt gttactcgct cacatttaat gttgatgaaa gctggctaca 88080
   ggaaggccag acgcgaatta tttttgatgg cgttaactcg gcgtttcatc tgtggtgcaa 88140
   cgggcgctgg gtcggttacg gccaggacag tcgtttgccg tctgaatttg acctgagcgc 88200
   atttttacgc gccggagaaa accgcctcgc ggtgatggtg ctgcgctgga gtgacggcag 88260
   ttatctggaa gatcaggata tgtggcggat gagcggcatt ttccgtgacg tctcgttgct 88320
   gcataaaccg actacacaaa tcagcgattt ccatgttgcc actcgcttta atgatgattt 88380
   cagccgcgct gtactggagg ctgaagttca gatgtgcggc gagttgcgtg actacctacg 88440
   ggtaacagtt tctttatggc agggtgaaac gcaggtcgcc agcggcaccg cgcctttcgg 88500
   cggtgaaatt atcgatgagc gtggtggtta tgccgatcgc gtcacactac gtctgaacgt 88560
   cgaaaacccg aaactgtgga gcgccgaaat cccgaatctc tatcgtgcgg tggttgaact 88620
   gcacaccgcc gacggcacgc tgattgaagc agaagcctgc gatgtcggtt tccgcgaggt 88680
   gcggattgaa aatggtctgc tgctgctgaa cggcaagccg ttgctgattc gaggcgttaa 88740
   ccgtcacgag catcatcctc tgcatggtca ggtcatggat gagcagacga tggtgcagga 88800
   tatcctgctg atgaagcaga acaactttaa cgccgtgcgc tgttcgcatt atccgaacca 88860
   tccgctgtgg tacacgctgt gcgaccgcta cggcctgtat gtggtggatg aagccaatat 88920
   tgaaacccac ggcatggtgc caatgaatcg tctgaccgat gatccgcgct ggctaccggc 88980
   gatgagcgaa cgcgtaacgc gaatggtgca gcgcgatcgt aatcacccga gtgtgatcat 89040
   ctggtcgctg gggaatgaat caggccacgg cgctaatcac gacgcgctgt atcgctggat 89100
   caaatctgtc gatccttccc gcccggtgca gtatgaaggc ggcggagccg acaccacggc 89160
   caccgatatt atttgcccga tgtacgcgcg cgtggatgaa gaccagccct tcccggctgt 89220
   gccgaaatgg tccatcaaaa aatggctttc gctacctgga gagacgcgcc cgctgatcct 89280
   ttgcgaatac gcccacgcga tgggtaacag tcttggcggt ttcgctaaat actggcaggc 89340
   gtttcgtcag tatccccgtt tacagggcgg cttcgtctgg gactgggtgg atcagtcgct 89400
   gattaaatat gatgaaaacg gcaacccgtg gtcggcttac ggcggtgatt ttggcgatac 89460
   gccgaacgat cgccagttct gtatgaacgg tctggtcttt gccgaccgca cgccgcatcc 89520
   agcgctgacg gaagcaaaac accagcagca gtttttccag ttccgtttat ccgggcaaac 89580
   catcgaagtg accagcgaat acctgttccg tcatagcgat aacgagctcc tgcactggat 89640
   ggtggcgctg gatggtaagc cgctggcaag cggtgaagtg cctctggatg tcgctccaca 89700
   aggtaaacag ttgattgaac tgcctgaact accgcagccg gagagcgccg ggcaactctg 89760
   gctcacagta cgcgtagtgc aaccgaacgc gaccgcatgg tcagaagccg ggcacatcag 89820
   cgcctggcag cagtggcgtc tggcggaaaa cctcagtgtg acgctccccg ccgcgtccca 89880
   cgccatcccg catctgacca ccagcgaaat ggatttttgc atcgagctgg gtaataagcg 89940
   ttggcaattt aaccgccagt caggctttct ttcacagatg tggattggcg ataaaaaaca 90000
   actgctgacg ccgctgcgcg atcagttcac ccgtgcaccg ctggataacg acattggcgt 90060
   aagtgaagcg acccgcattg accctaacgc ctgggtcgaa cgctggaagg cggcgggcca 90120
   ttaccaggcc gaagcagcgt tgttgcagtg cacggcagat acacttgctg atgcggtgct 90180
   gattacgacc gctcacgcgt ggcagcatca ggggaaaacc ttatttatca gccggaaaac 90240
   ctaccggatt gatggtagtg gtcaaatggc gattaccgtt gatgttgaag tggcgagcga 90300
   tacaccgcat ccggcgcgga ttggcctgaa ctgccagctg gcgcaggtag cagagcgggt 90360
   aaactggctc ggattagggc cgcaagaaaa ctatcccgac cgccttactg ccgcctgttt 90420
   tgaccgctgg gatctgccat tgtcagacat gtataccccg tacgtcttcc cgagcgaaaa 90480
   cggtctgcgc tgcgggacgc gcgaattgaa ttatggccca caccagtggc gcggcgactt 90540
   ccagttcaac atcagccgct acagtcaaca gcaactgatg gaaaccagcc atcgccatct 90600
   gctgcacgcg gaagaaggca catggctgaa tatcgacggt ttccatatgg ggattggtgg 90660
   cgacgactcc tggagcccgt cagtatcggc ggaattccag ctgagcgccg gtcgctacca 90720
   ttaccagttg gtctggtgtc aaaaataata ataaccgggc aggggggatc caattctgtg 90780
   agcgtatggc aaacgaagga aaaatagtta tagtagccgc actcgatggg acatttcaac 90840
   gtaaaccgtt taataatatt ttgaatctta ttccattatc tgaaatggtg gtaaaactaa 90900
   ctgctgtgtg tatgaaatgc tttaaggagg cttccttttc taaacgattg ggtgaggaaa 90960
   ccgagataga aataatagga ggtaatgata tgtatcaatc ggtgtgtaga aagtgttaca 91020
   tcgactcata atattatatt ttttatctaa aaaactaaaa ataaacattg attaaatttt 91080
   aatataatac ttaaaaatgg atgttgtgtc gttagataaa ccgtttatgt attttgagga 91140
   aattgataat gagttagatt acgaaccaga aagtgcaaat gaggtcgcaa aaaaactgcc 91200
   gtatcaagga cagttaaaac tattactagg agaattattt tttcttagta agttacagcg 91260
   acacggtata ttagatggtg ccaccgtagt gtatatagga tctgctcccg gtacacatat 91320
   acgttatttg agagatcatt tctataattt aggagtgatc atcaaatgga tgctaattga 91380
   cggccgccat catgatccta ttttaaatgg attgcgtgat gtgactctag tgactcggtt 91440
   cgttgatgag gaatatctac gatccatcaa aaaacaactg catccttcta agattatttt 91500
   aatttctgat gtgagatcca aacgaggagg aaatgaacct agtacggcgg atttactaag 91560
   taattacgct ctacaaaatg tcatgattag tattttaaac cccgtggcgt ctagtcttaa 91620
   atggagatgc ccgtttccag atcaatggat caaggacttt tatatcccac acggtaataa 91680
   aatgttacaa ccttttgctc cttcatattc agctgaaatg agattattaa gtatttatac 91740
   cggtgagaac atgagactga ctcgagttac caaattagac gctgtaaatt atgaaaaaaa 91800
   gatgtactac cttaataaga tcgtccgtaa caaagtagtt gttaactttg attatcctaa 91860
   tcaggaatat gactattttc acatgtactt tatgctgagg accgtgtact gcaataaaac 91920
   atttcctact actaaagcaa aggtactatt tctacaacaa tctatatttc gtttcttaaa 91980
   tattccaaca acatcaactg aaaaagttag tcatgaacca atacaacgta aaatatctag 92040
   caaaaattct atgtctaaaa acagaaatag caagagatcc gtacgcggta ataaatagaa 92100
   acgtactact gagatatact accgatatag agtataatga tttagttact ttaataaccg 92160
   ttagacataa aattgattct atgaaaactg tgtttcaggt atttaacgaa tcatccataa 92220
   attatactcc ggttgatgat gattatggag aaccaatcat tataacatcg tatcttcaaa 92280
   aaggtcataa caagtttcct gtaaattttc tatacataga tgtggtaata tctgacttat 92340
   ttcctagctt tgttagacta gatactacag aaactaatat agttaatagt gtactacaaa 92400
   caggcgatgg taaaaagact cttcgtcttc ccaaaatgtt agagacggaa atagttgtca 92460
   agattctcta tcgccctaat ataccattaa aaattgttag atttttccgc aataacatgg 92520
   taactggagt agagatagcc gatagatctg ttatttcagt cgctgattaa tcaattagta 92580
   gagatgagat aagaacatta taataatcaa taatatatct tatatcttat atcttatatc 92640
   ttatatcttg tttagaaaaa tgctaatatt aaaatagcta acgctagtaa tccaatcgga 92700
   agccatttga tatctataat agggtatcta atttcctgat ttaaatagcg gacagctata 92760
   ttctcggtag ctactcgttt ggaatcacaa acattattta catctaattt actatctgta 92820
   atggaaacgt ttcccaatga aatggtacaa tccgatacat tgcattttgt tatatttttt 92880
   tttaaagagg ctggtaacaa cgcatcgctt cgtttacatg gctcgtacca acaataatag 92940
   ggtaatcttg tatctattcc tatccgtact atgcttttat caggataaat acatttacat 93000
   cgtatatcgt ctttgttagc atcacagaat gcataaattt gttcgtccgt catgataaaa 93060
   atttaaagtg taaatataac tattattttt atagttgtaa taaaaaggga aatttgattg 93120
   tatactttcg gttctttaaa agaaactgac ttgataaaaa tggctgtaat ctctaaggtt 93180
   acgtatagtc tatatgatca aaaagagatt aatgctacag atattatcat tagtcatgtt 93240
   aaaaatgacg acgatatcgg taccgttaaa gatggtagac taggtgctat ggatggggca 93300
   ttatgtaaga cttgtgggaa aacggaattg gaatgtttcg gtcactgggg taaagtaagt 93360
   atttataaaa ctcatatagt taagcctgaa tttatttcag aaattattcg tttactgaat 93420
   catatatgta ttcactgcgg attattgcgt tcacgagaac cgtattccga cgatattaac 93480
   ctaaaagagt tatcgggaca cgctcttagg agattaaagg ataaaatatt atccaagaaa 93540
   aagtcatgtt ggaacagtga atgtatgcaa ccgtatcaaa aaattacttt ttcaaagaaa 93600
   aaggtttgtt tcgtcaacaa gttggatgat attaacgttc ctaattctct catctatcaa 93660
   aagttaattt ctattcatga aaagttttgg ccattattag aaattcatca atatccagct 93720
   aacttatttt atacagacta ctttcccatc cctccgttga ttattagacc ggctattagt 93780
   ttttggatag atagtatacc caaagagacc aatgaattaa cttacttatt aggtatgatc 93840
   gttaagaatt gtaacttgaa tgctgatgaa caggttatcc agaaggcggt aatagaatac 93900
   gatgatatta aaattatttc taataacact accagtatca atttatcata tattacatcc 93960
   ggcaaaaata atatgattag aagttatatc gtcgcccgac gaaaagatca gaccgctaga 94020
   tctgtaattg gtcccagtac atctatcacc gttaatgagg taggaatgcc cgcatatatt 94080
   agaaatacac ttacagaaaa gatatttgtt aatgccttta cagtggataa agttaaacaa 94140
   ctattagcgt caaaccaagt taaattttac tttaataaac gattaaacca attaacaaga 94200
   atacgccaag gaaagtttat taaaaataaa atacatttat tgcctggtga ttgggtagaa 94260
   gtagctgttc aagaatatac aagtattatt tttggaagac agccgtctct acatagatac 94320
   aacgtcatcg cttcatctat cagagctacc gaaggagata ctatcaaaat atctcccgga 94380
   attgccaact ctcaaaatgc tgatttcgac ggggatgagg aatggatgat attagaacaa 94440
   aatcctaaag ctgtaattga acaaagtatt cttatgtatc cgacgacgtt actcaaacac 94500
   gatattcatg gagcccccgt ttatggatct attcaagatg aaatcgtagc agcgtattca 94560
   ttgtttagga tacaagatct ttgtttagat gaagtattga acatcttggg gaaatatgga 94620
   agagagttcg atcctaaagg taaatgtaaa ttcagcggta aagatatcta tacttacttg 94680
   ataggtgaaa agattaatta tccgggtctc ttaaaggatg gtgaaattat tgcaaacgac 94740
   gtagatagta attttgttgt ggctatgagg catctgtcat tggctggact cttatccgat 94800
   cataagtcga acgtggaagg tatcaacttt attatcaagt catcttatgt ttttaagaga 94860
   tatctatcta tttacggttt tggggtgaca ttcaaagatc tgagaccaaa ttcgacgttc 94920
   actaataaat tggaggccat caacgtagaa aaaatagaac ttatcaaaga agcatacgcc 94980
   aaatatctca acgatgtaag agacgggaaa atagttccat tatctaaagc tttagaggcg 95040
   gactatgtgg aatccatgtt atccaacttg acaaatctta atatccgaga gatagaagaa 95100
   catatgagac aaacgctgat agatgatcca gataataacc tcctgaaaat ggccaaagcg 95160
   ggttataaag taaatcccac agaactaatg tatattctag gtacgtatgg acaacaaagg 95220
   attgatggtg aaccagcaga gactcgagta ttgggtagag ttttacctta ctatcttcca 95280
   gactctaagg atccagaagg aagaggttac attcttaatt ctttaacaaa aggattaacg 95340
   ggttctcaat attacttttc gatgctggtt gccagatctc aatctactga tatcgtctgt 95400
   gaaacatcac gtaccggaac actggctaga aaaatcatta aaaagatgga ggatatggtg 95460
   gtcgacggat acggacaagt agttataggt aatacgctca tcaagtacgc cgccaattat 95520
   accaaaattc taggctcagt atgtaaacct gtagatctta tctatccaga tgagtccatg 95580
   acttggtatt tggaaattag tgctctgtgg aataaaataa aacagggatt cgtttactct 95640
   cagaaacaga aacttgcaaa gaagacattg gcgccgttta atttcctagt attcgtcaaa 95700
   cccaccactg aggataatgc tattaaggtt aaggatctgt acgatatgat tcataacgtc 95760
   attgatgatg tgagagagaa atacttcttt acggtatcta atatagattt tatggagtat 95820
   atattcttga cgcatcttaa tccttctaga attagaatta caaaagaaac ggctatcact 95880
   atctttgaaa agttctatga aaaactcaat tatactctag gtggtggaac tcctattgga 95940
   attatttctg cacaggtatt gtctgagaag tttacacaac aagccctgtc cagttttcac 96000
   actactgaaa aaagtggtgc cgtcaaacaa aaacttggtt tcaacgagtt taataacttg 96060
   actaatttga gtaagaataa gaccgaaatt atcactctgg tatccgatga tatctctaaa 96120
   cttcaatctg ttaagattaa tttcgaattt gtatgtttgg gagaattaaa tccaaacatc 96180
   actcttcgaa aagaaacaga taaatatgta gtagatataa tagtcaatag attatacatc 96240
   aagagagcag aaataaccga attagtcgtc gaatatatga ttgaacgatt tatctccttt 96300
   agcgtcattg taaaggaatg gggtatggag acattcattg aggacgagga taatattaga 96360
   tttactgtct acctaaattt cgttgaaccg gaagaattga atcttagtaa gtttatgatg 96420
   gttcttccgg gggcagccaa caagggaaag attagtaaat tcaagattcc tatctctgat 96480
   tatacgggat atgacgactt caatcaaaca aaaaagctca ataagatgac tgtagaactc 96540
   atgaatctaa aagaattggg ttctttcgat ttggaaaacg tcaacgtgta tcctggagta 96600
   tggaatacat acgatatctt cggtatcgag gccgctcgtg aatacttgtg cgaagccatg 96660
   ttaaacacct atggagaagg gttcgattat ctgtatcagc cttgtgatct tctcgctagt 96720
   ttactatgtg ctagttacga accagaatca gtgaataaat tcaagttcgg cgcagctagt 96780
   actcttaaga gagctacgtt cggagacaat aaagcattgt taaacgcggc tcttcataaa 96840
   aagtcagaac ctattaacga taatagtagc tgccactttt ttagcaaggt ccctaatata 96900
   ggaactggat attacaaata ctttatcgac ttgggtcttc tcatgagaat ggaaaggaaa 96960
   ctatctgata agatatcttc tcaaaagatc aaggaaatgg aagaaacaga agacttttaa 97020
   ttcttatcaa taacatattt ttctatgatc tgtcttttaa acgatggatt ttccacaaat 97080
   gcgcctctca agtccctcat agaatgatac acgtataaaa aatatagcat aggcaatgac 97140
   tccttatttt tagacattag atatgccaaa atcatagccc cgcttctatt tactcccgca 97200
   gcacaatgaa ccaacacggg ctcgtttcgt tgatcacatt tagataaaaa ggcggttacg 97260
   tcgtcaaaat atttactaat atcggtagtt gtatcatcta ccaacggtat atgaataata 97320
   ttaatattag agttaggtaa tgtatattta tccatcgtca aatttaaaac atatttgaac 97380
   ttaacttcag atgatggtgc atccatagca tttttataat ttcccaaata cacattattg 97440
   gttacccttg tcattatagt gggagatttg gctttgtgca tatctccagt tgaacgtagt 97500
   agtaagtatt tatacaaact tttcttatcc atttataacg tacaaatgga taaaactact 97560
   ttatcggtaa acgcgtgtaa tttagaatac gttagagaaa aggctatagt aggcgtacaa 97620
   gcagccaaaa catcaacact tatattcttt gttattatat tggcaattag tgcgctatta 97680
   ctctggtttc agacgtctga taatccagtc tttaatgaat taacgagata tatgcgaatt 97740
   aaaaatacgg ttaacgattg gaaatcatta acggatagca aaacaaaatt agaaagtgat 97800
   agaggtagac ttctagccgc tggtaaggat gatatatttg aattcaaatg tgtggatttc 97860
   ggcgcctatt ttatagctat gcgattggat aagaaaacat atctgccgca agctattagg 97920
   cgaggtactg gagacgcgtg gatggttaaa aaggcggcaa aggtcgatcc atctgctcaa 97980
   caattttgtc agtatttgat aaaacacaag tctaataatg ttattacttg tggtaatgag 98040
   atgttaaatg aattaggtta tagcggttat tttatgtcac cgcattggtg ttccgatttt 98100
   agtaatatgg aatagtgtta gataaatgcg gtaacgaatg ttcctgtaag gaaccataac 98160
   agcttagatt taacgttaaa gatgagcata aacataataa acaaaattac aatcaaactt 98220
   ataacattaa tatcaaacaa tccaaaaaat gaaatcagtg gagtagtaaa cgcgtacata 98280
   actcctggat aacgtttagc agctgccgtt cctattctag accaaaaatt cggtttcatg 98340
   ttttcgaaac ggtattctgc aacaagtcga ggatcgtgtt ctacatattt ggcggcatta 98400
   tccagtatct gcctattgat cttcatttcg ttttcgattc tggctatttc aaaataaaat 98460
   cccgatgata gacctccaga ctttataatt tcatctacga tgttcagcgc cgtagtaact 98520
   ctaataatat aggctgataa gctaacatca taccctcctg tatatgtgaa tatggcatga 98580
   tttttgtcca ttacaagctc ggttttaact ttattgcctg taataatttc tctcatctgt 98640
   aggatatcta tttttttgtc atgcattgcc ttcaagacgg gacgaagaaa cgtaatatcc 98700
   tcaataacgt tatcgttttc tacaataact acatattcta cctttttatt ttctaactcg 98760
   gtaaaaaaat tagaatccca tagggctaaa tgtctagcga tatttctttt cgtttcctct 98820
   gtacacatag tgttacaaaa ccctgaaaag aagtgagtat acttgtcatc atttctaatg 98880
   tttcctccag tccactgtat aaacgcataa tccttgtaat gatctggatc atccttgact 98940
   accacaacat ttcttttttc tggcataact tcgttgtcct ttacatcatc gaacttctga 99000
   tcattaatat gctcatgaac attaggaaat gtttctgatg gaggtctatc aataactggc 99060
   acaacaataa caggagtttt caccgccgcc atttagttat tgaaattaat catatacaac 99120
   tctttaatac gagttatatt ttcgtctatc cattgtttca cattgacata tttcgacaaa 99180
   aagatataaa atgcgtattc caatgcttct ctgtttaatg aattactaaa atatacaaac 99240
   acgtcactgt ctggcaataa atgatatctt agaatattgt aacaatttat tttgtattgc 99300
   acatgttcgt gatctatgag ttcttcttcg aatggcatag gatctccgaa tctgaaaacg 99360
   tataaatagg agttagaata ataatatttg agagtattgg taatatataa actctttagc 99420
   ggtataatta gtttttttct ctcaatttct atttttagat gtgatggaaa aatgactaat 99480
   tttgtagcat tagtatcatg aactctaatc aaaatcttaa tatcttcgtc acacgttagc 99540
   tctttgaagt ttttaagaga tgcatcagtt ggttctacag atggagtagg tgcaacaatt 99600
   ttttgttcta cacatgtatg tactggagcc attgttttaa ctataatggt gcttgtatcg 99660
   aaaaacttta atgcagatag cggaagctct tcgccgcgac tttctacgtc gtaattgggt 99720
   tctaacgccg atctctgaat ggatactagt tttctaagtt ctaatgtgat tctctgaaaa 99780
   tgtaaatcca attcctccgg cattatagat gtgtatacat cggtaaataa aactatagta 99840
   tccaacgatc ccttctcgca aattctagtc ttaaccaaaa aatcgtatat aaccacggag 99900
   atggcgtatt taagagtgga ttcttctacc gttttgttct tggatgtcat ataggaaact 99960
   ataaagtccg cactactgtt aagaatgatt actaacgcaa ctatatagtt taaattaagc 100020
   attttggaaa cataaaataa ctctgtagac gatacttgac tttcgaataa gtttgcagac 100080
   aaacgaagaa agaacagacc tctcttaatt tcagaagaaa actttttttc gtattcctga 100140
   cgtctagagt ttatatcaat aagaaagtta agaattagtc ggttaatgtt gtatttcatt 100200
   acccaagttt gagatttcat aatattatca aaagacatga taatattaaa gataaagcgc 100260
   tgactatgaa cgaaatagct atatggttcg ctcaagaata tagtcttgtt aaacgtggaa 100320
   acgataactg tatttttaat cacgtcagcg gcatctaaat taaatatagg tatatttatt 100380
   ccacacactc tacaatatgc cacaccatct tcataataaa taaattcgtt agcaaaatta 100440
   ttaattttag tgaaatagtt agcgtcaact ttcatagctt ccttcaatct aatttgatgc 100500
   tcacacggtg cgaattccac tctaacatcc cttttccatg cctcaggttc atcgatctct 100560
   ataatatcta gttttttgcg tttcacaaac acaggctcgt ctctcgcgat gagatctgta 100620
   tagtaactat gtaaatgata actagataga aagatgtagc tatatagatg acgatccttt 100680
   aagagaggta taataacttt accccaatca gatagactgt tgttatggtc ttcggaaaaa 100740
   gaatttttat aaatttttcc agtattttcc aaatatacgt acttaacatc taaaaaatcc 100800
   ttaatgataa taggaatgga taatccgtct attttataaa gaaatacata tcgcacatta 100860
   tacttttttt tggaaatggg aataccgatg tgtctacata aatatgcaaa gtctaaatat 100920
   tttttagaga atcttagttg gtccaaattc ttttccaagt acggtaatag atttttcata 100980
   ttgaacggta tcttcttaat ctctggttct agttccgcat taaatgatga aactaagtca 101040
   ctatttttat aactaacgat tacatcacct ctaacatcat catttaccag aatactgatc 101100
   ttcttttgtc gtaaatacat gtctaatgtg ttaaaaaaaa gatcatacaa gttatacgtc 101160
   atttcatctg tggtattctt gtcattgaag gataaactcg tactaatctc ttctttaaca 101220
   gcctgttcaa atttatatcc tatatacgaa aaaatagcaa ccagtgtttg atcatccgcg 101280
   tcaatattct gttctatcgt agtgtataac aatcgtatat cttcttctgt gatagtcgat 101340
   acgttataaa ggttgataac gaaaatattt ttatttcgtg aaataaagtc atcgtaggat 101400
   tttggactta tattcgcgtc tagtagatat gcttttattt ttggaatgat ctcaattaga 101460
   atagtctctt tagagtccat ttaaagttac aaacaactag gaaattggtt tatgatgtat 101520
   aattttttta gtttttatag attctttatt ctatacttaa aaaatgaaaa taaatacaaa 101580
   ggttcttgag ggttgtgtta aattgaaagc gagaaataat cataaattat ttcattatcg 101640
   cgatatccgt taagtttgta tcgtaatggc gtggtcaatt acgaataaag cggatactag 101700
   tagcttcaca aagatggctg aaatcagagc tcatctaaaa aatagcgctg aaaataaaga 101760
   taaaaacgag gatattttcc cggaagatgt aataattcca tctactaagc ccaaaaccaa 101820
   acgagccact actcctcgta aaccagcggc tactaaaaga tcaaccaaaa aggaggaagt 101880
   ggaagaagaa gtagttatag aggaatatca tcaaacaact gaaaaaaatt ctccatctcc 101940
   tggagtcagc gacattgtag aaagcgtggc cgctgtagag ctcgatgata gcgacgggga 102000
   tgatgaacct atggtacaag ttgaagctgg taaagtaaat catagtgcta gaagcgatct 102060
   ctctgaccta aaggtggcta ccgacaatat cgttaaagat cttaagaaaa ttattactag 102120
   aatctctgca gtatcgacgg ttctagagga tgttcaagca gctggtatct ctagacaatt 102180
   tacttctatg actaaagcta ttacaacact atctgatcta gtcaccgagg gaaaatctaa 102240
   agttgttcgt aaaaaagtta aaacttgtaa gaagtaaatg cgtgcacttt tttataaaga 102300
   tggtaaactc tttaccgata ataatttttt aaatcctgta tcagacgata atccagcgta 102360
   tgaggttttg caacatgtta aaattcctac tcatttaaca gatgtagtag tatatgaaca 102420
   aacgtgggag gaggcgttaa ctagattaat ttttgtggga agtgattcaa aaggacgtag 102480
   acaatacttt tacggaaaaa tgcatgtaca gaatcgcaac gctaaaagag atcgtatttt 102540
   tgttagagta tataacgtta tgaaacgaat taattgtttt ataaacaaaa atataaagaa 102600
   atcgtccaca gattccaatt atcagttggc ggtttttatg ttaatggaaa ctatgttttt 102660
   tattagattt ggtaaaatga aatatcttaa ggagaatgaa acagtagggt tattaacact 102720
   aaaaaataaa cacatagaaa taagtcccga tgaaatagtt atcaagtttg taggaaagga 102780
   caaagtttca catgaatttg ttgttcataa gtctaataga ctatataaac cgctattgaa 102840
   actgacggat gattctagtc ccgaagaatt tctgttcaac aaactaagtg aacgaaaggt 102900
   atacgaatgt atcaaacagt ttggtattag aatcaaggat ctccgaacgt atggagtcaa 102960
   ttatacgttt ttatataatt tttggacaaa tgtaaagtcc atatctcctc ttccgtcacc 103020
   aaaaaagtta atagcattaa ctatcaaaca aactgctgaa gtggtaggtc atactccatc 103080
   aatttcaaaa agagcttata tggcaacgac tattttagaa atggtaaagg ataaaaattt 103140
   tttagatgta gtatctaaaa ctacgttcga tgaattccta tctatagtcg tagatcacgt 103200
   taaatcatct acggatggat gatatagatc tttacacaaa taattacaag accgataaat 103260
   ggaaatggat aagcgtatga aatctctcgc aatgacagct ttcttcggag agctaagcac 103320
   attagatatt atggcattga taatgtctat atttaaacgc catccaaaca ataccatttt 103380
   ttcagtggat aaggatggtc agtttatgat tgatttcgaa tacgataatt ataaggcttc 103440
   tcaatatttg gatctgaccc tcactccgat atctggagat gaatgcaaga ctcacgcatc 103500
   gagtatagcc gaacaattgg cgtgtgcgga tattattaaa gaggatatta gcgaatatat 103560
   caaaactact ccccgtctta aacgatttat aaaaaaatac cgcaatagat cagatactcg 103620
   tatcagtcga gatacagaaa agcttaaaat agctctagct aaaggcatag attacgaata 103680
   tataaaagac gcttgttaat aagtaaatga aaaaaaacta gtcgtttata ataaaacacg 103740
   atatggatgc caacgtagta tcattttcta ctattgcgac gtatatagac gctttagcga 103800
   agaatgcttc ggaattagaa cagaggtcta ccgcatacga aataaataat gaattggaac 103860
   tagtatttat taagccgcca ttgattactt tgacaaatgt agtgaatatc tctacgattc 103920
   aggaatcgtt tattcgattt accgttacta ataaggaagg tgttaaaatt agaactaaga 103980
   ttccattatc taaggtacat ggtctagatg taaaaaatgt acagttagta gatgctatag 104040
   ataacatagt ttgggaaaag aaatcattag tgacggaaaa tcgtcttcac aaagaatgct 104100
   tgttgagact atcgacagag gaacgtcata tatttttgga ttacaagaaa tatggatcct 104160
   ctatccgact agaattagtc aatcttattc aagcaaaaac aaaaaacttt acgatagact 104220
   ttaagctaaa atattttcta ggatccggtg cccagtctaa aagttctttg ttgcacgcta 104280
   ttaatcatcc aaagtcaagg cctaatacat ctctggaaat agaattcaca cctagagaca 104340
   atgaaacagt tccatatgat gaactaataa aggaattgac gactctctcg cgtcatatat 104400
   ttatggcttc tccagagaat gtaattcttt ctccgcctat taacgcgcct ataaaaacct 104460
   ttatgttgcc taaacaagat atagtaggtt tggatctgga aaatctatat gccgtaacta 104520
   agactgacgg cattcctata actatcagag ttacatcaaa cgggttgtat tgttatttta 104580
   cacatcttgg ttatattatt agatatcctg ttaagagaat aatagattcc gaagtagtag 104640
   tctttggtga ggcagttaag gataagaact ggaccgtata tctcattaag ctaatagagc 104700
   ctgtgaatgc aatcaatgat agactagaag aaagtaagta tgttgaatct aaactagtgg 104760
   atatttgtga tcggatagta ttcaagtcaa agaaatacga aggtccgttt actacaacta 104820
   gtgaagtcgt cgatatgtta tctacatatt taccaaagca accagaaggt gttattctgt 104880
   tctattcaaa gggacctaaa tctaacattg attttaaaat taaaaaggaa aatactatag 104940
   accaaactgc aaatgtagta tttaggtaca tgtccagtga accaattatc tttggagaat 105000
   cgtctatctt tgtagagtat aagaaattta gcaacgataa aggctttcct aaagaatatg 105060
   gttctggtaa gattgtgtta tataacggcg ttaattatct aaataatatc tattgtttgg 105120
   aatatattaa tacacataat gaagtgggta ttaagtccgt ggttgtacct attaagttta 105180
   tagcagaatt cttagttaat ggagaaatac ttaaacctag aattgataaa accatgaaat 105240
   atattaactc agaagattat tatggaaatc aacataatat catagtcgaa catttaagag 105300
   atcaaagcat caaaatagga gatatcttta acgaggataa actatcggat gtgggacatc 105360
   aatacgccaa taatgataaa tttagattaa atccagaagt tagttatttt acgaataaac 105420
   gaactagagg accgttggga attttatcaa actacgtcaa gactcttctt atttctatgt 105480
   attgttccaa aacattttta gacgattcca acaaacgaaa ggtattggcg attgattttg 105540
   gaaacggtgc tgacctggaa aaatactttt atggagagat tgcgttattg gtagcgacgg 105600
   atccggatgc tgatgctata gctagaggaa atgaaagata caacaaatta aactctggaa 105660
   ttaaaaccaa gtactacaaa tttgactaca ttcaggaaac tattcgatcc gatacatttg 105720
   tctctagtgt cagagaagta ttctattttg gaaagtttaa tatcatcgac tggcagtttg 105780
   ctatccatta ttcttttcat ccgagacatt atgctaccgt catgaataac ttatccgaac 105840
   taactgcttc tggaggcaag gtattaatca ctaccatgga cggagacaaa ttatcaaaat 105900
   taacagataa aaagactttt ataattcata agaatttacc tagtagcgaa aactatatgt 105960
   ctgtagaaaa aatagctgat gatagaatag tggtatataa tccatcaaca atgtctactc 106020
   caatgactga atacattatc aaaaagaacg atatagtcag agtgtttaac gaatacggat 106080
   ttgttcttgt agataacgtt gatttcgcta caattataga acgaagtaaa aagtttatta 106140
   atggcgcatc tacaatggaa gatagaccgt ctacaaaaaa ctttttcgaa ctaaatagag 106200
   gagccattaa atgtgaaggt ttagatgtcg aagacttact tagttactat gttgtttatg 106260
   tcttttctaa gcggtaaata ataatatggt atgggttctg atctcccagt tctaaatgca 106320
   ttaaataatt ccaatagagc gatttttgtt cctataggac cttccaactg tggatactct 106380
   gtattgttaa tagatatatt aatacttttg tcgggtaaca gaggttctac gtcttctaaa 106440
   aataaaagtt ttataacatc tggcctgttc ataaataaaa acttggcgat tctatatata 106500
   ctcttattat caaatctagc cattgtctta tagatgtgag ctactgtagg tgtaccattt 106560
   gattttcttt ctaatactat atatttctct cgaagaagtt cttgcacatc atctgggaat 106620
   aaaatactac tgttgagtaa atcagttatt ttttttatat cgatattgat ggacattttt 106680
   atagttaagg ataataagta tcccaaagtc gataacgacg ataacgaagt atttatactt 106740
   ttaggaaatc acaatgactt tatcagatta aaattaacaa aattaaagga gcatgtattt 106800
   ttttctgaat atattgtgac tccagataca tatggatctt tatgcgtcga attaaatggg 106860
   tctagttttc agcacggcgg tagatatata gaggtggagg aatttataga tgctggaaga 106920
   caagttagat ggtgttctac atccaatcat atatctaaag atatacccga agatatgcac 106980
   actgataaat ttgtcattta tgatatttat acgtttgatt cgttcaagaa taaacgattg 107040
   gtattcgtac aggtacctcc gtcgttagga gatgatagtc atttgactaa tccgttattg 107100
   tcaccgtatt atcgtaattc agtagccaga caaatggtca atgatatgat ttttaatcaa 107160
   gattcatttt taaaatattt attagaacat ctgattagaa gccactatag agtttctaaa 107220
   catataacaa tagttagata caaggatacc gaagaattaa atctaacgag aatatgttat 107280
   aatagagata agtttaaggc gtttgtattc gcttggttta acggcgtttc ggaaaatgaa 107340
   aaggtactag atacgtataa aaaggtatct aatttgatat aatgaattca gtgactgtat 107400
   cacacgcgcc atatactatt acttatcacg atgattggga accagtaatg agtcaattgg 107460
   tagagtttta taacgaagta gccagttggc tgctacgaga cgagacgtcg cctattcctg 107520
   ataagttctt tatacagttg aaacaaccgc ttagaaataa acgagtatgt gtgtgtggta 107580
   tagatccgta tccgaaagat ggaactggtg taccgttcga atcaccaaat tttacaaaaa 107640
   aatcaattaa ggagatagct tcatctatat ctagattaac cggagtaatt gattataaag 107700
   gttataacct taatataata gacggggtta taccctggaa ttattactta agttgtaaat 107760
   taggagaaac aaaaagtcac gcgatttact gggataagat ttccaagtta ctgctgcagc 107820
   atataactaa acacgttagt gttctttatt gtttgggtaa aacagatttc tcgaatatac 107880
   gggccaagtt agaatccccg gtaactacca tagtcggata tcatccagcg gctagagacc 107940
   gccaattcga gaaagataga tcatttgaaa ttatcaacgt tttactggaa ttagacaaca 108000
   aggcacctat aaattgggct caagggttta tttattaatg ctttagtgaa attttaactt 108060
   gtgttctaaa tggatgcggc tattagaggt aatgatgtta tctttgttct taagactata 108120
   ggtgtcccgt cagcgtgcag acaaaatgaa gatccaagat ttgtagaagc atttaaatgc 108180
   gacgagttag aaagatatat tgagaataat ccagaatgta cactattcga aagtcttagg 108240
   gatgaggaag catactctat agtcagaatt ttcatggatg tagatttaga cgcgtgtcta 108300
   gacgaaatag attatttaac ggccattcaa gattttatta tcgaggtgtc aaactgtgta 108360
   gctagattcg cgtttacaga atgcggcgcc attcatgaaa atgtaataaa atccatgaga 108420
   tctaattttt cattgactaa gtctacaaat agagataaaa caagttttca tattatcttt 108480
   ttagatacgt ataccactat ggatacattg atagctatga aacgaacact attagaatta 108540
   agtagatcat ctgaaaatcc actaaccaga tcgatagaca ctgccgtata taggagaaaa 108600
   acaactcttc gggttgtagg tactaggaaa aatccaaatt gcgacactat tcatgtaatg 108660
   caaccaccgc atgataatat agaagattac ctattcactt acgtggatat gaacaacaat 108720
   agttattact tttctctaca acaacgattg gaggatttag ttcctgataa gttatgggaa 108780
   ccagggttta tttcattcga agacgctata aaaagagttt caaaaatatt cattaattct 108840
   ataataaact ttaatgatct cgatgaaaat aattttacaa cggtaccact ggtcatagat 108900
   tacgtaacac cttgtgcatt atgtaaaaaa cgatcgcata aacatccgca tcaactatcg 108960
   ttggaaaatg gtgctattag aatttacaaa actggtaatc cacatagttg taaagttaaa 109020
   attgttccgt tggatggtaa taaactgttt aatattgcac aaagaatttt agacactaac 109080
   tctgttttat taaccgaacg aggagaccat atagtttgga ttaataattc atggaaattt 109140
   aacagcgaag aacccttgat aacaaaacta attttgtcaa taagacatca actacctaag 109200
   gaatattcaa gcgaattact ctgtccaaga aaacgaaaga ctgtagaagc taacatacga 109260
   gacatgttag tagattcagt ggagaccgat acctatccgg ataaacttcc gtttaaaaat 109320
   ggtgtattgg acctggtaga cggaatgttt tactctggag atgatgctaa aaaatatacg 109380
   tgtactgtat caaccggatt taaatttgac gatacaaagt tcgtcgaaga cagtccagaa 109440
   atggaagagt taatgaatat cattaacgat atccaaccat taacggatga aaataagaaa 109500
   aatagagagc tatatgaaaa aacattatct agttgtttat gcggtgctac caaaggatgt 109560
   ttaacattct tttttggaga aactgcaact ggaaagtcga caaccaaacg tttgttaaag 109620
   tctgctatcg gtgacctgtt tgttgagacg ggtcaaacaa ttttaacaga tgtattggat 109680
   aaaggaccta atccatttat cgctaacatg catttgaaaa gatctgtatt ctgtagcgaa 109740
   ctacctgatt ttgcctgtag tggatcaaag aaaattagat ctgacaatat taaaaagttg 109800
   acagaacctt gtgtcattgg aagaccgtgt ttctccaata aaattaataa tagaaaccat 109860
   gcgacaatca ttatcgatac taattacaaa cctgtttttg ataggataga taacgcatta 109920
   atgagaagaa ttgccgtcgt gcgattcaga acacactttt ctcaaccttc tggtagagag 109980
   gctgctgaaa ataatgacgc gtacgataaa gtcaaactat tagacgaggg gttagatggt 110040
   aaaatacaaa ataatatata tagatttgca tttctatact tgttggtgaa atggtacaaa 110100
   aaatatcatg ttcctattat gaaactatat cctacacccg aagagattcc tgactttgca 110160
   ttctatctca aaataggtac tctgttggta tctagctctg taaagcatat tccattaatg 110220
   acggacctct ccaaaaaggg atatatattg tacgataatg tggtcactct tccgttgact 110280
   actttccaac agaaaatatc caagtatttt aattctagac tatttggaca cgatatagag 110340
   agcttcatca atagacataa gaaatttgcc aatgttagtg atgaatatct gcaatatata 110400
   ttcatagagg atatttcatc tccgtaaata tatgctcata tatttataga agatatcaca 110460
   tatctaaatg aataccggaa tcatagattt atttgataat catgttgata gtataccaac 110520
   tatattacct catcagttag ctactctaga ttatctagtt agaactatca tagatgagaa 110580
   cagaagcgtg ttattgttcc atattatggg atcaggtaaa acaataatcg ctttgttgtt 110640
   cgccttggta gcttccagat ttaaaaaggt ttacattcta gtgccgaaca tcaacatctt 110700
   aaaaattttc aattataata tgggtgtagc tatgaacttg tttaatgacg aattcatagc 110760
   tgagaatatc tttattcatt ccacaacaag tttttattct cttaattata acgataacgt 110820
   cattaattat aacggattat ctcgctacaa taactctatt tttatcgttg atgaggcaca 110880
   taatatcttt gggaataata ctggagaact tatgaccgtg ataaaaaata aaaacaagat 110940
   tcctttttta ctattgtctg gatctcccat tactaacaca cctaatactc tgggtcatat 111000
   tatagattta atgtccgaag agacgataga ttttggtgaa attattagtc gtggtaagaa 111060
   agtaattcag acacttctta acgaacgcgg tgtgaatgta cttaaggatt tgcttaaagg 111120
   aagaatatca tattacgaaa tgcctgataa agatctacca acgataagat atcacggacg 111180
   taagtttcta gatactagag tagtatattg tcacatgtct aaacttcaag agagagatta 111240
   tatgattact agacgacagc tatgttatca tgaaatgttt gataaaaata tgtataacgt 111300
   gtcaatggca gtattgggac aacttaatct gatgaataat ttagatactt tatttcagga 111360
   acaggataag gaattgtacc caaatctgaa aataaataat ggcgtgttat acggagaaga 111420
   attggtaacg ttaaacatta gttccaaatt taaatacttt attaatcgga tacagacact 111480
   caacggaaaa cattttatat acttttctaa ttctacatat ggcggattgg taattaaata 111540
   tatcatgctc agtaatggat attctgaata taatggttct cagggaacta atccacatat 111600
   gataaacggc aaaccaaaaa catttgctat cgttactagt aaaatgaaat cgtctttaga 111660
   ggatctatta gatgtgtata attctcctga aaacgatgat ggtagtcaat tgatgttttt 111720
   gttttcatca aacattatgt ccgaatccta tactctaaaa gaggtaaggc atatttggtt 111780
   tatgactatc ccagatactt tttctcaata caaccaaatt cttggacgat ctattagaaa 111840
   attctcttac gccgatattt ctgaaccagt taatgtatat cttttagccg ccgtatattc 111900
   cgatttcaat gacgaagtga cgtcattaaa cgattacaca caggatgaat taattaatgt 111960
   tttaccattt gacatcaaaa agctgttgta tctaaaattt aagacgaaag aaacgaatag 112020
   aatatactct attcttcaag agatgtctga aacgtattct cttccaccac atccatcaat 112080
   tgtaaaagtt ttattgggag aattggtcag acaatttttt tataataatt ctcgtattaa 112140
   gtataacgat accaagttac ttaaaatggt tacatcagtt ataaaaaata aagaagacgc 112200
   taggaattac atagatgata ttgtaaacgg tcacttcttt gtatcgaata aagtatttga 112260
   taaatctctt ttatacaaat acgaaaacga tattattaca gtaccgttta gactttccta 112320
   cgaaccattt gtttggggag ttaactttcg taaagaatat aacgtggtat cttctccata 112380
   aaactgatga gatatataaa gaaataaatg tcgagctttg ttaccaatgg atacctttcc 112440
   gttacattgg aaccacacga gctgacgtta gacataaaaa ctaatattag gaatgccgta 112500
   tataagacgt atctccatag agaaattagt ggtaaaatgg ccaagaaaat agaaattcgt 112560
   gaagacgtgg aattacctct cggcgaaata gttaataatt ctgtagttat aaacgttccg 112620
   tgtgtaataa cctacgcgta ttatcacgtt ggggatatag tcagaggaac attaaacatc 112680
   gaagatgaat caaatgtaac tattcaatgt ggagatttaa tctgtaaact aagtagagat 112740
   tcgggtactg tatcatttag cgattcaaag tactgctttt ttcgaaatgg taatgcgtat 112800
   gacaatggca gcgaagtcac tgccgttcta atggaggctc aacaaggtat cgaatctagt 112860
   tttgtttttc tcgcgaatat cgtcgactca taaaaaagag aatagcggta agtataaaca 112920
   cgaatactat ggcaataatt gcgaatgttt tattctcttc gatatatttt tgataatatg 112980
   aaaaacatgt ctctctcaaa tcggacaacc atctcataaa atagttctcg cgcgctggag 113040
   aggtagttgc cgctcgtata atctctccag aataatatac ttgcgtgtcg tcgttcaatt 113100
   tatacggatt tctatagttc tctgttatat aatgcggttt gccctcatga ttagacgacg 113160
   acaatagtgt tctaaattta gatagttgat cagaatgaat gtttattggc gttggaaaaa 113220
   ttatccatac agcgtctgca gagtggttga tagttgttcc tagatatgta aaataatcca 113280
   acttactagg cagcaaattg tctagataaa atactgaatc aaacggtgca gacgtattgg 113340
   cggatctaat ggaatccaat tgattaacta tcttttgaaa atatacattt ttatgatcca 113400
   atacttgtaa gaatatagaa ataatgataa gtccatcatc gtgttttttt gcctcttcat 113460
   aagaactata ttttttctta ttccaatgaa caagattaat ctctccagag tatttgtaca 113520
   catctatcaa gtgattggat ccataatcgt cttcctttcc ccaatatata tgtagtgatg 113580
   ataacacata ttcattgggg agaaaccctc cacttatata tcctccttta aaattaatcc 113640
   ttactagttt tccagtgttc tggatagtgg ttggtttcga ctcattataa tgtatgtcta 113700
   acggcttcaa tcgcgcgtta gaaattgctt ttttagtttc tatattaata ggagatagtt 113760
   gttgcggcat agtaaaaatg aaatgataac tgtttaaaaa tagctcttag tatgggaatt 113820
   acaatggatg aggaagtgat atttgaaact cctagagaat taatatctat taaacgaata 113880
   aaagatattc caagatcaaa agacacgcat gtgtttgctg cgtgtataac aagtgacgga 113940
   tatccgttaa taggagctag aagaacttca ttcgcgttcc aggcgatatt atctcaacaa 114000
   aattcagatt ctatctttag agtatccact aaactattac ggtttatgta ctacaatgaa 114060
   ctaagagaaa tctttagacg gttgagaaaa ggttctatca acaatatcga tcctcacttt 114120
   gaagagttaa tattattggg tggtaaacta gataaaaagg aatctattaa agattgttta 114180
   agaagagaat taaaagagga aagtgatgaa cgtataacag taaaagaatt tggaaatgta 114240
   attctaaaac ttacaacacg ggataaatta tttaataaag tatatataag ttattgcatg 114300
   gcgtgtttta ttaatcaatc gttggaggat ttatcgcata ctagtattta caatgtagaa 114360
   attagaaaga ttaaatcatt aaatgattgt attaacgacg ataaatacga atatctgtct 114420
   tatatttata atatgctagt taatagtaaa tgaactttta cagatctagt ataattagtc 114480
   agattattaa gtataataga cgactagcta agtctattat ttgcgaggat gactctcaaa 114540
   ttattacact cacggcattc gttaaccaat gcctatggtg tcataaacga gtatccgtgt 114600
   ccgctatttt attaactact gataacaaaa tattagtatg taacagacga gatagttttc 114660
   tctattctga aataattaga actagaaaca tgtttagaaa gaaacgatta tttctgaatt 114720
   attccaatta tttgaacaaa caggaaagaa gtatactatc gtcatttttt tctctagatc 114780
   cagctactac tgataatgat agaatagacg ctatttatcc gggtggcata cccaaaaggg 114840
   gtgagaatgt tccagagtgt ttatccaggg aaattaaaga agaagttaat atagacaatt 114900
   cttttgtatt catagacact cggtttttta ttcatggcat catagaagat accattatta 114960
   ataaattttt tgaggtaatc ttctttgtcg gaagaatatc tctaacgagt gatcaaatca 115020
   ttgatacatt taaaagtaat catgaaatca aggatctaat atttttagat ccgaattcag 115080
   gtaatggact ccaatacgaa attgcaaaat atgctctaga tactgcaaaa cttaaatgtt 115140
   acggccatag aggatgttat tatgaatcat taaaaaaatt aactgaggat gattgattaa 115200
   aaaatataaa ttaatttacc atcgtgtatt tttataacgg gattgtccgg catatcatgt 115260
   agatagttac cgtctacatc gtatactcga ccatctacgc ctttaaatcc tctatttatt 115320
   gacattaatc tattagaatt ggaataccaa atattagtac cctcaattag tttattggta 115380
   atattttttt tagacgatag atcgatggct cttgaaacca aggttttcca accggactca 115440
   ttgtcgatcg gtgagaagtc tttttcatta gcatgaatcc attctaatga tgtatgttta 115500
   aacactctaa acaattggac aaattctttt gatttgcttt gaatgatttc aaataggtct 115560
   tcgtctacag taggcatacc attagataat ctagccatta taaagtgcac gtttacatat 115620
   ctacgttctg gaggagtaag aacgtgacta ttgagacgaa tggctcttcc tactatctga 115680
   cgaagagacg cctcgttcca tgtcatatct aaaatgaaga tatcattaat tgagaaaaaa 115740
   ctaataccct cgcctccact agaagagaat acgcatgttt taatgcattc tccgttagtg 115800
   tttgattctt ggttaaactc agccaccgcc ttgattctag tatcttttgt tctagatgag 115860
   aactctatat tagagatacc aaagactttg aaatatagta ataagatttc tattcctgac 115920
   tgattaacaa atggttcaaa gactagacat ttaccatggg atgctaatat tcccaaacat 115980
   acatctataa atttgacgct tttctctttt aattcagtaa atagagagat atcagccgca 116040
   ctagcatccc ctttcaatag ttctcccttt ttaaaggtat ctaatgcgga tttagaaaac 116100
   tctctatctc ttaatgaatt tttaaaatca ttatatagtg ttgctatctc ttgcgcgtat 116160
   tcgcccggat cacgattttg tctttcagga aagctatcga acgtaaacgt agtagccata 116220
   cgtctcagaa ttctaaatga tgatatacct gtttttattt cagcgagttt agccttttga 116280
   taaatttctt cttgcttttt cgacatatta acgtatcgca ttaatactgt tttcttagcg 116340
   aatgatgcag acccttctac gtcatcaaaa atagaaaact cgttattaac tatatacgaa 116400
   catagtcctc ctagtttgga gactaattct ttttcatcga ctagacgttt attctcaaat 116460
   agcgattggt gttgtaagga tcctggtcgt agtaagttaa ccaacatggt gaattcttgc 116520
   acactattga cgataggtgt agccgataaa caaatcatct tatggttttt taacgcaatg 116580
   gtcttagata aaaaattata tactgaacga gtaggacgga tcttaccatc ttctttgatt 116640
   aatgatttag aaatgaagtt atgacattca tcaataatga cgcatattct actcttggaa 116700
   ttaatagttt tgatattagt aaaaaattta tttctaaaat tttgatcatc gtaattaata 116760
   aaaatacaat ccttcgttat ctctggagcg tatctgagta tagtgttcat ccaaggatct 116820
   tctatcaaag cctttttcac caataagata atagcccaat tcgtataaat atccttaaga 116880
   tgtttgagaa tatatacagt agtcattgtt ttaccgacac ccgtttcatg gaacaataaa 116940
   agagaatgca tactgtctaa tcctaagaaa actcttgcta caaaatgttg ataatccttg 117000
   aggcgtacta cgtccgaccc catcatttca acgggcatat tagtagttct gcgtaaggca 117060
   taatcgatat aggccgcgtg tgatttactc atttatgagt gataagtaat aactatgttt 117120
   taaaaatcac agcagtagtt taactagtct tctctgatgt ttgttttcga tactttttga 117180
   atcagaagtc atactagaat aaagcaacga gtgaacgtaa tagagagctt cgtatactct 117240
   attcgaaaac tctaagaact tattaatgaa ttccgtatcc actggattgt ttaaaatact 117300
   aaattgaaca ctgttcacat ccttccaaga agaagactta gtgacggact taacatgaga 117360
   cataaataaa tccaaatttt ttttacaaac atcactagcc accataatgg cgctatcttt 117420
   caaccagcta tcgcttacgc attttagcag tctaacattt ttaaagagac tacaatatat 117480
   tctcatagta tcgattacac ctctaccgaa taaagttgga agtttaataa tacaatattt 117540
   ttcgtttaca aaatcaaata atggtcgaaa cacgtcgaag gttaacatct tataatcgct 117600
   aatgtataga ttgttttcag tgagatgatt attagattta atagcatctc gttcacgttt 117660
   gaacagttta ttgcgtgcgc tgaggtcggc aactacggcg tccgctttag tactcctccc 117720
   ataatacttt acgctattaa tctttaaaat ttcatagact ttatctagat cgctttctgg 117780
   taacatgata tcatgtgtaa aaagttttaa catgtcggtc ggcattctat ttagatcatt 117840
   aactctagaa atctgaagaa agtaattagc tccgtattcc agactaggta atgggctttt 117900
   acctagagac agattaagtt ctggcaatgt ttcataaaat ggaagaagga catgcgttcc 117960
   ctcccggata ttttttacaa tttcatccat ttacaactct atagtttgtt ttcattatta 118020
   ttagttatta tctcccataa tcttggtaat acttacccct tgatcgtaag ataccttata 118080
   caggtcatta catacaacta ccaattgttt ttgtacataa tagattggat ggttgacatc 118140
   catggtggaa taaactactc gaacagatag tttatctttc cccctagata cattagccgt 118200
   aatagttgtc ggcctaaaga atatctttgg tgtaaagtta aaagttaggg ttcttgttcc 118260
   attattgctt tttgtcagta gttcattata aattctcgag atgggtccgt tctctgaata 118320
   tagaacatca tttccaaatc taacttctag tctagaaata atatcggtct tattcttaaa 118380
   atctattccc ttgatgaagg gatcgttaat gaacaaatcc ttggcctttg attcggctga 118440
   tctattatct ccgttataga cgttacgttg actagtccaa agacttacag gaatagatgt 118500
   atcgatgatg ttgatactat gtgatatgtg agcaaagatt gttctcttag tggcatcact 118560
   atatgttcca gtaatggcgg aaaacttttt agaaatgtta tatataaaag aattttttcg 118620
   tgttccaaac attagcagat tagtatgaag ataaacactc atattatcag gaacattatc 118680
   aatttttaca tacacatcag catcttgaat agaaacgata ccatcttctg gaacctctac 118740
   gatctcggca gactccggat aaccagtcgg tgggccatcg ctaacaataa ctagatcatc 118800
   caacaatcta ctcacatatg catctatata atctttttca tcttgtgagt accctggata 118860
   cgaaataaat ttattatccg tatttccata ataaggttta gtataaacag agagagatgt 118920
   tgccgcatga acttcagtta cagtcgccgt tggttggttt atttgaccta ttactctcct 118980
   aggtttctct ataaacgatg gtttaatttg tacattctta accatatatc caataaagct 119040
   caattcagga acataaacaa attctttgtt gaacgtttca aagtcgaacg aagagtcacg 119100
   aataacgata tcggatactg gattgaaggt taccgttacg gtaatttttg aatcggatag 119160
   tttaagactg ctgaatgtat cttccacatc aaacggagtt ttaatataaa cgtatactgt 119220
   agatggttct ttaatagtgt cattaggagt taggccaata gaaatatcat taagttcact 119280
   agaatatcca gagtgtttca aagcaattgt attattgata caattattat ataattcttc 119340
   gccctcaatt tcccaaataa caccgttaca cgaagagata gatacgtgat taatacattt 119400
   atatccaaca tatggtacgt aactgaatct tcccatacct ttaacttctg gaagttccaa 119460
   actcagaacc aaatgattaa gcgcagtaat atactgatcc ctaatttcga agctagcgat 119520
   agcctgattg tctggaccat cgtttgtcat aactccggat agagaaatat attgcggcat 119580
   atacaaagtt ggaatttgac tatcgactgc gaagacatta gaccgtttaa tagagtcatc 119640
   cccaccgatc aaagaattaa tgatagtatt attcattttc tatttaaaat ggaaaaagct 119700
   tacaataaac tccgtagaga aatatctata atttgtgagt tttccttaaa gtaacagctt 119760
   ccgtaaacgc cgtctttatc tcttagtagg tttattgtat ttatgacctt ttccttatct 119820
   tcatagaata ctaaaggcaa caaagaaatt tttggttctt ctctaagagc tacgtgagac 119880
   ttaaccatag aagccaacga atccctacat attttagaac agaaataccc tacttcacca 119940
   cccttgtatg tctcaatact aataggtcta aaaaccaaat cttgattaca aaaccaacac 120000
   ttatcaatta cactatttgt cttaatagac acatctgcca tagatttata atactttggt 120060
   agtatacaag cgagtgcttc ttctttagcg ggcttaaaga ctgctttagg tgctgaaata 120120
   accacatctg gaaggcttac tcgcttagcc atttaattac ggaactattt ttttatactt 120180
   ctaatgagca agtagaaaac ctctcatcta caaaaacgta ctcgtgtcca taatcctcta 120240
   ccatagttac acgtttttta gatctcatat gtgctaaaaa gttttcccat actaattggt 120300
   tactattatt tttcgtataa tttttaacag tttgaggttt tagattttta gttacagaag 120360
   tgatatcgaa tattttatcc aaaaagaatg aataattaat tgtcttagaa ggagtgtttt 120420
   cttggcaaaa gaataccaag tgcttaaata tttctactac ttcattaatc ttttctgtac 120480
   tcagattcag tttctcatct tttacttgat tgattatttc aaagactaac ttataatcct 120540
   ttttatttat tctctcgtta gccttaagaa aactagatac aaaatttgca tctacatcat 120600
   ccgtggatat ttgatttttt tccatgatat ccaagagttc cgagataatt tctccagaac 120660
   attgatgaga caataatctc cgcaatacat ttctcaaatg aataagttta ttagacacgt 120720
   ggaagtttga ctttttttgt acctttgtac atttttgaaa taccgactcg caaaaaatac 120780
   aatattcata tccttgttca gatactatac cgttatgtct acaaccgcta cataatcgta 120840
   gattcatgtt aacactctac gtatctcgtc gtccaatatt ttatataaaa acattttatt 120900
   tctagacgtt gccagaaaat cctgtaatat ttttagtttt ttgggctgtg aataaagtat 120960
   cgccctaata ttgttaccgt cttccgccaa tatagtagtt aaattatccg cacatgcaga 121020
   agaacaccgc ttaggcggat tcagtacaat gttatatttt tcgtaccaac tcatttaaat 121080
   atcataatct aaaatagttc tgtaatatgt ctagcgctaa tatattgatc ataatcctgt 121140
   gcataaatta agatacaaca atgtctcgaa atcatcgaca tggcttcttc catagttaga 121200
   agatcgtcgt caaagttagc aacgtgattc atcaacattt gctgttttga ggcagcaaat 121260
   actgaaccgt cgccattcaa ccattcataa aaaccatcgt ctgaatccat tgataatttc 121320
   ttgtactggt ttttgagagc tcgcatcaat ctagcatttc tagctcccgg attgaaaaca 121380
   gaaagaggat cgtacatcca gggtccattt tctgtaaata gaatcgtata atgtcccttc 121440
   aagaagatat cagacgatcc acaatcaaag aattggtctc cgagtttgta acaaactgcg 121500
   gactttaacc tatacatgat accgtttagc ataatttctg gtgatacgtc aatcggagta 121560
   tcatctatta gagatctaaa gccggtgtaa cattctccac caaacatatt cttattctga 121620
   cgtcgttcta cataaaacat cattgctcca ttaacgataa caggggaatg aacagcacta 121680
   cccatcacat tagttcccaa tggatcaatg tgtgtaactc cagaacatct tccatagcct 121740
   atgttaggag gagcgaacac cactcttcca ctattgccat cgaatgccat agaataaata 121800
   tccttggaat tgatagaaat cggactgtcg gatgttgtga tcatcttcat aggattaaca 121860
   actatgtatg gtgccgcctg aagtttcata tcgtaactga tgccgtttat aggtctagcc 121920
   acagaaacca acgtaggtct aaatccaact atagacaaaa tagaagccaa tatctgttcc 121980
   tcatctgtca taacttgaga gcatccagta tgaataatct tcattagatg gggatctacc 122040
   gcatcatcat cgttacaata aaaaattccc attctaatgt tcataattgc ttttctaatc 122100
   atggtatgca tgtttgctct ctgaatctct gtggaaatta gatctgatac acctgtaatc 122160
   actatcggat tatcctccgt aagacgatta accaacaaca tataattata agactttact 122220
   tttctaaatt cataaagttg ctggattagg ctataggtgt ctccatgtac atacgcgttc 122280
   tcgagcgcag gaagtttaat accgaatagt gccatcagaa taggatgaat atagtaatta 122340
   gtttctggtt ttctataaat aaaagacaaa tcttgtgaac tagacatatc ggtaaaatgc 122400
   atggattgga atcgtgtagt cgacagaaga atatgatgat tagatggaga gtatatttta 122460
   tctaactctt tgagttggtc accgattcta ggactagctc gagaatgaat aagtactaaa 122520
   ggatgagtac atttcacaga aacactagca ttgttcaatg tgctctttac atgggtaagg 122580
   agttgaaata gctcgtttct atttgttctg acaatattta gtttattcat aatgttaagc 122640
   atatcctgaa tagtaaagtt agatgtgtca tacttgttag tagttagata tttagcaatt 122700
   gcattcccat catttctcaa tctcgtactc caatcatgcg tggatgctac ttcgtcgatg 122760
   gaaaccatac aatccttttt gataggctgt tgagattgat catttcctgc acgtttaggt 122820
   ttggtacgtt gatttctagc ccctgcggat ataaagtcat cgtctacaat ttgggacaat 122880
   gaattgcata cactacaaga caaagattta tcagaagtgt gaatatgatc ttcatctacc 122940
   aaagaaagag tttgattagt ataactagat tttagtcctg cgttagatgt taaaaaaaca 123000
   tcgctattga ccacggcttc cattatttat attcgtagtt tttactcgaa agcgtgattt 123060
   taatatccaa tcttattact tttggaatcg ttcaaaacct ttgactagtt gtagaatttg 123120
   atctattgcc ctacgcgtat actcccttgc atcatatacg ttcgtcacca gatcgtttgt 123180
   ttcggcctga agttggtgca tatctctttc aacattcgac atgagatcct taagggccat 123240
   atcgtctaga ttttgttgag atgctgctcc tggatttgga ttttgttgtg ctgttgtaca 123300
   tactgtacca ccagtaggtg taggagtaca tacagtggcc acaataggag gttgagaaag 123360
   tgtaaccgtt ggagtagtac aagaaatact tccatccgat tgttgtgtac atgtagttgt 123420
   tggtaacgtc tgagaaggtt gggtagatgg cggcgtcgtc gttttttgat ctttattaaa 123480
   tttagagata atatcctgaa cagcattgct cggcgtcaac gctggaagga gtgaactcgc 123540
   cggcgcatca gtatcttcag acagccaatc aaaaagatta gacatatcag atgatgtatt 123600
   agtttgttgt cgtggttttg gtgtaggagc agtactacta ggtagaagaa taggagccga 123660
   tgtagctgtt ggaaccggct gtggagttat atgaatagtt ggttgtagcg gttggatagg 123720
   ctgtctgctg gcggccatca tattatctct agctagttgt tctcgcaact gtctttgata 123780
   atacgactct tgagacttta gtcctatttc aatcgcttca tcctttttcg tatccggatc 123840
   cttttcttca gaataataga ttgacgactt tggtgtagag gattctgcca gcctctgtga 123900
   gaacttgtta aagaagtcca tttaaggctt taaaattgaa ttgcgattat aagattaaat 123960
   ggcagacaca gacgatatta tcgactatga atccgatgat ctcaccgaat acgaggatga 124020
   tgaagaagag gaagaagatg gagagtcact agaaactagt gatatagatc ccaaatcttc 124080
   ttataagatt gtagaatcag catccactca tatagaagat gcgcattcca atcttaaaca 124140
   tatagggaat catatatctg ctcttaaacg acgctatact agacgtataa gtctatttga 124200
   aatagcgggt ataatagcag aaagctataa cttgcttcaa cgaggaagat tacctctagt 124260
   ttcagaattt tctgacgaaa cgatgaagca aaatatgcta catgtaatta tacaagagat 124320
   agaggagggt tcttgtccta tagtcatcga aaagaacgga gaattgttgt cggtaaacga 124380
   ttttgacaaa gatggtctaa aattccatct agactatatt atcaaaattt ggaaacttca 124440
   aaaacgatat tagaatttat acgaatatcg ttctctaaat gtcacaatca agtctcgcat 124500
   gttcagcaat ttattgtcgt actttatatc gtgttcatta acgatatctt gcaaaatagt 124560
   aatgattcta tcttccttcg atagatattc ttcagagatt attgtcttat attctttctt 124620
   gttatcagat atgaatttga taagactttg aacattattg atacccgtct gtttaatttt 124680
   ttctacagat attttagttt tggcagattc tatcgtatct gtcaatagac atccaacatc 124740
   gacattcgac gtcaattgtc tataaatcaa cgtataaatt ttagaaataa cattagcgaa 124800
   ttgttgtgcg ttgatgtcgt tattctgaaa cagtatgatt ttaggtagca ttttcttaac 124860
   aaagagaacg tatttattgt tactcagttg aacagatgat atatccagat tactaacgca 124920
   tctgattccg tataccaaac tttcagaaga aatggtatac aattgtttgt attcattcaa 124980
   tgtctctttt tcagaaatta gtttagagtc gaatactgca ataattttca agagatagtt 125040
   ttcatcagat aagattttat ttagtgtaga tatgataaaa ctattgtttt gttggagaac 125100
   ttgatacgcc gcgttctctg tagtcgacgc tctcaaatgg gaaacaatct ccattatttt 125160
   tttggaatcg gatactatat cttcggtatc ttgacgcagt ctagtataca tagagttaag 125220
   agagattaga gtttgtacat taagcaacat gtctctaaat gtggctacaa acttttcctt 125280
   tttcacatca tctagtttat tatataccga tttcacaacg gcaccagatt taaggaacca 125340
   gaatgaaaaa ctctgataac tacaatattt catcatagtt acgattttat catcttctat 125400
   agttggtgta atagcgcata cctttttctc caagactgga accaacgtca taaaaatgtt 125460
   taaatcaaaa tccatatcaa catctgatgc gctaagacca gtctcgcgtt caagattatc 125520
   tttactaatg gtgacgaact catcgtataa aactctaagt ttgtccatta tttatttaca 125580
   gatttagttg tttaatttat ttgtgctctt ccagagttgg gatagtattt ttctaacgtc 125640
   ggtattatat tattaggatc tacgttcata tgtatcataa tattaatcat ccacgttttg 125700
   ataaatctat ctttagcttc tgaaataacg tatttaaaca aaggagaaaa atatttagct 125760
   acggcatcag acgcaataac attttttgta aatgtaacgt atttagacga cagatcttcg 125820
   ttaaaaagtt ttccatctat gtagaatcca tcggttgtta acaccattcc cgcgtcagat 125880
   tgaataggag tttgaatagt ttgttttgga aatagatcct tcaataactt atagttgggt 125940
   gggaaaaaat cgattttatc actagactct ttctttttta ctatcattac ctcatgaact 126000
   atttcttgaa tgagtatatg tattttcttt cctatatcgg acgcgttcat tggaaaatat 126060
   accatgtcgt taactataag aatattttta tcctcgttta caaactgaat aatatcagat 126120
   gtagttcgta aacgaactat atcatcacca gcacaacatc taactatatg atatccacta 126180
   gtttccttta gccgtttatt atcttgttcc atattagcag tcattccatc atttaagaag 126240
   gcgtcaaaaa taatagggag aaatgacatt ttggattctg ttacaacttt accaaaatta 126300
   aggatatacg gacttactat ctttttctca acgtcaattt gatgaacaca cgatgaaaat 126360
   gtgcttctat gagattgatc atgtagaaaa caacaaggga tacaatattt ccgcatatca 126420
   tgaaatatat taagaaatcc caccttatta tatttcccca aaggatccat gcacgtaaac 126480
   attatgccgt tatcattaat aaagacttct ttctcatcgg atctgtaaaa gttgttactg 126540
   atttttttca ttccaggatc tagataatta ataatgatgg gttttctatt cttattcttt 126600
   gtattttggc atatcctaga ccagtaaaca gtttccactt tggtaaaatc agcagacttt 126660
   tgaacgctat taaacatggc attaatggca ataactaaaa atgtaaaata tttttctatg 126720
   ttaggaatat ggtttttcac tttaatagat atatggtttt tggccaaaat gatagatatt 126780
   tttttatccg aggatagtaa aatattatta gtcgccgtct ctataaaaat gaagctagtc 126840
   tcgatatcca attttattct agaattgata ggagtcgcca aatgtacctt atacgttata 126900
   tctcccttga tgcgttccat ttgtgtatct atatcggaca caagatctgt aaatagtttt 126960
   acgttattaa tcatcacggt atcgccgtcg ctagataacg ctaatgtacc atccaagtcc 127020
   caaatggaga gatttaactg ttcatcgttt agaataaaat gattaccggt catattaata 127080
   aagtgttcat cgtatctaga taacaacgac ttataattaa tgtccaagtc ttgaactcgc 127140
   tgaatgatct tttttaaccc agttagtttt agattggtac gaaatatatt gttaaacttt 127200
   gattctacag taatgtccaa atctagttgt ggaaatactt ccatcaacat tgtttcaaac 127260
   ttgataatat tattatctac atcttcgtac gatccaaatt ccggaataga tgtatcgcac 127320
   gctctggcca cccagataac caaaaagtca cacgctccag gatatacatt gtataaaaag 127380
   ctatcgtttt ttagtagggt ttttttctgc gtgtatacga agggattaaa aatagtatta 127440
   tcaacgtaac tatattccaa attattctta tgagaataga taataatatc gtccttaata 127500
   tctaacaaat ttcctaaata tccctttaat tgagtcattc gaagcgtcaa tagaatatgt 127560
   ctcttaacta tttccggctg ttgtatattt aaatgacttc gtaaaaaata atatatgggc 127620
   gacttctcat ctatgtaatc atatggagtg agatataggg ctcgttctac ctcctgcccc 127680
   ttacccacct gtaataccaa ttgcggactt actatatatc gcatatttat atcgtggggt 127740
   aaagtgaaaa tctactaccg atgatgtaag tcttacaatg ttcgaaccag taccagatct 127800
   taatttggag gcctccgtag aactagggga ggtaaatata gatcaaacaa cacctatgat 127860
   aaaggagaat agcggtttta tatcccgcag tagacgtcta ttcgcccata gatctaagga 127920
   tgatgagaga aaactagcac tacgattctt tttacaaaga ctttattttt tagatcatag 127980
   agagattcat tatttgttca gatgcgttga cgctgtaaaa gacgtcacta ttaccaaaaa 128040
   aaataacatt atcgtggcgc cttatatagc acttttaact atcgcatcaa aaggatgcaa 128100
   acttacagaa acaatgattg aagcattctt tccagaacta tataatgaac atagtaagaa 128160
   atttaaattc aactctcaag tatccatcat ccaagaaaaa ctcggatacc agtttggaaa 128220
   ctatcacgtt tatgattttg aaccgtatta ctctacagta gctctggcta ttcgagatga 128280
   acattcatct ggcattttta atatccgtca agagagttat ctggtaagtt cattatctga 128340
   aataacatat agattttatc taattaatct aaaatctgat cttgttcaat ggagtgctag 128400
   tacgggcgct gtaattaatc aaatggtaaa tactgtattg attacagtgt atgaaaagtt 128460
   acaactggtc atagaaaatg attcacaatt tacatgttca ttggctgtgg aatcaaaact 128520
   tccaataaaa ttacttaaag atagaaatga attatttaca aaattcatta acgagttaaa 128580
   aaagaccagt tcattcaaga taagcaaacg cgataaggat acgctactaa aatattttac 128640
   ttaggactgg agttagaatt tatagacgac tcatttcgtt tatcattgtt actattatta 128700
   ctattactat cattattagt gttggcatta ttagtattct tcttgtcatc ttgttcagaa 128760
   atatacagca atgctatacc taatactaaa tacattatca tgctcgcaat ggctctaaca 128820
   acaacgaacc aaaatgaatt tggtcgtagc ttttgttcac aaaaatacat aaagaaatgt 128880
   ctacataaat ctatggcgcc attggctact tgaaatagcg ccagtcctcc tacagatttt 128940
   aatatagctg tataacatga catttattca tcatcaaaag agacagagtc accatctgtc 129000
   atatttagat tttttttcat gtgttcaaag tatcctctac tcatttcatt ataatagttt 129060
   atcatactta gaattttagg acggatcaat gagtaagact tgactagatc gtcagtagta 129120
   atttgtgcat cgtctattct gcatccgctt cgtcgaataa tgtatagcat cgctttgaga 129180
   ttctccatag ctatcaagtc tttatacaat gacatggaaa tatctgtgaa tactttatac 129240
   ttctccaaca tcgatgcctt aacatcatcg cctactttag cattgaaaat acgttctatt 129300
   gtgtagatgg atgtagcaag atttttaaac aacaatgcca tcttacacga tgattgcctc 129360
   aagtctccaa tcgtttgttt agaacgatta gctacagagt ccaatgcttg gctgactagc 129420
   atattattat ctttagaaat tgtattcttc aatgaggcgt ttatcatatc tgtgatttcg 129480
   ttagtcatat tacagtctga ctgggttgta atgttatcca acatatcacc tatggatacg 129540
   gtacacgtac cagcatttgt aataatccta tctaagatgt tgtatggcat tgcgcagaaa 129600
   atatcttctc ctgtaatatc tccactctcg ataaatctac tcagattatt cttaaatgcc 129660
   ttattctctg gagaaaagat atcagtgtcc atcatttcat taatagtata cgcagaaaag 129720
   ataccacgag tatcaattct atccaagata cttatcggtt ccgagtcaca gataatggtt 129780
   tcctctcctt cgggagatcc tgcatagaaa tatctaggac aatagtttct atactgtctg 129840
   taactctgat aatctctaaa gtcactaact gataccatga aattgagaag atcaaacgct 129900
   gaagtaatta atttttctgc ctcgttttta ctacaactag ttttcatcaa tgtagtgacg 129960
   atgtattgtt tagttacttt tggtctaata ctgatgatag agatattatt gcttcccata 130020
   atggatcttc tagtagtcac cttaaagccc attgatgcga atagcagata gataaagtct 130080
   tggtatgact cctttctaat atagtacgga ctacctttgt cacccaactt tatacccaca 130140
   taagccataa caacctcttt aatagccgtt tcatgaggtt tatcagccat gagcctgagt 130200
   agttggaaga atctcatgaa tcccgtctca gaaagtccta tatgcatgat agatttatct 130260
   ttcctgggaa actctcgtat agtcatagat gaaatactct ttaaagtttc tgaaataaga 130320
   ttagtaacag tcttacctcc gactactcta ggtaacaaac aaactctaat aggtgttttc 130380
   tctgcggaga taatatcaga aaggatagag caataagtag tattattgtg attataaaga 130440
   ccgaatacat aacaggtaga atttataaac atcatgtcct gaaggttttt agacttgtat 130500
   tcctcgtaat ccataccgtc ccaaaacatg gatttggtaa ctttgatagc cgtagatctt 130560
   tgttccttcg ccaacaggtt aaagaaatta ataaagaatt tgttgtttct atttatgtcc 130620
   acaaattgca cgtttggaag cgccacggtt acattcactg cagcattttg aggatcgcga 130680
   gtatgaagta cgatgttatt gtttactggt atatctggaa agaaatctac cagtctagga 130740
   ataagagatt gatatcgcat agaaatagta aagtttataa tctcatcatc gaagagcatt 130800
   ttgttaccat tgtaataaat atccactctg tcatatgtat aaatgaagta ctgttcaaac 130860
   atgatgagat gtttatatgt tggcatagta gtgagatcga cgtttggtaa tggcaatgta 130920
   ttaagattaa ctccataatg tctagcagca tctgcgatgt tataagcgtc gtcaaagcgg 130980
   ggtcgatctt gtattgttat atattgtcta acacctataa gattatcaaa atcttgtctg 131040
   cttaatacac cgttaacaat ttttgccttg aattctttta ttggtgcatt aataacatcc 131100
   ttatagagga tgttaaacaa ataagtgtta tcaaagttaa gatctggata tttcttttct 131160
   gctagaacat ccattgagtc ggagccatct ggtttaatat aaccaccgat aaatctagct 131220
   ctgtattctg tatccgtcaa tctaatatta agaaggtgtt gagtgaaagg tggaagatcg 131280
   taaaagctgt gagtattaat gataggatta gtttccgaac taatgttaat tggggtatta 131340
   ataatatcta tatttccagc gttaagtgta acattaaaca gttttaattc acgtgacgtg 131400
   gtatcaatta aataattaat gcccaatttg gatatagcag cctgaagctc atcttgttta 131460
   gttacggatc ctaatgagtt attaagcaat atatcgaacg gatgaacgaa ggttgtttta 131520
   agttggtcac atactttgta atctagacat agatgcggaa gaacggtaga aactatacga 131580
   aataaatatt cagagtcctc taattgatca agagtaacta ttgacttaat aggcatcatt 131640
   tatttagtat taaatgacga ccgtaccagt gacggatata caaaacgatt taattacaga 131700
   gttttcagaa gataattatc catctaacaa aaattatgaa ataactcttc gtcaaatgtc 131760
   tattctaact cacgttaaca acgtggtaga tagagaacat aatgccgccg tagtgtcatc 131820
   tccagaggaa atatcctcac aacttaatga agatctattt ccagatgatg attcaccggc 131880
   cactattatc gaacgagtac aacctcatac tactattatt gacgatactc cacctcctac 131940
   gtttcgtaga gagttattaa tatcggaaca acgtcaacaa cgagaaaaaa gatttaatat 132000
   tacagtatcg aaaaatgctg aagcaataat ggaatctaga tctatgataa cttctatgcc 132060
   aacacaaaca ccatccttgg gagtagttta tgataaagat aaaagaattc agatgttaga 132120
   ggatgaagtg gttaatctta gaaatcaacg atctaataca aaatcatctg ataatttaga 132180
   taattttacc aaaatactat ttggtaagac tccgtacaaa tcaacagaag ttaataagcg 132240
   tatagccatc gttaattatg caaatttgaa cgggtccccc ttatcagtcg aggacttgga 132300
   tgtttgttcg gaggatgaaa tagatagaat ctataaaacg attaaacaat atcacgaaag 132360
   tagaaaacga aaaattatcg tcactaacgt gattattatt gtcataaaca ttatcgagca 132420
   ggcattgcta aaactcggat ttgaagaaat caaaggactg agtaccgata tcacttcaga 132480
   aattatcgat gtggagatcg gagatgactg cgatgctgta gcatctaaac taggaatcgg 132540
   taacagtccg gttcttaata ttgtattgtt tatactcaag atattcgtta aacgaattaa 132600
   aattatttaa tttaatacat tcccatatcc agacaacaat cgtctggatt aatctgttcc 132660
   tgtcgtctca taccggacga catattaatc tttttattag tgggcatctt tttagatggt 132720
   ttctttttcc cagcattaac tgagtcgata cctagaagat cgtgattgat ctctccgacc 132780
   attccacgaa cttctaattg gccgtctctg acggtaccat aaactatttt accagcatta 132840
   gtaacagctt ggacaatctg accatccatc gcattgtacg atgtagtagt aactgttgtt 132900
   ctacgtctag gagcaccaga agtatttttg gagcccttgg atgttgatgt agaagaagac 132960
   gaggattttg attttggttt acatgtaata cattttgaac tctttgattt tgtatcacat 133020
   gcgccggcag tcacatctgt ttgagaatta agattattgt tgcctccttt gacggctgca 133080
   tctccaccga tttgcgctag tagattttta agctgtggtg taatcttatt aactgtttcg 133140
   atataatcat cgtaactgct tctaacggct aaattttttt tatccgccat ttagaagcta 133200
   aaaatatttt tatttatgca gaagatttaa ctagattata caatgaacta atatgatcct 133260
   tttccagatt atttacaaac ttggtatttt ttggttctgg aggaggcgaa tttaaattcg 133320
   gacttggatt cggattttgt aagttcttga tcttattata catcgagtat aggatggcga 133380
   cagtaactgc tacacaaata ccgatcaaaa gaagaatacc aatcatttat tgacaataac 133440
   ttcactattg atcaagtatg caatatatca tcttttcact aaataagtag taataatgat 133500
   tcaacaatgt cgagatatat ggacgataat aatttagttc atggaaatat cgctatgatt 133560
   ggtgtgaatg actccgctaa ctctgtgggg tgcgcagtgc tttccccaca tagaataaat 133620
   tagcattccg actgtgataa taataccaag tataaacgcc ataatactca atactttcca 133680
   tgtacgagtg ggactggtag acttactaaa gtcaataaag gcgaagatac acgaaagaat 133740
   caaaagaatg attccagcga ttagcacgcc ggaaaaataa tttccaatca taagcatcat 133800
   gtccatttaa ctaataaaaa ttttaaatcg ccgaatgaac aaagtggaat ataaaccata 133860
   taaaaacaat agtttgtact gcaaaaataa tatctatttt tgttttcgaa gatatggtaa 133920
   aattaaatag tagtacacag catgttataa ctaacagcag caacggctcg taattactta 133980
   tcatttacta gacgaaaagg tggtgggata ttttcttgct caaataatac gaatatatca 134040
   cccatccatt ttatgcgatg tttatatact ctaatcttta atagatctat agacgacggg 134100
   tttaccaaca atatagattt tatcgattca tctaatttaa acccttcctt aaacgtgaat 134160
   gatctattat ctggcataac gatgacccta cctgatgaat cggacaatgt actgggccat 134220
   gtagaataaa ttatcaacga attatcgtct acgaacattt atatcatttg ttttaatttt 134280
   aggacgcgaa taaatggata taaaatagaa aataacagat attacaacca gtgttatggc 134340
   cgcgcccaac caggtaggca gttttatttt atcttttact acaggttctc ctggatgtac 134400
   gtcaccaacg gcggacgtag ttctagtaca attagacgta agttccgctt gggaattttt 134460
   taacgctaaa gagttaacgt taatcgtgca cccaacgtat ttacatctag ttcgttgaac 134520
   atcttgatta taatataacc attttctatc tctagattcg tcagtgcact catgtaacca 134580
   acatacccta ggtcctaaat atttatctcc ggaattagat tttggataat tcgcgcacca 134640
   acaatttcta tttcctttat gatcgttaca aaagacgtat aatgccgtat ccccaaaagt 134700
   aaaataatca ggacgaataa ttctaataaa ctcagaacaa tatctcgcat ccatatgttt 134760
   ggagcaaata tcggaataag tagacatagc cggtttccgt tttgcacgta accattctaa 134820
   acaattgggg tttccaggat cgtttctaca aaatccagtc atgaaatcgt cacaatgttc 134880
   tgtcttgtaa ttattattaa atatttttgg acagtgtttg gtatttgtct tagaacaaca 134940
   ttttgccacg ctatcactat cgcccaggag ataatccttt tttataaaat gacatcgttg 135000
   cccggatgct atataatcag tagcgtgttt taaatcctta atatattcag gagttacctc 135060
   gttctgataa tagattaatg atccaggacg aaatttgaaa gaactacatg gttctccatg 135120
   aattaataca tattgtttag caaattcagg aactataaaa ctactacaat gatctatcga 135180
   cataccatct atcaaacaaa acttgggttt aatttctccc ggagatgttt cataatagta 135240
   cgtataactt tcttctgcaa acttaacagc tctattatat tcaggataat taaaacctaa 135300
   ttccatatat ttgtctcgta tatctgctat tcctggtgct attttgattc tattaagagt 135360
   aacagctgcc cccattctta ataatcgtca gtatttaaac tgttaaatgt tggtatatca 135420
   acatctacct tatttcccgc agtataaggt ttgttgcagg tatactgttc aggaatggtt 135480
   acatttatac ttcttctata gtcctgtctt tcgatgttca tcacatatgc aaagaacaga 135540
   ataaacaaaa taatgtaaga aataatatta aatatctgtg aattcgtaaa tacattgatt 135600
   gccataataa ttacagcagc tacaatacac acaatagaca ttcccacagt gttgccatta 135660
   cctccacgat acatttgagt tactaagcaa taggtaataa ctaagctagt aagaggcaat 135720
   agaaaagatg agataaatat catcaatata gagattagag gagggctata tagagccaag 135780
   acgaacaaaa tcaaaccgag taacgttcta acatcattat ttttgaagat tcccaaataa 135840
   tcattcattc ctccataatc gttttgcatc atacctccat ctttaggcat aaacgattgc 135900
   tgctgttcct ctgtaaataa atctttatca agcactccag cacccgcaga gaagtcgtca 135960
   agcatattgt aatatcttaa ataactcatt tatatattaa aaaatgtcac tattaaagat 136020
   ggagtataat ctttatgccg aactaaaaaa aatgacttgt ggtcaacccc taagtctttt 136080
   taacgaagac ggggatttcg tagaagttga accgggatca tcctttaagt ttctgatacc 136140
   taagggattt tacgcctctc cttccgtaaa gacgagtcta gtattcgaga cattaacaac 136200
   gaccgataat aaaatcacta gtatcaatcc aacaaatgcg ccaaagttat atcctcttca 136260
   acgcaaagtc gtatctgaag tagtttctaa tatgaggaaa atgatcgaat caaaacgtcc 136320
   tctatacatc actcttcact tggcgtgtgg atttggtaag actattacca cgtgttatct 136380
   tatggctaca cacggtagaa aaaccgtcat ttgcgtaccc aataaaatgt taatacatca 136440
   atggaagaca caggtagagg cagtcggatt ggaacataag atatccatag atggagtaag 136500
   tagtctatta aaggaactaa agactcaaag tccggatgta ttaatagtag tcagtagaca 136560
   tctgacaaac gatgcctttt gtaaatatat caataagcat tatgatttgt tcatcttgga 136620
   tgaatcacat acgtataatc tgatgaacaa tacagcagtt acaagatttt tagcgtatta 136680
   tcctccgatg atgtgttatt ttttaactgc tacacctaga ccatctaacc gaatttattg 136740
   taacagtatt attaatattg ccaagttatc caatctaaaa aaaactatct atgcagtaga 136800
   tagttttttt gagccatatt ccacagataa tattagacat atggtaaaac gactagatgg 136860
   accatctaat aaatatcata tatataccga gaagttatta tctgtagacg agcctagaaa 136920
   tcaacttatt cttgataccc tggtagaaga attcaagtca ggaactatta atcgcatttt 136980
   agttattact aaactacgtg aacatatggt attattctac aaacgattat tagatttttt 137040
   cggaccagag gttgtattta taggagacgc ccaaaataga cgtactccag atatggtcaa 137100
   atcaatcaag gaactaaata gatttatatt cgtatccacc ttattttatt ccggtactgg 137160
   tttagatatt cctagtttgg attcgttgtt catttgctcg gcagtaatca acaatatgca 137220
   aatagagcaa ttactaggga gggtatgtcg agaaacagaa ctattagata ggacggtata 137280
   tgtatttcct aacacatcca tcaaagaaat aaagtacatg ataggaaatt tcatgcaacg 137340
   aattattagt ctgtctgtag ataaactagg atttaaacaa aaaagttatc ggaaacatca 137400
   agaatccgat cccacttctg catgtacaac atcatccaga gaagaacgtg tattaaatag 137460
   aatatttaac tcgcaaaatc gttaagaagt ttaagcgacg atccgcatgc tgcgcaggcc 137520
   agtgtattac ccctcatagt attaatataa tccaatgata cttttgtgat gtcggaaatc 137580
   ttaaccaatt tagactgaca ggcagaacac gtcatgcaat catcatcgtc atcgataact 137640
   gtagtcttgg gcttcttttt gcgactcttc attccggaac gcacattggt gctatccatt 137700
   taggtagtaa aaaataagtc agaatatgcc ctataacacg atcgtgcaaa acctggtata 137760
   tcgtctctat ctttatcaca atatagtgta tcgacatttt tattattatt gacctcgttt 137820
   atcttggaac atggaatggg aacatttttg ttatcaacgg ccatctttgc cttaattcca 137880
   gatgttgtaa aattataact aaacagtcta tcatcgacac aaatgaaatt cttgtttaga 137940
   cgtttgtagt ttacgtatgc ggctcgttcg cgtctcattt tttcagatat tgcaggtact 138000
   ataatattaa aaataagaat gaaataacat aggattaaaa ataaagttat catgacttct 138060
   agcgctgatt taactaactt aaaagaatta cttagtctgt acaaaagttt gagattttca 138120
   gattctgcgg ctatagaaaa gtataattct ttggtagaat ggggaacatc tacttactgg 138180
   aaaataggcg tgcaaaaggt agctaatgtc gagacgtcaa tatctgatta ttatgatgag 138240
   gtaaaaaata aaccgtttaa tattgatccg ggctattaca ttttcttacc ggtatatttt 138300
   gggagcgtct ttatttattc gaagggtaaa aatatggtag aacttggatc tggaaactct 138360
   tttcaaatac cagatgatat gcgaagtgcg tgtaacaaag tattagacag cgataacgga 138420
   atagactttc tgagatttgt tttgttaaac aatagatgga taatggaaga tgctatatca 138480
   aaatatcagt ctccagttaa tatatttaaa ctagctagtg agtacggatt aaacataccc 138540
   aaatatttag aaattgaaat agaggaagac acattatttg acgacgagtt atactctatt 138600
   atagaacgct ctttcgatga taaatttcca aaaatatcca tatcgtatat taagttggga 138660
   gaacttagac ggcaagttgt agactttttc aaattctcat tcatgtatat tgagtccatc 138720
   aaggtagatc gtataggaga taatattttt attcctagcg ttataacaaa atcaggaaaa 138780
   aagatattag taaaagatgt agaccattta atacgatcca aggttagaga acatacattt 138840
   gtaaaagtaa aaaagaaaaa cacattttcc attttatacg actatgatgg aaacggaaca 138900
   gaaactagag gagaagtaat aaaacgaatt atagacacta taggacgaga ctattatgtt 138960
   aacggaaagt atttctctaa ggttggtagt gcaggcttaa agcaattgac taataaatta 139020
   gatattaatg agtgcgcaac tgtcgatgag ttagttgatg agattaataa atccggaact 139080
   gtaaaacgaa aaataaaaaa ccaatcagca tttgatttaa gcagagaatg tttgggatat 139140
   ccagaagcgg attttataac gttagttaat aacatgcggt tcaaaataga aaattgtaag 139200
   gttgtaaatt tcaatattga aaatactaat tgtttaaata acccgagtat tgaaactata 139260
   tatggaaact ttaaccagtt cgtctcaatc tttaatgtcg tcaccgatgt caaaaaaaga 139320
   ttattcgagt gaaataatat gcgcctttga tataggtgca aaaaatcctg ccagaactgt 139380
   tttagaagtc aaggataact ccgttagggt attggatata tcaaaattag actggagttc 139440
   tgattgggaa aggcgcatag ctaaagattt gtcacaatat gaatacacta cagttcttct 139500
   agaacgtcag cctagaaggt cgccgtatgt taaatttatc tattttatta aaggcttttt 139560
   atatcataca tcggctgcca aagttatttg cgtctcgcct gtcatgtctg gtaattcata 139620
   tagagatcga aaaaagagat cggtcgaagc atttcttgat tggatggaca cattcggatt 139680
   gcgagactcc gttccggata gacgcaaatt agacgatgta gcggatagtt tcaatttggc 139740
   tatgagatac gtattagata aatggaatac taattataca ccttataata ggtgtaaatc 139800
   tagaaattac ataaaaaaaa tgtaataacg ttagtaacgc cattatggat aatctattta 139860
   cctttctaca tgaaatagaa gatagatatg ccagaactat ttttaacttt catctaataa 139920
   gttgcgatga aataggagat atatatggtc ttatgaaaga acgcatttcc tcagaggata 139980
   tgtttgataa tatagtgtat aataaagata tacatcctgc cattaagaaa ctagtgtatt 140040
   gcgacatcca acttactaaa cacattatta atcagaatac gtatccggta tttaacgatt 140100
   cttcacaagt gaaatgttgt cattatttcg acataaactc agataatagc aatattagct 140160
   ctcgtacagt agagatattt gagagggaaa agtcatctct tgtatcatat attaaaacta 140220
   ccaataagaa gagaaaggtc aattacggcg aaataaagaa aactgttcat ggaggcacta 140280
   atgcaaatta cttttccggt aaaaagtctg acgagtatct gagtactaca gttagatcca 140340
   acattaatca accttggatc aaaaccatct ctaagaggat gagagttgat atcattaatc 140400
   actctatagt aacgcgtgga aaaagctcta tattacaaac tatagaaatt atttttacta 140460
   atagaacatg tgtgaaaata ttcaaggatt ctactatgca cattattcta tccaaggaca 140520
   aggatgaaaa ggggtgtata cacatgattg acaaattatt ctatgtctat tataatttat 140580
   ttctgttgtt cgaagatatc atccaaaacg agtactttaa agaagtagct aatgttgtaa 140640
   accacgtact cacggctacg gcattagatg agaaattatt cctaattaag aaaatggctg 140700
   aacacgatgt ttatggagtt agcaatttca aaatagggat gtttaacctg acatttatta 140760
   agtcgttgga tcataccgtt ttcccctctc tgttagatga ggatagcaaa ataaagtttt 140820
   ttaaggggaa aaagctcaat attgtagcat tacgatctct ggaggattgt ataaattacg 140880
   tgactaaatc cgagaatatg atagaaatga tgaaggaaag atcgactatt ttaaatagca 140940
   tagatataga aacggaatcg gtagatcgtc taaaagaatt gcttctaaaa tgaaaaaaaa 141000
   cactaattca gaaatggatc aacgactagg gtataagttt ttggtgcctg atcctaaagc 141060
   cggagttttt tatagaccgt tacatttcca atatgtatcg tattctaatt ttatattgca 141120
   tcgattgcat gaaatcttga ccgtcaagcg gccactctta tcgtttaaga ataatacaga 141180
   acgaattatg atagaaatta gcaatgttaa agtgactcct ccagattact cacctataat 141240
   cgcgagtatt aaaggtaaga gttatgacgc attagccacg ttcactgtaa atatctttaa 141300
   agaggtaatg accaaagagg gtatatccat cactaaaata agtagttatg agggaaaaga 141360
   ttctcatttg ataaaaattc cgctactaat aggatacggg aataaaaatc cacttgatac 141420
   agccaagtat cttgttccta atgtcatagg tggagtcttt atcaataaac aatctgtcga 141480
   aaaagtagga attaatctag tagaaaagat tacaacatgg ccaaaattta gggttgttaa 141540
   gccaaactca ttcactttct cgttttcctc cgtatcccct cctaatgtat taccgacaag 141600
   atatcgccat tacaagatat ctctggatat atcacaattg gaagcgttga atatatcatc 141660
   gacaaagaca tttataacgg tcaatattgt tttgctgtct caatatttat ctagagtgag 141720
   tctagaattc attagacgta gtttatcata cgatatgcct ccagaagttg tctatctagt 141780
   aaacgcgata atagatagtg ctaaacgaat tactgaatct attactgact ttaatattga 141840
   tacatacatt aatgacctgg tggaagctga acacattaaa caaaaatctc agttaacgat 141900
   caacgagttc aaatatgaaa tgctgcataa ctttttacct catatgaact atacacccga 141960
   tcaactaaag ggattttata tgatatcttt actaagaaag tttctctact gtatcttcca 142020
   cacttctaga tatccagata gagattcgat ggtttgtcat cgcatcctaa cgtacggcaa 142080
   atattttgag acgttggcac atgatgaatt agagaattac ataggcaaca tccgaaacga 142140
   tatcatgaac aatcacaaga acagaggcac ttacgcggta aacattcatg tactaacaac 142200
   tcccggactt aatcacgcgt tttctagctt attgagtgga aagttcaaaa agtcagacgg 142260
   tagttatcga acacatcctc actattcatg gatgcagaat atttctattc ctaggagtgt 142320
   tggattttat ccggatcaag taaagatttc aaagatgttt tctgtcagaa aataccatcc 142380
   aagtcaatat ctttactttt gttcatcaga cgttccggaa agaggtcctc aggtaggttt 142440
   agtatctcaa ttgtctgtct tgagttccat tacaaatata ctaacgtctg agtatttgga 142500
   tttggaaaag aaaatttgtg agtatatcag atcatattat aaagatgata taagttactt 142560
   tgaaacagga tttccaatca ctatagaaaa tgctctagtc gcatctctta atccaaatat 142620
   gatatgtgat tttgtaactg actttagacg tagaaaacgg atgggattct tcggtaactt 142680
   ggaggtaggt attactttag ttagggatca catgaatgaa attcgcatta atattggagc 142740
   gggaagatta gtcagaccat tcttggttgt ggataacgga gagctcatga tggatgtgtg 142800
   tccggagtta gaaagcagat tagacgacat gacattctct gacattcaga aagagtttcc 142860
   gcatgtcatc gaaatggtag atatagaaca atttactttt agtaacgtat gtgaatcggt 142920
   tcaaaaattt agaatgatgt caaaggatga aagaaagcaa tacgatttat gtgactttcc 142980
   tgccgaattt agagatggat atgtagcatc ttcattagtg ggaatcaatc acaattctgg 143040
   acccagagct attcttggat gtgctcaagc taaacaagct atctcttgtc tgagctcgga 143100
   tatacgaaat aaaatagaca atggaattca tttgatgtat ccagagaggc caatcgtgat 143160
   tagtaaggct ttagaaactt caaagattgc ggctaattgc ttcggccaac atgttactat 143220
   agcattaatg tcgtacaaag gtatcaatca agaggatgga attatcatca aaaaacaatt 143280
   tattcagaga ggcggtctcg atattgttac agccaagaaa catcaagtag aaattccatt 143340
   ggaaaacttt aataacaaag aaagagatag gtctaacgcc tattcaaaat tagaaagtaa 143400
   tggattagtt agactgaatg ctttcttgga atccggagac gctatggcac gaaatatctc 143460
   atcaagaact cttgaagatg attttgctag agataatcag attagcttcg atgtttccga 143520
   gaaatatacc gatatgtaca aatctcgcgt tgaacgagta caagtagaac ttactgacaa 143580
   agttaaggta cgagtattaa ccatgaaaga aagaagaccc attctaggag acaaatttac 143640
   cactagaacg agtcaaaagg gaacagtcgc gtatgtcgcg gatgaaacgg aacttccata 143700
   cgacgaaaat ggtatcacac cagatgtcat tattaattct acatccatct tctctagaaa 143760
   aactatatct atgttgatag aagttatttt aacagccgca tattctgcta agccgtacaa 143820
   caataaggga gaaaaccgac ctgtctgttt tcctagtagt aacgaaacat ccatcgatac 143880
   atatatgcaa ttcgctaaac aatgttatga gcattcaaat ccgaaattgt ccgatgaaga 143940
   attatcggat aaaatctttt gtgaaaagat tctctatgat cctgaaacgg ataagcctta 144000
   tgcatccaaa gtattttttg gaccaattta ttacttgcgt ctgaggcatt taactcagga 144060
   caaggcaacc gttagatgta gaggtaaaaa gacgaagctc attagacagg cgaatgaggg 144120
   acgaaaacgt ggaggaggta tcaagttcgg agaaatggag agagactgtt taatagcgca 144180
   tggtgcagcc aatactatta cagaagtttt gaaagattcg gaagaagatt atcaagatgt 144240
   gtatgtttgt gaaaattgtg gagacatagc agcacaaatc aagggtatta atacatgtct 144300
   tagatgttca aaacttaatc tctctcctct cttaacaaaa attgatacca cgcacgtatc 144360
   taaagtattt cttactcaaa tgaacgccag aggcgtaaaa gtcaaattag atttcgaacg 144420
   aaggcctcct tcgttttata aaccattaga taaagttgat ctcaagccgt cttttctggt 144480
   gtaatattct agtttggtag tagatacata tcaatatcat caaattcgag atccgaatta 144540
   taaaatgggc gtggattgtt aactatagaa tcggacgtct gatattcgaa aatctgtgga 144600
   gtttcaggtt ttggtggagg tgtaactgct acttgggata ctgaagtctg atattcagaa 144660
   agctgtggat gttctggttc ggcatccacc gatggtgtca catcactaat cggttcggta 144720
   acgtctgtgg atggaggtgc tacttctaca gaacctgtag cctcagttgt caacggagat 144780
   acatttttaa tgcgagaaaa tgtataattt ggtaatggtt tcttatgtgg atctgaagaa 144840
   gaggtaagat atctactaga aagataccga tcacgttcta gttctctttt gtagaactta 144900
   actttttctt tctccgcatc tagttgatat tccaacctct tcacgttact acgttcagat 144960
   tccaattcac gttcgcatgg gttacctccg cagtttttac gagcgatttc acgttcagcc 145020
   ttcatgcgtc tctccctctc tctatcgagt ttatcagagc agtctttctg aaggcgatcg 145080
   aactccataa atttctccaa cgctttgatt gtttccatag atttccgaag ttcagctttt 145140
   aggactgtga ttctttttct ttcgaattca cagctggatg tacaaccgtt tccattaccg 145200
   ccatctctaa gtttcttttc tagatcggca acatttcatc cccatgcctt ttacattcct 145260
   cgagtctact gtcgtcgaaa tatcgttcca gctccttttc gacatcaata actttagcac 145320
   gttgtctctc aagctctctt ttgtagttat ctgattccct ggcacgttta agatcttcat 145380
   gcaattgagt cagctcttaa cttcctctct tgcttcttcg tcatagtacg cgcaatcact 145440
   gtgagatcca ttgttaccac gtctacactc ggcgagctcg cgtttaagag attcaatttc 145500
   ccgtttgtat tggtccatgt ttccattgct accaccatta gatttacagg ctgctagttg 145560
   tcgttcgaga tcagaaatac gggttttctt ggaattgatt tcgtcgatgt acttggcatc 145620
   gaaacactta ttaagttctt tttccaattc tacgatttta tttctttcgc gagtcaattc 145680
   cctcctgtag taactatctg ttttgtcaga ttcacgctct ctacgtagac tttcttgcaa 145740
   gttactaatt tgttccctag cacgtccgag tttagtttta tatgctgaat agagttctga 145800
   ttcatccttt gagcagatct ctagcgatcg tttaagattc ctaattctag tctttagcct 145860
   atttacctcc tcagaagatg ttccgttacc gttgcgttta cactcgttaa gctgtctatc 145920
   aagatccatg attctatctc taagacgttg catctctctt tccatatcag cattgctttc 145980
   attattacgt ctgcagtcac tcaactgtct ttcaatatct gagattctat ctctaagacg 146040
   tcgcatctct ctctgtttcg gcattggttt cattattacg tttacagtcg ttcaactgtc 146100
   tttcaagatc tgatattcta gattggagtc tgctaatctc tgtagcattt tcacggcatt 146160
   cactcagttg tctttcaaga tctgaaattt tagattggag tctgctaatc tctgtaagat 146220
   ttcctcctcc gctctcgatg cagtcggtca acttattctc tagttctcta atacgcgaac 146280
   gcagtgcatc aacttcttgc gtgtcttcct ggttgcgtgt acattcatcg agatctctaa 146340
   cgcgtcgtcg ttcttcctca agttctctgc gtactacaga aagcgtgtcc ctatcttgtt 146400
   gatatttagc aatttctgat tctagagtac tgattttgct tacgtagtta ctaatagttg 146460
   tcttggcctt atcaagatcc tccttgtatt tgtcgcattc cttgatatcc ctacgaagtc 146520
   tggacagttc ccattcgaca ttacgacgtt tatcgatttc agctcggaga tcgtcatcgc 146580
   gttgttttag ccacatacga ctgagttcaa gttctcgttg acaagatcca tctacttttc 146640
   cattcctaat agtatccagt tccttttcta gttctgaacg catttctcgt tccctatcaa 146700
   gcgattctct caattctcgg atagtcttct tatcaatttc taataaatct gaaccatcat 146760
   ctgtcccatt ttgaatatcc ctgtgttctt tgatctcttt tgtaagtcgg tcgattcttt 146820
   cggttttata aacagaatcc ctttccaaag tcctaatctt actgagttta tcactaagtt 146880
   ctgcattcaa ttcggtgagt tttctcttgg cttcttccaa ctctgtttta aactctccac 146940
   tatttccgca ttcttcctcg catttatcta accattcaat tagtttatta ataactagtt 147000
   ggtaatcagc gattcctata gccgttcttg taattgtggg aacataatta ggatcttcta 147060
   atggattgta tggcttgata gcatcatctt tatcattatt agggggatgg acaaccttaa 147120
   ttggttggtc ctcatctcct ccagtagcgt gtggttcttc aataccagtg ttagtaatag 147180
   gcttaggcaa atgcttgtcg tacgcgggca cttcctcatc catcaagtat ttataatcgg 147240
   gttctacttc agaatattct tttctaagag acgcgacttc gggagttagt agaagaactc 147300
   tgtttctgta tctatcaacg ctggaatcaa tactcaagtt aaggatagcg aatacctcat 147360
   cgtcatcatc cgtatcttct gaaacaccat catatgacat ttcatgaagt ctaacgtatt 147420
   gataaataga atcagattta gtattaaaca gatccttaac ctttttagta aacgcatatg 147480
   tatattttag atctccagat ttcataatat gatcacatgc cttaaatgtc agtgcttcca 147540
   tgatataatc tggaacacta atgggtgacg aaaaagatac agcaccatat gctacgttga 147600
   taaataaatc tgaaccacta agtagataat gattaatgtt aagaaagagg aaatattcag 147660
   tgtataggta tgtcttggcg tcatatcttg tactaaacac gctaaacagt ttgttaatgt 147720
   gatcaatttc caatagatta attagagcag cgggaatacc aacaaacata ttaccacatc 147780
   cgtattttct atgaatatca catatcatgt taaaaaatct tgatagaaga gcgaatatct 147840
   cgtctgactt aatgagtcgt agttcagcag caacataagt cataactgta aatagaacat 147900
   actttcctgt agtgttgatt ctagactcca catcaacacc attattaaaa atagttttat 147960
   atacatcttt aatctgctct ccgttaatcg tcgaacgttc tagtatacgg aaacactttg 148020
   atttcttatc tgtagttaat gacttagtga tatcacgaag aatattacga attacatttc 148080
   ttgtttttct tgagagacct gattcagaac tcaactcatc gttccatagt ttttctacct 148140
   cagtggcgaa atctttggag tgcttggtac atttttcaat aaggttcgtg acctccattt 148200
   attataaaaa atttattcaa aacttaacta caatcgggta attataagat cgtagatctc 148260
   ccatgtggcg gaatactacc atctatcgca tgtggatgga cagtaggtaa tggccatggg 148320
   aacagtaatg tttgcatatt tatctttctt gctagtatta ctgcatattg tcccaatgtt 148380
   tcgatgtgat gttctaacct atcaactgcc gctgtatcac aacaatagtg tccgatgaaa 148440
   ttaagattat gatccaatgt gtttaatata tgattatcaa gtcttatacg atccgcgtct 148500
   tttttgacag gatcaggttc ttctacagga agaagtttcg gcctcttatg atattcatgt 148560
   ctgggaaacg gtggtctagg gtgaggctcc ggtatcggag tgggttttgg attataatca 148620
   tcatcgtcta tgacatcatc ttcgacttcg atatttattt tgctatcttg atgatgtcct 148680
   gtatcagttg cattttcagc actcgactga atattagcgc attcattgtc tattattacc 148740
   atatttctaa acccaaaatg tatgtgttga acatcagtac tatcgttgat gagtcttata 148800
   gcatgaattc gcttatcgtt atcgggttta tcttctgtca ccttagcaat tcctttttta 148860
   ttaaactcta cataatcata tccatttcta ttgtttgttc taatataaac gagtatagca 148920
   tcattgctaa atttttcaat agtatcaaaa acagaatatc ctaaaccata taatatatat 148980
   tcaggaacac tcaaactaaa tgtccaggat tctcctaaat acgtaaactt taatagtgcg 149040
   aaatcattca aaaatctacc acttatagat agatagtaca taaatgcgta tagtagtcta 149100
   cctatctctt tattatgaaa accggcatta cgatcatata tgtcgtgata tacctgtgat 149160
   ccgtttacgt taaaccataa atacatgggt gatcctataa acatgaattt atttctaatt 149220
   ctcagagcta tagttaattg accgtgtaat atttgcttac atgcatactt gatacgctta 149280
   ttaataagat ttttatcatt gctcgttatt tcagaatcgt atatataagg agtaccatcg 149340
   tgattcttac cagatattat acaaaatact atatataaaa tatattgacc cacgttagta 149400
   atcatataaa tgtttaacgt tttaaatttt gtattcaatg atccattatc atacgctatc 149460
   atggtcttgt aatattcatt ctttaaaata taatattgtg ttagccattg cattggagct 149520
   cctaatggag attttctatt ctcatccatt ttaggatagg ctttcataaa gtccctaata 149580
   acttcgtgaa taatgtttct atgttttcta ctgatgcatg tatttgcttc gattttttta 149640
   tcccatgttt catctatcat agatttaaac gcagtaatgc tcgcaacatt aacatcttga 149700
   accgttggta caattccgtt ccataaattt ataatgttcg ccatttatat aactcatttt 149760
   ttgaatatac ttttaattaa caaaagagtt aagttactca tatggacgcc gtccagtctg 149820
   aacatcaatc tttttagcca gagatatcat agccgctctt agagtttcag cgtgattttc 149880
   caacctaaat agaacttcat cgttgcgttt acaacacttt tctatttgtt caaactttgt 149940
   tgttacatta gtaatctttt tttccaaatt agttagccgt tgtttgagag tttcctcatt 150000
   gtcgtcttca tcggctttaa caattgcttc gcgtttagcc tctggctttt tagcagcctt 150060
   tgtagaaaaa aattcagttg ctggaattgc aagatcgtca tctccgggga aaagagttcc 150120
   gtccatttaa agtacagatt ttagaaactg acactctgcg ttatttatat ttggtacaac 150180
   acatggatta taaatattga tgttaataac atcagaaaat gtaaagtcta tacattgttg 150240
   catcgtgtta aattttctaa tggatctagt attattgggt ccaacttctg cctgaaatcc 150300
   aaatatggaa gcggatacaa aaccgtttcc tggataaacc acacatctcc acttttgctt 150360
   tacatcagaa attgtgtcgt tgacatcttg aactctccta tctaatgccg gtgttccacc 150420
   tatagatttt gaatattcga atgctgcatg agtagcatta aattccttaa tattgccata 150480
   attttcatat attgagtaac cctggataaa aagtaaacac accgcagccg tcgctaccac 150540
   aataaaaaaa attgatagag agttcattta taatctatta gaagctgaca aaattttttt 150600
   acacgcatca gacaatgctt taataaatag ttcaacatct acttttgtca tatcgaaccg 150660
   atggtatgat tctaacctag aattacatcc gaaaaagttg actatgttca tagtcattaa 150720
   gtcattaaca aacaacattc cagactctgg attataagac gatactgttt cgtcacaatt 150780
   acctacctta atcatgtgat tatgaatatt ggctattaga gcaccttcta agaaatctat 150840
   aatatctttg aaacacgatt taaaatcaaa ccacgaatat acttctacga agaaagttag 150900
   tttacccata ggagaaataa ctataaatgg agatctaaat acaaaatccg gatctatgat 150960
   agttttaaca ttattatatt ctctattaaa tacctccaca tctaaaaatg ttaattttga 151020
   aactatgtct tcgtttatta ccgtacctga actaaacgct ataagctcta ttgtttgaga 151080
   actctttaaa cgatattctt gaaatacatg taacaaagtt tcctttaact cggtcggttt 151140
   atctaccata gttacagaat ttgtatcctt atctataata taataatcaa aatcgtataa 151200
   agttatataa ttatcgcgtt cagattgtga tcttttcaaa tagactaaaa accccatttc 151260
   tctagtaagt atcttatgta tatgtttgta aaatatcttc atggtgggaa tatgctctac 151320
   cgcagttagc cattcctcat tgacagcggt agatgtatta gacaaaacta ttccaatgtt 151380
   taacaagggc cattttacga gattattaaa tccttgtttg ataaatgtag ccaatgaggg 151440
   ttcgagttca acgacgattg aattctcttc ccgcggatgc tgcatgatga acgacgggat 151500
   gttgttcgat tgatttggaa ttctttttcg actttttgtt tatattaaat attttaaaat 151560
   ttatagcgga tagcaattca tgtaccacgg ataatgtaga cgcgtattgc gcatcgatat 151620
   ctttattatt agataaattt atcaataaat gtgagaagtt tgcctcgtta aggtcttcca 151680
   tttaaatatt atataaacat ttgtgtttgt atcttattcg tcttttatgg aatagttttt 151740
   tactagtaaa gctgcaatta cacactttgt ccgtaaaaca taaatataaa caccagcttt 151800
   tatcaatcgt tccaaaaagt cgacggcgga catttttaac atggcatcta ttttaaatac 151860
   acttaggttt ttggaaaaaa catcatttta taattgtaac gattcaataa ctaaagaaaa 151920
   gattaagatt aaacataagg gaatgtcatt tgtattttat aagccaaagc attctaccgt 151980
   tgttaaatac ttgtctggag gaggtatata tcatgatgat ttggttgtat tggggaaggt 152040
   aacaattaat aatctaaaga tgatgctatt ttacatggat ttatcatatc atggagtgac 152100
   aagtagtgga gcaatttaca aattgggatc gtctatcgat agactttctc taaataggac 152160
   tattgttaca aaagttaata attatgatga tacatttttt gacgacgatg attgatcgct 152220
   attgcacaat tttgtttttg tactttctaa tatagtgttt aggttctttt tcatatgaga 152280
   atattgattt actaaaatat ctatgtttaa cttttgttct atgacgtcct tatcggcggt 152340
   atcggtacat atacgtaatt caccttcaca aaatacggag tcttcgataa taatagccaa 152400
   tcgattattg gatctagctg tctgtatcat attcaacatg tttaatatat cctttcgttt 152460
   cccctttaca ggcatcgatc gtagcatatt ttccgcgtct gatatggaaa tgttaaaact 152520
   acaaaaatgc gtaatgttag cccgtcctaa tattggtacg tgtctataag tttggcatag 152580
   tagaataata gacgtgttta aatgccttcc aaagtttaag aattctatta gagtattgca 152640
   ttttgatagt ttatcgccta catcatcaaa aataagtaaa aagtgtgctg attttttatg 152700
   attttgtgcg acagcaatac atttttctat gttactttta gttcgtatca gattatattc 152760
   tagagattcc tgactactaa cgaaattaat atgatttggc caaatgtatc catcataatc 152820
   tgggttataa acgggtgtaa acaagaatat atgtttatat tttttaacta gtgtagaaaa 152880
   cagagatagt aaatagatag tttttccaga tccagatcct cctgttaaaa ccattctaaa 152940
   cggcattttt aataaatttt ctcttgaaaa ttgtttttct tggaaacaat tcataattat 153000
   atttacagtt actaaattaa tttgataata aatcaaaata tggaaaacta aggttgttag 153060
   tagggaggag aacaaagaag gcacatcgtg atataaataa catttattat catgatgaca 153120
   ccagaaaacg acgaagagca gacatctgtg ttctccgcta ctgtttacgg agacaaaatt 153180
   cagggaaaga ataaacgcaa acgcgtgatt ggtctatgta ttagaatatc tatggttatt 153240
   tcactactat ctatgattac catgtccgcg tttctcatag tgcgcctaaa tcaatgcatg 153300
   tctgctaacg aggctgctat tactgacgcc gctgttgccg ttgctgctgc atcatctact 153360
   catagaaagg ttgcgtctag cactacacaa tatgatcaca aagaaagctg taatggttta 153420
   tattaccagg gttcttgtta tatattacat tcagactacc agttattctc ggatgctaaa 153480
   gcaaattgca ctgcggaatc atcaacacta cccaataaat ccgatgtctt gactacctgg 153540
   ctcattgatt atgttgagga tacatgggga tctgatggta atccaattac aaaaactaca 153600
   tccgattatc aagattctga tgtatcacaa gaagttagaa agtatttttg tgttaaaaca 153660
   atgaactaat atttattttt gtacattaat aaatgaaatc gcttaataga caaactgtaa 153720
   gtaggtttaa gaagttgtcg gtgccggccg ctataatgat gatactctca accattatta 153780
   gtggcatagg aacatttctg cattacaaag aagaactgat gcctagtgct tgcgccaatg 153840
   gatggataca atacgataaa cattgttatt tagatactaa cattaaaatg tctacagata 153900
   atgcggttta tcagtgtcgt aaattacgag ctagattgcc tagacctgat actagacatc 153960
   tgagagtatt gtttagtatt ttttataaag attattgggt aagtttaaaa aagaccaatg 154020
   ataaatggtt agatattaat aatgataaag atatagatat tagtaaatta acaaatttta 154080
   aacaactaaa cagtacgacg gatgctgaag cgtgttatat atacaagtct ggaaaactgg 154140
   ttaaaacagt atgtaaaagt actcaatctg tactatgtgt taaaaaattc tacaagtgac 154200
   aacaaaaaat gaattaataa taagtcgtta acgtacgccg ccatggacgc cgcgtttgtt 154260
   attactccaa tgggtgtgtt gactataaca gatacattgt atgatgatct cgatatctca 154320
   atcatggact ttataggacc atacattata ggtaacataa aaactgtcca aatagatgta 154380
   cgggatataa aatattccga catgcaaaaa tgctacttta gctataaggg taaaatagtt 154440
   cctcaggatt ctaatgattt ggctagattc aacatttata gcatttgtgc cgcatacaga 154500
   tcaaaaaata ccatcatcat agcatgcgac tatgatatca tgttagatat agaagataaa 154560
   catcagccat tttatctatt cccatctatt gatgttttta acgctacaat catagaagcg 154620
   tataacctgt atacagctgg agattatcat ctaatcatca atccttcaga taatctgaaa 154680
   atgaaattgt cgtttaattc ttcattctgc atatcagacg gcaatggatg gatcataatt 154740
   gatgggaaat gcaatagtaa ttttttatca taaaagttgt aaagtaaata ataaaacaat 154800
   aaatattgaa ctagtagtac gtatattgag caatcagaaa tgatgctggt acctcttatc 154860
   acggtgaccg tagttgcggg aacaatatta gtatgttata tattatatat ttgtaggaaa 154920
   aagatacgta ctgtctataa tgacaataaa attatcatga caaaattaaa aaagataaag 154980
   agttctaatt ccagcaaatc tagtaaatca actgatagcg aatcagactg ggaggatcac 155040
   tgtagtgcta tggaacaaaa caatgacgta gataatattt ctaggaatga gatattggac 155100
   gatgatagct tcgctggtag tttaatatgg gataacgaat ccaatgttat ggcgcctagc 155160
   acagaacaca tttacgatag tgttgctgga agcacgctgc taataaataa tgatcgtaat 155220
   gaacagacta tttatcagaa cactacagta gtaattaatg aaacggagac tgttgaagta 155280
   cttaatgaag ataccaaaca gaatcctaac tattcatcca atcctttcgt aaattataat 155340
   aaaaccagta tttgtagcaa gtcaaatccg tttattacag aacttaacaa taaatttagt 155400
   gagaataatc cgtttagacg agcacatagc gatgattatc ttaataagca agaacaagat 155460
   catgaacacg atgatataga atcatcggtc gtatcattgg tgtgattagt ttccttttta 155520
   taaaattgaa gtaatattta gtattattgc tgccgtcacg ttgtacaaat ggagatattc 155580
   cctgtattcg gcatttctaa aattagcaat tttattgcta ataatgactg tagatattat 155640
   atagatacag aacatcaaaa aattatatct gatgagatca atagacagat ggatgaaacg 155700
   gtacttctta ccaacatctt aagcgtagaa gttgtaaatg acaatgagat gtaccatctt 155760
   attcctcata gattatcgac gattatactc tgtattagtt ctgtcggagg atgtgttatc 155820
   tctatagata atgacatcaa tggcaaaaat attctaacct ttcccattga tcatgctgta 155880
   atcatatccc cactgagtaa atgtgtcgta gttagcaagg gtcctacaac catattggtt 155940
   gttaaagcgg atatacctag caaacgattg gtaacatcgt ttacaaacga catactatat 156000
   gtaaacaatc tgtcactgat taattatttg ccgttgtctg tattcattat tagacgagtc 156060
   accgactatt tggatagaca catatgcgat cagatatttg ctaataataa gtggtattcc 156120
   cttataacca tcgacgataa gcaatatcct attccatcaa actgtatagg tatgtcctct 156180
   gccaagtaca taaattctag catcgagcaa gatactttaa tccatgtttg taacctcgag 156240
   catccgttcg actcagtata caaaaaaatg cagtcgtaca attctctacc tatcaaggaa 156300
   caaatattgt acggtagaat tgataatata aatatgagca ttagtatttc tgtggattaa 156360
   tagatttcta gtatggggat cattaatcat ctctaatctc taaatacctc ataaaacgaa 156420
   aaaaaagcta ttatcaaata ctgtacggaa tggattcatt ctcttctctt tttatgaaac 156480
   tctgttgtat atctactgat aaaactggaa gcaaaaaatc tgatagaaag aataagaata 156540
   agatcaagga ttatatggaa cacgattatt ataaaataac aatagttcct ggttcctctt 156600
   ccacgtctac tagctcgtgg tattatacac atgcctagta atagtctctt tgcgttgacg 156660
   gaaagcagac tagaaataac aggctaaaat gttcagacac cataatagtt cccaacccag 156720
   ataataacag agttccatca acacattcct ttaaactcaa tcccaaaccc aaaaccgtta 156780
   aaatgtatcc ggccaattga tagtagataa tgaggtgtac agcgcatgat aatttacaca 156840
   gtaaccaaaa tgaaaatact ttagtaatta taagaaatat agacggtaat gtcatcatca 156900
   acaatccgat aatatgcctg agagtaaaca ttgacggata aaacaaaaat gctccgcata 156960
   actctatcat ggcaataaca caaccaaata cttgtaagat tcctaaatta gtagaaaata 157020
   caacgaatat cgatgtataa gtgatctcga gaaataataa gaataaagta atgcccgtaa 157080
   agataaacat caacattgtt tggtaatcat taaaccaatt agtatgaagt tgaactaatt 157140
   tcacagtaga ttttattcca gtgttatcct cgcatgtata agtacctggt aagatatctt 157200
   tatattccat aatcaatgag acatcactat ctgataacga atgaagtcta gcactagtat 157260
   gccatttact taatattgtc gtcttggaag ttttattata agttaaaata tcatggttat 157320
   ccaatttcca tctaatatac tttgtcggat tatctatagt acacggaata atgatggtat 157380
   cattacatgc tgtatactct atggtctttg tagttgttat aacaaccaac gtatagaggt 157440
   atatcaacga tattctaact cttgacattt tttatttatt taaaatgata cctttgttat 157500
   ttattttatt ctattttgct aacggtattg aatggcataa gtttgaaacg agtgaagaaa 157560
   taatttctac ttacttatta gacgacgtat tatacacggg tgttaatggg gcggtataca 157620
   cattttcaaa taataaacta aacaaaactg gtttaactaa taataattat ataacaacat 157680
   ctataaaagt agaggatgcg gataaggata cattagtatg cggaaccaat aacggaaatc 157740
   ccaaatgttg gaaaatagac ggttcagacg acccaaaaca tagaggtaga ggatacgctc 157800
   cttatcaaaa tagcaaagta acgataatca gtcacaacgg atgtgtacta tctgacataa 157860
   acatatcaaa agaaggaatt aaacgatgga gaagatttga cggaccatgt ggttatgatt 157920
   tattcacggc ggataacgta attccaaaag atggtttacg aggagcattc gtcgataaag 157980
   acggtactta tgacaaagtt tacattcttt tcactgatac tatcggctca aagagaattg 158040
   tcaaaattcc gtatatagca caaatgtgcc taaacgacga aggtggtcca tcatcattgt 158100
   ctagtcatag atggtcgacg tttctcaaag tcgaattaga atgtgatatc gacggaagaa 158160
   gttatagaca aattattcat tctagaacta taaaaacaga taatgatacg atactatatg 158220
   tattcttcga tagtccttat tccaagtccg cattatgtac ctattctatg aataccatta 158280
   aacaatcttt ttctacgtca aaattggaag gatatacaaa gcaattgccg tctccagctc 158340
   ctggtatatg tttaccagct ggaaaagttg ttccacatac cacgtttgaa gtcatagaac 158400
   aatataatgt actagatgat attataaagc ctttatctaa ccaacctatc ttcgaaggac 158460
   cgtctggtgt taaatggttc gatataaagg agaaggaaaa tgaacatcgg gaatatagaa 158520
   tatacttcat aaaagaaaat tctatatatt cgttcgatac aaaatctaaa caaactcgta 158580
   gctcgcaagt cgatgcgcga ctattttcag taatggtaac tgcgaaaccg ttatttatag 158640
   cagatatagg gataggagta ggaatgccac aaatgaaaaa aatacttaaa atgtaatctt 158700
   aatcgagtac accacacgac aatgaacaaa cataagacag attatgctgg ttatgcttgc 158760
   tgcgtaatat gcggtctaat tgtcggaatt atttttacag cgacactatt aaaagttgta 158820
   gaacgtaaat tagttcatac accattaata gataaaacga taaaagatgc atatattaga 158880
   gaagattgtc ctactgactg gataagctat aataataaat gtatccattt atctactgat 158940
   cgaaaaacct gggaggaagg acgtaatgca tgcaaagctc taaattcaaa ttcggatcta 159000
   attaagatag agactccaaa cgagttaagt tttttaagaa gccttagacg aggctattgg 159060
   gtaggagaat ccgaaatatt aaaccagaca accccatata attttatagc taagaatgcc 159120
   acgaagaatg gaactaaaaa acggaaatat atttgtagca caacgaatac tcccaaactg 159180
   cattcgtgtt acactatata acaattacac tacattttta tcataccact acttcggtta 159240
   gatgttttag aaaaaaataa atatcgccgt accgttcttg tttttataaa aataacaatt 159300
   aacaattatc aaattttttc tttaatattt tacgtggttg accattcttg gtggtaaaat 159360
   aatctcttag tgttggaatg gaatgctgtt taatgtttcc acactcatcg tatattttga 159420
   cgtatgtagt cacatcgttt acgcaatagt cagactgtag ttctatcatg cttcctacat 159480
   cagaaggagg aacagtttta aagtctcttg gttttaatct attaccgtta gttttcatga 159540
   aatcctttgt tttatccact tcacatttta aataaatgtc cactatacat tcttttgtta 159600
   attttactag atcgtcatgg gtcatagaat ttataggttc cgtagtccat ggatccaaac 159660
   tagcaaactt cgcgtatacg gtatcgcgat tagtgtatac accaactgta tgaaaattaa 159720
   gaaaacagtt taataaatca acagaaatat ttaatcctcc gtttgataca gatgcgccat 159780
   atttatggat ttcggattca cacgttgttt gtctgaggtg ttcgtctagt gttgcttcta 159840
   cgtaaacttc gattcccata tattctttat tgtcagaatc gcataccgat ttatcatcat 159900
   acactgtttg aaaactaaat ggtatacaca tcaaaataat aaataataac gagtacattc 159960
   tgcaatattg ttatcgtaat tggaaaaata gtgttcgagt gagttggatt atgtgagtat 160020
   tggattgtat attttatttt atattttgta ataagaataa aatgctaatg tcaagtttat 160080
   tccaatagat gtcttattaa aaacatatat aataaataac aatggctgaa tggcataaaa 160140
   ttatcgagga tatctcaaaa aataataagt tcgaggatgc cgccatcgtt gattacaaga 160200
   ctacaaagaa tgttctagct gctattccta acagaacatt tgccaagatt aatccgggtg 160260
   aaattattcc tctcatcact aatcgtaata ttctaaaacc tcttattggt cagaaatatt 160320
   gtattgtata tactaactct ctaatggatg agaacacgta tgctatggag ttgcttactg 160380
   ggtacgcccc tgtatctccg atcgttatag cgagaactca taccgcactt atatttttga 160440
   tgggtaagcc aacaacatcc agacgtgacg tgtatagaac gtgtagagat cacgctaccc 160500
   gtgtacgtgc aactggtaat taaaataaaa agtaatattc atatgtagtg tcaattttaa 160560
   atgatgatga tgaaatggat aatatccata ttgacgatgt caataatgcc ggtattggca 160620
   tacagttcat cgatttttag atttcattca gaggatgtgg aattatgtta tgggcatttg 160680
   tattttgata ggatctataa tgtagtaaat ataaaatata atccgcatat tccatataga 160740
   tataatttta ttaatcgcac gttaaccgta gatgaactag acgataatgt cttttttaca 160800
   catggttatt ttttaaaaca caaatatggt tcacttaatc ctagtttgat tgtctcatta 160860
   tcaggaaact taaaatataa tgatatacaa tgctcagtaa atgtatcgtg tctcattaaa 160920
   aatttggcaa cgagtacatc tactatatta acatctaaac ataagactta ttctctacat 160980
   cggtccacgt gtattactat aataggatac gattctatta tatggtataa agatataaat 161040
   gacaagtata atgacatcta tgattttact gcaatatgta tgctaatagc gtctacattg 161100
   atagtgacca tatacgtgtt taaaaaaata aaaatgaact cttaattatg ctatgctatt 161160
   agaaatggat aaaatcaaaa ttacggttga ttcaaaaatt ggtaatgttg ttaccatatc 161220
   gtataacttg gaaaagataa ctattgatgt cacacctaaa aagaaaaaag aaaaggatgt 161280
   attattagcg caatcagttg ctgtcgaaga ggcaaaagat gtcaaggtag aagaaaaaaa 161340
   tattatcgat attgaagatg acgatgatat ggatgtagaa agcgcgtaat acgatctata 161400
   aaaataagta tataaatact ttttatttac tgtactctta ctgtgtagtg gtgataccct 161460
   actcgattat ttttttaaaa aaaaaatact tattctgatt cttctagcca tttccgtgtt 161520
   cgtttgaatg ccacatcgac gtcaaagata ggggagtagt tgaaatctag ttctgcattg 161580
   ttggtacgca cctcaaatgt agtgttggat atcttcaacg tatagttgtt gagtagtgat 161640
   ggttttctaa atagaattct cttcatatca ttcttgcacg cgtacatttt tagcatccat 161700
   cttggaatcc tagatccttg ttctattccc aatggtttca tcaatagaag attaaacata 161760
   tcgtacgaac acgatggaga gtaatcgtag caaaagtaag catttccttt aatcttagat 161820
   cccggatact ggatatattt tgcagccaac acgtgcatcc atgcagcatt tcctacatat 161880
   acccggctat gcaccgcgtc atcatcgact gtacgataca taatgttacc gtgttgctta 161940
   cattgctcgt aaaagacttt catcaatttg tctccttctc cgtaaattcc agtgggtctt 162000
   aggcaacaag tatacaattt tgctccattc atgattacgg aattattggc tttcataacc 162060
   agttgctcgg ccatacgttt actttttgcg tatacatgtc ctggtgatat atcataaagg 162120
   gtatgctcat ggccgatgaa tggatcaccg tgtttattgg gtcctattgc ttccatgcta 162180
   ctagtataga tcaaatactt gattcctagg tccacacaag ctgccaaaat agtctgtgtt 162240
   ccataatagt ttactttcat gatttcatta tcggtgtatt ttccaaatac atccactaga 162300
   gcagccgtat gaataatcag atttacccca tctagcgctt ctctcacctt atcaaagtcg 162360
   tttatatcac attgtatata gtttataacc ttaactttcg aggttattgg ttgtggatct 162420
   tctacaatat ctatgactct gatttcttga acatcatctg cactaattaa cagttttact 162480
   atatacctgc ctagaaatcc ggcaccacca gtaaccgcgt acacggccat tgctgccact 162540
   cataatatca gactacttat tctattttac taaataatgg ctgtttgtat aatagaccac 162600
   gataatatca gaggagttat ttactttgaa ccagtccatg gaaaagataa agttttagga 162660
   tcagttattg gattaaaatc cggaacgtat agtttgataa ttcatcgtta cggagatatt 162720
   agtcaaggat gtgattccat aggcagtcca gaaatattta tcggtaacat ctttgtaaac 162780
   agatatggtg tagcatatgt ttatttagat acagatgtaa atatatttac aattattgga 162840
   aaggcgttat ctatttcaaa aaatgatcag agattagcgt gtggagttat tggtatttct 162900
   tacataaatg aaaagataat acattttctt acaattaacg agaatggcgt ttgatatatc 162960
   agttaatgcg tctaaaacaa taaatgcatt agtttacttt tctactcagc aaaataaatt 163020
   agtcatacgt aatgaagtta atgatacaca ctacactgtc gaatttgata gggacaaagt 163080
   agttgacacg tttatttcat ataatagaca taatgacacc atagagataa gaggggtgct 163140
   tccagaggaa actaatattg gttgcgcggt tagtatgact tacttgtata ataagtatag 163200
   ttttaaactg attttagcag aatatataag acacagaaat actatatccg gcaatattta 163260
   ttcggcattg atgacactag atgatttggc tattaaacag tatggagaca ttgatctatt 163320
   atttaatgag aaacttaaag tagactccga ttcgggacta tttgactttg tcaactttgt 163380
   aaaggatatg atatgttgtg attctagaat agtagtagct ctatctagtc tagtatctaa 163440
   acattgggaa ttgacaaata aaaaatatag gtgtatggca ttagccgaac atatatctga 163500
   tagtattcca atatctgagc tatctagact acgatacaat ctatgtaagt atctacgcgg 163560
   acacactgag agcatagagg ataaatttga ttattttgaa gacgatgatt cgtctacatg 163620
   ttctgccgta accgacaggg aaacggatgt ataatttttt ttatagcgtg aaggatatga 163680
   taaaaaatat aattgttgta tttatcccat tccaatcacc ttatatgatt ctgtaacaca 163740
   ataaaggagt cttatagatg tatagaggtc agatactggt ttgataaact gtttattcca 163800
   cataagtatg tttgacttta tggttagacc cgcatacttt aacaaatcac tgaaaattgg 163860
   agttaggtat tgacctctca gaatcagttg ccgttctgga acattaaatg tattttttat 163920
   gatatactcc aacgcattta tgtgggcata caacaagtca ttactaatgg aatattccaa 163980
   gagttttagt tgtctagtat ttaacaagag aagagatttc aacagactgt ttatgaactc 164040
   gaatgccgcc tcattgtcgc ttatattgat gatgtcgaat tctcccaata tcatcaccga 164100
   tgagtagctc atcttgttat cgggatccaa gttttctaaa gatgtcatta aaccctcgat 164160
   catgaatgga tttatcatca tcgtttttat gttggacatg agcttagtcc gtttgtccac 164220
   atctatagaa gatgatttct gaattatttc atatatctct ctctttaact ccaggaactt 164280
   gtcaggatgg tctactttaa tatgttctcg tctaagagat gaaaatcttt ggatggttgc 164340
   acgcgacttt tctctaaagg atgacgttgc ccaagatcct ctcttaaatg aatccatctt 164400
   atccttggac aagatggaca gtctattttc cttagatggt ttaatatttt tgttacccat 164460
   gatctataaa ggtagaccta atcgtctcgg atgaccatat atttattttc agttttatta 164520
   tacgcataaa ttgtaaaaaa tatgttaggt ttacgaaaat gtctcgtggg gcattaatcg 164580
   tttttgaagg attggacaaa tctggaaaaa caacacaatg tatgaacatc atggaatcaa 164640
   taccttcaaa cacaataaaa taccttaact ttcctcagag atccactgtc actggaaaga 164700
   tgatagatga ctatctaact cgtaaaaaaa cctataatga tcatatagtt aatctattat 164760
   tttgtgcaaa tagatgggag tttgcatctt ttatacaaga acaactagaa cagggaatta 164820
   ctttaatagt tgatagatac gcattttctg gagtagcgta tgccgccgct aaaggcgcgt 164880
   caatgactct cagtaagagt tatgaatctg gattgcctaa acccgactta gttatattct 164940
   tggaatctgg tagcaaagaa attaatagaa acgtcggtga ggaaatttat gaagatgtta 165000
   cattccaaca aaaggtatta caagaatata aaaaaatgat tgaagaagga gatattcatt 165060
   ggcaaattat ttcttctgaa ttcgaggaag atgtaaagaa ggagttgatt aagaatatag 165120
   ttatagaggc tatacacacg gttactggac cagtggggca actgtggatg taatagtgaa 165180
   attacatttt ttataaatag atgttagtac agtgttataa atggatgaag catattactc 165240
   tggcaacttg gaatcagtac tcggatacgt gtccgatatg cataccgaac tcgcatcaat 165300
   atctcaatta gttattgcca agatagaaac tatagataat gatatattaa acaaggacat 165360
   tgtaaatttt atcatgtgta gatcaaactt ggataatcca tttatctctt tcctagatac 165420
   tgtatatact attatagatc aagagaacta tcagactgag ttgattaatt cattagacga 165480
   caatgaaatt atcgattgta tagttaataa gtttatgagc ttttataagg ataacctaga 165540
   aaatatagta gatgctatca ttactctaaa atatataatg aataatccag attttaaaac 165600
   tacgtatgcc gaagtactcg gttccagaat agccgatata gatattaaac aagtgatacg 165660
   taagaatata ctacaattgt ctaataatat ccgcgaacga tatttgtgaa aatattaaaa 165720
   aaaaatactt tttttattaa atgacgtctt ttcgtgaatt tagaaaatta tgctgtgcta 165780
   tatatcacgc atcaggatat aaagaaaaat ctaaattaat tagagacttt ataacagata 165840
   gggatgataa atatttgatc attaagctat tgcttcccgg attagacgat agaatttata 165900
   acatgaacga taaacaaatt ataaaattat atagtataat atttaaacaa tctcaggaag 165960
   atatgctaca agatttagga tacggatata taggagacac tattaggact ttcttcaaag 166020
   agaacacaga aatccgtcca cgagataaaa gcattttaac tttagaagaa gtggatagtt 166080
   ttttaactac gttatcatcc gtaactaaag aatcgcatca aataaaatta ttgactgatg 166140
   tagcatctgt ttgtacatgt aatgatttaa aatgtgtagt catgcttatt gataaagatc 166200
   taaaaattaa agcgggtcct cggtacgtac ttaacgctat tagtcctaat gcctatgatg 166260
   tgtttagaaa atctaataac ttgaaagaga taatagaaaa ttcatctaaa caaaatctag 166320
   actctatatc tatttctgtt atgactccaa ttaatcccat gttagcggaa tcgtgtgatt 166380
   ctgtcaataa ggcgtttaaa aaatttccat caggaatgtt tgcggaagtc aaatacgatg 166440
   gtgaaagagt acaagttcat aaaaataata acgagtttgc cttctttagt agaaacatga 166500
   aaccagtact ctctcataaa gtggattatc tcaaagaata cataccgaaa gcatttaaaa 166560
   aagctacgtc tatcgtattg gattctgaaa ttgttcttgt agacgaacat aatgtaccgc 166620
   tcccgtttgg aagtttaggt atacacaaaa agaaagaata taaaaactct aacatgtgtt 166680
   tgttcgtgtt tgactgtttg tactttgatg gattcgatat gacggacatt ccattgtacg 166740
   aacgaagatc ttttctcaaa gatgttatgg ttgaaatacc caatagaata gtattctcag 166800
   agttgacgaa tattagtaac gagtctcagt taactgacgt attggatgat gcactaacga 166860
   gaaaattaga aggattggtc ttaaaagata ttaatggagt atacgaaccg ggaaagagaa 166920
   gatggttaaa aataaagcga gactatttga acgagggttc catggcagat tctgccgatt 166980
   tagtagtact aggtgcctac tatggtaaag gagcaaaggg tggtatcatg gcagtctttc 167040
   taatgggttg ttacgacgat gaatccggta aatggaagac ggttaccaag tgttcaggac 167100
   acgatgataa tacgttaagg gagttgcaag accaattaaa gatgattaaa attaacaagg 167160
   atcccaaaaa aattccagag tggttagtag ttaataaaat ctatattccc gattttgtag 167220
   tagaggatcc gaaacaatct cagatatggg aaatttcagg agcagagttt acatcttcca 167280
   agtcccatac cgcaaatgga atatccatta gatttcctag atttactagg ataagagagg 167340
   ataaaacgtg gaaagaatct actcatctaa acgatttagt aaacttgact aaatcttaat 167400
   agttacatac aaactgaaaa ttaaaataac actatttagt tggtggtcgc catggatggt 167460
   gttattgtat actgtctaaa cgcgctagta aaacatggcg aggaaataaa tcatataaaa 167520
   aatgatttca tgattaaacc atgttgtgaa agagtttgtg aaaaagtcaa gaacgttcac 167580
   attggcggac aatctaaaaa caatacagtg attgcagatt tgccatatat ggataatgcg 167640
   gtatccgatg tatgcaattc actgtataaa aagaatgtat caagaatatc cagatttgct 167700
   aatttgataa agatagatga cgatgacaag actcctactg gtgtatataa ttattttaaa 167760
   cctaaagatg ccattcctgt tatcatatct ataggaaagg ataaagatgt ctgtgaacta 167820
   ttaatctcat cagacatatc gtgtgcatgc gtggagttaa attcatatca cgtagccatt 167880
   cttcccatgg atgtttcctt ttttaccaaa ggaaatgcat cattgattat tctcctgttt 167940
   gatttctcta tcgatgcggc acctctctta agaagtgtaa ccgataataa tgttattata 168000
   tctagacacc agcgtctaca tgacgagctt ccgagttcca attggttcaa gttttacata 168060
   agtataaagt ccgactattg ttctatatta tatatggttg ttgatggatc tgtgatgcat 168120
   gcgatagctg ataatagaac tcacgcaatt attagcaaaa atatattaga caatactacg 168180
   attaacgatg agtgtagatg ctgttatttt gaaccacaga ttaggattct tgatagagat 168240
   gagatgctca atggatcatc gtgtgatatg aacagacatt gtattatgat gaatttacct 168300
   gatgtaggcg aatttggatc tagtatgttg gggaaatatg aacctgacat gattaagatt 168360
   gctctttcgg tggctggtaa tttaataaga aatcgagact acattcccgg gagacgagga 168420
   tatagctact acgtttacgg tatagcctct agataatttt tttaagcacg aaataaaaaa 168480
   cataatttta aaccaatcta tttcatacta ttttgtgtga tcaccatgga cataaagata 168540
   gatattagta tttctggtga taaatttacg gtgactacta ggagggaaaa tgaagaaaga 168600
   aaaaaatatc tacctctcca aaaagaaaaa actactgatg ttatcaaacc tgattatctt 168660
   gagtacgatg acttgttaga tagagatgag atgtttacta ttctagagga atattttatg 168720
   tacagaggtc tattaggcct cagaataaaa tatggacgac tctttaacga aattaaaaaa 168780
   ttcgacaatg atgcggaaga acaattcggt actatagaag aactcaagca gaaacttaga 168840
   ttaaattctg aagagggagc agataacttt atagattata taaaggtaca aaaacaggat 168900
   atcgtcaaac ttactgtata cgattgcata tctatgatag gattgtgtgc atgcgtggta 168960
   gatgtttgga gaaatgagaa actgttttct agatggaaat attgtttacg agcgattaaa 169020
   ctgtttattg atgatcacat gcttgataag ataaaatcta tactgcagaa tagactagta 169080
   tatgtggaaa tgtcatagaa agttaaaagt taatgagagc aaaaatatat aaggttgtat 169140
   tccatatttg ttattttttt ctgtaatagt tagaaaaata cattcgatgg tctatctatc 169200
   agattattat gtgttataag gtactttttc tcataataaa ctagagtatg agtaagatag 169260
   tgtttttcaa aacatataaa tctaaaattg atggatgaga tatacagcta ttaatttcga 169320
   aaatatattt taatctgata actttaaaca tggattttta atggtggttt aacgttttaa 169380
   aaaaagattt tgttattgta gtatatgata atattaaaag atggatataa agaatttgct 169440
   gactgcatgt actatttttt acattactac attggctacg gcagatatac ctacttcgtc 169500
   actgccacac gctccggtaa acggggcatg tgacgaggga gaatatcttg ataagaggca 169560
   taatcaatgt tgtaatcagt gtccacctgg agaatttgcc aaggttagat gtaatggtaa 169620
   cgataacaca aaatgtgaac gctgcccacc tcatacatat accgcaatcc ccaattactc 169680
   taatggatgt catcaatgta gaaaatgccc aacaggatca tttgataagg taaagtgtac 169740
   cggaacacag aacagtaaat gttcgtgtct tcctggttgg tattgcgcta ctgattcttc 169800
   acagactgaa gattgtcgag attgtatacc aaaaaggaga tgtccatgcg gatactttgg 169860
   tggaatagat gaacaaggaa atcctatttg taaatcgtgt tgtgttggtg aatattgcga 169920
   ctacctacgt aattatagac ttgatccatt tcctccatgc aaactatcta aatgtaatta 169980
   attatgattt tgatgataat gttaccatac attatatcgc tacttggtta gtgtattatt 170040
   cagtatgaag acctattaat aattacttat cttttgacga tcttgttata attataatat 170100
   aaaaatactt atggcatagt aactcataat tgctgacgcg ataaattcgt aataatctgt 170160
   tttgttcaaa tttttataag gaatctacag gcataaaaat aaaaatataa tttataatat 170220
   actcttacag cgcgccatca tgaataacag cagtgaattg attgctgtta ttaatggatt 170280
   tagaaatagt ggacgatttt gtgatattaa tatagttatt aatgatgaaa ggataaacgc 170340
   tcacagactc atcctatctg gagcctccga atatttttcc attctgtttt ccaataattt 170400
   tatcgattct aatgaatacg aagttaatct aagtcattta gattatcaaa gtgttaacga 170460
   tttgatcgat tacatttatg ggataccttt gagcctaact aacgataacg tgaaatatat 170520
   tctttcaacc gctgattttt tacaaattgg atctgctatt acggagtgtg aaaattacat 170580
   acttaaaaat ctttgttcta gaaactgtat cgatttctac atatacgctg ataaatataa 170640
   taacaagaaa atagaatcag cgtcgtttaa cacaatatta caaaatattt tgagactcat 170700
   caacgatgaa aactttaaat acttaacaga ggaatcaatg ataaaaattt taagcgatga 170760
   tatgttaaat ataaaaaatg aggatttcgc cccactaatt ctcattaaat ggttagagag 170820
   tactcaacaa ccatgcaccg tcgagttact taaatgcctc agaatatcat tgctttcccc 170880
   acaagttata aaatcacttt atagtcatcg actggttagt tcaatctacg aatgtataac 170940
   attcttaaac aatatagcat tcttggatga atcatttcct agataccata gcatcgagtt 171000
   gatatctatc ggtataagta attcgcatga taagatttcc ataaactgct acaatcataa 171060
   aaaaaataca tgggaaatga tatcttcacg tagatatagg tgtagtttcg cagtggccgt 171120
   cctggataat attatctata tgatgggtgg atatgatcag tccccgtata gaagttcaaa 171180
   ggttatagcg tacaatacat gtacaaattc ttggatatat gatataccag agctaaaata 171240
   tcctcgttct aattgtgggg gactggctga tgacgaatac atttattgta taggcggcat 171300
   acgcgatcag gattcatcgt tgacatctag tattgataga tggaagccat caaaaccata 171360
   ttggcagaag tatgctaaaa tgcgcgaacc aaaatgtgat atgggggttg cgatgttaaa 171420
   cggattaata tatgttatag gtggaatcgt taaaggtgac acgtgtaccg acgcactaga 171480
   gagtttatca gaagatggat ggatgaagca tcaacgtctt ccaataaaaa tgtccaatat 171540
   gtcgacgatt gttcatgatg gcaagattta tatatctgga ggttacaaca atagtagtgt 171600
   agttaatgta atatcgaatc tagtccttag ctataattcg atatatgatg aatggaccaa 171660
   attatcatca ttaaacattc ctagaattaa tcccgctcta tggtcagcgc ataataaatt 171720
   atatgtagga ggaggaatat ctgatgatgt tcgaactaat acatctgaga catacgacaa 171780
   agaaaaagat tgttggacat tggataatgg tcacgtgtta ccacgcaatt atataatgta 171840
   taaatgcgaa ccgattaaac ataaatatcc attggaaaaa acacagtaca cgaatgattt 171900
   tctaaagtat ttggaaagtt ttataggtag ttgatagaac aaaatacata attttgtaaa 171960
   aataaatcac tttttatact aatatgacac gattaccaat acttttgtta ctaatatcat 172020
   tagtatacgc tacacctttt cctcagacat ctaaaaaaat aggtgatgat gcaactctat 172080
   catgtaatcg aaataataca aatgactacg ttgttatgag tgcttggtat aaggagccca 172140
   attccattat tcttttagct gctaaaagcg acgtcttgta ttttgataat tataccaagg 172200
   ataaaatatc ttacgactct ccatacgatg atctagttac aactatcaca attaaatcat 172260
   tgactgctag agatgccggt acttatgtat gtgcattctt tatgacatca actacaaatg 172320
   acactgataa agtagattat gaagaatact ccacagagtt gattgtaaat acagatagtg 172380
   aatcgactat agacataata ctatctggat ctacacattc accggaaact agttctaaga 172440
   aacctgatta tatagataat tctaattgct cgtcggtatt cgaaatcggt acccggggat 172500
   cagcttgcat gcctgcagca attcccgagg ctgtagccga cgatggtgcg ccaggagagt 172560
   tgttgattca ttgtttgcct ccctgctgcg gtttttcacc gaagttcatg ccagtccagc 172620
   gtttttgcag cagaaaagcc gccgacttcg gtttgcggtc gcgagtgaag atccctttct 172680
   tgttaccgcc aacgcgcaat atgccttgcg aggtcgcaaa atcggcgaaa ttccatacct 172740
   gttcaccgac gacggcgctg acgcgatcaa agacgcggtg atacatatcc agccatgcac 172800
   actgatactc ttcactccac atgtcggtgt acattgagtg cagcccggct aacgtatcca 172860
   cgccgtattc ggtgatgata atcggctgat gcagtttctc ctgccaggcc agaagttctt 172920
   tttccagtac cttctctgcc gtttccaaat cgccgctttg gacataccat ccgtaataac 172980
   ggttcaggca cagcacatca aagagatcgc tgatggtatc ggtgtgagcg tcgcagaaca 173040
   ttacattgac gcaggtgatc ggacgcgtcg ggtcgagttt acgcgttgct tccgccagtg 173100
   gcgcgaaata ttcccgtgca ccttgcggac gggtatccgg ttcgttggca atactccaca 173160
   tcaccacgct tgggtggttt ttgtcacgcg ctatcagctc tttaatcgcc tgtaagtgcg 173220
   cttgctgagt ttccccgttg actgcctctt cgctgtacag ttctttcggc ttgttgcccg 173280
   cttcgaaacc aatgcctaaa gagaggttaa agccgacagc agcagtttca tcaatcacca 173340
   cgatgccatg ttcatctgcc cagtcgagca tctcttcagc gtaagggtaa tgcgaggtac 173400
   ggtaggagtt ggccccaatc cagtccatta atgcgtggtc gtgcaccatc agcacgttat 173460
   cgaatccttt gccacgcaag tccgcatctt catgacgacc aaagccagta aagtagaacg 173520
   gtttgtggtt aatcaggaac tgttcgccct tcactgccac tgaccggatg ccgacgcgaa 173580
   gcgggtagat atcacactct gtctggcttt tggctgtgac gcacagttca tagagataac 173640
   cttcacccgg ttgccagagg tgcggattca ccacttgcaa agtcccgcta gtgccttgtc 173700
   cagttgcaac cacctgttga tccgcatcac gcagttcaac gctgacatca ccattggcca 173760
   ccacctgcca gtcaacagac gcgtggttac agtcttgcgc gacatgcgtc accacggtga 173820
   tatcgtccac ccaggtgttc ggcgtggtgt agagcattac gctgcgatgg attccggcat 173880
   agttaaagaa atcatggaag taagactgct ttttcttgcc gttttcgtcg gtaatcacca 173940
   ttcccggcgg gatagtctgc cagttcagtt cgttgttcac acaaacggtg atacgtacac 174000
   ttttcccggc aataacatac ggcgtgacat cggcttcaaa tggcgtatag ccgccctgat 174060
   gctccatcac ttcctgatta ttgacccaca ctttgccgta atgagtgacc gcatcgaaac 174120
   gcagcacgat acgctggcct gcccaacctt tcggtataaa gacttcgcgc tgataccaga 174180
   cgttgcccgc ataattacga atatctgcat cggcgaactg atcgttaaaa ctgcctggca 174240
   cagcaattgc ccggctttct tgtaacgcgc tttcccacca acgctgatca attccacagt 174300
   tttcgcgatc cagactgaat gcccacaggc cgtcgagttt tttgatttca cgggttgggg 174360
   tttctacagg acgtaacatt ctagtcgagg aattcattta tagcatagaa aaaaacaaaa 174420
   tgaaattcta ctatattttt acatacatat attctaaata tgaaagtggt gattgtgact 174480
   agcgtagcat cgcttctaga catctatata ctatatagta ataccaatac tcaagactac 174540
   gaaactgata caatctctta tcatgtgggt aatgttctcg atgtcgatag ccatatgccc 174600
   ggtagttgcg atatacataa actgatcact aattccaaac ccacccgctt tttatagtaa 174660
   gtttttcacc cataaataat aaatacaata attaatttct cgtaaaagta gaaaatatat 174720
   tctaatttat tgcacggtaa ggaagtagaa tcataaagaa cagtgacgga tccccgatcc 174780
   ccgacctgca ggcatgcaag ctcgactccg gaaccaatta ctgataatgt agaagatcat 174840
   acagacaccg tcacatacac tagtgatagc attaatacag taagtgcatc atctggagaa 174900
   tccacaacag acgagactcc ggaaccaatt actgataaag aagatcatac agttacagac 174960
   actgtctcat acactacagt aagtacatca tctggaattg tcactactaa atcaaccacc 175020
   gatgatgcgg atctttatga tacgtacaat gataatgata cagtaccacc aactactgta 175080
   ggcggtagta caacctctat tagcaattat aaaaccaagg actttgtaga aatatttggt 175140
   attaccgcat taattatatt gtcggccgtg gcaattttct gtattacata ttatatatat 175200
   aataaacgtt cacgtaaata caaaacagag aacaaagtct agatttttga cttacataaa 175260
   tgtctgggat agtaaaatct atcatattga gcggaccatc tggtttagga aagacagcca 175320
   tagccaaaag actatgggaa tatatttgga tttgtggtgt cccataccac tagatttcct 175380
   cgtcctatgg aacgagaagg tgttgattac cattacgtta acagagaggc catctggaag 175440
   ggaatagccg ccggaaactt tctagaacat actgagtttt taggaaatat ttacggaact 175500
   tctaaaacag ctgtgaatac agcggctatt aataatcgta tttgtgtgat ggatctaaac 175560
   atcgacggtg ttagaagtct taaaaatacg tacctaatgc cttactcggt gtatataaga 175620
   cctacctctc ttaaaatggt tgagaccaag cttcgtcgta gaaacactga agctaacgat 175680
   gagattcatc gtcgtgtgat gttggcaaaa actgacatgg atgaggcagg tgaagccggt 175740
   ctattcgaca ctattatcat tgaagatgat gtgaatttag catatagtaa gttaattcag 175800
   atactacagg accgtattag aatgtatttt aacactaatt agagacttaa gacttaaaac 175860
   ttgataatta ataatataac tcgtttttat atgtgtctat ttcaacgtct aatgtattag 175920
   ttaaatatta aaacttacca cgtaaaactt aaaatttaaa atgatatttc attgacagat 175980
   agatcacaca ttatgaactt tcaaggactt gtgttaactg acaattgcaa aaatcaatgg 176040
   gtcgttggac cattaatagg aaaaggtgga ttcggtagta tttatactac taatgacaat 176100
   aattatgtag taaaaataga gcccaaagct aacggatcat tatttaccga acaggcattt 176160
   tatactagag tacttaaacc atccgttatc gaagaatgga aaaaatctca caatataaag 176220
   cacgtaggtc ttatcacgtg caaggcattt ggtctataca aatccattaa tgtggaatat 176280
   cgattcttgg taattaatag attaggtgca gatctagatg cggtgatcag agccaataat 176340
   aatagactac caaaaaggtc ggtgatgttg atcggaatcg aaatcttaaa taccatacaa 176400
   tttatgcacg agcaaggata ttctcacgga gatattaaag cgagtaatat agtcttagat 176460
   caaatagata agaataaatt atatctagtg gattacggat tggtttctaa attcatgtct 176520
   aatggcgaac atgttccatt tataagaaat ccaaataaaa tggataacgg tactctagaa 176580
   tttacaccta tagattcgca taaaggatac gttgtatcta gacgtggaga tctagaaaca 176640
   cttggatatt gtatgattag atggttggga ggtatcttac catggactaa gatatctgaa 176700
   acaaagaatt gtgcattagt aagtgccaca aaacagaaat atgttaacaa tactgcgact 176760
   ttgttaatga ccagtttgca atatgcacct agagaattgc tgcaatatat taccatggta 176820
   aactctttga catattttga ggaacccaat tacgacaagt ttcggcacat attaatgcag 176880
   ggtgtatatt attaagtgtg gtgtttggtc gataaaaatt aaaaaataac ttaatttatt 176940
   attgatctcg tgtgtacaac cgaaatcatg gcgatgtttt acgcacacgc tctcggtggg 177000
   tacgacgaga atcttcatgc ctttcctgga atatcatcga ctgttgccaa tgatgtcagg 177060
   aaatattctg ttgtgtcagt ttataataac aagtatgaca ttgtaaaaga caaatatatg 177120
   tggtgttaca gtcaggtgaa caagagatat attggagcac tgctgcctat gtttgagtgc 177180
   aatgaatatc tacaaattgg aaatccgatc catgatcaag aaggaaatca aatctctatc 177240
   atcacatatc gccacaaaaa ctactatgct ctaagcggaa tcgggtacga gagtctagac 177300
   ttgtgtttgg aaggagtagg gattcatcat cacgtacttg aaacaggaaa cgctgtatat 177360
   ggaaaagttc aacatgatta ttctactatc aaagagaagg ccaaagaaat gagtgcactt 177420
   agtccaggac ctatcatcga ttaccacgtc tggataggag attgtatctg tcaagttact 177480
   gctgtggacg tacatggaaa ggaaattatg aaaatgagat tcaaaaaggg tgcggtgctt 177540
   ccgatcccaa atctggtaaa agttaaactt ggggagaatg atacagaaaa tctttcttct 177600
   actatatcgg cggcaccatc gaggtaacca cctctctgga agacagcgtg aatcatgtac 177660
   tcatgaaacg tttggaaact atacgccata tgtggtctgt tgtatatgat cattttgata 177720
   ttgtgaatgg taaagaatgc tgttatgtgc atacgcattc atctaatcaa aatcctatac 177780
   cgagtactgt aaaaacaaat ttgtacatga agactatggg atcatgcatt caaatggatt 177840
   ccatggaagc tctagagtat cttagcgaac tgaaggaatc aggtggatgg agtcccagac 177900
   cagaaatgca ggaatttgaa tatccagatg gagtggaaga cactgaatca attgagagat 177960
   tggcagagga gttcttcaat agatcagaac ttcaggctgg tgaatcagtc aaatttggta 178020
   attctattaa ttgttaaaca tacatctgtt tcagctaagc aactaagaac acgtatacgg 178080
   cagcagcttc cttctatact ctcatctttt accaacacaa agggtggata tttgttcatt 178140
   ggagttgata ataatacaca caaagtattt ggattcacgg tgggttacga ctacctcaga 178200
   ctgatagaga atgatataga aaagcatatc aaaagacttc gtgttgtgca tttctgtgag 178260
   aagaaagagg acatcaagta cgcgtgtcga ttcatcaagg tatataaacc tggggatgag 178320
   actacctcga catacgtgtg cgctatcaaa gtggaaagat gctgttgtgc tgtgtttgca 178380
   gattggccag aatcatggta tatggatact aatggtatca agaagtattc tccagatgaa 178440
   tgggtgtcac atataaaatt ttaattaatg taactataga gaacaaataa taaggttgta 178500
   atatcatata gacaataact aacaattaat tagtaactgt tatctctttt ttaactaacc 178560
   aactaactat atacctatta atacatcgta attatagttc ttaacatcta ttaatcatta 178620
   attcgcttct ttaatttttt ataaactaac attgttaatt gaaaagggat aacatgttac 178680
   agaatataaa ttatatatgg atttttttaa aaaggaaata cttgactgga gtatatattt 178740
   atctcttcat tatatagcac gcgtgttttc caatttttcc acatcccata taatacagga 178800
   ttataatctc gttcgaacat acgagaaagt ggataaaaca atagttgatt ttttatctag 178860
   gttgccaaat ttattccata ttttagaata tggggaaaat attctacata tttattctat 178920
   ggatgatgct aatacgaata ttataatttt ttttctagat agagtattaa atattaataa 178980
   gaacgggtca tttatacaca atctcgggtt atcatcatcc attaatataa aagaatatgt 179040
   atatcaatta gttaataatg atcatccaga taataggata agactaatgc ttgaaaatgg 179100
   acgtagaaca agacattttt tgtcctatat atcagataca gttaatatct atatatgtat 179160
   tttaataaat catggatttt atatagatgc cgaagacagt tacggttgta cattattaca 179220
   tagatgtata tatcactata agaaatcaga atcagaatca tacaatgaat taattaagat 179280
   attgttaaat aatggatcag atgtagataa aaaagatacg tacggaaaca caccttttat 179340
   cctattatgt aaacacgata tcaacaacgt ggaattgttt gagatatgtt tagagaatgc 179400
   taatatagac tctgtagact ttaatagata tacacctctt cattatgtct catgtcgtaa 179460
   taaatatgat tttgtaaagt tattaatttc taaaggagca aatgttaatg cgcgtaataa 179520
   attcggaact actccatttt attgtggaat tatacacggt atctcgctta taaaactata 179580
   tttggaatca gacacagagt tagaaataga taatgaacat atagttcgtc atttaataat 179640
   ttttgatgct gttgaatctt tagattatct attatccaga ggagttattg atattaacta 179700
   tcgtactata tacaacgaaa catctattta cgacgctgtc agttataatg cgtataatac 179760
   gttggtctat ctattaaaca aaaatggtga ttttgagacg attactacta gtggatgtac 179820
   atgtatttcg gaagcagtcg caaacaacaa caaaataata atggaagtac tattgtctaa 179880
   acgaccatct ttgaaaatta tgatacagtc tatgatagca attactaaac ataaacagca 179940
   taatgcagat ttattgaaaa tgtgtataaa atatactgcg tgtatgaccg attatgatac 180000
   tcttatagat gtacagtcgc tacagcaata taaatggtat attttaagat gtttcgatga 180060
   aatagatatc atgaagagat gttatataaa aaataaaact gtattccaat tagttttttg 180120
   tatcaaagac attaatactt taatgagata cggtaaacat ccttctttcg tgaaatgcac 180180
   tagtctcgac gtatacggaa gtcgtgtacg taatatcata gcatctatta gatatcgtca 180240
   gagattaatt agtctattat ccaagaagct ggatcctgga gataaatggt cgtgttttcc 180300
   taacgaaata aaatataaaa tattggaaaa ctttaacgat aacgaactat ccacatatct 180360
   aaaaatctta taaacattat taaaatataa aatctaagta ggataaaatc acactacatc 180420
   attgtttcct tttagtgctc gacagtgtat actattttta acgctcataa ataaaaatga 180480
   aaacgatttc cgttgttacg ttgttatgcg tactacctgc tgttgtttat tcaacatgta 180540
   ctgtacccac tatgaataac gctaaattaa cgtctaccga aacatcgttt aatgataacc 180600
   agaaagttac gtttacatgt gatcagggat atcattcttt ggatccaaat gctgtctgtg 180660
   aaacagataa atggaaatac gaaaatccat gcaaaaaaat gtgcacagtt tctgattatg 180720
   tctctgaact atataataaa ccgctatacg aagtgaattc caccatgaca ctaagttgca 180780
   acggcgaaac aaaatatttt cgttgcgaag aaaaaaatgg aaatacttct tggaatgata 180840
   ctgttacgtg tcctaatgcg gaatgtcaac ctcttcaatt agaacacgga tcgtgtcaac 180900
   cagttaaaga aaaatactca tttggggaat atatgactat caactgtgat gttggatatg 180960
   aggttattgg tgcttcgtac ataagttgta cagctaattc ttggaatgtt attccatcat 181020
   gtcaacaaaa atgtgatatg ccgtctctat ctaacggatt aatttccgga tctacatttt 181080
   ctatcggtgg cgttatacat cttagttgta aaagtggttt tacactaacg gggtctccat 181140
   catccacatg tatcgacggt aaatggaatc ccatactccc aatatgtgta cgaactaacg 181200
   aaaaatttga tccagtggat gatggtcccg acgatgagac agatttgagc aaactctcga 181260
   aagacgttgt acaatatgaa caagaaatag aatcgttaga agcaacttat catataatca 181320
   tagtggcgtt gacaattatg ggcgtcatat ttttaatctc cgttatagta ttagtttgtt 181380
   cctgtgacaa aaataatgac caatataagt tccataaatt gctaccgtaa atataaatcc 181440
   gttaaaataa ttaataattt aataacaaac aagtatcaaa agattaaaga cttatagcta 181500
   gaatcaattg agatgtcttc ttcagtggat gttgatatct acgatgccgt tagagcattt 181560
   ttactcaggc actattataa caagagattt attgtgtatg gaagaagtaa cgccatatta 181620
   cataatatat acaggctatt tacaagatgc gccgttatac cgttcgatga tatagtacgt 181680
   actatgccaa atgaatcacg tgttaaacaa tgggtgatgg atacacttaa tggtataatg 181740
   atgaatgaac gcgatgtttc tgtaagcgtt ggcaccggaa tactattcat ggaaatgttt 181800
   ttcgattaca ataaaaatag tatcaacaat caactaatgt atgatataat taatagcgta 181860
   tctataattc tagctaatga gagatataga agcgctttta acgacgatgg tatatacatc 181920
   cgtagaaata tgattaacaa gttgtacgga tacgcatctc taactactat tggcacgatc 181980
   gctggaggtg tttgttatta tctgttgatg catctagtta gtttgtataa ataattattt 182040
   caatatacta gttaaaattt taagatttta aatgtataaa aaactaataa cgtttttatt 182100
   tgtaataggt gcattagcat cctattcgaa taatgagtac actccgttta ataaactgag 182160
   tgtaaaactc tatatagatg gagtagataa tatagaaaat tcatatactg atgataataa 182220
   tgaattggtg ttaaatttta aagagtacac aatttctatt attacagagt catgcgacgt 182280
   cggatttgat tccatagata tagatgttat aaacgactat aaaattattg atatgtatac 182340
   cattgactcg tctactattc aacgcagagg tcacacgtgt agaatatcta ccaaattatc 182400
   atgccattat gataagtacc cttatattca caaatatgat ggtgatgagc gacaatattc 182460
   tattactgca gagggaaaat gctataaagg aataaaatat gaaataagta tgatcaacga 182520
   tgatactcta ttgagaaaac atactcttaa aattggatct acttatatat ttgatcgtca 182580
   tggacatagt aatacatatt attcaaaata tgatttttaa aaatttaaaa tatattatca 182640
   cttcagtgac agtagtcaaa taacaaacaa caccatgaga tatattataa ttctcgcagt 182700
   tttgttcatt aatagtatac atgctaaaat aactagttat aagtttgaat ccgtcaattt 182760
   tgattccaaa attgaatgga ctggggatgg tctatacaat atatccctta aaaattatgg 182820
   catcaagacg tggcaaacaa tgtatacaaa tgtaccagaa ggaacatacg acatatccgc 182880
   atttccaaag aatgatttcg tatctttctg ggttaaattt gaacaaggcg attataaagt 182940
   ggaagagtat tgtacgggac tatgcgtcga agtaaaaatt ggaccaccga ctgtaacatt 183000
   gactgaatac gacgaccata tcaatttgta catcgagcat ccgtatgcta ctagaggtag 183060
   caaaaagatt cctatttaca aacgcggtga catgtgtgat atctacttgt tgtatacggc 183120
   taacttcaca ttcggagatt ctaaagaacc agtaccatat gatatcgatg actacgattg 183180
   cacgtctaca ggttgcagca tagactttgt cacaacagaa aaagtgtgcg tgacagcaca 183240
   gggagccaca gaagggtttc tcgaaaaaat tactccatgg agttcgaaag tatgtctgac 183300
   acctaaaaag agtgtatata catgcgcaat tagatccaaa gaagatgttc ccaatttcaa 183360
   ggacaaaatg gccagagtta tcaagagaaa atttaataca cagtctcaat cttatttaac 183420
   taaatttctc ggtagcacat caaatgatgt taccactttt cttagcatgc ttaacttgac 183480
   taaatattca taactaattt ttattaatga tacaaaaacg aaataaaact gcatattata 183540
   cactggttaa cgcccttata ggctctaacc attttcaaga tgaggtccct gattatagtc 183600
   cttctgttcc cctctatcat ctactccatg tctattagac gatgtgagaa gactgaagag 183660
   gaaacatggg gattgaaaat agggttgtgt ataattgcca aagatttcta tcccgaaaga 183720
   actgattgca gtgttcatct cccaactgca agtgaaggat tgataactga aggcaatgga 183780
   ttcagggata tacgaaacac cgataaatta taaaaaaagc aatgtgtccg ctgtttccgt 183840
   taataatact attttcgtaa ctggcggatt attcataaat aactctaata gcacgatcgt 183900
   ggttaacaat atggaaaaac ttgacattta taaagacaaa caatggtcga ttatagaaat 183960
   gcctatggct agggtatatc acggcattga ctcgacattt ggaatgttat attttgccgg 184020
   aggtctatcc gttaccgaac aatatggtaa tttagagaaa aacaacgaga tatcttgtta 184080
   caatcctaga acgaataagt ggtttgatat ttcatatact atttataaga tatccatatc 184140
   atcattgtgt aaactaaata acgtcttcta tgtatttagt aaggacattg gatatgtgga 184200
   aaagtatgat ggtgcatgga agttagtaca tgatcgtctc cccgctataa aggcattatc 184260
   aacttctcct tattgattga aaatgaaaat ataaatagtt tttatgtata gcagtattac 184320
   cctatagttt tattgcttac tactaacatg gatacagatg ttacaaatgt agaagatatc 184380
   ataaatgaaa tagatagaga gaaagaagaa atactaaaaa atgtagaaat tgaaaataat 184440
   aaaaacatta acaagaatca tcccaatgaa tatattagag aagcactcgt tattaatacc 184500
   agtagtaata gtgattccat tgataaagaa gttatagaat gtatcagtca cgatgtagga 184560
   atatagatca tatctactaa tttttataat cgatacaaaa cataaaaaac aactcgttat 184620
   tacatagcag gcatggaatc cttcaagtat tgttttgata acgatggcaa gaaatggatt 184680
   atcggaaata ctttatattc tggtaattca atactctata aggtcagaaa aaatttcact 184740
   agttcgttct acaattacgt aatgaagata gatcacaaat cacacaagcc attgttgtct 184800
   gaaatacgat tctatatatc tgtattggat cctttgacta tcgacaactg gacacgggaa 184860
   cgtggtataa agtatttggc tattccagat ctgtatggaa ttggagaaac cgatgattat 184920
   atgttcttcg ttataaagaa ttcgggaaga gtattcgccc caaaggatac tgaatcagtc 184980
   ttcgaagcat gcgtcactat gataaacacg ttagagttta tacactctcg aggatttacc 185040
   catggaaaaa tagaaccgag gaatatactg attagaaata aacgtctttc actaattgac 185100
   tattctagaa ctaacaaact atacaagagt ggaaactcac atatagatta caacgaggac 185160
   atgataactt caggaaatat caattatatg tgtgtagaca atcatcttgg agcaacagtt 185220
   tcaagacgag gagatttaga aatgttggga tattgcatga tagaatggtt cggtggcaaa 185280
   cttccatgga aaaacgaaag tagtataaaa gtaataaaac aaaaaaaaga atataaaaaa 185340
   tttatagcta ctttctttga ggactgtttt cctgaaggaa atgaacctct ggaattagtt 185400
   agatatatag aattagtata cacgttagat tattctcaaa ctcctaatta tgacagacta 185460
   cgtaaactgt ttatacaaga ttgaaattat attctttttt ttatagagtg tggtagtgtt 185520
   acggatatct aatattaata ttagactatc tctatcgcgc tacacgacca atatcgatta 185580
   ctatggatat cttctatgaa aggagagaat gtatttattt ctccagcgtc aatctcgtca 185640
   gtattgacaa tactgtatta tggagctaat ggatccactg ctgaacagct atcaaaatat 185700
   gtagaaacgg aggagaacac ggataaggtt agcgctcaga atatctcatt caaatccatg 185760
   aataaagtat atgggcgata ttctgccgtg tttaaagatt cctttttgag aaaaattggc 185820
   gataagtttc aaactgttga cttcactgat tgtcgcacta tagatgcaat caacaagtgt 185880
   gtagatatct ttactgaggg gaaaatcaat ccactattgg atgaaccatt gtctcctagc 185940
   aattagtgcc gtatacttta aagcaaaatg gttgacgcca ttcgaaaagg aatttaccag 186000
   tgattatccc ttttacgtat ctccgacgga aatggtagac gtaagtatga tgtctatgta 186060
   cggcaaggca tttaatcacg catctgtaaa agaatcattc ggcaactttt caatcataga 186120
   actgccatat gttggagata ctagtatgat ggtcattctt ccagacaaga ttgatggatt 186180
   agaatccata gaacaaaatc taacagatac aaattttaag aaatggtgtg actttatgga 186240
   tgctatgttt atagatgttc acattcccaa gtttaaggta acaggctcgt ataatctggt 186300
   ggatactcta gtaaagtcag gactgacaga ggtgttcggt tcaactggag attatagcaa 186360
   tatgtgtaat ttagatgtga gtgtcgacgc tatgatccac aaaacgtata tagatgtcaa 186420
   tgaagagtat acagaagcag ctgcagcaac ttctgtacta gtggcagact gtgcatcaac 186480
   aattacaaat gagttctgtg cagatcatcc gttcatctat gtgattaggc atgttgatgg 186540
   aaaaattctt ttcgttggta gatattgctc tccgacaact aattgttaac catttttttt 186600
   aaaaaaaata gaaaaaacat gtggtattag tgcaggtcgt tgttcttcca attgcaattg 186660
   gtaagatgac ggccaacttt agtacccacg tcttttcacc acagcactgt ggatgtgaca 186720
   gactgaccag tattgatgac gtcaaacaat gtttgactga atatatttat tggtcgtcct 186780
   atgcataccg caacaggcaa tgcgctggac aattgtattc cacactcctc tcttttagag 186840
   atgatgcgga attagtgttc atcgacattc gcgagctggt aaaaaatatg ccgtgggatg 186900
   atgtcaaaga ttgtacagaa atcatccgtt gttatatacc ggatgagcaa aaaaccatca 186960
   gagagatttc ggccatcatc ggactttgtg catatgctgc tacttactgg ggaggtgaag 187020
   accatcccac tagtaacagt ctgaacgcat tgtttgtgat gcttgagatg ctaaattacg 187080
   tggattataa catcatattc cggcgtatga attgatgagt tgtacatctt gacattttct 187140
   ttcttctctt ctccctttct tctcttctcc cttcctccct cttctccctt tcccagaaac 187200
   aaactttttt acccactata aaataaaatg agtatactac ctattatatt tcttcctata 187260
   tttttttatt cttcattcgt tcagactttt aacgcgtctg aatgtatcga caaagggcaa 187320
   tattttgcat cattcatgga gttagaaaac gagccagtaa tcttaccatg tcctcaaata 187380
   aatacgctat catccggata taatatatta gatattttat gggaaaaacg aggagcggat 187440
   aatgatagaa ttataccgat agataatggt agcaatatgc taattctgaa cccgacacaa 187500
   tcagactctg gtatttatat atgcattacc acgaacgaaa cctactgtga catgatgtcg 187560
   ttaaatttga caatcgtgtc tgtctcagaa tcaaatatag atcttatctc gtatccacaa 187620
   atagtaaatg agagatctac tggcgaaatg gtatgtccca atattaatgc atttattgct 187680
   agtaacgtaa acgcagatat tatatggagc ggacatcgac gccttagaaa taagagactt 187740
   aaacaacgga cacctggaat tattaccata gaagatgtta gaaaaaatga tgctggttat 187800
   tatacatgtg ttttagaata tatatacggt ggcaaaacat ataacgtaac cagaattgta 187860
   aaattagagg tacgggatag aataatacct tctactatgc aattaccaga tggcattgta 187920
   acttcaatag gtagtaattt gactattgca tgcagagtat cgttgagacc tcccacaacg 187980
   gacaccgacg tcttttggat aagtaatggt atgtattacg aagaagatga tggggacgga 188040
   aacggtagaa taagtgtagc aaataaaatc tatatgaccg ataagagacg tgttattaca 188100
   tcccggttaa acattaatcc tgtcaaggaa gaagatgcta caacgtttac gtgtatggcg 188160
   tttactattc ctagcatcag caaaacagtt actgttagta taacgtgaat gtatgttgtt 188220
   acatttccat gtcaattgag tttataagaa tttttataca ttatcttcca acaaacaatt 188280
   gacgaacgta ttgctatgat taactcccac gatactatgc atattattaa tcattaactt 188340
   gcagactata cctagtgcta ttttgacata ctcgtgttct tgtgtaattg cggtatctat 188400
   attattaaag tacgtaaatc tagctatagt tttattattt aattttagat aatataccgt 188460
   ctccttattt ttaaaaattg ccacatcctt tattaaatca tgaatgggaa tttctatgtc 188520
   atcgttagta tattgtgaac aacaagagca gatatctata ggaaagggtg gaatgcgata 188580
   cattgatcta tgtagtttta aaacacacgc gaactttgaa gaatttatat aaatcattcc 188640
   atcgatacat ccttctatgt tgacatgtat atatccagga attcttttat taatgtcagg 188700
   aaatgtataa actaaaacat tgcccgaaag cggtgcctct atctgcgtta tatccgttct 188760
   taacttacaa aatgtaacca atacctttgc atgacttgtt ttgttcggca acgttagttt 188820
   aaacttgacg aatggattaa ttacaatagc atgatccgcg catctattaa gtttttttac 188880
   tttaacgccc ttgtatgttt ttacagagac tttatctaaa tttctagtgc ttgtatgtgt 188940
   tataaatata acgggatata gaaccgaatc acctacctta gatacccaat tacattttat 189000
   cagatccaga taataaacaa attttgtcgc cctaactaat tctatattgt tatatatttt 189060
   acaattggtt atgatatcat gtaataactt ggagtctaac gcgcatcgtc gtacgtttat 189120
   acaattgtga tttagtgtag tatatctaca catgtatttt tccgcactat agtattctgg 189180
   actagtgata aaactatcgt tatatctgtc ttcaatgaac tcatcgagat attgctctct 189240
   gtcatattca tacacctgca taaactttct agacatctta caatccgtgt tattttagga 189300
   tcatatttac atatttacgg gtatatcaaa gatgttagat tagttaatgg gaatcgtcta 189360
   taataatgaa tattaaacaa ttatatgagg acttttacca caaagcatca taaaaatgag 189420
   tcgtcgtctg atttatgttt taaatatcaa ccgcaaatca actcataaaa tacaagagaa 189480
   tgaaatatat acatatttta gtcattgcaa tatagaccat acttctacag aacttgattt 189540
   tgtagttaaa aactatgatc taaacagacg acaacctgta actgggtata ctgcactaca 189600
   ctgctatttg tataataatt actttacaaa cgatgtactg aagatattat taaatcatga 189660
   cgtaaatgta acgatgaaaa ccagtagcgg acgtatgcct gtttatatat tgcttactag 189720
   atgttgtaat atttcacatg atgtagtgat agatatgata gacaaagata aaaaccactt 189780
   attacataga gactattcca acctattact agagtatata aaatctcgtt acatgttatt 189840
   gaaggaagag gatatcgatg agaacatagt atccacttta ttagataagg gaatcgatcc 189900
   taactttaaa caagacggat atacagcgtt acattattat tatttgtgtc tcgcacacgt 189960
   ttataaacta ggtgagtgta gaaaaccgat aacgataaaa aaggccaagc gaattatttc 190020
   tttgtttata caacatggag ctaatctaaa cgcgttagat aattgtggta atacaccatt 190080
   ccatttgtat cttagtattg aaatgtgtaa taatattcat atgactaaaa tgctgttgac 190140
   ttttaatccg aatttcaaaa tatgtaataa tcatggatta acgcctatac tatgttatat 190200
   aacttccgac tacatacaac acgatattct tgttatgtta atacatcact atgaaacaaa 190260
   tgttggagaa atgccgatag atgagcgtcg tatgatcgta ttcgagttta tcaaaacata 190320
   ttctacacgt ccggcagatt cgataactta tttgatgaat aggtttaaaa atataaatat 190380
   ttatacccgc tatgaaggaa agacattatt acacgtagca tgtgaatata ataatacaca 190440
   agtaatagat tatcttatac gtatcaacgg agatataaat gcgttaaccg acaataacaa 190500
   acacgctaca caactcatta tagataacaa agaaaattcc ccatatacca ttaattgttt 190560
   actgtatata cttagatata ttgtagataa gaatgtgata agatcgttgg tggatcaact 190620
   tccatctcta cctatcttcg atataaaatc atttgagaaa ttcatatcct actgtatact 190680
   tttagatgac acattttacg ataggcacgt taagaatcgc gattctaaaa cgtatcgata 190740
   cgcattttca aaatacatgt cgtttgataa atacgatggt ataataacta aatgtcacga 190800
   cgaaacaatg ttactcaaac tgtccactgt tctagacact acactatatg cagttttaag 190860
   atgtcataat tcgagaaagt taagaagata cctcaacgag ttaaaaaaat ataataacga 190920
   taagtccttt aaaatatatt ctaatattat gaatgagaga taccttaatg tatattataa 190980
   agatatgtac gtgtcaaagg tatatgataa actatttcct gttttcacag ataaaaattg 191040
   tctactaaca ttactacctt cagaaattat atacgaaata ttatacatgc tgacaattaa 191100
   cgatctttat aatatatcgt atccacctac caaagtatag ttgtattttt ctcatgcgat 191160
   gtgtgtaaaa aaactgatat tatataaata ttttagtgcc gtataataaa gatgacgatg 191220
   aaaatgatgg tacatatata tttcgtatca ttattgttat tgctattcca cagttacgcc 191280
   atagacatcg aaaatgaaat cacagaattc ttcaataaaa tgagagatac tctaccagct 191340
   aaagactcta aatggttgaa tccagcatgt atgttcggag gcacaatgaa tgatatagcc 191400
   gctctaggag agccattcag cgcaaagtgt cctcctattg aagacagtct tttatcgcac 191460
   agatataaag actatgtggt taaatgggag aggctagaaa agaatagacg gcgacaggtt 191520
   tctaataaac gtgttaaaca tggtgattta tggatagcca actatacatc taaattcagt 191580
   aaccgtaggt atttgtgcac cgtaactaca aagaatggtg actgtgttca gggtatagtt 191640
   agatctcata ttaaaaaacc tccttcatgc attccaaaaa catatgaact aggtactcat 191700
   gataagtatg gcatagactt atactgtgga attctttacg caaaacatta taataatata 191760
   acttggtata aagataataa ggaaattaat atcgacgaca ttaagtattc acaaacggga 191820
   aaggaattaa ttattcataa tccagagtta gaagatagcg gaagatacga ctgttacgtt 191880
   cattacgacg acgttagaat caagaatgat atcgtagtat caagatgtaa aatacttacg 191940
   gttataccgt cacaagacca caggtttaaa ctaatactag atccgaaaat caacgtaacg 192000
   ataggagaac ctgccaatat aacatgcact gctgtgtcaa cgtcattatt gatcgacgat 192060
   gtactgattg aatgggaaaa tccatccgga tggcttatag gattcgattt tgatgtatac 192120
   tctgttttaa ctagtagagg cggtatcacc gaggcgacct tgtactttga aaatgttact 192180
   gaagaatata taggtaatac atataaatgt cgtggacaca actattattt tgaaaaaacc 192240
   cttacaacta cagtagtatt ggagtaaata tacaatgcat ttttatatac attactgaat 192300
   tattattact gaattattat tactgaatta ttattaatta tatcgtattt gtgctataga 192360
   atggatgaag atacgcgact atctaggtat ttgtatctca ccgatagaga acatataaat 192420
   gtagactcta ttaaacagtt gtgtaaaata tcagatccta atgcatgtta tagatgtgga 192480
   tgtacggctt tacatgagta cttttataat tatagatcag tcaacggaaa atacaagtat 192540
   agatacaacg gttactatca atattattca tctagcgatt atgaaaatta taatgaatat 192600
   tattatgata gaactggtat gaacagtgag agtgataata tatcaatcaa aacagaatat 192660
   gaattctatg atgaaacaca agatcaaagt acacaactag taggttacga cattaaactc 192720
   aaaaccaatg aggatgattt tatggctatg atagatcagt gggtgtccat gattatatag 192780
   atgaatcaat taataaagta gtatatggaa gagagtctca cgtaagatgg cgggatatat 192840
   ggcaagaaca taatgatggc gtatacagta taggaaagga gtgcatagat aatatatacg 192900
   aagacaacca taccgtagac gaattctaca agatagacag cgtatcagat gtagatgacg 192960
   cggaacacat atctccgata actaaaaaac catagaatca gttgatgata atacctacat 193020
   ttctaatctt ccgtatacca tcaaatacaa aatattcgag caacaataag tattttttat 193080
   acctttaaaa ctgataaata aattttttct agtgatattt tggcaagatg agaatcctat 193140
   ttctcatcgc tttcatgtat gggtgtgttc actcatatgt taacgcggtt gaaaccaaat 193200
   gtccaaatct agacattgta acatcttctg gagaatttca ttgttcagga tgtgtggaac 193260
   atatgcctga gtttagctat atgtattggt tggcaaagga tatgaaatcg gacgaggata 193320
   ccaagtttat agaacatctg ggtgatggca tcaaagaaga tgaaaccgtt cgtaccacag 193380
   atagtggaat cgtcactcta cgtaaagtcc ttcatgtgac tgatactaat aaatttgata 193440
   attataggtt cacttgtgtc ctcgctacgc tagatggcat ttataaaaag aatgtttgct 193500
   gaagtagttg tgtgctacta tttttattta tgatataatc taatggaatt aatttgaatt 193560
   gatatttatc caatactaaa gattatatta gaatcaaatt aatcttttat acgagaaaaa 193620
   ataacgacat acgtcgtcaa caaattaaac tttttattta ttagttaact agcttataga 193680
   acttgctcat tgttatgttt ctaaaacggg tacggcatat aggacaatta tccgacgcac 193740
   cggtttctct tcgtgttcta tgccatatat tgatgcatgt tatgcaaaat atatgagtac 193800
   acgaatccaa taaaccaaag tatctatcgt tttgagtaaa caacttcata gcaaattcca 193860
   cattcttttt ctttacttac tctatacacg tcctcgtatt tatttagtat tttgatgata 193920
   tccaactcag aaatggttgt tgtattattg ggtgtatttg gagtataggt attattagct 193980
   atgtaccaat ttaccaaccc tcttaatatt gattgataat cacatcggtt atccaattaa 194040
   taactaaatt gtagtgtata tatagaccat atatgtttct atttttttga cagttacgta 194100
   tagtttcagt aagttttgat tgttgtattc ctgtatctct agataagtta gtcatatagt 194160
   cccttccggc gatacgtttt ttccaagccc gaaattgatt agccaaatgt gtatttattt 194220
   ttgtgatatt gatataatat ttcggataat gcatactgtt agtcttatat catttggttc 194280
   atctatgtat tgtaatattg ttacatgatc tatagatgat gtattgattt tggcaggatc 194340
   gaattccata tccgcgacta aacagtgaaa aaaatgtaaa tactttttaa attttaaatt 194400
   agtaaaactt ttttttattt tttatgattc caaaaatact gaatacaaag tcctaaatta 194460
   taaatatgga gatcatacta ccacaactta ttattatgta tacaaggccg gtgtaataga 194520
   tagatatata taattctatt acaccggcag acaattaccg accggtattt gtcgttacca 194580
   acataccgta taatatgtaa tatacaattc cataacccat tgacagttgt tatacatcaa 194640
   aattgcaatt cttttgatta cgatgttata agaatgtagt taattgatgt atgatgttaa 194700
   tgtgtcctct ttcctcttat aacatcgtaa tcaaaaactt ttttataata tatacctaat 194760
   aatgtgtctt aatagttctc gtgattcgtc aaacaatcat tcttataaaa tataataaag 194820
   caacgtaaaa acacataaaa ataagcgtaa ctaataagac aatggatatt tacgacgata 194880
   aaggtctaca gactattaaa ctgtttaata atgaatttga ttgtataagg aatgacatca 194940
   gagaattatt taaacatgta actgattccg atagtataca acttccgatg gaagacaatt 195000
   ctgatattat agaaaatatc agaaaaatac tatatagacg attaaaaaat gtagaatgtg 195060
   ttgacatcga taacacaata acttttatga aatacgatcc aaatgatgat aataagcgta 195120
   cgtgttctaa ttgggtaccc ttaactaata actatatgga atattgtcta gtaatatatt 195180
   tggaaacacc gatatgtgga ggcaaaataa aattatacca ccctacagga aatataaagt 195240
   cggataagga tattatgttt gcaaagactc tagactttaa atcaacgaaa gtgttaactg 195300
   gacgtaaaac aattgccgtt ctagacatat ccgtttcata taatagatca atgactacta 195360
   ttcactacaa cgacgacgtt gatatagata tacatactga taaaaatgga aaagagttat 195420
   gttattgtta tataacaata gatgatcatt acttggttga tgtggaaact ataggagtta 195480
   tagtcaatag atctggaaaa tgtctgttag taaataacca tctaggtata ggtatcgtta 195540
   aagataaacg tataagcgat agttttggag atgtatgtat ggatacaata tttgactttt 195600
   ctgaagcacg agagttattt tcattaacta atgatgataa caggaatata gcatgggaca 195660
   ctgataaact agacgatgat acagatatat ggactcccgt cacagaagat gattacaaat 195720
   ttctttctag actagtattg tatgcaaaat ctcaatcgga tactgtattt gactattatg 195780
   ttcttactgg tgatacggaa ccacccactg tattcatttt caaggtaact agattttact 195840
   ttaatatgcc gaaataaaaa atttttgtat aatatctaga ggtagaggta ttgtttagat 195900
   aaatacaaat aacatagata catcgcatac ttagcatttt tataaatata cataagacat 195960
   acactttata catttttgta aaaatactca taaaaaaatt tataaaaatt atggcacaac 196020
   catatcttgt ataggtagtt tagttcgtcg agtgaaccta taaacagata atagacaaca 196080
   cataataatg cctactaata caagcataat accgggagat gggatatatg acgttgtagt 196140
   gtttgggttt tctgaacgtt gatagtctac taatactaca tgctgacatc taatgcctgt 196200
   ataaccatga gagcatctac aatacatacc gtcaatatct ctagcgtgga tacagtcacc 196260
   gtgtaaacaa tatccatctc cctctggacc gcataatctg atagctggaa tatctgttgt 196320
   agcgtttgta atttctggca atgtcgtttc gatagcgtta ccactatcgg cgaatgatct 196380
   gattatcata gcagcgaaca acaacatcag ataatttatc aacatttttg atggattctg 196440
   tgtttatgct gtttctcagt gtgtgtttat gacaagattg ggaattttat attattaatt 196500
   cagtaatata aactaataat atattgttaa ttgtgtaaat aatataaaaa taacaataca 196560
   atattgaatg tgttgctgtt aaaaatgtat gtgttaatat aatagaataa aataaatgag 196620
   tatgatcatt ttagataacg attgatttta tcattaccgc ttcattctta tattctttgc 196680
   ttacggaacc tatatttaga aacatctact aacaattttt tatgcttgca ttattaatgg 196740
   tatgtaatat gattgattgt gtacgcaata ccaatttgtt aagtatgaat acggggtaca 196800
   aacataaatt gaaatttaac attatttatt tatgatatat atcgttatcg ttaggtctat 196860
   accatggata tctttaaaga actaatctta aaacaccctg atgaaaatgt tttgatttct 196920
   ccagtttcca ttttatctac tttatctatt ctaaatcatg gagcagctgg ttctacagct 196980
   gaacaactat caaaatatat agagaatatg aatgagaata cacccgatga caataatgat 197040
   gacatggagg tagatattcc gtattgtgcg acactagcta ccgcaaataa aatatacggt 197100
   agcgatagta tcgagttcca cgcctccttc ctacaaaaaa taaaagacga ttttcaaact 197160
   gtaaacttta ataatgctaa ccaaacaaag gaactaatca acgaatgggt taagacaatg 197220
   acaaatggta aaattaattc cttattgact agtccgctat ccattaatac tcgtatgaca 197280
   gttgttagcg ccgtccattt taaagcaatg tggaaatatc cattttctaa acatcttaca 197340
   tatacagaca agttttatat ttctaagaat atagttacca gcgttgatat gatggtgggt 197400
   accgagaata acttgcaata tgtacatatt aatgaattat tcggaggatt ctctattatc 197460
   gatattccat acgagggaaa ctctagtatg gtaattatac taccggacga catagaaggt 197520
   atatataaca tagaaaaaaa tataacagat gaaaaattta aaaaatggtg tggtatgtta 197580
   tctactaaaa gtatagactt gtatatgcca aagtttaaag tggaaatgac agaaccgtat 197640
   aatctggtac cgattttaga aaatttagga cttactaata tattcggata ttatgcagat 197700
   tttagcaaga tgtgtaatga aactatcact gtagaaaaat ttctacatac gacgtttata 197760
   gatgttaatg aggagtatac agaagcatcg gccgttacag gagtatttac gattaacttt 197820
   tcgatggtat atcgtacgaa ggtctacata aaccatccat tcatgtacat gattaaagac 197880
   accacaggac gtatactttt tatagggaaa tactgctatc cgcaataaat ataaacaaat 197940
   agacttttat aaagagtctt caacgataag tatatcgaca tactacttat gctgcgaaag 198000
   attctgaacg agaacgacta tctcaccctc ttggatcata tccgcactgc taaatactaa 198060
   atctccacta cactttttat catcttatga ggaatgattg ccttcgtgaa ataggaataa 198120
   ttagcaccag aatagctatg gattattgtg gtagagagtg cactattcta tgtcgtctac 198180
   tggatgaaga tgtgacgtac aaaaaaataa aactagaaat tgaaacgtgt cacaacttat 198240
   caaaacatat agatagacga ggaaacaatg cgctacattg ttacgtctcc aataaatgcg 198300
   atacagacat taagattgtt ctctcgcgga gtcgagagac tttgtagaaa caacgaagga 198360
   ttaactccgc taggagtata cagtaagcat agatacgtaa aatctcagat tgtgcatcta 198420
   ctgatatcca gctattcaaa ttcctctaac gaactcaagt cgaatataaa tgatttcgat 198480
   ctgtattcgg ataatatcga cttacgtctg ctaaaatacc taattgtgga taaacggata 198540
   cgtccgtcca agaatacgaa ttatgcaatc aatggtctcg gattggtgga tatatacgta 198600
   acgacgccta atccgagacc agaagtattg ctatggcttc ttaaatcaga atgttacagc 198660
   accggttacg tatttcgtac ctgtatgtac gacagtgata tgtgtaagaa ctctcttcat 198720
   tactatatat cgtctcatag agaatctcaa tctctatcca aggatgtaat taaatgtttg 198780
   atcgataaca atgtttccat ccatggcaga gacgaaggag gatctttacc catccaatac 198840
   tactggtctt tctcaaccat agatatagag attgttaaat tattattaat aaaggatgtg 198900
   gacacgtgta gagtatacga cgtcagccct atattagagg cgtattatct aaacaagcga 198960
   tttagagtaa ccccatataa tgtagacatg gaaatcgtta atcttcttat tgagagacgt 199020
   catactcttg tcgacgtaat gcgtagtatt acttcgtacg attccagaga atataaccac 199080
   tacatcatcg ataacattct aaagagattt agacaacagg atgaatccat cgtacaagcc 199140
   atactgataa actacttaca ttacggcgat atggtcgttc gatgcatgtt agataacgga 199200
   caacaactat cctctacacg actactttgt taataataat ctcgtcgatg taaacgtcgt 199260
   aaggtttatc gtggaaaata tggacacgcg gctgtaaatc acgtatcgaa caatggccgt 199320
   ctatgtatgt acggtctgat attatcgaga tttaataatt gcgggtatca ctgttatgaa 199380
   accatactga tagatgtatt tgatatacta agcaagtaca tggatgatat agatatgatc 199440
   gataactcta ctatattacg cggtcgatgt caataatata caatttgcaa agcggttatt 199500
   ggaatatgga gcgagtatca cgctcgataa tcaatacggc catccagaaa agcagttaca 199560
   gaagagaaaa caaaacgaaa tcgttgttct acaagatgtc tctccctacg acgattacta 199620
   cgtaaaaaag atactagcct actgcctatt aagggacgag tcattcgcgg aactacatag 199680
   taaattctgt ttaaacgagg actataaaag tgtatttatg aaaaatatat cattcgataa 199740
   gatagattcc atcatcgtga cataagtcgc ctcaaagaga ttcgaatctc cgacaccgac 199800
   ctgtatacgg tatcacagct atcttaaagc catacattca gacagtcaca tttcatttcc 199860
   catgtacgac gatctcatag aacagtgcca tctatcgatg gagcgtaaaa gtaaactcgt 199920
   cgacaaagca ctcaataaat tagagtctac catcggtcaa tctagactat cgtatttgcc 199980
   tccggaaatt atgcgcaata tcatctaaac agtatgttgt acggaaagaa ccattacaaa 200040
   tattatccat gatagaaaga aaatatctat atgattggag aagtaggaaa caggaacaag 200100
   acaacgatta ctacattatt aaatcatgaa gtccgtatta tactcgtata tattgtttct 200160
   ctcatgtata ataataaacg gaagagatat agcaccgcat gcaccatccg atggaaagtg 200220
   taaagacaac gaatacaaac gccataattt gtgtccggga acatacgctt ccagattatg 200280
   cgatagcaag actaacacac gatgtacgcc gtgtggttcg ggtaccttca catctcgcaa 200340
   taatcattta cccgcttgtc taagttgtaa cggaagacgc gatcgtgtaa cacgactcac 200400
   aatagaatct gtgaatgctc tcccggatat tattgtcttc tcaaaggatc atccggatgc 200460
   aaggcatgtg tttcccaaac aaaatgtgga ataggatacg gagtatccgg agacgtcatc 200520
   tgttctccgt gtggtctcgg aacatattct cacaccgtct cttccgcaga taaatgcgaa 200580
   cccgtaccca gaaatacgtt taactatatc gatgtggaaa ttaacctgta tccagttaac 200640
   gacacgtcgt gtactcggac gaccactacc ggtctcagcg aatccatctc aacgtcggaa 200700
   ctaactatta ctatgaatca taaagactgt aatcccgtat ttcgtgatgg atacttctcc 200760
   gttcttaata aggtagcgac ttcaggtttc tttacaggag aaaggtgtgc actctgaatt 200820
   tcgagattaa atgcaataac aaagattctt cctccaaaca gttaacgaaa gcaaagaatg 200880
   atactatcat gccgcattcg gagacagtaa ctctagtggg cgacatctat atactatata 200940
   gtaataccaa tactcaagac tacgaaactg atacaatctc ttatcatgtg ggtaatgttc 201000
   tcgatgtcga tagccatatg cccggtagtt gcgatataca taaactgatc actaattcca 201060
   aacccaccca ctttttatag taagtttttc acccataaat aataaataca ataattaatt 201120
   tctcgtaaaa gtagaaaata tattctaatt tattgcacgg taaggaagta gaatcataaa 201180
   gaacagtact caatcaatag caattatgaa acaatatatc gtcctggcat gcatgtgcct 201240
   ggcggcagct gctatgcctg ccagtcttca gcaatcatcc tcatcctcct cctcgtgtac 201300
   ggaagaagaa aacaaacatc atatgggaat cgatgttatt atcaaagtca caaagcaaga 201360
   ccaaacaccg accaatgata agatttgcca atccgtaacg gaaattacag agtccgagtc 201420
   agatccagat cccgaggtgg aatcagaaga tgattccaca tcagtcgagg atgtagatcc 201480
   tcctaccact tattactcca tcatcggtgg aggtctgaga atgaactttg gattcaccaa 201540
   atgtcctcag attaaatcca tctcagaatc cgctgatgga aacacagtga atgctagatt 201600
   gtccagcgtg tccccaggac aaggtaagga ctctcccgcg atcactcatg aagaagctct 201660
   tgctatgatc aaagactgtg aagtgtctat cgacatcaga tgtagcgaag aagagaaaga 201720
   cagcgacatc aagacccatc cagtactcgg gtctaacatc tctcataaga aagtgagtta 201780
   cgaagatatc atcggttcaa cgatcgtcga tacaaaatgc gtcaagaatc tagagtttag 201840
   cgttcgtatc ggagacatgt gcaaggaatc atctgaactt gaggtcaagg atggattcaa 201900
   gtatgtcgac ggatcggcat ctgaaggtgc aaccgatgat acttcactca tcgattcaac 201960
   aaaactcaaa gcgtgtgtct gaatcgataa ctctattcat ctgaaattgg atgagtaggg 202020
   ttaatcgaac gattcaggca caccacgaat taaaaaagtg taccggacac tatattccgg 202080
   tttgcaaaac aaaaatgttc ttaactacat tcacaaaaag ttacctctcg cgacttcttc 202140
   tttttctgtc tcaatagtgt gatacgatta tgacactatt cctattccta ttcctatttc 202200
   ctttcagagt atcacaaaaa tattaaacct ctttctgatg gtctcataaa aaaagtttta 202260
   caaaaatatt tttattctct ttctctcttt gatggtctca taaaaaaagt tttacaaaaa 202320
   tatttttatt ctctttctct ctttgatggt ctcataaaaa aagttttaca aaaatatttt 202380
   tattctcttt ctctctttga tggtctcata aaaaaagttt tacaaaaata tttttattct 202440
   ctttctctct ttgatggtct cataaaaaaa gttttacaaa aatattttta ttctctttct 202500
   ctctttgatg gtctcataaa aaaagtttta caaaaatatt tttattctct ttctctcttt 202560
   gatggtctca taaaaaaagt tttacaaaaa tatttttatt ctctttctct ctttgatggt 202620
   ctcataaaaa aagttttaca aaaatatttt tattctcttt ctctctttga tggtctcata 202680
   aaaaaagttt tacaaaaata tttttattct ctttctctct ttgatggtct cataaaaaaa 202740
   gttttacaaa aatattttta ttctctttct ctctttgatg gtctcataaa aaaagtttta 202800
   caaaaatatt tttattctct ttctctcttt gatggtctca taaaaaaagt tttacaaaaa 202860
   tatttttatt ctctttctct ctttgatggt ctcataaaaa agttttacaa aaatattttt 202920
   attctctttc tctctttgat ggtctcataa aaaagtttta caaaaatatt tttattctct 202980
   ttctctcttt gatggtctca taaaaaaagt tttacaaaaa tatttttatt ctctttctct 203040
   ctttgatggt ctcataa 203057
<210> 2
   <211> 190167
   <212> DNA
   <213> vaccinia virus
<220>
   <223> LIVP genome
<400> 2
   ttatgagacc atcaaagaga gaaagagaat aaaaatattt ttgtaaaact ttttttatga 60
   gaccatcaaa gagagaaaga gaataaaaat atttttgtaa aactttttta tgagaccatc 120
   aaagagagaa agagaataaa aatatttttg taaaactttt ttatgagacc atcaaagaga 180
   gaaagagaat aaaaatattt ttgtaaaact ttttttatga gaccatcaaa gagagaaaga 240
   gaataaaaat atttttgtaa aacttttttt atgagaccat caaagagaga aagagaataa 300
   aaatattttt gtaaaacttt ttttatgaga ccatcaaaga gagaaagaga ataaaaatat 360
   ttttgtaaaa ctttttttat gagaccatca aagagagaaa gagaataaaa atatttttgt 420
   aaaacttttt ttatgagacc atcaaagaga gaaagagaat aaaaatattt ttgtaaaact 480
   ttttttatga gaccatcaaa gagagaaaga gaataaaaat atttttgtaa aacttttttt 540
   atgagaccat caaagagaga aagagaataa aaatattttt gtaaaacttt ttttatgaga 600
   ccatcaaaga gagaaagaga ataaaaatat ttttgtaaaa ctttttttat gagaccatca 660
   aagagagaaa gagaataaaa atatttttgt aaaacttttt ttatgagacc atcaaagaga 720
   gaaagagaat aaaaatattt ttgtaaaact ttttttatga gaccatcaaa gagagaaaga 780
   gaataaaaat atttttgtaa aacttttttt atgagaccat cagaaagagg tttaatattt 840
   ttgtgatact ctgaaaggaa ataggaatag gaataggaat agtgtcataa tcgtatcaca 900
   ctattgagac agaaaaagaa gaagtcgcga gaggtaactt tttgtgaatg tagttaagaa 960
   catttttgtt ttgcaaaccg gaatatagtg tccggtacac ttttttaatt cgtggtgtgc 1020
   ctgaatcgtt cgattaaccc tactcatcca atttcagatg aatagagtta tcgattcaga 1080
   cacacgcttt gagttttgtt gaatcgatga gtgaagtatc atcggttgca ccttcagatg 1140
   ccgatccgtc gacatacttg aatccatcct tgacctcaag ttcagatgat tccttgcaca 1200
   tgtctccgat acgaacgcta aactctagat tcttgacgca ttttgtatcg acgatcgttg 1260
   aaccgatgat atcttcgtaa ctcactttct tatgagagat gttagacccg agtactggat 1320
   gggtcttgat gtcgctgtct ttctcttctt cgctacatct gatgtcgata gacacttcac 1380
   agtctttgat catagcaaga gcttcttcat gagtgatcgc gggagagtcc ttaccttgtc 1440
   ctggggacac gctggacaat ctagcattca ctgtgtttcc atcagcggat tctgagatgg 1500
   atttaatctg aggacatttg gtgaatccaa agttcattct cagacctcca ccgatgatgg 1560
   agtaataagt ggtaggagga tctacatcct cgactgatgt ggaatcatct tctgattcca 1620
   cctcgggatc tggatctgac tcggactctg taatttccgt tacggattgg caaatcttat 1680
   cattggtcgg tgtttggtct tgctttgtga ctttgataat aacatcgatt cccatatgat 1740
   gtttgttttc ttcttccgta cacgaggagg aggatgagga tgattgctga agactggcag 1800
   gcatagcagc tgccgccagg cacatgcatg ccaggacgat atattgtttc ataattgcta 1860
   ttgattgagt actgttcttt atgattctac ttccttaccg tgcaataaat tagaatatat 1920
   tttctacttt tacgagaaat taattattgt atttattatt tatgggtgaa aaacttacta 1980
   taaaaagtgg gtgggtttgg aattagtgat cagtttatgt atatcgcaac taccgggcat 2040
   atggctatcg acatcgagaa cattacccac atgataagag attgtatcag tttcgtagtc 2100
   ttgagtattg gtattactat atagtatata gatgtcgccc actagagtta ctgtctccga 2160
   atgcggcatg atagtatcat tctttgcttt cgttaactgt ttggaggaag aatctttgtt 2220
   attgcattta atctcgaaat tcagagtgca cacctttctc ctgtaaagaa acctgaagtc 2280
   gctaccttat taagaacgga gaagtatcca tcacgaaata cgggattaca gtctttatga 2340
   ttcatagtaa tagttagttc cgacgttgag atggattcgc tgagaccggt agtggtcgtc 2400
   cgagtacacg acgtgtcgtt aactggatac aggttaattt ccacatcgat atagttaaac 2460
   gtatttctgg gtacgggttc gcatttatct gcggaagaga cggtgtgaga atatgttccg 2520
   agaccacacg gagaacagat gacgtctccg gatactccgt atcctattcc acattttgtt 2580
   tgggaaacac atgccttgca tccggatgat cctttgagaa gacaataata tccgggagag 2640
   cattcacaga ttctattgtg agtcgtgtta cacgatcgcg tcttccgtta caacttagac 2700
   aagcgggtaa atgattattg cgagatgtga aggtacccga accacacggc gtacatcgtg 2760
   tgttagtctt gctatcgcat aatctggaag cgtatgttcc cggacacaaa ttatggcgtt 2820
   tgtattcgtt gtctttacac tttccatcgg atggtgcatg cggtgctata tctcttccgt 2880
   ttattattat acatgagaga aacaatatat acgagtataa tacggacttc atgatttaat 2940
   aatgtagtaa tcgttgtctt gttcctgttt cctacttctc caatcatata gatattttct 3000
   ttctatcatg gataatattt gtaatggttc tttccgtaca acatactgtt tagatgatat 3060
   tgcgcataat ttccggaggc aaatacgata gtctagattg accgatggta gactctaatt 3120
   tattgagtgc tttgtcgacg agtttacttt tacgctccat cgatagatgg cactgttcta 3180
   tgagatcgtc gtacatggga aatgaaatgt gactgtctga atgtatggct ttaagatagc 3240
   tgtgataccg tatacaggtc ggtgtcggag attcgaatct ctttgaggcg acttatgtca 3300
   cgatgatgga atctatctta tcgaatgata tatttttcat aaatacactt ttatagtcct 3360
   cgtttaaaca gaatttacta tgtagttccg cgaatgactc gtcccttaat aggcagtagg 3420
   ctagtatctt ttttacgtag taatcgtcgt agggagagac atcttgtaga acaacgattt 3480
   cgttttgttt tctcttctgt aactgctttt ctggatggcc gtattgatta tcgagcgtga 3540
   tactcgctcc atattccaat aaccgctttg caaattgtat attattgaca tcgaccgcgt 3600
   aatatagtag agttatcgat catatctata tcatccatgt acttgcttag tatatcaaat 3660
   acatctatca gtatggtttc ataacagtga tacccgcaat tattaaatct cgataatatc 3720
   agaccgtaca tacatagacg gccattgttc gatacgtgat ttacagccgc gtgtccatat 3780
   tttccacgat aaaccttacg acgtttacat cgacgagatt attattaaca aagtagtcgt 3840
   gtagaggata gttgttgtcc gttatctaac atgcatcgaa cgaccatatc gccgtaatgt 3900
   aagtagttta tcagtatggc ttgtacgatg gattcatcct gttgtctaaa tctctttaga 3960
   atgttatcga tgatgtagtg gttatattct ctggaatcgt acgaagtaat actacgcatt 4020
   acgtcgacaa gagtatgacg tctctcaata agaagattaa cgatttccat gtctacatta 4080
   tatggggtta ctctaaatcg cttgtttaga taatacgcct ctaatatagg gctgacgtcg 4140
   tatactctac acgtgtccac atcctttatt aataataatt taacaatctc tatatctatg 4200
   gttgagaaag accagtagta ttggatgggt aaagatcctc cttcgtctct gccatggatg 4260
   gaaacattgt tatcgatcaa acatttaatt acatccttgg atagagattg agattctcta 4320
   tgagacgata tatagtaatg aagagagttc ttacacatat cactgtcgta catacaggta 4380
   cgaaatacgt aaccggtgct gtaacattct gatttaagaa gccatagcaa tacttctggt 4440
   ctcggattag gcgtcgttac gtatatatcc accaatccga gaccattgat tgcataattc 4500
   gtattcttgg acggacgtat ccgtttatcc acaattaggt attttagcag acgtaagtcg 4560
   atattatccg aatacagatc gaaatcattt atattcgact tgagttcgtt agaggaattt 4620
   gaatagctgg atatcagtag atgcacaatc tgagatttta cgtatctatg cttactgtat 4680
   actcctagcg gagttaatcc ttcgttgttt ctacaaagtc tctcgactcc gcgagagaac 4740
   aatcttaatg tctgtatcgc atttattgga gacgtaacaa tgtagcgcat tgtttcctcg 4800
   tctatctata tgttttgata agttgtgaca cgtttcaatt tctagtttta tttttttgta 4860
   cgtcacatct tcatccagta gacgacatag aatagtgcac tctctaccac aataatccat 4920
   agctattctg gtgctaatta ttcctatttc acgaaggcaa tcattcctca taagatgata 4980
   aaaagtgtag tggagattta gtatttagca gtgcggatat gatccaagag ggtgagatag 5040
   tcgttctcgt tcagaatctt tcgcagcata agtagtatgt cgatatactt atcgttgaag 5100
   actctttata aaagtctatt tgtttatatt tattgcggat agcagtattt ccctataaaa 5160
   agtatacgtc ctgtggtgtc tttaatcatg tacatgaatg gatggtttat gtagaccttc 5220
   gtacgatata ccatcgaaaa gttaatcgta aatactcctg taacggccga tgcttctgta 5280
   tactcctcat taacatctat aaacgtcgta tgtagaaatt tttctacagt gatagtttca 5340
   ttacacatct tgctaaaatc tgcataatat ccgaatatat tagtaagtcc taaattttct 5400
   aaaatcggta ccagattata cggttctgtc atttccactt taaactttgg catatacaag 5460
   tctatacttt tagtagataa cataccacac cattttttaa atttttcatc tgttatattt 5520
   ttttctatgt tatatatacc ttctatgtcg tccggtagta taattaccat actagagttt 5580
   ccctcgtatg gaatatcgat aatagagaat cctccgaata attcattaat atgtacatat 5640
   tgcaagttat tctcggtacc caccatcata tcaacgctgg taactatatt cttagaaata 5700
   taaaacttgt ctgtatatgt aagatgttta gaaaatggat atttccacat tgctttaaaa 5760
   tggacggcgc taacaactgt catacgagta ttaatggata gcggactagt caataaggaa 5820
   ttaattttac catttgtcat tgtcttaacc cattcgttga ttagttcctt tgtttggtta 5880
   gcattattaa agtttacagt ttgaaaatcg tcttttattt tttgtaggaa ggaggcgtgg 5940
   aactcgatac tatcgctacc gtatatttta tttgcggtag ctagtgtcgc acaatacgga 6000
   atatctacct ccatgtcatc attattgtca tcgggtgtat tctcattcat attctctata 6060
   tattttgata gttgttcagc tgtagaacca gctgctccat gatttagaat agataaagta 6120
   gataaaatgg aaactggaga aatcaaaaca ttttcatcag ggtgttttaa gattagttct 6180
   ttaaagatat ccatggtata gacctaacga taacgatata tatcataaat aaataatgtt 6240
   aaatttcaat ttatgtttgt accccgtatt catacttaac aaattggtat tgcgtacaca 6300
   atcaatcata ttacatacca ttaataatgc aagcataaaa aattgttagt agatgtttct 6360
   aaatataggt tccgtaagca aagaatataa gaatgaagcg gtaatgataa aatcaatcgt 6420
   tatctaaaat gatcatactc atttatttta ttctattata ttaacacata catttttaac 6480
   agcaacacat tcaatattgt attgttattt ttatattatt tacacaatta acaatatatt 6540
   attagtttat attactgaat taataatata aaattcccaa tcttgtcata aacacacact 6600
   gagaaacagc ataaacacag aatccatcaa aaatgttgat aaattatctg atgttgttgt 6660
   tcgctgctat gataatcaga tcattcgccg atagtggtaa cgctatcgaa acgacattgc 6720
   cagaaattac aaacgctaca acagatattc cagctatcag attatgcggt ccagagggag 6780
   atggatattg tttacacggt gactgtatcc acgctagaga tattgacggt atgtattgta 6840
   gatgctctca tggttataca ggcattagat gtcagcatgt agtattagta gactatcaac 6900
   gttcagaaaa cccaaacact acaacgtcat atatcccatc tcccggtatt atgcttgtat 6960
   tagtaggcat tattatgtgt tgtctattat ctgtttatag gttcactcga cgaactaaac 7020
   tacctataca agatatggtt gtgccataat ttttataaat ttttttatga gtatttttac 7080
   aaaaatgtat aaagtgtatg tcttatgtat atttataaaa atgctaagta tgcgatgtat 7140
   ctatgttatt tgtatttatc taaacaatac ctctacctct agatattata caaaaatttt 7200
   ttatttcggc atattaaagt aaaatctagt taccttgaaa atgaatacag tgggtggttc 7260
   cgtatcacca gtaagaacat aatagtcaaa tacagtatcc gattgagatt ttgcatacaa 7320
   tactagtcta gaaagaaatt tgtaatcatc ttctgtgacg ggagtccata tatctgtatc 7380
   atcgtctagt ttatcagtgt cccatgctat attcctgtta tcatcattag ttaatgaaaa 7440
   taactctcgt gcttcagaaa agtcaaatat tgtatccata catacatctc caaaactatc 7500
   gcttatacgt ttatctttaa cgatacctat acctagatgg ttatttacta acagacattt 7560
   tccagatcta ttgactataa ctcctatagt ttccacatca accaagtaat gatcatctat 7620
   tgttatataa caataacata actcttttcc atttttatca gtatgtatat ctatatcaac 7680
   gtcgtcgttg tagtgaatag tagtcattga tctattatat gaaacggata tgtctagaac 7740
   ggcaattgtt ttacgtccag ttaacacttt cgttgattta aagtctagag tctttgcaaa 7800
   cataatatcc ttatccgact ttatatttcc tgtagggtgg tataatttta ttttgcctcc 7860
   acatatcggt gtttccaaat atattactag acaatattcc atatagttat tagttaaggg 7920
   tacccaatta gaacacgtac gcttattatc atcatttgga tcgtatttca taaaagttat 7980
   tgtgttatcg atgtcaacac attctacatt ttttaatcgt ctatatagta tttttctgat 8040
   attttctata atatcagaat tgtcttccat cggaagttgt atactatcgg aatcagttac 8100
   atgtttaaat aattctctga tgtcattcct tatacaatca aattcattat taaacagttt 8160
   aatagtctgt agacctttat cgtcgtaaat atccattgtc ttattagtta cgcttatttt 8220
   tatgtgtttt tacgttgctt tattatattt tataagaatg attgtttgac gaatcacgag 8280
   aactattaag acacattatt aggtatatat tataaaaaag tttttgatta cgatgttata 8340
   agaggaaaga ggacacatta acatcataca tcaattaact acattcttat aacatcgtaa 8400
   tcaaaagaat tgcaattttg atgtataaca actgtcaatg ggttatggaa ttgtatatta 8460
   catattatac ggtatgttgg taacgacaaa taccggtcgg taattgtctg ccggtgtaat 8520
   agaattatat atatctatct attacaccgg ccttgtatac ataataataa gttgtggtag 8580
   tatgatctcc atatttataa tttaggactt tgtattcagt atttttggaa tcataaaaaa 8640
   taaaaaaaag ttttactaat ttaaaattta aaaagtattt acattttttt cactgtttag 8700
   tcgcggatat ggaattcgat cctgccaaaa tcaatacatc atctatagat catgtaacaa 8760
   tattacaata catagatgaa ccaaatgata taagactaac agtatgcatt atccgaaata 8820
   ttatatcaat atcacaaaaa taaatacaca tttggctaat caatttcggg cttggaaaaa 8880
   acgtatcgcc ggaagggact atatgactaa cttatctaga gatacaggaa tacaacaatc 8940
   aaaacttact gaaactatac gtaactgtca aaaaaataga aacatatatg gtctatatat 9000
   acactacaat ttagttatta attggataac cgatgtgatt atcaatcaat attaagaggg 9060
   ttggtaaatt ggtacatagc taataatacc tatactccaa atacacccaa taatacaaca 9120
   accatttctg agttggatat catcaaaata ctaaataaat acgaggacgt gtatagagta 9180
   agtaaagaaa aagaatgtgg aatttgctat gaagttgttt actcaaaacg atagatactt 9240
   tggtttattg gattcgtgta ctcatatatt ttgcataaca tgcatcaata tatggcatag 9300
   aacacgaaga gaaaccggtg cgtcggataa ttgtcctata tgccgtaccc gttttagaaa 9360
   cataacaatg agcaagttct ataagctagt taactaataa ataaaaagtt taatttgttg 9420
   acgacgtatg tcgttatttt ttctcgtata aaagattaat ttgattctaa tataatcttt 9480
   agtattggat aaatatcaat tcaaattaat tccattagat tatatcataa ataaaaatag 9540
   tagcacacaa ctacttcagc aaacattctt tttataaatg ccatctagcg tagcgaggac 9600
   acaagtgaac ctataattat caaatttatt agtatcagtc acatgaagga ctttacgtag 9660
   agtgacgatt ccactatctg tggtacgaac ggtttcatct tctttgatgc catcacccag 9720
   atgttctata aacttggtat cctcgtccga tttcatatcc tttgccaacc aatacatata 9780
   gctaaactca ggcatatgtt ccacacatcc tgaacaatga aattctccag aagatgttac 9840
   aatgtctaga tttggacatt tggtttcaac cgcgttaaca tatgagtgaa cacacccata 9900
   catgaaagcg atgagaaata ggattctcat cttgccaaaa tatcactaga aaaaatttat 9960
   ttatcagttt taaaggtata aaaaatactt attgttgctc gaatattttg tatttgatgg 10020
   tatacggaag attagaaatg taggtattat catcaactga ttctatggtt ttatgtattc 10080
   tatcatgttt cactattgcg ttggaaataa tatcatatgc ttccacatat attttatttt 10140
   gttttaactc ataatactca cgtaattctg gattattggc atatctatga ataattttag 10200
   ctccatgatc agtaaatatt aatgagaaca tagtattacc acctaccatt atttttttca 10260
   tctcattcaa ttcttaattg caaagatcta tataatcatt atagcgttga cttatggact 10320
   ctggaatctt agacgatgta cagtcatcta taatcatggc atatttaata cattgtttta 10380
   tagcatagtc gttatctacg atgttagata tttctctcaa tgaatcaatc acacaatcta 10440
   atgtaggttt atgacataat agcattttca gcagttcaat gtttttagat tcgttgatgg 10500
   caatggctat acatgtatat ccgttatttg atctaatgtt gacatctgaa ccggattcta 10560
   gcagtaaaga tactagagat tgtttattat atctaacagc cttgtgaaga agtgtttctc 10620
   ctcgtttgtc aatcatgtta atgtctttaa gataaggtag gcaaatgttt atagtactaa 10680
   gaattgggca agcataagac atgtcacaaa gacccttttt tgtatgtata agtgtaaaaa 10740
   ttataacatc catagttgga tttacatagg tgtccaatcg ggatctctcc atcatcgaga 10800
   taattgatgg catctccctt ccttttttag tagatatttc atcgtgtaag aatcaatatt 10860
   aatatttcta aagtatccgt gtatagcctc tttatttacc acagctccat attccactag 10920
   agggatatcg ccgaatgtca tatactcaat tagtatatgt tggaggacat ccgagttcat 10980
   tgttttcaat atcaaagaga tggtttcctt atcatttctc catagtggta caatactaca 11040
   cattattccg tgcggctttc cattttccaa aaacaatttg accaaatcta aatctacatc 11100
   tttattgtat ctataatcac tatttagata atcagccata attcctcgag tgcaacatgt 11160
   tagatcgtct atatatgaat aagcagtgtt atctattcct ttcattaaca atttaacgat 11220
   gtctatatct atatgagatg acttaatata atattgaaga gctgtacaat agtttttatc 11280
   tataaaagac ggcttgattc cgtgattaat tagacattta acaacttccg gacgcacata 11340
   tgctctcgta tccgactctg aatacagatg agagatgata tacagatgca atacggtacc 11400
   gcaatttcgt agttgataat catcatacgc gtatcagtac tcgtcctcat aaagaacact 11460
   gcagccattt tctatgaaca aatcaataat tttagaaaca ggatcattgt cattacataa 11520
   ttttctataa ctgaacgatg gttttcacat ttaacactca agtcaaatcc atgttctacc 11580
   aacaccttta tcaagtcaac gtctacattt ttggatttca tatagctgaa tatattaaag 11640
   ttatttatgt tgctaaatcc agtggcttct agtagagcca tcgctatatc cttattaact 11700
   ttaacatgtc tactatttgt gtattcttct aatggggtaa gctgtctcca atttttgcgt 11760
   aatggattag tgccactgtc tagtagtagt ttgacgacct cgacattatt acaatgctca 11820
   ttaaaaaggt atgcgtgtaa agcattattc ttgaattggt tcctggtatc attaggatct 11880
   ctgtctttca acatctgttt aagttcatca agagccacct cctcattttc caaatagtca 11940
   aacattttga ctgaatgagc tactgtgaac tctatacacc cacacaacta atgtcattaa 12000
   atatcatgtc aaaaacttgt acaattatta ataaaaataa tttagtgttt aaattttacc 12060
   agttccagat tttacacctc cgttaatacc tccattaacc ccactggacg atcctcctcc 12120
   ccacattcca ccgccaccag atgtataagt tttagatcct ttattactac catcatgtcc 12180
   atggataaag acactccaca tgccgccact acccccttta gaagacatat taataagact 12240
   taaggacaag tttaacaata aaattaatca cgagtaccct actaccaacc tacactatta 12300
   tatgattata gtttctattt ttacagtacc ttgactaaag tttctagtca caagagcaat 12360
   actaccaacc tacactatta tatgattata gtttctattt ttataggaac gcgtacgaga 12420
   aaatcaaatg tctaatttct aacggtagtg ttgataaacg attgttatcc gcggatacct 12480
   cctctatcat gtcgtctatt ttcttacttt gttctattaa cttattagca ttatatatta 12540
   tttgattata aaacttatat tgcttattag cccaatctgt aaatatcgga ttattaacat 12600
   atcgtttctt tgtaggttta tttaacatgt acatcactgt aagcatgtcc ttaccattta 12660
   ttttaatttg acgcatatcc gcaatttctt tttcgcagtc ggttataaat tctatatatg 12720
   atggatacat gctacatgtg tacttataat cgactaatat gaagtacttg atacatattt 12780
   tcagtaacga tttattatta ccacctatga ataagtacct gtgatcgtct aggtaatcaa 12840
   ctgttttctt aatacattcg atggttggta atttactcag aataatttcc aatatcttaa 12900
   tatataattc tgctatttct gggatatatt tatctgccag tataacacaa atagtaatac 12960
   atgtaaaccc atattttgtt attatattaa tgtctgcgcc attatctatt aaccattcta 13020
   ctaggctgac actatgcgac tcaatacaat gataaagtat actacatcca tgtttatcta 13080
   ttttgtttat atcatcaata tacggcttac aaagttttag tatcgataac acatccaact 13140
   cacgcataga gaaggtaggg aataatggca taatatttat taggttatca tcattgtcat 13200
   tatctacaac taagtttcca ttttttaaaa tatactcgac aactttagga tctctattgc 13260
   caaatttttg aaaatattta tttatatgct taaatctata taatgtagct ccttcatcaa 13320
   tcatacattt aataacattg atgtatactg tatgataaga tacatattct aacaatagat 13380
   cttgtataga atctgtatat cttttaagaa ttgtggatat taggatatta ttacataaac 13440
   tattacacaa ttctaaaata taaaacgtat cacggtcgaa taatagttga tcaactatat 13500
   aattatcgat tttgtgattt ttcttcctaa actgtttacg taaatagtta gatagaatat 13560
   tcattagttc atgaccacta tagttactat cgaataacgc gtcaaatatt tcccgtttaa 13620
   tatcgcattt gtcaagataa taatagagtg tggtatgttc acgataagta taataacgca 13680
   tctctttttc gtgtgaaatt aaatagttta ttacgtccaa agatgtagca taaccatctt 13740
   gtgacctagt aataatataa taatagagaa ctgttttacc cattctatca tcataatcag 13800
   tggtgtagtc gtaatcgtaa tcgtctaatt catcatccca attataatat tcaccagcac 13860
   gtctaatctg ttctattttg atcttgtatc catactgtat gttgctacat gtaggtattc 13920
   ctttatccaa taatagttta aacacatcta cattgggatt tgatgttgta gcgtattttt 13980
   ctacaatatt aataccattt ttgatactat ttatttctat acctttcgaa attagtaatt 14040
   tcaataagtc tatattgatg ttatcagaac atagatattc gaatatatca aaatcattga 14100
   tatttttata gtcgactgac gacaataaca aaatcacaac atcgtttttg atattattat 14160
   ttttcttggt aacgtatgcc tttaatggag tttcaccatc atactcatat aatggatttg 14220
   caccactttc tatcaatgat tgtgcactgc tggcatcgat gttaaatgtt ttacaactat 14280
   catagagtat cttatcgtta accatgattg gttgttgatg ctatcgcatt ttttggtttc 14340
   tttcatttca gttatgtatg gatttagcac gtttgggaag catgagctca tatgatttca 14400
   gtactgtagt gtcagtacta ttagtttcaa taagatcaat ctctagatct atagaatcaa 14460
   aacacgatag gtcagaagat aatgaatatc tgtaggcttc ttgttgtact gtaacttctg 14520
   gttttgttag atggttgcat cgtgctttaa cgtcaatggt acaaatttta tcctcgcttt 14580
   gtgtatcata ttcgtcccta ctataaaatt gtatattcag attatcatgc gatgtgtata 14640
   cgctaacggt atcaataaac ggagcacacc atttagtcat aaccgtaatc caaaaatttt 14700
   taaagtatat cttaacgaaa gaagttgtgt cattgtctac ggtgtatggt actagatcct 14760
   cataagtgta tatatctaga gtaatgttta atttattaaa tggttgataa tatggatcct 14820
   cgtgacaatt tccgaagatg gaaataagac ataaacacgc aataaatcta attgcggaca 14880
   tggttactcc ttaaaaaaat acgaataatc accttggcta tttagtaagt gtcatttaac 14940
   actatactca tattaatcca tggactcata atctctatac gggattaacg gatgttctat 15000
   atacggggat gagtagttct cttctttaac tttatacttt ttactaatca tatttagact 15060
   gatgtatggg taatagtgtt tgaagagctc gttctcatca tcagaataaa tcaatatctc 15120
   tgtttttttg ttatacagat gtattacagc ctcatatatt acgtaataga acgtgtcatc 15180
   taccttatta actttcaccg catagttgtt tgcaaatacg gttaatcctt tgacctcgtc 15240
   gatttccgac caatctgggc gtataatgaa tctaaacttt aattgcttgt aatcattcga 15300
   aataattttt agtttgcatc cgtagttatc ccctttatgt aactgtaaat ttctcaacgc 15360
   gatatctcca ttaataatga tgtcgaattc gtgctgtata cccatactga atggatgaac 15420
   gaataccgac ggcgttaata gtaatttact ttttcatctt tacatattgg gtactagttt 15480
   tactatcata agtttataaa ttccacaagc tactatggaa taagccaacc atcttagtat 15540
   accacacatg tcttaaagtt tattaattaa ttacatgttg ttttatatat atcgctacga 15600
   atttaaagag aaattagttt aggaagaaaa attatctatc tacatcatca cgtctctgta 15660
   ttctacgata gagtgctact ttaagatgcg acagatccgt gtcatcaaat atatactcca 15720
   ttaaaatgat tattccggca gcgaacttga tattggatat atcacaacct ttgttaatat 15780
   ctacgacaat agacagcagt cccatggttc cataaacagt gagtttatct ttctttgaag 15840
   agatattttg tagagatctt ataaaactgt cgaatgacat cgcatttata tctttagcta 15900
   aatcgtatat gttaccatcg taatatctaa ccgcgtctat cttaaacgtt tccatcgctt 15960
   taaagacgtt tccgatagat ggtctcattt catcagtcat actgagccaa caaatataat 16020
   cgtgtataac atctttgata gaatcagact ctaaagaaaa cgaatcggct ttattatacg 16080
   cattcatgat aaacttaatg aaaaatgttt ttcgttgttt aagttggatg aatagtatgt 16140
   cttaataatt gttattattt cattaattaa tatttagtaa cgagtacact ctataaaaac 16200
   gagaatgaca taactaatca taactagtta tcaaagtgtc taggacgcgt aattttcata 16260
   tggtatagat cctgtaagca ttgtctgtat tctggagcta ttttctctat cgcattagtg 16320
   agttcagaat atgttataaa tttaaatcga ataacgaaca taactttagt aaagtcgtct 16380
   atattaactc ttttattttc tagccatcgt aataccatgt ttaagatagt atattctcta 16440
   gttactacga tctcatcgtt gtctagaata tcacatactg aatctacatc caattttaga 16500
   aattggtctg tgttacatat ctcttctata ttattgttga tgtattgtcg tagaaaacta 16560
   ttacgtagac cattttcttt ataaaacgaa tatatagtac tccaattatc tttaccgata 16620
   tatttgcaca cataatccat tctctcaatc actacatctt taagattttc gttgttaaga 16680
   tatttggcta aactatataa ttctattaga tcatcaacag aatcagtata tatttttcta 16740
   gatccaaaga cgaactcttt ggcgtcctct ataatattcc cagaaaagat attttcgtgt 16800
   tttagtttat cgagatctga tctgttcata tacgccatga ttgtacggta cgttatgata 16860
   accgcataaa ataaaaatcc attttcattt ttaaccaata ctattcataa ttgagattga 16920
   tgtaatactt tgttactttg aacgtaaaga cagtacacgg atccgtatct ccaacaagca 16980
   cgtagtaatc aaatttggtg ttgttaaact tcgcaatatt catcaattta gatagaaact 17040
   tatactcatc atctgtttta ggaatccatg tattattacc actttccaac ttatcattat 17100
   cccaggctat gtttcgtcca tcatcgttgc gcagagtgaa taattctttt gtattcggta 17160
   gttcaaatat atgatccatg catagatcgg caaagctatt gtagatgtga tttttcctaa 17220
   atctaatata aaactcgttt actagcaaac actttcctga tttatcgacc aagacacata 17280
   tggtttctaa atctatcaag tggtggggat ccatagttat gacgcagtaa catatattat 17340
   tacattcttg actgtcgcta atatctaaat atttattgtt atcgtattgg attctgcata 17400
   tagatggctt gtatgtcaaa gatatagaac acataaccaa tttatagtcg cgctttacat 17460
   tctcgaatct aaagttaaga gatttagaaa acattatatc ctcggatgat gttatcactg 17520
   tttctggagt aggatatatt aaagtcttta cagatttcgt ccgattcaaa taaatcacta 17580
   aataatatcc cacattatca tctgttagag tagtatcatt aaatctatta tattttatga 17640
   aagatatatc actgctcacc tctatatttc gtacattttt aaactgtttg tataatatct 17700
   ctctgataca atcagatata tctattgtgt cggtagacga taccgttaca tttgaattaa 17760
   tggtgttcca ttttacaact tttaacaagt tgaccaattc atttctaata gtatcaaact 17820
   ctccatgatt aaatatttta atagtatcca ttttatatca ctacggacac aaagtagctg 17880
   acataaacca ttgtataatt tttatgtttt atgtttatta gcgtacacat tttggaagtt 17940
   ccggcttcca tgtatttcct ggagagcaag tagatgatga ggaaccagat agtttatatc 18000
   cgtacttgca cttaaagtct acattgtcgt tgtatgagta tgatctttta aacccgctag 18060
   acaagtatcc gtttgatatt gtaggatgtg gacatttaac aatctgacac gtgggtggat 18120
   cggaccattc tcctcctgaa cacaggacac tagagttacc aatcaacgaa tatccactat 18180
   tgcaactata agttacaacg ctcccatcgg tataaaaatc ctcgtatccg ttatgtcttc 18240
   cgttggatat agatggaggg gattggcatt taacagattc acaaataggt gcctcgggat 18300
   tccataccat agatccagta gatcctaatt cacaatacga tttagattca ccgatcaact 18360
   gatatccgct attacaagag tacgttatac tagagccaaa gtctactcca ccaatatcaa 18420
   gttggccatt atcgatatct cgaggcgatg ggcatctccg tttaatacat tgattaaaga 18480
   gtgtccatcc agtacctgta catttagcat atataggtcc cattttttgc tttctgtatc 18540
   caggtagaca tagatattct atagtgtctc ctatgttgta attagcatta gcatcagtct 18600
   ccacactatt cttaaatttc atattaatgg gtcgtgacgg aatagtacag catgatagaa 18660
   cgcatcctat tcccaacaat gtcaggaacg tcacgctctc caccttcata tttatttatc 18720
   cgtaaaaatg ttatcctgga catcgtacaa ataataaaaa gcccatatat gttcgctatt 18780
   gtagaaattg tttttcacag ttgctcaaaa acgatggcag tgacttatga gttacgttac 18840
   actttggagt ctcatcttta gtaaacatat cataatattc gatattacga gttgacatat 18900
   cgaacaaatt ccaagtattt gattttggat aatattcgta ttttgcatct gctataatta 18960
   agatataatc accgcaagaa cacacgaaca tctttcctac atggttaaag tacatgtaca 19020
   attctatcca tttgtcttcc ttaactatat atttgtatag ataattacga gtctcgtgag 19080
   taattccagt aattacatag atgtcgccgt cgtactctac agcataaact atactatgat 19140
   gtctaggcat gggagacttt tttatccaac gatttttagt gaaacattcc acatcgttta 19200
   atactacata tttctcatac gtggtataaa ctccacccat tacatatata tcatcgttta 19260
   cgaataccga cgcgcctgaa tatctaggag taattaagtt tggaagtctt atccatttcg 19320
   aagtgccgtg tttcaaatat tctgccacac ccgttgaaat agaaaattct aatcctccta 19380
   ttacatataa ctttccatcg ttaacacaag tactaacttc tgattttaac gacgacatat 19440
   tagtaaccgt tttccatttt ttcgttttaa gatctacccg cgatacggaa taaacatgtc 19500
   tattgttaat catgccgcca ataatgtata gacaattatg taaaacattt gcattataga 19560
   attgtctatc tgtattaccg actatcgtcc aatattctgt tctaggagag taatgggtta 19620
   ttgtggatat ataatcagag tttttaatga ctactatatt atgttttata ccatttcgtg 19680
   tcactggctt tgtagatttg gatatagtta atcccaacaa tgatatagca ttgcgcatag 19740
   tattagtcat aaacttggga tgtaaaatgt tgatgatatc tacatcgttt ggatttttat 19800
   gtatccactt taataatatc atagctgtaa catcctcatg atttacgtta acgtcttcgt 19860
   gggataagat agttgtcagt tcatcctttg ataattttcc aaattctgga tcggatgtca 19920
   ccgcagtaat attgttgatt atttctgaca tcgacgcatt atatagtttt ttaattccat 19980
   atcttttaga aaagttaaac atccttatac aatttgtgaa attaatatta tgaatcatag 20040
   tttttacaca tagatctact acaggcggaa catcaattat tatggcagca actagtatca 20100
   tttctacatt gtttatggtg atgtttatct tcttccagcg catatagtct aatagcgatt 20160
   caaacgcgtg atagtttata ccattcaata taatcgcttc atcctttaga tggtgatcct 20220
   gaatgcgttt aaaaaaatta tacggagacg ccgtaataat ttccttattc acttgtataa 20280
   tttccccatt gatagaaaat attacgcttt ccattcttaa agtactataa gtaattatag 20340
   tataatgtaa acgtttatat attcaatatt tttataaaaa tcattttgac attaattcct 20400
   ttttaaattt ccgtctatca tctatagaaa cgtattctat gaatttataa aatgctttta 20460
   cgtgtcctat cgtaggcgat agaaccgcta aaaagcctat cgaatttcta caaaagaatc 20520
   tgttatatgg tatagggaga gtataaaaca ttaaatgtcc gtacttatta aagtattcag 20580
   tagccaatcc taactctttc gaatacttat taatggctct tgttctgtac gaatctattt 20640
   ttttgaacaa cggacctagt ggtatatctt gttctatgta tctaaaataa tgtctgacta 20700
   gatccgttag tttaatatcc gcagtcatct tgtctagaat ggcaaatcta actgcgggtt 20760
   taggctttag tttagtttct atatctacat ctatgtcttt atctaacacc aaaaatataa 20820
   tagctaatat tttattacaa tcatccggat attcttctac gatctcacta actaatgttt 20880
   ctttggttat actagtatag tcactatcgg acaaataaag aaaatcagat gatcgatgaa 20940
   taatacattt aaattcatca tctgtaagat ttttgagatg tctcattaga atattattag 21000
   ggttagtact cattatcatt cggcagctat tacttatttt attatttttc accatataga 21060
   tcaatcatta gatcatcaaa atatgtttca atcatcctaa agagtatggt gaatgactct 21120
   tcccatctaa tttctgaacg ttcaccaatg tctctagcca ctttggcact aatagcgatc 21180
   attcgcttag cgtcttctat attattaact ggttgattta atctatctag caatggaccg 21240
   tcggacagcg tcattctcat gttcttaatc aatgtacata catcgccgtc atctaccaat 21300
   tcatccaaca acataagctt tttaaaatca tcattataat aggtttgatc gttgtcattt 21360
   ctccaaagaa tatatctaat aagtagagtc ctcatgctta gttaacaact attttttatg 21420
   ttaaatcaat tagtacaccg ctatgtttaa tacttattca tattttagtt tttaggattg 21480
   agaatcaata caaaaattaa tgcatcatta attttagaaa tacttagttt ccacgtagtc 21540
   aatgaaacat ttgaactcat cgtacaggac gttctcgtac aggacgtaac tataaaccgg 21600
   tttatatttg ttcaagatag atacaaatcc gataactttt tttacgaatt ctacgggatc 21660
   cactttaaaa gtgtcatacc gggttctttt tattttttta aacagatcaa tggtgtgatg 21720
   ttgattaggt cttttacgaa tttgatatag aatagcgttt acatattctc cataatggtc 21780
   aatcgccatt tgttcgtatg tcataaattc tttaattata tgacactgtg tattatttag 21840
   ttcatccttg ttcattgtta ggaatctatc caaaatggca attatactag aactataggt 21900
   gcgttgtata cacatattga tgtgtctgtt tatacaatcc atgatatttg gatccatgct 21960
   actaccttcg ggtaaaattg tagcatcata taccatttct agtactttag gttcattatt 22020
   atccattgca gaggacgtca tgatcgaatc ataaaaaaat atattatttt tatgttattt 22080
   tgttaaaaat aatcatcgaa tacttcgtaa gatactcctt catgaacata atcagttaca 22140
   aaacgtttat atgaagtaaa gtatctacga tttttacaaa agtccggatg cataagtaca 22200
   aagtacgcga taaacggaat aataatagat ttatctagtc tatctttttc tatagctttc 22260
   atagttagat acatggtctc agaagtagga ttatgtaaca tcagcttcga taaaatgact 22320
   gggttattta gtcttacaca ttcgctcata catgtatgac cgttaactac agagtctaca 22380
   ctaaaatgat tgaacaatag atagtctacc attgtttcgt attcagatag tacagcgtag 22440
   tacatggcat cttcacaaat tatatcattg tctaatagat atttgacgca tcttatggat 22500
   cccacttcaa cagccatctt aaaatcatat tgctttcctt tatcattaat aatttctaga 22560
   acatcatctc tatcataaaa gatacaaata ttaactgttt gatccgtaat aacattgcta 22620
   gtcaatagca atttgttaat aagatgcgct gggctcaatg tcttaataag aagtgtaaga 22680
   ggactatctc cgaatttgtt ttgtttatta acatccgttg atggaagtaa aagatctata 22740
   atgtctacat tcttgactgt tttagagcat acaatatgga gaggtgtatt tccatcatga 22800
   tctggttttg agggactaat tcctagtttc atcatccatg agattgtaga agcttttgga 22860
   ttgtctgaca taagatgtct atgaatatga tttttgccaa atttatccac tatcctggct 22920
   tcgaatccga tggacattat ttttttaaac actctttctg aaggatctgt acacgccaac 22980
   aacggaccac atccttcttc atcaaccgag ttgttaatct tggctccata ctgtaccaat 23040
   aaatttattc tctctatgac ttcatcatct gttcccgaga gataatatag aggtgtttta 23100
   tgctgtttat cacacgcgtt tggatctgcg ccgtgcgtca gcagcatcgc gactattcta 23160
   ttattattaa ttttagaagc tatatgcaat ggataatttc catcatcatc cgtctcattt 23220
   ggagagtatc ctctatgaag aagttcttcg acaaatcgtt catctagtcc tttaattcca 23280
   caatacgcat gtagaatgtg ataattattt ccagaaggtt cgatagcttg tagcatattc 23340
   ctaaatacat ctaaattttt actattatat ttggcataaa gagatagata atactcggcc 23400
   gacataatgt tgtccattgt agtataaaaa ttaatatttc tatttctatt tctgtatatt 23460
   tgcaacaatt tactctctat aacaaatatc ataacttagt ttttttatgt caagaaggca 23520
   ctggtttagt tcatctataa atgtcacgcc ataactacca cgcatgccat actcagaatt 23580
   atgataaaga tatttatcct tggggtgtag gtaatgggga ttaatctttg ttggatcagt 23640
   ctctaagtta acacatgtca cacatgatcc atttatagtt atatcacacg atgatgattt 23700
   atgaattgat tccggaagat cgctatcgta ttttgtggtt ccacaattca tttccataca 23760
   tgttattgtc acactaatat tatgatgaac tttatctagc cgctgagtgg taaacaacag 23820
   aacagatagt ttattatctt taccaacacc ctcagccgct gccacaaatc tctgatccgt 23880
   atccatgatg gtcatgttta tttctagtcc gtatccagtc aacactatgt tagcatttct 23940
   gtcgatatag ctttcactca tatgacactc accaataata gtagaattaa tgtcgtaatt 24000
   tacaccaata gtgagttcgg cggcaaagta ccaataccgg taatcttgtc gaggaggaca 24060
   tatagtattc ttgtattcta ccgaataccc gagagatgcg atacaaaaga gcaagactaa 24120
   tttgtaaacc atcttactca aaatatgtaa caatagtacg atgcaatgag taagacaata 24180
   ggaaatctat cttatataca cataattatt ctatcaattt taccaattag ttagtgtaat 24240
   gttaacaaaa atgtgggaga atctaattag tttttcttta cacaattgac gtacatgagt 24300
   ctgagttcct tgtttttgct aattatttca tccaatttat tattcttgac gatatcgaga 24360
   tcttttgtat aggagtcaaa cttgtattca acatgctttt ctataatcat tttagctatt 24420
   tcggcatcat ccaatagtac attttccaga ttagcagaat agatattaat gtcgtatttg 24480
   aacagagcct gtaacatctc aatgtcttta ttatctatag ccaatttaat gtccggaatg 24540
   aagagaaggg aattattggt gtttgtcgac gtcatatagt cgagcaagag aatcatcata 24600
   tccacgtgtc cattttttat agtgatgtga atacaactaa ggagaatagc cagatcaaaa 24660
   gtagatggta tctctgaaag aaagtaggaa acaatactta catcattaag catgacggca 24720
   tgataaaatg aagttttcca tccagttttc ccatagaaca tcagtctcca atttttctta 24780
   acaaacagtt ttaccgtttg catgttacca ctatcaaccg cataatacaa tgcggtgttt 24840
   cccttgtcat caaattgtga atcatccagt ccactgaata gcaaaatctt tactattttg 24900
   gtatcttcca atgtggctgc ctgatgtaat ggaaattcat tctctagaag atttttcaat 24960
   gctccagcgt tcaacaacgt acatactaga cgcacgttat tatcagctat tgcataatac 25020
   aaggcactat gtccatggac atccgcctta aatgtatctt tactagagag aaagcttttc 25080
   agctgcttag acttccaagt attaattcgt gacagatcca tgtctgaaac gagacgctaa 25140
   ttagtgtata ttttttcatt ttttataatt ttgtcatatt gcaccagaat taataatatc 25200
   tttaatagat ctgattagta gatacatggc tatcgcaaaa caacatatac acatttaata 25260
   aaaataatat ttattaagaa aattcagatt tcacgtaccc atcaatataa ataaaataat 25320
   gattccttac accgtaccca tattaaggag attccacctt acccataaac aatataaatc 25380
   cagtaatatc atgtctgatg atgaacacaa atggtgtatt aaattccagt ttttcaggag 25440
   atgatctcgc cgtagctacc ataatagtag atgcctctgc tacagttcct tgttcgtcga 25500
   catctatctt tgcattctga aacattttat aaatatataa tgggtcccta gtcatatgtt 25560
   taaacgacgc attatctgga ttaaacatac taggagccat catttcggct atcgacttaa 25620
   tatccctctt attttcgata gaaaatttag ggagtttaag attgtacact ttattcccta 25680
   attgagacga ccaatagtct aattttgcag ccgtgataga atctgtgaaa tgggtcatat 25740
   tatcacctat tgccaggtac atactaatat tagcatcctt atacggaagg cgtaccatgt 25800
   catattcttt gtcatcgatt gtgattgtat ttccttgcaa tttagtaact acgttcatca 25860
   tgggaaccgt tttcgtaccg tacttattag taaaactagc attgcgtgtt ttagtgatat 25920
   caaacggata ttgccatata cctttaaaat atatagtatt aatgattgcc catagagtat 25980
   tattgtcgag catattagaa tctactacat tagacatacc ggatctacgt tctactatag 26040
   aattaatttt attaaccgca tctcgtctaa agtttaatct atataggccg aatctatgat 26100
   attgttgata atacgacggt ttaatgcaca cagtattatc tacgaaactt tgataagtta 26160
   gatcagtgta cgtatattta gatgttttca gcttagctaa tcctgatatt aattctgtaa 26220
   atgctggacc cagatctctt tttctcaaat ccatagtctt caataattct attctagtat 26280
   tacctgatgc aggcaatagc gacataaaca tagaaaacga ataaccaaac ggtgagaaga 26340
   caatattatc atcttgaata tttttatacg ctactatacc ggcattggta aatccttgta 26400
   gacgataggc ggacgctgaa cacgctaacg atagtatcaa taacgcaatc atgattttat 26460
   ggtattaata attaacctta tttttatgtt cggtataaaa aaattattga tgtctacaca 26520
   tccttttgta attgacatct atatatcctt ttgtataatc aactctaatc actttaactt 26580
   ttacagtttt ccctaccagt ttatccctat attcaacata tctatccata tgcatcttaa 26640
   cactctctgc caagatagct tcagagtgag gatagtcaaa aagataaata tatagagcat 26700
   aatcattctc gtatactctg ccctttatta catcacccgc attgggcaac gaataacaaa 26760
   atgcaagcat cttgttaacg ggctcgtaaa ttgggataaa aattatgttt ttattgtctt 26820
   atatctattt tattcaagag aatattcagg aatttctttt tccggttgta tctcgtcgca 26880
   gtatatatca tttgtacatt gtttcatatt ttttaatagt ttacaccttt tagtaggact 26940
   agtatcgtac aattcatagc tgtattttga attccaatca cgcataaaaa tatcttccaa 27000
   ttgttgacga agacctaatc catcatccgg tgtaatatta atagatgctc cacatgtatc 27060
   cgtaaagtaa tttcctgtcc aatttgaggt acctatatag gccgttttat cggttaccat 27120
   atatttggca tggtttaccc tagaatacgg aatgggagga tcagcatctg gtacaataaa 27180
   tagctttact tctatattta tgtttttaga ttttagcata gcgatagatc ttaaaaagtt 27240
   tctcatgata aacgaagatc gttgccagca actaatcaat agcttaacgg atacttgtct 27300
   gtctatagcg gatcttctta attcatcttc tatataaggc caaaacaaaa ttttacccgc 27360
   cttcgaataa ataataggga taaagttcat aacagataca taaacgaatt tactcgcatt 27420
   tctaatacat gacaataaag cggttaaatc attggttctt tccatagtac atagttgttg 27480
   cggtgcagaa gcaataaata cagagtgtgg aacgccgctt acgttaatac taagaggatg 27540
   atctgtatta taatacgacg gataaaagtt tttccaatta tatggtagat tgttaactcc 27600
   aagataccag tatacctcaa aaatttgagt gagatccgct gccaagttcc tattattgaa 27660
   gatcgcaata cccaattcct tgacctgagt tagtgatctc caatccatgt tagcgcttcc 27720
   taaataaata tgtgtattat cagatatcca aaattttgta tgaagaactc ctcctaggat 27780
   atttgtaata tctatgtatc gtacttcaac tccggccatt tgtagtcttt caacatcctt 27840
   taatggtttg ttagatttat taacggctac tctaactcgt actcctcttt tgggtaattg 27900
   tacaatctcg tttaatatta tcgtgccgaa attcgtaccc acttcatccg ataaactcca 27960
   ataaaaagat gatatatcta gtgtttttgt ggtattggat agaatttccc tccacatgtt 28020
   aaatgtagac aaatatactt tatcaaattg catacctata ggaatagttt ctgtaatcac 28080
   tgcgattgta ttatccggat tcattttatt tgttaaaaga ataatcctat atcacttcac 28140
   tctattaaaa atccaagttt ctatttcttt catgactgat tttttaactt catccgtttc 28200
   cttatgaaga tgatgtttgg caccttcata aatttttatt tctctattac aatttgcatg 28260
   ttgcatgaaa taatatgcac ctaaaacatc gctaatctta ttgtttgttc cctggagtat 28320
   gagagtcggg gggtgttaat cttggaaatt atttttctaa ccttgttggt agccttcaag 28380
   acctgactag caaatccagc cttaattttt tcatgattga ctaatgggtc gtattggtat 28440
   ttataaactt tatccatatc tctagatact gattctggac atagctttcc gactggcgca 28500
   tttggtgtga tggttcccat aagtttggca gctagcagat tcagtcttga aacagcatct 28560
   gcattaacta gaggagacat tagaatcatt gctgtaaaca agtttggatt atcgtaagag 28620
   gctagctccc atggaatgac ccaataagta gatttaatag ttaccacgtg ctgtaccaaa 28680
   gtcatcaatc atcatttttt caccattact tcttccatgt ccaatatgat catgtgagaa 28740
   tactaaaatt cctaacgatg atatgttttc agctagttcg tcataacgtc cagaatgttt 28800
   accagctcca tgacttatga atactaatgc cttaggatat gtaataggtt tccaatattt 28860
   acaatatatg taatcattgt ccagattgaa catacagttt gcactcatga ttcacgttat 28920
   ataactatca atattaacag ttcgtttgat gatcatatta tttttatgtt ttattgataa 28980
   ttgtaaaaac atacaattaa atcaatatag aggaaggaga cggctactgt cttttgtaag 29040
   atagtcatgg cgactaaatt agattatgag gatgctgttt tttactttgt ggatgatgat 29100
   aaaatatgta gtcgcgactc catcatcgat ctaatagatg aatatattac gtggagaaat 29160
   catgttatag tgtttaacaa agatattacc agttgtggaa gactgtacaa ggaattgatg 29220
   aagttcgatg atgtcgctat acggtactat ggtattgata aaattaatga gattgtcgaa 29280
   gctatgagcg aaggagacca ctacatcaat tttacaaaag tccatgatca ggaaagttta 29340
   ttcgctacca taggaatatg tgctaaaatc actgaacatt ggggatacaa aaagatttca 29400
   gaatctagat tccaatcatt gggaaacatt acagatttga tgaccgacga taatataaac 29460
   atcttgatac tttttctaga aaaaaaattg aattgatgat ataggggtct tcataacgca 29520
   taattattac gttagcattc tatatccgtg ttaaaaaaaa ttatcctatc atgtatttga 29580
   gagttttata tgtagcaaac atgatagctg tgatgccaat aagctttaga tattcacgcg 29640
   tgctagtgtt agggatggta ttatctggtg gtgaaatgtc cgttatataa tctacaaaac 29700
   aatcatcgca tatagtatgc gatagtagag taaacatttt tatagttttt actggattca 29760
   tacatcgtct acccaattcg gttataaatg aaattgtcgc caatcttaca cccaacccct 29820
   tgttatccat tagcatagta ttaacttcgt tatttatgtc ataaactgta aatgattttg 29880
   tagatgccat atcatacatg atattcatgt ccctattata atcattacta actttatcac 29940
   aatatatgtt gataatatct atatatgatc tagtctttgt gggcaactgt ctatacaagt 30000
   cgtctaaacg ttgtttactc atatagtatc gaacagccat cattacatgg tcccgttccg 30060
   ttgatagata atcgagtatg ttagtggact tgtcaaatct atataccata ttttctggaa 30120
   gtggatatac atagtcgtga tcaacattat tgctagcctc atcttctata tcctgtacta 30180
   taccattatc tatatcatct acataatcta tgatattatt acacataaac atcgacaaca 30240
   tactattgtt tattatctaa gtcctgttga tccaaaccct tgatctcctc tatttgtact 30300
   atctagagat tgtacttctt ccagttctgg ataatatata cgttgataga ttagctgagc 30360
   tattctatct ccagtattta cattaaacgt acattttcca ttattaataa gaatgactcc 30420
   tatgtttccc ctataatctt cgtctattac accacctcct atatcaatgc cttttagtga 30480
   cagaccagac ctaggagcta ttctaccata gcagaactta ggcatggaca tactaatatc 30540
   tgtcttaatt aactgtcttt ctcctggagg gatagtataa tcgtaagcgc tatacaaatc 30600
   atatccggca gcacccggcg attgcctagt aggagattta gctctgttag tttccttaac 30660
   aaatctaact ggtgagttaa tattcatgtt gaacataaaa ctaatatttt atttcaaaat 30720
   tatttaccat cccatatatt ccatgaataa gtgtgatgat tgtacacttc tatagtatct 30780
   atatacgatc cacgataaaa tcctcctatc aatagcagtt tattatccac tatgatcaat 30840
   tctggattat ccctcggata aataggatca tctatcagag tccatgtatt gctggattca 30900
   caataaaatt ccgcatttct accaaccaag aataaccttc taccgaacac taacgcgcat 30960
   gatttataat gaggataata agtggatggt ccaaactgcc actgatcatg attgggtagc 31020
   aaatattctg tagttgtatc agtttcagaa tgtcctccca ttacgtatat aacattgttt 31080
   atggatgcca ctgctggatt acatctaggt ttcagaagac tcggcatatt aacccaagca 31140
   gcatccccgt ggaaccaacg ctcaacagat gtgggatttg gtagacctcc tactacgtat 31200
   aatttattgt tagcgggtat cccgctagca tacagtctgg ggctattcat cggaggaatt 31260
   ggaatccaat tgtttgatat ataatttaca gctatagcat tgttatgtat ttcattgttc 31320
   atccatccac cgatgagata tactacttct ccaacatgag tacttgtaca catatggaat 31380
   atatctataa tttgatccat gttcatagga tactctatga atggatactt gtatgatttg 31440
   cgtggttgtt tatcacaatg aaatattttg gtacagtcta gtatccattt tacattattt 31500
   atacctctgg gagaaagata atttgacctg attacatttt tgataaggag tagcagattt 31560
   cctaatttat ttcttcgcct catataccac ttaatgacaa aatcaactac ataatcctca 31620
   tctggaacat ttagttcatc gctttctaga ataagtttca tagatagata atcaaaattg 31680
   tctatgatgt catcttccag ttccaaaaag tgtttggcaa taaagttttt agtatgacat 31740
   aagagattgg atagtccgta ttctataccc atcatgtaac actcgacaca atattccttt 31800
   ctaaaatctc gtaagataaa gtttatacaa gtgtagatga taaattctac agaggttaat 31860
   atagaagcac gtaataaatt gacgacgtta tgactatcta tatatacctt tccagtatat 31920
   gagtaaataa ctatagaagt taaactgtga atgtcaaggt ctagacaaac ccttgtaact 31980
   ggatctttat ttttcgtgta tttttgacgt aaatgtgtgc gaaagtaagg agataacttt 32040
   ttcaatatcg tagaattgac tattatattg cctcctatgg catcaataat tgttttgaat 32100
   ttcttagtca tagacaatgc taatatattc ttacagtaca cagtattgac aaatatcggc 32160
   atttatgttt ctttaaaagt caacatctag agaaaaatga ttatcttttt gagacataac 32220
   tcccattttt tggtattcac ccacacgttt ttcgaaaaaa ttagtttttc cttccaatga 32280
   tatattttcc atgaaatcaa acggattggt aacattataa atttttttaa atcccaattc 32340
   agaaatcaat ctatccgcga cgaattctat atatgttttc atcatttcac aattcattcc 32400
   tataagttta actggaagag ccgcagtaag aaattcttgt tcaatggata ctgcatctgt 32460
   tataatagat ctaacggttt cttcactcgg tggatgcaat aaatgtttaa acatcaaaca 32520
   tgcgaaatcg cagtgcagac cctcgtctct actaattagt tcgttggaaa acgtgagtcc 32580
   gggcattagg ccacgctttt taagccaaaa tatggaagcg aatgatccag aaaagaaaat 32640
   tccttctact gcagcaaagg caataagtct ctctccataa ccggcgctgt catgtatcca 32700
   cttttgagcc caatcggcct tcttttttac acaaggcatc gtttctatgg cattaaagag 32760
   atagtttttt tcattactat ctttaacata agtatcgatc aaaagactat acatttccga 32820
   atgaatgttt tcaatggcca tctgaaatcc gtagaaacat ctagcctcgg taatctgtac 32880
   ttctgtacaa aatcgttccg ccaaattttc attcactatt ccgtcactgg ctgcaaaaaa 32940
   cgccaataca tgttttataa aatatttttc gtctggtgtt agtttattcc aatcattgat 33000
   atctttagat atatctactt cttccactgt ccaaaatgat gcctctgcct ttttatacat 33060
   gttccagatg tcatgatatt ggattgggaa aataacaaat ctatttggat ttggtgcaag 33120
   gatgggttcc ataactaaat taacaataac aataaatttt ttttcagtta tctatatgcc 33180
   tgtacttgga ttttttgtac atcgatatcg ccgcaatcac tacaataatt acaagtatta 33240
   ttgatagcat tgttattagt actatcataa ttaaattatc tacattcatg ggtgctgaat 33300
   aatcgttatt atcatcatta tcattttgta attgtgacat catactagat aaatcgtttg 33360
   cgagattgtt gtgggaagcg ggcatggagg atgcattatc attattattt aacgccttcc 33420
   atttggattc acaaatgtta cgcacattca acattttatg gaaactataa ttttgtgaaa 33480
   acaaataaca agaaaactcg tcatcgttca aatttttaac gatagtaaac cgattaaacg 33540
   tcgagctaat ttctaacgct agcgactctg ttggatatgg gtttccagat atatatcttt 33600
   tcagttcccc tacgtatcta taatcatctg taggaaatgg aagatatttc catttatcta 33660
   ctgttcctaa tatcatatgt ggtggtgtag tagaaccatt aagcgcgaaa gatgttattt 33720
   cgcatcgtat tttaacttcg caataatttc tggttagata acgcactcta ccagtcaagt 33780
   caatgatatt agcctttaca gatatattca tagtagtcgt aacgatgact ccatctttta 33840
   gatgcgatac tcctttgtat gtaccagaat cttcgtacct caaactcgat atatttaaac 33900
   aagttaatga gatattaacg cgttttatga atgatgatat ataaccagaa gttttatcct 33960
   cggtggctag cgctataacc ttatcattat aataccaact agtgtgatta atatgtgaca 34020
   cgtcagtgtg ggtacaaata tgtacattat cgtctacgtc gtattcgata catccgcata 34080
   cagccaacaa atataaaatg acaaatactc taacgccgtt cgtacccatc ttgatgcggt 34140
   ttaataaatg ttttgatttc aatttattgt aaaaaaagat tcggttttat actgttcgat 34200
   attctcattg cttatatttt catctatcat ctccacacag tcaaatccgt ggttagcatg 34260
   cacctcatca accggtaaaa gactatcgga ctcttctatc attataactc tagaatattt 34320
   aatttggtca ttattaatca agtcaattat cttattttta acaaacgtga gtattttact 34380
   cattttttat aaaaactttt agaaatatac agactctatc gtgtgtctat atcttctttt 34440
   tatatccaat gtatttatgt ctgatttttc ttcatttatc atatataatg gtccaaattc 34500
   tacacgtgct tcggattcat ccagatcatt aaggttctta taattgtaac atccttctct 34560
   tccctcttct acatcttcct tcttattctt attcttagcg tcacagaatc taccacagca 34620
   ggatcccatg acgagcgtca tattaaacta atccattttc aattataata tatgattagt 34680
   aatgaccatt aaaataaaaa atattcttca taaccggcaa gaaagtgaaa agttcacatt 34740
   gaaactatgt cagtagtata catcatgaaa tgatgatata tatatactct attttggtgg 34800
   aggattatat gatataattc gtggataatc atttttaaga cacatttctt tattcgtaaa 34860
   tcttttcacg ttaaatgagt gtccatattt tgcaatttct tcatatgatg gcggtgtacg 34920
   tggacgaggc tgctcctgtt cttgttgtgg tcgccgactg tcgtgtctgc gtttagatcc 34980
   ctccattatc gcgattgcgt agatggagta ctattttata ccttgtaatt aaattttttt 35040
   attaattaaa cgtataaaaa cgttccgtat ctgtatttaa gagccagatt tcgtctaata 35100
   gaacaaatag ctacagtaaa aataactaga ataattgcta cacccactag aaaccacgga 35160
   tcgtaatacg gcaatcggtt ttcgataata ggtggaacgt atattttatt taaggactta 35220
   acaattgtct gtaaaccaca atttgcttcc gcggatcctg tattaactat ctgtaaaagc 35280
   atatgttgac cgggcggagc cgaacattct ccgatatcta atttctgtat atctataata 35340
   ttattaacct ccgcatacgc attacagttc ttttctagct tggataccgc actaggtaca 35400
   tcgtctagat ctattcctat ttcctcagcg atagctcttc tatccttttc cggaagcaat 35460
   gaaatcactt caataaatga ttcaaccatg agtgtgaaac taagtcgaga attactcatg 35520
   catttgttag ttattcggag cgcgcaattt ttaaactgtc ctataacctc tcctatatga 35580
   atagcacaag tgacattagt agggatagaa tgttgagcta atttttgtaa ataactatct 35640
   ataaaaagat tatacaaagt tttaaactct ttagtttccg ccatttatcc agtctgagaa 35700
   aatgtctctc ataataaatt tttccaagaa actaattggg tgaagaatgg aaacctttaa 35760
   tctatattta tcacagtctg ttttggtaca catgatgaat tcttccaatg ccgtactaaa 35820
   ttcgatatct ttttcgattt ctggatatgt ttttaataaa gtatgaacaa agaaatggaa 35880
   atcgtaatac cagttatgtt taactttgaa attgtttttt attttcttgt taatgattcc 35940
   agccacttgg gaaaagtcaa agtcgtttaa tgccgattta atacgttcat taaaaacaaa 36000
   ctttttatcc tttagatgaa ttattattgg ttcattggaa tcaaaaagta agatattatc 36060
   gggtttaaga tctgcgtgta aaaagttgtc gcaacagggt agttcgtaga ttttaatgta 36120
   taacagagcc atctgtaaaa agataaactt tatgtattgt accaaagatt taaatcctaa 36180
   tttgatagct aactcggtat ctactttatc tgccgaatac agtgctaggg gaaaaattat 36240
   aatgtttcct ctttcatatt cgtagttagt tctcttttca tgttcgaaaa agtgaaacat 36300
   gcggttaaaa tagtttataa cattaatatt actgttaata actgccggat aaaagtggga 36360
   tagtaatttc acgaatttga tactgtcctt tctctcgtta aacgccttta aaaaaacttt 36420
   agaagaatat ctcaatgaga gttcctgacc atccatagtt tgtatcaata atagcaacat 36480
   atgaagaaca cgtttataca gagtatgtaa aaatgttaat ttatagttta atcccatggc 36540
   ccacgcacac acgattaatt ttttttcatc tccctttaga ttgttgtata gaaatttggg 36600
   tactgtgaac tccgccgtag tttccatggg actatataat tttgtggcct cgaatacaaa 36660
   ttttactaca tagttatcta tcttaaagac tataccatat cctcctgtag atatgtgata 36720
   aaaatcgtcg tttataggat aaaatcgttt atccttttgt tggaaaaagg atgaattaat 36780
   gtaatcattc tcttctatct ttagtagtgt ttccttatta aaattcttaa aataatttaa 36840
   caatctaact gacggagccc aattttggtg taaatctaat tgggacatta tattgttaaa 36900
   atacaaacag tctcctaata taacagtatc tgataatcta tggggagaca tccattgata 36960
   ttcaggggat gaatcattgg caacacccat ttattgtaca aaaagcccca atttacaaac 37020
   gaaagtccag gtttgataga gacaaacaat taactatttt gtctctgttt ttaacacctc 37080
   cacagttttt aatttcttta gtaatgaaat tattcacaat atcagtatct tctttatcta 37140
   ccagagattt tactaacttg ataaccttgg ctgtctcatt caatagggta gtaatatttg 37200
   tatgtgtgat attgatatct ttttgaattg tttcttttag aagtgattct ttgatggtgc 37260
   cagcatacga attacaataa tgcagaaact cggttaacat gcaggaatta tagtaagcca 37320
   attccaattg ttgcctgtgt tgtattagag tgtcaatatg agcaatggtg tccttgcgtt 37380
   tctctgatag aatgcgagca gcgattttgg cgttatcatt tgacgatatt tctggaatga 37440
   cgaatcctgt ttctactaac tttttggtag gacaaagtga aacaatcaag aagatagctt 37500
   ctcctcctat ttgtggaaga aattgaactc ctctagatga tctactgacg atagtatctc 37560
   cttgacagat attggaccga attacagaag tacctggaat gtaaagccct gaaaccccct 37620
   cattttttaa gcagattgtt gccgtaaatc ctgcactatg cccaagatag agagctcctt 37680
   tggtgaatcc atctctatgt ttcagtttaa ccaagaaaca gtcagctggt ctaaaatttc 37740
   catctctatc taatacagca tctaacttga tgtcaggaac tatgaccggt ttaatgttat 37800
   atgtaacatt gagtaaatcc ttaagttcat aatcatcact gtcatcagtt atgtacgatc 37860
   caaacaatgt ttctaccggc atagtggata cgaagatgct atccatcaga atgtttccct 37920
   gattagtatt ttctatatag ctattcttct ttaaacgatt ttccaaatca gtaactatgt 37980
   tcattttttt aggagtagga cgcctagcca gtatggaaga ggattttcta gatcctctct 38040
   tcaacatctt tgatctcgat ggaatgcaaa accccatagt gaaacaacca acgataaaaa 38100
   taatattgtt tttcactttt tataatttta ccatctgact catggattca ttaatatctt 38160
   tataagagct actaacgtat aattctttat aactgaactg agatatatac accggatcta 38220
   tggtttccat aattgagtaa atgaatgctc ggcaataact aatggcaaat gtatagaaca 38280
   acgaaattat actagagttg ttaaagttaa tattttctat gagctgttcc aataaattat 38340
   ttgttgtgac tgcgttcaag tcataaatca tcttgatact atccagtaaa ccgtttttaa 38400
   gttctggaat attatcatcc cattgtaaag cccctaattc gactatcgaa tatcctgctc 38460
   tgatagcagt ttcaatatcg acggacgtca atactgtaat aaaggtggta gtattgtcat 38520
   catcgtgata aactacggga atatggtcgt tagtaggtac ggtaacttta cacaacgcga 38580
   tatataactt tccttttgta ccatttttaa cgtagttggg acgtcctgca gggtattgtt 38640
   ttgaagaaat gatatcgaga acagatttga tacgatattt gttggattcc tgattattca 38700
   ctataatata atctagacag atagatgatt cgataaatag agaaggtata tcgttggtag 38760
   gataatacat ccccattcca gtattctcgg atactctatt gatgacacta gttaagaaca 38820
   tgtcttctat tctagaaaac gaaaacatcc tacatggact cattaaaact tctaacgctc 38880
   ctgattgtgt ctcgaatgcc tcgtacaagg atttcaagga tgccatagat tctttgacca 38940
   acgatttaga attgcgttta gcatctgatt tttttattaa atcgaatggt cggctctctg 39000
   gtttgctacc ccaatgataa caatagtctt gtaaagataa accgcaagaa aatttatacg 39060
   catccatcca aataacccta gcaccatcgg atgatattaa tgtattatta tagattttcc 39120
   atccacaatt attgggccag tatactgtta gcaacggtat atcgaataga ttactcatgt 39180
   aacctactag aatgatagtt cgtgtactag tcataatatc tttaatccaa tctaaaaaat 39240
   ttaaaattag attttttaca ctgttaaagt taacaaaagt attacccggg tacgtggata 39300
   tcatatatgg cattggtcca ttatcagtaa tagctccata aactgatacg gcgatggttt 39360
   ttatatgtgt ttgatctaac gaggaagaaa ttcgcgccca caattcatct ctagatatgt 39420
   atttaatatc aaacggtaac acatcaattt cgggacgcgt atatgtttct aaatttttaa 39480
   tccaaatata atgatgacct atatgcccta ttatcatact gtcaactata gtacacctag 39540
   ggaacttacg atacatctgt ttcctataat cgttaaattt tacaaatcta taacatgcta 39600
   aaccttttga cgacagccat tcattaattt ctgatatgga atctgtattc tcgataccgt 39660
   atcgttctaa agccagtgct atatctccct gttcgtggga acgctttcgt ataatatcga 39720
   tcaacggata atctgaagtt tttggagaat aatatgactc atgatctatt tcgtccataa 39780
   acaatctaga cataggaatt ggaggcgatg atcttaattt tgtgcaatga gtcgtcaatc 39840
   ctataacttc taatcttgta atattcatca tcgacataat actatctatg ttatcatcgt 39900
   atattagtat accatgacct tcttcatttc gtgccaaaat gatatacagt cttaaatagt 39960
   tacgcaatat ctcaatagtt tcataattgt tagctgtttt catcaaggtt tgtatcctgt 40020
   ttaacatgat ggcgttctat aacgtctcta ttttctattt ttaatttttt aaatttttaa 40080
   cgatttactg tggctagata cccaatctct ctcaaatatt tttttagcct cgcttacaag 40140
   ctgtttatct atactattaa aactgacgaa tccgtgattt tggtaatggg ttccgtcgaa 40200
   atttgccgaa gtgatatgaa catattcgtc gtcgactatc aacaattttg tattattctg 40260
   aatagtgaaa accttcacag atagatcatt ttgaacacac aacgcatcta gacttttggc 40320
   ggttgccata gaatatacgt cgttcttatc ccaattacca actagaagtc tgatcttaac 40380
   tcctctatta atggctgctt ctataatgga gttgtaaatg tcgggccaat agtagctatt 40440
   accgtcgaca cgtgtagtgg gaactatggc caaatgttca atatctatac tagtcttagc 40500
   tgacctgagt ttatcaataa ctacatcggt atctagatct ctagaatatc ccaataggtg 40560
   ttccggagaa tcagtaaaga acactccacc tataggattc ttaatatgat acgcagtgct 40620
   aactggcaaa caacaagccg cagagcataa attcaaccat gaattttttg cgctattaaa 40680
   ggctttaaaa gtatcaaatc ttctacgaag atctgtggcc agcgggggat aatcagaata 40740
   tacacctaac gttttaatcg tatgtataga tcctccagta aatgacgcgt ttcctacata 40800
   acatctttca tcatctgaca cccaaaaaca accgagtagt agtcccacat tatttttttt 40860
   atctatatta acggttataa aatttatatc cgggcagtga ctttgtagct ctcccagatt 40920
   tcttttccct cgttcatcta gcaaaactat tattttaatc cctttttcag atgcctcttt 40980
   tagtttatca aaaataagcg ctcccctagt cgtactcaga ggattacaac aaaaagatgc 41040
   tatgtatata tatttcttag ctagagtgat aatttcgtta aaacattcaa atgttgttaa 41100
   atgatcggat ctaaaatcca tattttctgg tagtgtttct accagcctac attttgctcc 41160
   cgcaggtacc gatgcaaatg gccacattta gttaacataa aaacttatac atcctgttct 41220
   atcaacgatt ctagaatatc atcggctata tcgctaaaat tttcatcaaa gtcgacatca 41280
   caacctaact cagtcaatat attaagaagt tccatgatgt catcttcgtc tatttctata 41340
   tccgtatcca ttgtagattg ttgaccgatt atcgagttta aatcattact aatactcaat 41400
   ccttcagaat acaatctgtg tttcattgta aatttatagg cggtgtattt aagttggtag 41460
   attttcaatt atgtattaat atagcaacag tagttcttgc tcctccttga ttctagcatc 41520
   ctcttcatta ttttcttcta cgtacataaa catgtccaat acgttagaca acacaccgac 41580
   gatggcggcc gctacagaca cgaatatgac taaaccgatg accatttaaa aacccctctc 41640
   tagctttcac ttaaactgta tcgatcattc ttttagcaca tgtataatat aaaaacatta 41700
   ttctatttcg aatttaggct tccaaaaatt tttcatccgt aaaccgataa taatatatat 41760
   agacttgtta atagtcggaa taaatagatt aatgcttaaa ctatcatcat ctccacgatt 41820
   agagatacaa tatttacatt ctttttgctg tttcgaaact ttatcaatac acgttaatac 41880
   aaacccagga aggagatatt gaaactgagg ctgttgaaaa tgaaacggtg aatacaataa 41940
   ttcagataat gtaaaatcat gattccgtat tctgatgata ttagaactgc taatggatgt 42000
   cgatggtatg tatctaggag tatctatttt aacaaagcat cgatttgcta atatacaatt 42060
   atccttttga ttaattgtta ttttattcat attcttaaaa ggtttcatat ttatcaattc 42120
   ttctacatta aaaatttcca tttttaattt atgtagcccc gcaatactcc tcattacgtt 42180
   tcattttttg tctataatat ccattttgtt catctcggta catagattat ccaattgaga 42240
   agcgcattta gtagttttgt acattttaag tttattgacg aatcgtcgaa aactagttat 42300
   agttaacatt ttattatttg ataccctgat attaataccc ctgccgttac tattatttat 42360
   aactgatgta atccacgtaa cattggaatt aactatcgat agtaatgcat cgacgcttcc 42420
   aaaattgtct attataaact caccgataat ttttttattg catgttttca tattcattag 42480
   gattatcaaa tctttaatct tattacgatt gtatgcgttg atattacaag acgtcattct 42540
   aaaagacgga ggatctccat caaatgccag acaatcacgt acaaagtaca tggaaatagg 42600
   ttttgttcta ttgcgcatca tagatttata tagaacaccc gtagaaatac taatttgttt 42660
   tactctataa aatactaatg catctatttc atcgttttgt ataacgtctt tccaagtgtc 42720
   aaattccaaa tttttttcat tgatagtacc aaattcttct atctctttaa ctacttgcat 42780
   agataggtaa ttacagtgat gcctacatgc cgttttttga aactgaatag atgcgtctag 42840
   aagcgatgct acgctagtca caatcaccac tttcatattt agaatatata tatgtaaaaa 42900
   tatagtagaa tttcattttg tttttttcta tgctataaat gaattctcat tttgcatctg 42960
   ctcatactcc gttttatatt aataccaaag aaggaagata tctggttcta aaagccgtta 43020
   aagtatgcga tgttagaact gtagaatgcg aaggaagtaa agcttcctgc gtactcaaag 43080
   tagataaacc ctcatcgccc gcgtgtgaga gaagaccttc gtccccgtcc agatgcgaga 43140
   gaatgaataa ccctggaaaa caagttccgt ttatgaggac ggacatgcta caaaatatgt 43200
   tcgcggctaa tcgcgataat gtagcttcta gacttttgtc ctaaaatact attatatcct 43260
   tttcgatatt aataaatccg tgtcgtccag gttttttatc tctttcagta tgtgaataga 43320
   taggtatttt atctctattc atcatcgaat ttaagagatc cgataaacat tgtttgtatt 43380
   ctccagatgt cagcatctga tacaacaata tatgtgcaca taaacctctg gcacttattt 43440
   catgtacctt ccccttatca ctaaggagaa tagtatttga gaaatatgta tacatgatat 43500
   tatcatgaat tagatataca gaatttgtaa cactctcgaa atcacacgat gtgtcggcgt 43560
   taagatctaa tatatcactc gataacacat tttcatctag atacactaga cattttttaa 43620
   agctaaaata gtctttagta gtaacagtaa ctatgcgatt attttcatcg atgatacatt 43680
   tcatcggcat attattacgc ttaccatcaa agactatacc atgtgtatat ctaacgtatt 43740
   ctagcatggt tgccatacgc gcattaaact tttcaggatc tttggataga tcttccaatc 43800
   tatctatttg agaaaacatt tttatcatgt tcaatagttg aaacgtcgga tccactatat 43860
   agatattatc tataaagatt ttaggaacta cgttcatggt atcctggcga atattaaaac 43920
   tatcaatgat atgattatcg ttttcatctt ttatcaccat atagtttcta agatatggga 43980
   ttttacttaa tataatatta tttcccgtga taaattttat tagaaaggcc aaatctataa 44040
   gaaaagtcct agaattagtc tgaagaatat ctatatcgcc gtatagtata tttggattaa 44100
   ttagatatag agaatatgat ccgtaacata tacaactttt attatggcgt ctaagatatt 44160
   cttccatcaa cttattaaca tttttgacta gggaagatac attatgacgt cccattactt 44220
   ttgccttgtc tattactgcg acgttcatag aatttagcat atctcttgcc aattcttcca 44280
   ttgatgttac attataagaa attttagatg aaattacatt tggagcttta atagtaagaa 44340
   ctcctaatat gtccgtgtat gtggtcacta atacagattg tagttctata atcgtaaata 44400
   atttacctat attatatgtt tgagtctgtt tagaaaagta gctaagtata cgatctttta 44460
   tttctgatgc agatgtatta acatcggaaa aaaatctttt tttattcttt tttactaaag 44520
   atacaaatat gtctttgtta aaaacagtta ttttctgaat atttctagct tgtaatttta 44580
   acatatgata ttcgttcaca ctaggtactc tgcctaaata ggtttctata atctttaatg 44640
   taatattagg aaaagtattc tgatcaggat tcctattcat tttgaggatt taaaactctg 44700
   attattgtct aatatggtct ctacgcaaac tttttcacag agcgatagag tttttgataa 44760
   ctcgtttttc ttaagaaata taaaactact gtctccagag ctcgctctat cttttatttt 44820
   atctaattcg atacaaactc ctgatactgg ttcagaaagt aattcattaa ttttcagtcc 44880
   tttatagaag atatttaata tagataatac aaaatcttca gtttttgata tcgatctgat 44940
   tgatcctaga actagatata ttaataacgt gctcattagg cagtttatgg cagcttgata 45000
   attagatata gtatattcca gttcatattt attagatacc gcattgccca gattttgata 45060
   ttctatgaat tcctctgaaa ataaatccaa aataactaga cattctattt tttgtggatt 45120
   agtgtactct cttccctcta tcatgttcac tactggtgtc cacgatgata aatatctaga 45180
   gggaatataa tatagtccat aggatgccaa tctagcaatg tcgaataact gtaattttat 45240
   tcttcgctct tcattatgaa ttgattcttg aggtataaac ctaacacaaa ttatattatt 45300
   agacttttcg tatgtaatgt ctttcatgtt ataagttttt aatcctggaa tagaatctat 45360
   tttaatgagg cttttaaacg cagagttctc caacgagtca aagcataata ctctgttgtt 45420
   tttcttatat acgatgttac gattttcttc tttgaatgga ataggttttt gaattagttt 45480
   ataattacaa cataatagat aaggaagtgt gcaaatagta cgcggaaaaa acataatagc 45540
   tcccctgttt tcatccatgg ttttaagtaa atgatcactg gcttctttag tcaatggata 45600
   ttcgaacatt aaccgtttca tcatcattgg acagaatcca tatttcttaa tgtaaagagt 45660
   gatcaaatca ttgtgtttat tgtaccatct tgttgtaaat gtgtattcgg ttatcggatc 45720
   tgctcctttt tctattaaag tatcgatgtc aatctcgtct aagaattcaa ctatatcgac 45780
   atatttcatt tgtatacaca taaccattac taacgtagaa tgtataggaa gagatgtaac 45840
   gggaacaggg tttgttgatt cgcaaactat tctaatacat aattcttctg ttaatacgtc 45900
   ttgcacgtaa tctattatag atgccaagat atctatataa ttattttgta agatgatgtt 45960
   aactatgtga tctatataag tagtgtaata attcatgtat tttgatatat gttccaactc 46020
   tgtctttgtg atgtctagtt tcgtaatatc tatagcatcc tcaaaaaata tattcgcata 46080
   tattcccaag tcttcagttc tatcttctaa aaaatcttca acgtatggaa tataataatc 46140
   tattttacct cttctgatat cattaatgat atagtttttg acactatctt ctgtcaattg 46200
   attcttattc actatatcta agaaacggat agcgtcccta ggacgaacta ctgccattaa 46260
   tatctctatt atagcttctg gacataattc atctattata ccagaattaa tgggaactat 46320
   tccgtatcta tctaacatag ttttaagaaa gtcagaatct aagacttgat gttcatatat 46380
   tggttcatac atgaaatgat ctctattgat gatagtgact atttcattct ctgaaaattg 46440
   gtaactcatt ctatatatgc tttccttgtt gatgaaggat agaatatact caatagaatt 46500
   tgtaccaaca aactgttctc ttatgaatcg tatatcatca tctgaaataa tcatgtaagg 46560
   catacattta acaattagag acttgtctcc tgttatcaat atactattct tgtgataatt 46620
   tatgtgtgag gcaaatttgt ccacgttctt taattttgtt atagtagata tcaaatccaa 46680
   tggagctaca gttcttggct taaacagata tagtttttct ggaacaaatt ctacaacatt 46740
   attataaagg actttgggta gataagtggg atgaaatcct attttaatta atgcgatagc 46800
   cttgtcctcg tgcagatatc caaacgcttt tgtgatagta tggcattcat tgtctagaaa 46860
   cgctctacga atatctgtga cagatatcat ctttagagaa tatactagtc gcgttaatag 46920
   tactacaatt tgtatttttt aatctatctc aataaaaaaa ttaatatgta tgattcaatg 46980
   tataactaaa ctactaactg ttattgataa ctagaatcag aatctaatga tgacgtaacc 47040
   aagaagttta tctactgcca atttagctgc attattttta gcatctcgtt tagattttcc 47100
   atcggcctta tcgaatactc ttccgtcgat gtctacacag gcataaaatg taggagagtt 47160
   actaggccca actgattcaa tacgaaaaga ccaatctctc ctagtaattt ggcagtactc 47220
   attaataacg gtgacagggt tagcatcttt ccaatcaata atttttttag ccggaataac 47280
   atcatcaaaa gacttatgat cctctctcat tgatttttcg cgggatacat catctattat 47340
   gacgtcagcc atagcatcag catccggctt atccgcctcc gttgtcataa accaacgagg 47400
   aggaatatcg tcggagctgt acaccatagc actacgttga agatcgtaca gagctttatt 47460
   aacttctcgc ttctccatat taagttgtct agttagttgt gcagcagtag ctccttcgat 47520
   tccaatgttt ttaatagccg cacacacaat ctctgcgtca gaacgctcgt caatatagat 47580
   cttagacatt tttagagaga actaacgcaa ccagcaataa aactgaacct actttatcat 47640
   ttttttattc atcatcctct ggtggttcgt cgttcctatc aaatgtagct ctgattaacc 47700
   cgtcatctat aggtgatgct ggttctggag attctggagg agatggatta ttatctggaa 47760
   gaatctctgt tatttccttg ttttcatgta tcgattgcgt tgtaacatta agattgcgaa 47820
   atgctctaaa tttgggaggc ttaaagtgtt gtttgcaatc tctacacgcg tgtctaacta 47880
   gtggaggttc gtcagctgct ctagtttgaa tcatcatcgg cgtagtattc ctacttttac 47940
   agttaggaca cggtgtattg tatttctcgt cgagaacgtt aaaataatcg ttgtaactca 48000
   catcctttat tttatctata ttgtattcta ctcctttctt aatgcatttt ataccgaata 48060
   agagatagcg aaggaattct ttttcggtgc cgctagtacc cttaatcata tcacatagtg 48120
   ttttatattc caaatttgtg gcaatagacg gtttatttct atacgatagt ttgtttctgg 48180
   aatcctttga gtattctata ccaatattat tctttgattc gaatttagtt tcttcgatat 48240
   tagattttgt attacctata ttcttgatgt agtactttga tgatttttcc atggcccatt 48300
   ctattaagtc ttccaagttg gcatcatcca catattgtga tagtaattct cggatatcag 48360
   tagcggctac cgccattgat gtttgttcat tggatgagta actactaatg tatacatttt 48420
   ccatttataa cacttatgta ttaactttgt tcatttatat tttttcatta ttatgttgat 48480
   attaacaaaa gtgaatatat atatgttaat aattgtattg tggttatacg gctacaattt 48540
   tataatgagt gaaagtcagt gtccgatgat caatgacgat agctttactc tgaaaagaaa 48600
   gtatcaaatc gatagtgcgg agtcaacaat aaaaatggat aagaagagga taaagtttca 48660
   gaatagagcc aaaatggtaa aagaaataaa tcagacaata agagcagcac aaactcatta 48720
   cgagacattg aaactaggat acataaaatt taagagaatg attatgacta ctactctaga 48780
   agatatagca ccatctattc caaataatca gaaaacttat aaactattct cggacatttc 48840
   agccatcggc aaagcatcac agaatccgag taagatggta tatgctctgc tgctttacat 48900
   gtttcccaat ttgtttggag atgatcatag attcattcgt tatagaatgc atccaatgag 48960
   taaaatcaaa cacaagatct tctctccttt caaacttaat cttattagaa tattagtgga 49020
   agaaagattc tataataatg aatgcagatc taataaatgg agaataattg gaacacaagt 49080
   tgataaaatg ttgatagctg aatctgataa atatacaata gatgcaaggt ataacctaaa 49140
   acccatgtat agaatcaagg gagaatctga agaagatacc ctctttatca aacagatggt 49200
   agaacaatgt gtgacatccc aggaattggt ggaaaaagtg ttgaagatac tgtttagaga 49260
   tttgttcaag agtggagaat acaaagcgta cagatacgat gatgatgtag aaaatggatt 49320
   tattggattg gatacactaa aattaaacat tgttcatgat atagttgaac catgtatgcc 49380
   tgttcgtagg ccagtggcta agatactgtg taaagaaatg gtaaataaat actttgagaa 49440
   tccgctacat attattggta aaaatcttca agagtgcatt gactttgtta gtgaataggc 49500
   atttcatctt tctccaatac taattcaaat tgttaaatta ataatggata gtataaatag 49560
   ttattagtta taagatagta aaaataatta ttagaataag agtgtagtat catagataac 49620
   tctcttctat aaaaatggat tttattcgta gaaagtatct tatatacaca gtagaaaata 49680
   atatagattt tttaaaggat gatacattaa gtaaagtaaa caattttacc ctcaatcatg 49740
   tactagctct caagtatcta gttagcaatt ttcctcaaca cgttattact aaggatgtat 49800
   tagctaatac caattttttt gttttcatac atatggtacg atgttgtaaa gtgtacgaag 49860
   cggttttacg acacgcattt gatgcaccca cgttgtacgt taaagcattg actaagaatt 49920
   atttatcgtt tagtaacgca atacaatcgt acaaggaaac cgtgcataaa ctaacacaag 49980
   atgaaaaatt tttagaggtt gccgaataca tggacgaatt aggagaactt ataggcgtaa 50040
   attatgactt agttcttaat ccattatttc acggagggga acccatcaaa gatatggaaa 50100
   tcattttttt aaaactgttt aagaaaacag acttcaaagt tgttaaaaaa ttaagtgtta 50160
   taagattact tatttgggca tacctaagca agaaagatac aggcatagag tttgcggata 50220
   atgatagaca aaatatatac actctatttc aacaaactgg tagaatcgtc catagcaatc 50280
   taacagaaac gtttagagat tatatctttc ccggagataa gactagctat tgggtgtggt 50340
   taaacgaaag tatagctaat gatgcggata tcgttcttaa tagacacgcc attaccatgt 50400
   atgataaaat tcttagttat atatactctg agataaaaca aggacgcgtt aataaaaaca 50460
   tgcttaagtt agtttatatc tttgagcctg aaaaagatat cagagaactt ctgctagaaa 50520
   tcatatatga tattcctgga gatatcctat ctattattga tgcaaaaaac gacgattgga 50580
   aaaaatattt tattagtttt tataaagcta attttattaa cggtaataca tttattagtg 50640
   atagaacgtt taacgaggac ttattcagag ttgttgttca aatagatccc gaatatttcg 50700
   ataatgaacg aattatgtct ttattctcta cgagtgctgc ggacattaaa cgatttgatg 50760
   agttagatat taataacagt tatatatcta atataattta tgaggtgaac gatatcacat 50820
   tagatacaat ggatgatatg aagaagtgtc aaatctttaa cgaggatacg tcgtattatg 50880
   ttaaggaata caatacatac ctgtttttgc acgagtcgga tcccatggtc atagagaacg 50940
   gaatactaaa gaaactgtca tctataaaat ccaagagtag acggctgaac ttgtttagca 51000
   aaaacatttt aaaatattat ttagacggac aattggctcg tctaggtctt gtgttagatg 51060
   attataaagg agacttgtta gttaaaatga taaaccatct taagtctgtg gaggatgtat 51120
   ccgcattcgt tcgattttct acagataaaa accctagtat tcttccatcg ctaatcaaaa 51180
   ctattttagc tagttataat atttccatca tcgtcttatt tcaaaggttt ttgagagata 51240
   atctatatca tgtagaagaa ttcttggata aaagcatcca tctaaccaag acggataaga 51300
   aatatatact tcaattgata agacacggta gatcatagaa cagaccaaat atattattaa 51360
   taatttgtat atacatagat ataattatca cacatttttg ataaatggga actgctgcaa 51420
   caattcagac tcccaccaaa ttaatgaata aagaaaatgc agaaatgatt ttggaaaaaa 51480
   ttgttgatca tatagttatg tatattagtg acgaatcaag tgattcagaa aataatcctg 51540
   aatatattga ttttcgtaac agatacgaag actatagatc tctcattata aaaagtgatc 51600
   acgagtttgt aaagctatgt aaaaatcatg cggagaaaag ttctccagaa acgcaacaaa 51660
   tgattatcaa acacatatac gaacaatatc ttattccagt atctgaagta ctattaaaac 51720
   ctataatgtc catgggtgac ataattacat ataacggatg taaagacaat gaatggatgc 51780
   tagaacaact ctctacccta aactttaaca atctccgcac atggaactca tgtagcatag 51840
   gcaatgtaac gcgtctgttt tatacatttt ttagttatct gatgaaagat aaactaaata 51900
   tataagtata atcccattct aatactttaa cctgatgtat tagcatctta ttagaatatt 51960
   aacctaacta aaagacataa cataaaaact cattacatag ttgataaaaa gcggtaggat 52020
   ataaatatta tggctgccac cgttccgcgt tttgacgacg tgtacaaaaa tgcacaaaga 52080
   agaattctag atcaagaaac attttttagt agaggtctaa gtagaccgtt aatgaaaaac 52140
   acatatctat ttgataatta cgcgtatgga tggataccag aaactgcaat ttggagtagt 52200
   agatacgcaa acctagatgc tagtgactat tatcccattt cgttgggatt acttaaaaag 52260
   ttcgagtttc tcatgtctct atataaaggt cctattcccg tatatgaaga aaaagtaaat 52320
   actgaattca tagccaatgg atcgttctct ggtagatacg tatcatatct tcgaaagttt 52380
   tctgcccttc caacaaacga gtttattagt tttttgttac tgacttccat tccaatctat 52440
   aatatcttgt tctggtttaa aaacacacag tttgatatta ctaaacacac attattcaga 52500
   tacgtctata cagataatgc caaacacctg gcgttggcta ggtatatgca tcaaacagga 52560
   gactataagc ctttgtttag tcgtctcaaa gagaattata tatttaccgg tcccgttcca 52620
   ataagtatca aagatataga tcaccctaat cttagtagag caagaagtcc atccgattat 52680
   gagacattag ctaatattag tactatattg tactttacca agtatgatcc ggtattaatg 52740
   tttttattgt tttacgtacc tgggtattca attactacaa aaattactcc agccgtagaa 52800
   tatctaatgg ataaactgaa tctaacaaag agcgacgtac aactgttgta aattatttta 52860
   tgcttcgtaa aatgtaggtt ttgaaccaaa cattctttca aagaatgaga tgcataaaac 52920
   tttattatcc aatagattga ctatttcgga cgtcaatcgt ttaaagtaaa cttcgtaaaa 52980
   tattctttga tcactgccga gtttaaaact tctatcgata attgtctcat atgttttaat 53040
   atttacaagt tttttggtcc atggtacatt agccggacaa atatatgcaa aataatatcg 53100
   ttctccaagt tctatagttt ctggattatt tttattatat tcagtaacta aatacatatt 53160
   agggttatct gcggatttat aatttgagtg atgcattcta ctcaacataa ataattctag 53220
   aggagacgat ctactatcaa attcggatcg taaatctgtt tctaaagaac ggagaatatc 53280
   tatacatacc tgattagaat tcatccgtcc ttcagacaac atctcagaca gtctggtttt 53340
   gtacatctta atcatattct tatgaaactt ggaaacatct cttctagttt cactagtacc 53400
   tttattaatt ctctcaggta cagattttga attcgacgat gctgagtatt tcatcgttgt 53460
   atatttcttc ttcgattgca taatcagatt cttatatacc gcctcaaact ctattttaaa 53520
   attattaaac aatactctat tattaatcag tcgttctaac tctttcgcta tttctataga 53580
   cttatctaca tcttgactgt ctatctctgt aaacacggag tcggtatctc catacacgct 53640
   acgaaaacga aatctgtaat ctataggcaa cgatgttttc acaatcggat taatatctct 53700
   atcgtccata taaaatggat tacttaatgg attggcaaac cgtaacatac cgttagataa 53760
   ctctgctcca tttagtaccg attctagata caagatcatt ctacgtccta tggatgtgca 53820
   actcttagcc gaagcgtatg agtatagagc actatttcta aatcccatca gaccatatac 53880
   tgagttggct actatcttgt acgtatattg catggaatca tagatggcct tttcagttga 53940
   actggtagcc tgttttagca tctttttata tctggctctc tctgccaaaa atgttcttaa 54000
   tagtctagga atggttcctt ctatcgatct atcgaaaatt gctatttcag agatgaggtt 54060
   cggtagtcta ggttcacaat gaaccgtaat atatctagga ggtggatatt tctgaagcaa 54120
   tagctgatta tttatttctt cttccaatct attggtacta acaacgacac cgactaatgt 54180
   ttccggagat agatttccaa agatacacac attaggatac agactgttat aatcaaagat 54240
   taatacatta ttactaaaca ttttttgttt tggagcaaat accttaccgc cttcataagg 54300
   aaacttttgt tttgtttctg atctaactaa gatagtttta gtttccaaca atagctttaa 54360
   cagtggaccc ttgatgactg tactcgctct atattcgaat accatggatt gaggaagcac 54420
   atatgttgac gcacccgcgt ctgtttttgt ttctactcca taatactccc acaaatactg 54480
   acacaaacaa gcatcatgaa tacagtatct agccatatct aaagctatgt ttagattata 54540
   atccttatac atctgagcta aatcaacgtc atcctttccg aaagataatt tatatgtatc 54600
   attaggtaaa gtaggacata atagtacgac tttaaatcca ttttcccaaa tatctttacg 54660
   aattacttta catataatat cctcatcaac agtcacataa ttacctgtgg ttaaaacctt 54720
   tgcaaatgca gcggctttgc ctttcgcgtc tgtagtatcg tcaccgatga acgtcatttc 54780
   tctaactcct ctatttaata ctttacccat gcaactgaac gcgttcttgg atatagaatc 54840
   caatttgtac gaatccaatt tttcaaattt ttgaatgaat gaatatagat cgaaaaatat 54900
   agttccatta ttgttattaa cgtgaaacgt agtattggcc atgccgccta ctcccttatg 54960
   actagactga tttctctcat aaatacagag atgtacagct tcctttttgt ccggagatct 55020
   aaagataatt ttctctcctg ttaataactc tagacgatta gtaatatatc tcagatcaaa 55080
   gttatgtccg ttaaaggtaa cgacgtagtc gaacgttagt tccaacaatt gtttagctat 55140
   tcgtaacaaa actatttcag aacatagaac tagttctcgt tcgtaatcca tttccattag 55200
   tgactgtatc ctcaaacatc ctctatcgac ggcttcttgt atttcctgtt ccgttaacat 55260
   ctcttcatta atgagcgtaa acaataatcg tttaccactt aaatcgatat aacagtaact 55320
   tgtatgcgag attgggttaa taaatacaga aggaaacttc ttatcgaagt gacactctat 55380
   atctagaaat aagtacgatc ttgggatatc gaatctaggt atttttttag cgaaacagtt 55440
   acgtggatcg tcacaatgat aacatccatt gttaatcttt gtcaaatatt gctcgtccaa 55500
   cgagtaacat ccgtctggag atatcccgtt agaaatataa aaccaactaa tattgagaaa 55560
   ttcatccatg gtggcatttt gtatgctgcg tttctttggc tcttctatca accacatatc 55620
   tgcgacggag cattttctat ctttaatatc tagattataa cttattgtct cgtcaatgtc 55680
   tatagttctc atctttccca acggcctcgc attaaatgga ggaggagaca atgactgata 55740
   tatttcgtcc gtcactacgt aataaaagta atgaggaaat cgtataaata cggtctcgcc 55800
   atttcgacat ctggatttca gatataaaaa tctgttttca ccgtgacttt caaaccaatt 55860
   aatgcaccga acatccattt atagaattta gaaatatatt ttcatttaaa tgaatcccaa 55920
   acattgggga agagccgtat ggaccattat ttttatagta ctttcgcaag cgggtttaga 55980
   cggcaacata gaagcgtgta aacgaaaact atatactata gttagcactc ttccatgtcc 56040
   tgcatgtaga cggcacgcga ctatcgctat agaggacaat aatgtcatgt ctagcgatga 56100
   tctgaattat atttattatt ttttcatcag attatttaac aatttggcat ctgatcccaa 56160
   atacgcgatc gatgtgacaa aggttaaccc tttataaact taacccatta taaaacttat 56220
   gattagtcac aactgaaata accgcgtgat tattttttgg tataattcta cacggcatgg 56280
   tttctgtgac tatgaattca acccccgtta cattagtgaa atctttaaca aacagcaagg 56340
   gttcgtcaaa gacataaaac tcattgttta caatcgaaat agacccccta tcacacttaa 56400
   aataaaaaat atccttatcc tttaccacca aataaaattc tgattggtca atgtgaatgt 56460
   attcacttaa cagttccaca aatttattta ttaactccga ggcacataca tcgtcggtat 56520
   tttttatggc aaactttact cttccagcat ccgtttctaa aaaaatatta acgagttcca 56580
   tttatatcat ccaatattat tgaaatgacg ttgatggaca gatgatacaa ataagaaggt 56640
   acggtacctt tgtccaccat ctcctccaat tcatgctcta ttttgtcatt aactttaatg 56700
   tatgaaaaca gtacgccaca tgcttccatg acagtgtgta acactttgga tacaaaatgt 56760
   ttgacattag tataattgtc caagactgtc aatctataat agatagtagc tataatatat 56820
   tctatgatgg tattgaagaa gatgacaacc ttggcatatt gatcatttaa cacagacatg 56880
   gtatcaacag atagcttgaa tgaaagagaa tcagtaattg gaataagcgt cttctcgata 56940
   gagtgtccgt ataccaacat gtctgatatt ttgatgtatt ccattaaatt atttagtttt 57000
   ttctttttat tctcgttaaa cagcatttct gtcaacggac cccaacatcg ttgaccgatt 57060
   aagttttgat tgatttttcc gtgtaaggcg tatctagtca gatcgtatag cctatccaat 57120
   aatccatcgt ctgtgtgtag atcacatcgt acacttttta attctctata gaagagcgac 57180
   agacatctgg agcaattaca gacagcaatt tctttattct ctacagatgt aagatacttg 57240
   aagacattcc tatgatgatg cagaattttg gataacacgg tattgatggt atctgttacc 57300
   ataattcctt tgatggctga tagtgtcaga gcacaagatt tccaatcttt gacaattttt 57360
   agcaccatta tctttgtttt gatatctata tcagacagca tggtgcgtct gacaacacag 57420
   ggattaagac ggaaagatga aatgattctc tcaacatctt caatagatac cttgctattt 57480
   tttctggcat tatctatatg tgcgagaata tcctctagag aatcagtatc ctttttgatg 57540
   atagtggatc tcaatgacat gggacgttta aaccttctta ttctatcacc agattgcatg 57600
   gtgatttgtc ttctttcttt tatcataatg taatctctaa attcatcggc aaattgtcta 57660
   tatctaaaat cataatatga gatgtttacc tctacaaata tctgttcgtc caatgttaga 57720
   gtatttacat cagttttgta ttccaaatta aacatggcaa cggatttaat tttatattcc 57780
   tctattaagt cctcgtcgat aataacagaa tgtagataat catttaatcc gtcgtacatg 57840
   gttggaagat gcttgttgac aaaatcttta attgtcttga tgaaggtggg actatatcta 57900
   acatcttgat taataaaatt tataacattg tccataggat actttgtaac tagttttata 57960
   cacatctctt catcggtaag tttagacaga atatcgtgaa caggtggtat attatattca 58020
   tcagatatac gaagaacaat gtccaaatct atattgttta atatattata tagatgtagc 58080
   gtagctccta caggaatatc tttaactaag tcaatgattt catcaaccgt tagatctatt 58140
   ttaaagttaa tcatataggc attgattttt aaaaggtatg tagccttgac tacattctca 58200
   ttaattaacc attccaagtc attgtgtgta agaagattat attctatcat aagcttgact 58260
   acatttggtc ccgataccat taaagaattc ttatgatata aggaaacaga ttttaggtac 58320
   tcatctactc tacaagaatt ttggagagcc ttaacgatat cagtgacgtt tattatttca 58380
   ggaggaaaga atctaacatt gagaatatcg gaattaatag cttccagata cagtgatttt 58440
   ggcaatagtc cgtgtaatcc ataatccagt aacacgagct ggtgcttgct agacaccttt 58500
   tcaatgttta atttttttga aataagcttt gataaagcct tcctcgcaaa ttccggatac 58560
   atgaacatgt cggcgacatg attaagtatt gttttttcat tatttttata ttttctcaac 58620
   aagttctcaa taccccaata gatgatagaa tatcacccaa tgcgtccatg ttgtctattt 58680
   ccaacaggtc gctatatcca ccaatagaag tttttccaaa aaagattcta ggaacagttc 58740
   taccaccagt aatttgttca aaataatccc gcaattcatt ttcgggttta aattctttaa 58800
   tatcgacaat ttcatacgct cctcttttga aactaaactt atttagaata tccagtgcat 58860
   ttctacaaaa aggacatgta tacttgacaa aaattgtcac tttgttattg gccaaccttt 58920
   gttgtacaaa ttcctcggcc attttaatat ttaagtgata taaaactatc tcgacttatt 58980
   taactcttta gtcgagatat atggacgcag atagctatat gatagccaac tacagaaggc 59040
   aaacgctata aaaaacataa ttacgacgag catatttata aatattttta ttcagcatta 59100
   cttgatatag taatattagg cacagtcaaa cattcaacca ctctcgatac attaactctc 59160
   tcattttctt taacaaattc tgcaatatct tcgtaaaaag attcttgaaa ctttttagaa 59220
   tatctatcga ctctagatga aatagcgttc gtcaacatac tatgttttgt atacataaag 59280
   gcgcccattt taacagtttc tagtgacaaa atgctagcga tcctaggatc ctttagaatc 59340
   acatagattg acgattcgtc tctcttagta actctagtaa aataatcata caatctagta 59400
   cgcgaaataa tattatcctt gacttgagga gatctaaaca atctagtttt gagaacatcg 59460
   ataagttcat cgggaatgac atacatacta tctttaatag aactcttttc atccagttga 59520
   atggattcgt ccttaaccaa ctgattaatg agatcttcta ttttatcatt ttccagatga 59580
   tatgtatgtc cattaaagtt aaattgtgta gcgcttcttt ttagtctagc agccaatact 59640
   ttaacatcac taatatcgat atacaaagga gatgatttat ctatggcatt aagaattcgt 59700
   ttttcgacat ccgtcaaaac caattccttt ttgcctgtat catccagttt tccatccttt 59760
   gtaaagaaat tattttctac tagactatta ataagactga taaggattcc tccataattg 59820
   cacaatccaa actttttaac aaaactagac tttacgagat ctacaggaat gcgtacttca 59880
   ggttttttag cttgtgattt tttcttttgc ggacattttc tagtaaccaa ctcatctacc 59940
   atttcattga ttttagcagt gaaataagct ttcaatgcac gggcactgat actattgaaa 60000
   acgagttgat cttcaaattc cgccatttaa gttcaccaaa caacttttaa atacaaatat 60060
   atcaatagta gtagaataag aactataaaa aaaataataa ttaaccaacc ccaacaaccg 60120
   gtattattag ttgatgtggt agttttctca tcacttagaa cagatttaac aatttctata 60180
   aagtctgtca aatcatcttc cggagacccc ataaatacac caaatatagc ggcgtacaac 60240
   ttatccattt atacattgaa tattggcttt tctttatcgc tatcttcatc atattcatca 60300
   tcaatatcaa caagtcccag attacgagcc agatcttctt ctacattttc agtcattgat 60360
   acacgttcac tatctccaga gagtccgata acgttagcca ccacttctct atcaatgatt 60420
   agtttcttga gcgcgaaagt aatttttgtt tccgttccgg atctatagaa gacgataggt 60480
   gtgataattg ccttggccaa ttgtctttct cttttactga gtgattctag ttcaccttct 60540
   atagatctga gaatggatga ttctccagtc gaaacatatt ctaccatgga tccgtttaat 60600
   ttgttgatga agatggattc atccttaaat gttttctctg taatagtttc caccgaaaga 60660
   ctatgcaaag aatttggaat gcgttccttg tgcttaatgt ttccatagac ggcttctaga 60720
   agttgataca acataggact agccgcggta acttttattt ttagaaagta tccatcgctt 60780
   ctatcttgtt tagatttatt tttataaagt ttagtctctc cttccaacat aataaaagtg 60840
   gaagtcattt gactagataa actatcagta agttttatag agatagacga acaattagcg 60900
   tattgagaag catttagtgt aacgtattcg atacattttg cattagattt actaatcgat 60960
   tttgcatact ctataacacc cgcacaagtc tgtagagaat cgctagatgc agtaggtctt 61020
   ggtgaagttt caactctctt cttgattacc ttactcatga ttaaacctaa ataattgtac 61080
   tttgtaatat aatgatatat attttcactt tatctcattt gagaataaaa atgtttttgt 61140
   ttaaccactg catgatgtac agatttcgga atcgcaaacc accagtggtt ttattttatc 61200
   cttgtccaat gtgaattgaa tgggagcgga tgcgggtttc gtacgtagat agtacattcc 61260
   cgtttttaga ccgagactcc atccgtaaaa atgcatactc gttagtttgg aataactcgg 61320
   atctgctata tggatattca tagattgact ttgatcgatg aaggctcccc tgtctgcagc 61380
   catttttatg atcgtctttt gtggaatttc ccaaatagtt ttataaactc gcttaatatc 61440
   ttctggaagg tttgtattct gaatggatcc accatctgcc ataatcctat tcttgatctc 61500
   atcattccat aattttctct cggttaaaac tctaaggaga tgcggattaa ctacttgaaa 61560
   ttctccagac aatactctcc gagtgtaaat attactggta tacggttcca ccgactcatt 61620
   atttcccaaa atttgagcag ttgatgcagt cggcataggt gccaccaata aactatttct 61680
   aagaccgtat gttctgattt tatcttttag aggttcccaa ttccaaagat ccgacggtac 61740
   aacattccaa agatcatatt gtagaatacc gttactggcg tacgatccta catatgtatc 61800
   gtatggtcct tccttctcag ctagttcaca actcgcctct aatgcaccgt aataaatggt 61860
   ttcgaagatc ttcttattta gatcttgtgc ttccaggcta tcaaatggat aatttaagag 61920
   aataaacgcg tccgctaatc cttgaacacc aataccgata ggtctatgtc tcttattaga 61980
   gatttcagct tctggaatag gataataatt aatatctata attttattga gatttctgac 62040
   aattactttg accacatcct tcagtttgag aaaatcaaat cgcccatcta ttacaaacat 62100
   gttcaaggca acagatgcca gattacaaac ggctacctca ttagcatccg catattgtat 62160
   tatctcagtg caaagattac tacacttgat agttcctaaa ttttgttgat tactcttttt 62220
   gttacacgca tccttataaa gaatgaatgg agtaccagtt tcaatctgag attctataat 62280
   cgctttccag acgactcgag cctttattat agatttgtat ctcctttctc tttcgtatag 62340
   tgtatacaat cgttcgaact cgtctcccca aacattgtcc aatccaggac attcatccgg 62400
   acacatcaac gaccactctc cgtcatcctt cactcgtttc ataaagagat caggaatcca 62460
   aagagctata aatagatctc tggttctatg ttcctcgttt cctgtattct ttttaagatc 62520
   gaggaacgcc ataatatcag aatgccacgg ttccaagtat atggccataa ctccaggccg 62580
   tttgtttcct ccctgatcta tgtatctagc ggtgttatta taaactctca acattggaat 62640
   aataccgttt gatataccat tggtaccgga gatatagctt ccactggcac gaatattact 62700
   aattgataga cctattcccc ctgccatttt agagattaat gcgcatcgtt ttaacgtgtc 62760
   atagataccc tctatgctat catcgatcat gttaagtaga aaacagctag acatttggtg 62820
   acgactagtt cccgcattaa ataaggtagg agaagcgtgc gtaaaccatt tttcagaaag 62880
   tagattgtac gtctcaatag ctgagtctat atcccattga tgaattccta ctgcgacacg 62940
   cattaacatg tgctgaggtc tttcaacgat cttgttgttt attttcaaca agtaggattt 63000
   ttccaaagtt ttaaaaccaa aatagttgta tgaaaagtct cgttcgtaaa taataaccga 63060
   gttgagttta tccttatatt tgttaactat atccatggtg atacttgaaa taatcggaga 63120
   atgtttccca tttttaggat taacatagtt gaataaatcc tccatcactt cactaaatag 63180
   tttttttgtt tccttgtgta gatttgatac ggctattctg gcggctagaa tggcataatc 63240
   cggatgttgt gtagtacaag tggctgctat ttcggctgcc agagtgtcca attctaccgt 63300
   tgttactcca ttatatattc cttgaataac cttcatagct attttaatag gatctatatg 63360
   atccgtgttt aagccataac ataattttct aatacgagac gtgattttat caaacatgac 63420
   attttccttg tatccatttc gtttaatgac aaacattttt gttggtgtaa taaaaaaatt 63480
   atttaacttt tcattaatag ggatttgacg tatgtagcgt acaaaatgat tgttcctggt 63540
   atatagataa agagtcctat atatttgaaa atcgttacgg ctcgattaaa ctttaatgat 63600
   tgcatagtga atatatcatt aggatttaac tccttgacta tcatggcggc gccagaaatt 63660
   accatcaaaa gcattaatac agttataccg atcgcagtta gaacggttat agcatccacc 63720
   atttatatct aaaaattaga tcaaagaata tgtgacaaag tcctagttgt atactgagaa 63780
   ttgacgaaac aatgtttctt acatattttt ttcttattag taaccgactt aatagtagga 63840
   actggaaaac tagacttgat tattctataa gtatagatac ccttccaaat aatattctct 63900
   ttgataaaag ttccagaaaa tgtagaattt tttaaaaagt tatcttttgc tattaccaag 63960
   attgtgttta gacgcttatt attaatatga gtgatgaaat ccacaccgcc tctagatatc 64020
   gcctttattt ccacattaga tggtaaatcc aatagtgaaa ctatcttttt aggaatgtat 64080
   ggactcgcgt ttagaggagt aaacgtctta ggcgtcggaa aggatgattc atcaaacgaa 64140
   taaacaattt cacaaatgga tgttaatgta ttagtaggaa attttttgac gctattggaa 64200
   ttgaagattc taatggatga tgttctacct atttcatccg ataacatgtt aatttccgac 64260
   accaacggtt ttaatatttc gatgatatac ggtagtctct ctttcggact tatatagctt 64320
   attccacaat acgagtcatt atatactcca aaaaacaaaa taactagtat aaaatctgta 64380
   tcgaatggga aaaacgaaat tatcgacata ggtatagaat ccggaacatt gaacgtatta 64440
   atacttaatt ctttttctgt ggtaagtacc gataggttat tgacattgta tggttttaaa 64500
   tattctataa cttgagactt gatagatatt agtgatgaat tgaaaattat ttttatcacc 64560
   acgtgtgttt caggatcatc gtcgacgccc gtcaaccaac cgaacggagt aaaataaata 64620
   tcattaatat atgctctaga tattagtatt tttatcaatc ctttgattat catcttctcg 64680
   taggcgaatg attccatgat caagagtgat ttaagaacat cctccggagt attaatgggc 64740
   ttagtaaaca gtccatcgtt gcaataataa aagttatcca agttaaagga tattatgcat 64800
   tcgtttaaag atatcacctc atctgacgga gacaattttt tggtaggttt tagagacttt 64860
   gaagctactt gtttaacaaa gttattcatc gtcgtctact attctattta attttgtagt 64920
   taatttatca catatcacat taattgactt tttggtccac ttttccatac gtttatattc 64980
   ttttaatcct gcgttatccg tttccgttat atccagggat agatcttgca agttaaatag 65040
   aatgctctta aataatgtca ttttcttatc cgctaaaaat ttaaagaatg tataaacctt 65100
   tttcagagat ttgaaactct taggtggtgt cctagtacac aatatcataa acaaactaat 65160
   aaacattcca cattcagatt ccaacagctg attaacttcc acattaatac agcctatttt 65220
   cgctccaaat gtacattcga aaaatctgaa taaaacatcg atgtcacaat ttgtattatc 65280
   caatacagaa tgtttgtgat tcgtgttaaa accatcggag aaggaataga aataaaaatt 65340
   attatagtgg tggaattcag ttggaatatt gcctccggag tcataaaagg atactaaaca 65400
   ttgtttttta tcataaatta cacatttcca atgagacaaa taacaaaatc caaacattac 65460
   aaatctagag gtagaacttt taattttgtc tttaagtata tacgataaga tatgtttatt 65520
   cataaacgcg tcaaattttt catgaatcgc taaggagttt aagaatctca tgtcaaattg 65580
   tcctatataa tccacttcgg atccataagc aaactgagag actaagttct taatacttcg 65640
   attgctcatc caggctcctc tctcaggctc tattttcatc ttgacgacct ttggattttc 65700
   accagtatgt attcctttac gtgataaatc atcgattttc aaatccattt gtgagaagtc 65760
   tatcgcctta gatacttttt cccgtagtcg aggtttaaaa aaatacgcta acggtatact 65820
   agtaggtaac tcaaagacat catatataga atggtaacgc gtctttaact cgtcggttaa 65880
   ctctttcttt tgatcgagtt cgtcgctact attgggtctg ctcaggtgcc ccgactctac 65940
   tagttccaac atcataccga taggaataca agacactttg ccggcggttg tagatttatc 66000
   atatttttcc actacatatc cgttacaatt tgttaaaaat ttagatacat ctatattgct 66060
   acataatcca gctagtgaat atatatgaca taataaattg gtaaatccta gttctggtat 66120
   tttactaatt actaaatctg tatatctttc catttatcat ggaaaagaat ttaccagata 66180
   tcttcttttt tccaaactgc gttaatgtat tctcttacaa atattcacaa gatgaattca 66240
   gtaatatgag taaaacggaa cgtgatagtt tctcattggc cgtgtttcca gttataaaac 66300
   atagatggca taacgcacac gttgtaaaac ataaaggaat atacaaagtt agtacagaag 66360
   cacgtggaaa aaaagtatct cctccatcac taggaaaacc cgcacacata aacctaaccg 66420
   cgaagcaata tatatacagt gaacacacaa taagctttga atgttatagt tttctaaaat 66480
   gtataacaaa tacagaaatc aattcgttcg atgagtatat attaagagga ctattagaag 66540
   ctggtaatag tttacagata ttttccaatt ccgtaggtaa acgaacagat actataggtg 66600
   tactagggaa taagtatcca tttagcaaaa ttccattggc ctcattaact cctaaagcac 66660
   aacgagagat attttcagcg tggatttctc atagacctgt agttttaact ggaggaactg 66720
   gagtgggtaa gacgtcacag gtacccaagt tattgctttg gtttaattat ttatttggtg 66780
   gattctctac tctagataaa atcactgact ttcacgaaag accagtcatt ctatctcttc 66840
   ctaggatagc tttagttaga ttgcatagca ataccatttt aaaatcattg ggatttaagg 66900
   tactagatgg atctcctatt tctttacggt acggatctat accggaagaa ttaataaaca 66960
   aacaaccaaa aaaatatgga attgtatttt ctacccataa gttatctcta acaaaactat 67020
   ttagttatgg cactcttatt atagacgaag ttcatgagca tgatcaaata ggagatatta 67080
   ttatagcagt agcgagaaag catcatacga aaatagattc tatgttttta atgactgcca 67140
   cgttagagga tgacagggaa cggctaaaag tatttttacc taatcccgca tttatacata 67200
   ttcctggaga tacactgttt aaaattagcg aggtatttat tcataataag ataaatccat 67260
   cttccagaat ggcatacata gaagaagaaa agagaaattt agttactgct atacagatgt 67320
   atactcctcc tgatggatca tccggtatag tctttgtggc atccgttgca cagtgtcacg 67380
   aatataaatc atatttagaa aaaagattac cgtatgatat gtatattatt catggtaagg 67440
   tcttagatat agacgaaata ttagaaaaag tgtattcatc acctaatgta tcgataatta 67500
   tttctactcc ttatttggaa tccagcgtta ctatacgcaa tgttacacac atttatgata 67560
   tgggtagagt ttttgtcccc gctccttttg gaggatcgca acaatttatt tctaaatcta 67620
   tgagagatca acgaaaagga agagtaggaa gagttaatcc tggtacatac gtctatttct 67680
   atgatctgtc ttatatgaag tctatacagc gaatagattc agaatttcta cataattata 67740
   tattgtacgc taataagttt aatctaacac tccccgaaga tttgtttata atccctacaa 67800
   atttggatat tctatggcgt acaaaggaat atatagactc gttcgatatt agtacagaaa 67860
   catggaataa attattatcc aattattata tgaagatgat agagtatgct aaactttatg 67920
   tactaagtcc tattctcgct gaggagttgg ataactttga gaggacggga gaattaacta 67980
   gtattgtacg agaagccatt ttatctctaa atttacaaat taagatttta aattttaaac 68040
   ataaagatga tgatacgtat atacactttt gtaaaatatt attcggtgtc tataacggaa 68100
   caaacgctac tatatattat catagacctc taacgggata tatgaatatg atttcagata 68160
   ctatatttgt tcctgtagat aataactaaa aatcaaactc taatgaccac atcttttttt 68220
   agagatgaaa aattttccac atctcctttt gtagacacga ctaaacattt tgcagaaaaa 68280
   agtttattag tgtttagata atcgtatact tcatcagtgt agatagtaaa tgtgaacaga 68340
   taaaaggtat tcttgctcaa tagattggta aattccatag aatatattaa tcctttcttc 68400
   ttgagatccc acatcatttc aaccagagac gttttatcca atgatttacc tcgtactata 68460
   ccacatacaa aactagattt tgcagtgacg tcgtacctgg tattcctacc aaacaaaatt 68520
   ttacttttag ttcttttaga aaattctaag gtagaatctc tatttgccaa tatgtcatct 68580
   atggaattac cactagcaaa aaatgataga aatatatatt gatacatcgc agctggtttt 68640
   gatctactat actttaaaaa cgaatcagat tccataattg cctgtatatc atcagctgaa 68700
   aaactatgtt ttacacgtat tccttcggca tttcttttta atgatatatc ttgtttagac 68760
   aatgataaag ttatcatgtc catgagagac gcgtctccgt atcgtataaa tatttcatta 68820
   gatgttagac gcttcattag gggtatactt ctataaggtt tcttaatcag tccatcattg 68880
   gttgcgtcaa gaactactat cggatgttgt tgggtatctc tagtgttaca catggcctta 68940
   ctaaagtttg ggtaaataac tatgatatct ctattaatta tagatgcata tatttcattc 69000
   gtcaaggata ttagtatcga cttgctatcg tcattaatac gtgtaatgta atcatataaa 69060
   tcatgcgata gccaaggaaa atttaaatag atgttcatca tataatcgtc gctataattc 69120
   atattaatac gttgacattg actaatttgt aatatagcct cgccacgaag aaagctctcg 69180
   tattcagttt catcgataaa ggataccgtt aaatataact ggttgccgat agtctcatag 69240
   tctattaagt ggtaagtttc gtacaaatac agaatcccta aaatattatc taatgttgga 69300
   ttaatcttta ccataactgt ataaaatgga gacggagtca taactatttt accgtttgta 69360
   cttactggaa tagatgaagg aataatctcc ggacatgctg gtaaagaccc aaatgtctgt 69420
   ttgaagaaat ccaatgttcc aggtcctaat ctcttaacaa aaattacgat attcgatccc 69480
   gatatccttt gcattctatt taccagcata tcacgaacta tattaagatt atctatcatg 69540
   tctattctcc caccgttata taaatcgcct ccgctaagaa acgttagtat atccatacaa 69600
   tggaatactt catttctaaa atagtattcg ttttctaatt ctttaatgtg aaatcgtata 69660
   ctagaaaggg aaaaattatc tttgagtttt ccgttagaaa agaaccacga aactaatgtt 69720
   ctgattgcgt ccgattccgt tgctgaatta atggatttac accaaaaact catataactt 69780
   ctagatgtag aagcattcgc taaaaaatta gtagaatcaa aggatataag tagatgttcc 69840
   aacaagtgag caattcccaa gatttcatct atatcattct cgaatccgaa attagaaatt 69900
   cccaagtaga tatccttttt catccgatcg ttgatgaaaa tacgaacttt attcggtaag 69960
   acaatcattt actaaggagt aaaataggaa gtaatgttcg tatgtcgtta tcatcgtata 70020
   aattaaaggt gtgtttttta ccattaagtg acattataat tttaccaata ttggaattat 70080
   aatataggtg tatttgcgca ctcgcgacgg ttgatgcatc ggtaaatata gctgtatcta 70140
   atgttctagt cggtatttca tcatttcgct gtctaataat agcgttttct ctatctgttt 70200
   ccattacagc tgcctgaagt ttattggtcg gataatatgt aaaataataa gaaatacata 70260
   cgaataacaa aaataaaata agatataata aagatgccat ttagagatct aattttgttt 70320
   aacttgtcca aattcctact tacagaagat gaggaatcgt tggagatagt gtcttcctta 70380
   tgtagaggat ttgaaatatc ttataatgac ttgataactt actttccaga taggaaatac 70440
   cataaatata tttataaagt atttgaacat gtagatttat cggaggaatt aagtatggaa 70500
   ttccatgata caactctgag agatttagtc tatcttagat tgtacaagta ttccaagtgt 70560
   atacggccgt gttataaatt aggagataat ctaaaaggca tagttgttat aaaggacagg 70620
   aatatttata ttagggaagc aaatgatgac ttgatagaat atctcctcaa ggaatacact 70680
   cctcagattt atacatattc taatgagcgc gtccccataa ctggttcaaa attaattctt 70740
   tgtggatttt ctcaagttac atttatggcg tatacaacgt cgcatataac aacaaataaa 70800
   aaggtagatg ttctcgtttc caaaaaatgt atagatgaac tagtcgatcc aataaattat 70860
   caaatacttc aaaatttatt tgataaagga agcggaacaa taaacaaaat actcaggaag 70920
   atattttatt cggtaacagg tggccaaact ccataggtag ctttttctat ttcggatttt 70980
   agaatttcca aattcaccag cgatttatcg gttttggtga aatccaagga tttattaatg 71040
   tccacaaatg ccatttgttt tgtctgtgga ttgtatttga aaatggaaac gatgtagtta 71100
   gatagatgcg ctgcgaagtt tcctattagg gttccgcgct tcacgtcacc cagcatactt 71160
   gaatcaccat cctttaaaaa aaatgataag atatcaacat ggagtatatc atactcgaat 71220
   tttaattctt ctactgactc actgacattt tcacaaatac tacaatacgg tttaccgaaa 71280
   ataatcagta cgttcttcat ttatgggtat caaaaactta aaatcgttac tgctggaaaa 71340
   taaatcactg acgatattag atgataattt atacaaagta tacaatggaa tatttgtgga 71400
   tacaatgagt atttatatag ccgtcgccaa ttgtgtcaga aacttagaag agttaactac 71460
   ggtattcata aaatacgtaa acggatgggt aaaaaaggga gggcatgtaa ccctttttat 71520
   cgatagagga agtataaaaa ttaaacaaga cgttagagac aagagacgta aatattctaa 71580
   attaaccaag gacagaaaaa tgttagaatt agaaaagtgt acatccgaaa tacaaaatgt 71640
   taccggattt atggaagaag aaataaaggc agaaatgcaa ttaaaaatcg ataaactcac 71700
   atttcaaata tatttatctg attctgataa cataaaaata tcattgaatg agatactaac 71760
   acatttcaac aataatgaga atgttacatt attttattgt gatgaacgag acgcagaatt 71820
   cgttatgtgt ctcgaggcta aaacacattt ctctaccaca ggagaatggc cgttgataat 71880
   aagtaccgat caggatacta tgctatttgc atctgctgat aatcatccta agatgataaa 71940
   aaacttaact caactgttta aatttgttcc ctcggcagag gataactatt tagcaaaatt 72000
   aacggcgtta gtgaatggat gtgatttctt tcctggactc tatggggcat ctataacacc 72060
   caccaactta aacaaaatac aattgtttag tgattttaca atcgataata tagtcactag 72120
   tttggcaatt aaaaattatt atagaaagac taactctacc gtagacgtgc gtaatattgt 72180
   tacgtttata aacgattacg ctaatttaga cgatgtctac tcgtatattc ctccttgtca 72240
   atgcactgtt caagaattta tattttccgc attagatgaa aaatggaatg aatttaaatc 72300
   atcttattta gaaagcgtgc cgttaccctg ccaattaatg tacgcgttag aaccacgcaa 72360
   ggagattgat gtttcagaag ttaaaacttt atcatcttat atagatttcg aaaatactaa 72420
   atcagatatc gatgttataa aatctatatc ctcgatcttc ggatattcta acgaaaactg 72480
   taacacgata gtattcggca tctataagga taatttacta ctgagtataa ataattcatt 72540
   ttactttaac gatagtctgt taataaccaa tactaaaagt gataatataa taaatatagg 72600
   ttactagatt aaaaatggtg ttccaactcg tgtgctctac atgcggtaaa gatatttctc 72660
   acgaacgata taaattgatt atacgaaaaa aatcattaaa ggatgtactc gtcagtgtaa 72720
   agaacgaatg ttgtaggtta aaattatcta cacaaataga acctcaacgt aacttaacag 72780
   tgcaacctct attggatata aactaatatg gatccggtta attttatcaa gacatatgcg 72840
   cctagaggtt ctattatttt tattaattat accatgtcat taacaagtca tttgaatcca 72900
   tcgatagaaa aacatgtggg tatttattat ggtacgttat tatcggaaca cttggtagtt 72960
   gaatctacct atagaaaagg agttcgaata gtcccattgg atagtttttt tgaaggatat 73020
   cttagtgcaa aagtatacat gttagagaat attcaagtta tgaaaatagc agctgatacg 73080
   tcattaactt tattgggtat tccgtatgga tttggtcatg atagaatgta ttgttttaaa 73140
   ttggtagctg actgttataa aaatgccggt attgatacat cgtctaaacg aatattgggc 73200
   aaagatattt ttctgagcca aaacttcaca gacgataata gatggataaa gatatatgat 73260
   tctaataatt taacattttg gcaaattgat taccttaaag ggtgagttaa tatgcataac 73320
   tactcctccg ttgttttttc cctcgttctt tttcttaacg ttgtttgcca tcactctcat 73380
   aatgtaaaga tattctaaaa tggtaaactt ttgcatatcg gacgcagaaa ttggtataaa 73440
   tgttgtaatt gtattatttc ccgtcaatgg actagtcaca gctccatcag ttttatatcc 73500
   tttagagtat ttctcactcg tgtctaacat tctagagcat tccatgatct gtttatcgtt 73560
   gatattggcc ggaaagatag attttttatt ttttattata ttactattgg caattgtaga 73620
   tataacttct ggtaaatatt tttctacctt ttcaatctct tctattttca agccggctat 73680
   atattctgct atattgttgc tagtatcaat accttttctg gctaagaagt catatgtggt 73740
   attcactata tcagttttaa ctggtagttc cattagcctt tccacttctg cagaataatc 73800
   agaaattggt tctttaccag aaaatccagc tactataata ggctcaccga tgatcattgg 73860
   caaaatccta tattgtacca gattaatgag agcatatttc atttccaata attctgctag 73920
   ttcttgagac attgatttat ttgatgaatc tagttggttc tctagatact ctaccatttc 73980
   tgccgcatac aataacttgt tagataaaat cagggttatc aaagtgttta gcgtggctag 74040
   aatagtgggc ttgcatgtat taaagaatgc ggtagtatga gtaaaccgtt ttaacgaatt 74100
   atatagtctc cagaaatctg tggcgttaca tacatgagcc gaatgacatc gaagattgtc 74160
   caatattttt aatagctgct ctttgtccat tatttctata tttgactcgc aacaattgta 74220
   gataccatta atcaccgatt cctttttcga tgccggacaa tagcacaatt gtttagcttt 74280
   ggactctatg tattcagaat taatagatat atctctcaat acagattgca ctatacattt 74340
   tgaaactatg tcaaaaattg tagaacgacg ctgttctgca gccatttaac tttaaataat 74400
   ttacaaaaat ttaaaatgag catccgtata aaaatcgata aactgcgcca aattgtggca 74460
   tatttttcag agttcagtga agaagtgtct ataaatgtag actcgacgga tgagttaatg 74520
   tatatttttg ccgccttggg cggatctgta aacatttggg caattatacc tctaagtgca 74580
   tcagtgtttt accgaggagc cgaaaatatt gtgtttaatc ttcctgtgtc caaggtaaaa 74640
   tcgtgtttgt gtagttttca caatgatgcc atcatagata tagaacctga tctggaaaat 74700
   aatctagtaa aactttctag ttatcatgta gtaagtgtcg attgtaacaa ggaactgatg 74760
   cctattagga cagatactac tatttgtcta agtatagatc aaaagaaatc ttacgtgttt 74820
   aattttcaca agtatgaaga aaaatgttgt ggtagaaccg tcattcatct agaatggttg 74880
   ttgggcttta tcaagtgtat tagtcagcat cagcatctgg ctattatgtt taaagatgac 74940
   aatattatta tgaagactcc tggtaatact gatgcgtttt ccagggaata ttctatgact 75000
   gaatgttctc aagaactaca aaagttttct ttcaaaatag ctatctcgtc tctcaacaaa 75060
   ctacgaggat tcaaaaagag agtcaatgtt tttgaaacta gaatcgtaat ggataatgac 75120
   gataacattt taggaatgtt gttttcggat agagttcaat cctttaagat caacatcttt 75180
   atggcgtttt tagattaata ctttcaatga gataaatatg ggtggcggag taagtgttga 75240
   gctccctaaa cgggatccgc ctccgggagt acccactgat gagatgttat taaacgtgga 75300
   taaaatgcat gacgtgatag ctcccgctaa gcttttagaa tatgtgcata taggaccact 75360
   agcaaaagat aaagaggata aagtaaagaa aagatatcca gagtttagat tagtcaacac 75420
   aggacccggt ggtctttcgg cattgttaag acaatcgtat aatggaaccg cacccaattg 75480
   ctgtcgcact tttaatcgta ctcattattg gaagaaggat ggaaagatat cagataagta 75540
   tgaagagggt gcagtattag aatcgtgttg gccagacgtt cacgacactg gaaaatgcga 75600
   tgttgattta ttcgactggt gtcaggggga tacgttcgat agaaacatat gccatcagtg 75660
   gatcggttca gcctttaata ggagtaatag aactgtagag ggtcaacaat cgttaataaa 75720
   tctgtataat aagatgcaaa cattatgtag taaagatgct agtgtaccaa tatgcgaatc 75780
   atttttgcat tatttacgcg cacacaatac agaagatagc aaagagatga tcgattatat 75840
   tctaagacaa cagtctgcgg actttaaaca gaaatatatg agatgtagtt atcccactag 75900
   agataagtta gaagagtcat taaaatatgc ggaacctcga gaatgttggg atccagagtg 75960
   ttcgaatgcc aatgttaatt tcttactaac acgtaattat aataatttag gactttgcaa 76020
   tattgtacga tgtaatacca gcgtgaacaa cttacagatg gataaaactt cctcattaag 76080
   attgtcatgt ggattaagca atagtgatag attttctact gttcccgtca atagagcaaa 76140
   agtagttcaa cataatatta aacactcgtt cgacctaaaa ttgcatttga tcagtttatt 76200
   atctctcttg gtaatatgga tactaattgt agctatttaa atgggtgccg cggcaagcat 76260
   acagacgacg gtgaatacac tcagcgaacg tatctcgtct aaattagaac aagaagcgaa 76320
   cgctagtgct caaacaaaat gtgatataga aatcggaaat ttttatatcc gacaaaacca 76380
   tggatgtaac ctcactgtta aaaatatgtg ctctgcggac gcggatgctc agttggatgc 76440
   tgtgttatca gccgctacag aaacatatag tggattaaca ccggaacaaa aagcatacgt 76500
   gccagctatg tttactgctg cgttaaacat tcagacgagt gtaaacactg ttgttagaga 76560
   ttttgaaaat tatgtgaaac agacttgtaa ttctagcgcg gtcgtcgata acaaattaaa 76620
   gatacaaaac gtaatcatag atgaatgtta cggagcccca ggatctccaa caaatttgga 76680
   atttattaat acaggatcta gcaaaggaaa ttgtgccatt aaagcgttga tgcaattgac 76740
   gactaaggcc actactcaaa tagcacctag acaagttgct ggtacaggag ttcagtttta 76800
   tatgattgtt atcggtgtta taatattggc agcgttgttt atgtactatg ccaagcgtat 76860
   gttgttcaca tccaccaatg ataaaatcaa acttatttta gccaataagg aaaacgtcca 76920
   ttggactact tacatggaca cattctttag aacttctccg atggttattg ctaccacgga 76980
   tatgcaaaac tgaaaatata ttgataatat tttaatagat taacatggaa gttatcgctg 77040
   atcgtctaga cgatatagtg aaacaaaata tagcggatga aaaatttgta gattttgtta 77100
   tacacggtct agagcatcaa tgtcctgcta tacttcgacc attaattagg ttgtttattg 77160
   atatactatt atttgttata gtaatttata tttttacggt acgtctagta agtagaaatt 77220
   atcaaatgtt gttggtggtg ctagtcatca cattaactat tttttattac tttatactat 77280
   aatagtacta gactgacttc taacaaacat ctcacctgcc ataaataaat gcttgatatt 77340
   aaagtcttct atttctaaca ctattccatc tgtggaaaat aatactctga cattatcgct 77400
   aattgacaca tcggtgagtg atatgcctat aaagtaataa tcttctttgg gcacatatac 77460
   cagtgtacca ggttctaaca acctatttac tggtgctcct gtagcatact ttttctttac 77520
   cttgagaata tccatcgttt gcttggtcaa tagcgatatg tgatttttta tcaaccactc 77580
   aaaaaagtaa ttggagtgtt catatcctct acgggctatt gtctcatggc cgtgtatgaa 77640
   atttaagtaa cacgactgtg gtagatttgt tctatagagc cgattgccgc aaatagatag 77700
   aactaccaat atgtctgtac aaatgttaaa cattaattga ttaacagaaa aaacaatgtt 77760
   cgttctggga atagaaacca gatcaaaaca aaattcgtta gaatatatgc cacgtttata 77820
   catggaatat aaaataacta cagtttgaaa aataacagta tcatttaaac atttaacttg 77880
   cggggttaat ctcacaactt tactgttttt gaactgttca aaatatagca tcgatccgtg 77940
   agaaatacgt ttagccgcct ttaatagagg aaatcccacc gcctttctgg atctcaccaa 78000
   cgacgatagt tctgaccagc aactcatttc ttcatcatcc acctgtttta acatataata 78060
   ggcaggagat agatatccgt cattgcaata ttccttctcg taggcacaca atctaatatt 78120
   gataaaatct ccattctctt ctctgcattt attatcttgt ctcggtggct gattaggctg 78180
   tggtcttggt ttaggccttg gtctatcgtt gttgaatcta ttttggtcat taaatctttc 78240
   atttcttcct ggtatatttc tatcacctcg tttggttgga tttttgtcta tattatcgtt 78300
   tgtaacatcg gtacgggtat tcatttatca caaaaaaaac ttctctaaat gagtctactg 78360
   ctagaaaacc tcatcgaaga agataccata ttttttgcag gaagtatatc tgagtatgat 78420
   gatttacaaa tggttattgc cggcgcaaaa tccaaatttc caagatctat gctttctatt 78480
   tttaatatag tacctagaac gatgtcaaaa tatgagttgg agttgattca taacgaaaat 78540
   atcacaggag caatgtttac cacaatgtat aatataagaa acaatttggg tctaggagat 78600
   gataaactaa ctattgaagc cattgaaaac tatttcttgg atcctaacaa tgaagttatg 78660
   cctcttatta ttaataatac ggatatgact gccgtcattc ctaaaaaaag tggtaggaga 78720
   aagaataaga acatggttat cttccgtcaa ggatcatcac ctatcttgtg cattttcgaa 78780
   actcgtaaaa agattaatat ttataaagaa aatatggaat ccgcgtcgac tgagtataca 78840
   cctatcggag acaacaaggc tttgatatct aaatatgcgg gaattaatgt cctgaatgtg 78900
   tattctcctt ccacatccat aagattgaat gccatttacg gattcaccaa taaaaataaa 78960
   ctagagaaac ttagtactaa taaggaacta gaatcgtata gttctagccc tcttcaagaa 79020
   cccattaggt taaatgattt tctgggacta ttggaatgtg ttaaaaagaa tattcctcta 79080
   acagatattc cgacaaagga ttgattacta taaatggaga atgttcctaa tgtatacttt 79140
   aatcctgtgt ttatagagcc cacgtttaaa cattctttat taagtgttta taaacacaga 79200
   ttaatagttt tatttgaagt attcgttgta ttcattctaa tatatgtatt ttttagatct 79260
   gaattaaata tgttcttcat gcctaaacga aaaatacccg atcctattga tagattacga 79320
   cgtgctaatc tagcgtgtga agacgataaa ttaatgatct atggattacc atggatgaca 79380
   actcaaacat ctgcgttatc aataaatagt aaaccgatag tgtataaaga ttgtgcaaag 79440
   cttttgcgat caataaatgg atcacaacca gtatctctta acgatgttct tcgcagatga 79500
   tgattcattt tttaagtatt tggctagtca agatgatgaa tcttcattat ctgatatatt 79560
   gcaaatcact caatatctag actttctgtt attattattg atccaatcaa aaaataaatt 79620
   agaagctgtg ggtcattgtt atgaatctct ttcagaggaa tacagacaat tgacaaaatt 79680
   cacagacttt caagatttta aaaaactgtt taacaaggtc cctattgtta cagatggaag 79740
   ggtcaaactt aataaaggat atttgttcga ctttgtgatt agtttgatgc gattcaaaaa 79800
   agaatcctct ctagctacca ccgcaataga tcctgttaga tacatagatc ctcgtcgtga 79860
   tatcgcattt tctaacgtga tggatatatt aaagtcgaat aaagtgaaca ataattaatt 79920
   ctttattgtc atcatgaacg gcggacatat tcagttgata atcggcccca tgttttcagg 79980
   taaaagtaca gaattaatta gacgagttag acgttatcaa atagctcaat ataaatgcgt 80040
   gactataaaa tattctaacg ataatagata cggaacggga ctatggacgc atgataagaa 80100
   taattttgaa gcattggaag caactaaact atgtgatgtc ttggaatcaa ttacagattt 80160
   ctccgtgata ggtatcgatg aaggacagtt ctttccagac attgtttaat tctgtgagcg 80220
   tatggcaaac gaaggaaaaa tagttatagt agccgcactc gatgggacat ttcaacgtaa 80280
   accgtttaat aatattttga atcttattcc attatctgaa atggtggtaa aactaactgc 80340
   tgtgtgtatg aaatgcttta aggaggcttc cttttctaaa cgattgggtg aggaaaccga 80400
   gatagagata ataggaggta atgatatgta tcaatcggtg tgtagaaagt gttacgtcgg 80460
   ctcataatat tatatttttt atctaaaaaa ctaaaaataa acattgatta aattttaata 80520
   taatacttaa aaatggatgt tgtgtcgtta gataaaccgt ttatgtattt tgaggaaatt 80580
   gataatgagt tagattacga accagaaagt gcaaatgagg tcgcaaaaaa actgccgtat 80640
   caaggacagt taaaactatt actaggagaa ttattttttc ttagtaagtt acagcgacac 80700
   ggtatattag atggtgccac cgtagtgtat ataggatcgg ctcctggtac acatatacgt 80760
   tatttgagag atcatttcta taatttagga gtgatcatca aatggatgct aattgacggc 80820
   cgccatcatg atcctatttt aaatggattg cgtgatgtaa ctctagtgac tcggttcgtt 80880
   gatgaggaat atctacgatc catcaaaaaa caactgcatc cttctaagat tattttaatt 80940
   tctgatgtaa gatccaaacg aggaggaaat gaacctagta cggcggattt actaagtaat 81000
   tacgctctac aaaatgtcat gattagtatt ttaaaccccg tggcgtctag tcttaaatgg 81060
   agatgcccgt ttccagatca atggatcaag gacttttata tcccacacgg taataaaatg 81120
   ttacaacctt ttgctccttc atattcagct gaaatgagat tattaagtat ttataccggt 81180
   gagaacatga gactgactcg agttaccaaa ttagacgctg taaattatga aaaaaagatg 81240
   tactacctta ataagatcgt ccgtaacaaa gtagttgtta actttgatta tcctaatcag 81300
   gaatatgact attttcacat gtactttatg ctgaggaccg tgtactgcaa taaaacattt 81360
   cctactacta aagcaaaggt actatttcta caacaatcta tatttcgttt cttaaatatt 81420
   ccaacaacat caactgaaaa agttagtcat gaaccaatac aacgtaaaat atctagcaaa 81480
   aattctatgt ctaaaaacag aaatagcaag agatccgtac gcggtaataa atagaaacgt 81540
   actactgaga tatactaccg atatagagta taatgattta gttactttaa taaccgttag 81600
   acataaaatt gattctatga aaactgtgtt tcaggtattt aacgaatcat ccataaatta 81660
   tactccggtt gatgatgatt atggagaacc aatcattata acatcgtatc ttcaaaaagg 81720
   tcataacaag tttcctgtaa attttctata catagatgtg gtaatatctg acttatttcc 81780
   tagctttgtt agactagata ctacagaaac taatatagtt aatagtgtac tacaaacagg 81840
   cgatggtaaa aagactcttc gtcttcccaa aatgttagag acggaaatag ttgtcaagat 81900
   tctctatcgc cctaatatac cattaaaaat tgttagattt ttccgcaata acatggtaac 81960
   tggagtagag atagccgata gatctgttat ttcagtcgct gattaatcaa ttagtagaga 82020
   tgagataaga acattataat aatcaataat atatcttata tcttatatct tatatcttat 82080
   atcttgttta gaaaaatgct aatattaaaa tagctaacgc tagtaatcca atcggaagcc 82140
   atttgatatc tataataggg tatctaattt cctgatttaa atagcggaca gctatattct 82200
   cggtagctac tcgtttggaa tcacaaacat tatttacatc taatttacta tctgtaatgg 82260
   aaacgtttcc caatgaaatg gtacaatccg atacattgca ttttgttata ttttttttta 82320
   aagaggctgg taacaacgca tcgcttcgtt tacatggctc gtaccaacaa taatagggta 82380
   atcttgtatc tattcctatc cgtactatgc ttttatcagg ataaatacat ttacatcgta 82440
   tatcgtcttt gttagcatca cagaatgcat aaatttgttc gtccgtcatg ataaaaattt 82500
   aaagtgtaaa tataactatt atttttatag ttgtaataaa aagggaaatt tgattgtata 82560
   ctttcggttc tttaaaagaa actgacttga taaaaatggc tgtaatctct aaggttacgt 82620
   atagtctata tgatcaaaaa gagattaatg ctacagatat tatcattagt catgttaaaa 82680
   atgacgacga tatcggtacc gttaaagatg gtagactagg tgctatggat ggggcattat 82740
   gtaagacttg tgggaaaacg gaattggaat gtttcggtca ctggggtaaa gtaagtattt 82800
   ataaaactca tatagttaag cctgaattta tttcagaaat tattcgttta ctgaatcata 82860
   tatgtattca ctgcggatta ttgcgttcac gagaaccgta ttccgacgat attaacctaa 82920
   aagagttatc gggacacgct cttaggagat taaaggataa aatattatcc aagaaaaagt 82980
   catgttggaa cagtgaatgt atgcaaccgt atcaaaaaat tactttttca aagaaaaagg 83040
   tttgtttcgt caacaagttg gatgatatta acgttcctaa ttctctcatc tatcaaaagt 83100
   taatttctat tcatgaaaag ttttggccat tattagaaat tcatcaatat ccagctaact 83160
   tattttatac agactacttt cccatccctc cgttgattat tagaccggct attagttttt 83220
   ggatagatag tatacccaaa gagaccaatg aattaactta cttattaggt atgatcgtta 83280
   agaattgtaa cttgaatgct gatgaacagg ttatccagaa ggcggtaata gaatacgatg 83340
   atattaaaat tatttctaat aacactacca gtatcaattt atcatatatt acatccggca 83400
   aaaataatat gattagaagt tatatcgtcg cccgacgaaa agatcagacc gctagatctg 83460
   taattggtcc cagtacatct atcaccgtta atgaggtagg aatgcccgca tatattagaa 83520
   atacacttac agaaaagata tttgttaatg cctttacagt ggataaagtt aaacaactat 83580
   tagcgtcaaa ccaagttaaa ttttacttta ataaacgatt aaaccaatta acaagaatac 83640
   gccaaggaaa gtttattaaa aataaaatac atttattgcc tggtgattgg gtagaagtag 83700
   ctgttcaaga atatacaagt attatttttg gaagacagcc gtctctacat agatacaacg 83760
   tcatcgcttc atctatcaga gctaccgaag gagatactat caaaatatct cccggaattg 83820
   ccaactctca aaatgctgat ttcgacgggg atgaggaatg gatgatatta gaacaaaatc 83880
   ctaaagctgt aattgaacaa agtattctta tgtatccgac gacgttactc aaacacgata 83940
   ttcatggagc ccccgtttat ggatctattc aagatgaaat cgtagcagcg tattcattgt 84000
   ttaggataca agatctttgt ttagatgaag tattgaacat cttggggaaa tatggaagag 84060
   agttcgatcc taaaggtaaa tgtaaattca gcggtaaaga tatctatact tacttgatag 84120
   gtgaaaagat taattatccg ggtctcttaa aggatggtga aattattgca aacgacgtag 84180
   atagtaattt tgttgtggct atgaggcatc tgtcattggc tggactctta tccgatcata 84240
   agtcgaacgt ggaaggtatc aactttatta tcaagtcatc ttatgttttt aagagatatc 84300
   tatctattta cggttttggg gtgacattca aagatctgag accaaattcg acgttcacta 84360
   ataaattgga ggccatcaac gtagaaaaaa tagaacttat caaagaagca tacgccaaat 84420
   atctcaacga tgtaagagac gggaaaatag ttccattatc taaagcttta gaggcggact 84480
   atgtggaatc catgttatcc aacttgacaa atcttaatat ccgagagata gaagaacata 84540
   tgagacaaac gctgatagat gatccagata ataacctcct gaaaatggcc aaagcgggtt 84600
   ataaagtaaa tcccacagaa ctaatgtata ttctaggtac gtatggacaa caaaggattg 84660
   atggtgaacc agcagagact cgagtattgg gtagagtttt accttactat cttccagact 84720
   ctaaggatcc agaaggaaga ggttacattc ttaattcttt aacaaaagga ttaacgggtt 84780
   ctcaatatta cttttcgatg ctggttgcca gatctcaatc tactgatatc gtctgtgaaa 84840
   catcacgtac cggaacactg gctagaaaaa tcattaaaaa gatggaggat atggtggtcg 84900
   acggatacgg acaagtagtt ataggtaata cgctcatcaa gtacgccgcc aattatacca 84960
   aaattctagg ctcagtatgt aaacctgtag atcttatcta tccagatgag tccatgactt 85020
   ggtatttgga aattagtgct ctgtggaata aaataaaaca gggattcgtt tactctcaga 85080
   aacagaaact tgcaaagaag acattggcgc cgtttaattt cctagtattc gtcaaaccca 85140
   ccactgagga taatgctatt aaggttaagg atctgtacga tatgattcat aacgtcattg 85200
   atgatgtgag agagaaatac ttctttacgg tatctaatat agattttatg gagtatatat 85260
   tcttgacgca tcttaatcct tctagaatta gaattacaaa agaaacggct atcactatct 85320
   ttgaaaagtt ctatgaaaaa ctcaattata ctctaggtgg tggaactcct attggaatta 85380
   tttctgcaca ggtattgtct gagaagttta cacaacaagc cctgtccagt tttcacacta 85440
   ctgaaaaaag tggtgccgtc aaacaaaaac ttggtttcaa cgagtttaat aacttgacta 85500
   atttgagtaa gaataagacc gaaattatca ctctggtatc cgatgatatc tctaaacttc 85560
   aatctgttaa gattaatttc gaatttgtat gtttgggaga attaaatcca aacatcactc 85620
   ttcgaaaaga aacagataaa tatgtagtag atataatagt caatagatta tacatcaaga 85680
   gagcagaaat aaccgaatta gtcgtcgaat atatgattga acgatttatc tcctttagcg 85740
   tcattgtaaa ggaatggggt atggagacat tcattgagga cgaggataat attagattta 85800
   ctgtctacct aaatttcgtt gaaccggaag aattgaatct tagtaagttt atgatggttc 85860
   ttccgggggc agccaacaag ggaaagatta gtaaattcaa gattcctatc tctgattata 85920
   cgggatatga cgacttcaat caaacaaaaa agctcaataa gatgactgta gaactcatga 85980
   atctaaaaga attgggttct ttcgatttgg aaaacgtcaa cgtgtatcct ggagtatgga 86040
   atacatacga tatcttcggt atcgaggccg ctcgtgaata cttgtgcgaa gccatgttaa 86100
   acacctatgg agaagggttc gattatctgt atcagccttg tgatcttctc gctagtttac 86160
   tatgtgctag ttacgaacca gaatcagtga ataaattcaa gttcggcgca gctagtactc 86220
   ttaagagagc tacgttcgga gacaataaag cattgttaaa cgcggctctt cataaaaagt 86280
   cagaacctat taacgataat agtagctgcc acttttttag caaggtccct aatataggaa 86340
   ctggatatta caaatacttt atcgacttgg gtcttctcat gagaatggaa aggaaactat 86400
   ctgataagat atcttctcaa aagatcaagg aaatggaaga aacagaagac ttttaattct 86460
   tatcaataac atatttttct atgatctgtc ttttaaacga tggattttcc acaaatgcgc 86520
   ctctcaagtc cctcatagaa tgatacacgt ataaaaaata tagcataggc aatgactcct 86580
   tatttttaga cattagatat gccaaaatca tagccccgct tctatttact cccgcagcac 86640
   aatgaaccaa cacgggctcg tttcgttgat cacatttaga taaaaaggcg gttacgtcgt 86700
   caaaatattt actaatatcg gtagttgtat catctaccaa cggtatatga ataatattaa 86760
   tattagagtt aggtaatgta tatttatcca tcgtcaaatt taaaacatat ttgaacttaa 86820
   cttcagatga tggtgcatcc atagcatttt tataatttcc caaatacaca ttattggtta 86880
   cccttgtcat tatagtggga gatttggctt tgtgcatatc tccagttgaa cgtagtagta 86940
   agtatttata caaacttttc ttatccattt ataacgtaca aatggataaa actactttat 87000
   cggtaaacgc gtgtaattta gaatacgtta gagaaaaggc tatagtaggc gtacaagcag 87060
   ccaaaacatc aacacttata ttctttgtta ttatattggc aattagtgcg ctattactct 87120
   ggtttcagac gtctgataat ccagtcttta atgaattaac gagatatatg cgaattaaaa 87180
   atacggttaa cgattggaaa tcattaacgg atagcaaaac aaaattagaa agtgatagag 87240
   gtagacttct agccgctggt aaggatgata tatttgaatt caaatgtgtg gatttcggcg 87300
   cctattttat agctatgcga ttggataaga aaacatatct gccgcaagct attaggcgag 87360
   gtactggaga cgcgtggatg gttaaaaagg cggcaaaggt cgatccatct gctcaacaat 87420
   tttgtcagta tttgataaaa cacaagtcta ataatgttat tacttgtggt aatgagatgt 87480
   taaatgaatt aggttatagc ggttatttta tgtcaccgca ttggtgttcc gattttagta 87540
   atatggaata gtgttagata aatgcggtaa cgaatgttcc tgtaaggaac cataacagct 87600
   tagatttaac gttaaagatg agcataaaca taataaacaa aattacaatc aaacttataa 87660
   cattaatatc aaacaatcca aaaaatgaaa tcagtggagt agtaaacgcg tacataactc 87720
   ctggataacg tttagcagct gccgttccta ttctagacca aaaattcggt ttcatgtttt 87780
   cgaaacggta ttctgcaaca agtcgaggat cgtgttctac atatttggcg gcattatcca 87840
   gtatctgcct attgatcttc atttcgtttt cgattctggc tatttcaaaa taaaatcccg 87900
   atgatagacc tccagacttt ataatttcat ctacgatgtt cagcgccgta gtaactctaa 87960
   taatataggc tgataagcta acatcatacc ctcctgtata tgtgaatatg gcatgatttt 88020
   tgtccattac aagctcggtt ttaactttat tgcctgtaat aatttctctc atctgtagga 88080
   tatctatttt tttgtcatgc attgccttca agacgggacg aagaaacgta atatcctcaa 88140
   taacgttatc gttttctaca ataactacat attctacctt tttattttct aactcggtaa 88200
   aaaaattaga atcccatagg gctaaatgtc tagcgatatt tcttttcgtt tcctctgtac 88260
   acatagtgtt acaaaaccct gaaaagaagt gagtatactt gtcatcattt ctaatgtttc 88320
   ctccagtcca ctgtataaac gcataatcct tgtaatgatc tggatcatcc ttgactacca 88380
   caacatttct tttttctggc ataacttcgt tgtcctttac atcatcgaac ttctgatcat 88440
   taatatgctc atgaacatta ggaaatgttt ctgatggagg tctatcaata actggcacaa 88500
   caataacagg agttttcacc gccgccattt agttattgaa attaatcata tacaactctt 88560
   taatacgagt tatattttcg tctatccatt gtttcacatt gacatatttc gacaaaaaga 88620
   tataaaatgc gtattccaat gcttctctgt ttaatgaatt actaaaatat acaaacacgt 88680
   cactgtctgg caataaatga tatcttagaa tattgtaaca atttattttg tattgcacat 88740
   gttcgtgatc tatgagttct tcttcgaatg gcataggatc tccgaatctg aaaacgtata 88800
   aataggagtt agaataataa tatttgagag tattggtaat atataaactc tttagcggta 88860
   taattagttt ttttctctca atttctattt ttagatgtga tggaaaaatg actaattttg 88920
   tagcattagt atcatgaact ctaatcaaaa tcttaatatc ttcgtcacac gttagctctt 88980
   tgaagttttt aagagatgca tcagttggtt ctacagatgg agtaggtgca acaatttttt 89040
   gttctacaca tgtatgtact ggagccattg ttttaactat aatggtgctt gtatcgaaaa 89100
   actttaatgc agatagcgga agctcttcgc cgcgactttc tacgtcgtaa ttgggttcta 89160
   acgccgatct ctgaatggat actagttttc taagttctaa tgtgattctc tgaaaatgta 89220
   aatccaattc ctccggcatt atagatgtgt atacatcggt aaataaaact atagtatcca 89280
   acgatccctt ctcgcaaatt ctagtcttaa ccaaaaaatc gtatataacc acggagatgg 89340
   cgtatttaag agtggattct tctaccgttt tgttcttgga tgtcatatag gaaactataa 89400
   agtccgcact actgttaaga atgattacta acgcaactat atagtttaaa ttaagcattt 89460
   tggaaacata aaataactct gtagacgata cttgactttc gaataagttt gcagacaaac 89520
   gaagaaagaa cagacctctc ttaatttcag aagaaaactt tttttcgtat tcctgacgtc 89580
   tagagtttat atcaataaga aagttaagaa ttagtcggtt aatgttgtat ttcattaccc 89640
   aagtttgaga tttcataata ttatcaaaag acatgataat attaaagata aagcgctgac 89700
   tatgaacgaa atagctatat ggttcgctca agaatatagt cttgttaaac gtggaaacga 89760
   taactgtatt tttaatcacg tcagcggcat ctaaattaaa tataggtata tttattccac 89820
   acactctaca atatgccaca ccatcttcat aataaataaa ttcgttagca aaattattaa 89880
   ttctagtgaa atagttagcg tcaactttca tagcttcctt caatctaatt tgatgctcac 89940
   acggtgcgaa ttccactcta acatcccttt tccatgcctc aggttcatcg atctctataa 90000
   tatctagttt tttgcgtttc acaaacacag gctcgtctct cgcgatgaga tctgtatagt 90060
   aactatgtaa atgataacta gatagaaaga tgtagctata tagatgacga tcctttaaga 90120
   gaggtataat aactttaccc caatcagata gactgttgtt atggtcttcg gaaaaagaat 90180
   ttttataaat ttttccagta ttttccaaat atacgtactt aacatctaaa aaatccttaa 90240
   tgataatagg aatggataat ccgtctattt tataaagaaa tacatatcgc acattatact 90300
   tttttttgga aatgggaata ccgatgtgtc tacataaata tgcaaagtct aaatattttt 90360
   tagagaatct tagttggtcc aaattctttt ccaagtacgg taatagattt ttcatattga 90420
   acggtatctt cttaatctct ggttctagtt ccgcattaaa tgatgaaact aagtcactat 90480
   ttttataact aacgattaca tcacctctaa catcatcatt taccagaata ctgatcttct 90540
   tttgtcgtaa atacatgtct aatgtgttaa aaaaaagatc atacaagtta tacgtcattt 90600
   catctgtggt attcttgtca ttgaaggata aactcgtact aatctcttct ttaacagcct 90660
   gttcaaattt atatcctata tacgaaaaaa tagcaaccag tgtttgatca tccgcgtcaa 90720
   tattctgttc tatcgtagtg tataacaatc gtatatcttc ttctgtgata gtcgatacgt 90780
   tataaaggtt gataacgaaa atatttttat ttcgtgaaat aaagtcatcg taggattttg 90840
   gacttatatt cgcgtctagt agatatgctt ttatttttgg aatgatctca attagaatag 90900
   tctctttaga gtccatttaa agttacaaac aactaggaaa ttggtttatg atgtataatt 90960
   tttttagttt ttatagattc tttattctat acttaaaaaa tgaaaataaa tacaaaggtt 91020
   cttgagggtt gtgttaaatt gaaagcgaga aataatcata aattatttca ttatcgcgat 91080
   atccgttaag tttgtatcgt aatggcgtgg tcaattacga ataaagcgga tactagtagc 91140
   ttcacaaaga tggctgaaat cagagctcat ctaaaaaata gcgctgaaaa taaagataaa 91200
   aacgaggata ttttcccgga agatgtaata attccatcta ctaagcccaa aaccaaacga 91260
   gccactactc ctcgtaaacc agcggctact aaaagatcaa ccaaaaagga ggaagtggaa 91320
   gaagaagtag ttatagagga atatcatcaa acaactgaaa aaaattctcc atctcctgga 91380
   gtcagcgaca ttgtagaaag cgtggccgct gtagagctcg atgatagcga cggggatgat 91440
   gaacctatgg tacaagttga agctggtaaa gtaaatcata gtgctagaag cgatctctct 91500
   gacctaaagg tggctaccga caatatcgtt aaagatctta agaaaattat tactagaatc 91560
   tctgcagtat cgacggttct agaggatgtt caagcagctg gtatctctag acaatttact 91620
   tctatgacta aagctattac aacactatct gatctagtca ccgagggaaa atctaaagtt 91680
   gttcgtaaaa aagttaaaac ttgtaagaag taaatgcgtg cactttttta taaagatggt 91740
   aaactcttta ccgataataa ttttttaaat cctgtatcag acgataatcc agcgtatgag 91800
   gttttgcaac atgttaaaat tcctactcat ttaacagatg tagtagtata tgaacaaacg 91860
   tgggaggagg cgttaactag attaattttt gtgggaagtg attcaaaagg acgtagacaa 91920
   tacttttacg gaaaaatgca tgtacagaat cgcaacgcta aaagagatcg tatttttgtt 91980
   agagtatata acgttatgaa acgaattaat tgttttataa acaaaaatat aaagaaatcg 92040
   tccacagatt ccaattatca gttggcggtt tttatgttaa tggaaactat gttttttatt 92100
   agatttggta aaatgaaata tcttaaggag aatgaaacag tagggttatt aacactaaaa 92160
   aataaacaca tagaaataag tcccgatgaa atagttatca agtttgtagg aaaggacaaa 92220
   gtttcacatg aatttgttgt tcataagtct aatagactat ataaaccgct attgaaactg 92280
   acggatgatt ctagtcccga agaatttctg ttcaacaaac taagtgaacg aaaggtatac 92340
   gaatgtatca aacagtttgg tattagaatc aaggatctcc gaacgtatgg agtcaattat 92400
   acgtttttat ataatttttg gacaaatgta aagtccatat ctcctcttcc gtcaccaaaa 92460
   aagttaatag cattaactat caaacaaact gctgaagtgg taggtcatac tccatcaatt 92520
   tcaaaaagag cttatatggc aacgactatt ttagaaatgg taaaggataa aaatttttta 92580
   gatgtagtat ctaaaactac gttcgatgaa ttcctatcta tagtcgtaga tcacgttaaa 92640
   tcatctacgg atggatgata tagatcttta cacaaataat tacaagaccg ataaatggaa 92700
   atggataagc gtatgaaatc tctcgcaatg acagctttct tcggagagct aagcacatta 92760
   gatattatgg cattgataat gtctatattt aaacgccatc caaacaatac cattttttca 92820
   gtggataagg atggtcagtt tatgattgat ttcgaatacg ataattataa ggcttctcaa 92880
   tatttggatc tgaccctcac tccgatatct ggagatgaat gcaagactca cgcatcgagt 92940
   atagccgaac aattggcgtg tgcggatatt attaaagagg atattagcga atatatcaaa 93000
   actactcccc gtcttaaacg atttataaaa aaataccgca atagatcaga tactcgtatc 93060
   agtcgagata cagaaaagct taaaatagct ctagctaaag gcatagatta cgaatatata 93120
   aaagacgctt gttaataagt aaatgaaaaa aaactagtcg tttataataa aacacgatat 93180
   ggatgccaac gtagtatcat tttctactat tgcgacgtat atagacgctt tagcgaagaa 93240
   tgcttcggaa ttagaacaga ggtctaccgc atacgaaata aataatgaat tggaactagt 93300
   atttattaag ccgccattga ttactttgac aaatgtagtg aatatctcta cgattcagga 93360
   atcgtttatt cgatttaccg ttactaataa ggaaggtgtt aaaattagaa ctaagattcc 93420
   attatctaag gtacatggtc tagatgtaaa aaatgtacag ttagtagatg ctatagataa 93480
   catagtttgg gaaaagaaat cattagtgac ggaaaatcgt cttcacaaag aatgcttgtt 93540
   gagactatcg acagaggaac gtcatatatt tttggattac aagaaatatg gatcctctat 93600
   ccgactagaa ttagtcaatc ttattcaagc aaaaacaaaa aactttacga tagactttaa 93660
   gctaaaatat tttctaggat ccggtgccca gtctaaaagt tctttgttgc acgctattaa 93720
   tcatccaaag tcaaggccta atacatctct ggaaatagaa ttcacaccta gagacaatga 93780
   aacagttcca tatgatgaac taataaagga attgacgact ctctcgcgtc atatatttat 93840
   ggcttctcca gagaatgtaa ttctttctcc gcctattaac gcgcctataa aaacctttat 93900
   gttgcctaaa caagatatag taggtttgga tctggaaaat ctatatgccg taactaagac 93960
   tgacggcatt cctataacta tcagagttac atcaaacggg ttgtattgtt attttacaca 94020
   tcttggttat attattagat atcctgttaa gagaataata gattccgaag tagtagtctt 94080
   tggtgaggca gttaaggata agaactggac cgtatatctc attaagctaa tagagcctgt 94140
   gaatgcaatc aatgatagac tagaagaaag taagtatgtt gaatctaaac tagtggatat 94200
   ttgtgatcgg atagtattca agtcaaagaa atacgaaggt ccgtttacta caactagtga 94260
   agtcgtcgat atgttatcta catatttacc aaagcaacca gaaggtgtta ttctgttcta 94320
   ttcaaaggga cctaaatcta acattgattt taaaattaaa aaggaaaata ctatagacca 94380
   aactgcaaat gtagtattta ggtacatgtc cagtgaacca attatctttg gagaatcgtc 94440
   tatctttgta gagtataaga aatttagcaa cgataaaggc tttcctaaag aatatggttc 94500
   tggtaagatt gtgttatata acggcgttaa ttatctaaat aatatctatt gtttggaata 94560
   tattaataca cataatgaag tgggtattaa gtccgtggtt gtacctatta agtttatagc 94620
   agaattctta gttaatggag aaatacttaa acctagaatt gataaaacca tgaaatatat 94680
   taactcagaa gattattatg gaaatcaaca taatatcata gtcgaacatt taagagatca 94740
   aagcatcaaa ataggagata tctttaacga ggataaacta tcggatgtgg gacatcaata 94800
   cgccaataat gataaattta gattaaatcc agaagttagt tattttacga ataaacgaac 94860
   tagaggaccg ttgggaattt tatcaaacta cgtcaagact cttcttattt ctatgtattg 94920
   ttccaaaaca tttttagacg attccaacaa acgaaaggta ttggcgattg attttggaaa 94980
   cggtgctgac ctggaaaaat acttttatgg agagattgcg ttattggtag cgacggatcc 95040
   ggatgctgat gctatagcta gaggaaatga aagatacaac aaattaaact ctggaattaa 95100
   aaccaagtac tacaaatttg actacattca ggaaactatt cgatccgata catttgtctc 95160
   tagtgtcaga gaagtattct attttggaaa gtttaatatc atcgactggc agtttgctat 95220
   ccattattct tttcatccga gacattatgc taccgtcatg aataacttat ccgaactaac 95280
   tgcttctgga ggcaaggtat taatcactac catggacgga gacaaattat caaaattaac 95340
   agataaaaag acttttataa ttcataagaa tttacctagt agcgaaaact atatgtctgt 95400
   agaaaaaata gctgatgata gaatagtggt atataatcca tcaacaatgt ctactccaat 95460
   gactgaatac attatcaaaa agaacgatat agtcagagtg tttaacgaat acggatttgt 95520
   tcttgtagat aacgttgatt tcgctacaat tatagaacga agtaaaaagt ttattaatgg 95580
   cgcatctaca atggaagata gaccgtctac aaaaaacttt ttcgaactaa atagaggagc 95640
   cattaaatgt gaaggtttag atgtcgaaga cttacttagt tactatgttg tttatgtctt 95700
   ttctaagcgg taaataataa tatggtatgg gttctgatct cccagttcta aatgcattaa 95760
   ataattccaa tagagcgatt tttgttccta taggaccttc caactgtgga tactctgtat 95820
   tgttaataga tatattaata cttttgtcgg gtaacagagg ttctacgtct tctaaaaata 95880
   aaagttttat aacatctggc ctgttcataa ataaaaactt ggcgattcta tatatactct 95940
   tattatcaaa tctagccatt gtcttataga tgtgagctac tgtaggtgta ccatttgatt 96000
   ttctttctaa tactatatat ttctctcgaa gaagttcttg cacatcatct gggaataaaa 96060
   tactactgtt gagtaaatca gttatttttt ttatatcgat attgatggac atttttatag 96120
   ttaaggataa taagtatccc aaagtcgata acgacgataa cgaagtattt atacttttag 96180
   gaaatcacaa tgactttatc agattaaaat taacaaaatt aaaggagcat gtattttttt 96240
   ctgaatatat tgtgactcca gatacatatg gatctttatg cgtcgaatta aatgggtcta 96300
   gttttcagca cggcggtaga tatatagagg tggaggaatt tatagatgct ggaagacaag 96360
   ttagatggtg ttctacatcc aatcatatat ctaaagatat acccgaagat atgcacactg 96420
   ataaatttgt catttatgat atttatacgt ttgattcgtt caagaataaa cgattggtat 96480
   tcgtacaggt acctccgtcg ttaggagatg atagtcattt gactaatccg ttattgtcac 96540
   cgtattatcg taattcagta gccagacaaa tggtcaatga tatgattttt aatcaagatt 96600
   catttttaaa atatttatta gaacatctga ttagaagcca ctatagagtt tctaaacata 96660
   taacaatagt tagatacaag gataccgaag aattaaatct aacgagaata tgttataata 96720
   gagataagtt taaggcgttt gtattcgctt ggtttaacgg cgtttcggaa aatgaaaagg 96780
   tactagatac gtataaaaag gtatctaatt tgatataatg aattcagtga ctgtatcaca 96840
   cgcgccatat actattactt atcacgatga ttgggaacca gtaatgagtc aattggtaga 96900
   gttttataac gaagtagcca gttggctgct acgagacgag acgtcgccta ttcctgataa 96960
   gttctttata cagttgaaac aaccgcttag aaataaacga gtatgtgtgt gtggtataga 97020
   tccgtatccg aaagatggaa ctggtgtacc gttcgaatca ccaaatttta caaaaaaatc 97080
   aattaaggag atagcttcat ctatatctag attaaccgga gtaattgatt ataaaggtta 97140
   taaccttaat ataatagacg gggttatacc ctggaattat tacttaagtt gtaaattagg 97200
   agaaacaaaa agtcacgcga tttactggga taagatttcc aagttactgc tgcagcatat 97260
   aactaaacac gttagtgttc tttattgttt gggtaaaaca gatttctcga atatacgggc 97320
   caagttagaa tccccggtaa ctaccatagt cggatatcat ccagcggcta gagaccgcca 97380
   attcgagaaa gatagatcat ttgaaattat caacgtttta ctggaattag acaacaaggc 97440
   acctataaat tgggctcaag ggtttattta ttaatgcttt agtgaaattt taacttgtgt 97500
   tctaaatgga tgcggctatt agaggtaatg atgttatctt tgttcttaag actataggtg 97560
   tcccgtcagc gtgcagacaa aatgaagatc caagatttgt agaagcattt aaatgcgacg 97620
   agttagaaag atatattgag aataatccag aatgtacact attcgaaagt cttagggatg 97680
   aggaagcata ctctatagtc agaattttca tggatgtaga tttagacgcg tgtctagacg 97740
   aaatagatta tttaacggcc attcaagatt ttattatcga ggtgtcaaac tgtgtagcta 97800
   gattcgcgtt tacagaatgc ggcgccattc atgaaaatgt aataaaatcc atgagatcta 97860
   atttttcatt gactaagtct acaaatagag ataaaacaag ttttcatatt atctttttag 97920
   atacgtatac cactatggat acattgatag ctatgaaacg aacactatta gaattaagta 97980
   gatcatctga aaatccacta accagatcga tagacactgc cgtatatagg agaaaaacaa 98040
   ctcttcgggt tgtaggtact aggaaaaatc caaattgcga cactattcat gtaatgcaac 98100
   caccgcatga taatatagaa gattacctat tcacttacgt ggatatgaac aacaatagtt 98160
   attacttttc tctacaacaa cgattggagg atttagttcc tgataagtta tgggaaccag 98220
   ggtttatttc attcgaagac gctataaaaa gagtttcaaa aatattcatt aattctataa 98280
   taaactttaa tgatctcgat gaaaataatt ttacaacggt accactggtc atagattacg 98340
   taacaccttg tgcattatgt aaaaaacgat cgcataaaca tccgcatcaa ctatcgttgg 98400
   aaaatggtgc tattagaatt tacaaaactg gtaatccaca tagttgtaaa gttaaaattg 98460
   ttccgttgga tggtaataaa ctgtttaata ttgcacaaag aattttagac actaactctg 98520
   ttttattaac cgaacgagga gaccatatag tttggattaa taattcatgg aaatttaaca 98580
   gcgaagaacc cttgataaca aaactaattt tgtcaataag acatcaacta cctaaggaat 98640
   attcaagcga attactctgt ccaagaaaac gaaagactgt agaagctaac atacgagaca 98700
   tgttagtaga ttcagtggag accgatacct atccggataa acttccgttt aaaaatggtg 98760
   tattggacct ggtagacgga atgttttact ctggagatga tgctaaaaaa tatacgtgta 98820
   ctgtatcaac cggatttaaa tttgacgata caaagttcgt cgaagacagt ccagaaatgg 98880
   aagagttaat gaatatcatt aacgatatcc aaccattaac ggatgaaaat aagaaaaata 98940
   gagagctata tgaaaaaaca ttatctagtt gtttatgcgg tgctaccaaa ggatgtttaa 99000
   cattcttttt tggagaaact gcaactggaa agtcgacaac caaacgtttg ttaaagtctg 99060
   ctatcggtga cctgtttgtt gagacgggtc aaacaatttt aacagatgta ttggataaag 99120
   gacctaatcc atttatcgct aacatgcatt tgaaaagatc tgtattctgt agcgaactac 99180
   ctgattttgc ctgtagtgga tcaaagaaaa ttagatctga caatattaaa aagttgacag 99240
   aaccttgtgt cattggaaga ccgtgtttct ccaataaaat taataataga aaccatgcga 99300
   caatcattat cgatactaat tacaaacctg tttttgatag gatagataac gcattaatga 99360
   gaagaattgc cgtcgtgcga ttcagaacac acttttctca accttctggt agagaggctg 99420
   ctgaaaataa tgacgcgtac gataaagtca aactattaga cgaggggtta gatggtaaaa 99480
   tacaaaataa tatatataga tttgcatttc tatacttgtt ggtgaaatgg tacaaaaaat 99540
   atcatgttcc tattatgaaa ctatatccta cacccgaaga gattcctgac tttgcattct 99600
   atctcaaaat aggtactctg ttggtatcta gctctgtaaa gcatattcca ttaatgacgg 99660
   acctctccaa aaagggatat atattgtacg ataatgtggt cactcttccg ttgactactt 99720
   tccaacagaa aatatccaag tattttaatt ctagactatt tggacacgat atagagagct 99780
   tcatcaatag acataagaaa tttgccaatg ttagtgatga atatctgcaa tatatattca 99840
   tagaggatat ttcatctccg taaatatatg ctcatatatt tatagaagat atcacatatc 99900
   taaatgaata ccggaatcat agatttattt gataatcatg ttgatagtat accaactata 99960
   ttacctcatc agttagctac tctagattat ctagttagaa ctatcataga tgagaacaga 100020
   agcgtgttat tgttccatat tatgggatca ggtaaaacaa taatcgcttt gttgttcgcc 100080
   ttggtagctt ccagatttaa aaaggtttac attctagtgc cgaacatcaa catcttaaaa 100140
   attttcaatt ataatatggg tgtagctatg aacttgttta atgacgaatt catagctgag 100200
   aatatcttta ttcattccac aacaagtttt tattctctta attataacga taacgtcatt 100260
   aattataacg gattatctcg ctacaataac tctattttta tcgttgatga ggcacataat 100320
   atctttggga ataatactgg agaacttatg accgtgataa aaaataaaaa caagattcct 100380
   tttttactat tgtctggatc tcccattact aacacaccta atactctggg tcatattata 100440
   gatttaatgt ccgaagagac gatagatttt ggtgaaatta ttagtcgtgg taagaaagta 100500
   attcagacac ttcttaacga acgcggtgtg aatgtactta aggatttgct taaaggaaga 100560
   atatcatatt acgaaatgcc tgataaagat ctaccaacga taagatatca cggacgtaag 100620
   tttctagata ctagagtagt atattgtcac atgtctaaac ttcaagagag agattatatg 100680
   attactagac gacagctatg ttatcatgaa atgtttgata aaaatatgta taacgtgtca 100740
   atggcagtat tgggacaact taatctgatg aataatttag atactttatt tcaggaacag 100800
   gataaggaat tgtacccaaa tctgaaaata aataatggcg tgttatacgg agaagaattg 100860
   gtaacgttaa acattagttc caaatttaaa tactttatta atcggataca gacactcaac 100920
   ggaaaacatt ttatatactt ttctaattct acatatggcg gattggtaat taaatatatc 100980
   atgctcagta atggatattc tgaatataat ggttctcagg gaactaatcc acatatgata 101040
   aacggcaaac caaaaacatt tgctatcgtt actagtaaaa tgaaatcgtc tttagaggat 101100
   ctattagatg tgtataattc tcctgaaaac gatgatggta gtcaattgat gtttttgttt 101160
   tcatcaaaca ttatgtccga atcctatact ctaaaagagg taaggcatat ttggtttatg 101220
   actatcccag atactttttc tcaatacaac caaattcttg gacgatctat tagaaaattc 101280
   tcttacgccg atatttctga accagttaat gtatatcttt tagccgccgt atattccgat 101340
   ttcaatgacg aagtgacgtc attaaacgat tacacacagg atgaattaat taatgtttta 101400
   ccatttgaca tcaaaaagct gttgtatcta aaatttaaga cgaaagaaac gaatagaata 101460
   tactctattc ttcaagagat gtctgaaacg tattctcttc caccacatcc atcaattgta 101520
   aaagttttat tgggagaatt ggtcagacaa tttttttata ataattctcg tattaagtat 101580
   aacgatacca agttacttaa aatggttaca tcagttataa aaaataaaga agacgctagg 101640
   aattacatag atgatattgt aaacggtcac ttctttgtat cgaataaagt atttgataaa 101700
   tctcttttat acaaatacga aaacgatatt attacagtac cgtttagact ttcctacgaa 101760
   ccatttgttt ggggagttaa ctttcgtaaa gaatataacg tggtatcttc tccataaaac 101820
   tgatgagata tataaagaaa taaatgtcga gctttgttac caatggatac ctttccgtta 101880
   cattggaacc acacgagctg acgttagaca taaaaactaa tattaggaat gccgtatata 101940
   agacgtatct ccatagagaa attagtggta aaatggccaa gaaaatagaa attcgtgaag 102000
   acgtggaatt acctctcggc gaaatagtta ataattctgt agttataaac gttccgtgtg 102060
   taataaccta cgcgtattat cacgttgggg atatagtcag aggaacatta aacatcgaag 102120
   atgaatcaaa tgtaactatt caatgtggag atttaatctg taaactaagt agagattcgg 102180
   gtactgtatc atttagcgat tcaaagtact gcttttttcg aaatggtaat gcgtatgaca 102240
   atggcagcga agtcactgcc gttctaatgg aggctcaaca aggtatcgaa tctagttttg 102300
   tttttctcgc gaatatcgtc gactcataaa aaagagaata gcggtaagta taaacacgaa 102360
   tactatggca ataattgcga atgttttatt ctcttcgata tatttttgat aatatgaaaa 102420
   acatgtctct ctcaaatcgg acaaccatct cataaaatag ttctcgcgcg ctggagaggt 102480
   agttgccgct cgtataatct ctccagaata atatacttgc gtgtcgtcgt tcaatttata 102540
   cggatttcta tagttctctg ttatataatg cggtttgccc tcatgattag acgacgacaa 102600
   tagtgttcta aatttagata gttgatcaga atgaatgttt attggcgttg gaaaaattat 102660
   ccatacagcg tctgcagagt ggttgatagt tgttcctaga tatgtaaaat aatccaactt 102720
   actaggcagc aaattgtcta gataaaatac tgaatcaaac ggtgcagacg tattggcgga 102780
   tctaatggaa tccaattgat taactatctt ttgaaaatat acatttttat gatccaatac 102840
   ttgtaagaat atagaaataa tgataagtcc atcatcgtgt ttttttgcct cttcataaga 102900
   actatatttt ttcttattcc aatgaacaag attaatctct ccagagtatt tgtacacatc 102960
   tatcaagtga ttggatccat aatcgtcttc ctttccccaa tatatatgta gtgatgataa 103020
   cacatattca ttggggagaa accctccact tatatatcct cctttaaaat taatccttac 103080
   tagttttcca gtgttctgga tagtggttgg tttcgactca ttataatgta tgtctaacgg 103140
   cttcaatcgc gcgttagaaa ttgctttttt agtttctata ttaataggag atagttgttg 103200
   cggcatagta aaaatgaaat gataactgtt taaaaatagc tcttagtatg ggaattacaa 103260
   tggatgagga agtgatattt gaaactccta gagaattaat atctattaaa cgaataaaag 103320
   atattccaag atcaaaagac acgcatgtgt ttgctgcgtg tataacaagt gacggatatc 103380
   cgttaatagg agctagaaga acttcattcg cgttccaggc gatattatct caacaaaatt 103440
   cagattctat ctttagagta tccactaaac tattacggtt tatgtactac aatgaactaa 103500
   gagaaatctt tagacggttg agaaaaggtt ctatcaacaa tatcgatcct cactttgaag 103560
   agttaatatt attgggtggt aaactagata aaaaggaatc tattaaagat tgtttaagaa 103620
   gagaattaaa agaggaaagt gatgaacgta taacagtaaa agaatttgga aatgtaattc 103680
   taaaacttac aacacgggat aaattattta ataaagtata tataagttat tgcatggcgt 103740
   gttttattaa tcaatcgttg gaggatttat cgcatactag tatttacaat gtagaaatta 103800
   gaaagattaa atcattaaat gattgtatta acgacgataa atacgaatat ctgtcttata 103860
   tttataatat gctagttaat agtaaatgaa cttttacaga tctagtataa ttagtcagat 103920
   tattaagtat aatagacgac tagctaagtc tattatttgc gaggatgact ctcaaattat 103980
   tacactcacg gcattcgtta accaatgcct atggtgtcat aaacgagtat ccgtgtccgc 104040
   tattttatta actactgata acaaaatatt agtatgtaac agacgagata gttttctcta 104100
   ttctgaaata attagaacta gaaacatgtt tagaaagaaa cgattatttc tgaattattc 104160
   caattatttg aacaaacagg aaagaagtat actatcgtca tttttttctc tagatccagc 104220
   tactactgat aatgatagaa tagacgctat ttatccgggt ggcataccca aaaggggtga 104280
   gaatgttcca gagtgtttat ccagggaaat taaagaagaa gttaatatag acaattcttt 104340
   tgtattcata gacactcggt tttttattca tggcatcata gaagatacca ttattaataa 104400
   attttttgag gtaatcttct ttgtcggaag aatatctcta acgagtgatc aaatcattga 104460
   tacatttaaa agtaatcatg aaatcaagga tctaatattt ttagatccga attcaggtaa 104520
   tggactccaa tacgaaattg caaaatatgc tctagatact gcaaaactta aatgttacgg 104580
   ccatagagga tgttattatg aatcattaaa aaaattaact gaggatgatt gattaaaaaa 104640
   tataaattaa tttaccatcg tgtattttta taacgggatt gtccggcata tcatgtagat 104700
   agttaccgtc tacatcgtat actcgaccat ctacgccttt aaatcctcta tttattgaca 104760
   ttaatctatt agaattggaa taccaaatat tagtaccctc aattagttta ttggtaatat 104820
   tttttttaga cgatagatcg atggctcttg aaaccaaggt tttccaaccg gactcattgt 104880
   cgatcggtga gaagtctttt tcattagcat gaatccattc taatgatgta tgtttaaaca 104940
   ctctaaacaa ttggacaaat tcttttgatt tgctttgaat gatttcaaat aggtcttcgt 105000
   ctacagtagg cataccatta gataatctag ccattataaa gtgcacgttt acatatctac 105060
   gttctggagg agtaagaacg tgactattga gacgaatggc tcttcctact atctgacgaa 105120
   gagacgcctc gttccatgtc atatctaaaa tgaagatatc attaattgag aaaaaactaa 105180
   taccctcgcc tccactagaa gagaatacgc atgttttaat gcattctccg ttagtgtttg 105240
   attcttggtt aaactcagcc accgccttga ttctagtatc ttttgttcta gatgagaact 105300
   ctatattaga gataccaaag actttgaaat atagtaataa gatttctatt cctgactgat 105360
   taacaaatgg ttcaaagact agacatttac catgggatgc taatattccc aaacatacat 105420
   ctataaattt gacgcttttc tcttttaatt cagtaaatag agagatatca gccgcactag 105480
   catccccttt caatagttct ccctttttaa aggtatctaa tgcggattta gaaaactctc 105540
   tatctcttaa tgaattttta aaatcattat atagtgttgc tatctcttgc gcgtattcgc 105600
   ccggatcacg attttgtctt tcaggaaagc tatcgaacgt aaacgtagta gccatacgtc 105660
   tcagaattct aaatgatgat atacctgttt ttatttcagc gagtttagcc ttttgataaa 105720
   tttcttcttg ctttttcgac atattaacgt atcgcattaa tactgttttc ttagcgaatg 105780
   atgcagaccc ttctacgtca tcaaaaatag aaaactcgtt attaactata tacgaacata 105840
   gtcctcctag tttggagact aattcttttt catcgactag acgtttattc tcaaatagcg 105900
   attggtgttg taaggatcct ggtcgtagta agttaaccaa catggtgaat tcttgcacac 105960
   tattgacgat aggtgtagcc gataaacaaa tcatcttatg gttttttaac gcaatggtct 106020
   tagataaaaa attatatact gaacgagtag gacggatctt accatcttct ttgattaatg 106080
   atttagaaat gaagttatga cattcatcaa taatgacgca tattctactc ttggaattaa 106140
   tagttttgat attagtaaaa aatttatttc taaaattttg atcatcgtaa ttaataaaaa 106200
   tacaatcctt cgttatctct ggagcgtatc tgagtatagt gctcatccaa ggatcttcta 106260
   tcaaagcctt tttcaccaat aagataatag cccaattcgt ataaatatcc ttaagatgtt 106320
   tgagaatata tacagtagtc attgttttac cgacacccgt ttcatggaac aataaaagag 106380
   aatgcatact gtctaatcct aagaaaactc ttgctacaaa atgttgataa tccttgaggc 106440
   gtactacgtc cgaccccatc atttcaacgg gcatattagt agttctgcgt aaggcataat 106500
   cgatataggc cgcgtgtgat ttactcattt atgagtgata agtaataact atgttttaaa 106560
   aatcacagca gtagtttaac tagtcttctc tgatgtttgt tttcgatact ttttgaatca 106620
   gaagtcatac tagaataaag caacgagtga acgtaataga gagcttcgta tactctattc 106680
   gaaaactcta agaacttatt aatgaattcc gtatccactg gattgtttaa aatactaaat 106740
   tgaacactgt tcacatcctt ccaagaagaa gacttagtga cggacttaac atgagacata 106800
   aataaatcca aatttttttt acaaacatca ctagccacca taatggcgct atctttcaac 106860
   cagctatcgc ttacgcattt tagcagtcta acatttttaa agagactaca atatattctc 106920
   atagtatcga ttacacctct accgaataaa gttggaagtt taataataca atatttttcg 106980
   tttacaaaat caaataatgg tcgaaacacg tcgaaggtta acatcttata atcgctaatg 107040
   tatagattgt tttcagtgag atgattatta gatttaatag catctcgttc acgtttgaac 107100
   agtttattgc gtgcgctgag gtcggcaact acggcgtccg ctttagtact cctcccataa 107160
   tactttacgc tattaatctt taaaatttca tagactttat ctagatcgct ttctggtaac 107220
   atgatatcat gtgtaaaaag ttttaacatg tcggtcggca ttctatttag atcattaact 107280
   ctagaaatct gaagaaagta attagctccg tattccagac taggtaatgg gcttttacct 107340
   agagacagat taagttctgg caatgtttca taaaatggaa gaaggacatg cgttccctcc 107400
   cggatatttt ttacaatttc atccatttac aactctatag tttgttttca ttattattag 107460
   ttattatctc ccataatctt ggtaatactt accccttgat cgtaagatac cttatacagg 107520
   tcattacata caactaccaa ttgtttttgt acataataga ttggatggtt gacatccatg 107580
   gtggaataaa ctactcgaac agatagttta tctttccccc tagatacatt agccgtaata 107640
   gttgtcggcc taaagaatat ctttggtgta aagttaaaag ttagggttct tgttccatta 107700
   ttgctttttg tcagtagttc attataaatt ctcgagatgg gtccgttctc tgaatataga 107760
   acatcatttc caaatctaac ttctagtcta gaaataatat cggtcttatt cttaaaatct 107820
   attcccttga tgaagggatc gttaatgaac aaatccttgg cctttgattc ggctgatcta 107880
   ttatctccgt tatagacgtt acgttgacta gtccaaagac ttacaggaat agatgtatcg 107940
   atgatgttga tactatgtga tatgtgagca aagattgttc tcttagtggc atcactatat 108000
   gttccagtaa tggcggaaaa ctttttagaa atgttatata taaaagaatt ttttcgtgtt 108060
   ccaaacatta gcagattagt atgaagataa acactcatat tatcaggaac attatcaatt 108120
   tttacataca catcagcatc ttgaatagaa acgataccat cttctggaac ctctacgatc 108180
   tcggcagact ccggataacc agtcggtggg ccatcgctaa caataactag atcatccaac 108240
   aatctactca catatgcatc tatataatct ttttcatctt gtgagtaccc tggatacgaa 108300
   ataaatttat tatccgtatt tccataataa ggtttagtat aaacagagag agatgttgcc 108360
   gcatgaactt cagttacagt cgccgttggt tggtttattt gacctattac tctcctaggt 108420
   ttctctataa acgatggttt aatttgtaca ttcttaacca tatatccaat aaagctcaat 108480
   tcaggaacat aaacaaattc tttgttgaac gtttcaaagt cgaacgaaga gtcacgaata 108540
   acgatatcgg atactggatt gaaggttacc gttacggtaa tttttgaatc ggatagttta 108600
   agactgctga atgtatcttc cacatcaaac ggagttttaa tataaacgta tactgtagat 108660
   ggttctttaa tagtgtcatt aggagttagg ccaatagaaa tatcattaag ttcactagaa 108720
   tatccagagt gtttcaaagc aattgtatta ttgatacaat tattatataa ttcttcgccc 108780
   tcaatttccc aaataacacc gttacacgaa gagatagata cgtgattaat acatttatat 108840
   ccaacatatg gtacgtaact gaatcttccc atacctttaa cttctggaag ttccaaactc 108900
   agaaccaaat gattaagcgc agtaatatac tgatccctaa tttcgaagct agcgatagcc 108960
   tgattgtctg gaccatcgtt tgtcataact ccggatagag aaatatattg cggcatatac 109020
   aaagttggaa tttgactatc gactgcgaag acattagacc gtttaataga gtcatcccca 109080
   ccgatcaaag aattaatgat agtattattc attttctatt taaaatggaa aaagcttaca 109140
   ataaactccg tagagaaata tctataattt gtgagttttc cttaaagtaa cagcttccgt 109200
   aaacgccgtc tttatctctt agtaggttta ttgtatttat gaccttttcc ttatcttcat 109260
   agaatactaa aggcaacaaa gaaatttttg gttcttctct aagagctacg tgagacttaa 109320
   ccatagaagc caacgaatcc ctacatattt tagaacagaa ataccctact tcaccaccct 109380
   tgtatgtctc aatactaata ggtctaaaaa ccaaatcttg attacaaaac caacacttat 109440
   caattacact atttgtctta atagacacat ctgccataga tttataatac tttggtagta 109500
   tacaagcgag tgcttcttct ttagcgggct taaagactgc tttaggtgct gaaataacca 109560
   catctggaag gcttactcgc ttagccattt aattacggaa ctattttttt atacttctaa 109620
   tgagcaagta gaaaacctct catctacaaa aacgtactcg tgtccataat cctctaccat 109680
   agttacacgt tttttagatc tcatatgtgc taaaaagttt tcccatacta attggttact 109740
   attatttttc gtataatttt taacagtttg aggttttaga tttttagtta cagaagtgat 109800
   atcgaatatt ttatccaaaa agaatgaata attaattgtc ttagaaggag tgttttcttg 109860
   gcaaaagaat accaagtgct taaatatttc tactacttca ttaatctttt ctgtactcag 109920
   attcagtttc tcatctttta cttgattgat tatttcaaag actaacttat aatccttttt 109980
   atttattctc tcgttagcct taagaaaact agatacaaaa tttgcatcta catcatccgt 110040
   ggatatttga tttttttcca tgatatccaa gagttccgag ataatttctc cagaacattg 110100
   atgagacaat aatctccgca atacatttct caaatgaata agtttattag acacgtggaa 110160
   gtttgacttt ttttgtacct ttgtacattt ttgaaatacc gactcgcaaa aaatacaata 110220
   ttcatatcct tgttcagata ctataccgtt atgtctacaa ccgctacata atcgtagatt 110280
   catgttaaca ctctacgtat ctcgtcgtcc aatattttat ataaaaacat tttatttcta 110340
   gacgttgcca gaaaatcctg taatattttt agttttttgg gctgtgaata aagtatcgcc 110400
   ctaatattgt taccgtcttc cgccaatata gtagttaaat tatccgcaca tgcagaagaa 110460
   caccgcttag gcggattcag tacaatgtta tatttttcgt accaactcat ttaaatatca 110520
   taatctaaaa tagttctgta atatgtctag cgctaatata ttgatcataa tcctgtgcat 110580
   aaattaagat acaacaatgt ctcgaaatca tcgacatggc ttcttccata gttagaagat 110640
   cgtcgtcaaa gttagcaacg tgattcatca acatttgctg ttttgaggca gcaaatactg 110700
   aaccgtcgcc attcaaccat tcataaaaac catcgtctga atccattgat aatttcttgt 110760
   actggttttt gagagctcgc atcaatctag catttctagc tcccggattg aaaacagaaa 110820
   gaggatcgta catccagggt ccattttctg taaatagaat cgtataatgt cccttcaaga 110880
   agatatcaga cgatccacaa tcaaagaatt ggtctccgag tttgtaacaa actgcggact 110940
   ttaacctata catgataccg tttagcataa tttctggtga tacgtcaatc ggagtatcat 111000
   ctattagaga tctaaagccg gtgtaacatt ctccaccaaa catattctta ttctgacgtc 111060
   gttctacata aaacatcatt gctccattaa cgataacagg ggaatgaaca gcactaccca 111120
   tcacattagt tcccaatgga tcaatgtgtg taactccaga acatcttcca tagcctatgt 111180
   taggaggagc gaacaccact cttccactat tgccatcgaa tgccatagaa taaatatcct 111240
   tggaattgat agaaatcgga ctgtcggatg ttgtgatcat cttcatagga ttaacaacta 111300
   tgtatggtgc cgcctgaagt ttcatatcgt aactgatgcc gtttataggt ctagccacag 111360
   aaaccaacgt aggtctaaat ccaactatag acaaaataga agccaatatc tgttcctcat 111420
   ctgtcataac ttgagagcat ccagtatgaa taatcttcat tagatgggga tctaccgcat 111480
   catcatcgtt acaataaaaa attcccattc taatgttcat aattgctttt ctaatcatgg 111540
   tatgcatgtt tgctctctga atctctgtgg aaattagatc tgatacacct gtaatcacta 111600
   tcggattatc ctccgtaaga cgattaacca acaacatata attataagac tttacttttc 111660
   taaattcata aagttgctgg attaggctat aggtgtctcc atgtacatac gcgttctcga 111720
   gcgcaggaag tttaataccg aatagtgcca tcagaatagg atgaatatag taattagttt 111780
   ctggttttct ataaataaaa gacaaatctt gtgaactaga catatcggta aaatgcatgg 111840
   attggaatcg tgtagtcgac agaagaatat gatgattaga tggagagtat attttatcta 111900
   actctttgag ttggtcaccg attctaggac tagctcgaga atgaataagt actaaaggat 111960
   gagtacattt cacagaaaca ctagcattgt tcaatgtgct ctttacatgg gtaaggagtt 112020
   gaaatagctc gtttctattt gttctgacaa tatttagttt attcataatg ttaagcatat 112080
   cctgaatagt aaagttagat gtgtcatact tgttagtagt tagatattta gcaattgcat 112140
   tcccatcatt tctcaatctc gtactccaat catgcgtgga tgctacttcg tcgatggaaa 112200
   ccatacaatc ctttttgata ggctgttgag attgatcatt tcctgcacgt ttaggtttgg 112260
   tacgttgatt tctagcccct gcggatataa agtcatcgtc tacaatttgg gacaatgaat 112320
   tgcatacact acaagacaaa gatttatcag aagtgtgaat atgatcttca tctaccaaag 112380
   aaagagtttg attagtataa ctagatttta gtcctgcgtt agatgttaaa aaaacatcgc 112440
   tattgaccac ggcttccatt atttatattc gtagttttta ctcgaaagcg tgattttaat 112500
   atccaatctt attacttttg gaatcgttca aaacctttga ctagttgtag aatttgatct 112560
   attgccctac gcgtatactc ccttgcatca tatacgttcg tcaccagatc gtttgtttcg 112620
   gcctgaagtt ggtgcatatc tctttcaaca ttcgacatga gatccttaag ggccatatcg 112680
   tctagatttt gttgagatgc tgctcctgga tttggatttt gttgtgctgt tgtacatact 112740
   gtaccaccag taggtgtagg agtacataca gtggccacaa taggaggttg agaaagtgta 112800
   accgttggag tagtacaaga aatacttcca tccgattgtt gtgtacatgt agttgttggt 112860
   aacgtctgag aaggttgggt agatggcggc gtcgtcgttt tttgatcttt attaaattta 112920
   gagataatat cctgaacagc attgctcggc gtcaacgctg gaaggagtga actcgccggc 112980
   gcatcagtat cttcagacag ccaatcaaaa agattagaca tatcagatga tgtattagtt 113040
   tgttgtcgtg gttttggtgt aggagcagta ctactaggta gaagaatagg agccgatgta 113100
   gctgttggaa ccggctgtgg agttatatga atagttggtt gtagcggttg gataggctgt 113160
   ctgctggcgg ccatcatatt atctctagct agttgttctc gcaactgtct ttgataatac 113220
   gactcttgag actttagtcc tatttcaatc gcttcatcct ttttcgtatc cggatccttt 113280
   tcttcagaat aatagattga cgactttggt gtagaggatt ctgccagcct ctgtgagaac 113340
   ttgttaaaga agtccattta aggctttaaa attgaattgc gattataaga ttaaatggca 113400
   gacacagacg atattatcga ctatgaatcc gatgatctca ccgaatacga ggatgatgaa 113460
   gaagaggaag aagatggaga gtcactagaa actagtgata tagatcccaa atcttcttat 113520
   aagattgtag aatcagcatc cactcatata gaagatgcgc attccaatct taaacatata 113580
   gggaatcata tatctgctct taaacgacgc tatactagac gtataagtct atttgaaata 113640
   gcgggtataa tagcagaaag ctataacttg cttcaacgag gaagattacc tctagtttca 113700
   gaattttctg acgaaacgat gaagcaaaat atgctacatg taattataca agagatagag 113760
   gagggttctt gtcctatagt catcgaaaag aacggagaat tgttgtcggt aaacgatttt 113820
   gacaaagatg gtctaaaatt ccatctagac tatattatca aaatttggaa acttcaaaaa 113880
   cgatattaga atttatacga atatcgttct ctaaatgtca caatcaagtc tcgcatgttc 113940
   agcaatttat tgtcgtactt tatatcgtgt tcattaacga tatcttgcaa aatagtaatg 114000
   attctatctt ccttcgatag atattcttca gagattattg tcttatattc tttcttgtta 114060
   tcagatatga atttgataag actttgaaca ttattgatac ccgtctgttt aattttttct 114120
   acagatattt tagttttggc agattctatc gtatctgtca atagacatcc aacatcgaca 114180
   ttcgacgtca attgtctata aatcaacgta taaattttag aaataacatt agcgaattgt 114240
   tgtgcgttga tgtcgttatt ctgaaacagt atgattttag gtagcatttt cttaacaaag 114300
   agaacgtatt tattgttact cagttgaaca gatgatatat ccagattact aacgcatctg 114360
   attccgtata ccaaactttc agaagaaatg gtatacaatt gtttgtattc attcaatgtc 114420
   tctttttcag aaattagttt agagtcgaat actgcaataa ttttcaagag atagttttca 114480
   tcagataaga ttttatttag tgtagatatg ataaaactat tgttttgttg gagaacttga 114540
   tacgccgcgt tctctgtagt cgacgctctc aaatgggaaa caatctccat tatttttttg 114600
   gaatcggata ctatatcttc ggtatcttga cgcagtctag tatacataga gttaagagag 114660
   attagagttt gtacattaag caacatgtct ctaaatgtgg ctacaaactt ttcctttttc 114720
   acatcatcta gtttattata taccgatttc acaacggcac cagatttaag gaaccagaat 114780
   gaaaaactct gataactaca atatttcatc atagttacga ttttatcatc ttctatagtt 114840
   ggtgtaatag cgcatacctt tttctccaag actggaacca acgtcataaa aatgtttaaa 114900
   tcaaaatcca tatcaacatc tgatgcgcta agaccagtct cgcgttcaag attatcttta 114960
   ctaatggtga cgaactcatc gtataaaact ctaagtttgt ccattattta tttacagatt 115020
   tagttgttta atttatttgt gctcttccag agttgggata gtatttttct aacgtcggta 115080
   ttatattatt aggatctacg ttcatatgta tcataatatt aatcatccac gttttgataa 115140
   atctatcttt agcttctgaa ataacgtatt taaacaaagg agaaaaatat ttagctacgg 115200
   catcagacgc aataacattt tttgtaaatg taacgtattt agacgacaga tcttcgttaa 115260
   aaagttttcc atctatgtag aatccatcgg ttgttaacac cattcccgcg tcagattgaa 115320
   taggagtttg aatagtttgt tttggaaata gatccttcaa taacttatag ttgggtggga 115380
   aaaaatcgat tttatcacta gactctttct tttttactat cattacctca tgaactattt 115440
   cttgaatgag tatatgtatt ttctttccta tatcggacgc gttcattgga aaatatacca 115500
   tgtcgttaac tataagaata tttttatcct cgtttacaaa ctgaataata tcagatgtag 115560
   ttcgtaaacg aactatatca tcaccagcac aacatctaac tatatgatat ccactagttt 115620
   cctttagccg tttattatct tgttccatat tagcagtcat tccatcattt aagaaggcgt 115680
   caaaaataat agggagaaat gacattttgg attctgttac aactttacca aaattaagga 115740
   tatacggact tactatcttt ttctcaacgt caatttgatg aacacacgat gaaaatgtgc 115800
   ttctatgaga ttgatcatgt agaaaacaac aagggataca atatttccgc atatcatgaa 115860
   atatattaag aaatcccacc ttattatatt tccccaaagg atccatgcac gtaaacatta 115920
   tgccgttatc attaataaag acttctttct catcggatct gtaaaagttg ttactgattt 115980
   ttttcattcc aggatctaga taattaataa tgatgggttt tctattctta ttctttgtat 116040
   tttggcatat cctagaccag taaacagttt ccactttggt aaaatcagca gacttttgaa 116100
   cgctattaaa catggcatta atggcaataa ctaaaaatgt aaaatatttt tctatgttag 116160
   gaatatggtt tttcacttta atagatatat ggtttttggc caaaatgata gatatttttt 116220
   tatccgagga tagtaaaata ttattagtcg ccgtctctat aaaaatgaag ctagtctcga 116280
   tatccaattt tattctagaa ttgataggag tcgccaaatg taccttatac gttatatctc 116340
   ccttgatgcg ttccatttgt gtatctatat cggacacaag atctgtaaat agttttacgt 116400
   tattaatcat cacggtatcg ccgtcgctag ataacgctaa tgtaccatcc aagtcccaaa 116460
   tggagagatt taactgttca tcgtttagaa taaaatgatt accggtcata ttaataaagt 116520
   gttcatcgta tctagataac aacgacttat aattaatgtc caagtcttga actcgctgaa 116580
   tgatcttttt taacccagtt agttttagat tggtacgaaa tatattgtta aactttgatt 116640
   ctacagtaat gtccaaatct agttgtggaa atacttccat caacattgtt tcaaacttga 116700
   taatattatt atctacatct tcgtacgatc caaattccgg aatagatgta tcgcacgctc 116760
   tggccaccca gataaccaaa aagtcacacg ctccaggata tacattgtat aaaaagctat 116820
   cgttttttag tagggttttt ttctgcgtgt atacgaaggg attaaaaata gtattatcaa 116880
   cgtaactata ttccaaatta ttcttatgag aatagataat aatatcgtcc ttaatatcta 116940
   acaaatttcc taaatatccc tttaattgag tcattcgaag cgtcaataga atatgtctct 117000
   taactatttc cggctgttgt atatttaaat gacttcgtaa aaaataatat atgggcgact 117060
   tctcatctat gtaatcatat ggagtgagat atagggctcg ttctacctcc tgccccttac 117120
   ccacctgtaa taccaattgc ggacttacta tatatcgcat atttatatcg tggggtaaag 117180
   tgaaaatcta ctaccgatga tgtaagtctt acaatgttcg aaccagtacc agatcttaat 117240
   ttggaggcct ccgtagaact aggggaggta aatatagatc aaacaacacc tatgataaag 117300
   gagaatagcg gttttatatc ccgcagtaga cgtctattcg cccatagatc taaggatgat 117360
   gagagaaaac tagcactacg attcttttta caaagacttt attttttaga tcatagagag 117420
   attcattatt tgttcagatg cgttgacgct gtaaaagacg tcactattac caaaaaaaat 117480
   aacattatcg tggcgcctta tatagcactt ttaactatcg catcaaaagg atgcaaactt 117540
   acagaaacaa tgattgaagc attctttcca gaactatata atgaacatag taagaaattt 117600
   aaattcaact ctcaagtatc catcatccaa gaaaaactcg gataccagtt tggaaactat 117660
   cacgtttatg attttgaacc gtattactct acagtagctc tggctattcg agatgaacat 117720
   tcatctggca tttttaatat ccgtcaagag agttatctgg taagttcatt atctgaaata 117780
   acatatagat tttatctaat taatctaaaa tctgatcttg ttcaatggag tgctagtacg 117840
   ggcgctgtaa ttaatcaaat ggtaaatact gtattgatta cagtgtatga aaagttacaa 117900
   ctggtcatag aaaatgattc acaatttaca tgttcattgg ctgtggaatc aaaacttcca 117960
   ataaaattac ttaaagatag aaatgaatta tttacaaaat tcattaacga gttaaaaaag 118020
   accagttcat tcaagataag caaacgcgat aaggatacgc tactaaaata ttttacttag 118080
   gactggagtt agaatttata gacgactcat ttcgtttatc attgttacta ttattactat 118140
   tactatcatt attagtgttg gcattattag tattcttctt gtcatcttgt tcagaaatat 118200
   acagcaatgc tatacctaat actaaataca ttatcatgct cgcaatggct ctaacaacaa 118260
   cgaaccaaaa tgaatttggt cgtagctttt gttcacaaaa atacataaag aaatgtctac 118320
   ataaatctat ggcgccattg gctacttgaa atagcgccag tcctcctaca gattttaata 118380
   tagctgtata acatgacatt tattcatcat caaaagagac agagtcacca tctgtcatat 118440
   ttagattttt tttcatgtgt tcaaagtatc ctctactcat ttcattataa tagtttatca 118500
   tacttagaat tttaggacgg atcaatgagt aagacttgac tagatcgtca gtagtaattt 118560
   gtgcatcgtc tattctgcat ccgcttcgtc gaataatgta tagcatcgct ttgagattct 118620
   ccatagctat caagtcttta tacaatgaca tggaaatatc tgtgaatact ttatacttct 118680
   ccaacatcga tgccttaaca tcatcgccta ctttagcatt gaaaatacgt tctattgtgt 118740
   agatggatgt agcaagattt ttaaacaaca atgccatctt acacgatgat tgcctcaagt 118800
   ctccaatcgt ttgtttagaa cgattagcta cagagtccaa tgcttggctg actagcatat 118860
   tattatcttt agaaattgta ttcttcaatg aggcgtttat catatctgtg atttcgttag 118920
   tcatattaca gtctgactgg gttgtaatgt tatccaacat atcacctatg gatacggtac 118980
   acgtaccagc atttgtaata atcctatcta agatgttgta tggcattgcg cagaaaatat 119040
   cttctcctgt aatatctcca ctctcgataa atctactcag attattctta aatgccttat 119100
   tctctggaga aaagatatca gtgtccatca tttcattaat agtatacgca gaaaagatac 119160
   cacgagtatc aattctatcc aagatactta tcggttccga gtcacagata atggtttcct 119220
   ctccttcggg agatcctgca tagaaatatc taggacaata gtttctatac tgtctgtaac 119280
   tctgataatc tctaaagtca ctaactgata ccatgaaatt gagaagatca aacgctgaag 119340
   taattaattt ttctgcctcg tttttactac aactagtttt catcaatgta gtgacgatgt 119400
   attgtttagt tacttttggt ctaatactga tgatagagat attattgctt cccataatgg 119460
   atcttctagt agtcacctta aagcccattg atgcgaatag cagatagata aagtcttggt 119520
   atgactcctt tctaatatag tacggactac ctttgtcacc caactttata cccacataag 119580
   ccataacaac ctctttaata gccgtttcat gaggtttatc agccatgagc ctgagtagtt 119640
   ggaagaatct catgaatccc gtctcagaaa gtcctatatg catgatagat ttatctttcc 119700
   tgggaaactc tcgtatagtc atagatgaaa tactctttaa agtttctgaa ataagattag 119760
   taacagtctt acctccgact actctaggta acaaacaaac tctaataggt gttttctctg 119820
   cggagataat atcagaaagg atagagcaat aagtagtatt attgtgatta taaagaccga 119880
   atacataaca ggtagaattt ataaacatca tgtcctgaag gtttttagac ttgtattcct 119940
   cgtaatccat accgtcccaa aacatggatt tggtaacttt gatagccgta gatctttgtt 120000
   ccttcgccaa caggttaaag aaattaataa agaatttgtt gtttctattt atgtccacaa 120060
   attgcacgtt tggaagcgcc acggttacat tcactgcagc attttgagga tcgcgagtat 120120
   gaagtacgat gttattgttt actggtatat ctggaaagaa atctaccagt ctaggaataa 120180
   gagattgata tcgcatagaa atagtaaagt ttataatctc atcatcgaag agcattttgt 120240
   taccattgta ataaatatcc actctgtcat atgtataaat gaagtactgt tcaaacatga 120300
   tgagatgttt atatgttggc atagtagtga gatcgacgtt tggtaatggc aatgtattaa 120360
   gattaactcc ataatgtcta gcagcatctg cgatgttata agcgtcgtca aagcggggtc 120420
   gatcttgtat tgttatatat tgtctaacac ctataagatt atcaaaatct tgtctgctta 120480
   atacaccgtt aacaattttt gccttgaatt cttttattgg tgcattaata acatccttat 120540
   agaggatgtt aaacaaataa gtgttatcaa agttaagatc tggatatttc ttttctgcta 120600
   gaacatccat tgagtcggag ccatctggtt taatataacc accgataaat ctagctctgt 120660
   attctgtatc cgtcaatcta atattaagaa ggtgttgagt gaaaggtgga agatcgtaaa 120720
   agctgtgagt attaatgata ggattagttt ccgaactaat gttaattggg gtattaataa 120780
   tatctatatt tccagcgtta agtgtaacat taaacagttt taattcacgt gacgtggtat 120840
   caattaaata attaatgccc aatttggata tagcagcctg aagctcatct tgtttagtta 120900
   cggatcctaa tgagttatta agcaatatat cgaacggatg aacgaaggtt gttttaagtt 120960
   ggtcacatac tttgtaatct agacatagat gcggaagaac ggtagaaact atacgaaata 121020
   aatattcaga gtcctctaat tgatcaagag taactattga cttaataggc atcatttatt 121080
   tagtattaaa tgacgaccgt accagtgacg gatatacaaa acgatttaat tacagagttt 121140
   tcagaagata attatccatc taacaaaaat tatgaaataa ctcttcgtca aatgtctatt 121200
   ctaactcacg ttaacaacgt ggtagataga gaacataatg ccgccgtagt gtcatctcca 121260
   gaggaaatat cctcacaact taatgaagat ctatttccag atgatgattc accggccact 121320
   attatcgaac gagtacaacc tcatactact attattgacg atactccacc tcctacgttt 121380
   cgtagagagt tattaatatc ggaacaacgt caacaacgag aaaaaagatt taatattaca 121440
   gtatcgaaaa atgctgaagc aataatggaa tctagatcta tgataacttc tatgccaaca 121500
   caaacaccat ccttgggagt agtttatgat aaagataaaa gaattcagat gttagaggat 121560
   gaagtggtta atcttagaaa tcaacgatct aatacaaaat catctgataa tttagataat 121620
   tttaccaaaa tactatttgg taagactccg tacaaatcaa cagaagttaa taagcgtata 121680
   gccatcgtta attatgcaaa tttgaacggg tcccccttat cagtcgagga cttggatgtt 121740
   tgttcggagg atgaaataga tagaatctat aaaacgatta aacaatatca cgaaagtaga 121800
   aaacgaaaaa ttatcgtcac taacgtgatt attattgtca taaacattat cgagcaggca 121860
   ttgctaaaac tcggatttga agaaatcaaa ggactgagta ccgatatcac ttcagaaatt 121920
   atcgatgtgg agatcggaga tgactgcgat gctgtagcat ctaaactagg aatcggtaac 121980
   agtccggttc ttaatattgt attgtttata ctcaagatat tcgttaaacg aattaaaatt 122040
   atttaattta atacattccc atatccagac aacaatcgtc tggattaatc tgttcctgtc 122100
   gtctcatacc ggacgacata ttaatctttt tattagtggg catcttttta gatggtttct 122160
   ttttcccagc attaactgag tcgataccta gaagatcgtg attgatctct ccgaccattc 122220
   cacgaacttc taattggccg tctctgacgg taccataaac tattttacca gcattagtaa 122280
   cagcttggac aatctgacca tccatcgcat tgtacgatgt agtagtaact gttgttctac 122340
   gtctaggagc accagaagta tttttggagc ccttggatgt tgatgtagaa gaagacgagg 122400
   attttgattt tggtttacat gtaatacatt ttgaactctt tgattttgta tcacatgcgc 122460
   cggcagtcac atctgtttga gaattaagat tattgttgcc tcctttgacg gctgcatctc 122520
   caccgatttg cgctagtaga tttttaagct gtggtgtaat cttattaact gtttcgatat 122580
   aatcatcgta actgcttcta acggctaaat tttttttatc cgccatttag aagctaaaaa 122640
   tatttttatt tatgcagaag atttaactag attatacaat gaactaatat gatccttttc 122700
   cagattattt acaaacttgg tattttttgg ttctggagga ggcgaattta aattcggact 122760
   tggattcgga ttttgtaagt tcttgatctt attatacatc gagtatagga tggcgacagt 122820
   aactgctaca caaataccga tcaaaagaag aataccaatc atttattgac aataacttca 122880
   ctattgatca agtatgcaat atatcatctt ttcactaaat aagtagtaat aatgattcaa 122940
   caatgtcgag atatatggac gataataatt tagttcatgg aaatatcgct atgattggtg 123000
   tgaatgactc cgctaactct gtggggtgcg cagtgctttc cccacataga ataaattagc 123060
   attccgactg tgataataat accaagtata aacgccataa tactcaatac tttccatgta 123120
   cgagtgggac tggtagactt actaaagtca ataaaggcga agatacacga aagaatcaaa 123180
   agaatgattc cagcgattag cacgccggaa aaataatttc caatcataag catcatgtcc 123240
   atttaactaa taaaaatttt aaatcgccga atgaacaaag tggaatataa accatataaa 123300
   aacaatagtt tgtactgcaa aaataatatc tatttttgtt ttcgaagata tggtaaaatt 123360
   aaatagtagt acacagcatg ttataactaa cagcagcaac ggctcgtaat tacttatcat 123420
   ttactagacg aaaaggtggt gggatatttt cttgctcaaa taatacgaat atatcaccca 123480
   tccattttat gcgatgttta tatactctaa tctttaatag atctatagac gacgggttta 123540
   ccaacaatat agattttatc gattcatcta atttaaaccc ttccttaaac gtgaatgatc 123600
   tattatctgg cataacgatg accctacctg atgaatcgga caatgtactg ggccatgtag 123660
   aataaattat caacgaatta tcgtctacga acatttatat catttgtttt aattttagga 123720
   cgcgaataaa tggatataaa atagaaaata acagatatta caaccagtgt tatggccgcg 123780
   cccaaccagg taggcagttt tattttatct tttactacag gttctcctgg atgtacgtca 123840
   ccaacggcgg acgtagttct agtacaatta gacgtaagtt ccgcttggga attttttaac 123900
   gctaaagagt taacgttaat cgtgcaccca acgtatttac atctagttcg ttgaacatct 123960
   tgattataat ataaccattt tctatctcta gattcgtcag tgcactcatg taaccaacat 124020
   accctaggtc ctaaatattt atctccggaa ttagattttg gataattcgc gcaccaacaa 124080
   tttctatttc ctttatgatc gttacaaaag acgtataatg ccgtatcccc aaaagtaaaa 124140
   taatcaggac gaataattct aataaactca gaacaatatc tcgcatccat atgtttggag 124200
   caaatatcgg aataagtaga catagccggt ttccgttttg cacgtaacca ttctaaacaa 124260
   ttggggtttc caggatcgtt tctacaaaat ccagtcatga aatcgtcaca atgttctgtc 124320
   ttgtaattat tattaaatat ttttggacag tgtttggtat ttgtcttaga acaacatttt 124380
   gccacgctat cactatcgcc caggagataa tcctttttta taaaatgaca tcgttgcccg 124440
   gatgctatat aatcagtagc gtgttttaaa tccttaatat attcaggagt tacctcgttc 124500
   tgataataga ttaatgatcc aggacgaaat ttgaaagaac tacatggttc tccatgaatt 124560
   aatacatatt gtttagcaaa ttcaggaact ataaaactac tacaatgatc tatcgacata 124620
   ccatctatca aacaaaactt gggtttaatt tctcccggag atgtttcata atagtacgta 124680
   taactttctt ctgcaaactt aacagctcta ttatattcag gataattaaa acctaattcc 124740
   atatatttgt ctcgtatatc tgctattcct ggtgctattt tgattctatt aagagtaaca 124800
   gctgccccca ttcttaataa tcgtcagtat ttaaactgtt aaatgttggt atatcaacat 124860
   ctaccttatt tcccgcagta taaggtttgt tgcaggtata ctgttcagga atggttacat 124920
   ttatacttct tctatagtcc tgtctttcga tgttcatcac atatgcaaag aacagaataa 124980
   acaaaataat gtaagaaata atattaaata tctgtgaatt cgtaaataca ttgattgcca 125040
   taataattac agcagctaca atacacacaa tagacattcc cacagtgttg ccattacctc 125100
   cacgatacat ttgagttact aagcaatagg taataactaa gctagtaaga ggcaatagaa 125160
   aagatgagat aaatatcatc aatatagaga ttagaggagg gctatataga gccaagacga 125220
   acaaaatcaa accgagtaac gttctaacat cattattttt gaagattccc aaataatcat 125280
   tcattcctcc ataatcgttt tgcatcatac ctccatcttt aggcataaac gattgctgct 125340
   gttcctctgt aaataaatct ttatcaagca ctccagcacc cgcagagaag tcgtcaagca 125400
   tattgtaata tcttaaataa ctcatttata tattaaaaaa tgtcactatt aaagatggag 125460
   tataatcttt atgccgaact aaaaaaaatg acttgtggtc aacccctaag tctttttaac 125520
   gaagacgggg atttcgtaga agttgaaccg ggatcatcct ttaagtttct gatacctaag 125580
   ggattttacg cctctccttc cgtaaagacg agtctagtat tcgagacatt aacaacgacc 125640
   gataataaaa tcactagtat caatccaaca aatgcgccaa agttatatcc tcttcaacgc 125700
   aaagtcgtat ctgaagtagt ttctaatatg aggaaaatga tcgaatcaaa acgtcctcta 125760
   tacatcactc ttcacttggc gtgtggattt ggtaagacta ttaccacgtg ttatcttatg 125820
   gctacacacg gtagaaaaac cgtcatttgc gtacccaata aaatgttaat acatcaatgg 125880
   aagacacagg tagaggcagt cggattggaa cataagatat ccatagatgg agtaagtagt 125940
   ctattaaagg aactaaagac tcaaagtccg gatgtattaa tagtagtcag tagacatctg 126000
   acaaacgatg ccttttgtaa atatatcaat aagcattatg atttgttcat cttggatgaa 126060
   tcacatacgt ataatctgat gaacaataca gcagttacaa gatttttagc gtattatcct 126120
   ccgatgatgt gttatttttt aactgctaca cctagaccat ctaaccgaat ttattgtaac 126180
   agtattatta atattgccaa gttatccaat ctaaaaaaaa ctatctatgc agtagatagt 126240
   ttttttgagc catattccac agataatatt agacatatgg taaaacgact agatggacca 126300
   tctaataaat atcatatata taccgagaag ttattatctg tagacgagcc tagaaatcaa 126360
   cttattcttg ataccctggt agaagaattc aagtcaggaa ctattaatcg cattttagtt 126420
   attactaaac tacgtgaaca tatggtatta ttctacaaac gattattaga ttttttcgga 126480
   ccagaggttg tatttatagg agacgcccaa aatagacgta ctccagatat ggtcaaatca 126540
   atcaaggaac taaatagatt tatattcgta tccaccttat tttattccgg tactggttta 126600
   gatattccta gtttggattc gttgttcatt tgctcggcag taatcaacaa tatgcaaata 126660
   gagcaattac tagggagggt atgtcgagaa acagaactat tagataggac ggtatatgta 126720
   tttcctaaca catccatcaa agaaataaag tacatgatag gaaatttcat gcaacgaatt 126780
   attagtctgt ctgtagataa actaggattt aaacaaaaaa gttatcggaa acatcaagaa 126840
   tccgatccca cttctgcatg tacaacatca tccagagaag aacgtgtatt aaatagaata 126900
   tttaactcgc aaaatcgtta agaagtttaa gcgacgatcc gcatgctgcg caggccagtg 126960
   tattacccct catagtatta atataatcca atgatacttt tgtgatgtcg gaaatcttaa 127020
   ccaatttaga ctgacaggca gaacacgtca tgcaatcatc atcgtcatcg ataactgtag 127080
   tcttgggctt ctttttgcga ctcttcattc cggaacgcac attggtgcta tccatttagg 127140
   tagtaaaaaa taagtcagaa tatgccctat aacacgatcg tgcaaaacct ggtatatcgt 127200
   ctctatcttt atcacaatat agtgtatcga catttttatt attattgacc tcgtttatct 127260
   tggaacatgg aatgggaaca tttttgttat caacggccat ctttgcctta attccagatg 127320
   ttgtaaaatt ataactaaac agtctatcat cgacacaaat gaaattcttg tttagacgtt 127380
   tgtagtttac gtatgcggct cgttcgcgtc tcattttttc agatattgca ggtactataa 127440
   tattaaaaat aagaatgaaa taacatagga ttaaaaataa agttatcatg acttctagcg 127500
   ctgatttaac taacttaaaa gaattactta gtctgtacaa aagtttgaga ttttcagatt 127560
   ctgcggctat agaaaagtat aattctttgg tagaatgggg aacatctact tactggaaaa 127620
   taggcgtgca aaaggtagct aatgtcgaga cgtcaatatc tgattattat gatgaggtaa 127680
   aaaataaacc gtttaatatt gatccgggct attacatttt cttaccggta tattttggga 127740
   gcgtctttat ttattcgaag ggtaaaaata tggtagaact tggatctgga aactcttttc 127800
   aaataccaga tgatatgcga agtgcgtgta acaaagtatt agacagcgat aacggaatag 127860
   actttctgag atttgttttg ttaaacaata gatggataat ggaagatgct atatcaaaat 127920
   atcagtctcc agttaatata tttaaactag ctagtgagta cggattaaac atacccaaat 127980
   atttagaaat tgaaatagag gaagacacat tatttgacga cgagttatac tctattatag 128040
   aacgctcttt cgatgataaa tttccaaaaa tatccatatc gtatattaag ttgggagaac 128100
   ttagacggca agttgtagac tttttcaaat tctcattcat gtatattgag tccatcaagg 128160
   tagatcgtat aggagataat atttttattc ctagcgttat aacaaaatca ggaaaaaaga 128220
   tattagtaaa agatgtagac catttaatac gatccaaggt tagagaacat acatttgtaa 128280
   aagtaaaaaa gaaaaacaca ttttccattt tatacgacta tgatggaaac ggaacagaaa 128340
   ctagaggaga agtaataaaa cgaattatag acactatagg acgagactat tatgttaacg 128400
   gaaagtattt ctctaaggtt ggtagtgcag gcttaaagca attgactaat aaattagata 128460
   ttaatgagtg cgcaactgtc gatgagttag ttgatgagat taataaatcc ggaactgtaa 128520
   aacgaaaaat aaaaaaccaa tcagcatttg atttaagcag agaatgtttg ggatatccag 128580
   aagcggattt tataacgtta gttaataaca tgcggttcaa aatagaaaat tgtaaggttg 128640
   taaatttcaa tattgaaaat actaattgtt taaataaccc gagtattgaa actatatatg 128700
   gaaactttaa ccagttcgtc tcaatcttta atgtcgtcac cgatgtcaaa aaaagattat 128760
   tcgagtgaaa taatatgcgc ctttgatata ggtgcaaaaa atcctgccag aactgtttta 128820
   gaagtcaagg ataactccgt tagggtattg gatatatcaa aattagactg gagttctgat 128880
   tgggaaaggc gcatagctaa agatttgtca caatatgaat acactacagt tcttctagaa 128940
   cgtcagccta gaaggtcgcc gtatgttaaa tttatctatt ttattaaagg ctttttatat 129000
   catacatcgg ctgccaaagt tatttgcgtc tcgcctgtca tgtctggtaa ttcatataga 129060
   gatcgaaaaa agagatcggt cgaagcattt cttgattgga tggacacatt cggattgcga 129120
   gactccgttc cggatagacg caaattagac gatgtagcgg atagtttcaa tttggctatg 129180
   agatacgtat tagataaatg gaatactaat tatacacctt ataataggtg taaatctaga 129240
   aattacataa aaaaaatgta ataacgttag taacgccatt atggataatc tatttacctt 129300
   tctacatgaa atagaagata gatatgccag aactattttt aactttcatc taataagttg 129360
   cgatgaaata ggagatatat atggtcttat gaaagaacgc atttcctcag aggatatgtt 129420
   tgataatata gtgtataata aagatataca tcctgccatt aagaaactag tgtattgcga 129480
   catccaactt actaaacaca ttattaatca gaatacgtat ccggtattta acgattcttc 129540
   acaagtgaaa tgttgtcatt atttcgacat aaactcagat aatagcaata ttagctctcg 129600
   tacagtagag atatttgaga gggaaaagtc atctcttgta tcatatatta aaactaccaa 129660
   taagaagaga aaggtcaatt acggcgaaat aaagaaaact gttcatggag gcactaatgc 129720
   aaattacttt tccggtaaaa agtctgacga gtatctgagt actacagtta gatccaacat 129780
   taatcaacct tggatcaaaa ccatctctaa gaggatgaga gttgatatca ttaatcactc 129840
   tatagtaacg cgtggaaaaa gctctatatt acaaactata gaaattattt ttactaatag 129900
   aacatgtgtg aaaatattca aggattctac tatgcacatt attctatcca aggacaagga 129960
   tgaaaagggg tgtatacaca tgattgacaa attattctat gtctattata atttatttct 130020
   gttgttcgaa gatatcatcc aaaacgagta ctttaaagaa gtagctaatg ttgtaaacca 130080
   cgtactcacg gctacggcat tagatgagaa attattccta attaagaaaa tggctgaaca 130140
   cgatgtttat ggagttagca atttcaaaat agggatgttt aacctgacat ttattaagtc 130200
   gttggatcat accgttttcc cctctctgtt agatgaggat agcaaaataa agttttttaa 130260
   ggggaaaaag ctcaatattg tagcattacg atctctggag gattgtataa attacgtgac 130320
   taaatccgag aatatgatag aaatgatgaa ggaaagatcg actattttaa atagcataga 130380
   tatagaaacg gaatcggtag atcgtctaaa agaattgctt ctaaaatgaa aaaaaacact 130440
   aattcagaaa tggatcaacg actagggtat aagtttttgg tgcctgatcc taaagccgga 130500
   gttttttata gaccgttaca tttccaatat gtatcgtatt ctaattttat attgcatcga 130560
   ttgcatgaaa tcttgaccgt caagcggcca ctcttatcgt ttaagaataa tacagaacga 130620
   attatgatag aaattagcaa tgttaaagtg actcctccag attactcacc tataatcgcg 130680
   agtattaaag gtaagagtta tgacgcatta gccacgttca ctgtaaatat ctttaaagag 130740
   gtaatgacca aagagggtat atccatcact aaaataagta gttatgaggg aaaagattct 130800
   catttgataa aaattccgct actaatagga tacgggaata aaaatccact tgatacagcc 130860
   aagtatcttg ttcctaatgt cataggtgga gtctttatca ataaacaatc tgtcgaaaaa 130920
   gtaggaatta atctagtaga aaagattaca acatggccaa aatttagggt tgttaagcca 130980
   aactcattca ctttctcgtt ttcctccgta tcccctccta atgtattacc gacaagatat 131040
   cgccattaca agatatctct ggatatatca caattggaag cgttgaatat atcatcgaca 131100
   aagacattta taacggtcaa tattgttttg ctgtctcaat atttatctag agtgagtcta 131160
   gaattcatta gacgtagttt atcatacgat atgcctccag aagttgtcta tctagtaaac 131220
   gcgataatag atagtgctaa acgaattact gaatctatta ctgactttaa tattgataca 131280
   tacattaatg acctggtgga agctgaacac attaaacaaa aatctcagtt aacgatcaac 131340
   gagttcaaat atgaaatgct gcataacttt ttacctcata tgaactatac acccgatcaa 131400
   ctaaagggat tttatatgat atctttacta agaaagtttc tctactgtat cttccacact 131460
   tctagatatc cagatagaga ttcgatggtt tgtcatcgca tcctaacgta cggcaaatat 131520
   tttgagacgt tggcacatga tgaattagag aattacatag gcaacatccg aaacgatatc 131580
   atgaacaatc acaagaacag aggcacttac gcggtaaaca ttcatgtact aacaactccc 131640
   ggacttaatc acgcgttttc tagcttattg agtggaaagt tcaaaaagtc agacggtagt 131700
   tatcgaacac atcctcacta ttcatggatg cagaatattt ctattcctag gagtgttgga 131760
   ttttatccgg atcaagtaaa gatttcaaag atgttttctg tcagaaaata ccatccaagt 131820
   caatatcttt acttttgttc atcagacgtt ccggaaagag gtcctcaggt aggtttagta 131880
   tctcaattgt ctgtcttgag ttccattaca aatatactaa cgtctgagta tttggatttg 131940
   gaaaagaaaa tttgtgagta tatcagatca tattataaag atgatataag ttactttgaa 132000
   acaggatttc caatcactat agaaaatgct ctagtcgcat ctcttaatcc aaatatgata 132060
   tgtgattttg taactgactt tagacgtaga aaacggatgg gattcttcgg taacttggag 132120
   gtaggtatta ctttagttag ggatcacatg aatgaaattc gcattaatat tggagcggga 132180
   agattagtca gaccattctt ggttgtggat aacggagagc tcatgatgga tgtgtgtccg 132240
   gagttagaaa gcagattaga cgacatgaca ttctctgaca ttcagaaaga gtttccgcat 132300
   gtcatcgaaa tggtagatat agaacaattt acttttagta acgtatgtga atcggttcaa 132360
   aaatttagaa tgatgtcaaa ggatgaaaga aagcaatacg atttatgtga ctttcctgcc 132420
   gaatttagag atggatatgt agcatcttca ttagtgggaa tcaatcacaa ttctggaccc 132480
   agagctattc ttggatgtgc tcaagctaaa caagctatct cttgtctgag ctcggatata 132540
   cgaaataaaa tagacaatgg aattcatttg atgtatccag agaggccaat cgtgattagt 132600
   aaggctttag aaacttcaaa gattgcggct aattgcttcg gccaacatgt tactatagca 132660
   ttaatgtcgt acaaaggtat caatcaagag gatggaatta tcatcaaaaa acaatttatt 132720
   cagagaggcg gtctcgatat tgttacagcc aagaaacatc aagtagaaat tccattggaa 132780
   aactttaata acaaagaaag agataggtct aacgcctatt caaaattaga aagtaatgga 132840
   ttagttagac tgaatgcttt cttggaatcc ggagacgcta tggcacgaaa tatctcatca 132900
   agaactcttg aagatgattt tgctagagat aatcagatta gcttcgatgt ttccgagaaa 132960
   tataccgata tgtacaaatc tcgcgttgaa cgagtacaag tagaacttac tgacaaagtt 133020
   aaggtacgag tattaaccat gaaagaaaga agacccattc taggagacaa atttaccact 133080
   agaacgagtc aaaagggaac agtcgcgtat gtcgcggatg aaacggaact tccatacgac 133140
   gaaaatggta tcacaccaga tgtcattatt aattctacat ccatcttctc tagaaaaact 133200
   atatctatgt tgatagaagt tattttaaca gccgcatatt ctgctaagcc gtacaacaat 133260
   aagggagaaa accgacctgt ctgttttcct agtagtaacg aaacatccat cgatacatat 133320
   atgcaattcg ctaaacaatg ttatgagcat tcaaatccga aattgtccga tgaagaatta 133380
   tcggataaaa tcttttgtga aaagattctc tatgatcctg aaacggataa gccttatgca 133440
   tccaaagtat tttttggacc aatttattac ttgcgtctga ggcatttaac tcaggacaag 133500
   gcaaccgtta gatgtagagg taaaaagacg aagctcatta gacaggcgaa tgagggacga 133560
   aaacgtggag gaggtatcaa gttcggagaa atggagagag actgtttaat agcgcatggt 133620
   gcagccaata ctattacaga agttttgaaa gattcggaag aagattatca agatgtgtat 133680
   gtttgtgaaa attgtggaga catagcagca caaatcaagg gtattaatac atgtcttaga 133740
   tgttcaaaac ttaatctctc tcctctctta acaaaaattg ataccacgca cgtatctaaa 133800
   gtatttctta ctcaaatgaa cgccagaggc gtaaaagtca aattagattt cgaacgaagg 133860
   cctccttcgt tttataaacc attagataaa gttgatctca agccgtcttt tctggtgtaa 133920
   tattctagtt tggtagtaga tacatatcaa tatcatcaaa ttcgagatcc gaattataaa 133980
   atgggcgtgg attgttaact atagaatcgg acgtctgata ttcgaaaatc tgtggagttt 134040
   caggttttgg tggaggtgta actgctactt gggatactga agtctgatat tcagaaagct 134100
   gtggatgttc tggttcggca tccaccgatg gtgtcacatc actaatcggt tcggtaacgt 134160
   ctgtggatgg aggtgctact tctacagaac ctgtagcctc agttgtcaac ggagatacat 134220
   ttttaatgcg agaaaatgta taatttggta atggtttctt atgtggatct gaagaagagg 134280
   taagatatct actagaaaga taccgatcac gttctagttc tcttttgtag aacttaactt 134340
   tttctttctc cgcatctagt tgatattcca acctcttcac gttactacgt tcagattcca 134400
   attcacgttc gcatgggtta cctccgcagt ttttacgagc gatttcacgt tcagccttca 134460
   tgcgtctctc cctctctcta tcgagtttat cagagcagtc tttctgaagg cgatcgaact 134520
   ccataaattt ctccaacgct ttgattgttt ccatagattt ccgaagttca gcttttagga 134580
   ctgtgattct ttttctttcg aattcacagc tggatgtaca accgtttcca ttaccgccat 134640
   ctctaagttt cttttctaga tcggcaacat ttcatcccca tgccttttac attcctcgag 134700
   tctactgtcg tcgaaatatc gttccagctc cttttcgaca tcaataactt tagcacgttg 134760
   tctctcaagc tctcttttgt agttatctga ttccctggca cgtttaagat cttcatgcaa 134820
   ttgagtcagc tcttaacttc ctctcttgct tcttcgtcat agtacgcgca atcactgtga 134880
   gatccattgt taccacgtct acactcggcg agctcgcgtt taagagattc aatttcccgt 134940
   ttgtattggt ccatgtttcc attgctacca ccattagatt tacaggctgc tagttgtcgt 135000
   tcgagatcag aaatacgggt tttcttggaa ttgatttcgt cgatgtactt ggcatcgaaa 135060
   cacttattaa gttctttttc caattctacg attttatttc tttcgcgagt caattccctc 135120
   ctgtagtaac tatctgtttt gtcagattca cgctctctac gtagactttc ttgcaagtta 135180
   ctaatttgtt ccctagcacg tccgagttta gttttatatg ctgaatagag ttctgattca 135240
   tcctttgagc agatctctag cgatcgttta agattcctaa ttctagtctt tagcctattt 135300
   acctcctcag aagatgttcc gttaccgttg cgtttacact cgttaagctg tctatcaaga 135360
   tccatgattc tatctctaag acgttgcatc tctctttcca tatcagcatt gctttcatta 135420
   ttacgtctgc agtcactcaa ctgtctttca atatctgaga ttctatctct aagacgtcgc 135480
   atctctctct gtttcggcat tggtttcatt attacgttta cagtcgttca actgtctttc 135540
   aagatctgat attctagatt ggagtctgct aatctctgta gcattttcac ggcattcact 135600
   cagttgtctt tcaagatctg aaattttaga ttggagtctg ctaatctctg taagatttcc 135660
   tcctccgctc tcgatgcagt cggtcaactt attctctagt tctctaatac gcgaacgcag 135720
   tgcatcaact tcttgcgtgt cttcctggtt gcgtgtacat tcatcgagat ctctaacgcg 135780
   tcgtcgttct tcctcaagtt ctctgcgtac tacagaaagc gtgtccctat cttgttgata 135840
   tttagcaatt tctgattcta gagtactgat tttgcttacg tagttactaa tagttgtctt 135900
   ggccttatca agatcctcct tgtatttgtc gcattccttg atatccctac gaagtctgga 135960
   cagttcccat tcgacattac gacgtttatc gatttcagct cggagatcgt catcgcgttg 136020
   ttttagccac atacgactga gttcaagttc tcgttgacaa gatccatcta cttttccatt 136080
   cctaatagta tccagttcct tttctagttc tgaacgcatt tctcgttccc tatcaagcga 136140
   ttctctcaat tctcggatag tcttcttatc aatttctaat aaatctgaac catcatctgt 136200
   cccattttga atatccctgt gttctttgat ctcttttgta agtcggtcga ttctttcggt 136260
   tttataaaca gaatcccttt ccaaagtcct aatcttactg agtttatcac taagttctgc 136320
   attcaattcg gtgagttttc tcttggcttc ttccaactct gttttaaact ctccactatt 136380
   tccgcattct tcctcgcatt tatctaacca ttcaattagt ttattaataa ctagttggta 136440
   atcagcgatt cctatagccg ttcttgtaat tgtgggaaca taattaggat cttctaatgg 136500
   attgtatggc ttgatagcat catctttatc attattaggg ggatggacaa ccttaattgg 136560
   ttggtcctca tctcctccag tagcgtgtgg ttcttcaata ccagtgttag taataggctt 136620
   aggcaaatgc ttgtcgtacg cgggcacttc ctcatccatc aagtatttat aatcgggttc 136680
   tacttcagaa tattcttttc taagagacgc gacttcggga gttagtagaa gaactctgtt 136740
   tctgtatcta tcaacgctgg aatcaatact caagttaagg atagcgaata cctcatcgtc 136800
   atcatccgta tcttctgaaa caccatcata tgacatttca tgaagtctaa cgtattgata 136860
   aatagaatca gatttagtat taaacagatc cttaaccttt ttagtaaacg catatgtata 136920
   ttttagatct ccagatttca taatatgatc acatgcctta aatgtcagtg cttccatgat 136980
   ataatctgga acactaatgg gtgacgaaaa agatacagca ccatatgcta cgttgataaa 137040
   taaatctgaa ccactaagta gataatgatt aatgttaaga aagaggaaat attcagtgta 137100
   taggtatgtc ttggcgtcat atcttgtact aaacacgcta aacagtttgt taatgtgatc 137160
   aatttccaat agattaatta gagcagcggg aataccaaca aacatattac cacatccgta 137220
   ttttctatga atatcacata tcatgttaaa aaatcttgat agaagagcga atatctcgtc 137280
   tgacttaatg agtcgtagtt cagcagcaac ataagtcata actgtaaata gaacatactt 137340
   tcctgtagtg ttgattctag actccacatc aacaccatta ttaaaaatag ttttatatac 137400
   atctttaatc tgctctccgt taatcgtcga acgttctagt atacggaaac actttgattt 137460
   cttatctgta gttaatgact tagtgatatc acgaagaata ttacgaatta catttcttgt 137520
   ttttcttgag agacctgatt cagaactcaa ctcatcgttc catagttttt ctacctcagt 137580
   ggcgaaatct ttggagtgct tggtacattt ttcaataagg ttcgtgacct ccatttatta 137640
   taaaaaattt attcaaaact taactacaat cgggtaatta taagatcgta gatctcccat 137700
   gtggcggaat actaccatct atcgcatgtg gatggacagt aggtaatggc catgggaaca 137760
   gtaatgtttg catatttatc tttcttgcta gtattactgc atattgtccc aatgtttcga 137820
   tgtgatgttc taacctatca actgccgctg tatcacaaca atagtgtccg atgaaattaa 137880
   gattatgatc caatgtgttt aatatatgat tatcaagtct tatacgatcc gcgtcttttt 137940
   tgacaggatc aggttcttct acaggaagaa gtttcggcct cttatgatat tcatgtctgg 138000
   gaaacggtgg tctagggtga ggctccggta tcggagtggg ttttggatta taatcatcat 138060
   cgtctatgac atcatcttcg acttcgatat ttattttgct atcttgatga tgtcctgtat 138120
   cagttgcatt ttcagcactc gactgaatat tagcgcattc attgtctatt attaccatat 138180
   ttctaaaccc aaaatgtatg tgttgaacat cagtactatc gttgatgagt cttatagcat 138240
   gaattcgctt atcgttatcg ggtttatctt ctgtcacctt agcaattcct tttttattaa 138300
   actctacata atcatatcca tttctattgt ttgttctaat ataaacgagt atagcatcat 138360
   tgctaaattt ttcaatagta tcaaaaacag aatatcctaa accatataat atatattcag 138420
   gaacactcaa actaaatgtc caggattctc ctaaatacgt aaactttaat agtgcgaaat 138480
   cattcaaaaa tctaccactt atagatagat agtacataaa tgcgtatagt agtctaccta 138540
   tctctttatt atgaaaaccg gcattacgat catatatgtc gtgatatacc tgtgatccgt 138600
   ttacgttaaa ccataaatac atgggtgatc ctataaacat gaatttattt ctaattctca 138660
   gagctatagt taattgaccg tgtaatattt gcttacatgc atacttgata cgcttattaa 138720
   taagattttt atcattgctc gttatttcag aatcgtatat ataaggagta ccatcgtgat 138780
   tcttaccaga tattatacaa aatactatat ataaaatata ttgacccacg ttagtaatca 138840
   tataaatgtt taacgtttta aattttgtat tcaatgatcc attatcatac gctatcatgg 138900
   tcttgtaata ttcattcttt aaaatataat attgtgttag ccattgcatt ggagctccta 138960
   atggagattt tctattctca tccattttag gataggcttt cataaagtcc ctaataactt 139020
   cgtgaataat gtttctatgt tttctactga tgcatgtatt tgcttcgatt tttttatccc 139080
   atgtttcatc tatcatagat ttaaacgcag taatgctcgc aacattaaca tcttgaaccg 139140
   ttggtacaat tccgttccat aaatttataa tgttcgccat ttatataact cattttttga 139200
   atatactttt aattaacaaa agagttaagt tactcatatg gacgccgtcc agtctgaaca 139260
   tcaatctttt tagccagaga tatcatagcc gctcttagag tttcagcgtg attttccaac 139320
   ctaaatagaa cttcatcgtt gcgtttacaa cacttttcta tttgttcaaa ctttgttgtt 139380
   acattagtaa tctttttttc caaattagtt agccgttgtt tgagagtttc ctcattgtcg 139440
   tcttcatcgg ctttaacaat tgcttcgcgt ttagcctctg gctttttagc agcctttgta 139500
   gaaaaaaatt cagttgctgg aattgcaaga tcgtcatctc cggggaaaag agttccgtcc 139560
   atttaaagta cagattttag aaactgacac tctgcgttat ttatatttgg tacaacacat 139620
   ggattataaa tattgatgtt aataacatca gaaaatgtaa agtctataca ttgttgcatc 139680
   gtgttaaatt ttctaatgga tctagtatta ttgggtccaa cttctgcctg aaatccaaat 139740
   atggaagcgg atacaaaacc gtttcctgga taaaccacac atctccactt ttgctttaca 139800
   tcagaaattg tgtcgttgac atcttgaact ctcctatcta atgccggtgt tccacctata 139860
   gattttgaat attcgaatgc tgcatgagta gcattaaatt ccttaatatt gccataattt 139920
   tcatatattg agtaaccctg gataaaaagt aaacacaccg cagccgtcgc taccacaata 139980
   aaaaaaattg atagagagtt catttataat ctattagaag ctgacaaaat ttttttacac 140040
   gcatcagaca atgctttaat aaatagttca acatctactt ttgtcatatc gaaccgatgg 140100
   tatgattcta acctagaatt acatccgaaa aagttgacta tgttcatagt cattaagtca 140160
   ttaacaaaca acattccaga ctctggatta taagacgata ctgtttcgtc acaattacct 140220
   accttaatca tgtgattatg aatattggct attagagcac cttctaagaa atctataata 140280
   tctttgaaac acgatttaaa atcaaaccac gaatatactt ctacgaagaa agttagttta 140340
   cccataggag aaataactat aaatggagat ctaaatacaa aatccggatc tatgatagtt 140400
   ttaacattat tatattctct attaaatacc tccacatcta aaaatgttaa ttttgaaact 140460
   atgtcttcgt ttattaccgt acctgaacta aacgctataa gctctattgt ttgagaactc 140520
   tttaaacgat attcttgaaa tacatgtaac aaagtttcct ttaactcggt cggtttatct 140580
   accatagtta cagaatttgt atccttatct ataatataat aatcaaaatc gtataaagtt 140640
   atataattat cgcgttcaga ttgtgatctt ttcaaataga ctaaaaaccc catttctcta 140700
   gtaagtatct tatgtatatg tttgtaaaat atcttcatgg tgggaatatg ctctaccgca 140760
   gttagccatt cctcattgac agcggtagat gtattagaca aaactattcc aatgtttaac 140820
   aagggccatt ttacgagatt attaaatcct tgtttgataa atgtagccaa tgagggttcg 140880
   agttcaacga cgattgaatt ctcttcccgc ggatgctgca tgatgaacga cgggatgttg 140940
   ttcgattgat ttggaattct ttttcgactt tttgtttata ttaaatattt taaaatttat 141000
   agcggatagc aattcatgta ccacggataa tgtagacgcg tattgcgcat cgatatcttt 141060
   attattagat aaatttatca ataaatgtga gaagtttgcc tcgttaaggt cttccattta 141120
   aatattatat aaacatttgt gtttgtatct tattcgtctt ttatggaata gttttttact 141180
   agtaaagctg caattacaca ctttgtccgt aaaacataaa tataaacacc agcttttatc 141240
   aatcgttcca aaaagtcgac ggcggacatt tttaacatgg catctatttt aaatacactt 141300
   aggtttttgg aaaaaacatc attttataat tgtaacgatt caataactaa agaaaagatt 141360
   aagattaaac ataagggaat gtcatttgta ttttataagc caaagcattc taccgttgtt 141420
   aaatacttgt ctggaggagg tatatatcat gatgatttgg ttgtattggg gaaggtaaca 141480
   attaataatc taaagatgat gctattttac atggatttat catatcatgg agtgacaagt 141540
   agtggagcaa tttacaaatt gggatcgtct atcgatagac tttctctaaa taggactatt 141600
   gttacaaaag ttaataatta tgatgataca ttttttgacg acgatgattg atcgctattg 141660
   cacaattttg tttttgtact ttctaatata gtgtttaggt tctttttcat atgagaatat 141720
   tgatttacta aaatatctat gtttaacttt tgttctatga cgtccttatc ggcggtatcg 141780
   gtacatatac gtaattcacc ttcacaaaat acggagtctt cgataataat agccaatcga 141840
   ttattggatc tagctgtctg tatcatattc aacatgttta atatatcctt tcgtttcccc 141900
   tttacaggca tcgatcgtag catattttcc gcgtctgata tggaaatgtt aaaactacaa 141960
   aaatgcgtaa tgttagcccg tcctaatatt ggtacgtgtc tataagtttg gcatagtaga 142020
   ataatagacg tgtttaaatg ccttccaaag tttaagaatt ctattagagt attgcatttt 142080
   gatagtttat cgcctacatc atcaaaaata agtaaaaagt gtgctgattt tttatgattt 142140
   tgtgcgacag caatacattt ttctatgtta cttttagttc gtatcagatt atattctaga 142200
   gattcctgac tactaacgaa attaatatga tttggccaaa tgtatccatc ataatctggg 142260
   ttataaacgg gtgtaaacaa gaatatatgt ttatattttt taactagtgt agaaaacaga 142320
   gatagtaaat agatagtttt tccagatcca gatcctcctg ttaaaaccat tctaaacggc 142380
   atttttaata aattttctct tgaaaattgt ttttcttgga aacaattcat aattatattt 142440
   acagttacta aattaatttg ataataaatc aaaatatgga aaactaaggt tgttagtagg 142500
   gaggagaaca aagaaggcac atcgtgatat aaataacatt tattatcatg atgacaccag 142560
   aaaacgacga agagcagaca tctgtgttct ccgctactgt ttacggagac aaaattcagg 142620
   gaaagaataa acgcaaacgc gtgattggtc tatgtattag aatatctatg gttatttcac 142680
   tactatctat gattaccatg tccgcgtttc tcatagtgcg cctaaatcaa tgcatgtctg 142740
   ctaacgaggc tgctattact gacgccgctg ttgccgttgc tgctgcatca tctactcata 142800
   gaaaggttgc gtctagcact acacaatatg atcacaaaga aagctgtaat ggtttatatt 142860
   accagggttc ttgttatata ttacattcag actaccagtt attctcggat gctaaagcaa 142920
   attgcactgc ggaatcatca acactaccca ataaatccga tgtcttgact acctggctca 142980
   ttgattatgt tgaggataca tggggatctg atggtaatcc aattacaaaa actacatccg 143040
   attatcaaga ttctgatgta tcacaagaag ttagaaagta tttttgtgtt aaaacaatga 143100
   actaatattt atttttgtac attaataaat gaaatcgctt aatagacaaa ctgtaagtag 143160
   gtttaagaag ttgtcggtgc cggccgctat aatgatgata ctctcaacca ttattagtgg 143220
   cataggaaca tttctgcatt acaaagaaga actgatgcct agtgcttgcg ccaatggatg 143280
   gatacaatac gataaacatt gttatttaga tactaacatt aaaatgtcta cagataatgc 143340
   ggtttatcag tgtcgtaaat tacgagctag attgcctaga cctgatacta gacatctgag 143400
   agtattgttt agtatttttt ataaagatta ttgggtaagt ttaaaaaaga ccaatgataa 143460
   atggttagat attaataatg ataaagatat agatattagt aaattaacaa attttaaaca 143520
   actaaacagt acgacggatg ctgaagcgtg ttatatatac aagtctggaa aactggttaa 143580
   aacagtatgt aaaagtactc aatctgtact atgtgttaaa aaattctaca agtgacaaca 143640
   aaaaatgaat taataataag tcgttaacgt acgccgccat ggacgccgcg tttgttatta 143700
   ctccaatggg tgtgttgact ataacagata cattgtatga tgatctcgat atctcaatca 143760
   tggactttat aggaccatac attataggta acataaaaac tgtccaaata gatgtacggg 143820
   atataaaata ttccgacatg caaaaatgct actttagcta taagggtaaa atagttcctc 143880
   aggattctaa tgatttggct agattcaaca tttatagcat ttgtgccgca tacagatcaa 143940
   aaaataccat catcatagca tgcgactatg atatcatgtt agatatagaa gataaacatc 144000
   agccatttta tctattccca tctattgatg tttttaacgc tacaatcata gaagcgtata 144060
   acctgtatac agctggagat tatcatctaa tcatcaatcc ttcagataat ctgaaaatga 144120
   aattgtcgtt taattcttca ttctgcatat cagacggcaa tggatggatc ataattgatg 144180
   ggaaatgcaa tagtaatttt ttatcataaa agttgtaaag taaataataa aacaataaat 144240
   attgaactag tagtacgtat attgagcaat cagaaatgat gctggtacct cttatcacgg 144300
   tgaccgtagt tgcgggaaca atattagtat gttatatatt atatatttgt aggaaaaaga 144360
   tacgtactgt ctataatgac aataaaatta tcatgacaaa attaaaaaag ataaagagtt 144420
   ctaattccag caaatctagt aaatcaactg atagcgaatc agactgggag gatcactgta 144480
   gtgctatgga acaaaacaat gacgtagata atatttctag gaatgagata ttggacgatg 144540
   atagcttcgc tggtagttta atatgggata acgaatccaa tgttatggcg cctagcacag 144600
   aacacattta cgatagtgtt gctggaagca cgctgctaat aaataatgat cgtaatgaac 144660
   agactattta tcagaacact acagtagtaa ttaatgaaac ggagactgtt gaagtactta 144720
   atgaagatac caaacagaat cctaactatt catccaatcc tttcgtaaat tataataaaa 144780
   ccagtatttg tagcaagtca aatccgttta ttacagaact taacaataaa tttagtgaga 144840
   ataatccgtt tagacgagca catagcgatg attatcttaa taagcaagaa caagatcatg 144900
   aacacgatga tatagaatca tcggtcgtat cattggtgtg attagtttcc tttttataaa 144960
   attgaagtaa tatttagtat tattgctgcc gtcacgttgt acaaatggag atattccctg 145020
   tattcggcat ttctaaaatt agcaatttta ttgctaataa tgactgtaga tattatatag 145080
   atacagaaca tcaaaaaatt atatctgatg agatcaatag acagatggat gaaacggtac 145140
   ttcttaccaa catcttaagc gtagaagttg taaatgacaa tgagatgtac catcttattc 145200
   ctcatagatt atcgacgatt atactctgta ttagttctgt cggaggatgt gttatctcta 145260
   tagataatga catcaatggc aaaaatattc taacctttcc cattgatcat gctgtaatca 145320
   tatccccact gagtaaatgt gtcgtagtta gcaagggtcc tacaaccata ttggttgtta 145380
   aagcggatat acctagcaaa cgattggtaa catcgtttac aaacgacata ctatatgtaa 145440
   acaatctgtc actgattaat tatttgccgt tgtctgtatt cattattaga cgagtcaccg 145500
   actatttgga tagacacata tgcgatcaga tatttgctaa taataagtgg tattccctta 145560
   taaccatcga cgataagcaa tatcctattc catcaaactg tataggtatg tcctctgcca 145620
   agtacataaa ttctagcatc gagcaagata ctttaatcca tgtttgtaac ctcgagcatc 145680
   cgttcgactc agtatacaaa aaaatgcagt cgtacaattc tctacctatc aaggaacaaa 145740
   tattgtacgg tagaattgat aatataaata tgagcattag tatttctgtg gattaataga 145800
   tttctagtat ggggatcatt aatcatctct aatctctaaa tacctcataa aacgaaaaaa 145860
   aagctattat caaatactgt acggaatgga ttcattctct tctcttttta tgaaactctg 145920
   ttgtatatct actgataaaa ctggaagcaa aaaatctgat agaaagaata agaataagat 145980
   caaggattat atggaacacg attattataa aataacaata gttcctggtt cctcttccac 146040
   gtctactagc tcgtggtatt atacacatgc ctagtaatag tctctttgcg ttgacggaaa 146100
   gcagactaga aataacaggc taaaatgttc agacaccata atagttccca acccagataa 146160
   taacagagtt ccatcaacac attcctttaa actcaatccc aaacccaaaa ccgttaaaat 146220
   gtatccggcc aattgatagt agataatgag gtgtacagcg catgataatt tacacagtaa 146280
   ccaaaatgaa aatactttag taattataag aaatatagac ggtaatgtca tcatcaacaa 146340
   tccgataata tgcctgagag taaacattga cggataaaac aaaaatgctc cgcataactc 146400
   tatcatggca ataacacaac caaatacttg taagattcct aaattagtag aaaatacaac 146460
   gaatatcgat gtataagtga tctcgagaaa taataagaat aaagtaatgc ccgtaaagat 146520
   aaacatcaac attgtttggt aatcattaaa ccaattagta tgaagttgaa ctaatttcac 146580
   agtagatttt attccagtgt tatcctcgca tgtataagta cctggtaaga tatctttata 146640
   ttccataatc aatgagacat cactatctga taacgaatga agtctagcac tagtatgcca 146700
   tttacttaat attgtcgtct tggaagtttt attataagtt aaaatatcat ggttatccaa 146760
   tttccatcta atatactttg tcggattatc tatagtacac ggaataatga tggtatcatt 146820
   acatgctgta tactctatgg tctttgtagt tgttataaca accaacgtat agaggtatat 146880
   caacgatatt ctaactcttg acatttttta tttatttaaa atgatacctt tgttatttat 146940
   tttattctat tttgctaacg gtattgaatg gcataagttt gaaacgagtg aagaaataat 147000
   ttctacttac ttattagacg acgtattata cacgggtgtt aatggggcgg tatacacatt 147060
   ttcaaataat aaactaaaca aaactggttt aactaataat aattatataa caacatctat 147120
   aaaagtagag gatgcggata aggatacatt agtatgcgga accaataacg gaaatcccaa 147180
   atgttggaaa atagacggtt cagacgaccc aaaacataga ggtagaggat acgctcctta 147240
   tcaaaatagc aaagtaacga taatcagtca caacggatgt gtactatctg acataaacat 147300
   atcaaaagaa ggaattaaac gatggagaag atttgacgga ccatgtggtt atgatttatt 147360
   cacggcggat aacgtaattc caaaagatgg tttacgagga gcattcgtcg ataaagacgg 147420
   tacttatgac aaagtttaca ttcttttcac tgatactatc ggctcaaaga gaattgtcaa 147480
   aattccgtat atagcacaaa tgtgcctaaa cgacgaaggt ggtccatcat cattgtctag 147540
   tcatagatgg tcgacgtttc tcaaagtcga attagaatgt gatatcgacg gaagaagtta 147600
   tagacaaatt attcattcta gaactataaa aacagataat gatacgatac tatatgtatt 147660
   cttcgatagt ccttattcca agtccgcatt atgtacctat tctatgaata ccattaaaca 147720
   atctttttct acgtcaaaat tggaaggata tacaaagcaa ttgccgtctc cagctcctgg 147780
   tatatgttta ccagctggaa aagttgttcc acataccacg tttgaagtca tagaacaata 147840
   taatgtacta gatgatatta taaagccttt atctaaccaa cctatcttcg aaggaccgtc 147900
   tggtgttaaa tggttcgata taaaggagaa ggaaaatgaa catcgggaat atagaatata 147960
   cttcataaaa gaaaattcta tatattcgtt cgatacaaaa tctaaacaaa ctcgtagctc 148020
   gcaagtcgat gcgcgactat tttcagtaat ggtaactgcg aaaccgttat ttatagcaga 148080
   tatagggata ggagtaggaa tgccacaaat gaaaaaaata cttaaaatgt aatcttaatc 148140
   gagtacacca cacgacaatg aacaaacata agacagatta tgctggttat gcttgctgcg 148200
   taatatgcgg tctaattgtc ggaattattt ttacagcgac actattaaaa gttgtagaac 148260
   gtaaattagt tcatacacca ttaatagata aaacgataaa agatgcatat attagagaag 148320
   attgtcctac tgactggata agctataata ataaatgtat ccatttatct actgatcgaa 148380
   aaacctggga ggaaggacgt aatgcatgca aagctctaaa ttcaaattcg gatctaatta 148440
   agatagagac tccaaacgag ttaagttttt taagaagcct tagacgaggc tattgggtag 148500
   gagaatccga aatattaaac cagacaaccc catataattt tatagctaag aatgccacga 148560
   agaatggaac taaaaaacgg aaatatattt gtagcacaac gaatactccc aaactgcatt 148620
   cgtgttacac tatataacaa ttacactaca tttttatcat accactactt cggttagatg 148680
   ttttagaaaa aaataaatat cgccgtaccg ttcttgtttt tataaaaata acaattaaca 148740
   attatcaaat tttttcttta atattttacg tggttgacca ttcttggtgg taaaataatc 148800
   tcttagtgtt ggaatggaat gctgtttaat gtttccacac tcatcgtata ttttgacgta 148860
   tgtagtcaca tcgtttacgc aatagtcaga ctgtagttct atcatgcttc ctacatcaga 148920
   aggaggaaca gttttaaagt ctcttggttt taatctatta ccgttagttt tcatgaaatc 148980
   ctttgtttta tccacttcac attttaaata aatgtccact atacattctt ttgttaattt 149040
   tactagatcg tcatgggtca tagaatttat aggttccgta gtccatggat ccaaactagc 149100
   aaacttcgcg tatacggtat cgcgattagt gtatacacca actgtatgaa aattaagaaa 149160
   acagtttaat aaatcaacag aaatatttaa tcctccgttt gatacagatg cgccatattt 149220
   atggatttcg gattcacacg ttgtttgtct gaggtgttcg tctagtgttg cttctacgta 149280
   aacttcgatt cccatatatt ctttattgtc agaatcgcat accgatttat catcatacac 149340
   tgtttgaaaa ctaaatggta tacacatcaa aataataaat aataacgagt acattctgca 149400
   atattgttat cgtaattgga aaaatagtgt tcgagtgagt tggattatgt gagtattgga 149460
   ttgtatattt tattttatat tttgtaataa gaataaaatg ctaatgtcaa gtttattcca 149520
   atagatgtct tattaaaaac atatataata aataacaatg gctgaatggc ataaaattat 149580
   cgaggatatc tcaaaaaata ataagttcga ggatgccgcc atcgttgatt acaagactac 149640
   aaagaatgtt ctagctgcta ttcctaacag aacatttgcc aagattaatc cgggtgaaat 149700
   tattcctctc atcactaatc gtaatattct aaaacctctt attggtcaga aatattgtat 149760
   tgtatatact aactctctaa tggatgagaa cacgtatgct atggagttgc ttactgggta 149820
   cgcccctgta tctccgatcg ttatagcgag aactcatacc gcacttatat ttttgatggg 149880
   taagccaaca acatccagac gtgacgtgta tagaacgtgt agagatcacg ctacccgtgt 149940
   acgtgcaact ggtaattaaa ataaaaagta atattcatat gtagtgtcaa ttttaaatga 150000
   tgatgatgaa atggataata tccatattga cgatgtcaat aatgccggta ttggcataca 150060
   gttcatcgat ttttagattt cattcagagg atgtggaatt atgttatggg catttgtatt 150120
   ttgataggat ctataatgta gtaaatataa aatataatcc gcatattcca tatagatata 150180
   attttattaa tcgcacgtta accgtagatg aactagacga taatgtcttt tttacacatg 150240
   gttatttttt aaaacacaaa tatggttcac ttaatcctag tttgattgtc tcattatcag 150300
   gaaacttaaa atataatgat atacaatgct cagtaaatgt atcgtgtctc attaaaaatt 150360
   tggcaacgag tacatctact atattaacat ctaaacataa gacttattct ctacatcggt 150420
   ccacgtgtat tactataata ggatacgatt ctattatatg gtataaagat ataaatgaca 150480
   agtataatga catctatgat tttactgcaa tatgtatgct aatagcgtct acattgatag 150540
   tgaccatata cgtgtttaaa aaaataaaaa tgaactctta attatgctat gctattagaa 150600
   atggataaaa tcaaaattac ggttgattca aaaattggta atgttgttac catatcgtat 150660
   aacttggaaa agataactat tgatgtcaca cctaaaaaga aaaaagaaaa ggatgtatta 150720
   ttagcgcaat cagttgctgt cgaagaggca aaagatgtca aggtagaaga aaaaaatatt 150780
   atcgatattg aagatgacga tgatatggat gtagaaagcg cgtaatacga tctataaaaa 150840
   taagtatata aatacttttt atttactgta ctcttactgt gtagtggtga taccctactc 150900
   gattattttt ttaaaaaaaa aatacttatt ctgattcttc tagccatttc cgtgttcgtt 150960
   tgaatgccac atcgacgtca aagatagggg agtagttgaa atctagttct gcattgttgg 151020
   tacgcacctc aaatgtagtg ttggatatct tcaacgtata gttgttgagt agtgatggtt 151080
   ttctaaatag aattctcttc atatcattct tgcacgcgta catttttagc atccatcttg 151140
   gaatcctaga tccttgttct attcccaatg gtttcatcaa tagaagatta aacatatcgt 151200
   acgaacacga tggagagtaa tcgtagcaaa agtaagcatt tcctttaatc ttagatcccg 151260
   gatactggat atattttgca gccaacacgt gcatccatgc agcatttcct acatataccc 151320
   ggctatgcac cgcgtcatca tcgactgtac gatacataat gttaccgtgt tgcttacatt 151380
   gctcgtaaaa gactttcatc aatttgtctc cttctccgta aattccagtg ggtcttaggc 151440
   aacaagtata caattttgct ccattcatga ttacggaatt attggctttc ataaccagtt 151500
   gctcggccat acgtttactt tttgcgtata catgtcctgg tgatatatca taaagggtat 151560
   gctcatggcc gatgaatgga tcaccgtgtt tattgggtcc tattgcttcc atgctactag 151620
   tatagatcaa atacttgatt cctaggtcca cacaagctgc caaaatagtc tgtgttccat 151680
   aatagtttac tttcatgatt tcattatcgg tgtattttcc aaatacatcc actagagcag 151740
   ccgtatgaat aatcagattt accccatcta gcgcttctct caccttatca aagtcgttta 151800
   tatcacattg tatatagttt ataaccttaa ctttcgaggt tattggttgt ggatcttcta 151860
   caatatctat gactctgatt tcttgaacat catctgcact aattaacagt tttactatat 151920
   acctgcctag aaatccggca ccaccagtaa ccgcgtacac ggccattgct gccactcata 151980
   atatcagact acttattcta ttttactaaa taatggctgt ttgtataata gaccacgata 152040
   atatcagagg agttatttac tttgaaccag tccatggaaa agataaagtt ttaggatcag 152100
   ttattggatt aaaatccgga acgtatagtt tgataattca tcgttacgga gatattagtc 152160
   aaggatgtga ttccataggc agtccagaaa tatttatcgg taacatcttt gtaaacagat 152220
   atggtgtagc atatgtttat ttagatacag atgtaaatat atttacaatt attggaaagg 152280
   cgttatctat ttcaaaaaat gatcagagat tagcgtgtgg agttattggt atttcttaca 152340
   taaatgaaaa gataatacat tttcttacaa ttaacgagaa tggcgtttga tatatcagtt 152400
   aatgcgtcta aaacaataaa tgcattagtt tacttttcta ctcagcaaaa taaattagtc 152460
   atacgtaatg aagttaatga tacacactac actgtcgaat ttgataggga caaagtagtt 152520
   gacacgttta tttcatataa tagacataat gacaccatag agataagagg ggtgcttcca 152580
   gaggaaacta atattggttg cgcggttagt atgacttact tgtataataa gtatagtttt 152640
   aaactgattt tagcagaata tataagacac agaaatacta tatccggcaa tatttattcg 152700
   gcattgatga cactagatga tttggctatt aaacagtatg gagacattga tctattattt 152760
   aatgagaaac ttaaagtaga ctccgattcg ggactatttg actttgtcaa ctttgtaaag 152820
   gatatgatat gttgtgattc tagaatagta gtagctctat ctagtctagt atctaaacat 152880
   tgggaattga caaataaaaa atataggtgt atggcattag ccgaacatat atctgatagt 152940
   attccaatat ctgagctatc tagactacga tacaatctat gtaagtatct acgcggacac 153000
   actgagagca tagaggataa atttgattat tttgaagacg atgattcgtc tacatgttct 153060
   gccgtaaccg acagggaaac ggatgtataa ttttttttat agcgtgaagg atatgataaa 153120
   aaatataatt gttgtattta tcccattcca atcaccttat atgattctgt aacacaataa 153180
   aggagtctta tagatgtata gaggtcagat actggtttga taaactgttt attccacata 153240
   agtatgtttg actttatggt tagacccgca tactttaaca aatcactgaa aattggagtt 153300
   aggtattgac ctctcagaat cagttgccgt tctggaacat taaatgtatt ttttatgata 153360
   tactccaacg catttatgtg ggcatacaac aagtcattac taatggaata ttccaagagt 153420
   tttagttgtc tagtatttaa caagagaaga gatttcaaca gactgtttat gaactcgaat 153480
   gccgcctcat tgtcgcttat attgatgatg tcgaattctc ccaatatcat caccgatgag 153540
   tagctcatct tgttatcggg atccaagttt tctaaagatg tcattaaacc ctcgatcatg 153600
   aatggattta tcatcatcgt ttttatgttg gacatgagct tagtccgttt gtccacatct 153660
   atagaagatg atttctgaat tatttcatat atctctctct ttaactccag gaacttgtca 153720
   ggatggtcta ctttaatatg ttctcgtcta agagatgaaa atctttggat ggttgcacgc 153780
   gacttttctc taaaggatga cgttgcccaa gatcctctct taaatgaatc catcttatcc 153840
   ttggacaaga tggacagtct attttcctta gatggtttaa tatttttgtt acccatgatc 153900
   tataaaggta gacctaatcg tctcggatga ccatatattt attttcagtt ttattatacg 153960
   cataaattgt aaaaaatatg ttaggtttac gaaaatgtct cgtggggcat taatcgtttt 154020
   tgaaggattg gacaaatctg gaaaaacaac acaatgtatg aacatcatgg aatcaatacc 154080
   ttcaaacaca ataaaatacc ttaactttcc tcagagatcc actgtcactg gaaagatgat 154140
   agatgactat ctaactcgta aaaaaaccta taatgatcat atagttaatc tattattttg 154200
   tgcaaataga tgggagtttg catcttttat acaagaacaa ctagaacagg gaattacttt 154260
   aatagttgat agatacgcat tttctggagt agcgtatgcc gccgctaaag gcgcgtcaat 154320
   gactctcagt aagagttatg aatctggatt gcctaaaccc gacttagtta tattcttgga 154380
   atctggtagc aaagaaatta atagaaacgt cggtgaggaa atttatgaag atgttacatt 154440
   ccaacaaaag gtattacaag aatataaaaa aatgattgaa gaaggagata ttcattggca 154500
   aattatttct tctgaattcg aggaagatgt aaagaaggag ttgattaaga atatagttat 154560
   agaggctata cacacggtta ctggaccagt ggggcaactg tggatgtaat agtgaaatta 154620
   cattttttat aaatagatgt tagtacagtg ttataaatgg atgaagcata ttactctggc 154680
   aacttggaat cagtactcgg atacgtgtcc gatatgcata ccgaactcgc atcaatatct 154740
   caattagtta ttgccaagat agaaactata gataatgata tattaaacaa ggacattgta 154800
   aattttatca tgtgtagatc aaacttggat aatccattta tctctttcct agatactgta 154860
   tatactatta tagatcaaga gaactatcag actgagttga ttaattcatt agacgacaat 154920
   gaaattatcg attgtatagt taataagttt atgagctttt ataaggataa cctagaaaat 154980
   atagtagatg ctatcattac tctaaaatat ataatgaata atccagattt taaaactacg 155040
   tatgccgaag tactcggttc cagaatagcc gatatagata ttaaacaagt gatacgtaag 155100
   aatatactac aattgtctaa taatatccgc gaacgatatt tgtgaaaata ttaaaaaaaa 155160
   atactttttt tattaaatga cgtcttttcg tgaatttaga aaattatgct gtgctatata 155220
   tcacgcatca ggatataaag aaaaatctaa attaattaga gactttataa cagataggga 155280
   tgataaatat ttgatcatta agctattgct tcccggatta gacgatagaa tttataacat 155340
   gaacgataaa caaattataa aattatatag tataatattt aaacaatctc aggaagatat 155400
   gctacaagat ttaggatacg gatatatagg agacactatt aggactttct tcaaagagaa 155460
   cacagaaatc cgtccacgag ataaaagcat tttaacttta gaagaagtgg atagtttttt 155520
   aactacgtta tcatccgtaa ctaaagaatc gcatcaaata aaattattga ctgatgtagc 155580
   atctgtttgt acatgtaatg atttaaaatg tgtagtcatg cttattgata aagatctaaa 155640
   aattaaagcg ggtcctcggt acgtacttaa cgctattagt cctaatgcct atgatgtgtt 155700
   tagaaaatct aataacttga aagagataat agaaaattca tctaaacaaa atctagactc 155760
   tatatctatt tctgttatga ctccaattaa tcccatgtta gcggaatcgt gtgattctgt 155820
   caataaggcg tttaaaaaat ttccatcagg aatgtttgcg gaagtcaaat acgatggtga 155880
   aagagtacaa gttcataaaa ataataacga gtttgccttc tttagtagaa acatgaaacc 155940
   agtactctct cataaagtgg attatctcaa agaatacata ccgaaagcat ttaaaaaagc 156000
   tacgtctatc gtattggatt ctgaaattgt tcttgtagac gaacataatg taccgctccc 156060
   gtttggaagt ttaggtatac acaaaaagaa agaatataaa aactctaaca tgtgtttgtt 156120
   cgtgtttgac tgtttgtact ttgatggatt cgatatgacg gacattccat tgtacgaacg 156180
   aagatctttt ctcaaagatg ttatggttga aatacccaat agaatagtat tctcagagtt 156240
   gacgaatatt agtaacgagt ctcagttaac tgacgtattg gatgatgcac taacgagaaa 156300
   attagaagga ttggtcttaa aagatattaa tggagtatac gaaccgggaa agagaagatg 156360
   gttaaaaata aagcgagact atttgaacga gggttccatg gcagattctg ccgatttagt 156420
   agtactaggt gcctactatg gtaaaggagc aaagggtggt atcatggcag tctttctaat 156480
   gggttgttac gacgatgaat ccggtaaatg gaagacggtt accaagtgtt caggacacga 156540
   tgataatacg ttaagggagt tgcaagacca attaaagatg attaaaatta acaaggatcc 156600
   caaaaaaatt ccagagtggt tagtagttaa taaaatctat attcccgatt ttgtagtaga 156660
   ggatccgaaa caatctcaga tatgggaaat ttcaggagca gagtttacat cttccaagtc 156720
   ccataccgca aatggaatat ccattagatt tcctagattt actaggataa gagaggataa 156780
   aacgtggaaa gaatctactc atctaaacga tttagtaaac ttgactaaat cttaatagtt 156840
   acatacaaac tgaaaattaa aataacacta tttagttggt ggtcgccatg gatggtgtta 156900
   ttgtatactg tctaaacgcg ctagtaaaac atggcgagga aataaatcat ataaaaaatg 156960
   atttcatgat taaaccatgt tgtgaaagag tttgtgaaaa agtcaagaac gttcacattg 157020
   gcggacaatc taaaaacaat acagtgattg cagatttgcc atatatggat aatgcggtat 157080
   ccgatgtatg caattcactg tataaaaaga atgtatcaag aatatccaga tttgctaatt 157140
   tgataaagat agatgacgat gacaagactc ctactggtgt atataattat tttaaaccta 157200
   aagatgccat tcctgttatc atatctatag gaaaggataa agatgtctgt gaactattaa 157260
   tctcatcaga catatcgtgt gcatgcgtgg agttaaattc atatcacgta gccattcttc 157320
   ccatggatgt ttcctttttt accaaaggaa atgcatcatt gattattctc ctgtttgatt 157380
   tctctatcga tgcggcacct ctcttaagaa gtgtaaccga taataatgtt attatatcta 157440
   gacaccagcg tctacatgac gagcttccga gttccaattg gttcaagttt tacataagta 157500
   taaagtccga ctattgttct atattatata tggttgttga tggatctgtg atgcatgcga 157560
   tagctgataa tagaactcac gcaattatta gcaaaaatat attagacaat actacgatta 157620
   acgatgagtg tagatgctgt tattttgaac cacagattag gattcttgat agagatgaga 157680
   tgctcaatgg atcatcgtgt gatatgaaca gacattgtat tatgatgaat ttacctgatg 157740
   taggcgaatt tggatctagt atgttgggga aatatgaacc tgacatgatt aagattgctc 157800
   tttcggtggc tggtaattta ataagaaatc gagactacat tcccgggaga cgaggatata 157860
   gctactacgt ttacggtata gcctctagat aattttttta agcacgaaat aaaaaacata 157920
   attttaaacc aatctatttc atactatttt gtgtgatcac catggacata aagatagata 157980
   ttagtatttc tggtgataaa tttacggtga ctactaggag ggaaaatgaa gaaagaaaaa 158040
   aatatctacc tctccaaaaa gaaaaaacta ctgatgttat caaacctgat tatcttgagt 158100
   acgatgactt gttagataga gatgagatgt ttactattct agaggaatat tttatgtaca 158160
   gaggtctatt aggcctcaga ataaaatatg gacgactctt taacgaaatt aaaaaattcg 158220
   acaatgatgc ggaagaacaa ttcggtacta tagaagaact caagcagaaa cttagattaa 158280
   attctgaaga gggagcagat aactttatag attatataaa ggtacaaaaa caggatatcg 158340
   tcaaacttac tgtatacgat tgcatatcta tgataggatt gtgtgcatgc gtggtagatg 158400
   tttggagaaa tgagaaactg ttttctagat ggaaatattg tttacgagcg attaaactgt 158460
   ttattgatga tcacatgctt gataagataa aatctatact gcagaataga ctagtatatg 158520
   tggaaatgtc atagaaagtt aaaagttaat gagagcaaaa atatataagg ttgtattcca 158580
   tatttgttat ttttttctgt aatagttaga aaaatacatt cgatggtcta tctatcagat 158640
   tattatgtgt tataaggtac tttttctcat aataaactag agtatgagta agatagtgtt 158700
   tttcaaaaca tataaatcta aaattgatgg atgagatata cagctattaa tttcgaaaat 158760
   atattttaat ctgataactt taaacatgga tttttaatgg tggtttaacg ttttaaaaaa 158820
   agattttgtt attgtagtat atgataatat taaaagatgg atataaagaa tttgctgact 158880
   gcatgtacta ttttttacat tactacattg gctacggcag atatacctac ttcgtcactg 158940
   ccacacgctc cggtaaacgg ggcatgtgac gagggagaat atcttgataa gaggcataat 159000
   caatgttgta atcagtgtcc acctggagaa tttgccaagg ttagatgtaa tggtaacgat 159060
   aacacaaaat gtgaacgctg cccacctcat acatataccg caatccccaa ttactctaat 159120
   ggatgtcatc aatgtagaaa atgcccaaca ggatcatttg ataaggtaaa gtgtaccgga 159180
   acacagaaca gtaaatgttc gtgtcttcct ggttggtatt gcgctactga ttcttcacag 159240
   actgaagatt gtcgagactg tataccaaaa aggagatgtc catgcggata ctttggtgga 159300
   atagatgaac aaggaaatcc tatttgtaaa tcgtgttgtg ttggtgaata ttgcgactac 159360
   ctacgtaatt atagacttga tccatttcct ccatgcaaac tatctaaatg taattaatta 159420
   tgattttgat gataatgtta ccatacatta tatcgctact tggttagtgt attattcagt 159480
   atgaagacct attaataatt acttatcttt tgacgatctt gttataatta taatataaaa 159540
   atacttatgg catagtaact cataattgct gacgcgataa attcgtaata atctgttttg 159600
   ttcaaatttt tataaggaat ctacaggcat aaaaataaaa atataattta taatatactc 159660
   ttacagcgcg ccatcatgaa taacagcagt gaattgattg ctgttattaa tggatttaga 159720
   aatagtggac gattttgtga tattaatata gttattaatg atgaaaggat aaacgctcac 159780
   agactcatcc tatctggagc ctccgaatat ttttccattc tgttttccaa taattttatc 159840
   gattctaatg aatacgaagt taatctaagt catttagatt atcaaagtgt taacgatttg 159900
   atcgattaca tttatgggat acctttgagc ctaactaacg ataacgtgaa atatattctt 159960
   tcaaccgctg attttttaca aattggatct gctattacgg agtgtgaaaa ttacatactt 160020
   aaaaatcttt gttctagaaa ctgtatcgat ttctacatat acgctgataa atataataac 160080
   aagaaaatag aatcagcgtc gtttaacaca atattacaaa atattttgag actcatcaac 160140
   gatgaaaact ttaaatactt aacagaggaa tcaatgataa aaattttaag cgatgatatg 160200
   ttaaatataa aaaatgagga tttcgcccca ctaattctca ttaaatggtt agagagtact 160260
   caacaaccat gcaccgtcga gttacttaaa tgcctcagaa tatcattgct ttccccacaa 160320
   gttataaaat cactttatag tcatcgactg gttagttcaa tctacgaatg tataacattc 160380
   ttaaacaata tagcattctt ggatgaatca tttcctagat accatagcat cgagttgata 160440
   tctatcggta taagtaattc gcatgataag atttccataa actgctacaa tcataaaaaa 160500
   aatacatggg aaatgatatc ttcacgtaga tataggtgta gtttcgcagt ggccgtcctg 160560
   gataatatta tctatatgat gggtggatat gatcagtccc cgtatagaag ttcaaaggtt 160620
   atagcgtaca atacatgtac aaattcttgg atatatgata taccagagct aaaatatcct 160680
   cgttctaatt gtgggggact ggctgatgac gaatacattt attgtatagg cggcatacgc 160740
   gatcaggatt catcgttgac atctagtatt gatagatgga agccatcaaa accatattgg 160800
   cagaagtatg ctaaaatgcg cgaaccaaaa tgtgatatgg gggttgcgat gttaaacgga 160860
   ttaatatatg ttataggtgg aatcgttaaa ggtgacacgt gtaccgacgc actagagagt 160920
   ttatcagaag atggatggat gaagcatcaa cgtcttccaa taaaaatgtc caatatgtcg 160980
   acgattgttc atgatggcaa gatttatata tctggaggtt acaacaatag tagtgtagtt 161040
   aatgtaatat cgaatctagt ccttagctat aattcgatat atgatgaatg gaccaaatta 161100
   tcatcattaa acattcctag aattaatccc gctctatggt cagcgcataa taaattatat 161160
   gtaggaggag gaatatctga tgatgttcga actaatacat ctgaaacata cgacaaagaa 161220
   aaagattgtt ggacattgga taatggtcac gtgttaccac gcaattatat aatgtataaa 161280
   tgcgaaccga ttaaacataa atatccattg gaaaaaacac agtacacgaa tgattttcta 161340
   aagtatttgg aaagttttat aggtagttga tagaacaaaa tacataattt tgtaaaaata 161400
   aatcactttt tatactaata tgacacgatt accaatactt ttgttactaa tatcattagt 161460
   atacgctaca ccttttcctc agacatctaa aaaaataggt gatgatgcaa ctctatcatg 161520
   taatcgaaat aatacaaatg actacgttgt tatgagtgct tggtataagg agcccaattc 161580
   cattattctt ttagctgcta aaagcgacgt cttgtatttt gataattata ccaaggataa 161640
   aatatcttac gactctccat acgatgatct agttacaact atcacaatta aatcattgac 161700
   tgctagagat gccggtactt atgtatgtgc attctttatg acatcgccta caaatgacac 161760
   tgataaagta gattatgaag aatactccac agagttgatt gtaaatacag atagtgaatc 161820
   gactatagac ataatactat ctggatctac acattcaccg gaaactagtt ctgagaaacc 161880
   tgattatata gataattcta attgctcgtc ggtattcgaa atcgcgactc cggaaccaat 161940
   tactgataat gtagaagatc atacagacac cgtcacatac actagtgata gcattaatac 162000
   agtaagtgca tcatctggag aatccacaac agacgagact ccggaaccaa ttactgataa 162060
   agaagaagat catacagtta cagacactgt ctcatacact acagtaagta catcatctgg 162120
   aattgtcact actaaatcaa ccaccgatga tacgtacaat gataatgata cagtaccacc 162180
   aactactgta ggcggtagta caacctctat tagcaattat aaaaccaagg actttgtaga 162240
   aatatttggt attaccgcat taattatatt gtcggccgtg gcaatattct gtattacgta 162300
   ttatatatgt aataaacgtt cacgtaaata caaaacagag aacaaagtct agatttttga 162360
   cttacataaa tgtctgggat agtaaaatct atcatattga gcgggccatc tggtttagga 162420
   aagacagcca tagccaaaag actatgggaa tatatttgga tttgtggtgt cccataccac 162480
   tagatttcct cgtcctatgg aacgagaagg tgtcgattac cattacgtta acagagaggc 162540
   catctggaag ggaatagccg ccggaaactt tctagaacat actgagtttt taggaaatat 162600
   ttacggaact tctaaaactg ctgtgaatac agcggctatt aataatcgta tttgtgtgat 162660
   ggatctaaac atcgatggcg ttagaagtct taaaaatacg tacctaatgc cttactcggt 162720
   gtatataaga cctacctctc ttaaaatggt tgagaccaag cttcgttgta gaaacactga 162780
   agcggatgat gagattcatc gtcgtgtgat gttggcaaaa actgacatgg atgaggcagg 162840
   tgaagccggt ctattcgaca ctattatcat tgaagatgat gtgaatttag catatagtaa 162900
   gttaattcag atactacagg accgtattag aatgtatttt aacactaatt agagacttaa 162960
   gacttaaaac ttgataatta ataatataac tcgtttttat atgtgtctat ttcaacgtct 163020
   aatgtattag ttaaatatta aaacttacca cgtaaaactt aaaatttaaa atgatatttc 163080
   attgacagat agatcacaca ttatgaactt tcaaggactt gtgttaactg acaattgcaa 163140
   aaatcaatgg gtcgttggac cattaatagg aaaaggtgga ttcggtagta tttatactac 163200
   taatgacaat aattatgtag taaaaataga gcccaaagct aacggatcat tatttaccga 163260
   acaggcattt tatactagag tacttaaacc atccgttatc gaagaatgga aaaaatctca 163320
   caatataaag cacgtaggtc ttatcacgtg caaggcattt ggtctataca aatccattaa 163380
   tgtggaatat cgattcttgg taattaatag attaggtgca gatctagatg cggtgatcag 163440
   agccaataat aatagactac caaaaaggtc ggtgatgttg atcggaatcg aaatcttaaa 163500
   taccatacaa tttatgcacg agcaaggata ttctcacgga gatattaaag cgagtaatat 163560
   agtcttagat caaatagata agaataaatt atatctagtg gattacggat tggtttctaa 163620
   attcatgtct aatggcgaac atgttccatt tataagaaat ccaaataaaa tggataacgg 163680
   tactctagaa tttacaccta tagattcgca taaaggatac gttgtatcta gacgtggaga 163740
   tctagaaaca cttggatatt gtatgattag atggttggga ggtatcttac catggactaa 163800
   gatatctgaa acaaagaatt gtgcattagt aagtgccaca aaacagaaat atgttaacaa 163860
   tactgcgact ttgttaatga ccagtttgca atatgcacct agagaattgc tgcaatatat 163920
   taccatggta aactctttga catattttga ggaacccaat tacgacaagt ttcggcacat 163980
   attaatgcag ggtgtatatt attaagtgtg gtgtttggtc gataaaaatt aaaaaataac 164040
   ttaatttatt attgatctcg tgtgtacaac cgaaatcatg gcgatgtttt acgcacacgc 164100
   tctcggtggg tacgacgaga atcttcatgc ctttcctgga atatcatcga ctgttgccaa 164160
   tgatgtcagg aaatattctg ttgtgtcagt ttataataac aagtatgaca ttgtaaaaga 164220
   caaatatatg tggtgttaca gtcaggtgaa caagagatat attggagcac tgctgcctat 164280
   gtttgagtgc aatgaatatc tacaaattgg aaatccgatc catgatcaag aaggaaatca 164340
   aatctctatc atcacatatc gccacaaaaa ctactatgct ctaagcggaa tcgggtacga 164400
   gagtctagac ttgtgtttgg aaggagtagg gattcatcat cacgtacttg aaacaggaaa 164460
   cgctgtatat ggaaaagttc aacatgatta ttctactatc aaagagaagg ccaaagaaat 164520
   gagtgcactt agtccaggac ctatcatcga ttaccacgtc tggataggag attgtatctg 164580
   tcaagttact gctgtggacg tacatggaaa ggaaattatg aaaatgagat tcaaaaaggg 164640
   tgcggtgctt ccgatcccaa atctggtaaa agttaaactt ggggagaatg atacagaaaa 164700
   tctttcttct actatatcgg cggcaccatc gaggtaacca cctctctgga agacagcgtg 164760
   aatcatgtac tcatgaaacg tttggaaact atacgccata tgtggtctgt tgtatatgat 164820
   cattttgata ttgtgaatgg taaagaatgc tgttatgtgc atacgcattc atctaatcaa 164880
   aatcctatac cgagtactgt aaaaacaaat ttgtacatga agactatggg atcatgcatt 164940
   caaatggatt ccatggaagc tctagagtat cttagcgaac tgaaggaatc aggtggatgg 165000
   agtcccagac cagaaatgca ggaatttgaa tatccagatg gagtggaaga cactgaatca 165060
   attgagagat tggcagagga gttcttcaat agatcagaac ttcaggctgg tgaatcagtc 165120
   aaatttggta attctattaa ttgttaaaca tacatctgtt tcagctaagc aactaagaac 165180
   acgtatacgg cagcagcttc cttctatact ctcatctttt accaacacaa agggtggata 165240
   tttgttcatt ggagttgata ataatacaca caaagtattt ggattcacgg tgggttacga 165300
   ctacctcaga ctgatagaga atgatataga aaagcatatc aaaagacttc gtgttgtgca 165360
   tttctgtgag aagaaagagg acatcaagta cgcgtgtcga ttcatcaagg tatataaacc 165420
   tggggatgag actacctcga catacgtgtg cgctatcaaa gtggaaagat gctgttgtgc 165480
   tgtgtttgca gattggccag aatcatggta tatggatact aatggtatca agaagtattc 165540
   tccagatgaa tgggtgtcac atataaaatt ttaattaatg taactataga gaacaaataa 165600
   taaggttgta atatcatata gacaataact aacaattaat tagtaactgt tatctctttt 165660
   ttaactaacc aactaactat atacctatta atacatcgta attatagttc ttaacatcta 165720
   ttaatcatta attcgcttct ttaatttttt ataaactaac attgttaatt gaaaagggat 165780
   aacatgttac agaatataaa ttatatatgg atttttttaa aaaggaaata cttgactgga 165840
   gtatatattt atctcttcat tatatagcac gcgtgttttc caatttttcc acatcccata 165900
   taatacagga ttataatctc gttcgaacat acgagaaagt ggataaaaca atagttgatt 165960
   ttttatctag gttgccaaat ttattccata ttttagaata tggggaaaat attctacata 166020
   tttattctat ggatgatgct aatacgaata ttataatttt ttttctagat agagtattaa 166080
   atattaataa gaacgggtca tttatacaca atctcgggtt atcatcatcc attaatataa 166140
   aagaatatgt atatcaatta gttaataatg atcatccaga taataggata agactaatgc 166200
   ttgaaaatgg acgtagaaca agacattttt tgtcctatat atcagataca gttaatatct 166260
   atatatgtat tttaataaat catggatttt atatagatgc cgaagacagt tacggttgta 166320
   cattattaca tagatgtata tatcactata agaaatcaga atcagaatca tacaatgaat 166380
   taattaagat attgttaaat aatggatcag atgtagataa aaaagatacg tacggaaaca 166440
   caccttttat cctattatgt aaacacgata tcaacaacgt ggaattgttt gagatatgtt 166500
   tagagaatgc taatatagac tctgtagact ttaatagata tacacctctt cattatgtct 166560
   catgtcgtaa taaatatgat tttgtaaagt tattaatttc taaaggagca aatgttaatg 166620
   cgcgtaataa attcggaact actccatttt attgtggaat tatacacggt atctcgctta 166680
   taaaactata tttggaatca gacacagagt tagaaataga taatgaacat atagttcgtc 166740
   atttaataat ttttgatgct gttgaatctt tagattatct attatccaga ggagttattg 166800
   atattaacta tcgtactata tacaacgaaa catctattta cgacgctgtc agttataatg 166860
   cgtataatac gttggtctat ctattaaaca aaaatggtga ttttgagacg attactacta 166920
   gtggatgtac atgtatttcg gaagcagtcg caaacaacaa caaaataata atggaagtac 166980
   tattgtctaa acgaccatct ttgaaaatta tgatacagtc tatgatagca attactaaac 167040
   ataaacagca taatgcagat ttattgaaaa tgtgtataaa atatactgcg tgtatgaccg 167100
   attatgatac tcttatagat gtacagtcgc tacagcaata taaatggtat attttaagat 167160
   gtttcgatga aatagatatc atgaagagat gttatataaa aaataaaact gtattccaat 167220
   tagttttttg tatcaaagac attaatactt taatgagata cggtaaacat ccttctttcg 167280
   tgaaatgcac tagtctcgac gtatacggaa gtcgtgtacg taatatcata gcatctatta 167340
   gatatcgtca gagattaatt agtctattat ccaagaagct ggatcctgga gataaatggt 167400
   cgtgttttcc taacgaaata aaatataaaa tattggaaaa ctttaacgat aacgaactat 167460
   ccacatatct aaaaatctta taaacattat taaaatataa aatctaagta ggataaaatc 167520
   acactacatc attgtttcct tttagtgctc gacagtgtat actattttta acgctcataa 167580
   ataaaaatga aaacgatttc cgttgttacg ttgttatgcg tactacctgc tgttgtttat 167640
   tcaacatgta ctgtacccac tatgaataac gctaaattaa cgtctaccga aacatcgttt 167700
   aatgataacc agaaagttac gtttacatgt gatcagggat atcattcttt ggatccaaat 167760
   gctgtctgtg aaacagataa atggaaatac gaaaatccat gcaaaaaaat gtgcacagtt 167820
   tctgattatg tctctgaact atataataaa ccgctatacg aagtgaattc caccatgaca 167880
   ctaagttgca acggcgaaac aaaatatttt cgttgcgaag aaaaaaatgg aaatacttct 167940
   tggaatgata ctgttacgtg tcctaatgcg gaatgtcaac ctcttcaatt agaacacgga 168000
   tcgtgtcaac cagttaaaga aaaatactca tttggggaat atatgactat caactgtgat 168060
   gttggatatg aggttattgg tgcttcgtac ataagttgta cagctaattc ttggaatgtt 168120
   attccatcat gtcaacaaaa atgtgatatg ccgtctctat ctaacggatt aatttccgga 168180
   tctacatttt ctatcggtgg cgttatacat cttagttgta aaagtggttt tacactaacg 168240
   gggtctccat catccacatg tatcgacggt aaatggaatc ccatactccc aatatgtgta 168300
   cgaactaacg aaaaatttga tccagtggat gatggtcccg acgatgagac agatttgagc 168360
   aaactctcga aagacgttgt acaatatgaa caagaaatag aatcgttaga agcaacttat 168420
   catataatca tagtggcgtt gacaattatg ggcgtcatat ttttaatctc cgttatagta 168480
   ttagtttgtt cctgtgacaa aaataatgac caatataagt tccataaatt gctaccgtaa 168540
   atataaatcc gttaaaataa ttaataattt aataacaaac aagtatcaaa agattaaaga 168600
   cttatagcta gaatcaattg agatgtcttc ttcagtggat gttgatatct acgatgccgt 168660
   tagagcattt ttactcaggc actattataa caagagattt attgtgtatg gaagaagtaa 168720
   cgccatatta cataatatat acaggctatt tacaagatgc gccgttatac cgttcgatga 168780
   tatagtacgt actatgccaa atgaatcacg tgttaaacaa tgggtgatgg atacacttaa 168840
   tggtataatg atgaatgaac gcgatgtttc tgtaagcgtt ggcaccggaa tactattcat 168900
   ggaaatgttt ttcgattaca ataaaaatag tatcaacaat caactaatgt atgatataat 168960
   taatagcgta tctataattc tagctaatga gagatataga agcgctttta acgacgatgg 169020
   tatatacatc cgtagaaata tgattaacaa gttgtacgga tacgcatctc taactactat 169080
   tggcacgatc gctggaggtg tttgttatta tctgttgatg catctagtta gtttgtataa 169140
   ataattattt caatatacta gttaaaattt taagatttta aatgtataaa aaactaataa 169200
   cgtttttatt tgtaataggt gcattagcat cctattcgaa taatgagtac actccgttta 169260
   ataaactgag tgtaaaactc tatatagatg gagtagataa tatagaaaat tcatatactg 169320
   atgataataa tgaattggtg ttaaatttta aagagtacac aatttctatt attacagagt 169380
   catgcgacgt cggatttgat tccatagata tagatgttat aaacgactat aaaattattg 169440
   atatgtatac cattgactcg tctactattc aacgcagagg tcacacgtgt agaatatcta 169500
   ccaaattatc atgccattat gataagtacc cttatattca caaatatgat ggtgatgagc 169560
   gacaatattc tattactgca gagggaaaat gctataaagg aataaaatat gaaataagta 169620
   tgatcaacga tgatactcta ttgagaaaac atactcttaa aattggatct acttatatat 169680
   ttgatcgtca tggacatagt aatacatatt attcaaaata tgatttttaa aaatttaaaa 169740
   tatattatca cttcagtgac agtagtcaaa taacaaacaa caccatgaga tatattataa 169800
   ttctcgcagt tttgttcatt aatagtatac atgctaaaat aactagttat aagtttgaat 169860
   ccgtcaattt tgattccaaa attgaatgga ctggggatgg tctatacaat atatccctta 169920
   aaaattatgg catcaagacg tggcaaacaa tgtatacaaa tgtaccagaa ggaacatacg 169980
   acatatccgc atttccaaag aatgatttcg tatctttctg ggttaaattt gaacaaggcg 170040
   attataaagt ggaagagtat tgtacgggac tatgcgtcga agtaaaaatt ggaccaccga 170100
   ctgtaacatt gactgaatac gacgaccata tcaatttgta catcgagcat ccgtatgcta 170160
   ctagaggtag caaaaagatt cctatttaca aacgcggtga catgtgtgat atctacttgt 170220
   tgtatacggc taacttcaca ttcggagatt ctaaagaacc agtaccatat gatatcgatg 170280
   actacgattg cacgtctaca ggttgcagca tagactttgt cacaacagaa aaagtgtgcg 170340
   tgacagcaca gggagccaca gaagggtttc tcgaaaaaat tactccatgg agttcgaaag 170400
   tatgtctgac acctaaaaag agtgtatata catgcgcaat tagatccaaa gaagatgttc 170460
   ccaatttcaa ggacaaaatg gccagagtta tcaagagaaa atttaataca cagtctcaat 170520
   cttatttaac taaatttctc ggtagcacat caaatgatgt taccactttt cttagcatgc 170580
   ttaacttgac taaatattca taactaattt ttattaatga tacaaaaacg aaataaaact 170640
   gcatattata cactggttaa cgcccttata ggctctaacc attttcaaga tgaggtccct 170700
   gattatagtc cttctgttcc cctctatcat ctactccatg tctattagac gatgtgagaa 170760
   gactgaagag gaaacatggg gattgaaaat agggttgtgt ataattgcca aagatttcta 170820
   tcccgaaaga actgattgca gtgttcatct cccaactgca agtgaaggat tgataactga 170880
   aggcaatgga ttcagggata tacgaaacac cgataaatta taaaaaaagc aatgtgtccg 170940
   ctgtttccgt taataatact attttcgtaa ctggcggatt attcataaat aactctaata 171000
   gcacgatcgt ggttaacaat atggaaaaac ttgacattta taaagacaaa caatggtcga 171060
   ttatagaaat gcctatggct agggtatatc acggcattga ctcgacattt ggaatgttat 171120
   attttgccgg aggtctatcc gttaccgaac aatatggtaa tttagagaaa aacaacgaga 171180
   tatcttgtta caatcctaga acgaataagt ggtttgatat ttcatatact atttataaga 171240
   tatccatatc atcattgtgt aaactaaata acgtcttcta tgtatttagt aaggacattg 171300
   gatatgtgga aaagtatgat ggtgcatgga agttagtaca tgatcgtctc cccgctataa 171360
   aggcattatc aacttctcct tattgattga aaatgaaaat ataaatagtt tttatgtata 171420
   gcagtattac cctatagttt tattgcttac tactaacatg gatacagatg ttacaaatgt 171480
   agaagatatc ataaatgaaa tagatagaga gaaagaagaa atactaaaaa atgtagaaat 171540
   tgaaaataat aaaaacatta acaagaatca tcccaatgaa tatattagag aagcactcgt 171600
   tattaatacc agtagtaata gtgattccat tgataaagaa gttatagaat gtatcagtca 171660
   cgatgtagga atatagatca tatctactaa tttttataat cgatacaaaa cataaaaaac 171720
   aactcgttat tacatagcag gcatggaatc cttcaagtat tgttttgata acgatggcaa 171780
   gaaatggatt atcggaaata ctttatattc tggtaattca atactctata aggtcagaaa 171840
   aaatttcact agttcgttct acaattacgt aatgaagata gatcacaaat cacacaagcc 171900
   attgttgtct gaaatacgat tctatatatc tgtattggat cctttgacta tcgacaactg 171960
   gacacgggaa cgtggtataa agtatttggc tattccagat ctgtatggaa ttggagaaac 172020
   cgatgattat atgttcttcg ttataaagaa ttcgggaaga gtattcgccc caaaggatac 172080
   tgaatcagtc ttcgaagcat gcgtcactat gataaacacg ttagagttta tacactctcg 172140
   aggatttacc catggaaaaa tagaaccgag gaatatactg attagaaata aacgtctttc 172200
   actaattgac tattctagaa ctaacaaact atacaagagt ggaaactcac atatagatta 172260
   caacgaggac atgataactt caggaaatat caattatatg tgtgtagaca atcatcttgg 172320
   agcaacagtt tcaagacgag gagatttaga aatgttggga tattgcatga tagaatggtt 172380
   cggtggcaaa cttccatgga aaaacgaaag tagtataaaa gtaataaaac aaaaaaaaga 172440
   atataaaaaa tttatagcta ctttctttga ggactgtttt cctgaaggaa atgaacctct 172500
   ggaattagtt agatatatag aattagtata cacgttagat tattctcaaa ctcctaatta 172560
   tgacagacta cgtaaactgt ttatacaaga ttgaaattat attctttttt ttatagagtg 172620
   tggtagtgtt acggatatct aatattaata ttagactatc tctatcgcgc tacacgacca 172680
   atatcgatta ctatggatat cttctatgaa aggagagaat gtatttattt ctccagcgtc 172740
   aatctcgtca gtattgacaa tactgtatta tggagctaat ggatccactg ctgaacagct 172800
   atcaaaatat gtagaaacgg aggagaacac ggataaggtt agcgctcaga atatctcatt 172860
   caaatccatg aataaagtat atgggcgata ttctgccgtg tttaaagatt cctttttgag 172920
   aaaaattggc gataagtttc aaactgttga cttcactgat tgtcgcacta tagatgcaat 172980
   caacaagtgt gtagatatct ttactgaggg gaaaatcaat ccactattgg atgaaccatt 173040
   gtctcctagc aattagtgcc gtatacttta aagcaaaatg gttgacgcca ttcgaaaagg 173100
   aatttaccag tgattatccc ttttacgtat ctccgacgga aatggtagac gtaagtatga 173160
   tgtctatgta cggcaaggca tttaatcacg catctgtaaa agaatcattc ggcaactttt 173220
   caatcataga actgccatat gttggagata ctagtatgat ggtcattctt ccagacaaga 173280
   ttgatggatt agaatccata gaacaaaatc taacagatac aaattttaag aaatggtgtg 173340
   actttatgga tgctatgttt atagatgttc acattcccaa gtttaaggta acaggctcgt 173400
   ataatctggt ggatactcta gtaaagtcag gactgacaga ggtgttcggt tcaactggag 173460
   attatagcaa tatgtgtaat ttagatgtga gtgtcgacgc tatgatccac aaaacgtata 173520
   tagatgtcaa tgaagagtat acagaagcag ctgcagcaac ttctgtacta gtggcagact 173580
   gtgcatcaac aattacaaat gagttctgtg cagatcatcc gttcatctat gtgattaggc 173640
   atgttgatgg aaaaattctt ttcgttggta gatattgctc tccgacaact aattgttaac 173700
   catttttttt aaaaaaaata gaaaaaacat gtggtattag tgcaggtcgt tgttcttcca 173760
   attgcaattg gtaagatgac ggccaacttt agtacccacg tcttttcacc acagcactgt 173820
   ggatgtgaca gactgaccag tattgatgac gtcaaacaat gtttgactga atatatttat 173880
   tggtcgtcct atgcataccg caacaggcaa tgcgctggac aattgtattc cacactcctc 173940
   tcttttagag atgatgcgga attagtgttc atcgacattc gcgagctggt aaaaaatatg 174000
   ccgtgggatg atgtcaaaga ttgtacagaa atcatccgtt gttatatacc ggatgagcaa 174060
   aaaaccatca gagagatttc ggccatcatc ggactttgtg catatgctgc tacttactgg 174120
   ggaggtgaag accatcccac tagtaacagt ctgaacgcat tgtttgtgat gcttgagatg 174180
   ctaaattacg tggattataa catcatattc cggcgtatga attgatgagt tgtacatctt 174240
   gacattttct ttcttctctt ctccctttct tctcttctcc cttcctccct cttctccctt 174300
   tcccagaaac aaactttttt acccactata aaataaaatg agtatactac ctattatatt 174360
   tcttcctata tttttttatt cttcattcgt tcagactttt aacgcgtctg aatgtatcga 174420
   caaagggcaa tattttgcat cattcatgga gttagaaaac gagccagtaa tcttaccatg 174480
   tcctcaaata aatacgctat catccggata taatatatta gatattttat gggaaaaacg 174540
   aggagcggat aatgatagaa ttataccgat agataatggt agcaatatgc taattctgaa 174600
   cccgacacaa tcagactctg gtatttatat atgcattacc acgaacgaaa cctactgtga 174660
   catgatgtcg ttaaatttga caatcgtgtc tgtctcagaa tcaaatatag atcttatctc 174720
   gtatccacaa atagtaaatg agagatctac tggcgaaatg gtatgtccca atattaatgc 174780
   atttattgct agtaacgtaa acgcagatat tatatggagc ggacatcgac gccttagaaa 174840
   taagagactt aaacaacgga cacctggaat tattaccata gaagatgtta gaaaaaatga 174900
   tgctggttat tatacatgtg ttttagaata tatatacggt ggcaaaacat ataacgtaac 174960
   cagaattgta aaattagagg tacgggatag aataatacct tctactatgc aattaccaga 175020
   tggcattgta acttcaatag gtagtaattt gactattgca tgcagagtat cgttgagacc 175080
   tcccacaacg gacaccgacg tcttttggat aagtaatggt atgtattacg aagaagatga 175140
   tggggacgga aacggtagaa taagtgtagc aaataaaatc tatatgaccg ataagagacg 175200
   tgttattaca tcccggttaa acattaatcc tgtcaaggaa gaagatgcta caacgtttac 175260
   gtgtatggcg tttactattc ctagcatcag caaaacagtt actgttagta taacgtgaat 175320
   gtatgttgtt acatttccat gtcaattgag tttataagaa tttttataca ttatcttcca 175380
   acaaacaatt gacgaacgta ttgctatgat taactcccac gatactatgc atattattaa 175440
   tcattaactt gcagactata cctagtgcta ttttgacata ctcgtgttct tgtgtaattg 175500
   cggtatctat attattaaag tacgtaaatc tagctatagt tttattattt aattttagat 175560
   aatataccgt ctccttattt ttaaaaattg ccacatcctt tattaaatca tgaatgggaa 175620
   tttctatgtc atcgttagta tattgtgaac aacaagagca gatatctata ggaaagggtg 175680
   gaatgcgata cattgatcta tgtagtttta aaacacacgc gaactttgaa gaatttatat 175740
   aaatcattcc atcgatacat ccttctatgt tgacatgtat atatccagga attcttttat 175800
   taatgtcagg aaatgtataa actaaaacat tgcccgaaag cggtgcctct atctgcgtta 175860
   tatccgttct taacttacaa aatgtaacca atacctttgc atgacttgtt ttgttcggca 175920
   acgttagttt aaacttgacg aatggattaa ttacaatagc atgatccgcg catctattaa 175980
   gtttttttac tttaacgccc ttgtatgttt ttacagagac tttatctaaa tttctagtgc 176040
   ttgtatgtgt tataaatata acgggatata gaaccgaatc acctacctta gatacccaat 176100
   tacattttat cagatccaga taataaacaa attttgtcgc cctaactaat tctatattgt 176160
   tatatatttt acaattggtt atgatatcat gtaataactt ggagtctaac gcgcatcgtc 176220
   gtacgtttat acaattgtga tttagtgtag tatatctaca catgtatttt tccgcactat 176280
   agtattctgg actagtgata aaactatcgt tatatctgtc ttcaatgaac tcatcgagat 176340
   attgctctct gtcatattca tacacctgca taaactttct agacatctta caatccgtgt 176400
   tattttagga tcatatttac atatttacgg gtatatcaaa gatgttagat tagttaatgg 176460
   gaatcgtcta taataatgaa tattaaacaa ttatatgagg acttttacca caaagcatca 176520
   taaaaatgag tcgtcgtctg atttatgttt taaatatcaa ccgcaaatca actcataaaa 176580
   tacaagagaa tgaaatatat acatatttta gtcattgcaa tatagaccat acttctacag 176640
   aacttgattt tgtagttaaa aactatgatc taaacagacg acaacctgta actgggtata 176700
   ctgcactaca ctgctatttg tataataatt actttacaaa cgatgtactg aagatattat 176760
   taaatcatga cgtaaatgta acgatgaaaa ccagtagcgg acgtatgcct gtttatatat 176820
   tgcttactag atgttgtaat atttcacatg atgtagtgat agatatgata gacaaagata 176880
   aaaaccactt attacataga gactattcca acctattact agagtatata aaatctcgtt 176940
   acatgttatt gaaggaagag gatatcgatg agaacatagt atccacttta ttagataagg 177000
   gaatcgatcc taactttaaa caagacggat atacagcgtt acattattat tatttgtgtc 177060
   tcgcacacgt ttataaacta ggtgagtgta gaaaaccgat aacgataaaa aaggccaagc 177120
   gaattatttc tttgtttata caacatggag ctaatctaaa cgcgttagat aattgtggta 177180
   atacaccatt ccatttgtat cttagtattg aaatgtgtaa taatattcat atgactaaaa 177240
   tgctgttgac ttttaatccg aatttcaaaa tatgtaataa tcatggatta acgcctatac 177300
   tatgttatat aacttccgac tacatacaac acgatattct tgttatgtta atacatcact 177360
   atgaaacaaa tgttggagaa atgccgatag atgagcgtcg tatgatcgta ttcgagttta 177420
   tcaaaacata ttctacacgt ccggcagatt cgataactta tttgatgaat aggtttaaaa 177480
   atataaatat ttatacccgc tatgaaggaa agacattatt acacgtagca tgtgaatata 177540
   ataatacaca agtaatagat tatcttatac gtatcaacgg agatataaat gcgttaaccg 177600
   acaataacaa acacgctaca caactcatta tagataacaa agaaaattcc ccatatacca 177660
   ttaattgttt actgtatata cttagatata ttgtagataa gaatgtgata agatcgttgg 177720
   tggatcaact tccatctcta cctatcttcg atataaaatc atttgagaaa ttcatatcct 177780
   actgtatact tttagatgac acattttacg ataggcacgt taagaatcgc gattctaaaa 177840
   cgtatcgata cgcattttca aaatacatgt cgtttgataa atacgatggt ataataacta 177900
   aatgtcacga cgaaacaatg ttactcaaac tgtccactgt tctagacact acactatatg 177960
   cagttttaag atgtcataat tcgagaaagt taagaagata cctcaacgag ttaaaaaaat 178020
   ataataacga taagtccttt aaaatatatt ctaatattat gaatgagaga taccttaatg 178080
   tatattataa agatatgtac gtgtcaaagg tatatgataa actatttcct gttttcacag 178140
   ataaaaattg tctactaaca ttactacctt cagaaattat atacgaaata ttatacatgc 178200
   tgacaattaa cgatctttat aatatatcgt atccacctac caaagtatag ttgtattttt 178260
   ctcatgcgat gtgtgtaaaa aaactgatat tatataaata ttttagtgcc gtataataaa 178320
   gatgacgatg aaaatgatgg tacatatata tttcgtatca ttattgttat tgctattcca 178380
   cagttacgcc atagacatcg aaaatgaaat cacagaattc ttcaataaaa tgagagatac 178440
   tctaccagct aaagactcta aatggttgaa tccagcatgt atgttcggag gcacaatgaa 178500
   tgatatagcc gctctaggag agccattcag cgcaaagtgt cctcctattg aagacagtct 178560
   tttatcgcac agatataaag actatgtggt taaatgggag aggctagaaa agaatagacg 178620
   gcgacaggtt tctaataaac gtgttaaaca tggtgattta tggatagcca actatacatc 178680
   taaattcagt aaccgtaggt atttgtgcac cgtaactaca aagaatggtg actgtgttca 178740
   gggtatagtt agatctcata ttaaaaaacc tccttcatgc attccaaaaa catatgaact 178800
   aggtactcat gataagtatg gcatagactt atactgtgga attctttacg caaaacatta 178860
   taataatata acttggtata aagataataa ggaaattaat atcgacgaca ttaagtattc 178920
   acaaacggga aaggaattaa ttattcataa tccagagtta gaagatagcg gaagatacga 178980
   ctgttacgtt cattacgacg acgttagaat caagaatgat atcgtagtat caagatgtaa 179040
   aatacttacg gttataccgt cacaagacca caggtttaaa ctaatactag atccgaaaat 179100
   caacgtaacg ataggagaac ctgccaatat aacatgcact gctgtgtcaa cgtcattatt 179160
   gatcgacgat gtactgattg aatgggaaaa tccatccgga tggcttatag gattcgattt 179220
   tgatgtatac tctgttttaa ctagtagagg cggtatcacc gaggcgacct tgtactttga 179280
   aaatgttact gaagaatata taggtaatac atataaatgt cgtggacaca actattattt 179340
   tgaaaaaacc cttacaacta cagtagtatt ggagtaaata tacaatgcat ttttatatac 179400
   attactgaat tattattact gaattattat tactgaatta ttattaatta tatcgtattt 179460
   gtgctataga atggatgaag atacgcgact atctaggtat ttgtatctca ccgatagaga 179520
   acatataaat gtagactcta ttaaacagtt gtgtaaaata tcagatccta atgcatgtta 179580
   tagatgtgga tgtacggctt tacatgagta cttttataat tatagatcag tcaacggaaa 179640
   atacaagtat agatacaacg gttactatca atattattca tctagcgatt atgaaaatta 179700
   taatgaatat tattatgata gaactggtat gaacagtgag agtgataata tatcaatcaa 179760
   aacagaatat gaattctatg atgaaacaca agatcaaagt acacaactag taggttacga 179820
   cattaaactc aaaaccaatg aggatgattt tatggctatg atagatcagt gggtgtccat 179880
   gattatatag atgaatcaat taataaagta gtatatggaa gagagtctca cgtaagatgg 179940
   cgggatatat ggcaagaaca taatgatggc gtatacagta taggaaagga gtgcatagat 180000
   aatatatacg aagacaacca taccgtagac gaattctaca agatagacag cgtatcagat 180060
   gtagatgacg cggaacacat atctccgata actaaaaaac catagaatca gttgatgata 180120
   atacctacat ttctaatctt ccgtatacca tcaaatacaa aatattcgag caacaataag 180180
   tattttttat acctttaaaa ctgataaata aattttttct agtgatattt tggcaagatg 180240
   agaatcctat ttctcatcgc tttcatgtat gggtgtgttc actcatatgt taacgcggtt 180300
   gaaaccaaat gtccaaatct agacattgta acatcttctg gagaatttca ttgttcagga 180360
   tgtgtggaac atatgcctga gtttagctat atgtattggt tggcaaagga tatgaaatcg 180420
   gacgaggata ccaagtttat agaacatctg ggtgatggca tcaaagaaga tgaaaccgtt 180480
   cgtaccacag atagtggaat cgtcactcta cgtaaagtcc ttcatgtgac tgatactaat 180540
   aaatttgata attataggtt cacttgtgtc ctcgctacgc tagatggcat ttataaaaag 180600
   aatgtttgct gaagtagttg tgtgctacta tttttattta tgatataatc taatggaatt 180660
   aatttgaatt gatatttatc caatactaaa gattatatta gaatcaaatt aatcttttat 180720
   acgagaaaaa ataacgacat acgtcgtcaa caaattaaac tttttattta ttagttaact 180780
   agcttataga acttgctcat tgttatgttt ctaaaacggg tacggcatat aggacaatta 180840
   tccgacgcac cggtttctct tcgtgttcta tgccatatat tgatgcatgt tatgcaaaat 180900
   atatgagtac acgaatccaa taaaccaaag tatctatcgt tttgagtaaa caacttcata 180960
   gcaaattcca cattcttttt ctttacttac tctatacacg tcctcgtatt tatttagtat 181020
   tttgatgata tccaactcag aaatggttgt tgtattattg ggtgtatttg gagtataggt 181080
   attattagct atgtaccaat ttaccaaccc tcttaatatt gattgataat cacatcggtt 181140
   atccaattaa taactaaatt gtagtgtata tatagaccat atatgtttct atttttttga 181200
   cagttacgta tagtttcagt aagttttgat tgttgtattc ctgtatctct agataagtta 181260
   gtcatatagt cccttccggc gatacgtttt ttccaagccc gaaattgatt agccaaatgt 181320
   gtatttattt ttgtgatatt gatataatat ttcggataat gcatactgtt agtcttatat 181380
   catttggttc atctatgtat tgtaatattg ttacatgatc tatagatgat gtattgattt 181440
   tggcaggatc gaattccata tccgcgacta aacagtgaaa aaaatgtaaa tactttttaa 181500
   attttaaatt agtaaaactt ttttttattt tttatgattc caaaaatact gaatacaaag 181560
   tcctaaatta taaatatgga gatcatacta ccacaactta ttattatgta tacaaggccg 181620
   gtgtaataga tagatatata taattctatt acaccggcag acaattaccg accggtattt 181680
   gtcgttacca acataccgta taatatgtaa tatacaattc cataacccat tgacagttgt 181740
   tatacatcaa aattgcaatt cttttgatta cgatgttata agaatgtagt taattgatgt 181800
   atgatgttaa tgtgtcctct ttcctcttat aacatcgtaa tcaaaaactt ttttataata 181860
   tatacctaat aatgtgtctt aatagttctc gtgattcgtc aaacaatcat tcttataaaa 181920
   tataataaag caacgtaaaa acacataaaa ataagcgtaa ctaataagac aatggatatt 181980
   tacgacgata aaggtctaca gactattaaa ctgtttaata atgaatttga ttgtataagg 182040
   aatgacatca gagaattatt taaacatgta actgattccg atagtataca acttccgatg 182100
   gaagacaatt ctgatattat agaaaatatc agaaaaatac tatatagacg attaaaaaat 182160
   gtagaatgtg ttgacatcga taacacaata acttttatga aatacgatcc aaatgatgat 182220
   aataagcgta cgtgttctaa ttgggtaccc ttaactaata actatatgga atattgtcta 182280
   gtaatatatt tggaaacacc gatatgtgga ggcaaaataa aattatacca ccctacagga 182340
   aatataaagt cggataagga tattatgttt gcaaagactc tagactttaa atcaacgaaa 182400
   gtgttaactg gacgtaaaac aattgccgtt ctagacatat ccgtttcata taatagatca 182460
   atgactacta ttcactacaa cgacgacgtt gatatagata tacatactga taaaaatgga 182520
   aaagagttat gttattgtta tataacaata gatgatcatt acttggttga tgtggaaact 182580
   ataggagtta tagtcaatag atctggaaaa tgtctgttag taaataacca tctaggtata 182640
   ggtatcgtta aagataaacg tataagcgat agttttggag atgtatgtat ggatacaata 182700
   tttgactttt ctgaagcacg agagttattt tcattaacta atgatgataa caggaatata 182760
   gcatgggaca ctgataaact agacgatgat acagatatat ggactcccgt cacagaagat 182820
   gattacaaat ttctttctag actagtattg tatgcaaaat ctcaatcgga tactgtattt 182880
   gactattatg ttcttactgg tgatacggaa ccacccactg tattcatttt caaggtaact 182940
   agattttact ttaatatgcc gaaataaaaa atttttgtat aatatctaga ggtagaggta 183000
   ttgtttagat aaatacaaat aacatagata catcgcatac ttagcatttt tataaatata 183060
   cataagacat acactttata catttttgta aaaatactca taaaaaaatt tataaaaatt 183120
   atggcacaac catatcttgt ataggtagtt tagttcgtcg agtgaaccta taaacagata 183180
   atagacaaca cataataatg cctactaata caagcataat accgggagat gggatatatg 183240
   acgttgtagt gtttgggttt tctgaacgtt gatagtctac taatactaca tgctgacatc 183300
   taatgcctgt ataaccatga gagcatctac aatacatacc gtcaatatct ctagcgtgga 183360
   tacagtcacc gtgtaaacaa tatccatctc cctctggacc gcataatctg atagctggaa 183420
   tatctgttgt agcgtttgta atttctggca atgtcgtttc gatagcgtta ccactatcgg 183480
   cgaatgatct gattatcata gcagcgaaca acaacatcag ataatttatc aacatttttg 183540
   atggattctg tgtttatgct gtttctcagt gtgtgtttat gacaagattg ggaattttat 183600
   attattaatt cagtaatata aactaataat atattgttaa ttgtgtaaat aatataaaaa 183660
   taacaataca atattgaatg tgttgctgtt aaaaatgtat gtgttaatat aatagaataa 183720
   aataaatgag tatgatcatt ttagataacg attgatttta tcattaccgc ttcattctta 183780
   tattctttgc ttacggaacc tatatttaga aacatctact aacaattttt tatgcttgca 183840
   ttattaatgg tatgtaatat gattgattgt gtacgcaata ccaatttgtt aagtatgaat 183900
   acggggtaca aacataaatt gaaatttaac attatttatt tatgatatat atcgttatcg 183960
   ttaggtctat accatggata tctttaaaga actaatctta aaacaccctg atgaaaatgt 184020
   tttgatttct ccagtttcca ttttatctac tttatctatt ctaaatcatg gagcagctgg 184080
   ttctacagct gaacaactat caaaatatat agagaatatg aatgagaata cacccgatga 184140
   caataatgat gacatggagg tagatattcc gtattgtgcg acactagcta ccgcaaataa 184200
   aatatacggt agcgatagta tcgagttcca cgcctccttc ctacaaaaaa taaaagacga 184260
   ttttcaaact gtaaacttta ataatgctaa ccaaacaaag gaactaatca acgaatgggt 184320
   taagacaatg acaaatggta aaattaattc cttattgact agtccgctat ccattaatac 184380
   tcgtatgaca gttgttagcg ccgtccattt taaagcaatg tggaaatatc cattttctaa 184440
   acatcttaca tatacagaca agttttatat ttctaagaat atagttacca gcgttgatat 184500
   gatggtgggt accgagaata acttgcaata tgtacatatt aatgaattat tcggaggatt 184560
   ctctattatc gatattccat acgagggaaa ctctagtatg gtaattatac taccggacga 184620
   catagaaggt atatataaca tagaaaaaaa tataacagat gaaaaattta aaaaatggtg 184680
   tggtatgtta tctactaaaa gtatagactt gtatatgcca aagtttaaag tggaaatgac 184740
   agaaccgtat aatctggtac cgattttaga aaatttagga cttactaata tattcggata 184800
   ttatgcagat tttagcaaga tgtgtaatga aactatcact gtagaaaaat ttctacatac 184860
   gacgtttata gatgttaatg aggagtatac agaagcatcg gccgttacag gagtatttac 184920
   gattaacttt tcgatggtat atcgtacgaa ggtctacata aaccatccat tcatgtacat 184980
   gattaaagac accacaggac gtatactttt tatagggaaa tactgctatc cgcaataaat 185040
   ataaacaaat agacttttat aaagagtctt caacgataag tatatcgaca tactacttat 185100
   gctgcgaaag attctgaacg agaacgacta tctcaccctc ttggatcata tccgcactgc 185160
   taaatactaa atctccacta cactttttat catcttatga ggaatgattg ccttcgtgaa 185220
   ataggaataa ttagcaccag aatagctatg gattattgtg gtagagagtg cactattcta 185280
   tgtcgtctac tggatgaaga tgtgacgtac aaaaaaataa aactagaaat tgaaacgtgt 185340
   cacaacttat caaaacatat agatagacga ggaaacaatg cgctacattg ttacgtctcc 185400
   aataaatgcg atacagacat taagattgtt ctctcgcgga gtcgagagac tttgtagaaa 185460
   caacgaagga ttaactccgc taggagtata cagtaagcat agatacgtaa aatctcagat 185520
   tgtgcatcta ctgatatcca gctattcaaa ttcctctaac gaactcaagt cgaatataaa 185580
   tgatttcgat ctgtattcgg ataatatcga cttacgtctg ctaaaatacc taattgtgga 185640
   taaacggata cgtccgtcca agaatacgaa ttatgcaatc aatggtctcg gattggtgga 185700
   tatatacgta acgacgccta atccgagacc agaagtattg ctatggcttc ttaaatcaga 185760
   atgttacagc accggttacg tatttcgtac ctgtatgtac gacagtgata tgtgtaagaa 185820
   ctctcttcat tactatatat cgtctcatag agaatctcaa tctctatcca aggatgtaat 185880
   taaatgtttg atcgataaca atgtttccat ccatggcaga gacgaaggag gatctttacc 185940
   catccaatac tactggtctt tctcaaccat agatatagag attgttaaat tattattaat 186000
   aaaggatgtg gacacgtgta gagtatacga cgtcagccct atattagagg cgtattatct 186060
   aaacaagcga tttagagtaa ccccatataa tgtagacatg gaaatcgtta atcttcttat 186120
   tgagagacgt catactcttg tcgacgtaat gcgtagtatt acttcgtacg attccagaga 186180
   atataaccac tacatcatcg ataacattct aaagagattt agacaacagg atgaatccat 186240
   cgtacaagcc atactgataa actacttaca ttacggcgat atggtcgttc gatgcatgtt 186300
   agataacgga caacaactat cctctacacg actactttgt taataataat ctcgtcgatg 186360
   taaacgtcgt aaggtttatc gtggaaaata tggacacgcg gctgtaaatc acgtatcgaa 186420
   caatggccgt ctatgtatgt acggtctgat attatcgaga tttaataatt gcgggtatca 186480
   ctgttatgaa accatactga tagatgtatt tgatatacta agcaagtaca tggatgatat 186540
   agatatgatc gataactcta ctatattacg cggtcgatgt caataatata caatttgcaa 186600
   agcggttatt ggaatatgga gcgagtatca cgctcgataa tcaatacggc catccagaaa 186660
   agcagttaca gaagagaaaa caaaacgaaa tcgttgttct acaagatgtc tctccctacg 186720
   acgattacta cgtaaaaaag atactagcct actgcctatt aagggacgag tcattcgcgg 186780
   aactacatag taaattctgt ttaaacgagg actataaaag tgtatttatg aaaaatatat 186840
   cattcgataa gatagattcc atcatcgtga cataagtcgc ctcaaagaga ttcgaatctc 186900
   cgacaccgac ctgtatacgg tatcacagct atcttaaagc catacattca gacagtcaca 186960
   tttcatttcc catgtacgac gatctcatag aacagtgcca tctatcgatg gagcgtaaaa 187020
   gtaaactcgt cgacaaagca ctcaataaat tagagtctac catcggtcaa tctagactat 187080
   cgtatttgcc tccggaaatt atgcgcaata tcatctaaac agtatgttgt acggaaagaa 187140
   ccattacaaa tattatccat gatagaaaga aaatatctat atgattggag aagtaggaaa 187200
   caggaacaag acaacgatta ctacattatt aaatcatgaa gtccgtatta tactcgtata 187260
   tattgtttct ctcatgtata ataataaacg gaagagatat agcaccgcat gcaccatccg 187320
   atggaaagtg taaagacaac gaatacaaac gccataattt gtgtccggga acatacgctt 187380
   ccagattatg cgatagcaag actaacacac gatgtacgcc gtgtggttcg ggtaccttca 187440
   catctcgcaa taatcattta cccgcttgtc taagttgtaa cggaagacgc gatcgtgtaa 187500
   cacgactcac aatagaatct gtgaatgctc tcccggatat tattgtcttc tcaaaggatc 187560
   atccggatgc aaggcatgtg tttcccaaac aaaatgtgga ataggatacg gagtatccgg 187620
   agacgtcatc tgttctccgt gtggtctcgg aacatattct cacaccgtct cttccgcaga 187680
   taaatgcgaa cccgtaccca gaaatacgtt taactatatc gatgtggaaa ttaacctgta 187740
   tccagttaac gacacgtcgt gtactcggac gaccactacc ggtctcagcg aatccatctc 187800
   aacgtcggaa ctaactatta ctatgaatca taaagactgt aatcccgtat ttcgtgatgg 187860
   atacttctcc gttcttaata aggtagcgac ttcaggtttc tttacaggag aaaggtgtgc 187920
   actctgaatt tcgagattaa atgcaataac aaagattctt cctccaaaca gttaacgaaa 187980
   gcaaagaatg atactatcat gccgcattcg gagacagtaa ctctagtggg cgacatctat 188040
   atactatata gtaataccaa tactcaagac tacgaaactg atacaatctc ttatcatgtg 188100
   ggtaatgttc tcgatgtcga tagccatatg cccggtagtt gcgatataca taaactgatc 188160
   actaattcca aacccaccca ctttttatag taagtttttc acccataaat aataaataca 188220
   ataattaatt tctcgtaaaa gtagaaaata tattctaatt tattgcacgg taaggaagta 188280
   gaatcataaa gaacagtact caatcaatag caattatgaa acaatatatc gtcctggcat 188340
   gcatgtgcct ggcggcagct gctatgcctg ccagtcttca gcaatcatcc tcatcctcct 188400
   cctcgtgtac ggaagaagaa aacaaacatc atatgggaat cgatgttatt atcaaagtca 188460
   caaagcaaga ccaaacaccg accaatgata agatttgcca atccgtaacg gaaattacag 188520
   agtccgagtc agatccagat cccgaggtgg aatcagaaga tgattccaca tcagtcgagg 188580
   atgtagatcc tcctaccact tattactcca tcatcggtgg aggtctgaga atgaactttg 188640
   gattcaccaa atgtcctcag attaaatcca tctcagaatc cgctgatgga aacacagtga 188700
   atgctagatt gtccagcgtg tccccaggac aaggtaagga ctctcccgcg atcactcatg 188760
   aagaagctct tgctatgatc aaagactgtg aagtgtctat cgacatcaga tgtagcgaag 188820
   aagagaaaga cagcgacatc aagacccatc cagtactcgg gtctaacatc tctcataaga 188880
   aagtgagtta cgaagatatc atcggttcaa cgatcgtcga tacaaaatgc gtcaagaatc 188940
   tagagtttag cgttcgtatc ggagacatgt gcaaggaatc atctgaactt gaggtcaagg 189000
   atggattcaa gtatgtcgac ggatcggcat ctgaaggtgc aaccgatgat acttcactca 189060
   tcgattcaac aaaactcaaa gcgtgtgtct gaatcgataa ctctattcat ctgaaattgg 189120
   atgagtaggg ttaatcgaac gattcaggca caccacgaat taaaaaagtg taccggacac 189180
   tatattccgg tttgcaaaac aaaaatgttc ttaactacat tcacaaaaag ttacctctcg 189240
   cgacttcttc tttttctgtc tcaatagtgt gatacgatta tgacactatt cctattccta 189300
   ttcctatttc ctttcagagt atcacaaaaa tattaaacct ctttctgatg gtctcataaa 189360
   aaaagtttta caaaaatatt tttattctct ttctctcttt gatggtctca taaaaaaagt 189420
   tttacaaaaa tatttttatt ctctttctct ctttgatggt ctcataaaaa aagttttaca 189480
   aaaatatttt tattctcttt ctctctttga tggtctcata aaaaaagttt tacaaaaata 189540
   tttttattct ctttctctct ttgatggtct cataaaaaaa gttttacaaa aatattttta 189600
   ttctctttct ctctttgatg gtctcataaa aaaagtttta caaaaatatt tttattctct 189660
   ttctctcttt gatggtctca taaaaaaagt tttacaaaaa tatttttatt ctctttctct 189720
   ctttgatggt ctcataaaaa aagttttaca aaaatatttt tattctcttt ctctctttga 189780
   tggtctcata aaaaaagttt tacaaaaata tttttattct ctttctctct ttgatggtct 189840
   cataaaaaaa gttttacaaa aatattttta ttctctttct ctctttgatg gtctcataaa 189900
   aaaagtttta caaaaatatt tttattctct ttctctcttt gatggtctca taaaaaaagt 189960
   tttacaaaaa tatttttatt ctctttctct ctttgatggt ctcataaaaa agttttacaa 190020
   aaatattttt attctctttc tctctttgat ggtctcataa aaaagtttta caaaaatatt 190080
   tttattctct ttctctcttt gatggtctca taaaaaaagt tttacaaaaa tatttttatt 190140
   ctctttctct ctttgatggt ctcataa 190167
<210> 3
   <211> 1854
   <212> DNA
   <213> Homo sapiens
<220>
   <223> norepinephrine transporter cDNA
<400> 3
   atgcttctgg cgcggatgaa cccgcaggtg cagcccgaga acaacggggc ggacacgggt 60
   ccagagcagc cccttcgggc gcgcaaaact gcggagctgc tggtggtgaa ggagcgcaac 120
   ggcgtccagt gcctgctggc gccccgcgac ggcgacgcgc agccccggga gacctggggc 180
   aagaagatcg acttcctgct gtccgtagtc ggcttcgcag tggacctggc caacgtgtgg 240
   cgcttcccct acctctgcta caagaacggc ggcggtgcct tcttgatccc gtacacactg 300
   ttccttatca tcgcggggat gcccctgttc tacatggagc tggctctggg acagtacaac 360
   cgggaggggg ctgccaccgt ttggaaaatc tgcccattct tcaaaggcgt tggctatgct 420
   gtcatcctga tcgccctgta cgttggcttc tactacaacg tcatcatcgc ctggtcactc 480
   tactacctct tctcctcctt caccctcaac ctgccctgga ccgactgtgg ccacacctgg 540
   aacagcccca actgtaccga ccccaagctc ctcaatggct ccgtgcttgg caaccacacc 600
   aagtactcca agtacaagtt cacgccggca gccgagtttt atgagcgtgg tgtcctgcac 660
   cttcacgaga gcagcgggat tcatgacatc ggcctgcccc agtggcagct cttgctctgt 720
   ctgatggtcg tcgtcatcgt cttgtatttt agcctctgga aaggggtgaa gacatcagga 780
   aaggtggtgt ggatcacagc cacgctgcct tacttcgtgc tgttcgtgct cctggtccat 840
   ggcgtcacgc tgcccggagc ctccaatggc atcaatgcct acctgcacat cgacttctac 900
   cgcttgaaag aggccacggt atggattgat gccgcaactc agatattttt ttccttgggg 960
   gctggatttg gagtattgat tgcatttgcc agttacaaca aatttgacaa caactgttac 1020
   agggatgccc tgctgaccag cagcatcaac tgtatcacca gcttcgtctc tgggttcgcc 1080
   atcttctcca tccttggtta catggcccat gaacacaagg tcaacattga ggatgtggcc 1140
   acagaaggag ctggcctagt gttcatcctg tatccagagg ccatttctac cctgtctgga 1200
   tctacattct gggctgttgt gtttttcgtc atgctcctgg cgctgggcct tgacagctca 1260
   atgggaggca tggaggctgt catcacgggc ctggcagatg acttccaggt cctgaagcga 1320
   caccggaaac tcttcacatt tggcgtcacc ttcagcactt tccttctcgc cctgttctgc 1380
   ataaccaagg gtggaattta cgtcttgacc ctcctggaca cctttgctgc gggcacctcc 1440
   atcctttttg ctgtcctcat ggaagccatc ggagtttcct ggttttatgg agtggacagg 1500
   ttcagcaacg acatccagca gatgatgggg ttcaggccgg gtctatactg gagactgtgc 1560
   tggaagttcg tcagtcctgc cttcctcctg ttcgtggttg tggtcagcat catcaacttc 1620
   aagccactca cctacgacga ctacatcttc ccgccctggg ccaactgggt ggggtggggc 1680
   atcgccctgt cctccatggt cctggtgccc atctacgtca tctataagtt cctcagcacg 1740
   cagggctctc tttgggagag actggcctat ggcatcacgc cagagaacga gcaccacctg 1800
   gtggctcaga gggacatcag acagttccag ttgcaacact ggctggccat ctga       1854
<210> 4
   <211> 617
   <212> PRT
   <213> Homo sapiens
<220>
   <223> Norepinephrine transporter (P23975)
<400> 4
<210> 5
   <211> 643
   <212> PRT
   <213> Homo sapiens
<220>
   <223> NIS (Q92911)
<400> 5
<210> 6
   <211> 1929
   <212> DNA
   <213> Homo sapiens
<220>
   <223> NIS ORF
<400> 6
   atggaggccg tggagaccgg ggaacggccc accttcggag cctgggacta cggggtcttt 60
   gccctcatgc tcctggtgtc cactggcatc gggctgtggg tcgggctggc tcggggcggg 120
   cagcgcagcg ctgaggactt cttcaccggg ggccggcgcc tggcggccct gcccgtgggc 180
   ctgtcgctgt ctgccagctt catgtcggcc gtgcaggtgc tgggcgtgcc gtcggaggcc 240
   tatcgctatg gcctcaagtt cctctggatg tgcctgggcc agcttctgaa ctcggtcctc 300
   accgccctgc tcttcatgcc cgtcttctac cgcctgggcc tcaccagcac ctacgagtac 360
   ctggagatgc gcttcagccg cgcagtgcgg ctctgcggga ctttgcagta cattgtagcc 420
   acgatgctgt acaccggcat cgtaatctac gcaccggccc tcatcctgaa ccaagtgacc 480
   gggctggaca tctgggcgtc gctcctgtcc accggaatta tctgcacctt ctacacggct 540
   gtgggcggca tgaaggctgt ggtctggact gatgtgttcc aggtcgtggt gatgctaagt 600
   ggcttctggg ttgtcctggc acgcggtgtc atgcttgtgg gcgggccccg ccaggtgctc 660
   acgctggccc agaaccactc ccggatcaac ctcatggact ttaaccctga cccgaggagc 720
   cgctatacat tctggacttt tgtggtgggt ggcacgttgg tgtggctctc catgtatggc 780
   gtgaaccagg cgcaggtgca gcgctacgtg gcttgccgca cagagaagca ggccaagctg 840
   gccctgctca tcaaccaggt cggcctgttc ctgatcgtgt ccagcgctgc ctgctgtggc 900
   atcgtcatgt ttgtgttcta cactgactgc gaccctctcc tcctggggcg catctctgcc 960
   ccagaccagt acatgcctct gctggtgctg gacatcttcg aagatctgcc tggagtcccc 1020
   gggcttttcc tggcctgtgc ttacagtggc accctcagca cagcatccac cagcatcaat 1080
   gctatggctg cagtcactgt agaagacctc atcaaacctc ggctgcggag cctggcaccc 1140
   aggaaactcg tgattatctc caaggggctc tcactcatct acggatcggc ctgtctcacc 1200
   gtggcagccc tgtcctcact gctcggagga ggtgtccttc agggctcctt caccgtcatg 1260
   ggagtcatca gcggccccct gctgggagcc ttcatcttgg gaatgttcct gccggcctgc 1320
   aacacaccgg gcgtcctcgc gggactaggc gcgggcttgg cgctgtcgct gtgggtggcc 1380
   ttgggcgcca cgctgtaccc acccagcgag cagaccatga gggtcctgcc atcgtcggct 1440
   gcccgctgcg tggctctctc agtcaacgcc tctggcctcc tggacccggc tctcctccct 1500
   gctaacgact ccagcagggc ccccagctca ggaatggacg ccagccgacc cgccttagct 1560
   gacagcttct atgccatctc ctatctctat tacggtgccc tgggcacgct gaccactgtg 1620
   ctgtgcggag ccctcatcag ctgcctgaca ggccccacca agcgcagcac cctggccccg 1680
   ggattgttgt ggtgggacct cgcacggcag acagcatcag tggcccccaa ggaagaagtg 1740
   gccatcctgg atgacaactt ggtcaagggt cctgaagaac tccccactgg aaacaagaag 1800
   ccccctggct tcctgcccac caatgaggat cgtctgtttt tcttggggca gaaggagctg 1860
   gagggggctg gctcttggac cccctgtgtt ggacatgatg gtggtcgaga ccagcaggag 1920
   acaaacctc                                                         1929
<210> 7
   <211> 264
   <212> PRT
   <213> Artificial Sequence
<220>
   <223> tTF-RGD protein
<400> 7
   Met Glu Thr Pro Ala Trp Pro Arg Val Pro Arg Pro Glu Thr Ala Val
   1               5                   10                  15
   Ala Arg Thr Leu Leu Leu Gly Trp Val Phe Ala Gln Val Ala Gly Ala
               20                  25                  30
   Ser Gly Thr Thr Asn Thr Val Ala Ala Tyr Asn Leu Thr Trp Lys Ser
           35                  40                  45
   Thr Asn Phe Lys Thr Ile Leu Glu Trp Glu Pro Lys Pro Val Asn Gln
       50                  55                  60
   Val Tyr Thr Val Gln Ile Ser Thr Lys Ser Gly Asp Trp Lys Ser Lys
   65                  70                  75                  80
   Cys Phe Tyr Thr Thr Asp Thr Glu Cys Asp Leu Thr Asp Glu Ile Val
                   85                  90                  95
   Lys Asp Val Lys Gln Thr Tyr Leu Ala Arg Val Phe Ser Tyr Pro Ala
               100                 105                 110
   Gly Asn Val Glu Ser Thr Gly Ser Ala Gly Glu Pro Leu Tyr Glu Asn
           115                 120                 125
   Ser Pro Glu Phe Thr Pro Tyr Leu Glu Thr Asn Leu Gly Gln Pro Thr
       130                 135                 140
   Ile Gln Ser Phe Glu Gln Val Gly Thr Lys Val Asn Val Thr Val Glu
   145                 150                 155                 160
   Asp Glu Arg Thr Leu Val Arg Arg Asn Asn Thr Phe Leu Ser Leu Arg
                   165                 170                 175
   Asp Val Phe Gly Lys Asp Leu Ile Tyr Thr Leu Tyr Tyr Trp Lys Ser
               180                 185                 190
   Ser Ser Ser Gly Lys Lys Thr Ala Lys Thr Asn Thr Asn Glu Phe Leu
           195                 200                 205
   Ile Asp Val Asp Lys Gly Glu Asn Tyr Cys Phe Ser Val Gln Ala Val
       210                 215                 220
   Ile Pro Ser Arg Thr Val Asn Arg Lys Ser Thr Asp Ser Pro Val Glu
   225                 230                 235                 240
   Cys Met Gly Gln Glu Lys Gly Glu Phe Arg Gly Arg Gly Asp Ser Pro
                   245                 250                 255
   Asp Tyr Lys Asp Asp Asp Asp Lys
               260
<210> 8
   <211> 272
   <212> PRT
   <213> Artificial Sequence
<220>
   <223> G6-FLAG protein sequence
<400> 8
   Met Glu Thr Asp Thr Leu Leu Leu Trp Val Leu Leu Leu Trp Val Pro
   1               5                   10                  15
   Gly Ser Thr Gly Asp Glu Val Gln Leu Val Glu Ser Gly Gly Gly Leu
               20                  25                  30
   Val Gln Pro Gly Gly Ser Leu Arg Leu Ser Cys Ala Ala Ser Gly Phe
           35                  40                  45
   Thr Ile Ser Asp Tyr Trp Ile His Trp Val Arg Gln Ala Pro Gly Lys
       50                  55                  60
   Gly Leu Glu Trp Val Ala Gly Ile Thr Pro Ala Gly Gly Tyr Thr Tyr
   65                  70                  75                  80
   Tyr Ala Asp Ser Val Lys Gly Arg Phe Thr Ile Ser Ala Asp Thr Ser
                   85                  90                  95
   Lys Asn Thr Ala Tyr Leu Gln Met Asn Ser Leu Arg Ala Glu Asp Thr
               100                 105                 110
   Ala Val Tyr Tyr Cys Ala Arg Phe Val Phe Phe Leu Pro Tyr Ala Met
           115                 120                 125
   Asp Tyr Trp Gly Gln Gly Thr Leu Val Thr Val Ser Ser Gly Gly Gly
       130                 135                 140
   Gly Ser Gly Gly Gly Gly Ser Gly Gly Gly Gly Ser Asp Ile Gln Met
   145                 150                 155                 160
   Thr Gln Ser Pro Ser Ser Leu Ser Ala Ser Val Gly Asp Arg Val Thr
                   165                 170                 175
   Ile Thr Cys Arg Ala Ser Gln Asp Val Ser Thr Ala Val Ala Trp Tyr
               180                 185                 190
   Gln Gln Lys Pro Gly Lys Ala Pro Lys Leu Leu Ile Tyr Ser Ala Ser
           195                 200                 205
   Phe Leu Tyr Ser Gly Val Pro Ser Arg Phe Ser Gly Ser Gly Ser Gly
       210                 215                 220
   Thr Asp Phe Thr Leu Thr Ile Ser Ser Leu Gln Pro Glu Asp Phe Ala
   225                 230                 235                 240
   Thr Tyr Tyr Cys Gln Gln Gly Tyr Gly Asn Pro Phe Thr Phe Gly Gln
                   245                 250                 255
   Gly Thr Lys Val Glu Ile Lys Arg Asp Tyr Lys Asp Asp Asp Asp Lys
               260                 265                 270
<210> 9
   <211> 207
   <212> PRT
   <213> Artificial Sequence
<220>
   <223> IL-24
<400> 9
   Met Asn Phe Gln Gln Arg Leu Gln Ser Leu Trp Thr Leu Ala Ser Arg
   1               5                   10                  15
   Pro Phe Cys Pro Pro Leu Leu Ala Thr Ala Ser Gln Met Gln Met Val
               20                  25                  30
   Val Leu Pro Cys Leu Gly Phe Thr Leu Leu Leu Trp Ser Gln Val Ser
           35                  40                  45
   Gly Ala Gln Gly Gln Glu Phe His Phe Gly Pro Cys Gln Val Lys Gly
       50                  55                  60
   Val Val Pro Gln Lys Leu Trp Glu Ala Phe Trp Ala Val Lys Asp Thr
   65                  70                  75                  80
   Met Gln Ala Gln Asp Asn Ile Thr Ser Ala Arg Leu Leu Gln Gln Glu
                   85                  90                  95
   Val Leu Gln Asn Val Ser Asp Ala Glu Ser Cys Tyr Leu Val His Thr
               100                 105                 110
   Leu Leu Glu Phe Tyr Leu Lys Thr Val Phe Lys Asn Tyr His Asn Arg
           115                 120                 125
   Thr Val Glu Val Arg Thr Leu Lys Ser Phe Ser Thr Leu Ala Asn Asn
       130                 135                 140
   Phe Val Leu Ile Val Ser Gln Leu Gln Pro Ser Gln Glu Asn Glu Met
   145                 150                 155                 160
   Phe Ser Ile Arg Asp Ser Ala His Arg Arg Phe Leu Leu Phe Arg Arg
                   165                 170                 175
   Ala Phe Lys Gln Leu Asp Val Glu Ala Ala Leu Thr Lys Ala Leu Gly
               180                 185                 190
   Glu Val Asp Ile Leu Leu Thr Trp Met Gln Lys Phe Tyr Lys Leu
           195                 200                 205
<210> 10
   <211> 520
   <212> PRT
   <213> Artificial Sequence
<220>
   <223> SIL-6R/IL-6 fusion
<400> 10
   Met Leu Ala Val Gly Cys Ala Leu Leu Ala Ala Leu Leu Ala Ala Pro
   1               5                   10                  15
   Gly Ala Ala Leu Ala Pro Arg Arg Cys Pro Ala Gln Glu Val Ala Arg
               20                  25                  30
   Gly Val Leu Thr Ser Leu Pro Gly Asp Ser Val Thr Leu Thr Cys Pro
           35                  40                  45
   Gly Val Glu Pro Glu Asp Asn Ala Thr Val His Trp Val Leu Arg Lys
       50                  55                  60
   Pro Ala Ala Gly Ser His Pro Ser Arg Trp Ala Gly Met Gly Arg Arg
   65                  70                  75                  80
   Leu Leu Leu Arg Ser Val Gln Leu His Asp Ser Gly Asn Tyr Ser Cys
                   85                  90                  95
   Tyr Arg Ala Gly Arg Pro Ala Gly Thr Val His Leu Leu Val Asp Val
               100                 105                 110
   Pro Pro Glu Glu Pro Gln Leu Ser Cys Phe Arg Lys Ser Pro Leu Ser
           115                 120                 125
   Asn Val Val Cys Glu Trp Gly Pro Arg Ser Thr Pro Ser Leu Thr Thr
       130                 135                 140
   Lys Ala Val Leu Leu Val Arg Lys Phe Gln Asn Ser Pro Ala Glu Asp
   145                 150                 155                 160
   Phe Gln Glu Pro Cys Gln Tyr Ser Gln Glu Ser Gln Lys Phe Ser Cys
                   165                 170                 175
   Gln Leu Ala Val Pro Glu Gly Asp Ser Ser Phe Tyr Ile Val Ser Met
               180                 185                 190
   Cys Val Ala Ser Ser Val Gly Ser Lys Phe Ser Lys Thr Gln Thr Phe
           195                 200                 205
   Gln Gly Cys Gly Ile Leu Gln Pro Asp Pro Pro Ala Asn Ile Thr Val
       210                 215                 220
   Thr Ala Val Ala Arg Asn Pro Arg Trp Leu Ser Val Thr Trp Gln Asp
   225                 230                 235                 240
   Pro His Ser Trp Asn Ser Ser Phe Tyr Arg Leu Arg Phe Glu Leu Arg
                   245                 250                 255
   Tyr Arg Ala Glu Arg Ser Lys Thr Phe Thr Thr Trp Met Val Lys Asp
               260                 265                 270
   Leu Gln His His Cys Val Ile His Asp Ala Trp Ser Gly Leu Arg His
           275                 280                 285
   Val Val Gln Leu Arg Ala Gln Glu Glu Phe Gly Gln Gly Glu Trp Ser
       290                 295                 300
   Glu Trp Ser Pro Glu Ala Met Gly Thr Pro Trp Thr Glu Ser Arg Ser
   305                 310                 315                 320
   Pro Pro Ala Arg Gly Gly Gly Gly Ser Gly Gly Gly Gly Ser Val Glu
                   325                 330                 335
   Pro Val Pro Pro Gly Glu Asp Ser Lys Asp Val Ala Ala Pro His Arg
               340                 345                 350
   Gln Pro Leu Thr Ser Ser Glu Arg Ile Asp Lys Gln Ile Arg Tyr Ile
           355                 360                 365
   Leu Asp Gly Ile Ser Ala Leu Arg Lys Glu Thr Cys Asn Lys Ser Asn
       370                 375                 380
   Met Cys Glu Ser Ser Lys Glu Ala Leu Ala Glu Asn Asn Leu Asn Leu
   385                 390                 395                 400
   Pro Lys Met Ala Glu Lys Asp Gly Cys Phe Gln Ser Gly Phe Asn Glu
                   405                 410                 415
   Glu Thr Cys Leu Val Lys Ile Ile Thr Gly Leu Leu Glu Phe Glu Val
               420                 425                 430
   Tyr Leu Glu Tyr Leu Gln Asn Arg Phe Glu Ser Ser Glu Glu Gln Ala
           435                 440                 445
   Arg Ala Val Gln Met Ser Thr Lys Val Leu Ile Gln Phe Leu Gln Lys
       450                 455                 460
   Lys Ala Lys Asn Leu Asp Ala Ile Thr Thr Pro Asp Pro Thr Thr Asn
   465                 470                 475                 480
   Ala Ser Leu Leu Thr Lys Leu Gln Ala Gln Asn Gln Trp Leu Gln Asp
                   485                 490                 495
   Met Thr Thr His Leu Ile Leu Arg Ser Phe Lys Glu Phe Leu Gln Ser
               500                 505                 510
   Ser Leu Arg Ala Leu Arg Gln Met
           515                 520
<210> 11
   <211> 121
   <212> PRT
   <213> Artificial Sequence
<220>
   <223> human plasminogen kringle 5 domain
<400> 11
   Met Tyr Arg Met Gln Leu Leu Ser Cys Ile Ala Leu Ser Leu Ala Leu
    1               5                  10                  15
   Val Thr Asn Ser Pro Val Val Leu Leu Pro Asp Val Glu Thr Pro Ser
               20                  25                  30
   Glu Glu Asp Cys Met Phe Gly Asn Gly Lys Gly Tyr Arg Gly Lys Arg
           35                  40                  45
   Ala Thr Thr Val Thr Gly Thr Pro Cys Gln Asp Trp Ala Ala Gln Glu
       50                  55                  60
   Pro His Arg His Ser Ile Phe Thr Pro Glu Thr Asn Pro Arg Ala Gly
   65                  70                  75                  80
   Leu Glu Lys Asn Tyr Cys Arg Asn Pro Asp Gly Asp Val Gly Gly Pro
                   85                  90                  95
   Trp Cys Tyr Thr Thr Asn Pro Arg Lys Leu Tyr Asp Tyr Cys Asp Val
               100                 105                 110
   Pro Gln Cys Ala Ala Pro Ser Phe Asp
           115                 120
<210> 12
   <211> 624
   <212> DNA
   <213> Artificial Sequence
<220>
   <223> IL-24 cDNA
<400> 12
   atgaattttc aacagaggct gcaaagcctg tggactttag ccagcagacc cttctgccct 60
   cctttgctgg cgacagcctc tcaaatgcag atggttgtgc tcccttgcct gggttttacc 120
   ctgcttctct ggagccaggt atcaggggcc cagggccaag aattccactt tgggccctgc 180
   caagtgaagg gggttgttcc ccagaaactg tgggaagcct tctgggctgt gaaagacact 240
   atgcaagctc aggataacat cacgagtgcc cggctgctgc agcaggaggt tctgcagaac 300
   gtctcggatg ctgagagctg ttaccttgtc cacaccctgc tggagttcta cttgaaaact 360
   gttttcaaaa actaccacaa tagaacagtt gaagtcagga ctctgaagtc attctctact 420
   ctggccaaca actttgttct catcgtgtca caactgcaac ccagtcaaga aaatgagatg 480
   ttttccatca gagacagtgc acacaggcgg tttctgctat tccggagagc attcaaacag 540
   ttggacgtag aagcagctct gaccaaagcc cttggggaag tggacattct tctgacctgg 600
   atgcagaaat tctacaagct ctga                                        624
<210> 13
   <211> 1572
   <212> DNA
   <213> Artificial Sequence
<220>
   <223> SIL-6R/IL-6 fusion CDNA
<400> 13
   atgctggccg tcggctgcgc gctgctggct gccctgctgg ccgcgccggg agcggcgctg 60
   gccccaaggc gctgccctgc gcaggaggtg gcgagaggcg tgctgaccag tctgccagga 120
   gacagcgtga ctctgacctg cccgggggta gagccggaag acaatgccac tgttcactgg 180
   gtgctcagga agccggctgc aggctcccac cccagcagat gggctggcat gggaaggagg 240
   ctgctgctga ggtcggtgca gctccacgac tctggaaact attcatgcta ccgggccggc 300
   cgcccagctg ggactgtgca cttgctggtg gatgttcccc ccgaggagcc ccagctctcc 360
   tgcttccgga agagccccct cagcaatgtt gtttgtgagt ggggtcctcg gagcacccca 420
   tccctgacga caaaggctgt gctcttggtg aggaagtttc agaacagtcc ggccgaagac 480
   ttccaggagc cgtgccagta ttcccaggag tcccagaagt tctcctgcca gttagcagtc 540
   ccggagggag acagctcttt ctacatagtg tccatgtgcg tcgccagtag tgtcgggagc 600
   aagttcagca aaactcaaac ctttcagggt tgtggaatct tgcagcctga tccgcctgcc 660
   aacatcacag tcactgccgt ggccagaaac ccccgctggc tcagtgtcac ctggcaagac 720
   ccccactcct ggaactcatc tttctacaga ctacggtttg agctcagata tcgggctgaa 780
   cggtcaaaga cattcacaac atggatggtc aaggacctcc agcatcactg tgtcatccac 840
   gacgcctgga gcggcctgag gcacgtggtg cagcttcgtg cccaggagga gttcgggcaa 900
   ggcgagtgga gcgagtggag cccggaggcc atgggcacgc cttggacaga atccaggagt 960
   cctccagctc gaggaggtgg aggttctgga ggtggaggtt ctgtcgagcc agtaccccca 1020
   ggagaagatt ccaaagatgt agccgcccca cacagacagc cactcacctc ttcagaacga 1080
   attgacaaac aaattcggta catcctcgac ggcatctcag ccctgagaaa ggagacatgt 1140
   aacaagagta acatgtgtga aagcagcaaa gaggcactgg cagaaaacaa cctgaacctt 1200
   ccaaagatgg ctgaaaaaga tggatgcttc caatctggat tcaatgagga gacttgcctg 1260
   gtgaaaatca tcactggtct tttggagttt gaggtatacc tagagtacct ccagaacaga 1320
   tttgagagta gtgaggaaca agccagagct gtgcagatga gtacaaaagt cctgatccag 1380
   ttcctgcaga aaaaggcaaa gaatctagat gcaataacca cccctgaccc aaccacaaat 1440
   gccagcctgc tgacgaagct gcaggcacag aaccagtggc tgcaggacat gacaactcat 1500
   ctcattctgc gcagctttaa ggagttcctg cagtccagcc tgagggctct tcggcaaatg 1560
   tagcatgggc ac                                                    1572
<210> 14
   <211> 366
   <212> DNA
   <213> Homo sapiens
<220>
   <223> DNA encoding the human plasminogen kringle 5 domain
<400> 14
   atgtacagga tgcaactcct gtcttgcatt gcactaagtc ttgcacttgt cacgaattcg 60
   cctgttgtcc tgcttccaga tgtagagact ccttccgaag aagactgtat gtttgggaat 120
   gggaaaggat accgaggcaa gagggcgacc actgttactg ggacgccatg ccaggactgg 180
   gctgcccagg agccccatag acacagcatt ttcactccag agacaaatcc acgggcgggt 240
   ctggaaaaaa attactgccg taaccctgat ggtgatgtag gtggtccctg gtgctacacg 300
   acaaatccaa gaaaacttta cgactactgt gatgtccctc agtgtgcggc cccttcattt 360
   gattag                                                           366